# EUROPEAN PATENT SPECIFICATION

(11) **EP 2 327 767 B1**
(45) Date of publication and mention of the grant of the patent: **29.04.2015**
(21) Application number: 10190399.5
(22) Date of filing: 15.05.2002
(51) Int. Cl.: C12N 9/00, C12N 9/78, C12N 1/20, C12N 15/00, C07H 21/02, C12P 1/00, C12P 7/00, C12P 7/62, C07K 1/00

(54) **Nitrilases**
Nitralasen
Nitralases

(30) Priority: 21.06.2001 US 300189 P; 30.07.2001 US 309006 P; 22.01.2002 US 351336 P
(43) Date of publication of application: 01.06.2011
(62) Divisional of application: 08075753.7
(73) Proprietor: BASF Enzymes LLC, San Diego, CA 92121 (US)
(72) Inventor: Chaplin, Jennifer Ann, San Diego, CA 92130 (US); Weiner, David Paul, Del Mar, CA 92104 (US); Milan, Aileen, San Diego, CA 92126 (US); Chi, Ellen, Del Mar, CA 92014 (US); Short, Jay M, Rancho Santa Fe, CA 92067-7214 (US); Madden, Mark, deceased (US); Burk, Mark, San Diego, CA 92130 (US); Robertson, Dan, Belmont, MA 02478 (US); Desantis, Grace, San Diego, CA 92129 (US)
(74) Representative: Wakerley, Helen Rachael

(56) References cited:
- EP-A2- 0 780 471

## Description

The invention relates generally to the field of molecular biology, biochemistry and chemistry, and particularly to enzymatic proteins having nitrilase activity. The invention also relates to polynucleotides encoding the enzymes, and to uses of such polynucleotides and enzymes.

### BACKGROUND OF THE INVENTION

There are naturally occurring enzymes which have great potential for use in industrial chemical processes for the conversion of nitriles to a wide range of useful products and intermediates. Such enzymes include nitrilases which are capable of converting nitriles directly to carboxylic acids. Nitrilase enzymes are found in a wide range of mesophilic micro-organisms, including species of *Bacillus, Norcardia, Bacteridium, Rhodococcus, Micrococcus, Brevibacterium, Alcaligenes, Acinetobacter, Corynebacterium, Fusarium* and *Klebsiella.* Additionally, there are thermophilic nitrilases which exist in bacteria.

There are two major routes from a nitrile to an analogous acid: (1) a nitrilase catalyzes the direct hydrolysis of a nitrile to a carboxylic acid with the concomitant release of ammonia; or (2) a nitrile hydratase adds a molecule of water across the carbon-nitrogen bonding system to give the corresponding amide, which can then act as a substrate for an amidase enzyme which hydrolyzes the carbon-nitrogen bond to give the carboxylic acid product with the concomitant release of ammonia. The nitrilase enzyme therefore provides the more direct route to the acid.

A nitrile group offers many advantages in devising synthetic routes in that it is often easily introduced into a molecular structure and can be carried through many processes as a masked acid or amide group. This is only of use, however, if the nitrile can be unmasked at the relevant step in the synthesis. Cyanide represents a widely applicable C₁-synthon (cyanide is one of the few water-stable carbanions) which can be employed for the synthesis of a carbon framework. However, further transformations of the nitrile thus obtained are impeded due to the harsh reaction conditions required for its hydrolysis using normal chemical synthesis procedures. The use of enzymes to catalyze the reactions of nitriles is attractive because nitrilase enzymes are able to effect reactions with fewer environmentally hazardous reagents and by-products than in many traditional chemical methods. Indeed, the chemoselective biocatalytic hydrolysis of nitriles represents a valuable alternative because it occurs at ambient temperature and near physiological pH.

The importance of asymmetric organic synthesis in drug design and discovery has fueled the search for new synthetic methods and chiral precursors which can be utilized in developing complex molecules of biological interest. One important class of chiral molecules are the α-substituted carboxylic acids, which include the α-amino acids. These molecules have long been recognized as important chiral precursors to a wide variety of complex biologically active molecules, and a great deal of research effort has been dedicated to the development of methods for the synthesis of enantiomerically pure α-amino acids and chiral medicines.

Of particular use to synthetic chemists who make chiral medicines would be an enzyme system which is useful under non-sterile conditions, which is useful in non-biological laboratories, which is available in a form convenient for storage and use; which has broad substrate specificity, which acts on poorly water soluble substrates; which has predictable product structure; which provides a choice of acid or amide product; and which is capable of chiral differentiation. Accordingly, there is a need for efficient, inexpensive, high-yield synthetic methods for producing enantiomerically pure α-substituted carboxylic acids, such as, for example, α-amino acids and α-hydroxy acids.

Throughout this application, various publications are referenced by author and date. The disclosures of these publications in their entireties are hereby incorporated by reference into this application in order to more fully describe the state of the art as known to those skilled therein as of the date of the invention described and claimed herein.

### SUMMARY OF THE INVENTION

The present invention is directed to an isolated, synthetic or recombinant nucleic acid comprising (a) a nucleic acid sequence having at least 70% sequence identity to SEQ ID NO: 297; wherein the nucleic acid encodes at least one polypeptide having nitrilase activity; or (b) a nucleic acid sequence complementary to (a). In an embodiment of the invention, the nucleic acid comprises consecutive nucleotides having a sequence at least 70%, 71%, 72%, 73%, 74%, 75%, 76%, 77%, 78%, 79%, 80%, 81%, 82%, 83%, 84%, 85%, 90%, 91%, 92%, 93%, 94%, 95%, 96%, 97%, 98%, 99%, or more, or 100% identical to SEQ ID NO 297. In an embodiment of the invention, the nucleic acid comprises consecutive nucleotides having a sequence substantially identical to the SEQ ID NO. A fragment of the nucleic acid may encode a polypeptide having nitrilase activity. An isolated nucleic acid may hybridize to any one of the nucleic acids under stringent conditions. The stringent conditions may comprise at least 50% formamide, and about 37°C to about 42°C.

A nucleic acid probe may comprise from about 15 nucleotides to about 50 nucleotides, wherein at least 15 consecutive nucleotides are at least 50% complementary to a nucleic acid target region within a nucleic acid sequence of the invention. The nucleic acid probe may comprise consecutive nucleotides which are at least 55% complementary to the nucleic acid target region, or the consecutive nucleotides are at least 60%, 65%, 70%, 75%, 80%, 85%, 90%, 95%, 97%, 98% or 100% complementary to the nucleic acid target region. The nucleic acid may consist essentially of from about 20 to about 50 nucleotides.

The invention provides for a nucleic acid vector capable of replication in a host cell, wherein the vector comprises the nucleic acid of the invention. The invention also provides for a host cell comprising the nucleic acid. The invention also provides for a non-human host organism comprising the host cell. In one embodiment, the host organism comprises a gram negative bacterium, a gram positive bacterium or a eukaryotic non-human organism. In another embodiment, the gram negative bacterium comprises *Escherichia coli,* or *Pseudomonas fluorescens.* In a further embodiment, the gram positive bacterium comprises *Streptomyces diversa, Lactobacillus gasseri, Lactococcus lactis, Lactococcus cremoris,* or *Bacillus subtilis.* In a further embodiment, the eukaryotic organism comprises *Saccharomyces cerevisiae, Schizosaccharomyces pombe, Pichia pastoris, Kluyveromyces lactis, Hansenula plymorpha,* or *Aspergillus niger.*

The invention provides for an isolated nucleic acid encoding a polypeptide comprising consecutive amino acids having a sequence at least 70% identical to SEQ ID NO: 298 wherein the polypeptide has nitrilase activity. In one embodiment, the polypeptide comprises consecutive amino acids having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, or 100% identity to the SEQ YID NO.

An isolated nucleic acid may encode a polypeptide comprising at least 10 consecutive amino acids having a sequence identical to a portion of an amino acid sequence of SEQ ID NO: 298.

An isolated polypeptide is provided, comprising consecutive amino acids having a sequence at least 70% identical to SEQ ID NO: 298 wherein the polypeptide has nitrilase activity. In one embodiment of the invention, the polypeptide comprises consecutive amino acids having a sequence at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, or 100% identical to the SEQ ID NO.

The disclosure provides an isolated nucleic acid comprising consecutive nucleotides having a sequence as set forth in any one of the following SEQ ID NOS:1, 3, 5, 7, 9, 11, 13, 15, 17, 19, 21, 23, 25, 27, 29, 31, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, or 385 (hereinafter referred to as "Group A nucleic acids"), as well as nucleic acids having specified minimum % of sequence identity.

The disclosure also provides a purified polypeptide comprising consecutive amino acid residues having a sequence as set forth in any one of the following SEQ ID NOS:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384 and 386 (hereinafter referred to as "Group B amino acid sequences"), as well as polypeptides having specified minimum % of sequence identity.

A fragment of the polypeptide which is at least 50 amino acids in length may have nitrilase activity. A peptidomimetic of the polypeptide or a fragment thereof may have nitrilase activity. A codon-optimized polypeptide or a fragment thereof, may have nitrilase activity, wherein the codon usage is optimized for a particular organism or cell. Narum et al. Infect. Immun. 2001 Dec, 69(12):7250-3 describes codon-optimzation in the mouse system. Outchkourov et al. Protein Expr. Purif. 2002 Feb; 24(1):18-24 describes codon-optimization in the yeast system. Feng et al. Biochemistry 2000 Dec 19, 39(50):15399-409 describes codon-optimization in E. *coli.* Humphreys et al. Protein Expr. Purif. 2000 Nov, 20(2):252-64 describes how codon usage affects secretion in *E. coli.*

In one embodiment, the organism or cell comprises a gram negative bacterium, a gram positive bacterium or a eukaryotic non-human organism. In another embodiment of the invention, the gram negative bacterium comprises *Escherichia coli,* or *Pseudomonas fluorescens.* In another embodiment of the invention, the gram positive bacterium comprise *Streptomyces diversa, Lactobacillus gasseri, Lactococcus lactis, Lactococcus cremoris,* or *Bacillus subtilis.* In another embodiment of the invention, the eukaryotic organism comprises *Saccharomyces cerevisiae, Schizosaccharomyces pombe, Pichia pastoris, Kluyveromyces lactis, Hansenula plymorpha,* or *Aspergillus niger.*

There is disclosed a purified antibody that specifically binds to the polypeptide of the invention or a fragment thereof, having nitrilase activity, as well as a fragment of the antibody that specifically binds to a polypeptide having nitrilase activity.

The invention provides for an enzyme preparation which comprises at least one of the polypeptides of the invention, wherein the preparation is liquid or dry. The enzyme preparation includes a buffer, cofactor, or second or additional protein. In one embodiment the preparation is affixed to a solid support. In one embodiment of the invention, the solid support can be a gel, a resin, a polymer, a ceramic, a glass, a microelectrode and any combination thereof. In another embodiment, the preparation can be encapsulated in a gel or a bead.

The invention further provides for a composition which comprises at least one nucleic acid of the invention which comprises at least one polypeptide of the invention or a fragment thereof, or a peptidomimetic thereof, having nitrilase activity, or any combination thereof.

The invention provides for a method for hydrolyzing a nitrile to a carboxylic acid comprising contacting the molecule with at least one polypeptide of the invention or a fragment thereof, or a peptidomimetic thereof, having nitrilase activity, under conditions suitable for nitrilase activity. In one embodiment, the conditions comprise aqueous conditions. In another embodiment, the conditions comprise a pH of about 8.0 and/or a temperature from about 37° C to about 45° C.

The invention provides for a method for hydrolyzing a cyanohydrin moiety or an aminonitrile moiety of a molecule, the method comprising contacting the molecule with at least one polypeptide of the invention, or a fragment thereof, or a peptidomimetic thereof, having nitrilase activity, under conditions suitable for nitrilase activity.

The invention provides for a method for making a chiral α-hydroxy acid molecule, a chiral amino acid molecule, a chiral β-hydroxy acid molecule, or a chiral gamma-hydroxy acid molecule, the method comprising admixing a molecule having a cyanohydrin moiety or an aminonitrile moiety with at least one polypeptide of the invention or a fragment thereof, or a peptidomimetic thereof, having enantio-selective nitrilase activity. In one embodiment, the chiral molecule is an *(R)*-enantiomer. In another embodiment, the chiral molecule is an *(S)*-enantiomer. In one embodiment of the invention, one particular enzyme can have R-specificity for one particular substrate and the same enzyme can have S-specificity for a different particular substrate.

The invention also provides for a method for making a composition or an intermediate thereof, the method comprising admixing a precursor of the composition or intermediate, wherein the precursor comprises a cyanohydrin moiety or an aminonitrile moiety, with at least one polypeptide of the invention or a fragment or peptidomimetic thereof having nitrilase activity, hydrolyzing the cyanohydrin or the aminonitrile moiety in the precursor thereby making the composition or the intermediate thereof. In one embodiment, the composition or intermediate thereof comprises *(S)*-2-amino-4-phenyl butanoic acid. In a further embodiment, the composition or intermediate thereof comprises an L-amino acid. In a further embodiment, the composition comprises a food additive or a pharmaceutical drug.

The invention provides for a method for making an *(R)*-ethyl 4-cyano-3-hydroxybutyric acid, the method comprising contacting a hydroxyglutaryl nitrile with at least one polypeptide of the invention or a fragment or peptidomimetic thereof having nitrilase activity that selectively produces an *(R)*-enantiomer, so as to make *(R)*-ethyl 4-cyano-3-hydroxybutyric acid. In one embodiment, the *ee* is at least 95% or at least 99%. In another embodiment, the hydroxyglutaryl nitrile comprises 1,3-di-cyano-2-hydroxy-propane or 3-hydroxyglutaronitrile.

The invention also provides a method for making an *(S)*-ethyl 4-cyano-3-hydroxybutyric acid, the method comprising contacting a hydroxyglutaryl nitrile with at least one polypeptide of the invention or a fragment or peptidomimetic thereof having nitrilase activity that selectively produces an *(S)*-enantiomer, so as to make *(S)-*ethyl 4-cyano-3-hydroxybutyric acid.

The invention provides a method for making an *(R)*-mandelic acid, the method comprising admixing a mandelonitrile with at least one polypeptide having an amino acid sequence of the invention or any fragment or peptidomimetic thereof having appropriate nitrilase activity. In one embodiment, the *(R)*-mandelic acid comprises *(R)*-2-chloromandelic acid. In another embodiment, the *(R)*-mandelic acid comprises an aromatic ring substitution in the *ortho*-, *meta*-, or *para-* positions; a 1-naphthyl derivative of *(R)*-mandelic acid, a pyridyl derivative of *(R)*-mandelic acid or a thienyl derivative of *(R)*-mandelic acid or a combination thereof.

The invention provides a method for making an *(S)*-mandelic acid, the method comprising admixing a mandelonitrile with at least one polypeptide having an amino acid sequence of the invention or any fragment or peptidomimetic thereof having nitrilase activity. In one embodiment, the *(S)*-mandelic acid comprises (*S*)-methyl benzyl cyanide and the mandelonitrile comprises *(S)*-methoxy-benzyl cyanide. In one embodiment, the *(S)*-mandelic acid comprises an aromatic ring substitution in the *ortho-*, *meta*-, or *para-* positions; a 1-naphthyl derivative of *(S)*-mandelic acid, a pyridyl derivative of *(S)*-mandelic acid or a thienyl derivative of *(S)*-mandelic acid or a combination thereof.

The invention also provides a method for making an *(S)*-phenyl lactic acid derivative or an *(R)*-phenyllactic acid derivative, the method comprising admixing a phenyllactonitrile with at least one polypeptide of the invention or any active fragment or peptidomimetic thereof that selectively produces an *(S)*-enantiomer or an *(R)-*enantiomer, thereby producing an *(S)*-phenyl lactic acid derivative or an *(R)*-phenyl lactic acid derivative.

The invention provides for a method for making the polypeptide of the invention or a fragment thereof, the method comprising (a) introducing a nucleic acid encoding the polypeptide into a host cell under conditions that permit production of the polypeptide by the host cell, and (b) recovering the polypeptide so produced.

The invention provides for a method for generating a nucleic acid variant encoding a polypeptide having nitrilase activity, wherein the variant has an altered biological activity from that which naturally occurs, the method comprising (a) modifying the nucleic acid by (i) substituting one or more nucleotides for a different nucleotide, wherein the nucleotide comprises a natural or non-natural nucleotide; (ii) deleting one or more nucleotides, (iii) adding one or more nucleotides, or (iv) any combination thereof. In one embodiment, the non-natural nucleotide comprises inosine. In another embodiment, the method further comprises assaying the polypeptides encoded by the modified nucleic acids for altered nitrilase activity, thereby identifying the modified nucleic acid(s) encoding a polypeptide having altered nitrilase activity. In one embodiment, the modifications of step (a) are made by PCR, error-prone PCR, shuffling, oligonucleotide-directed mutagenesis, assembly PCR, sexual PCR mutagenesis, in vivo mutagenesis, cassette mutagenesis, recursive ensemble mutagenesis, exponential ensemble mutagenesis, site-specific mutagenesis, gene reassembly, gene site saturated mutagenesis, ligase chain reaction, *in vitro* mutagenesis, ligase chain reaction, oligonuclteotide synthesis, any DNA-generating technique and any combination thereof. In another embodiment, the method further comprises at least one repetition of the modification step (a).

The invention further provides a method for making a polynucleotide from two or more nucleic acids, the method comprising: (a) identifying regions of identity and regions of diversity between two or more nucleic acids, wherein at least one of the nucleic acids comprises a nucleic acid of the invention; (b) providing a set of oligonucleotides which correspond in sequence to at least two of the two or more nucleic acids; and, (c) extending the oligonucleotides with a polymerase, thereby making the polynucleotide.

A screening assay for identifying a nitrilase may comprise: (a) providing a plurality of nucleic acids or polypeptides comprising at least one of the nucleic acids of the invention, or at least one of the polypeptides of the invention; (b) obtaining polypeptide candidates to be tested for nitrilase activity from the plurality; (c) testing the candidates for nitrilase activity; and (d) identifying those polypeptide candidates which are nitrilases. The method may further comprise modifying at least one of the nucleic acids or polypeptides prior to testing the candidates for nitrilase activity. The testing of step (c) may further comprise testing for improved expression of the polypeptide in a host cell or host organism. The testing of step (c) may further comprise testing for nitrilase activity within a pH range from about pH 3 to about pH 12. The testing of step (c) may further comprise testing for nitrilase activity within a pH range from about pH 5 to about pH 10. The testing of step (c) may further comprise testing for nitrilase activity within a temperature range from about 4 °C to about 80 °C. The testing of step (c) may further comprise testing for nitrilase activity within a temperature range from about 4 °C to about 55 °C. The testing of step (c) may further comprise testing for nitrilase activity which results in an enantioselective reaction product. The testing of step (c) may further comprise testing for nitrilase activity which results in a regio-selective reaction product.

The invention provides for use of the nucleic acids of the invention, or a fragment or peptidomimetic thereof having nitrilase activity, in a process designed to optimize an aspect of the gene or an aspect of the polypeptide encoded by the gene. In one embodiment, the process comprises introducing modifications into the nucleotide sequence of the nucleic acid. In another embodiment, the modifications are introduced by PCR, error-prone PCR, shuffling, oligonucleotide-directed mutagenesis, assembly PCR, sexual PCR mutagenesis, in vivo mutagenesis, cassette mutagenesis, recursive ensemble mutagenesis, exponential ensemble mutagenesis, site-specific mutagenesis, gene reassembly, gene site saturated mutagenesis, ligase chain reaction, *in vitro* mutagenesis, ligase chain reaction, oligonucleotide synthesis, any other DNA-generating technique or any combination thereof. In a further embodiment, the process is repeated.

The invention provides for use of the polypeptide of the invention, or a fragment or peptidomimetic thereof having nitrilase activity, in an industrial process. In one embodiment, the process is for production of a pharmaceutical composition, the process is for production of a chemical, the process is for production of a food additive, the process is catalyzing the breakdown of waste, or the process is production of a drug intermediate. In a further embodiment, the process comprises use of the polypeptide to hydrolyze a hydroxyglutarylnitrile substrate. In a further embodiment, the process is for production of LIPITOR™. In another embodiment, the process is production of a detergent. In another embodiment, the process is production of a food product.

The invention provides for use of a nucleic acid of the invention, or a fragment thereof encoding a polypeptide having nitrilase activity, in the preparation of a transgenic organism.

A kit may comprise (a) the nucleic acid of the inventions, or a fragment thereof encoding a polypeptide having nitrilase activity, or (b) the polypeptide of the invention, or a fragment or a peptidomimetic thereof having nitrilase activity, or a combination thereof; and (c) a buffer.

The invention provides for a method for modifying a molecule comprising: (a) mixing a polypeptide of the invention or a fragment or peptidomimetic thereof having nitrilase activity, with a starting molecule to produce a reaction mixture; (b) reacting the starting molecule with the polypeptide to produce the modified molecule.

The invention provides for a method for identifying a modified compound comprising: (a) admixing a polypeptide of the invention, or a fragment or peptidomimetic thereof having nitrilase activity, with a starting compound to produce a reaction mixture and thereafter a library of modified starting compounds; (b) testing the library to determine whether a modified starting compound is present within the library which exhibits a desired activity; (c) identifying the modified compound exhibiting the desired activity.

A computer readable medium may have stored thereon at least one nucleotide sequence selected from the group consisting of: SEQ ID NO: 1, 3, 5, 7, 9, 11, 13, 15, 17, 19, 21, 23, 25, 27, 29, 31, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383, and 385 and/or at least one amino acid sequence selected from the group consisting of: SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384 and 386.

A computer system may comprise a processor and a data storage device, wherein the data storage device has stored thereon at least one nucleotide sequence selected from the group consisting of SEQ ID NO: 1, 3, 5, 7, 9, 11, 13, 15, 17, 19, 21, 23, 25, 27, 29, 31, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, 377, 379, 381, 383 and 385 and/or at least one amino acid sequence selected from the group consisting of: SEQ ID NO:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382, 384 and 386. The computer system may further comprise a sequence comparison algorithm and a data storage device having at least one reference sequence stored thereon. The sequence comparison algorithm may comprise a computer program that identifies polymorphisms.

A method for identifying a feature in a sequence which comprises: (a) inputting the sequence into a computer; (b) running a sequence feature identification program on the computer so as to identify a feature within the sequence; and (c) identifying the feature in the sequence, wherein the sequence comprises at least one of SEQ ID NOS:1-386 or any combination thereof.

An assay for identifying a functional fragment of a polypeptide which comprises: (a) obtaining a fragment of at least one polypeptide of the invention; (b) contacting at least one fragment from step (a) with a substrate having a cyanohydrin moiety or an aminonitrile moiety under reaction conditions suitable for nitrilase activity; (c) measuring the amount of reaction product produced by each at least one fragment from step (b); and (d) identifying the at least one fragment which is capable of producing a nitrilase reaction product; thereby identifying a functional fragment of the polypeptide. The fragment of step (a) may be obtained by synthesizing the fragment. The fragment of step (a) may be obtained by fragmenting the polypeptides. An assay for identifying a functional variant of a polypeptide comprises: (a) obtaining at least one variant of at least one polypeptide of the invention; (b) contacting at least one variant from step (a) with a substrate having a cyanohydrin moiety or an aminonitrile moiety under reaction conditions suitable for nitrilase activity; (c) measuring the amount of reaction product produced by each at least one variant from step (b); and (d) identifying the at least one variant which is capable of producing a nitrilase reaction product; thereby identifying a functional variant of the polypeptide.

Other aspects of the invention are set out in the accompanying claims.

### BRIEF DESCRIPTION OF THE DRAWINGS

**Figure 1** shows chemical reaction schemes wherein stereoselective nitrilases hydrolyze a cyanohydrin or an aminonitrile to produce a chiral α-hydroxy acid or α-amino acid.

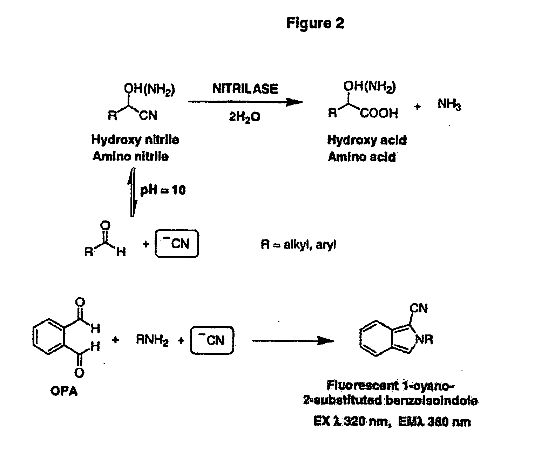

**Figure 2** illustrates an OPA based cyanide detection assay used for identifying the presence of nitrilase activity.
**Figure 3** is an illustration of a spectroscopic system for the detection and quantification of α-hydroxy acids based on stereoselective lactate dehydrogenases.
**Figure 4** is an illustration of a spectroscopic system for the detection and quantification of α-amino acids based on stereoselective amino acid oxidase.
**Figure 5** is a flow diagram illustrating the steps of a nitrilase screening method.
**Figures 6A-6E** are chromatograms characteristic of the substrate and product combination for D-phenylglycine showing a blank sample (Fig. 6A), an enzymatic reaction sample (Fig. 6B); a negative control consisting of cell lysate in buffer (Fig. 6C); a chiral analysis of phenylglycine (Fig. 6D); and coelution of the nitrile peak with the D-enantiomer (Fig. 6E).
**Figures 7A-7E** illustrate chromatograms which are characteristic of substrate and product combinations for (R)-2-chloromandelic acid. Fig. 7A shows only 2-chloromandelonitrile in buffer; Fig. 7B shows a cloromandelic acid standard. The chromatogram in Fig. 7C shows the appearance of product and the reduction of substrate peaks.
**Figures 8A-8B** illustrate chromatograms characteristic of substrate and product combinations for (S)-phenyllactic acid.
**Figures 9A-9B** illustrate chromatograms characteristic of substrate and product combinations for L-2-methylphenylglycine.
**Figures 10A-10C** illustrate chromatograms characteristic of substrate and product combinations for L-*tert*-leucine.
**Figures 11A-11C** illustrate chromatograms characteristic of substrate and product combinations for (S)-2-amino-6-hydroxy hexanoic acid.
**Figures 12A-12D** illustrate chromatograms characteristic of substrate and product combinations for 4-methyl-D-leucine and 4-methyl-L-leucine.
**Figures 13A-13B** illustrate chromatograms characteristic of substrate and product combinations for (*S*)-cyclohexylmandelic acid.

### DETAILED DESCRIPTION OF THE INVENTION

The present invention relates to nitrilases, nucleic acids encoding nitrilases, and uses therefor. As used herein, the term "nitrilase" encompasses any polypeptide having nitrilase activity, *i.e*., the ability to hydrolyze nitriles into their corresponding carboxylic acids and ammonia. Nitrilases have commercial utility as biocatalysts for use in the synthesis of enantioselective aromatic and aliphatic amino acids or hydroxy acids.

Nitrilase chemistry is as follows:

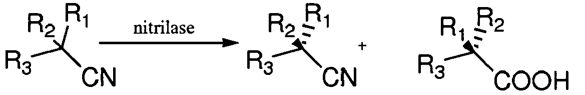

A nitrilase reaction for the preparation of hydroxy acids is as follows:

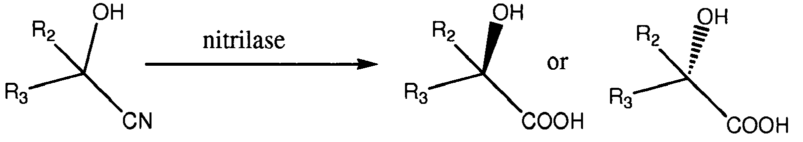

A nitrilase reaction for the preparation of amino acids is as follows:

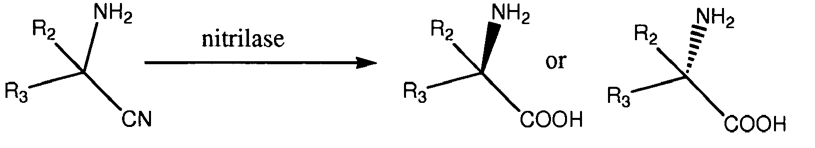

In addition, in each of the foregoing hydrolysis reactions, two water molecules are consumed and one ammonia molecule is released.

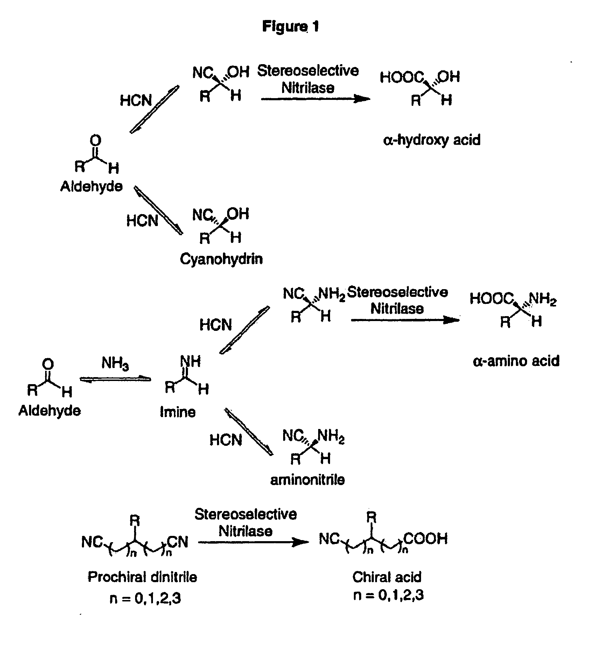

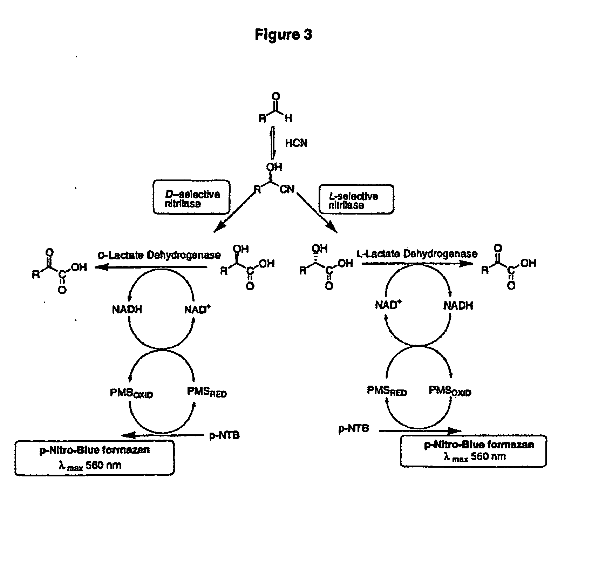

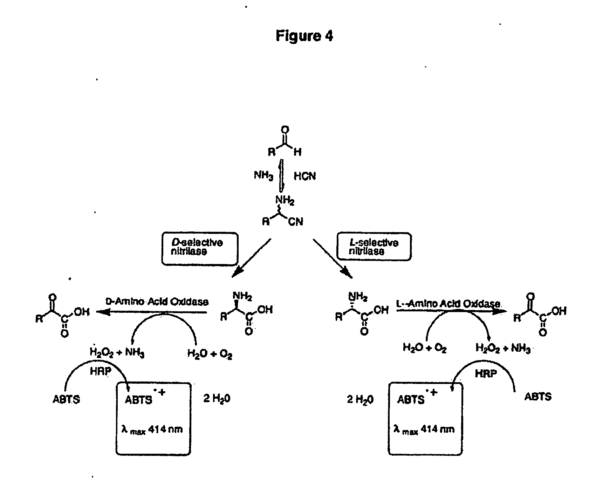

There are several different types of assays which can be performed to test for the presence of nitrilase activity in a sample or to test whether a particular polypeptide exhibits nitrilase activity. For example, assays can detect the presence or absence of products or by-products from a chemical reaction catalyzed by a nitrilase. For example, the presence of nitrilase activity can be detected by the production of α-hydroxy acids or α-amino acids from, respectively, cyanohydrins or aminonitriles, and the level of nitrilase activity can be quantified by measuring the relative quantities of the reaction products produced. Figure 1 shows chemical reaction schemes using stereoselective nitrilases to create chiral α-hydroxy acids or α-amino acids in high yield. The starting material is an aldehyde or an imine which is produced from an aldehyde by reaction with ammonia. Reaction of the aldehyde or imine with hydrogen cyanide results in the production of enantiomeric mixtures of the corresponding cyanohydrins and aminonitriles. A stereoselective nitrilase can then be used to stereoselectively convert one enantiomer into the corresponding α-hydroxy acid or α-amino acid. Figure 3 illustrates schematically the stereoselective nitrilase-dependent production and spectrophotometric detection of α-hydroxy acids based on lactate dehydrogenase conversion of the α-hydroxy acids to the corresponding α-keto acids and concomitant oxidation-reduction of a detectable dye. Figure 4 illustrates schematically the stereoselective nitrilase-dependent production and spectrophotometric detection of α-amino acids based on amino acid oxidase conversion of the α-amino acids to the corresponding α-keto acids and concomitant oxidation-reduction of a detectable dye.

Nitrilases contemplated for use in the practice of the present invention include those which stereoselectively hydrolyze nitriles or cyanohydrins into their corresponding acids and ammonia. Nitrilases of the disclosure include, for example, those set forth in the Group B amino acid sequences. Some nitrilases which stereoselectively hydrolyze their substrates are set forth in the Tables hereinbelow.

The nitrilases described herein share the following additional characteristics: (1) full-length amino acid sequences from about 333 amino acids to about 366 amino acids, (2) aggregation and activity as homo-multimers of about 2 subunits to about 16 subunits, (3) presence of a catalytic triad of the consecutive amino acids Glu-Lys-Cys, (4) pH optima from about pH 5 to about pH 9, and (5) temperature optima from about 0°C to about 100°C, or from about 40°C to about 50°C.

### Consensus Sequences Among New Nitrilases

The nitrilases disclosed herein were studied using bioinformatics and sequence comparison programs and the following consensus information was collected. Three regions of conserved motifs were identified within the nitrilase polypeptides. These correspond to the catalytic triad (E-K-C) present in nitrilase enzymes. (H. Pace and C. Brenner (Jan. 15, 2001) "The Nitrilase Superfamily: classification, structure and function" Genome Biology Vol. 2, No. 1, pp 1-9.)

The abbreviations used herein are conventional one letter codes for the amino acids: A, alanine; B, asparagine or aspartic acid; C, cysteine; D aspartic acid; E, glutamate, glutamic acid; F, phenylalanine; G, glycine; H histidine; I isoleucine; K, lysine; L, leucine; M, methionine; N, asparagine; P, proline; Q, glutamine; R, arginine; S, serine; T, threonine; V, valine; W, tryptophan; Y, tyrosine; Z, glutamine or glutamic acid. See L. Stryer, Biochemistry, 1988, W. H. Freeman and Company, New York.

The computer sequence comparisons made among the nitrilase polypeptide sequences of the invention resulted in the identification of these motifs within each amino acid sequence:

The following residues (those that are underlined) are completely conserved among all of the identified nitrilases: the third amino acid in the first motif or region (E, glutamate); the second residue in the second motif (R, arginine); the third residue in the second motif (K, lysine); the third residue in the third motif (C, cysteine); and the fifth residue in the third motif (E, glutamate).

In the boxes, upper case letters indicate 90% or greater consensus among the nitrilases of the invention, while lower case letters indicate 50% or greater consensus. An italicized letter indicates 30% or greater consensus among the nitrilases of the invention. A dot in a box indicates a residue which is not conserved.

The sequences of nitrilases in the nitrilase branch of the nitrilase superfamily were described as having a catalytic triad in the Pace and Brenner article (Genome Biology, 2001, Vol. 2, No. 1, pp. 1-9). However, the catalytic triad regions of the nitrilases of this invention differ from those previously identified in the Pace and Brenner reference in the following ways:

Differences in the first motif: The F in the first box of the first motif is conserved in 90% of the nitrilases of the invention, rather than in only 50% of those previously identified. The fourth residue of the first motif is a "t", threonine in the nitrilases of this invention, and it is found at 50% or greater consensus. However, that residue was identified by Pace and Brenner as "a" (alanine). The last residue of the first motif was identified as "f" (phenylalanine) and was indicated to occur at 50% or greater consensus. However, the nitrilases of this invention only show "f" (phenylalanine occurring at 30% consensus.

Differences in the second motif: There is an "r" (arginine) in the first box of the second motif of the nitrilases of this invention. However, the Pace and Brenner consensus shows an "h" (histidine) in that position. The "R" (arginine) in the second box is completely conserved in the nitrilases of the present invention, however that residue only appears at 90% consensus in the Pace and Brenner reference. The "L" (leucine) in the fourth box of the second motif is conserved in 90% or more of the nitrilases of this invention. However, the Pace and Brenner nitrilases only showed conservation of that residue in 50% of the sequences. Similarly, the "P" (proline) at the sixth box of the second motif is conserved in 90% or more of the nitrilases of this invention. However, the Pace and Brenner nitrilases only showed conservation of that residue in 50% of the sequences.

Differences in the third motif: The "L in the first box is conserved at 90% or greater in the nitrilases of the invention. However, the Pace and Brenner reference only shows that residue appearing 50% of the time. Finally, the sixth box in the third motif in the nitrilases of the invention show a histidine 50% of the time or more. However, the Pace and Brenner reference indicates that that position shows an asparagine ("n") 50% of the time.

The disclosure provides for an isolated polypeptide having nitrilase activity which polypeptide comprises three regions, wherein the first region comprises five amino acids and wherein the first amino acid of the first region is F and the fourth amino acid of the first region is T. An isolated polypeptide having nitrilase activity may comprise three regions, wherein the second region comprises seven amino acids and wherein the first amino acid of the second region is R, wherein the second amino acid of the second region is R, and wherein the sixth amino acid of the second region is P. An isolated polypeptide having nitrilase activity may comprise three regions, wherein the third region comprises nine amino acids and wherein the first amino acid of the third region is L and the sixth amino acid of the third region is H.

An isolated polypeptide having nitrilase activity may comprise three consenus subsequences, wherein the first consensus subsequence is FPETF, wherein the second consensus subsequence is RRKLXPT, and wherein the third consensus subsequence is LXCWEHXXP.

An isolated polypeptide having nitrilase activity may comprise three consenus subsequences, wherein the first consensus subsequence is FPEXX, wherein the second consensus subsequence is XRKLXPT, and wherein the third consensus subsequence is LXCWEXXXP.

In accordance with the present invention, methods are provided for producing enantiomerically pure α-substituted carboxylic acids. The enantiomerically pure α-substituted carboxylic acids produced by the methods of the present invention have the following structure: 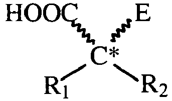 wherein:
R₁ ≠ R₂ and R₁ and R₂ are otherwise independently -H, substituted or unsubstituted alkyl, alkenyl, alkynyl, aryl, heteroaryl, cycloalkyl, or heterocyclic, wherein said substituents are lower alkyl, hydroxy, alkoxy, amino, mercapto, cycloalkyl, heterocyclic, aryl, heteroaryl, aryloxy, or halogen or optionally R₁ and R₂ are directly or indirectly covalently joined to form a functional cyclic moiety, and E is -N(Rₓ)₂ or - OH, wherein each Rₓ is independently -H or lower alkyl.

As used herein, the term "alkyl" refers to straight or branched chain or cyclic hydrocarbon groups of from 1 to 24 carbon atoms, including methyl, ethyl, n-propyl, isopropyl, n-butyl, isobutyl, tert-butyl, n-pentyl, n-hexyl, and the like. The term "lower alkyl" refers to monovalent straight or branched chain or cyclic radicals of from one to about six carbon atoms.

As used herein, "alkenyl" refers to straight or branched chain or cyclic hydrocarbon groups having one or more carbon-carbon double bonds, and having in the range of about 2 to about 24 carbon atoms.

As used herein, "alkynyl" refers to straight or branched chain or cyclic hydrocarbon groups having at least one carbon-carbon triple bond, and having in the range of about 2 to about 24 carbon atoms.

As used herein, "cycloalkyl" refers to cyclic hydrocarbon groups containing in the range of about 3 to about 14 carbon atoms.

As used herein, "heterocyclic" refers to cyclic groups containing one or more heteroatoms *(e.g.,* N, O, S, P, Se, B, etc.) as part of the ring structure, and having in the range of about 3 to about 14 carbon atoms.

As used herein, "aryl" refers to aromatic groups (*i.e*., cyclic groups with conjugated double-bond systems) having in the range of about 6 to about 14 carbon atoms.

As used herein with respect to a chemical group or moiety, the term "substituted" refers to such a group or moiety further bearing one or more non-hydrogen substituents. Examples of such substituents include, without limitation, oxy *(e.g.,* in a ketone, aldehyde, ether, or ester), hydroxy, alkoxy (of a lower alkyl group), amino, thio, mercapto (of a lower alkyl group), cycloalkyl, substituted cycloalkyl, heterocyclic, substituted heterocyclic, aryl, substituted aryl, heteroaryl, substituted heteroaryl, aryloxy, substituted aryloxy, halogen, trifluoromethyl, cyano, nitro, nitrone, amino, amido, -C(O)H, acyl, oxyacyl, carboxyl, carbamate, sulfonyl, sulfonamide, sulfuryl, and the like.

In preferred embodiments, the enantiomerically pure α-substituted carboxylic acid produced by the methods of the present invention is an α-amino acid or α-hydroxy acid. In some embodiments, the enantiomerically pure α-amino acid is D-phenylalanine, D-phenylglycine, L-methylphenylglycine, L-*tert*-leucine, D-alanine, or D-hydroxynorleucine ((S)-2-amino-6-hydroxy hexanoic acid), R-pantolactone, 2-chloromandelic acid, or (S)- or (R)-mandelic acid and the enantiomerically pure α-hydroxy acid is (*S*)-cyclohexylmandelic acid. As used herein, a "small molecule" encompasses any molecule having a molecular weight from at least 25 Daltons.

The term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent up or down (higher or lower).

As used herein, the word "or" means any one member of a particular list and also includes any combination of members of that list.

The phrase "nucleic acid" as used herein refers to a naturally occurring or synthetic oligonucleotide or polynucleotide, whether DNA or RNA or DNA-RNA hybrid, single-stranded or double-stranded, sense or antisense, which is capable of hybridization to a complementary nucleic acid by Watson-Crick base-pairing. Nucleic acids of the disclosure can also include nucleotide analogs (*e.g*., BrdU), and non-phosphodiester internucleoside linkages (*e.g*., peptide nucleic acid (PNA) or thiodiester linkages). In particular, nucleic acids can include, without limitation, DNA, RNA, cDNA, gDNA, ssDNA or dsDNA or any combination thereof. A "nucleic acid" may include, for example, a nucleic acid encoding a polypeptide as set forth in the Group B amino acid sequences, and variants thereof. The phrase "a nucleic acid sequence" as used herein refers to a consecutive list of abbreviations, letters, characters or words, which represent nucleotides. A nucleic acid can be a "probe" which is a relatively short nucleic acid, usually less than 100 nucleotides in length. Often a nucleic acid probe is from about 50 nucleotides in length to about 10 nucleotides in length. A "target region" of a nucleic acid is a portion of a nucleic acid that is identified to be of interest.

A "coding region" of a nucleic acid is the portion of the nucleic acid which is transcribed and translated in a sequence-specific manner to produce into a particular polypeptide or protein when placed under the control of appropriate regulatory sequences. The coding region is said to encode such a polypeptide or protein.

The term "gene" refers to a coding region operably joined to appropriate regulatory sequences capable of regulating the expression of the polypeptide in some manner. A gene includes untranslated regulatory regions of DNA (*e.g*., promoters, enhancers, repressors, etc.) preceding (upstream) and following (downstream) the coding region (open reading frame, ORF) as well as, where applicable, intervening sequences (*i.e*., introns) between individual coding regions (*i.e*., exons).

"Polypeptide" as used herein refers to any peptide, oligopeptide, polypeptide, gene product, expression product, or protein. A polypeptide is comprised of consecutive amino acids. The term "polypeptide" encompasses naturally occurring or synthetic molecules.

In addition, as used herein, the term "polypeptide" refers to amino acids joined to each other by peptide bonds or modified peptide bonds, *e.g*., peptide isosteres, and may contain modified amino acids other than the 20 gene-encoded amino acids. The polypeptides can be modified by either natural processes, such as post-translational processing, or by chemical modification techniques which are well known in the art. Modifications can occur anywhere in the polypeptide, including the peptide backbone, the amino acid side-chains and the amino or carboxyl termini. It will be appreciated that the same type of modification can be present in the same or varying degrees at several sites in a given polypeptide. Also a given polypeptide can have many types of modifications. Modifications include, without limitation, acetylation, acylation, ADP-ribosylation, amidation, covalent cross-linking or cyclization, covalent attachment of flavin, covalent attachment of a heme moiety, covalent attachment of a nucleotide or nucleotide derivative, covalent attachment of a lipid or lipid derivative, covalent attachment of a phosphytidylinositol, disulfide bond formation, demethylation, formation of cysteine or pyroglutamate, formylation, gamma-carboxylation, glycosylation, GPI anchor formation, hydroxylation, iodination, methylation, myristolyation, oxidation, pergylation, proteolytic processing, phosphorylation, prenylation, racemization, selenoylation, sulfation, and transfer-RNA mediated addition of amino acids to protein such as arginylation. (See Proteins - Structure and Molecular Properties 2nd Ed., T.E. Creighton, W.H. Freeman and Company, New York (1993); Posttranslational Covalent Modification of Proteins, B.C. Johnson, Ed., Academic Press, New York, pp. 1-12 (1983)).

As used herein, the term "amino acid sequence" refers to a list of abbreviations, letters, characters or words representing amino acid residues.

As used herein, the term "isolated" means that a material has been removed from its original environment. For example, a naturally-occurring polynucleotide or polypeptide present in a living animal is not isolated, but the same polynucleotide or polypeptide, separated from some or all of the coexisting materials in the natural system, is isolated. Such polynucleotides can be part of a vector and/or such polynucleotides or polypeptides could be part of a composition, and would be isolated in that such a vector or composition is not part of its original environment.

As used herein with respect to nucleic acids, the term "recombinant" means that the nucleic acid is covalently joined and adjacent to a nucleic acid to which it is not adjacent in its natural environment. Additionally, as used herein with respect to a particular nucleic acid in a population of nucleic acids, the term "enriched" means that the nucleic acid represents 5% or more of the number of nucleic acids in the population of molecules. Typically, the enriched nucleic acids represent 15% or more of the number of nucleic acids in the population of molecules. More typically, the enriched nucleic acids represent 50%, 90% or more of the number of nucleic acids in the population molecules.

"Recombinant" polypeptides or proteins refer to polypeptides or proteins produced by recombinant DNA techniques, *i.e*., produced from cells transformed by an exogenous recombinant DNA construct encoding the desired polypeptide or protein. "Synthetic" polypeptides or proteins are those prepared by chemical synthesis (*e.g*., solid-phase peptide synthesis). Chemical peptide synthesis is well known in the art (see, *e.g*., Merrifield (1963), Am. Chem. Soc. 85:2149-2154; Geysen et al. (1984), Proc. Natl. Acad. Sci., USA 81:3998) and synthesis kits and automated peptide synthesizer are commercially available (*e.g.*, Cambridge Research Biochemicals, Cleveland, United Kingdom; Model 431A synthesizer from Applied Biosystems, Inc., Foster City, CA). Such equipment provides ready access to the peptides of the invention, either by direct synthesis or by synthesis of a series of fragments that can be coupled using other known techniques.

As used herein with respect to pairs of nucleic acid or amino acid sequences, "identity" refers to the extent to which the two sequences are invariant at positions within the sequence which can be aligned. The percent identity between two given sequences can be calculated using an algorithm such as BLAST (Altschul et al. (1990), J. Mol. Biol. 215:403-410). See www.ncbi.nlm.nih.gov/Education/BLASTinfo. When using the BLAST algorithm for sequences no longer than 250 nucleotides or about 80 amino acids ("short queries"), the search parameters can be as follows: the filter is off, the scoring matrix is PAM30, the word size is 3 or 2, the E value is 1000 or more, and the gap costs are 11, 1. For sequences longer than 250 nucleotides or 80 amino acid residues, the default search parameters can be used. The BLAST website provides advice for special circumstances which is to be followed in such circumstances.

As used herein, "homology" has the same meaning as "identity" in the context of nucleotide sequences. However, with respect to amino acid sequences, "homology" includes the percentage of identical and conservative amino acid substitutions. Percentages of homology can be calculated according to the algorithms of Smith and Waterman (1981), Adv. Appl. Math. 2:482.

As used herein in the context of two or more nucleic acid sequences, two sequences are "substantially identical" when they have at least 99.5% nucleotide identity, when compared and aligned for maximum correspondence, as measured using the known sequence comparison algorithms described above. In addition, for purposes of determining whether sequences are substantially identical, synonymous codons in a coding region may be treated as identical to account for the degeneracy of the genetic code. Typically, the region for determination of substantial identity must span at least about 20 residues, and most commonly the sequences are substantially identical over at least about 25-200 residues.

As used herein in the context of two or more amino acid sequences, two sequences are "substantially identical" when they have at least 99.5% identity, when compared and aligned for maximum correspondence, as measured using the known sequence comparison algorithms described above. In addition, for purposes of determining whether sequences are substantially identical, conservative amino acid substitutions may be treated as identical if the polypeptide substantially retains its biological function.

"Hybridization" refers to the process by which a nucleic acid strand joins with a complementary strand through hydrogen bonding at complementary bases. Hybridization assays can be sensitive and selective so that a particular sequence of interest can be identified even in samples in which it is present at low concentrations. Stringent conditions are defined by concentrations of salt or formamide in the prehybridization and hybridization solutions, or by the hybridization temperature, and are well known in the art. Stringency can be increased by reducing the concentration of salt, increasing the concentration of formamide, or raising the hybridization temperature. In particular, as used herein, "stringent hybridization conditions" include 42°C in 50% formamide, 5X SSPE, 0.3% SDS, and 200 ng/ml sheared and denatured salmon sperm DNA, and equivalents thereof. Variations on the above ranges and conditions are well known in the art.

The term "variant" refers to polynucleotides or polypeptides of the invention modified at one or more nucleotides or amino acid residues (respectively) and wherein the encoded polypeptide or polypeptide retains nitrilase activity. Variants can be produced by any number of means including, for example, error-prone PCR, shuffling, oligonucleotide-directed mutagenesis, assembly PCR, sexual PCR mutagenesis, *in vivo* mutagenesis, cassette mutagenesis, recursive ensemble mutagenesis, exponential ensemble mutagenesis, site-specific mutagenesis, gene reassembly, gene site-saturated mutagenesis or any combination thereof.

Methods of making peptidomimetics based upon a known sequence is described, for example, in U.S. Patent Nos. 5,631,280; 5,612,895; and 5,579,250. Use of peptidomimetics can involve the incorporation of a non-amino acid residue with non-amide linkages at a given position. A peptidomimetic may have a bond, a peptide backbone or an amino acid component replaced with a suitable mimic. Examples of unnatural amino acids which may be suitable amino acid mimics include β-alanine, L-α-amino butyric acid, L-γ-amino butyric acid, L-α-amino isobutyric acid, L-ε-amino caproic acid, 7-amino heptanoic acid, L-aspartic acid, L-glutamic acid, N-ε-Boc-N-α-CBZ-L-lysine, N-ε-Boc-N-α-Fmoc-L-lysine, L-methionine sulfone, L-norleucine, L-norvaline, N-α-Boc-N-δCBZ-L-ornithine, N-δ-Boc-N-α-CBZ-L-ornithine, Boc-p-nitro-L-phenylalanine, Boc-hydroxyproline, Boc-L-thioproline.

As used herein, "small molecule" encompasses a molecule having a molecular weight from about 20 Daltons to about 1.5 kiloDaltons.

The molecular biological techniques, such as subcloning, were performed using routine methods which would be well known to one of skill in the art. (Sambrook, J. Fritsch, EF, Maniatis, T. (1989) Molecular Cloning: A Laboratory Manual (2nd ed.), Cold Spring Harbor Laboratory Press, Plainview NY.).

**Table 2 includes the SEQ ID NOS, the Closest Hit (BLAST) Organism, the Closest Hit (BLAST) percentage amino acid identity and the percent nucleotide identity for the nitrilases of the disclosure.**

| **SEQ ID NO:** | **Top Public** | **Public** | **Public** | **Public** | **Public** | **Public Protein** | **Public Nucleotide** | **Length of SEQ ID NO. (# of Amino Acids)** | **Public Sequence** |
|---|---|---|---|---|---|---|---|---|---|
| | **Hit** | **E-Value** | **Definition** | **Organism** | **EC Number** | **% Identity** | **% Identity** | | **Length** |
| 1,2 | gi\|15229 934\|ref\| NP_190 017.11 | 0.00000 006 | nitrilase 1 [Arabidopsis thaliana]. | Arabidopsis thaliana Eukaryota | N/A | 20 | N/A | 312 | 346 |
| 3, 4 | gi\|14211 396\|gb\| AAK57 436.11 | 8E-56 | nitrilase-like protein [Brassica napus]. | Brassica napus Eukaryota | N/A | 41 | 51 | 326 | 350 |
| 5, 6 | gi\|15143 035\|emb ICAC50 776.1\| | e-113 | unnamed protein product [unidentified]. | unidentified unclassified. | N/A | 62 | 67 | 334 | 346 |
| 7, 8 | gi\|74359 80\|pir\|\|T 03739 | 9E-63 | nitrilase (EC 3.5.5.1) 4B - common tobacco. | Nicotiana tabacum Eukaryota | 3.5.5.1 | 41 | 50 | 332 | 348 |
| 9, 10 | gi\|74359 80\|pir\|\|T 03739 | 1E-55 | nitrilase (EC 3.5.5.1) 4B - common tobacco. | Nicotiana tabacum Eukaryota | 3.5.5.1 | 39 | 48 | 314 | 348 |
| 11, 12 | gi\|15795 659\|emb ICAC88 237.11 | 5E-42 | unnamed protein product [Rhodococcus rhodochrous]. | Rhodococcusrhodochrous Bacteria | N/A | 32 | 46 | 321 | 366 |
| 13, 14 | gi\|15143 035\|emb ICAC50 776.1\| | e-109 | unnamed protein product [unidentified]. | unidentified unclassified. | N/A | 62 | 66 | 337 | 346 |
| 15, 16 | gi\|15143 035\|emb ICAC50 776.11 | e-131 | unnamed protein product [unidentified]. | unidentified unclassified. | N/A | 65 | 64 | 348 | 346 |
| 17, 18 | gi\|74359 78\|pir\|\|S 77025 | e-100 | nitrilase (EC 3.5.5.1)-Synechocystis sp. (strain PCC 6803). | Synechocystis sp. Bacteria | 3.5.5.1 | 52 | 58 | 330 | 346 |
| 19, 20 | gi\|15143 035\|emb ICAC50 776.1\| | e-107 | unnamed protein product [unidentified]. | unidentified unclassified. | N/A | 55 | 60 | 349 | 346 |
| 21, 22 | gi\|15143 035\|emb ICAC50 776.1\| | e-111 | unnamed protein product [unidentified]. | unidentified unclassified. | N/A | 59 | 63 | 354 | 346 |
| 23, 24 | gi\|74359 78\|pir\|\|S 77025 | e-101 | nitrilase (EC 3.5.5.1)-Synechocystis sp. (strain PCC 6803). | Synechocystis sp. Bacteria | 3.5.5.1 | 55 | 57 | 334 | 346 |
| 25, 26 | gi\|48355 88\|dbj\|B AA7767 9.1\| | 2E-50 | nitrilase-like protein [Oryza sativa]. | Oryza sativa Eukaryota | N/A | 38 | 48 | 312 | 362 |
| 27, 28 | gir\|15242 205\|ref\| NP_197 622.1\| | 4E-62 | Nitrilase 4 (sp P46011) [Arabidopsis thaliana]. | Arabidopsis thaliana Eukaryota | N/A | 38 | N/A | 351 | 355 |
| 29, 30 | gi\|15143 035\|emb ICAC50 776.11 | e-106 | unnamed protein product [unidentified]. | unidentified unclassified. | N/A | 61 | 66 | 338 | 346 |
| 31, 32 | gi\|14211 396\|gb\| AAK57 436.11 | 3E-51 | nitrilase-like protein [Brassica napus]. | Brassicanapus Eukaryota | N/A | 38 | 49 | 310 | 350 |
| 33, 34 | gi\|15143 035\|emb ICAC50 776.1\| | e-150 | unnamed protein product [unidentified]. | unidentified unclassified. | N/A | 80 | 78 | 341 | 346 |
| 35, 36 | gi\|15902 867\|ref\| NP_358 417.11 | 0.00000 002 | Beta-alanine synthase or beta-ureidopro pionase [Streptococcus pneumoniae R6]. | Streptococcus pneumoniae R6 Bacteria | N/A | 21 | 41 | 313 | 291 |
| 37, 38 | gi\|15143 037\|emb ICAC50 777.1\| | 2E-56 | unnamed protein product [unidentified]. | unidentified unclassified. | N/A | 39 | 47 | 330 | 337 |
| 39, 40 | gi\|74359 78\|pir\|\|S 77025 | 1E-97 | nitrilase (EC 3.5.5.1)-Synechocystis sp. (strain PCC 6803). | Synechocystis sp. Bacteria | 3.5.5.1 | 50 | 58 | 335 | 346 |
| 41, 42 | gi\|66248 86\|emb\| CAA68 934.3\| | 2E-60 | nitrilase 2 [Arabidopsis thaliana]. | Arabidopsis thaliana Eukaryota | N/A | 41 | 52 | 321 | 339 |
| 43, 44 | gi\|74359 78\|pir\|\|S 77025 | 8E-96 | nitrilase (EC 3.5.5.1)-Synechocystis sp. (strain PCC 6803). | Synechocystis sp. Bacteria | 3.5.5.1 | 51 | 58 | 330 | 346 |
| 45, 46 | gi\|48355 88\|dbj\|B AA7767 9.1\| | 6E-54 | nitrilase-like protein [Oryza sativa]. | Oryzasativa Eukaryota | N/A | 39 | 51 | 331 | 362 |
| 47, 48 | gi\|15143 037\|emb ICAC50 777.1\| | e-146 | unnamed protein product [unidentified]. | unidentified unclassified. | N/A | 74 | 68 | 337 | 337 |
| 49, 50 | gi\|74359 78\|pir\|\|S 77025 | e-106 | nitrilase (EC 3.5.5.1)-Synechocystis sp. (strain PCC 6803). | Synechocystis sp. Bacteria | 3.5.5.1 | 59 | 58 | 345 | 346 |
| 51, 52 | gi\|75109 01\|pir\|\|T 27679 | 5E-47 | probable nitrilase (EC 3.5.5.1) ZK1058.6 Caenorha bditis elegans. | Caenorhabditis elegans Eukaryota | 3.5.5.1 | 36 | 48 | 298 | 305 |
| 53, 54 | gi\|74359 80\|pir\|\|T 03739 | 5E-61 | nitrilase (EC 3.5.5.1) 4B - common tobacco. | Nicotiana tabacum Eukaryota | 3.5.5.1 | 41 | 51 | 317 | 348 |
| 55, 56 | gi\|15143 035\|emb ICAC50 776.1\| | e-111 | unnamed protein product [unidentified]. | unidentified unclassified. | N/A | 60 | 67 | 338 | 346 |
| 57, 58 | gi\|15143 037\|emb ICAC50 777.1\| | e-146 | unnamed protein product [unidentified]. | unidentified unclassified. | N/A | 74 | 68 | 337 | 337 |
| 59, 60 | gi\|22940 01\|emb\| CAA02 248.1\| | 9E-50 | unnamed protein product [unidentified]. | unidentified unclassified. | N/A | 35 | 49 | 328 | 354 |
| 61, 62 | gi\|15795 659\|emb ICAC88 237.11 | 2E-43 | unnamed protein product [Rhodococcus rhodochrous]. | Rhodococus rhodochrous Bacteria | N/A | 33 | 46 | 321 | 366 |
| 63, 64 | gi\|48355 88\|dbj\|B AA7767 9.11 | 6E-67 | nitrilase-like protein [Oryza sativa]. | Oryza sativa Eukaryota | N/A | 44 | 51 | 325 | 362 |
| 65, 66 | gi\|39141 63\|sp\|Q4 2965\|N RL4_T OBAC | 5E-63 | NITRILA SE 4. | Nicotiana tabacum Eukaryota | N/A | 43 | 49 | 333 | 349 |
| 67, 68 | gi\|15242 205\|ref\| NP_197 622.1\| | 1E-50 | Nitrilase 4 (sp P46011) [Arabidopsis thaliana]. | Arabidopsis thaliana Eukaryota | N/A | 39 | N/A | 311 | 355 |
| 69, 70 | gi\|74359 80\|pir\|\|T 03739 | 2E-55 | nitrilase (EC 3.5.5.1) 4B - common tobacco. | Nicotiana tabacum Eukaryota | 3.5.5.1 | 43 | 48 | 312 | 348 |
| 71,72 | gi\|74359 80\|pir\|\|T 03739 | 2E-58 | nitrilase (EC 3.5.5.1) 4B - common tobacco. | Nicotiana tabacum Eukaryota | 3.5.5.1 | 39 | 52 | 321 | 348 |
| 73, 74 | gi\|15229 934\|ref\| NP_190 017.1\| | 2E-53 | nitrilase 1 [Arabidopsis thaliana]. | 1 Arabidop sis thaliana Eukaryota | N/A | 34 | N/A | 344 | 346 |
| 75, 76 | gi\|74359 78\|pir\|\|S 77025 | 4E-98 | nitrilase (EC 3.5.5.1)-Synechocystis sp. (strain PCC 6803). | Synechocystis sp. Bacteria | 3.5.5.1 | 48 | 50 | 374 | 346 |
| 77, 78 | gi\|15242 205\|ref\| NP_197 622.11 | 8E-62 | Nitrilase 4 (sp P46011) [Arabidopsis thaliana]. | Arabidopsis thaliana Eukaryota | N/A | 38 | N/A | 351 | 355 |
| 79, 80 | gi\|74359 80\|pir\|\|T 03739 | 5E-54 | nitrilase (EC 3.5.5.1) 4B - common tobacco. | Nicotiana tabacum Eukaryota | 3.5.5.1 | 38 | 46 | 329 | 348 |
| 81, 82 | gi\|74359 78\|pir\|\|S 77025 | 3E-94 | nitrilase (EC 3.5.5.1)-Synechocystis sp. (strain PCC 6803). | Synechocystis sp. Bacteria | 3.5.5.1 | 51 | 56 | 330 | 346 |
| 83, 84 | gi\|74359 78\|pir\|\|S 77025 | e-114 | nitrilase (EC 3.5.5.1)-Synechocystis sp. (strain PCC 6803). | Synechocystis sp. Bacteria | 3.5.5.1 | 61 | 59 | 356 | 346 |
| 85,86 | gi\|15143 035\|emb ICAC50 776.11 | e-115 | unnamed protein product [unidentified]. | unidentified unclassified. | N/A | 63 | 66 | 337 | 346 |
| 87,88 | gi\|14699 12\|gb\|A AB0522 0.1\| | 3E-54 | nitrilase 2. | Arabidopsis thaliana Eukaryota | N/A | 36 | 46 | 353 | 339 |
| 89,90 | gi\|15242 205\|ref\| NP_197 622.1\| | 5E-62 | Nitrilase 4 (sp P46011) [Arabidopsis thaliana]. | Arabidopsis thaliana Eukaryota | N/A | 41 | N/A | 305 | 355 |
| 91,92 | gi\|14211 396\|gb\| AAK57 436.11 | 6E-58 | nitrilase-like protein [Brassica napus]. | Brassica napus Eukaryota | N/A | 35 | 50 | 312 | 350 |
| 93,94 | gi\|74359 78\|pir\|\|S 77025 | e-112 | nitrilase (EC 3.5.5.1)-Synechocystis sp. (strain PCC 6803). | Synechocystis sp. Bacteria | 3.5.5.1 | 60 | 61 | 325 | 346 |
| 95,96 | gi\|66248 86\|emb\| CAA68 934.3\| | 1E-60 | nitrilase 2 [Arabidopsis thaliana]. | Arabidopsis thaliana Eukaryota | N/A | 42 | 53 | 321 | 339 |
| 97,98 | gi\|15143 035\|emb ICAC50 776.1\| | e-109 | unnamed protein product [unidentified]. | unidentified unclassified. | N/A | 62 | 67 | 338 | 346 |
| 99, 100 | gi\|74359 78\|pir\|\|S 77025 | e-123 | nitrilase (EC 3.5.5.1)-Synechocystis sp. (strain PCC 6803). | Synechocystis sp. Bacteria | 3.5.5.1 | 62 | 61 | 337 | 346 |
| 101, 102 | gi\|15143 035\|emb ICAC50 776.1\| | e-121 | unnamed protein product [unidentified]. | unidentified unclassified. | N/A | 62 | 66 | 354 | 346 |
| 103, 104 | gi\|15143 037\|emb ICAC50 777.11 | 2E-41 | unnamed protein product [unidentified]. | unidentified unclassified. | N/A | 34 | 49 | 314 | 337 |
| 105, 106 | gi\|74359 78\|pir\|\|S 77025 | 1E-97 | nitrilase (EC 3.5.5.1)-Synechoc ystis sp. (strain PCC 6803). | Synechocystis sp. Bacteria | 3.5.5.1 | 54 | 58 | 324 | 346 |
| 107, 108 | gi\|74359 78\|pir\|\|S 77025 | e-113 | nitrilase (EC 3.5.5.1)-Synechocystis sp. (strain PCC 6803). | Synechocystis sp. Bacteria | 3.5.5.1 | 61 | 61 | 326 | 346 |
| 109, 110 | gi\|74359 78\|pir\|\|S 77025 | e-113 | nitrilase (EC 3.5.5.1)-Synechocystis sp. (strain PCC 6803). | Synechocystis sp. Bacteria | 3.5.5.1 | 55 | 57 | 363 | 346 |
| 111, 112 | gi\|74359 78\|pir\|\|S 77025 | e-121 | nitrilase (EC 3.5.5.1)-Synechocystis sp. (strain PCC 6803). | Synechocystis sp. Bacteria | 3.5.5.1 | 64 | 61 | 329 | 346 |
| 113, 114 | gi\|15162 360\|gb\| AAK90 913.11 | 4E-09 | AGR_pA T_799p [Agrobacterium tumefaciens]. | Plasmid Agrobacterium tumefaciens Bacteria | N/A | 26 | 47 | 330 | 600 |
| 115, 116 | gi\|74359 78\|pir\|\|S 77025 | e-115 | nitrilase (EC 3.5.5.1)-Synechocystis sp. (strain PCC 6803). | Synechocystis sp. Bacteria | 3.5.5.1 | 59 | 61 | 330 | 346 |
| 117, 118 | gi\|74359 78\|pir\|\|S 77025 | e-106 | nitrilase (EC 3.5.5.1)-Synechocystis sp. (strain PCC 6803). | Synechocystis sp. Bacteria | 3.5.5.1 | 64 | 64 | 318 | 346 |
| 119, 120 | gi\|15795 659\|emb ICAC88 237.11 | 1E-40 | unnamed protein product [Rhodococcus rhodochrous]. | Rhodococcus rhodochrous Bacteria | N/A | 30 | 49 | 327 | 366 |
| 121, 122 | gi\|15229 932\|ref\| NP_190 016.1\| | 1E-45 | nitrilase 2 [Arabidopsis thaliana]. | Arabidopsis thaliana Eukaryota | N/A | 30 | N/A | 385 | 339 |
| 123, 124 | gir\|14211 396\|gb\| AAK57 436.1\| | 9E-52 | nitrilase-like protein [Brassica napus]. | Brassica napus Eukaryota | N/A | 35 | 51 | 329 | 350 |
| 125, 126 | gi\|74359 78\|pir\|\|S 77025 | e-125 | nitrilase (EC 3.5.5.1)-Synechocystis sp. (strain PCC 6803). | Synechocystis sp. Bacteria | 3.5.5.1 | 63 | 61 | 349 | 346 |
| 127, 128 | gi\|74359 78\|pir\|\|S 77025 | e-117 | nitrilase (EC 3.5.5.1)-Synechoc ystis sp. (strain PCC 6803). | Synechocystis sp. Bacteria | 3.5.5.1 | 61 | 60 | 334 | 346 |
| 129, 130 | gi\|74359 78\|pir\|\|S 77025 | e-125 | nitrilase (EC 3.5.5.1)-Synechocystis sp. (strain PCC 6803). | Synechocystis sp. Bacteria | 3.5.5.1 | 64 | 63 | 336 | 346 |
| 131, 132 | gi\|74359 78\|pir\|\|S 77025 | e-125 | nitrilase (EC 3.5.5.1)-Synechocystis sp. (strain PCC 6803). | Synechocystis sp. Bacteria | 3.5.5.1 | 63 | 63 | 336 | 346 |
| 133, 134 | gi\|74359 78\|pir\|\|S 77025 | e-120 | nitrilase (EC 3.5.5.1)-Synechocystis sp. (strain PCC 6803). | Synechocystis sp. Bacteria | 3.5.5.1 | 63 | 60 | 341 | 346 |
| 135, 136 | gi\|74359 78\|pir\|\|S 77025 | e-124 | nitrilase (EC 3.5.5.1)-Synechocystis sp. (strain PCC 6803). | Synechocystis sp. Bacteria | 3.5.5.1 | 64 | 63 | 336 | 346 |
| 137, 138 | gi\|74359 78\|pir\|\|S 77025 | e-102 | nitrilase (EC 3.5.5.1)-Synechocystis sp. (strain PCC 6803). | Synechocystis sp. Bacteria | 3.5.5.1 | 54 | 56 | 325 | 346 |
| 139, 140 | gi\|15143 037\|emb ICAC50 777.1\| | e-123 | unnamed protein product [unidentified]. | unidentified unclassified. | N/A | 65 | 63 | 332 | 337 |
| 141, 142 | gi\|15143 037\|emb ICAC50 777.1\| | 3E-49 | unnamed protein product [unidentified]. | unidentified unclassified. | N/A | 32 | 47 | 341 | 337 |
| 143, 144 | gi\|15143 037\|emb ICAC50 777.1\| | 8E-55 | unnamed protein product [unidentified]. | unidentified unclassified. | N/A | 30 | 44 | 373 | 337 |
| 145, 146 | gi\|15143 035\|emb ICAC50 776.1\| | e-110 | unnamed protein product [unidentified]. | unidentified unclassified. | N/A | 58 | 63 | 337 | 346 |
| 147, 148 | gi\|15143 037\|emb ICAC50 777.1\| | 2E-52 | unnamed protein product [unidentified]. | unidentified unclassified. | N/A | 33 | 42 | 365 | 337 |
| 149, 150 | gi\|15229 932\|ref\| NP_190 016.1\| | 5E-63 | nitrilase 2 [Arabidopsis thaliana]. | Arabidopsis thaliana Eukaryota | N/A | 43 | N/A | 313 | 339 |
| 151, 152 | gi\|74359 78\|pir\|\|S 77025 | 6E-96 | nitrilase (EC 3.5.5.1)-Synechocystis sp. (strain PCC 6803). | Synechocystis sp. Bacteria | 3.5.5.1 | 51 | 58 | 330 | 346 |
| 153, 154 | gi\|74359 78\|pir\|\|S 77025 | 7E-46 | nitrilase (EC 3.5.5.1)-Synechocystis sp. (strain PCC 6803). | Synechoc ystis sp. Bacteria | 3.5.5.1 | 31 | 44 | 357 | 346 |
| 155, 156 | gi\|15143 035\|emb ICAC50 776.1\| | e-101 | unnamed protein product [unidentified]. | unidentified unclassified. | N/A | 60 | 66 | 346 | 346 |
| 157, 158 | gi\|74359 78\|pir\|\|S 77025 | 3E-99 | nitrilase (EC 3.5.5.1)-Synechocystis sp. (strain PCC 6803). | Synechocystis sp. Bacteria | 3.5.5.1 | 52 | 55 | 336 | 346 |
| 159, 160 | gi\|74359 80\|pir\|\|T 03739 | 7E-52 | nitrilase (EC 3.5.5.1) 4B - common tobacco. | Nicotiana tabacum Eukaryota | 3.5.5.1 | 41 | 52 | 309 | 348 |
| 161, 162 | gi\|74359 78\|pir\|\|S 77025 | e-111 | nitrilase (EC 3.5.5.1)-Synechocystis sp. (strain PCC 6803). | Synechocystis sp. Bacteria | 3.5.5.1 | 58 | 60 | 335 | 346 |
| 163, 164 | gi\|74359 78\|pir\|\|S 77025 | e-101 | nitrilase (EC 3.5.5.1)-Synechocystis sp. (strain PCC 6803). | Synechocystis sp. Bacteria | 3.5.5.1 | 57 | 58 | 325 | 346 |
| 165, 166 | gi\|15143 035\|emb ICAC50 776.1\| | e-108 | unnamed protein product [unidentified]. | unidentified unclassified. | N/A | 63 | 65 | 335 | 346 |
| 167, 168 | gi\|15143 035\|emb ICAC50 776.1\| | e-111 | unnamed protein product [unidentified]. | unidentified unclassified. | N/A | 61 | 66 | 338 | 346 |
| 169, 170 | gi\|15143 035\|emb ICAC50 776.1\| | e-122 | unnamed protein product [unidentified]. | unidentified unclassified. | N/A | 60 | 65 | 358 | 346 |
| 171, 172 | gi\|66248 86\|emb\| CAA68 934.3\| | 9E-61 | nitrilase [Arabidopsis thaliana]. | 2 Arabidopsis thaliana Eukaryota | N/A | 38 | 48 | 336 | 339 |
| 173, 174 | gi\|74359 78\|pir\|\|S 77025 | e-101 | nitrilase (EC 3.5.5.1)-Synechocystis sp. (strain PCC 6803). | Synechocystis sp. Bacteria | 3.5.5.1 | 54 | 56 | 330 | 346 |
| 175, 176 | gi\|15143 037\|emb ICAC50 777.1\| | 2E-52 | unnamed protein product [unidentified]. | unidentified unclassified. | N/A | 39 | 49 | 314 | 337 |
| 177, 178 | gi\|15795 659\|emb ICAC88 237.1\| | 9E-41 | unnamed protein product [Rhodococcus rhodochrous]. | Rhodococcus rhodochrous Bacteria | N/A | 32 | 50 | 315 | 366 |
| 179, 180 | gi\|74359 80\|pir\|\|T 03739 | 3E-53 | nitrilase (EC 3.5.5.1) 4B - common tobacco. | Nicotiana tabacum Eukaryota | 3.5.5.1 | 38 | 46 | 304 | 348 |
| 181, 182 | gi\|74359 78\|pir\|\|S 77025 | e-121 | nitrilase (EC 3.5.5.1)-Synechocystis sp. (strain PCC 6803). | Synechocystis sp. Bacteria | 3.5.5.1 | 64 | 61 | 329 | 346 |
| 183, 184 | gi\|74359 78\|pir\|\|S 77025 | e-118 | nitrilase (EC 3.5.5.1)-Synechocystis sp. (strain PCC 6803). | Synechocystis sp. Bacteria | 3.5.5.1 | 63 | 63 | 333 | 346 |
| 185, 186 | gi\|15143 035\|emb ICAC50 776.1\| | e-110 | unnamed protein product [unidentified]. | unidentified unclassified. | N/A | 60 | 67 | 338 | 346 |
| 187, 188 | gi\|15143 035\|emb ICAC50 776.1\| | e-113 | unnamed protein product [unidentified]. | unidentified unclassified. | N/A | 59 | 62 | 352 | 346 |
| 189, 190 | gi\|15143 035\|emb ICAC50 776.1\| | e-101 | unnamed protein product [unidentified]. | unidentified unclassified. | N/A | 56 | 61 | 334 | 346 |
| 191, 192 | gi\|15242 205\|ref\| NP_197 622.1\| | 6E-56 | Nitrilase 4 (sp P46011) [Arabidopsis thaliana]. | Arabidopsis thaliana Eukaryota | N/A | 37 | N/A | 314 | 355 |
| 193, 194 | gi\|74359 80\|pir\|\|T 03739 | 3E-58 | nitrilase (EC 3.5.5.1) 4B - common tobacco. | Nicotiana tabacum Eukaryota | 3.5.5.1 | 39 | 51 | 321 | 348 |
| 195, 196 | gi\|74359 78\|pir\|\|S 77025 | 5E-89 | nitrilase (EC 3.5.5.1)-Synechocystis sp. (strain PCC 6803). | Synechocystis sp. Bacteria | 3.5.5.1 | 50 | 57 | 330 | 346 |
| 197, 198 | gi\|15143 035\|emb ICAC50 776.1\| | e-110 | unnamed protein product [unidentified]. | unidentified unclassified. | N/A | 65 | 68 | 338 | 346 |
| 199, 200 | gi\|11266 289\|pir\|\| T49147 | 1E-58 | nitrilase 1 - Arabidops is thaliana. | Arabidopsis thaliana Eukaryota | N/A | 38 | N/A | 330 | 346 |
| 201, 202 | gi\|48355 88\|dbj\|B AA7767 9.1\| | 5E-50 | nitrilase-like protein [Oryza sativa]. | Oryza sativa Eukaryota | N/A | 39 | 49 | 309 | 362 |
| 203, 204 | gi\|74359 80\|pir\|\|T 03739 | 4E-57 | nitrilase (EC 3.5.5.1) 4B - common tobacco. | Nicotiana tabacum Eukaryota | 3.5.5.1 | 39 | 50 | 321 | 348 |
| 205, 206 | gi\|74359 78\|pir\|\|S 77025 | 1E-99 | nitrilase (EC 3.5.5.1)-Synechocystis sp. (strain PCC 6803). | Synechocystis sp. Bacteria | 3.5.5.1 | 55 | 56 | 322 | 346 |
| 207, 208 | gi\|74359 80\|pir\|\|T 03739 | 5E-59 | nitrilase (EC 3.5.5.1) 4B - common tobacco. | Nicotiana tabacum Eukaryota | 3.5.5.1 | 40 | 51 | 321 | 348 |
| 209, 210 | gi\|74359 78\|pir\|\|S 77025 | 2E-95 | nitrilase (EC 3.5.5.1)-Synechoc ystis sp. (strain PCC 6803). | Synechoc ystis sp. Bacteria | 3.5.5.1 | 53 | 55 | 330 | 346 |
| 211,212 | gi\|15143 035\|embICAC50 776.1\| | e-117 | unnamed protein product [unidentified]. | unidentified unclassified. | N/A | 64 | 67 | 353 | 346 |
| 213, 214 | gi\|74359 78\|pir\|\|S 77025 | e-101 | nitrilase (EC 3.5.5.1) -Synechoc ystis sp. (strain PCC 6803). | Synechocystis sp. Bacteria | 3.5.5.1 | 53 | 57 | 330 | 346 |
| 215, 216 | gi\|15143 035\|emb ICAC50 776.1\| | e-114 | unnamed protein product [unidentified]. | unidentified unclassified. | N/A | 62 | 61 | 335 | 346 |
| 217, 218 | gi\|15143 035\|emb ICAC50 776.1\| | e-115 | unnamed protein product [unidentified]. | unidentified unclassified. | N/A | 58 | 62 | 336 | 346 |
| 219, 220 | gi\|74359 78\|pir\|\|S 77025 | e-101 | nitrilase (EC 3.5.5.1) -Synechocystis sp. (strain PCC 6803). | Synechocystis sp. Bacteria | 3.5.5.1 | 53 | 59 | 331 | 346 |
| 221, 222 | gi\|41738 2\|sp\|Q02 068\|NR L1_RH ORH | e-122 | ALIPHATIC NITRILASE. | Rhodococcus rhodochrous Bacteria | N/A | 58 | 62 | 381 | 383 |
| 223, 224 | gi\|74359 78\|pir\|\|S 77025 | 3E-94 | nitrilase (EC 3.5.5.1)-Synechocystis sp. (strain PCC 6803). | Synechocystis sp. Bacteria | 3.5.5.1 | 51 | 56 | 331 | 346 |
| 225, 226 | gi\|74359 78\|pir\|\|S 77025 | 6E-99 | nitrilase (EC 3.5.5.1)-Synechocystis sp. (strain PCC 6803). | Synechocystis sp. Bacteria | 3.5.5.1 | 53 | 57 | 316 | 346 |
| 227, 228 | gi\|74359 78\|pir\|\|S 77025 | e-121 | nitrilase (EC 3.5.5.1)-Synechocystis sp. (strain PCC 6803). | Synechocystis sp. Bacteria | 3.5.5.1 | 62 | 60 | 344 | 346 |
| 229, 230 | gi\|75109 01\|pir\|\|T 27679 | 3E-48 | probable nitrilase (EC 3.5.5.1) ZK1058.6 Caenorha bditis elegans. | Caenorha bditis elegans Eukaryota | 3.5.5.1 | 36 | 48 | 324 | 305 |
| 231, 232 | gi\|15143 035\|emb ICAC50 776.1\| | e-125 | unnamed protein product [unidentified]. | unidentified unclassified. | N/A | 63 | 65 | 353 | 346 |
| 233, 234 | gi\|74359 78\|pir\|\|S 77025 | e-101 | nitrilase (EC 3.5.5.1)-Synechocystis sp. (strain PCC 6803). | Synechocystis sp. Bacteria | 3.5.5.1 | 54 | 55 | 333 | 346 |
| 235, 236 | gi\|74359 78\|pir\|\|S 77025 | 1E-90 | (EC 3.5.5.1)-Synechocystis sp. (strain PCC 6803). | Synechocystis sp. Bacteria | 3.5.5.1 | 51 | 57 | 330 | 346 |
| 237, 238 | gi\|74359 78\|pir\|\|S 77025 | 7E-97 | (EC 3.5.5.1)-Synechocystis sp. (strain PCC 6803). | Synechocystis sp. Bacteria | 3.5.5.1 | 53 | 56 | 330 | 346 |
| 239, 240 | gi\|74359 80\|pir\|\|T 03739 | 8E-59 | nitrilase (EC 3.5.5.1) 4B - common tobacco. | Nicotiana tabacum Eukaryota | 3.5.5.1 | 40 | 50 | 322 | 348 |
| 241, 242 | gi\|66248 86\|emb\| CAA68 934.3\| | 8E-59 | nitrilase 2 [Arabidopsis thaliana]. | Arabidopsis thaliana Eukaryota | N/A | 40 | 50 | 323 | 339 |
| 243, 244 | gi\|15143 037\|emb ICAC50 777.1\| | 2E-51 | unnamed protein product [unidentified]. | unidentified unclassified. | N/A | 38 | 47 | 332 | 337 |
| 245, 246 | gi\|66248 86\|emb\| CAA68 934.31 | 5E-56 | nitrilase 2 [Arabidopsis thaliana]. | Arabidopsis thaliana Eukaryota | N/A | 38 | 50 | 332 | 339 |
| 247, 248 | gi\|74359 80\|pir\|\|T 03739 | 9E-61 | nitrilase (EC 3.5.5.1) 4B-common tobacco. | Nicotiana tabacum Eukaryota | 3.5.5.1 | 42 | 49 | 329 | 348 |
| 249, 250 | gi\|15143 035\|emb ICAC50 776.1\| | e-113 | unnamed protein product [unidentified]. | unidentified unclassified. | N/A | 62 | 67 | 338 | 346 |
| 251, 252 | gi\|74359 78\|pir\|\|S 77025 | e-100 | nitrilase (EC 3.5.5.1)-Synechocystis sp. (strain PCC 6803). | Synechocystis sp. Bacteria | 3.5.5.1 | 55 | 57 | 325 | 346 |
| 253, 254 | gi\|74359 80\|pir\|\|T 03739 | 1E-56 | nitrilase (EC 3.5.5.1) 4B - common tobacco. | Nicotiana tabacum Eukaryota | 3.5.5.1 | 40 | 51 | 307 | 348 |
| 255, 256 | gi\|15229 936\|ref\| NP_190 018.1\| | 4E-63 | nitrilase 3 [Arabidopsis thaliana]. | Arabidopsis thaliana Eukaryota | N/A | 40 | N/A | 334 | 346 |
| 257, 258 | gi\|48355 88\|dbj\|B AA7767 9.1\| | 6E-51 | nitrilase-like protein [Oryza sativa]. | Oryza sativa Eukaryota | N/A | 38 | 48 | 313 | 362 |
| 259, 260 | gi\|74359 78\|pir\|\|S 77025 | e-113 | nitrilase (EC 3.5.5.1)-Synechocystis sp. (strain PCC 6803). | Synechocystis sp. Bacteria | 3.5.5.1 | 60 | 61 | 326 | 346 |
| 261, 262 | gi\|15143 035\|emb ICAC50 776.1\| | e-114 | unnamed protein product [unidentified]. | unidentified unclassified. | N/A | 62 | 67 | 337 | 346 |
| 263, 264 | gi\|74359 78\|pir\|\|S 77025 | 9E-98 | nitrilase (EC 3.5.5.1)-Synechocystis sp. (strain PCC 6803). | Synechocystis sp. Bacteria | 3.5.5.1 | 53 | 54 | 337 | 346 |
| 265, 266 | gi\|14590 532\|ref\| NP_142 600.1\| | 8E-17 | hypothetical protein [Pyrococcus horikoshii]. | Pyrococc us horikoshii Archaea | N/A | 21 | 34 | 332 | 262 |
| 267, 268 | gi\|15143 035\|emb ICAC50 776.1\| | e-102 | unnamed protein product [unidentified]. | unidentified unclassified. | N/A | 53 | 64 | 345 | 346 |
| 269, 270 | gi\|15143 035\|emb ICAC50 776.1\| | 8E-97 | unnamed protein product [unidentified]. | unidentified unclassified. | N/A | 57 | 61 | 337 | 346 |
| 271, 272 | gi\|15229 934\|ref\| NP_190 017.1\| | 3E-59 | nitrilase 1 [Arabidopsis thaliana]. | Arabidopsis thaliana Eukaryota | N/A | 41 | N/A | 321 | 346 |
| 273, 274 | gi\|74359 78\|pir\|\|S 77025 | e-141 | nitrilase (EC 3.5.5.1)-Synechocystis sp. (strain PCC 6803). | Synechocystis sp. Bacteria | 3.5.5.1 | 69 | 67 | 340 | 346 |
| 275, 276 | gi\|74359 80\|pir\|\|T 03739 | 8E-42 | nitrilase (EC 3.5.5.1) 4B - common tobacco. | Nicotiana tabacum Eukaryota | 3.5.5.1 | 35 | 48 | 282 | 348 |
| 277, 278 | gi\|15242 205\|ref\| NP_197 622.1\| | 4E-62 | Nitrilase 4 (sp P46011) [Arabidopsis thaliana]. | Arabidopsis thaliana Eukaryota | N/A | 38 | N/A | 351 | 355 |
| 279, 280 | gi\|15143 035\|emb ICAC50 776.1\| | e-102 | unnamed protein product [unidentified]. | unidentified unclassified. | N/A | 57 | 63 | 337 | 346 |
| 281, 282 | gi\|75109 01\|pir\|\|T 27679 | 5E-55 | probable nitrilase (EC 3.5.5.1) ZK1058.6 Caenorhabditis elegans. | Caenorhabditis elegans Eukaryota | 3.5.5.1 | 39 | 46 | 311 | 305 |
| 283, 284 | gi\|15143 035\|emb ICAC50 776.1\| | e-111 | unnamed protein product [unidentified]. | unidentified unclassified. | N/A | 61 | 66 | 338 | 346 |
| 285, 286 | gi\|15242 205\|ref\| NP_197 622.1\| | 1E-64 | Nitrilase 4 (sp P46011) [Arabidopsis thaliana]. | Arabidopsis thaliana Eukaryota | N/A | 44 | N/A | 305 | 355 |
| 287, 288 | gi\|66248 86\|emb\| CAA68 934.3\| | 2E-55 | nitrilase 2 [Arabidopsis thaliana]. | Arabidopsis thaliana Eukaryota | N/A | 39 | 51 | 311 | 339 |
| 289, 290 | gi\|48355 88\|dbj\|B AA7767 9.11 | 3E-58 | nitrilase-like protein [Oryza sativa]. | Oryza sativa Eukaryota | N/A | 43 | 51 | 306 | 362 |
| 291, 292 | gi\|5143 037\|emb ICAC50 777.11 | e-123 | unnamed protein product [unidentified]. | unidentified unclassified. | N/A | 64 | 63 | 333 | 337 |
| 293, 294 | gil16331 918lref NP_442 646.11 | 1E-97 | nitrilase [Synechocystis sp. PCC 6803]. | Synechocystis sp. PCC 6803 Bacteria | N/A | 50 | N/A | 335 | 346 |
| 295, 296 | gi\|15229 932\|ref\| NP_190 016.11 | 6E-35 | nitrilase 2 [Arabidopsis thaliana]. | Arabidopsis thaliana Eukaryota | N/A | 27 | N/A | 377 | 339 |
| 297, 298 | gi\|15143 037\|emb \|CAC50 777.1\| | 3E-44 | unnamed protein product [unidentified]. | unidentified unclassified. | N/A | 34 | 47 | 352 | 337 |
| 299, 300 | gi\|16331 918\|ref\| NP_442 646.11 | e-103 | nitrilase [Synechocystis sp. PCC 6803]. | Synechocystis sp. PCC 6803 Bacteria | N/A | 56 | N/A | 328 | 346 |
| 301, 302 | gi\|74359 80\|pir\|\|T 03739 | 6E-57 | nitrilase (EC 3.5.5.1) 4B - common tobacco. | Nicotiana tabacum Eukaryota | 3.5.5.1 | 37 | 46 | 335 | 348 |
| 303, | gi\|15143 035\|emb | e-112 | unnamed protein | unidentified | N/A | 60 | 59 | 336 | 346 |
| 304 | ICAC50 776.11 | | product [unidentified]. | unclassified. | | | | | |
| 305, 306 | gi\|15143 035\|emb \|CAC50 776.1\| | e-121 | unnamed protein product [unidentified]. | unidentified unclassified. | N/A | 59 | N/A | 355 | 346 |
| 307, 308 | gi\|74359 80\|pir\|\|T 03739 | 5E-61 | nitrilase (EC 3.5.5.1) 4B - common tobacco. | Nicotiana tabacum Eukaryota | 3.5.5.1 | 43 | 50 | 313 | 348 |
| 309, 310 | gi\|41738 2\|sp\|Q02 068\|NR L1_RH ORH | 1E-43 | ALIPHATIC NITRILASE. | Rhodococcus rhodochrous Bacteria | N/A | 34 | 53 | 316 | 383 |
| 311, 312 | gi\|16331 918\|ref\| NP_442 646.1\| | e-126 | nitrilase [Synechocystis sp. PCC 6803]. | Synechocystis sp. PCC 6803 Bacteria | N/A | 64 | N/A | 336 | 346 |
| 313, 314 | gi\|15143 035\|emb ICAC50 776.11 | 1E-49 | unnamed protein product [unidentified]. | unidentified unclassified. | N/A | 36 | 50 | 328 | 346 |
| 315, 316 | gi\|15229 936\|ref\| NP_190 018.11 | 4E-64 | nitrilase 3 [Arabidopsis thaliana]. | Arabidopsis thaliana Eukaryota | N/A | 42 | N/A | 319 | 346 |
| 317, 318 | gi\|16331 918\|ref\| NP_442 646.1\| | e-102 | nitrilase [Synechocystis sp. PCC 6803]. | Synechocystis sp. PCC 6803 Bacteria | N/A | 54 | N/A | 330 | 346 |
| 319, 320 | gi\|16331 918\|ref\| NP_442 646.11 | e-118 | nitrilase [Synechocystis sp. PCC 6803]. | Synechocystis sp. PCC 6803 Bacteria | N/A | 62 | N/A | 338 | 346 |
| 321, 322 | gi\|16331 918\|ref\| NP_442 646.1\| | e-100 | nitrilase [Synechocystis sp. PCC 6803]. | Synechocystis sp. PCC 6803 Bacteria | N/A | 53 | N/A | 330 | 346 |
| 323, 324 | gi\|15229 936\|ref\| NP_190 018.1\| | 3E-52 | nitrilase 3 [Arabidopsis thaliana]. | Arabidopsis thaliana Eukaryota | N/A | 42 | N/A | 316 | 346 |
| 325, 326 | gi\|16331 918\|ref\| NP_442 646.11 | e-121 | nitrilase [Synechocystis sp. PCC 6803]. | Synechocystis sp. PCC 6803 Bacteria | N/A | 64 | N/A | 358 | 346 |
| 327, 328 | gi\|75109 01\|pir\|\|T 27679 | 5E-40 | probable nitrilase (EC 3.5.5.1) ZK1058.6 Caenorhabditis elegans. | Caenorhabditis elegans Eukaryota | 3.5.5.1 | 30 | 46 | 324 | 305 |
| 329, 330 | gi\|16331 918\|ref\| NP_442 646.1\| | e-113 | nitrilase [Synechocystis sp. PCC 6803]. | Synechocystis sp. PCC 6803 Bacteria | N/A | 60 | N/A | 340 | 346 |
| 331, 332 | gi\|15143 035\|emb ICAC50 776.1\| | 2E-98 | unnamed protein product [unidentified]. | unidentified unclassified. | N/A | 53 | 60 | 346 | 346 |
| 333, | gi\|15143 035\|emb | e-111 | unnamed protein | unidentified | N/A | 61 | 63 | 345 | 346 |
| 334 | ICAC50 776.1\| | | product [unidentified]. | unclassified. | | | | | |
| 335, 336 | gi\|16331 918\|ref\| NP_442 646.11 | e-120 | [Synechocystis sp. PCC 6803]. | nitrilase Synechocystis sp. PCC 6803 Bacteria | N/A | 62 | N/A | 350 | 346 |
| 337, 338 | gi\|17557 111\|ref\| NP_497 791.11 | 3E-22 | [Caenorhabditis elegans]. | Nitrilase Caenorhabditis elegans Eukaryota | N/A | 28 | N/A | 313 | 305 |
| 339, 340 | gi\|15143 035\|emb \|CAC50 776.1\| | 5E-25 | unnamed protein product [unidentified]. | unidentified unclassified. | N/A | 27 | N/A | 329 | 346 |
| 341, 342 | gi\|15143 035\|emb \|CAC50 776.1\| | 2E-11 | unnamed protein product [unidentified]. | unidentified unclassified. | N/A | 26 | N/A | 342 | 346 |
| 343, 344 | gi\|15229 932\|ref\| NP_190 016.1\| | 8E-18 | nitrilase 2 [Arabidopsis thaliana]. | Arabidopsis thaliana Eukaryota | N/A | 28 | N/A | 303 | 339 |
| 345, 346 | gi\|15143 035\|emb ICAC50 776.1\| | 3E-50 | unnamed protein product [unidentified]. | unidentified unclassified. | N/A | 40 | N/A | 329 | 346 |
| 347, 348 | gi\|15143 037\|emb ICAC50 777.1\| | 2E-11 | unnamed protein product [unidentified]. | unidentified unclassified. | N/A | 22 | N/A | 297 | 337 |
| 349, 350 | gi\|41738 4\|sp\|Q03 2171 | 5.00E-86 | Aliphatic Nitrilase | Rhodococcus rhodochrous | N/A | 43.3 | 53.7 | 333 | 366 |
| 351, 352 | gi\|15229 936\|ref\| NP_190 018.1\| | 1E-11 | nitrilase 3 [Arabidopsis thaliana]. | Arabidopsis thaliana Eukaryota | N/A | 22 | N/A | 301 | 346 |
| 353, 354 | gi\|15143 035\|emb ICAC50 776.1\| | 2E-10 | unnamed protein product [unidentified]. | unidentified unclassified. | N/A | 29 | N/A | 312 | 346 |
| 355, 356 | gi\|17231 038\|ref\| NP_487 586.1\| | 0.2 | heterocyst differentiation protein [Nostoc sp. PCC 7120]. | Nostoc sp. PCC 7120 Bacteria | N/A | 25 | N/A | 325 | 779 |
| 357, 358 | gi\|15242 205\|ref\| NP_197 622.1\| | 1E-15 | Nitrilase 4 (sp P46011) [Arabidop sis thaliana]. | Arabidopsis thaliana Eukaryota | N/A | 23 | N/A | 300 | 355 |
| 359, 360 | gi\|16331 918\|ref\| NP_442 646.1\| | 1E-58 | [Synechocystis sp. PCC 6803]. | Synechocystis sp. PCC 6803 Bacteria | N/A | 40 | N/A | 335 | 346 |
| 361, 362 | gi\|5795 659\|emb ICAC88 237.11 | 0.007 | unnamed protein product [Rhodococcus rhodochrous]. | Rhodococcus rhodochrous Bacteria | N/A | 24 | N/A | 302 | 366 |
| 363, 364 | gi\|6331 918\|ref\| NP_442 646.1\| | 1E-19 | [Synechocystis sp. PCC 6803]. | Synechocystis sp. PCC 6803 Bacteria | N/A | 22 | N/A | 335 | 346 |
| 365, 366 | gi\|48355 88\|dbj\|B AA7767 9.1\| | 0.004 | nitrilase-like protein [Oryza sativa]. | Oryza sativa Eukaryota | N/A | 17 | N/A | 315 | 362 |
| 367, 368 | gi\|41738 2\|sp\|Q02 068\|NR L1_RH ORH | 0.023 | ALIPHATIC NITRILASE. | Rhodococcus rhodochrous Bacteria | N/A | 18 | N/A | 317 | 383 |
| 369, 370 | gi\|17546 542\|ref\| NP_519 944.1\| | 0.00000 001 | PROBABLE NITRILASE PROTEIN [Ralstonia solanacearum]. | Ralstonia solanacearum Bacteria | N/A | 19 | N/A | 346 | 343 |
| 371, 372 | gi\|15143 037\|emb ICAC50 777.11 | 9E-23 | unnamed protein product [unidentified]. | unidentified unclassified. | N/A | 24 | N/A | 327 | 337 |
| 373, 374 | gi\|21206 06\|pir\|\|J C4212 | 7E-47 | nitrilase (EC 3.5.5.1) - Comamon as testosteroni. | Comamonas testosteroni Bacteria | 3.5.5.1 | 32 | N/A | 351 | 354 |
| 375, 376 | gi\|14211 396\|gb\| AAK57 436.1\| | 5E-59 | nitrilase-like protein [Brassica napus]. | Brassica napus Eukaryota | N/A | 36 | N/A | 312 | 350 |
| 377, 378 | gi\|59539 61\| | 1.00E-102 | Sequence 1 from patent US | N/A | N/A | *NlA* | N/A | 349 | N/A |
| 379, 380 | gi\|14211 396\|gb\| AAK57 436.11 | 6E-58 | 5872000. nitrilase-like protein [Brassica napus]. | Brassica napus Eukaryota | N/A | 38 | N/A | 311 | 350 |
| 381, 382 | gi\|17546 542\|ref\| NP_519 944.11 | 2E-40 | PROBABLE NITRILASE PROTEIN [Ralstonia solanacearum]. | Ralstonia solanacearum Bacteria | N/A | 33 | N/A | 314 | 343 |
| 383, 384 | gi\|15143 037\|emb ICAC50 777.1\| | e-108 | unnamed protein product [unidentified]. | unidentified unclassified. | N/A | 55 | 55 | 345 | 337 |
| 385, 386 | gi\|15143 035\|emb ICAC50 776.1\| | e-107 | unnamed protein product [unidentified]. | unidentified unclassified. | N/A | 57 | 57 | 337 | 346 |

### Computer Systems

Any nucleic acid sequence and/or polypeptide sequence of the invention can be stored, recorded, and manipulated on any medium which can be read and accessed by a computer. As used herein, the words "recorded" and "stored" refer to a process for storing information on a computer medium. A computer readable medium may have recorded thereon at least 2, 5, 10, 15 or 20 nucleic acid sequences as set forth in SEQ ID NOS: 1-386, and sequences substantially identical thereto. The comparison among and between nucleic acid sequences or polypeptide sequences of the disclosure is disclosed, and the comparison between sequences of the disclosure and other sequences by a computer. Computer readable media include magnetically readable media, optically readable media, electronically readable media and magnetic/optical media. For example, the computer readable media may be a hard disk, a floppy disk, a magnetic tape, CD-ROM, Digital Versatile Disk (DVD), Random Access Memory (RAM), or Read Only Memory (ROM) as well as other types of other media known to those skilled in the art.

Systems (e.g., internet based systems), particularly computer systems may store and manipulate the sequence information described herein. As used herein, "a computer system" refers to the hardware components, software components, and data storage components used to analyze a sequence (either nucleic acid or polypeptide) as set forth in at least any one of SEQ ID NOS: 1-386 and sequences substantially identical thereto. The computer system typically includes a processor for processing, accessing and manipulating the sequence data. The processor can be any well-known type of central processing unit, such as, for example, the Pentium III from Intel Corporation, or similar processor from Sun, Motorola, Compaq, AMD or International Business Machines.

Typically the computer system is a general purpose system that comprises the processor and one or more internal data storage components for storing data, and one or more data retrieving devices for retrieving the data stored on the data storage components.

The computer system may include a processor connected to a bus which is connected to a main memory (preferably implemented as RAM) and one or more internal data storage devices, such as a hard drive and/or other computer readable media having data recorded thereon. The computer system may further include one or more data retrieving device for reading the data stored on the internal data storage devices.

The data retrieving device may represent, for example, a floppy disk drive, a compact disk drive, a magnetic tape drive, or a modem capable of connection to a remote data storage system (*e*.*g*., via the internet) etc. In some embodiments, the internal data storage device is a removable computer readable medium such as a floppy disk, a compact disk, a magnetic tape, etc. containing control logic and/or data recorded thereon. The computer system may advantageously include or be programmed by appropriate software for reading the control logic and/or the data from the data storage component once inserted in the data retrieving device.

The computer system includes a display which is used to display output to a computer user. It should also be noted that the computer system can be linked to other computer systems in a network or wide area network to provide centralized access to the computer system. The computer system may further comprise a sequence comparison algorithm. A "sequence comparison algorithm" refers to one or more programs which are implemented (locally or remotely) on the computer system to compare a nucleotide sequence with other nucleotide sequences and/or compounds stored within a data storage means.

### Uses of Nitrilases

Nitrilases have been identified as key enzymes for the production of chiral α-hydroxy acids, which are valuable intermediates in the fine chemicals industry, and as pharmaceutical intermediates. The nitrilase enzymes of the invention are useful to catalyze the stereoselective hydrolysis of cyanohydrins and aminonitriles, producing chiral α-hydroxy- and α-amino acids, respectively.

Stereoselective enzymes provide a key advantage over chemical resolution methods as they do not require harsh conditions and are more environmentally compatible. The use of nitrilases is of particular interest for the production of chiral amino acids and α-hydroxy acids. Using a stereoselective nitrilase, dynamic resolution conditions can be established, due to the racemisation of the substrate under aqueous conditions. Thus 100% theoretical yields are achievable.

This invention is directed to the nitrilases which have been discovered and isolated from naturally occurring sources. This invention is also directed to evolving novel genes and gene pathways from diverse and extreme environmental sources. In an effort to develop the most extensive assortment of enzymes available, DNA was extracted directly from samples that have been collected from varying habitats around the globe. From these efforts, the largest collection of environmental genetic libraries in the world was developed. Through extensive high-throughput screening of these libraries, 192 new sequence-unique nitrilase enzymes have been discovered to date. Previous to this invention, fewer than 20 microbial-derived nitrilases had been reported in the literature and public databases.

Biocatalysts, such as nitrilases, play an important role in catalyzing metabolic reactions in living organisms. In addition, biocatalysts have found applications in the chemical industry, where they can perform many different reactions. Some examples of the advantages of the use of nitrilases is that they provide: high enantio-, chemo- and regio- selectivity; they function under mild reaction conditions; they provide direct access to products - with minimal protection; they have high catalytic efficiencies; they produce reduced waste compared with the chemical alternatives; they are easily immobilized as enzymes or cells; they are recoverable, recyclable and are capable of being manipulated via molecular biological techniques; they can be regenerated in whole cell processes; they are tolerant to organic solvents; and importantly, they can be evolved or optimized. Optimized nitrilases are presented herein as working examples of the invention.

Nitrilases catalyze the hydrolysis of nitrile moieties generating the corresponding carboxylic acid. Conventional chemical hydrolysis of nitriles requires strong acid or base and high temperature. However, one advantage of the invention is that nitrilases are provided which perform this reaction under mild conditions. Wide ranges of nitrile substrates can be transformed by nitrilases with high enantio-, chemo- and regio- selectivity.

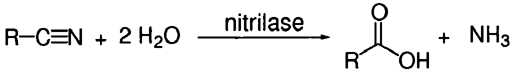

**Table 3 - Some characteristics of Nitrilases of the Invention**

| **Previously Discovered Nitrilases** | **New Nitrilases** | |
|---|---|---|
| **Limitations** | **New Features** | **Benefits** |
| < 20 reported | >180 newly discovered | Access to a wider |
| Homologous | Unique nitrilases, many with little homology to previously known nitrilases | substrate range |
| Narrow substrate activity spectrum | Broad substrate activity spectrum | |
| Very few shown to be enantioselective | Enantioselective; both enantiomers accessible | Product with high enantiomeric excess and minimal waste production |
| Limited stability profile | Stable in a variety of conditions | Potential use in a wide range of process conditions |
| Inconsistent supply | Consistent supply | Reliable source of product |
| Not applicable | Amenable to optimization | Good source material leads to better product |

Dynamic Kinetic Resolution: The use of the nitrilases allows discrimination between two rapidly equilibrating enantiomers to give a single product in 100% theoretical yield. Nitrilases are utilized for dynamic resolution of key cyanohydrins and aminonitriles to produce enantiomerically pure α-carboxylic and α-amino acids. Newly discovered nitrilases disclosed herein yield products with >95% enantiomeric excess (ee) with and >95% yield. The nitrilases perform this transformation efficiently under mild conditions in aqueous solution or in the presence of organic solvent.

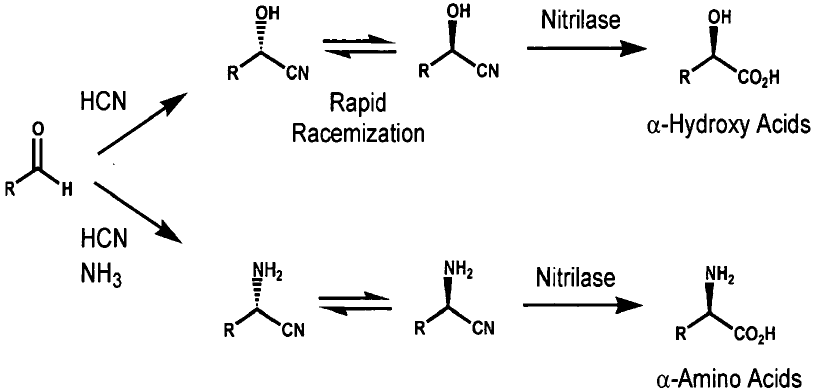

These products shown above also include the opposite enanatiomers, although they are not shown. The disclosure provides an isolated nucleic acid having a sequence as set forth in any one of the Group A nucleic acid sequences, having a sequence substantially identical thereto, or having a sequence complementary thereto.

An isolated nucleic acid may include at least 20 consecutive nucleotides identical to a portion of a nucleotide sequence as set forth in the Group A nucleic acid sequences, having a sequence substantially identical thereto, or having a sequence complementary thereto.

An isolated nucleic acid may encode a polypeptide having a sequence as set forth in the Group B amino acid sequences, or having a sequence substantially identical thereto.

An isolated nucleic acid may encode a polypeptide having at least 10 consecutive amino acids identical to a portion of a sequence as set forth in the Group B amino acid sequences, or having a sequence substantially identical thereto.

The disclosure provides a substantially purified polypeptide comprising consecutive amino acid residues having a sequence as set forth in the Group B amino acid sequences, or having a sequence substantially identical thereto.

An isolated antibody may specifically bind to a polypeptide of the disclosure. A fragment of the antibody may retain the ability to specifically bind the polypeptide.

A method of producing a polypeptide having a sequence as set forth in the Group B amino acid sequences, and sequences substantially identical thereto may include introducing a nucleic acid encoding the polypeptide into a host cell, wherein the nucleic acid is operably joined to a promoter, and culturing the host cell under conditions that allow expression of the nucleic acid.

A method of producing a polypeptide having at least 10 consecutive amino acids from a sequence as set forth in the Group B amino acid sequences, and sequences substantially identical thereto may include introducing a nucleic acid encoding the polypeptide into a host cell, wherein the nucleic acid is operably joined to a promoter, and culturing the host cell under conditions that allow expression of the nucleic acid, thereby producing the polypeptide.

A method of generating a variant of a nitrilase may include choosing a nucleic acid sequence as set forth in the Group A nucleic acid sequences, and changing one or more nucleotides in the sequence to another nucleotide, deleting one or more nucleotides in the sequence, or adding one or more nucleotides to the sequence.

Assays for identifying functional variants of the Group B amino acid sequences that retain the enzymatic function of the polypeptides of the Group B amino acid sequences may include contacting a polypeptide comprising consecutive amino acid residues having a sequence identical to a sequence of the Group B amino acid sequences or a portion thereof, having a sequence substantially identical to a sequence of the Group B amino acid sequences or a portion thereof, or having a sequence which is a variant of a sequence of the Group B amino acid sequences that retains nitrilase activity, with a substrate molecule under conditions which allow the polypeptide to function, and detecting either a decrease in the level of substrate or an increase in the level of a specific reaction product of the reaction between the polypeptide and the substrate, thereby identifying a functional variant of such sequences.

### Modification of Polypeptides of the Invention

Enzymes are highly selective catalysts. Their hallmark is the ability to catalyze reactions with exquisite stereo-selectivity, regio-selectivity, and chemo-selectivity that is unparalleled in conventional synthetic chemistry. Moreover, enzymes are remarkably versatile. They can be tailored to function in organic solvents, operate at extreme pHs (for example, acidic or basic conditions) extreme temperatures (for example, high temperatures and low temperatures), extreme salinity levels (for example, high salinity and low salinity), and catalyze reactions with compounds that can be structurally unrelated to their natural, physiological substrates except for the enzymatic active site.

Methods may modify polypeptides having nitrilase activity or polynucleotides encoding such polypeptides in order to obtain new polypeptides which retain nitrilase activity but which are improved with respect to some desired characteristic. Such improvements can include the ability to function (*i.e*., exhibit nitrilase activity) in organic solvents, operate at extreme or uncharacteristic pHs, operate at extreme or uncharacteristic temperatures, operate at extreme or uncharacteristic salinity levels, catalyze reactions with different substrates, etc.

The present invention directed to methods of using nitrilases so as to exploit the unique catalytic properties of these enzymes. Whereas the use of biocatalysts (*i.e*., purified or crude enzymes) in chemical transformations normally requires the identification of a particular biocatalyst that reacts with a specific starting compound, the present invention uses selected biocatalysts and reaction conditions that are specific for functional groups that are present in many starting compounds. Each biocatalyst is specific for one functional group, or several related functional groups, and can react with many starting compounds containing this functional group.

Enzymes react at specific sites within a starting compound without affecting the rest of the molecule, a process which is very difficult to achieve using traditional chemical methods. This high degree of specificity provides the means to identify a single active compound within a library of compounds. The library is characterized by the series of biocatalytic reactions used to produce it, a so-called "biosynthetic history." Screening the library for biological activities and tracing the biosynthetic history identifies the specific reaction sequence producing the active compound. The reaction sequence is repeated and the structure of the synthesized compound determined. This mode of identification, unlike other synthesis and screening approaches, does not require immobilization technologies, and compounds can be synthesized and tested free in solution using virtually any type of screening assay. It is important to note, that the high degree of specificity of enzyme reactions on functional groups allows for the "tracking" of specific enzymatic reactions that make up the biocatalytically produced library. (For further teachings on modification of molecules, including small molecules, see PCT Application No. PCT/US94/09174).

In the chimerization of a family of related nitrilase genes and their encoded family of related products the sequences of a plurality of nitrilase nucleic acids *(e.g.,* the Group A nucleic acids) serve as nitrilase "templates" which are aligned using a sequence comparison algorithm such as those described above. One or more demarcation points are then identified in the aligned template sequences, which are located at one or more areas of homology. The demarcation points can be used to delineate the boundaries of nucleic acid building blocks, which are used to generate chimeric nitrilases. Thus, the demarcation points identified and selected in the nitrilase template molecules serve as potential chimerization points in the assembly of the chimeric nitrilase molecules.

Typically, a useful demarcation point is an area of local identity between at least two progenitor templates, but preferably the demarcation point is an area of identity that is shared by at least half of the templates, at least two thirds of the templates, at least three fourths of the templates, or at nearly all of the templates.

The building blocks, which are defined by the demarcation points, can then be mixed (either literally, in solution, or theoretically, on paper or in a computer) and reassembled to form chimeric nitrilase genes. In one embodiment, the gene reassembly process is performed exhaustively in order to generate an exhaustive library of all possible combinations. In other words, all possible ordered combinations of the nucleic acid building blocks are represented in the set of finalized chimeric nucleic acid molecules. At the same time, however, the order of assembly of each building block in the 5' to 3' direction in each combination is designed to reflect the order in the templates, and to reduce the production of unwanted, inoperative products.

The gene reassembly process may be performed systematically, in order to generate a compartmentalized library with compartments that can be screened systematically, e.g., one by one. This provides that, through the selective and judicious use of specific nucleic acid building blocks, coupled with the selective and judicious use of sequentially stepped assembly reactions, an experimental design can be achieved where specific sets of chimeric products are made in each of several reaction vessels. This allows a systematic examination and screening procedure to be performed. Thus, it allows a potentially very large number of chimeric molecules to be examined systematically in smaller groups.

The synthetic nature of the step in which the building blocks are generated or reassembled allows the design and introduction of sequences of nucleotides (*e*.*g*., codons or introns or regulatory sequences) that can later be optionally removed in an *in vitro* process (*e.g*., by mutagenesis) or in an *in vivo* process (*e.g*., by utilizing the gene splicing ability of a host organism). The introduction of these nucleotides may be desirable for many reasons, including the potential benefit of creating a useful demarcation point.

The synthetic gene reassembly method utilizes a plurality of nucleic acid building blocks, each of which has two ligatable ends. Some examples of the two ligatable ends on each nucleic acid building block includes, but are not limited to, two blunt ends, or one blunt end and one overhang, or two overhangs. In a further, nonlimiting example, the overhang can include one base pair, 2 base pairs, 3 base pairs, 4 base pairs or more.

A double-stranded nucleic acid building block can be of variable size. Preferred sizes for building blocks range from about 1 base pair (bp) (not including any overhangs) to about 100,000 base pairs (not including any overhangs). Other preferred size ranges are also provided, which have lower limits of from about 1 bp to about 10,000 bp (including every integer value in between), and upper limits of from about 2 bp to about 100,000 bp (including every integer value in between).

A double-stranded nucleic acid building block may be generated by first generating two single stranded nucleic acids and allowing them to anneal to form a double-stranded nucleic acid building block. The two strands of a double-stranded nucleic acid building block may be complementary at every nucleotide apart from any that form an overhang; thus containing no mismatches, apart from any overhang(s). Alternatively, the two strands of a double-stranded nucleic acid building block can be complementary at fewer than every nucleotide, apart from any overhang(s). In particular, mismatches between the strands can be used to introduce codon degeneracy using methods such as the site-saturation mutagenesis described herein.

*In vivo* shuffling of molecules is also useful in providing variants and can be performed utilizing the natural property of cells to recombine multimers. While recombination *in vivo* has provided the major natural route to molecular diversity, genetic recombination remains a relatively complex process that involves (1) the recognition of homologies; (2) strand cleavage, strand invasion, and metabolic steps leading to the production of recombinant chiasma; and finally (3) the resolution of chiasma into discrete recombined molecules. The formation of the chiasma requires the recognition of homologous sequences.

Thus, a method may produce a chimeric or recombinant polynucleotide from at least a first polynucleotide and a second polynucleotide *in vivo.* This can be used to produce a recombinant polynucleotide by introducing at least a first polynucleotide and a second polynucleotide which share at least one region of partial sequence homology (*e.g*., the Group A nucleic acid sequences, and combinations thereof) into a suitable host cell. The regions of partial sequence homology promote processes which result in sequence reorganization producing a recombinant polynucleotide. Such hybrid polynucleotides can result from intermolecular recombination events which promote sequence integration between DNA molecules. In addition, such hybrid polynucleotides can result from intramolecular reductive reassortment processes which utilize repeated sequences to alter a nucleotide sequence within a DNA molecule.

Recombinant polynucleotides which encode biologically active variant polypeptides (*e*.*g*., a nitrilase variant may be generated). For example, a polynucleotide may encode a particular enzyme from one microorganism. An enzyme encoded by a first polynucleotide from one organism can, for example, function effectively under a particular environmental condition, *e*.*g*., high salinity. An enzyme encoded by a second polynucleotide from a different organism can function effectively under a different environmental condition, such as extremely high temperature. A recombined polynucleotide containing sequences from the first and second original polynucleotides encodes a variant enzyme which exhibits characteristics of both enzymes encoded by the original polynucleotides. Thus, the enzyme encoded by the recombined polynucleotide can function effectively under environmental conditions shared by each of the enzymes encoded by the first and second polynucleotides, *e*.*g*., high salinity and extreme temperatures.

A variant polypeptide can exhibit specialized enzyme activity not displayed in the original enzymes. For example, following recombination and/or reductive reassortment of polynucleotides encoding nitrilase activity, the resulting variant polypeptide encoded by a recombined polynucleotide can be screened for specialized nitrilase activity obtained from each of the original enzymes, *i.e*., the temperature or pH at which the nitrilase functions. Sources of the original polynucleotides may be isolated from individual organisms ("isolates"), collections of organisms that have been grown in defined media ("enrichment cultures"), or, uncultivated organisms ("environmental samples"). The use of a culture-independent approach to derive polynucleotides encoding novel bioactivities from environmental samples is most preferable since it allows one to access untapped resources of biodiversity. The microorganisms from which the polynucleotide may be prepared include prokaryotic microorganisms, such as *Xanthobacter*, *Eubacteria* and *Archaebacteria,* and lower eukaryotic microorganisms such as fungi, some algae and protozoa. Polynucleotides may be isolated from environmental samples in which case the nucleic acid may be recovered without culturing of an organism or recovered from one or more cultured organisms. In one aspect, such microorganisms may be extremophiles, such as hyperthermophiles, psychrophiles, psychrotrophs, halophiles, barophiles and acidophiles. Polynucleotides encoding enzymes isolated from extremophilic microorganisms are particularly preferred. Such enzymes may function at temperatures above 100°C in terrestrial hot springs and deep sea thermal vents, at temperatures below 0°C in arctic waters, in the saturated salt environment of the Dead Sea, at pH values around 0 in coal deposits and geothermal sulfur-rich springs, or at pH values greater than 11 in sewage sludge.

Examples of mammalian expression systems that can be employed to express recombinant proteins include the COS-7, C127, 3T3, CHO, HeLa and BHK cell lines. Mammalian expression vectors comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice and polyadenylation sites may be used to provide the required nontranscribed genetic elements. U.S. Patent No. 6,054,267 is relevant.

Host cells containing the polynucleotides of interest can be cultured in conventional nutrient media modified as appropriate for activating promoters, selecting transformants or amplifying genes. The culture conditions, such as temperature, pH and the like, are those previously used with the host cell selected for expression, and will be apparent to the ordinarily skilled artisan. Clones, which are identified as having a desired enzyme activity or other property may then be sequenced to identify the recombinant polynucleotide sequence encoding the enzyme having the desired activity or property.

In one embodiment, the invention provides for the isolated nitrilases as either an isolated nucleic acid or an isolated polypeptide wherein the nucleic acid or the polypeptide was prepared by recovering DNA from a DNA population derived from at least one uncultivated microorganism, and transforming a host with recovered DNA to produce a library of clones which is screened for the specified protein, *e*.*g*. nitrilase activity. U.S. Patent No. 6,280,926, Short, provides descriptions of such methods.

A method for producing a biologically active recombinant nitrilase polypeptide and screening such a polypeptide for desired activity or property may be by:
1) introducing at least a first nitrilase polynucleotide and a second nitrilase polynucleotide, said at least first nitrilase polynucleotide and second nitrilase polynucleotide sharing at least one region of sequence homology, into a suitable host cell;
2) growing the host cell under conditions which promote sequence reorganization resulting in a recombinant nitrilase polynucleotide;
3) expressing a recombinant nitrilase polypeptide encoded by the recombinant nitrilase polynucleotide;
4) screening the recombinant nitrilase polypeptide for the desired activity or property; and
5) isolating the recombinant nitrilase polynucleotide encoding the recombinant nitrilase polypeptide.

Examples of vectors which may be used include viral particles, baculovirus, phage, plasmids, phagemids, cosmids, fosmids, bacterial artificial chromosomes, viral DNA (*e*.*g*., vaccinia, adenovirus, fowlpox virus, pseudorabies and derivatives of SV40), P1-based artificial chromosomes, yeast plasmids, yeast artificial chromosomes, and any other vectors specific for the hosts of interest (*e.g*., *Bacillus, Aspergillus* and yeast). Large numbers of suitable vectors are known to those of skill in the art, and are commercially available. Examples of bacterial vectors include pQE vectors (Qiagen, Valencia, CA); pBluescript plasmids, pNH vectors, and lambda-ZAP vectors (Stratagene, La Jolla, CA); and pTRC99a, pKK223-3, pDR540, and pRIT2T vectors (Pharmacia, Peapack, NJ). Examples of eukaryotic vectors include pXT1 and pSG5 vectors (Stratagene, La Jolla, CA); and pSVK3, pBPV, pMSG, and pSVLSV40 vectors (Pharmacia, Peapack, NJ). However, any other plasmid or other vector may be used so long as they are replicable and viable in the host.

A preferred type of vector contains an f-factor (or fertility factor) origin of replication. The f-factor in *E*. *coli* is a plasmid which effects high frequency transfer of itself during conjugation and less frequent transfer of the bacterial chromosome itself. Particularly preferred is to use cloning vectors referred to as "fosmids" or bacterial artificial chromosome (BAC) vectors. These are derived from *E. coli* f-factor which is able to stably integrate large segments of genomic DNA.

The DNA sequence in the expression vector is operably joined to appropriate expression control sequences, including a promoter, to direct RNA synthesis. Useful bacterial promoters include lacI, lacZ, T3, T7, gpt, lambda P_{R}, P_{L} and trp. Useful eukaryotic promoters include CMV immediate early, HSV thymidine kinase, early and late SV40, LTRs from retrovirus, and mouse metallothionein-I. Selection of the appropriate vector and promoter is well within the level of ordinary skill in the art. The expression vector also contains a ribosome binding site for translation initiation and a transcription terminator. The vector may also include appropriate sequences for amplifying expression. Promoter regions can be selected from any desired gene using CAT (chloramphenicol transferase) vectors or other vectors with selectable markers.

In addition, the expression vectors can contain one or more selectable marker genes to provide a phenotypic trait for selection of transformed host cells. Useful selectable markers include dihydrofolate reductase or neomycin resistance for eukaryotic cell culture, or tetracycline or ampicillin resistance in *E*. *coli.*

The vector may be introduced into the host cells using any of a variety of techniques, including transformation, transfection, transduction, viral infection, gene guns, or Ti-mediated gene transfer. Particular methods include calcium phosphate transfection, DEAE-Dextran mediated transfection, lipofection, or electroporation

Reductive Reassortment - In another aspect, variant nitrilase polynucleotides can be generated by the process of reductive reassortment. Whereas recombination is an "inter-molecular" process which, in bacteria, is generally viewed as a "recA-dependent" phenomenon, the process of "reductive reassortment" occurs by an "intra-Molecular", recA-independent process. In this aspect, the invention can rely on the ability of cells to mediate reductive processes to decrease the complexity of quasi-repeated sequences in the cell by deletion. The method involves the generation of constructs containing consecutive repeated or quasi-repeated sequences (original encoding sequences), the insertion of these sequences into an appropriate vector, and the subsequent introduction of the vector into an appropriate host cell. The reassortment of the individual molecular identities occurs by combinatorial processes between the consecutive sequences in the construct possessing regions of homology, or between quasi-repeated units. The reassortment process recombines and/or reduces the complexity and extent of the repeated sequences, and results in the production of novel molecular species. Various treatments may be applied to enhance the rate of reassortment, such as ultra-violet light or DNA damaging chemicals. In addition, host cell lines displaying enhanced levels of "genetic instability" can be used.

Repeated Sequences - Repeated or "quasi-repeated" sequences play a role in genetic instability. "Quasi-repeats" are repeats that are not identical in structure but, rather, represent an array of consecutive sequences which have a high degree of similarity or identity sequences. The reductive reassortment or deletion process in the cell reduces the complexity of the resulting construct by deleting sequences between positions within quasi-repeated sequences. Because the deletion (and potentially insertion) events can occur virtually anywhere within the quasi-repetitive units, these sequences provide a large repertoire of potential variants.

When the quasi-repeated sequences are all ligated in the same orientation, for instance head-to-tail or vice versa, the endpoints of a deletion are, for the most part, equally likely to occur anywhere within the quasi-repeated sequences. In contrast, when the units are presented head-to-head or tail-to-tail, the inverted quasi-repeated sequences can form a duplex which delineates the endpoints of the adjacent units and thereby favors deletion of discrete units. Therefore, it is preferable that the quasi-repeated sequences are joined in the same orientation because random orientation of quasi-repeated sequences will result in the loss of reassortment efficiency, while consistent orientation of the sequences will offer the highest efficiency. Nonetheless, although having fewer of the contiguous sequences in the same orientation decreases the efficiency or reductive reassortment, it may still provide sufficient variation for the effective recovery of novel molecules.

Sequences can be assembled in a head-to-tail orientation using any of a variety of methods, including the following:
a) Primers can be utilized that include a poly-A head and poly-T tail which, when made single-stranded, would provide orientation. This is accomplished by having the first few bases of the primers made from RNA and hence easily removed by RNAse H.
b) Primers can be utilized that include unique restriction cleavage sites. Multiple sites, a battery of unique sequences, and repeated synthesis and ligation steps would be required.
c) The inner few bases of the primer can be thiolated and an exonuclease used to produce properly tailed molecules.

The recovery of the reassorted sequences relies on the identification of cloning vectors with a reduced repetitive index (RI). The reassorted coding sequences can then be recovered by amplification. The products are recloned and expressed. The recovery of cloning vectors with reduced RI can be effected by:
1) The use of vectors only stably maintained when the construct is reduced in complexity.
2) The physical recovery of shortened vectors by physical procedures. In this case, the cloning vector would be recovered using standard plasmid isolation procedures and then size-fractionated using standard procedures (*e.g*., agarose gel or column with a low molecular weight cut off).
3) The recovery of vectors containing interrupted genes can be selected when insert size decreases.
4) The use of direct selection techniques wherein an expression vector is used and the appropriate selection is carried out.

Coding sequences from related organisms may demonstrate a high degree of homology but nonetheless encode quite diverse protein products. These types of sequences are particularly useful as quasi-repeats. However, while the examples illustrated below demonstrate the reassortment of coding sequences with a high degree of identity (quasi-repeats), this process is not limited to nearly identical repeats.

Quasi-repetitive coding sequences derived from three different species are obtained. Each sequence encodes a protein with a distinct set of properties. Each of the sequences differs by one or more base pairs at unique positions in the sequences which are designated "A", "B" and "C". The quasi-repeated sequences are separately or collectively amplified and ligated into random assemblies such that all possible permutations and combinations are available in the population of ligated molecules. The number of quasi-repeat units can be controlled by the assembly conditions. The average number of quasi-repeated units in a construct is defined as the repetitive index (RI).

Once formed, the constructs can be size-fractionated on an agarose gel according to published protocols, inserted into a cloning vector, and transfected into an appropriate host cell. The cells can then be propagated to allow reductive reassortment to occur. The rate of the reductive reassortment process may be stimulated by the introduction of DNA damage if desired. Whether the reduction in RI is mediated by deletion formation between repeated sequences by an "intra-molecular" mechanism, or mediated by recombination-like events through "inter-molecular" mechanisms is immaterial. The end result is a reassortment of the molecules into all possible combinations.

In another aspect, prior to or during recombination or reassortment, polynucleotides of the invention or polynucleotides generated by the methods described herein can be subjected to agents or processes which promote the introduction of mutations into the original polynucleotides. The introduction of such mutations would increase the diversity of resulting hybrid polynucleotides and polypeptides encoded therefrom. The agents or processes which promote mutagenesis include, but are not limited to: (+)-CC-1065, or a synthetic analog such as (+)-CC-1065-(N3-adenine) (Sun et al. (1992), Biochemistry 31(10):2822-9); an N-acetylated or deacetylated 4'-fluoro-4-aminobiphenyl adduct capable of inhibiting DNA synthesis (see, for example, van de Poll et al. (1992), Carcinogenesis 13(5):751-8); or a N-acetylated or deacetylated 4-aminobiphenyl adduct capable of inhibiting DNA synthesis (see also, Van de Poll et al. (1992), *supra*); trivalent chromium, a trivalent chromium salt, a polycyclic aromatic hydrocarbon ("PAH") DNA adduct capable of inhibiting DNA replication, such as 7-bromomethyl-benz[a]anthracene ("BMA"), tris(2,3-dibromopropyl)phosphate ("Tris-BP"), 1,2-dibromo-3-chloropropane ("DBCP"), 2-bromoacrolein (2BA), benzo[a]pyrene-7,8-dihydrodiol-9-10-epoxide ("BPDE"), a platinum(II) halogen salt, N-hydroxy-2-amino-3-methylimidazo[4,5-f]-quinoline ("N-hydroxy-IQ"), and N-hydroxy-2-amino-1-methyl-6-phenylimidazo[4,5-f]-pyridine ("N-hydroxy-PhIP"). Especially preferred means for slowing or halting PCR amplification consist of UV light (+)-CC-1065 and (+)-CC-1065-(N3-Adenine). Particularly encompassed means are DNA adducts or polynucleotides comprising the DNA adducts from the polynucleotides or polynucleotides pool, which can be released or removed by a process including heating the solution comprising the polynucleotides prior to further processing.

GSSM™ - The invention also provides for the use of codon primers containing a degenerate N,N,G/T sequence to introduce point mutations into a polynucleotide, so as to generate a set of progeny polypeptides in which a full range of single amino acid substitutions is represented at each amino acid position, a method referred to as gene site-saturated mutagenesis (GSSM™). The oligos used are comprised contiguously of a first homologous sequence, a degenerate N,N,G/T sequence, and possibly a second homologous sequence. The progeny translational products from the use of such oligos include all possible amino acid changes at each amino acid site along the polypeptide, because the degeneracy of the N,N,G/T sequence includes codons for all 20 amino acids.

In one aspect, one such degenerate oligo (comprising one degenerate N,N,G/T cassette) is used for subjecting each original codon in a parental polynucleotide template to a full range of codon substitutions. In another aspect, at least two degenerate N,N,G/T cassettes are used - either in the same oligo or not, for subjecting at least two original codons in a parental polynucleotide template to a full range of codon substitutions. Thus, more than one N,N,G/T sequence can be contained in one oligo to introduce amino acid mutations at more than one site. This plurality of N,N,G/T sequences can be directly contiguous, or separated by one or more additional nucleotide sequences. In another aspect, oligos serviceable for introducing additions and deletions can be used either alone or in combination with the codons containing an N,N,G/T sequence, to introduce any combination or permutation of amino acid additions, deletions, and/or substitutions.

In a particular exemplification, it is possible to simultaneously mutagenize two or more contiguous amino acid positions using an oligo that contains contiguous N,N,G/T triplets, *i.e*., a degenerate (N,N,G/T)ₙ sequence.

This may provide for the use of degenerate cassettes having less degeneracy than the N,N,G/T sequence. For example, it may be desirable in some instances to use a degenerate triplet sequence comprised of only one N, where said N can be in the first second or third position of the triplet. Any other bases including any combinations and permutations thereof can be used in the remaining two positions of the triplet. Alternatively, it may be desirable in some instances to use a degenerate N,N,N triplet sequence, or an N,N, G/C triplet sequence.

It is appreciated, however, that the use of a degenerate triplet (such as N,N,G/T or an N,N, G/C triplet sequence) as disclosed herein is advantageous for several reasons. In one aspect, this provides a means to systematically and fairly easily generate the substitution of the full range of the 20 possible amino acids into each and every amino acid position in a polypeptide. Thus, for a 100 amino acid polypeptide, it provides a way to systematically and fairly easily generate 2000 distinct species (*i.e*., 20 possible amino acids per position times 100 amino acid positions). It is appreciated that there is provided, through the use of an oligo containing a degenerate N,N,G/T or an N,N, G/C triplet sequence, 32 individual sequences that code for the 20 possible amino acids. Thus, in a reaction vessel in which a parental polynucleotide sequence is subjected to saturation mutagenesis using one such oligo, there are generated 32 distinct progeny polynucleotides encoding 20 distinct polypeptides. In contrast, the use of a non-degenerate oligo in site-directed mutagenesis leads to only one progeny polypeptide product per reaction vessel.

The disclosure also provides for the use of nondegenerate oligonucleotides, which can optionally be used in combination with degenerate primers disclosed. It is appreciated that in some situations, it is advantageous to use nondegenerate oligos to generate specific point mutations in a working polynucleotide. This provides a means to generate specific silent point mutations, point mutations leading to corresponding amino acid changes, and point mutations that cause the generation of stop codons and the corresponding expression of polypeptide fragments.

Thus, in one embodiment, each saturation mutagenesis reaction vessel contains polynucleotides encoding at least 20 progeny polypeptide molecules such that all 20 amino acids are represented at the one specific amino acid position corresponding to the codon position mutagenized in the parental polynucleotide. The 32-fold degenerate progeny polypeptides generated from each saturation mutagenesis reaction vessel can be subjected to clonal amplification (*e*.*g*., cloned into a suitable *E*. *coli* host using an expression vector) and subjected to expression screening. When an individual progeny polypeptide is identified by screening to display a favorable change in property (when compared to the parental polypeptide), it can be sequenced to identify the correspondingly favorable amino acid substitution contained therein.

It is appreciated that upon mutagenizing each and every amino acid position in a parental polypeptide using saturation mutagenesis as disclosed herein, favorable amino acid changes may be identified at more than one amino acid position. One or more new progeny molecules can be generated that contain a combination of all or part of these favorable amino acid substitutions. For example, if 2 specific favorable amino acid changes are identified in each of 3 amino acid positions in a polypeptide, the permutations include 3 possibilities at each position (no change from the original amino acid, and each of two favorable changes) and 3 positions. Thus, there are 3 x 3 x 3 or 27 total possibilities, including 7 that were previously examined - 6 single point mutations (*i.e*., 2 at each of three positions) and no change at any position.

In yet another aspect, site-saturation mutagenesis can be used together with shuffling, chimerization, recombination and other mutagenizing processes, along with screening. This invention provides for the use of any mutagenizing process(es), including saturation mutagenesis, in an iterative manner. In one exemplification, the iterative use of any mutagenizing process(es) is used in combination with screening.

Thus, polynucleotides and polypeptides can be derived by saturation mutagenesis in combination with additional mutagenization processes, such as process where two or more related polynucleotides are introduced into a suitable host cell such that a hybrid polynucleotide is generated by recombination and reductive reassortment.

In addition to performing mutagenesis along the entire sequence of a gene, mutagenesis can be used to replace each of any number of bases in a polynucleotide sequence, wherein the number of bases to be mutagenized can be each integer from about 15 to about 100,000. Thus, instead of mutagenizing every position along a molecule, one can subject every or a discrete number of bases *(e.g.,* a subset totaling from about 15 to about 100,000) to mutagenesis. A separate nucleotide may be used for mutagenizing each position or group of positions along a polynucleotide sequence. A group of 3 positions to be mutagenized can be a codon. In one embodiment, the mutations are introduced using a mutagenic primer, containing a heterologous cassette, also referred to as a mutagenic cassette. For example, cassettes can have from about 1 to about 500 bases. Each nucleotide position in such heterologous cassettes can be N, A, C, G, T, A/C, A/G, A/T, C/G, C/T, G/T, C/G/T, A/G/T, A/C/T, A/C/G, or E, where E is any base that is not A, C, G, or T.

In a general sense, saturation mutagenesis comprises mutagenizing a complete set of mutagenic cassettes (for example, each cassette is about 1-500 bases in length) in a defined polynucleotide sequence to be mutagenized (for example, the sequence to be mutagenized is from about 15 to about 100,000 bases in length). Thus, a group of mutations (ranging from about 1 to about 100 mutations) is introduced into each cassette to be mutagenized. A grouping of mutations to be introduced into one cassette can be different or the same from a second grouping of mutations to be introduced into a second cassette during the application of one round of saturation mutagenesis. Such groupings are exemplified by deletions, additions, groupings of particular codons, and groupings of particular nucleotide cassettes.

Defined sequences to be mutagenized include a whole gene, pathway, cDNA, entire open reading frame (ORF), promoter, enhancer, repressor/transactivator, origin of replication, intron, operator, or any polynucleotide functional group. Generally, a "defined sequence" for this purpose may be any polynucleotide that a 15 base-polynucleotide sequence, and polynucleotide sequences of lengths between about 15 bases and about 15,000 bases (this invention specifically names every integer in between). Considerations in choosing groupings of codons include types of amino acids encoded by a degenerate mutagenic cassette.

In a particularly preferred exemplification a grouping of mutations that can be introduced into a mutagenic cassette, this specifically provides for degenerate codon substitutions (using degenerate oligos) that code for 2, 3, 4, 5, 6, 7, 8, 9, 10, 11, 12, 13, 14, 15, 16, 17, 18, 19, and 20 amino acids at each position, and a library of polypeptides encoded thereby.

An isolated nucleic acid may comprise one of the sequences of the Group A nucleic acid sequences, sequences substantially identical thereto, sequences complementary thereto, or a fragment comprising at least 10, 15, 20, 25, 30, 35, 40, 50, 75, 100, 150, 200, 300, 400, or 500 consecutive bases of one of the sequences of the Group A nucleic acid sequences. The isolated nucleic acids may comprise DNA, including cDNA, genomic DNA, and synthetic DNA. The DNA may be double-stranded or single-stranded, and if single stranded may be the coding strand or non-coding (anti-sense) strand. Alternatively, the isolated nucleic acids may comprise RNA.

As discussed in more detail below, the isolated nucleic acid sequences may be used to prepare one of the polypeptides of the Group B amino acid sequences, and sequences substantially identical thereto, or fragments comprising at least 5, 10, 15, 20, 25, 30, 35, 40, 50, 75, 100, or 150 consecutive amino acids of one of the polypeptides of the Group B amino acid sequences, and sequences substantially identical thereto.

Alternatively, the nucleic acid sequences may be mutagenized using conventional techniques, such as site directed mutagenesis, or other techniques familiar to those skilled in the art, to introduce silent changes into the polynucleotides of the Group A nucleic acid sequences, and sequences substantially identical thereto. As used herein, "silent changes" include, for example, changes which do not alter the amino acid sequence encoded by the polynucleotide. Such changes may be desirable in order to increase the level of the polypeptide produced by host cells containing a vector encoding the polypeptide by introducing codons or codon pairs which occur frequently in the host organism.

Polynucleotides may have nucleotide changes which result in amino acid substitutions, additions, deletions, fusions and truncations in the polypeptides of the disclosure (*e*.*g*., the Group B amino acid sequences). Such nucleotide changes may be introduced using techniques such as site-directed mutagenesis, random chemical mutagenesis, exonuclease III deletion, and other recombinant DNA techniques. Alternatively, such nucleotide changes may be naturally occurring allelic variants which are isolated by identifying nucleic acid sequences which specifically hybridize to probes comprising at least 10, 15, 20, 25, 30, 35, 40, 50, 75, 100, 150, 200, 300, 400, or 500 consecutive bases of one of the sequences of the Group A nucleic acid sequences, and sequences substantially identical thereto (or the sequences complementary thereto) under conditions of high, moderate, or low stringency as provided herein.

### Immobilized Enzyme Solid Supports

The enzymes, fragments thereof and nucleic acids which encode the enzymes and fragments can be affixed to a solid support. This is often economical and efficient in the use of the enzymes in industrial processes. For example, a consortium or cocktail of enzymes (or active fragments thereof), which are used in a specific chemical reaction, can be attached to a solid support and dunked into a process vat. The enzymatic reaction can occur. Then, the solid support can be taken out of the vat, along with the enzymes affixed thereto, for repeated use. The isolated nucleic acid may be affixed to a solid support, which may be selected from the group of a gel, a resin, a polymer, a ceramic, a glass, a microelectrode and any combination thereof.

For example, solid supports useful include gels. Some examples of gels include sepharose, gelatin, glutaraldehyde, chitosan-treated glutaraldehyde, albumin-glutaraldehyde, chitosan-Xanthan, toyopearl gel (polymer gel), alginate, alginate-polylysine, carrageenan, agarose, glyoxyl agarose, magnetic agarose, dextran-agarose, poly(Carbamoyl Sulfonate) hydrogel, BSA-PEG hydrogel, phosphorylated polyvinyl alcohol (PVA), monoaminoethyl-N-aminoethyl (MANA), amino, or any combination thereof.

Another solid support are resins or polymers. Some examples of resins or polymers include cellulose, acrylamide, nylon, rayon, polyester, anion-exchange resin, AMBERLITE™ XAD-7, AMBERLITE™ XAD-8, AMBERLITE™ IRA-94, AMBERLITE™ IRC-50, polyvinyl, polyacrylic, polymethacrylate, or any combination thereof. Another type of solid support useful is ceramic. Some examples include non-porous ceramic, porous ceramic, SiO₂, Al₂O₃. Another type of solid support useful is glass. Some examples include non-porous glass, porus glass, aminopropyl glass or any combination thereof. Another type of solid support which can be used is a mcroelectrode. An example is a polyethyleneimine-coated magnetite. Graphitic particles can be used as a solid support. Another example of a solid support is a cell, such as a red blood cell.

### Methods of immobilization

There are many methods which would be known to one of skill in the art for immobilizing enzymes or fragments thereof, or nucleic acids, onto a solid support. Some examples of such methods include electrostatic droplet generation, electrochemical means, via adsorption, via covalent binding, via cross-linking, via a chemical reaction or process, via encapsulation, via entrapment, via calcium alginate, or via poly (2-hydroxyethyl methacrylate). Like methods are described in Methods in Enzymology, Immobilized Enzymes and Cells, Part C. 1987. Academic Press. Edited by S. P. Colowick and N. O. Kaplan. Volume 136; and Immobilization of Enzymes and Cells. 1997. Humana Press. Edited by G. F. Bickerstaff. Series: Methods in Biotechnology, Edited by J. M. Walker.

Probes - The isolated nucleic acids of the Group A nucleic acid sequences, sequences substantially identical thereto, complementary sequences, or a fragment comprising at least 10, 15, 20, 25, 30, 35, 40, 50, 75, 100, 150, 200, 300, 400, or 500 consecutive bases of one of the foregoing sequences may also be used as probes to determine whether a biological sample, such as a soil sample, contains an organism having a nucleic acid sequence of the disclosure or an organism from which the nucleic acid was obtained. In such procedures, a biological sample potentially harboring the organism from which the nucleic acid was isolated is obtained and nucleic acids are obtained from the sample. The nucleic acids are contacted with the probe under conditions which permit the probe to specifically hybridize to any complementary sequences which are present therein.

Where necessary, conditions which permit the probe to specifically hybridize to complementary sequences may be determined by placing the probe in contact with complementary sequences from samples known to contain the complementary sequence as well as control sequences which do not contain the complementary sequence. Hybridization conditions, such as the salt concentration of the hybridization buffer, the formamide concentration of the hybridization buffer, or the hybridization temperature, can be varied to identify conditions which allow the probe to hybridize specifically to complementary nucleic acids. Stringent hybridization conditions are recited herein.

Hybridization may be detected by labeling the probe with a detectable agent such as a radioactive isotope, a fluorescent dye or an enzyme capable of catalyzing the formation of a detectable product. Many methods for using the labeled probes to detect the presence of complementary nucleic acids in a sample are familiar to those skilled in the art. These include Southern Blots, Northern Blots, colony hybridization procedures, and dot blots. Protocols for each of these procedures are provided in Ausubel et al. (1997), Current Protocols in Molecular Biology, John Wiley & Sons, Inc., and Sambrook et al. (1989), Molecular Cloning: A Laboratory Manual 2d Ed., Cold Spring Harbor Laboratory Press, the entire disclosures of which are incorporated herein by reference.

In one example, a probe DNA is "labeled" with one partner of a specific binding pair (*i.e*., a ligand) and the other partner of the pair is bound to a solid matrix to provide ease of separation of target from its source. For example, the ligand and specific binding partner can be selected from, in either orientation, the following: (1) an antigen or hapten and an antibody or specific binding fragment thereof; (2) biotin or iminobiotin and avidin or streptavidin; (3) a sugar and a lectin specific therefor; (4) an enzyme and an inhibitor therefor; (5) an apoenzyme and cofactor; (6) complementary homopolymeric oligonucleotides; and (7) a hormone and a receptor therefor. In one example, the solid phase is selected from: (1) a glass or polymeric surface; (2) a packed column of polymeric beads; and (3) magnetic or paramagnetic particles.

Alternatively, more than one probe (at least one of which is capable of specifically hybridizing to any complementary sequences which are present in the nucleic acid sample), may be used in an amplification reaction to determine whether the sample contains an organism containing a nucleic acid sequence of the disclosure (*e*.*g*., an organism from which the nucleic acid was isolated). Typically, the probes comprise oligonucleotides. In one embodiment, the amplification reaction may comprise a PCR reaction. PCR protocols are described in Ausubel et al. (1997), *supra,* and Sambrook et al. (1989), *supra.* Alternatively, the amplification may comprise a ligase chain reaction, 3SR, or strand displacement reaction. (See Barany (1991), PCR Methods and Applications 1:5-16; Fahy et al. (1991), PCR Methods and Applications 1:25-33; and Walker et al. (1992), Nucleic Acid Research 20:1691-1696.

Probes derived from sequences near the ends of a sequence as set forth in the Group A nucleic acid sequences, and sequences substantially identical thereto, may also be used in chromosome walking procedures to identify clones containing genomic sequences located adjacent to the nucleic acid sequences as set forth above. Such methods allow the isolation of genes which encode additional proteins from the host organism.

An isolated nucleic acid sequence as set forth in the Group A nucleic acid sequences, sequences substantially identical thereto, sequences complementary thereto, or a fragment comprising at least 10, 15, 20, 25, 30, 35, 40, 50, 75, 100, 150, 200, 300, 400, or 500 consecutive bases of one of the foregoing sequences may be used as probes to identify and isolate related nucleic acids. In some embodiments, the related nucleic acids may be cDNAs or genomic DNAs from organisms other than the one from which the nucleic acid was isolated. For example, the other organisms may be related organisms. In such procedures, a nucleic acid sample is contacted with the probe under conditions which permit the probe to specifically hybridize to related sequences. Hybridization of the probe to nucleic acids from the related organism is then detected using any of the methods described above.

In nucleic acid hybridization reactions, the conditions used to achieve a particular level of stringency will vary, depending on the nature of the nucleic acids being hybridized. For example, the length of the nucleic acids, the amount of complementarity between the nucleic acids, the nucleotide sequence composition (*e.g*., G-C rich v. A-T rich content), and the nucleic acid type (*e.g*., RNA v. DNA) can be considered in selecting hybridization conditions. Stringency may be varied by conducting the hybridization at varying temperatures below the melting temperatures of the probes. The melting temperature, Tm, is the temperature (under defined ionic strength and pH) at which 50% of the target sequence hybridizes to a perfectly complementary probe. Stringent conditions are selected to be equal to or about 5°C lower than the Tm for a particular probe. The melting temperature of the probe may be calculated using the following formulas:

For probes between 14 and 70 nucleotides in length the melting temperature (Tm) is calculated using the formula: Tm=81.5+16.6(log [Na⁺])+0.41(fraction G+C)-(600/N) where N is the length of the probe.

If the hybridization is carried out in a solution containing formamide, the melting temperature may be calculated using the equation: Tm=81.5+16.6(log [Na⁺])+0.41(fraction G+C)-(0.63% formamide)-(600/N) where N is the length of the probe.

Expression Libraries - Expression libraries can be created using the polynucleotides of the disclosure in combination with expression vectors and appropriate host cells. The library allows for the *in vivo* expression of the polypeptides which are encoded by the polynucleotides of the disclosure. After such expression libraries have been generated one can include the additional step of "biopanning" such libraries prior to screening by cell sorting. The "biopanning" procedure refers to a process for identifying clones having a specified biological activity by screening for sequence identity in a library of clones prepared by (i) selectively isolating target DNA derived from at least one microorganism by use of at least one probe DNA comprising at least a portion of a DNA sequence encoding a polypeptide having a specified biological activity (*e.g*., nitrilase activity); and (ii) optionally transforming a host with the isolated target DNA to produce a library of clones which are screened for the specified biological activity.

The probe DNA used for selectively isolating the target DNA of interest from the DNA derived from at least one microorganism can be a full-length coding region sequence or a partial coding region sequence of DNA for an enzyme of known activity. The original DNA library can be probed using mixtures of probes comprising at least a portion of DNA sequences encoding enzymes having the specified enzyme activity. These probes or probe libraries are single-stranded and the microbial DNA which is probed has been converted into single-stranded form. The probes that are particularly suitable are those derived from DNA encoding enzymes having an activity similar or identical to the specified enzyme activity that is to be screened.

Having prepared a multiplicity of clones from DNA selectively isolated from an organism, such clones are screened for a specific enzyme activity and to identify the clones having the specified enzyme characteristics.

The screening for enzyme activity may be affected on individual expression clones or may be initially affected on a mixture of expression clones to ascertain whether or not the mixture has one or more specified enzyme activities. If the mixture has a specified enzyme activity, then the individual clones may be rescreened for such enzyme activity or for a more specific activity. Thus, for example, if a clone mixture has nitrilase activity, then the individual clones may be recovered and screened to determine which of such clones has nitrilase activity.

As described with respect to one of the above aspects, there is provided a process for enzyme activity screening of clones containing selected DNA derived from a microorganism which process includes: screening a library for specified enzyme activity, said library including a plurality of clones, said clones having been prepared by recovering from genomic DNA of a microorganism selected DNA, which DNA is selected by hybridization to at least one DNA sequence which is all or a portion of a DNA sequence encoding an enzyme having the specified activity; and transforming a host with the selected DNA to produce clones which are screened for the specified enzyme activity.

A DNA library derived from a microorganism may be subjected to a selection procedure to select therefrom DNA which hybridizes to one or more probe DNA sequences which is all or a portion of a DNA sequence encoding an enzyme having the specified enzyme activity by:
(a) contacting the single-stranded DNA population from the DNA library with the DNA probe bound to a ligand under stringent hybridization conditions so as to produce a duplex between the probe and a member of the DNA library;
(b) contacting the duplex with a solid phase specific binding partner for the ligand so as to produce a solid phase complex;
(c) separating the solid phase complex from the non-duplexed members of the DNA library;
(d) denaturing the duplex to release the member of the DNA library;
(e) creating a complementary DNA strand of the member from step (d) so as to make the member a double-stranded DNA;
(f) introducing the double-stranded DNA into a suitable host so as to express a polypeptide which is encoded by the member DNA; and
(g) determining whether the polypeptide expressed exhibits the specified enzymatic activity.

In another aspect, the process includes a preselection to recover DNA including signal or secretion sequences. In this manner it is possible to select from the genomic DNA population by hybridization as hereinabove described only DNA which includes a signal or secretion sequence. The following paragraphs describe the protocol for this, the nature and function of secretion signal sequences in general and a specific exemplary application of such sequences to an assay or selection process.

A particularly embodiment of this aspect further comprises, after (a) but before (b) above, the steps of:
(i) contacting the single-stranded DNA population of (a) with a ligand-bound oligonucleotide probe that is complementary to a secretion signal sequence unique to a given class of proteins under hybridization conditions to form a double-stranded DNA duplex;
(ii) contacting the duplex of (i) with a solid phase specific binding partner for said ligand so as to produce a solid phase complex;
(iii) separating the solid phase complex from the single-stranded DNA population of (a);
(iv) denaturing the duplex so as to release single-stranded DNA members of the genomic population; and
(v) separating the single-stranded DNA members from the solid phase bound probe.

The DNA which has been selected and isolated to include a signal sequence is then subjected to the selection procedure hereinabove described to select and isolate therefrom DNA which binds to one or more probe DNA sequences derived from DNA encoding an enzyme(s) having the specified enzyme activity. This procedure is described and exemplified in U.S. Pat. No. 6,054,267.

*In vivo* biopanning may be performed utilizing a (fluorescence activated cell sorter) FACS-based machine. Complex gene libraries are constructed with vectors which contain elements which stabilize transcribed RNA. For example, the inclusion of sequences which result in secondary structures such as hairpins which are designed to flank the transcribed regions of the RNA would serve to enhance their stability, thus increasing their half life within the cell. The probe molecules used in the biopanning process consist of oligonucleotides labeled with reporter molecules that only fluoresce upon binding of the probe to a target molecule. These probes are introduced into the recombinant cells from the library using one of several transformation methods. The probe molecules bind to the transcribed target mRNA resulting in DNA/RNA heteroduplex molecules. Binding of the probe to a target will yield a fluorescent signal that is detected and sorted by the FACS machine during the screening process.

In some embodiments, the nucleic acid encoding one of the polypeptides of the Group B amino acid sequences, sequences substantially identical thereto, or fragments comprising at least about 5, 10, 15, 20, 25, 30, 35, 40, 50, 75, 100, or 150 consecutive amino acids thereof is assembled in appropriate phase with a leader sequence capable of directing secretion of the translated polypeptide or fragment thereof. Optionally, the nucleic acid can encode a fusion polypeptide in which one of the polypeptides of the Group B amino acid sequences, sequences substantially identical thereto, or fragments comprising at least 5, 10, 15, 20, 25, 30, 35, 40, 50, 75, 100, or 150 consecutive amino acids thereof is fused to heterologous peptides or polypeptides, such as N-terminal identification peptides which impart desired characteristics, such as increased stability or simplified purification.

The host cell may be any of the host cells familiar to those skilled in the art, including prokaryotic cells, eukaryotic cells, mammalian cells, insect cells, or plant cells. As representative examples of appropriate hosts, there may be mentioned: bacterial cells, such as *E. coli, Streptomyces, Bacillus subtilis, Salmonella typhimurium* and various species within the genera *Pseudomonas, Streptomyces,* and *Staphylococcus,* fungal cells, such as yeast, insect cells such as *Drosophila* S2 and *Spodoptera* Sf9, animal cells such as CHO, COS or Bowes melanoma, and adenoviruses. The selection of an appropriate host is within the abilities of those skilled in the art.

Where appropriate, the engineered host cells can be cultured in conventional nutrient media modified as appropriate for activating promoters, selecting transformants or amplifying the genes of the disclosure. Following transformation of a suitable host strain and growth of the host strain to an appropriate cell density, the selected promoter may be induced by appropriate means (*e*.*g*., temperature shift or chemical induction) and the cells may be cultured for an additional period to allow them to produce the desired polypeptide or fragment thereof.

Cells are typically harvested by centrifugation, disrupted by physical or chemical means, and the resulting crude extract is retained for further purification. Microbial cells employed for expression of proteins can be disrupted by any convenient method, including freeze-thaw cycling, sonication, mechanical disruption, or use of cell lysing agents. Such methods are well known to those skilled in the art. The expressed polypeptide or fragment thereof can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the polypeptide. If desired, high performance liquid chromatography (HPLC) can be employed for final purification steps.

Various mammalian cell culture systems can also be employed to express recombinant protein. Examples of mammalian expression systems include the COS-7 lines of monkey kidney fibroblasts (described by Gluzman (1981), Cell 23:175,), and other cell lines capable of expressing proteins from a compatible vector, such as the C127, 3T3, CHO, HeLa and BHK cell lines.

The disclosure also relates to variants of the polypeptides of the Group B amino acid sequences, sequences substantially identical thereto, or fragments comprising at least 5, 10, 15, 20, 25, 30, 35, 40, 50, 75, 100, or 150 consecutive amino acids thereof. In particular, the variants may differ in amino acid sequence from the polypeptides of the Group B amino acid sequences, and sequences substantially identical thereto, by one or more substitutions, additions, deletions, fusions and truncations, which may be present in any combination.

The variants may be naturally occurring or created *in vitro.* In particular, such variants may be created using genetic engineering techniques such as site directed mutagenesis, random chemical mutagenesis, Exonuclease III deletion procedures, and standard cloning techniques. Alternatively, such variants, fragments, analogs, or derivatives may be created using chemical synthesis or modification procedures.

Other methods of making variants are also familiar to those skilled in the art. These include procedures in which nucleic acid sequences obtained from natural isolates are modified to generate nucleic acids which encode polypeptides having characteristics which enhance their value in industrial or laboratory applications. In such procedures, a large number of variant sequences having one or more nucleotide differences with respect to the sequence obtained from the natural isolate are generated and characterized. Typically, these nucleotide differences result in amino acid changes with respect to the polypeptides encoded by the nucleic acids from the natural isolates.

### Error Prone PCR

For example, variants may be created using error prone PCR. In error prone PCR, PCR is performed under conditions where the copying fidelity of the DNA polymerase is low, such that a high rate of point mutations is obtained along the entire length of the PCR product. Error prone PCR is described in Leung et al. (1989), Technique 1:11-15 and Caldwell et al. (1992), PCR Methods Applic. 2:28-33. Briefly, in such procedures, nucleic acids to be mutagenized are mixed with PCR primers and reagents (e.g., reaction buffer, MgCl₂, MnCl₂, Taq polymerase and an appropriate concentration of dNTPs) for achieving a high rate of point mutation along the entire length of the PCR product. For example, the reaction may be performed using 20 fmoles of nucleic acid to be mutagenized, 30 pmoles of each PCR primer, a reaction buffer comprising 50mM KCl, 10mum Tris HCl (pH 8.3) and 0.01% gelatin, 7mM MgCl₂, 0.5mM MnCl₂, 5 units of Taq polymerase, 0.2mM dGTP, 0.2mM dATP, 1mM dCTP, and 1mM dTTP. PCR may be performed for 30 cycles of 94°C for 1 min, 45°C for 1 min, and 72°C for 1 min. However, it will be appreciated that these parameters may be varied as appropriate. The mutagenized nucleic acids are cloned into an appropriate vector and the activities of the polypeptides encoded by the mutagenized nucleic acids are evaluated.

Variants also may be created using oligonucleotide directed mutagenesis to generate site-specific mutations in any cloned DNA of interest. Oligonucleotide mutagenesis is described in Reidhaar-Olson et al. (1988), Science, 241:53-57. Briefly, in such procedures a plurality of double stranded oligonucleotides bearing one or more mutations to be introduced into the cloned DNA are synthesized and inserted into the cloned DNA to be mutagenized. Clones containing the mutagenized DNA are recovered and the activities of the polypeptides they encode are assessed.

### Assembly PCR

Another method for generating variants is assembly PCR. Assembly PCR involves the assembly of a PCR product from a mixture of small DNA fragments. A large number of different PCR reactions occur in parallel in the same vial, with the products of one reaction priming the products of another reaction. Assembly PCR is described in U.S. Pat. No. 5,965,408.

### Sexual PCR mutagenesis

Still another method of generating variants is sexual PCR mutagenesis. In sexual PCR mutagenesis, forced homologous recombination occurs between DNA molecules of different but highly related DNA sequence *in vitro*, as a result of random fragmentation of the DNA molecule based on sequence homology, followed by fixation of the crossover by primer extension in a PCR reaction. Sexual PCR mutagenesis is described in Stemmer (1994), Proc. Natl. Acad. Sci. USA 91:10747-10751. Briefly, in such procedures a plurality of nucleic acids to be recombined are digested with DNAse to generate fragments having an average size of about 50-200 nucleotides. Fragments of the desired average size are purified and resuspended in a PCR mixture. PCR is conducted under conditions which facilitate recombination between the nucleic acid fragments. For example, PCR may be performed by resuspending the purified fragments at a concentration of 10-30 ng/:1 in a solution of 0.2mM of each dNTP, 2.2mM MgCl₂, 50mM KCl, 10m Tris HCl, pH 9.0, and 0.1% Triton X-100. 2.5 units of Taq polymerase per 100:1 of reaction mixture is added and PCR is performed using the following regime: 94°C for 60 seconds, 94°C for 30 seconds, 50-55°C for 30 seconds, 72°C for 30 seconds (30-45 times) and 72°C for 5 minutes. However, it will be appreciated that these parameters may be varied as appropriate. In some embodiments, oligonucleotides may be included in the PCR reactions. In other embodiments, the Klenow fragment of DNA polymerase I may be used in a first set of PCR reactions and Taq polymerase may be used in a subsequent set of PCR reactions. Recombinant sequences are isolated and the activities of the polypeptides they encode are assessed.

### In vivo Mutagenesis

Variants may also be created by *in vivo* mutagenesis. In some embodiments, random mutations in a sequence of interest are generated by propagating the sequence of interest in a bacterial strain, such as an *E. coli* strain, which carries mutations in one or more of the DNA repair pathways. Such "mutator" strains have a higher random mutation rate than that of a wild-type parent. Propagating the DNA in one of these strains will eventually generate random mutations within the DNA. Mutator strains suitable for use for in vivo mutagenesis are described in PCT Publication No. WO 91/16427.

### Cassette Mutagenesis

Variants may also be generated using cassette mutagenesis. In cassette mutagenesis a small region of a double stranded DNA molecule is replaced with a synthetic oligonucleotide "cassette" that differs from the native sequence. The oligonucleotide often contains completely and/or partially randomized native sequence.

### Recursive Ensemble Mutagenesis

Recursive ensemble mutagenesis may also be used to generate variants. Recursive ensemble mutagenesis is an algorithm for protein engineering (protein mutagenesis) developed to produce diverse populations of phenotypically related mutants whose members differ in amino acid sequence. This method uses a feedback mechanism to control successive rounds of combinatorial cassette mutagenesis. Recursive ensemble mutagenesis is described in Arkin et al. (1992), Proc. Natl. Acad. Sci. USA, 89:7811-7815.

### Exponential Ensemble Mutagenesis

In some embodiments, variants are created using exponential ensemble mutagenesis. Exponential ensemble mutagenesis is a process for generating combinatorial libraries with a high percentage of unique and functional mutants, wherein small groups of residues are randomized in parallel to identify, at each altered position, amino acids which lead to functional proteins. Exponential ensemble mutagenesis is described in Delegrave et al. (1993), Biotechnology Research 11:1548-1552.

### Random and site-directed mutagenesis

Random and site-directed mutagenesis is described in Arnold (1993), Current Opinions in Biotechnology 4:450-455.

### Shuffling Procedures

In some embodiments, the variants are created using shuffling procedures wherein portions of a plurality of nucleic acids which encode distinct polypeptides are fused together to create chimeric nucleic acid sequences which encode chimeric polypeptides as described in U.S. Patent. Nos. 5,965,408 and 5,939,250.

The variants of the polypeptides of the Group B amino acid sequences may be variants in which one or more of the amino acid residues of the polypeptides of the Group B amino acid sequences are substituted with a conserved or non-conserved amino acid residue (*e*.*g*, a conserved amino acid residue) and such substituted amino acid residue may or may not be one encoded by the genetic code.

Conservative substitutions are those that substitute a given amino acid in a polypeptide by another amino acid of like characteristics. Typically seen as conservative substitutions are the following replacements: replacements of an aliphatic amino acid such as Alanine, Valine, Leucine and Isoleucine with another aliphatic amino acid; replacement of a Serine with a Threonine or vice versa; replacement of an acidic residue such as Aspartic acid and Glutamic acid with another acidic residue; replacement of a residue bearing an amide group, such as Asparagine and Glutamine, with another residue bearing an amide group; exchange of a basic residue such as Lysine and Arginine with another basic residue; and replacement of an aromatic residue such as Phenylalanine, Tyrosine with another aromatic residue.

Other variants are those in which one or more of the amino acid residues of the polypeptides of the Group B amino acid sequences includes a substituent group.

Still other variants are those in which the polypeptide is associated with another compound, such as a compound to increase the half-life of the polypeptide (for example, polyethylene glycol).

Additional variants are those in which additional amino acids are fused to the polypeptide, such as a leader sequence, a secretory sequence, a proprotein sequence or a sequence which facilitates purification, enrichment, or stabilization of the polypeptide.

In some embodiments, the fragments, derivatives and analogs retain the same biological function or activity as the polypeptides of the Group B amino acid sequences, and sequences substantially identical thereto. In other embodiments, the fragment, derivative, or analog includes a proprotein, such that the fragment, derivative, or analog can be activated by cleavage of the proprotein portion to produce an active polypeptide.

Polypeptides or fragments thereof may have at least about 85%, at least about 90%, at least about 95%, or more than about 95% homology to one of the polypeptides of the Group B amino acid sequences, sequences substantially identical thereto, or a fragment comprising at least 5, 10, 15, 20, 25, 30, 35, 40, 50, 75, 100, or 150 consecutive amino acids thereof. Percent identity may be determined using any of the programs described above which aligns the polypeptides or fragments being compared and determines the extent of amino acid homology or similarity between them. It will be appreciated that amino acid "homology" includes conservative amino acid substitutions such as those described above. The fragments can be used to generate antibodies. These antibodies can be used to immobilize nitrilases can be used in industrial processes. Polynucleotides encoding the nitrilases can be used in a similar way.

Alternatively, the homologous polypeptides or fragments may be obtained through biochemical enrichment or purification procedures. The sequence of potentially homologous polypeptides or fragments may be determined by proteolytic digestion, gel electrophoresis and/or microsequencing. The sequence of the prospective homologous polypeptide or fragment can be compared to one of the polypeptides of the Group B amino acid sequences, sequences substantially identical thereto, or a fragment comprising at least about 5, 10, 15, 20, 25, 30, 35, 40, 50, 75, 100, or 150 consecutive amino acids thereof using any of the programs described herein.

An assay may identify fragments or variants of the Group B amino acid sequences, or sequences substantially identical thereto, which retain the enzymatic function of the polypeptides of the Group B amino acid sequences, and sequences substantially identical thereto. For example, the fragments or variants of the polypeptides, may be used to catalyze biochemical reactions, which indicate that said fragment or variant retains the enzymatic activity of the polypeptides in Group B amino acid sequences.

The assay for determining if fragments of variants retain the enzymatic activity of the polypeptides of the Group B amino acid sequences, and sequences substantially identical thereto includes the steps of: contacting the polypeptide fragment or variant with a substrate molecule under conditions which allow the polypeptide fragment or variant to function, and detecting either a decrease in the level of substrate or an increase in the level of the specific reaction product of the reaction between the polypeptide and substrate.

The polypeptides of the Group B amino acid sequences, sequences substantially identical thereto or fragments comprising at least 5, 10, 15, 20, 25, 30, 35, 40, 50, 75, 100, or 150 consecutive amino acids thereof may be used in a variety of applications. For example, the polypeptides or fragments thereof may be used to catalyze biochemical reactions. There is provided a process for utilizing a polypeptide of the Group B amino acid sequences, and sequences substantially identical thereto or polynucleotides encoding such polypeptides for hydrolyzing aminonitriles. In such procedures, a substance containing a haloalkane compound is contacted with one of the polypeptides of the Group B amino acid sequences, and sequences substantially identical thereto under conditions which facilitate the hydrolysis of the compound.

Antibodies - The polypeptides of Group B amino acid sequences, sequences substantially identical thereto or fragments comprising at least 5, 10, 15, 20, 25, 30, 35, 40, 50, 75, 100, or 150 consecutive amino acids thereof, may also be used to generate antibodies which bind specifically to the enzyme polypeptides or fragments. The resulting antibodies may be used in immunoaffinity chromatography procedures to isolate or purify the polypeptide or to determine whether the polypeptide is present in a biological sample. In such procedures, a protein preparation, such as an extract, or a biological sample is contacted with an antibody capable of specifically binding to one of the polypeptides of the Group B amino acid sequences, sequences substantially identical thereto, or fragments of the foregoing sequences.

In immunoaffinity procedures, the antibody is attached to a solid support, such as a bead or column matrix. The protein preparation is placed in contact with the antibody under conditions under which the antibody specifically binds to one of the polypeptides of the Group B amino acid sequences, sequences substantially identical thereto, or fragments thereof. After a wash to remove non-specifically bound proteins, the specifically bound polypeptides are eluted.

The ability of proteins in a biological sample to bind to the antibody may be determined using any of a variety of procedures familiar to those skilled in the art. For example, binding may be determined by labeling the antibody with a detectable label such as a fluorescent agent, an enzymatic label, or a radioisotope. Alternatively, binding of the antibody to the sample may be detected using a secondary antibody having such a detectable label thereon. Particular assays include ELISA assays, sandwich assays, radioimmunoassays, and Western Blots.

The antibodies can be attached to solid supports and used to immobilize nitrilases. Such immobilized nitrilases can be used, as described above, in industrial chemical processes for the conversion of nitriles to a wide range of useful products and intermediates.

Polyclonal antibodies generated against the polypeptides of the Group B amino acid sequences, and sequences substantially identical thereto, or fragments comprising at least 5, 10, 15, 20, 25, 30, 35, 40, 50, 75, 100, or 150 consecutive amino acids thereof can be obtained by direct injection of the polypeptides into an animal or by administering the polypeptides to an animal. The antibody so obtained will then bind the polypeptide itself. In this manner, even a sequence encoding only a fragment of the polypeptide can be used to generate antibodies which may bind to the whole native polypeptide. Such antibodies can then be used to isolate the polypeptide from cells expressing that polypeptide.

For preparation of monoclonal antibodies, any technique which provides antibodies produced by continuous cell line cultures can be used. Examples include the hybridoma technique (Kohler and Milstein (1975), Nature, 256:495-497, the trioma technique, the human B-cell hybridoma technique (Kozbor et al. (1983), Immunology Today 4:72, and the EBV-hybridoma technique (Cole et al. (1985), in Monoclonal Antibodies and Cancer Therapy, Alan R. Liss, Inc., pp. 77-96.

Techniques described for the production of single chain antibodies (U.S. Pat. No. 4,946,778) can be adapted to produce single chain antibodies to the polypeptides of, for example, the Group B amino acid sequences, or fragments thereof. Alternatively, transgenic mice may be used to express humanized antibodies to these polypeptides or fragments.

Antibodies generated against a polypeptide of the Group B amino acid sequences, sequences substantially identical thereto, or fragments comprising at least 5, 10, 15, 20, 25, 30, 35, 40, 50, 75, 100, or 150 consecutive amino acids thereof may be used in screening for similar polypeptides from other organisms and samples. In such techniques, polypeptides from the organism are contacted with the antibody and those polypeptides which specifically bind to the antibody are detected. Any of the procedures described above may be used to detect antibody binding. One such screening assay is described in "Methods for Measuring Cellulase Activities", Methods in Enzymology, 160:87-116.

### Use of Whole Cells Comprising A Nucleic Acid

There may be used whole cells which have been transformed with nucleic acid (or an active fragment thereof) encoding one or more of the nitrilases of the disclosure. Such a whole cell may be used in performing a nitrilase reaction on a substrate. Therefore, methods of hydrolyzing a cyanohydrin or aminonitrile linkage may use a whole cell comprising at least one nucleic acid or polypeptide disclosed herein (SEQ ID NOS: 1-386). For example, a whole cell which is stably transfected (the disclosure also encompasses transiently transfected or transformed whole cells) with a nucleic acid encoding a nitrilase is included. Such a cell is useful as a reagent in a reaction mixture to act on a substrate and exhibit nitrilase activity.

### Sequence Analysis Software

Percent identity or homology between two or more sequences is typically measured using sequence analysis software (*e*.*g*., Sequence Analysis Software Package of the Genetics Computer Group, University of Wisconsin Biotechnology Center, Madison, WI). Such software matches similar sequences by assigning a percent identity or homology to various deletions, substitutions and other modifications. The term "percent identity," in the context of two or more nucleic acids or polypeptide sequences, refers to the percentage of nucleotides or amino acid residues that are the same when compared after being aligned for maximum correspondence over a designated region or comparison "window." Under some algorithms, a conservative amino acid substitution can be considered "identical" and a change at a wobble site of a codon can be considered "identical."

"Alignment" refers to the process of lining up two or more sequences to achieve maximal correspondence for the purpose of assessing the degree of identity or homology, as defined within the context of the relevant alignment algorithm.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated for a particular algorithm. Default program parameters can be used, or alternative parameters can be designated. The sequence comparison algorithm then calculates the percent identity or homology for the test sequences relative to the reference sequence, based on the program parameters.

A "comparison window", as used herein, is a segment of the contiguous positions in a nucleic acid or an amino acid sequence consisting of from 20 to 600, usually about 50 to about 200, more usually about 100 to about 150 nucleotides or residues, which may be compared to a reference sequence of the same or different number of contiguous positions after the two sequences are optimally aligned. Methods of alignment of sequences for comparison are well-known in the art. Optimal alignment of sequences for comparison can be conducted, e.g., by the local homology algorithm of Smith and Waterman (1981), Adv. Appl. Math. 2:482, by the homology alignment algorithm of Needleman and Wunsch (1970), J. Mol. Biol 48:443, by the search for similarity method of Pearson and Lipman (1988), Proc. Natl. Acad. Sci. USA 85:2444-2448, by computerized implementations of these algorithms, or by manual alignment and visual inspection. Other algorithms for determining homology or identity include, for example, the BLAST program (Basic Local Alignment Search Tool, National Center for Biological Information), BESTFIT, FASTA, and TFASTA (Wisconsin Genetics Software Package, Genetics Computer Group, Madison, WI), ALIGN, AMAS (Analysis of Multiply Aligned Sequences), AMPS (Alignment of Multiple Protein Sequence), ASSET (Aligned Segment Statistical Evaluation Tool), BANDS, BESTSCOR, BIOSCAN (Biological Sequence Comparative Analysis Node), BLIMPS (BLocks IMProved Searcher), Intervals and Points, BMB, CLUSTAL V, CLUSTAL W, CONSENSUS, LCONSENSUS, WCONSENSUS, Smith-Waterman algorithm, DARWIN, Las Vegas algorithm, FNAT (Forced Nucleotide Alignment Tool), Framealign, Framesearch, DYNAMIC, FILTER, FSAP (Fristensky Sequence Analysis Package), GAP (Global Alignment Program), GENAL, GIBBS, GenQuest, ISSC (Sensitive Sequence Comparison), LALIGN (Local Sequence Alignment), LCP (Local Content Program), MACAW (Multiple Alignment Construction and Analysis Workbench), MAP (Multiple Alignment Program), MBLKP, MBLKN, PIMA (Pattern-Induced Multi-sequence Alignment), SAGA (Sequence Alignment by Genetic Algorithm) and WHAT-IF. Such alignment programs can also be used to screen genome databases to identify polynucleotide sequences having substantially identical sequences. A number of genome databases are available, for example, a substantial portion of the human genome is available as part of the Human Genome Sequencing Project (J. Roach, http://weber.u.Washington.edu/∼roach/human_genome_progress 2.html) (Gibbs, 1995). At least twenty-one other genomes have already been sequenced, including, for example, *M. genitalium* (Fraser et al., 1995), *M. jannaschii* (Bult et al., 1996), *H. influenza* (Fleischmann et al., 1995), *E. coli* (Blattner et al., 1997), and yeast (*S. cerevisiae*) (Mewes et al., 1997), and *D*. *melanogaster* (Adams et al., 2000). Significant progress has also been made in sequencing the genomes of model organism, such as mouse, *C*. *elegans,* and *Arabadopsis sp.* Several databases containing genomic information annotated with some functional information are maintained by different organizations, and are accessible via the internet, for example, http://wwwtigr.org/tdb; http://www.genetics.wisc.edu; http://genome-www.stanford.edu/~ball; http://hiv-web.lanl.gov; http://www.ncbi.nlm.nih.gov; http://www.ebi.ac.uk; http://Pasteur.fr/other/biology; and http://www.genome.wi.mit.edu.

Examples of useful algorithms are the BLAST and the BLAST 2.0 algorithms, which are described in Altschul et al. (1977), Nuc. Acids Res. 25:3389-3402, and Altschul et al. (1990), J. Mol. Biol. 215:403-410, respectively. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al., *supra*). These initial neighborhood word hits act as seeds for initiating searches to find longer HSPs containing them. The word hits are extended in both directions along each sequence for as far as the cumulative alignment score can be increased. Cumulative scores are calculated using the parameter M (reward score for a pair of matching residues; always >0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. For nucleotide sequences, the BLASTN program uses as defaults a wordlength (W) of 11, an expectation (E) of 10, M=5, N=-4 and a comparison of both strands. For amino acid sequences, the BLASTP program uses as defaults a wordlength (W) of 3, an expectation (E) of 10, and the BLOSUM62 scoring matrix (see Henikoff and Henikoff (1989), Proc. Natl. Acad. Sci. USA 89:10915).

The BLAST algorithm also performs a statistical analysis of the similarity between two sequences (see, *e.g.,* Karlin and Altschul (1993), Proc. Natl. Acad. Sci. USA 90:5873). One measure of similarity provided by BLAST algorithm is the smallest sum probability (P(N)), which provides an indication of the probability by which a match between two nucleotide or amino acid sequences would occur by chance. For example, a nucleic acid is considered similar to a references sequence if the smallest sum probability in a comparison of the test nucleic acid to the reference nucleic acid is less than about 0.2, less than about 0.01, or less than about 0.001.

In one embodiment, protein and nucleic acid sequence homologies are evaluated using the Basic Local Alignment Search Tool ("BLAST"). In particular, five specific BLAST programs are used to perform the following task:
(1) BLASTP and BLAST3 compare an amino acid query sequence against a protein sequence database;
(2) BLASTN compares a nucleotide query sequence against a nucleotide sequence database;
(3) BLASTX compares the six-frame conceptual translation products of a query nucleotide sequence (both strands) against a protein sequence database;
(4) TBLASTN compares a query protein sequence against a nucleotide sequence database translated in all six reading frames (both strands); and
(5) TBLASTX compares the six-frame translations of a nucleotide query sequence against the six-frame translations of a nucleotide sequence database.

The BLAST programs identify homologous sequences by identifying similar segments, which are referred to herein as "high-scoring segment pairs," between a query amino or nucleic acid sequence and a test sequence which may be obtained from a protein or nucleic acid sequence database. High-scoring segment pairs are identified (*i.e*., aligned) by means of a scoring matrix, many of which are known in the art. In one example, the scoring matrix used is the BLOSUM62 matrix (Gonnet et al. (1992), Science 256:1443-1445; Henikoff and Henikoff (1993), Proteins 17:49-61). In another example, the PAM or PAM250 matrices may also be used (see, *e.g*., Schwartz and Dayhoff, eds. (1978), Matrices for Detecting Distance Relationships: Atlas of Protein Sequence and Structure, Washington: National Biomedical Research Foundation). BLAST programs are accessible through the U.S. National Library of Medicine, *e*.*g*., at www.ncbi.nlm.nih.gov.

The parameters used with the above algorithms may be adapted depending on the sequence length and degree of homology studied. In some embodiments, the parameters may be the default parameters used by the algorithms in the absence of instructions from the user.

A method for modifying small molecules may comprise contacting a polypeptide encoded by a polynucleotide described herein or enzymatically active fragments thereof with a small molecule to produce a modified small molecule. A library of modified small molecules is tested to determine if a modified small molecule is present within the library which exhibits a desired activity. A specific biocatalytic reaction which produces the modified small molecule of desired activity is identified by systematically eliminating each of the biocatalytic reactions used to produce a portion of the library, and then testing the small molecules produced in the portion of the library for the presence or absence of the modified small molecule with the desired activity. The specific biocatalytic reactions, which produce the modified small molecule of, desired activity is optionally repeated. The biocatalytic reactions are conducted with a group of biocatalysts that react with distinct structural moieties found within the structure of a small molecule, each biocatalyst is specific for one structural moiety or a group of related structural moieties; and each biocatalyst reacts with many different small molecules which contain the distinct structural moiety. Some embodiments of the use of the nitrilases are:

α-hydroxy acid - Nitrilases produce α-hydroxy acids through hydrolysis of cyanohydrins. Production of mandelic acid and derivatives thereof is an example of this. A significant application of this type involves commercial production of *(R)-*mandelic acid in both high yield and high enantioselectivity from mandelonitrile. Mandelic acid and derivatives have found broad application as intermediates and resolving agents for the production of many chiral pharmaceutical and agricultural products. Previous attempts to employ the few known nitrilases in processes using analogous substrates have been plagued by significantly lower activity, productivity, and selectivity.

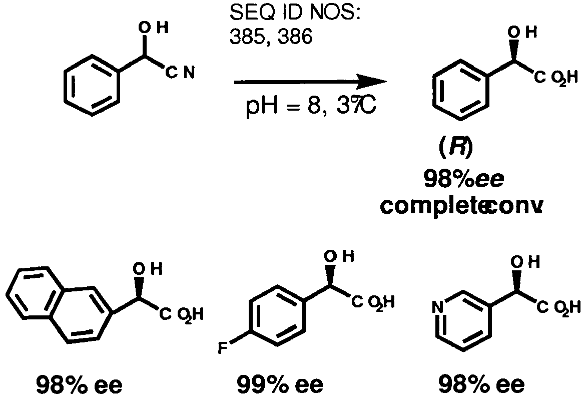

### Phenyllactic acid derivatives

An additional application is in the production of *(S)*-phenyl lactic acid derivatives in both high yield and high enantioselectivity. Phenyl lactic acid derivatives have found broad application in the production of many chiral pharmaceutical and agricultural products.

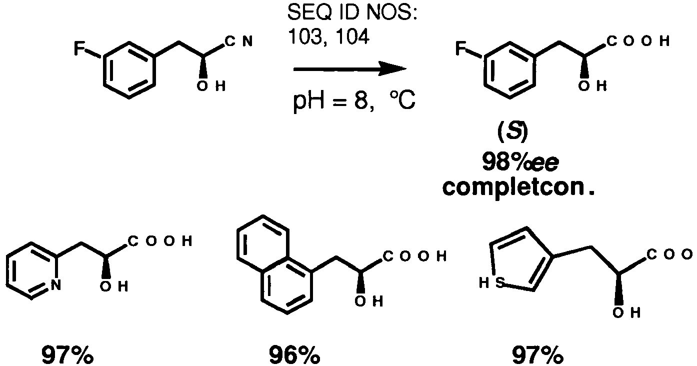

### β-hydroxy acid

With important commercial considerations, nitrilases are provided produce either enantiomer of 4-cyano-3-hydroxybutyric acid, the *(R)*-enanatiomer of which is a key intermediate in the synthesis of the blockbuster drug LIPITOR™. 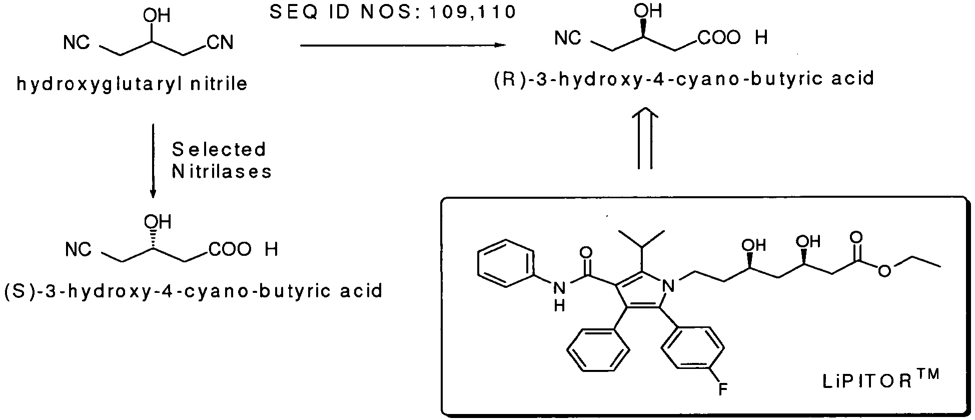 The following nitrilases are more examples of nitrilases useful in converting hydroxyglutarylnitrile to (R)-3-hydroxy-4-cyano-butyric acid: SEQ ID NOS:205, 206, SEQ ID NOS:207, 208, SEQ ID NOS:195, 196, SEQ ID NOS:43, 44, SEQ ID NOS:321, 322, and SEQ ID NOS:237, 238. The above schematic indicates that "selected nitrilases" can be used to convert hydroxyglutarylnitrile to (S)-3-hydroxy-4-cyano-butyric acid: SEQ ID NOS:107, 108, SEQ ID NOS:109, 110, SEQ ID NOS:111, 112, SEQ ID NOS:127, 128, SEQ ID NOS:129, 130, SEQ ID NOS: 133, 134, SEQ ID NOS: 113, 114, SEQ ID NOS: 145, 146, SEQ ID NOS:101, 102, SEQ ID NOS:179, 180, SEQ NOS:201, 202, SEQ ID NOS:159, 160, SEQ ID NOS: 177, 178, SEQ ID NOS:181, 182, SEQ ID NOS:183, 184, SEQ ID NOS:185, 186, SEQ ID NOS:57, 58, SEQ ID NOS: 197, 198, SEQ ID NOS:59, 60, SEQ ID NOS:67, 68, and SEQ ID NOS:359, 360.

The invention will be further described with reference to the following examples and reference examples; however, it is to be understood that the invention is not limited to such examples. Rather, in view of the present disclosure which describes the current best mode, many modifications and variations would present themselves to those of skill in the art without departing from the scope of this invention. All changes, modifications, and variations coming within the meaning and range of equivalency of the claims are to be considered within their scope.

### Example 1

### Phagemid infections

For each library to be screened for nitrilases, an infection was set up as follows: 5ml of an OD₆₀₀nm=1 resuspension of SEL700 cells and 1ml of the phagemid library to be screened were combined. The combination was incubated in a 37°C waterbath for 45 min.

Using the infection, serial dilutions were made in 10mM MgSO₄, using 10µl aliquots of the infection.

| titer of library | dilutions to make |
|---|---|
| ∼10⁵ cfu/ml | 10⁻¹ dilution |
| ∼10⁶ cfu/ml | 10⁻¹, 10⁻² dilution |
| ∼10⁷ cfu/ml | 10⁻¹, 10⁻², 10⁻³ dilution |

60µl of each of the following dilutions were deposited onto a small LB-kan⁵⁰ plate:

| titer of library | dilutions to make |
|---|---|
| ∼10⁵ cfu/ml | undiluted infection, 10⁻¹ dilution |
| ∼10⁶ cfu/ml | 10⁻¹, 10⁻² dilutions |
| ∼10⁷ cfu/ml | 10⁻², 10⁻³ dilutions |

The cells in the infection were centrifuged in a tabletop centrifuge at 4°C, 4.6k rpm, 10 min to form pellets. The supernatant was decanted from the resulting pellets. The cells were resuspended in residual liquid. All of the resuspended cells were deposited onto a single large LB-kan⁵⁰ plate. All plates were incubated at 30°C overnight.

### Example 2

### Selection Screenings

The cells of each infection plate were resuspended with ∼4mls 10mM MgSO₄. The resuspensions were placed in a tube. The remaining cells on each plate were resuspended with ∼3mls 10mM MgSO₄ and combined with the first resuspension from the same plate. The volume of each tube was brought to 12ml with 10mM MgSO₄, The tubes were vortexed vigorously. The tubes were centrifuged in a tabletop centrifuge at 4°C and 4.6k for 10min to form pellets. The supernatant was decanted from each resuspension. The washed cells in each tube were resuspended with 10ml 10mM MgSO₄. The resuspensions from each library were stored at 4°C until the selection cultures were ready to be set up.

For each resuspension, selection cultures were set up using the following process:
1) The nitrilase selection medium was prepared, using: 1XM9 medium with 0.2% glucose, no nitrogen and 50µg/ml kanamycin (for pBK phagemid libraries only; use ampicillin for pBS libraries).
2) 5ml of the medium was aliquoted into a 50ml screw top conical tube.
3) 25µl of the stored resuspension was added to the tube.
4) 5µl of adiponitrile was added to the tube, to bring the final concentration to 8.8mM. Additional nitrile substrates may be used, in place of adiponitrile.
5) The resulting combination was cultured at 30°C.

Steps 1-5 were repeated for each nitrile substrate.

### Example 3

### Isolation of a positive nitrilase clone from selection cultures

Ten (10) µl of selection culture with growth was streaked out onto a small LB-kan⁵⁰ plate and allowed to grow for 2 nights at 30°C. Five isolated cfu were picked and each was grown in 2ml nitrilase selection medium at 30°C. Each culture was monitored (where growth indicates positive cfu was picked), and was removed when monitoring indicated that it was in a stationary phase of growth. One (1) ml of culture was used to do a plasmid preparation and was eluted with 40µl elution buffer. Five to eight (5-8) µl DNA was cut with Pst 1/Xho I or Sac I/Kpn I restriction enzymes to remove insert from vector. A restriction fragment length polymorphism (RFLP) determination was carried out to identify the size of the insert. The insert was sequenced.

### Example 4

### Screening and Characterization of Nitrilases

Nitrilases were screened against target substrates. Of those showing hydrolytic activity in a primary screen, enzymes with enantioselectivities above 20% enantiomeric excess (ee) were selected for further characterization. Those enzymes were selected based on: 1) having activity against one of the substrates of interest and 2) exhibition of greater than 35% ee (enantiomeric excess). The results of this screening process are set forth in Table 1 above. The products used for screening were: D-Phenylglycine, L-Phenyllactic acid, (R) 2-chloromandelic acid, (*S*)-Cyclohexylmandelic acid, L-2-methylphenylglycine, (S)-2-amino-6-hydroxy hexanoic acid, and 4-methyl-L-leucine.

### Screening of nitrilases against target substrate D-Phenylglycine,

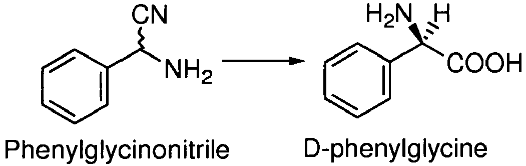

The hydrolysis of phenylglycinonitrile was performed. Some of these enzymes showed an *ee* higher than 20% and those were selected for preliminary characterization.

Based on the preliminary characterization experiments, a number of putative hits were identified on phenylglycinonitrile and a large amount of data was accumulated on these enzymes. The data revealed many common properties: the majority of the enzymes had pH optima for activity at pH 7 and, in general, the enantioselectivity was enhanced at the lower pH values. The enzymes were found to be more active at higher temperature, particularly 38°C, although this temperature often resulted in lower enantioselectivities. The use of water-miscible co-solvents in the reaction was shown to be a practical option. The inclusion of 10-25% methanol (v/v) in the enzyme reactions did not substantially affect enzyme activity and in many cases, led to an increase in enantioselectivity. The use of biphasic systems has also shown some promise, with the enzymes maintaining their level of activity with the addition of up to 70% (v/v) of hexane and, in some cases, toluene. The use of ethyl acetate in the biphasic systems, however, led to lower activity.

Of the enzymes identified active on phenylglycinonitrile, the enantioselectivity of several enzymes was shown to remain above the success criterion of 35% *ee.* The preliminary characterization data indicated that some of the enzymes exhibited high enantioselectivities for D-phenylglycine, with corresponding conversion to product of 40-60%. Further investigation suggested that the rate of activity of some of these enzymes was faster than the rate of racemization of the substrate. Reducing the concentration of enzyme led to improved enantioselectivity; therefore, it appears that some benefit could be gained by control of the relative rates of the chemical racemization and the enzyme activity.

### Screening of nitrilases against target substrate (R)-2-chloromandelic acid

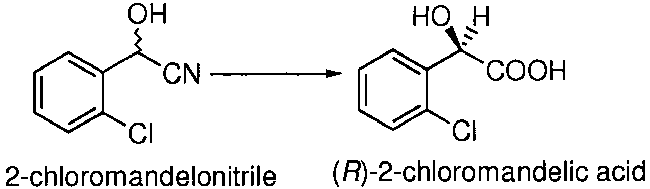

Enzymes were identified which showed activity on 2-chloromandelonitrile. A high degree of overlap existed between the enzymes which were active on 2-chloromandelonitrile and phenylglycinonitrile. Many of these enzymes also formed a distinct sequence family.

Higher temperatures and neutral pH appeared to lead to the highest activity for the active enzymes. For the majority of the nitrilases, the enantioselectivity also increased at higher temperatures, particularly 38°C. The enzymes retained their activity in the presence of up to 25% methanol or 10% isopropanol; in many of these cases, the enantioselectivity was also enhanced. Activity in biphasic systems was largely comparable to aqueous conditions, particularly with hexane as the nonaqueous phase; varying tolerances to toluene were observed between the different nitrilases.

**Table 4. Examples of enzymes for enantioselective hydrolysis of 2-chloromandelonitrile.**

| **SEQ ID NOS:** | **Conversion To Product** | **Time For Highest Conversion (h)** | **ee (%)** | **Turnover** | **Specific Activity (µmol Product/mg Nitrilase/h)** | **Conditions** |
|---|---|---|---|---|---|---|
| | | | | **(g Product/kg Nitrilase/h)** | | |
| 385, 386 | 22% | 8 | 92 | 1014 | 88 | pH 7, 38°C; 20% MeOH |
| 169, 170 | 36% | 1 | 92 | 29278 | 39 | pH 7, 38°C; 20% MeOH |
| 185, 186 | 44% | 1 | 79 | 14559 | 99 | pH 7, 38°C |
| 47, 48 | 43% | 5 | 65 | 2475 | 20 | pH 7, 38°C; 10% MeOH |
| 197, 198 | >95% | 1 | 87 | 45564 | 149 | pH 6, 38°C; 10% MeOH |
| 187, 188 | 53% | 1 | 82 | 14100 | 80 | pH 7, 38°C; 10% MeOH |
| 217, 218 | >95% | 2 | 85 | 19773 | 185 | pH 7, 38°C; 10% MeOH |
| 55, 56 | >95% | 1 | 93 | 52951 | 329 | pH 8, 38°C; 10% MeOH |
| 167, 168 | >95% | 2 | 88 | 14895 | 100 | pH 8, 38°C; 10% MeOH |
| 15, 16 | 55% | 1 | 81 | 25825 | 308 | pH 5, 38°C; 10% MeOH |

**Table 4A. Summary of optimal conditions determined from characterization experiments for enantioselective hydrolysis of 2-chloromandelonitrile.**

| **SEQ ID NOS:** | **Optimum pH** | **Optimum Temp °C** | **Solvent Tolerance** |
|---|---|---|---|
| 385, 386 | 7 | 38 | 25% MeOH |
| 169, 170 | 5 | 38 | 25% MeOH, 10% IPA |
| 185, 186 | 7 | 38 | 25% MeOH, 10% IPA |
| 47, 48 | 7 | 38 | 10% MeOH |
| 197, 198 | 6 | 55 | 25% MeOH, 10% IPA |
| 187, 188 | 7 | 38 | 10% MeOH; 40% IPA |
| 217, 218 | 7 | 38 | 25% MeOH, 10% IPA, 70% hexane, 40% toluene |
| 55, 56 | 7 | 38 | 10% MeOH, IPA, 70% hexane |
| 167, 168 | 9 | 38 | 10% MeOH, IPA, 70% hexane |
| 15, 16 | 7 | 38 | 25% MeOH, 10% IPA, 70% hexane. 40% toluene |

### Screening of nitrilases against target substrate (S)-phenyllactic acid:

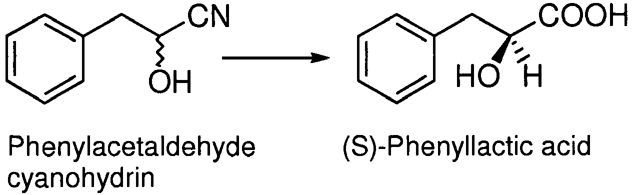

Many of the nitrilases tested were active on phenaylacetaldehyde cyanohydrin. Many of these enzymes were part of two related sequence families and were distinct from those enzymes that were active on phenylglycinonitrile and chloromandelonitrile.

The pH optima of the enzymes was generally above pH 7 (*i.e.* pH 8 or 9), with higher enantioselectivities being exhibited at these levels. Most of the enzymes showed superior activity at higher temperature, particularly 38°C. The effect of temperature on the enantioselectivities of the enzymes varied; in most cases, this property was slightly lower at higher temperatures. While the enzymes were tolerant towards the addition of co-solvents, particularly 10% (v/v) methanol, no advantage in activity or enantioselectivity was gained by such additions. The use of a biphasic system was again shown to be feasible.

**Table 5. Summary of optimal conditions determined from characterization experiments for enantioselective hydrolysis of phenylacetaldehyde cyanohydrin**

| **SEQ ID NOS:** | **Optimum pH** | **Optimum Temp °C** | **Solvent Tolerance** |
|---|---|---|---|
| 103, 104 | 7 | 55 | 10% MeOH, IPA |
| 99, 100 | 8 | 38 | 10% MeOH, 70% hexane, toluene |
| 183, 184 | 9 | 38 | 10% MeOH, IPA, 70% toluene, hexane |
| 173, 174 | 5 | 38-55 | 25% MeOH, IPA, 70% hexane, toluene |
| 213, 214 | 7 | 38 | 10% MeOH, 25% IPA, 70% hexane, toluene |
| 61, 62 | 7 | 38 | 10% MeOH, 70% hexane, toluene |
| 205, 206 | 8 | 38-55 | 10% MeOH, IPA, 40% hexane, toluene |
| 207, 208 | 8 | 38 | 10% MeOH, 70% hexane |
| 309, 210 | 8 | 38 | 10% MeOH, 40% hexane, toluene |
| 195, 196 | 8 | 38 | 10% MeOH, 40% hexane, toluene |
| 43, 44 | 9 | 38 | 10% MeOH, 40% hexane |
| 161, 162 | 9 | 38 | 25% MeOH, IPA, 10% hexane, toluene |
| 175, 176 | 6 | 38-55 | 10% MeOH, IPA, 40% hexane |
| 293, 294 | 6 | 38 | 10% MeOH, IPA, 40% hexane |

**Table 6. Summary of hit enzymes for enantioselective hydrolysis of phenylacetaldehyde cyanohydrin**

| **SEQ ID NOS:** | **Conversion To Product (%)** | **Time For Highest Conversion (h)** | **ee (%)** | **Turnover (g Product/kg Nitrilase/h)** | **Specific Activity (µmol Product/mg Nitrilase/h)** | **Experimental Conditions¹** |
|---|---|---|---|---|---|---|
| 103, 104 | 77 | 3 | 94 | 2339* | 14* | pH 7, 38 °C |
| 99, 100 | 90 | 4 | 82 | 19787 | 119 | pH 8, 38°C |
| 183, 184 | 49 | 1 | 57 | 28153 | 169 | pH 9, 38°C |
| 173, 174 | 20 | 2 | 83 | 3200 | 27 | pH 5, 38 °C |
| 213, 214 | 70 | 6 | 86 | 4372 | 26 | pH 7, 38 °C |
| 61, 62 | 56 | 5 | 80 | 5500 | 33 | pH 7, 38 °C |
| 205, 206 | 90 | 6 | 73 | 4458 | 27 | pH 8, 38°C |
| 207, 208 | 78 | 4 | 81 | 9190 | 55 | pH 8, 38°C |
| 209, 210 | 98 | 4 | 75 | 8343 | 50 | pH 8, 38°C |
| 195, 196 | 89 | 4 | 89 | 6874 | 41 | pH 8, 38°C |
| 43, 44 | 40 | 5 | 84 | 3879 | 23 | pH 9, 38°C |
| 161, 162 | >95 | 2 | 39 | 16430 | 98 | pH 9, 38°C |
| 175, 176 | 87 | 8 | 45 | 5065 | 30 | pH 6, 38°C |
| 293, 294 | >95 | 8 | 65 | 7725 | 46 | pH 6, 38°C |

| | | | | | | |
|---|---|---|---|---|---|---|
| ¹. Experiments were carried out using 25 mM substrate, at the pH and temperature conditions noted. *. Turnover in (g product/kg protein/h) and specific activity (:mol product/mg protein/h) | | | | | | |

### Screening of nitrilases against target substrate L-2-methylphenylglycine

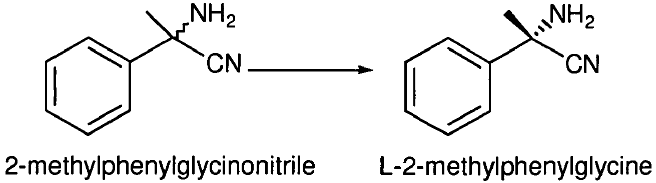

Nitrilases have shown activity on this substrate and preferentially yielded the D-2-methylphenylglycine, rather than the required L-2-methylphenylglcyine.

**Table 7. Summary of activity and enantioselectivity for SEQ ID NOS:189, 190 on 2-methylphenylglycinonitrile**

| **SEQ ID NOS:** | **Conversion To Product (%)** | **Time For Highest Conversion (h)** | **ee (%) for D-enatiomer** | **Turnover (g Product/kg Nitrilase/h)** | **Specific Activity (µmol Product/mg Nitrilase/h)** | **Experimental Conditions¹** |
|---|---|---|---|---|---|---|
| 189, 190 | 50% | 4.5 h | 45 | 2426 | 13 | pH 7 38 °C, 10% MeOH |

### Screening of nitrilases against target substrate L-hydroxynorleucine ((S)-2-amino-6-hydroxy hexanoic acid)

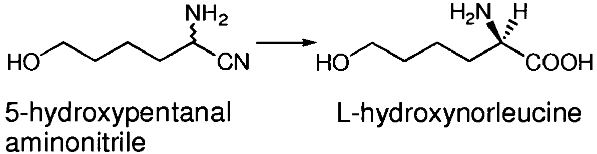

A number of nitrilases, which showed activity on 2-amino-6-hydroxy hexanenitrile, were isolated. All of these enzymes showed enantioselectivity towards the L-isomer of the product.

As shown in Table 8, the enzymes all showed higher enantioselectivities at higher pH and appeared to more susceptible to the addition of solvents than the other nitrilases tested. Although activity was detected in the presence of organic solvents, it was generally lower than that of the aqueous control. Once again, the activity of the enzymes was negatively affected by the acid product and aldehyde starting material.

**Table 8. Summary of optimal conditions determined from characterization experiments for enantioselective hydrolysis of 2-amino-6-hydroxy hexanenitrile.**

| **SEQ ID NOS:** | **Optimum pH** | **Optimum Temp °C** | **Solvent** |
|---|---|---|---|
| 217,218 | 9 | 38 | 10% MeOH |
| 55, 56 | 9 | 38 | None |
| 187, 188 | 9 | 38 | 10% MeOH |
| 167, 168 | 9 | 38 | None |
| 221, 222 | 9 | 38 | |

A range of hydrolytic activities was observed among the confirmed hit enzymes for 2-amino-6-hydroxy hexanenitrile.

**Table 9. Summary of hit enzymes for enantioselective hydrolysis of 2-amino-6-hydroxy hexanenitrile.**

| **SEQ ID NOS:** | **Conversion To Product (%)** | **Time For Highest Conversion (h)** | **ee (%)** | **Turnover (g Product/kg Nitrilase/h)** | **Specific Activity (µmol Product/mg Nitrilase/h))** | **Experimental Conditions¹** |
|---|---|---|---|---|---|---|
| 217, 218 | >95 | 1.5 | 52 | 33712 | 229 | pH 9, 38 °C |
| 55, 56 | 80 | 3 | 55 | 11221 | 76 | pH 9, 38 °C |
| 187, 188 | 50 | 6 | 60 | 1238 | 4 | pH 9, 38 °C, 10% MeOH |
| 167, 168 | 35 | 6 | 54 | 1684 | 11 | pH 9, 38 °C |
| 221, 222 | 80 | 3 | 55 | 9901 | 148 | pH 9, 38 °C |

### Screening of nitrilases against target substrate 4-methyl-D-leucine and 4-methyl-L-leucine

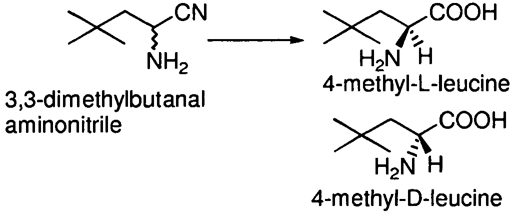

Hydrolysis of 2-amino-4,4-dimethyl pentanenitrile was performed by several of the nitrilases. Of these, some were shown to hydrolyse the nitrile to the L-isomer of the corresponding acid and were selected for further characterization.

**Table 10. Summary of hit enzymes for enantioselective hydrolysis of 2-amino-4,4-dimethyl pentanenitrile**

| **SEQ ID NOS:** | **Conversion To Product (%)** | **Time For Highest Conversion (h)** | **ee (%)** | **Turnover (g Product/kg Nitrilase/h)** | **Specific Activity (µmol Product/mg Nitrilase/h)** | **Conditions¹** |
|---|---|---|---|---|---|---|
| 103, 104 | 30 | 0.5 | 91 | 12489 | 36 | pH 7, room temp |
| 59, 60 | 30 | 0.5 | >99 | 33806 | 233 | pH 7, room temp |
| 221,222 | 32 | 7.5 | 79 | 1098 | 7 | pH 6, 38 °C |

**Table 11. Summary of optimal conditions determined from characterization experiments for enantioselective hydrolysis of 2-amino-4,4-dimethyl pentanenitrile**

| **SEQ ID NOS:** | **Optimum pH** | **Optimum Temp °C** | **Solvent Tolerance** |
|---|---|---|---|
| 103, 104 | 7 | 23 | 25% MeOH, 10% IPA |
| 59, 60 | 8 | 23 | 25% MeOH |
| 221, 222 | 6 | 38 | 25% MeOH, 10% IPA |

### Screening of nitrilases against target substrate (S)-cyclohexylmandelic acid

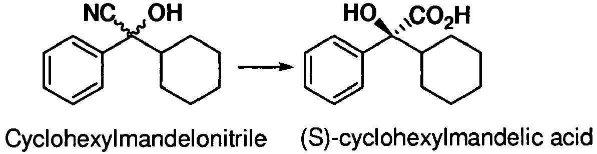

### Screening of nitrilases against target substrate Mandelonitrile

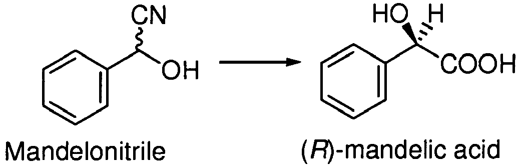

The nitrilase collection was also screened on mandelonitrile. The nitrilases actively hydrolyzed both phenylglycinonitrile and chloromandelonitrile.

### Enzymatic assay for determination of enantioselectivity

In the design of a spectroscopic system for determination of the chiral α-hydroxy acids and α-amino acids, an enzyme based assay which permits the detection of product formation and enantioselectivity was developed and used.

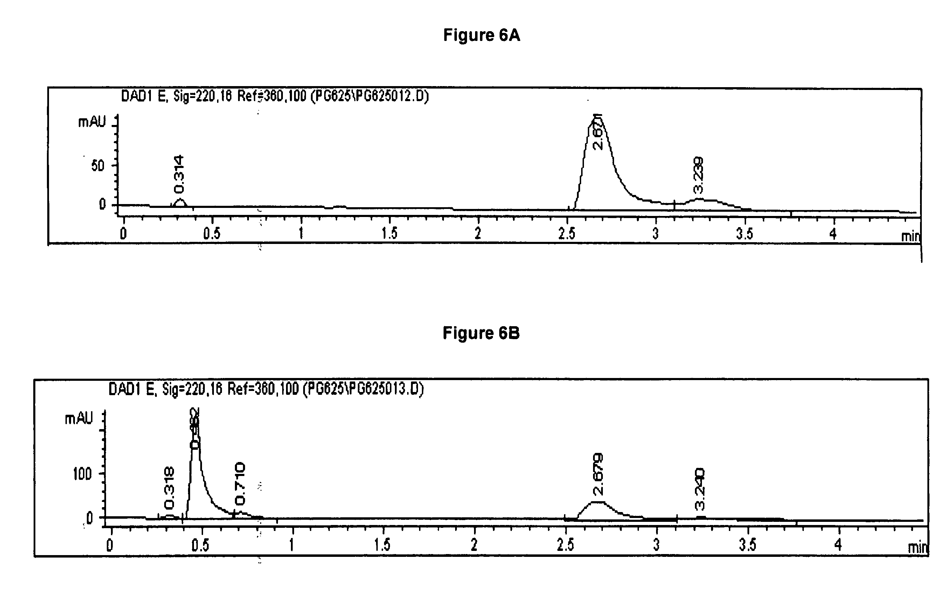

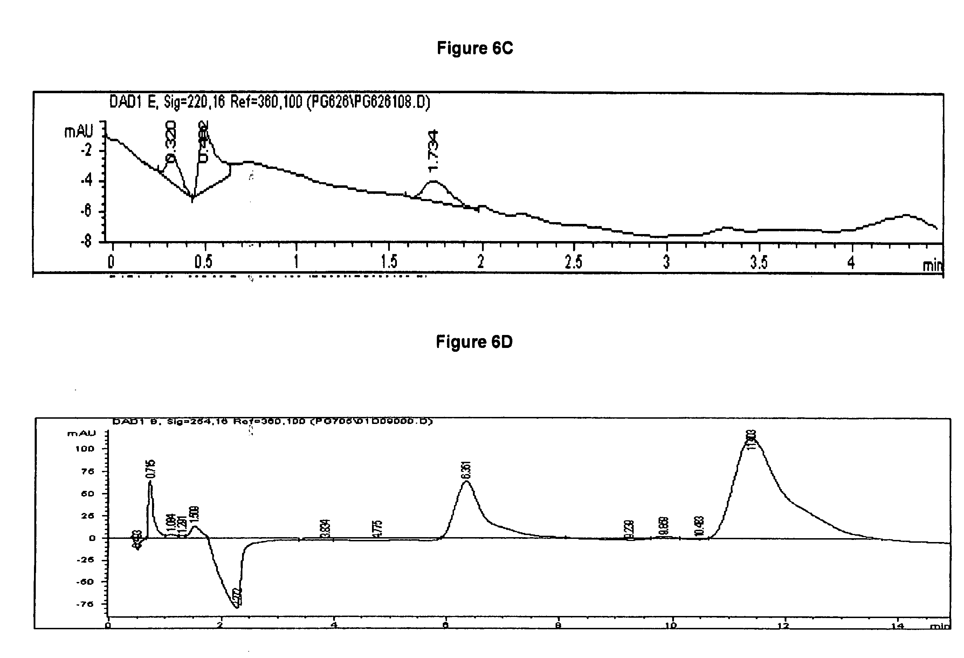

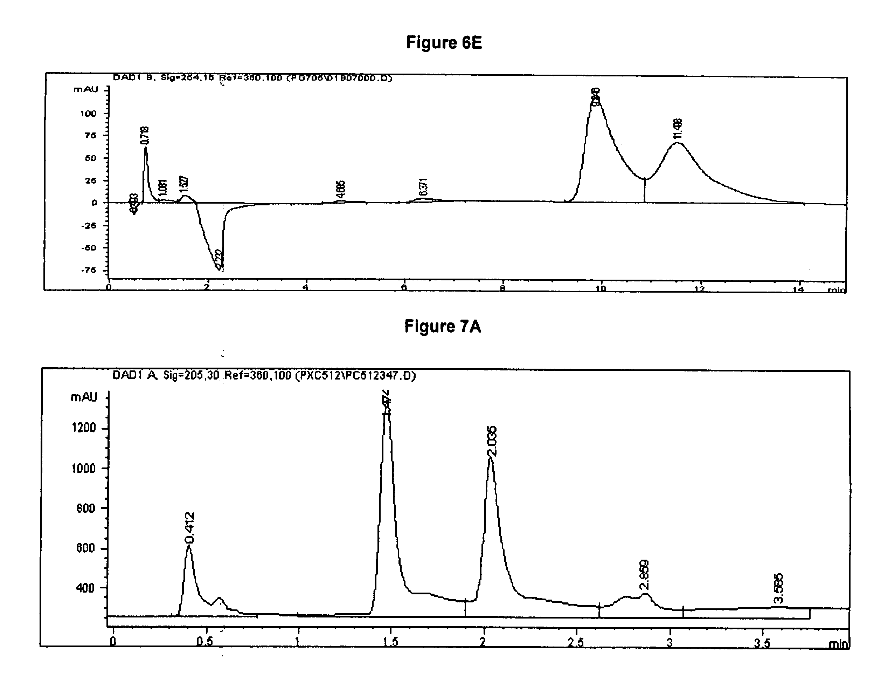

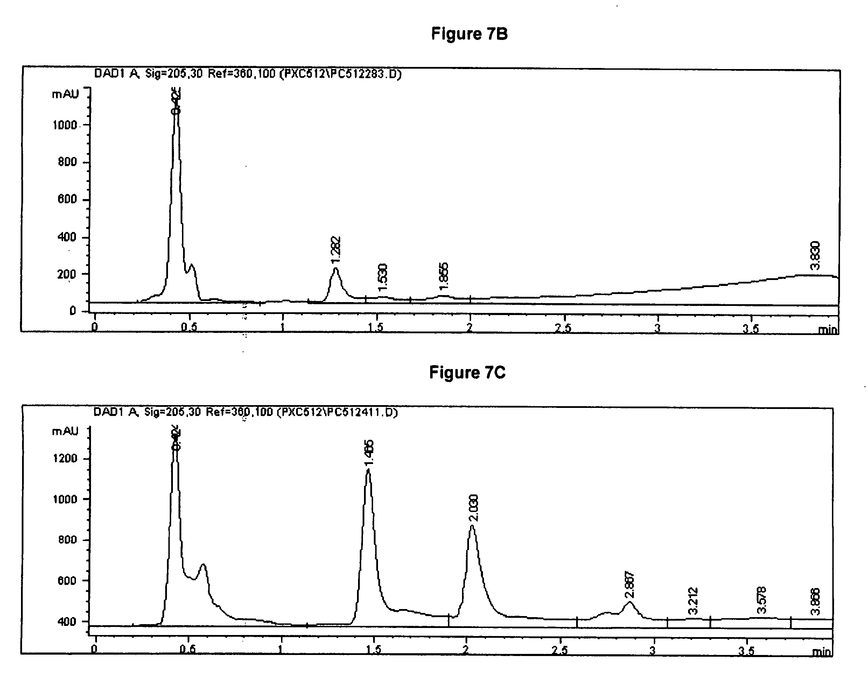

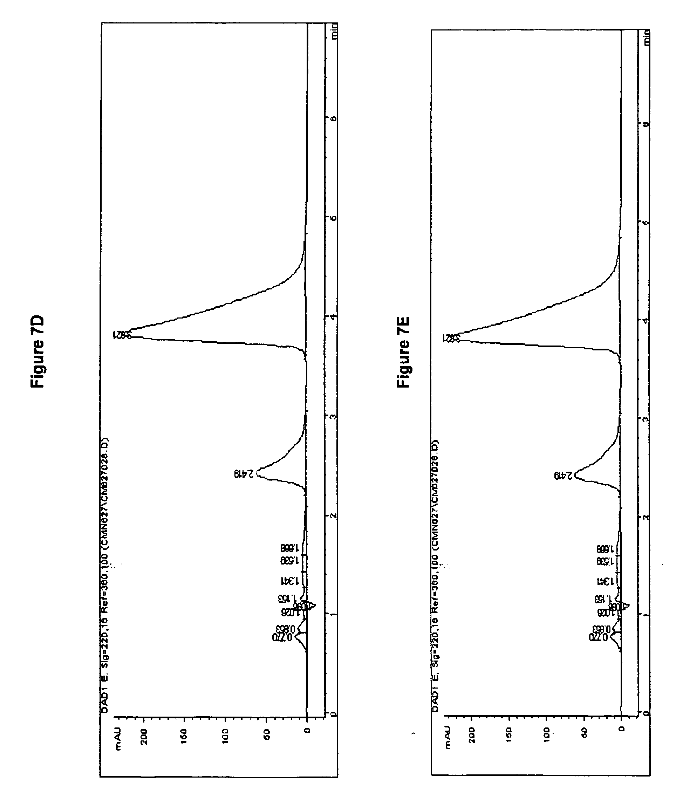

Spectroscopic systems for the detection of α-hydroxy- and for α-amino- acids based on lactate dehydrogenase (L-LDH & D-LDH) and on amino acid oxidase (L-AA Oxid & D-AA Oxid) are described in Figures 6 and 7. These enzymes were chosen because they are reported to have reasonably broad substrate ranges while still retaining near absolute enantiospecificity.

The overall feasibility of this system has been established (Table 12). Neither the parent hydroxynitrile nor the aminonitrile is metabolized by the secondary or detection enzyme and thus starting material does not interfere. Cell lysate which is not heat treated results in background activity for the LDH system; however, heat inactivation eliminates the background activity. Cell lysate does not appear to interfere in the AA Oxidase assay. One concern is the inactivation of the AA Oxidase, which utilizes a FMN co-factor, by residual cyanide. However, the control studies indicated that at 2 mM PGN (which could release up to 2 mM HCN) inactivation is not a problem. This assay is suitable for automation of 384 well (or possibly greater density) microtiter plates.

**Table 12: Summary of Identification of Secondary Enzyme to Chiral Detection of Acid Product.**

| **SUBSTRATE** | **ENZYME WITH SUITABLE ACTIVITY FOUND FROM COMMERCIAL SOURCE** |
|---|---|
| **Hydroxy Acid Products:** | |
| L-lactic acid | YES |
| D-lactic acid | YES |
| L-phenyl lactic acid | YES |
| D-phenyl lactic acid | YES |
| S-cyclohexylmandelic acid¹ | *Not applicable* |
| R-cyclohexylmandelic acid¹ | *Not applicable* |

| **Amino Acid Products:** | |
|---|---|
| 4-methyl-L-leucine | YES |
| 4-methyl-L/D-leucine | YES (D-unknown) |
| D-phenylalanine | YES |
| R-phenylglycine | YES |
| L-homophenyllactic acid | YES |
| D-homophenyllactic acid | YES |
| L-homophenylalanine | YES |
| D-homophenylalanine | YES |
| (S)-2-amino-6-hydroxy hexanoic acid | YES |
| (R/S)-2-amino-6-hydroxy hexanoic acid | YES (D-unknown) |
| L-methylphenylglycine¹ | **1. *Not Applicable*** |
| D-methylphenylglycine¹ | *Not Applicable* |

| | |
|---|---|
| ¹: The assay will not be applicable to cyclohexylmandelic acid and 2-methylphenylglycine, as tertiary alcohols are not amenable to this particular oxidation | |

### Example 5

### Standard assay conditions

The following solutions were prepared:
- Substrate stock solution: 50 mM of the aminonitrile substrate in 0.1 M phosphate buffer (pH 7) or 50 mM of the cyanohydrin substrate in 0.1 M Na Acetate buffer (pH 5)
- Enzyme stock solution: 3.33 ml of 0.1 M phosphate buffer (pH 7) to each vial of 20 mg of lyophilized cell lysate (final concentration 6 mg protein/ml)

### Procedure:

- Add 100 µl of the 50 mM substrate solution to the appropriate number of wells of a 96-well plate
- Add 80 µl of buffer to each well
- Add 20 µl of enzyme solution to each well
- Blank controls were set up by substitution of 20 µl of buffer for the enzyme solution
- Negative controls consisting of 20 µl of enzyme solution in 180 µl of buffer were also included in many of the experiments. Once it had been established that the cell lysate did not interfere with the detection of the products, these controls were not included.

### Sampling of reactions:

- The reactions were sampled by removing an aliquot from each well (15-50 µl) and diluting the samples as follows:
- Samples for non-chiral HPLC analysis:
- Phenylglycine, 2-chloromandelic acid and phenyllactic acid: initially, the samples were diluted 2-fold with water and a further 2-fold with methanol or acetonitrile (final dilution: 4-fold). It was found that an 8-fold dilution of these samples led to improved chromatographic separation
- (S)-2-amino-6-hydroxy hexanoic acid, 4-methylleucine, *t*-leucine, 2-methylphenylglycine and cyclohexylmandelic acid: samples were diluted 1:1 with methanol or acetonitrile. The choice of solvent was based on the solvent used in the HPLC analysis method.
- Samples for chiral HPLC analysis:
- Phenylglycine, 2-chloromandelic acid and phenyllactic acid: as described above for the non-chiral analyses, the samples for chiral analyses were initially diluted 2-fold and in the later stages of the project, at 4-fold.
- (S)-2-amino-6-hydroxy hexanoic acid, 4-methylleucine, *t*-leucine, 2-methylphenylglycine: samples were diluted 1:1 with methanol or acetonitrile
- For each experiment, a standard curve of the product was included in the HPLC run. The curve was plotted on an X-Y axis and the concentration of product in the samples calculated from the slope of these curves.
- For the preliminary characterization experiments, samples were taken such that the activity of the enzymes was in the linear phase; this was performed so that differences in the effects of the parameters on the rate of reaction, rather than the complete conversion, could be determined. The sampling times are denoted in the tables included in the text.
- The samples were analyzed by HPLC, using the methods outlined in Table 20 and 21.

### Example 6

### Determination of the Effect of pH on enzyme activity and enantioselectivity

The effect of pH on the enzyme activity and enantioselectivity was studied by performance of the standard assay in a range of different buffers:
0.1 M Citrate Phosphate pH 5
0.1 M Citrate Phosphate pH 6
0.1 M Sodium Phosphate pH 7
0.1 M Tris-HCl pH 8
M Tris-HCl pH 9

The samples were analyzed by non-chiral and chiral HPLC methods and examples of the results are presented in Tables 5, 8 and 11 herein.

### Example 7

### Determination of the Effect of temperature on enzyme activity and enantioselectivity

The effect of temperature on the activity and enantioselectivity was investigated by performing the standard assay at room temperature, 38°C and 55°C. The samples were analyzed by non-chiral and chiral HPLC methods and examples of the results are given in Tables 5, 8 and 11 herein.

### Example 8

### Determination of the Effect of solvents on enzyme activity and enantioselectivity

The enzyme reactions were performed in the presence of cosolvents and as biphasic systems, in order to investigate the effect of water-miscible and water-immiscible solvents on the enzymes. In the presence of cosolvents, the reactions were run under standard conditions, with substitution of the buffer with methanol or isopropanol. The final concentrations of solvent in the reactions was 0, 10, 25 and 40% (v/v).

The biphasic reactions were also carried out under standard conditions, with a layer of water-immiscible organic solvent forming the nonaqueous phase. The solvent was added at the following levels: 0%, 10%, 40% and 70% (v/v) of the aqueous phase. The samples from these reactions were evaporated by centrifugation under vacuum and redissolved in a 50:50 mixture of methanol or acetonitrile and water. The samples were analyzed by non-chiral and chiral HPLC methods.

### Example 9

### Determination of the Effect of process components on

### enzyme activity and enantioselectivity

### Activity

The effect of the process components on the activity of the enzymes was established by addition of the individual components to the enzymatic reaction. These components included the starting materials for the nitrile synthesis, aldehyde, cyanide and ammonium, as well as triethylamine, which is added in catalytic amounts to the nitrile synthesis reaction. The concentrations of the reactants were selected with possible process conditions in mind and were adapted to the levels of reactants used in the enzyme assays. In some cases, the solubility of the aldehydes and products was relatively low; in these cases, the highest level of solubility was added to the reactions as the highest level and 10% of this level as the lower value.

The enzymatic reactions were carried out under standard conditions, with addition of one or more of the following components: benzaldehyde, phenylglycine, phenylacetaldehyde, phenyllactic acid, 2-chlorobenzaldehyde, 2-chloromandelic acid, 5-hydroxypentanal, (S)-2-amino-6-hydroxy hexanoic acid, 4-methylleucine, KCN, Triethylamine, NH₄Cl. Control reactions were performed under standard conditions, with no additive. The samples were analyzed by non-chiral HPLC.

### Stability

The stability of the enzymes to process conditions was monitored by incubation of the enzymes in the presence of the individual reaction components for predetermined time periods, prior to assay of the enzyme activity under standard conditions. In these experiments, the enzymes were incubated at a concentration of 1.2 mg protein/ml in the presence of each of the following reaction components: methanol, benzaldehyde, phenylglycine, phenylacetaldehyde, phenyllactic acid, 2-chlorobenzaldehyde, 2-chloromandelic acid, 5-hydroxypentanal, (S)-2-amino-6-hydroxy hexanoic acid, KCN, NH₄Cl.

### Assay conditions:

At 0, 2, 6 and 24 hours of incubation in the particular additive, 50 µl of the enzyme solution was removed, 50 µl of a 50 mM substrate stock solution added and the enzyme activity assayed under standard conditions. After substrate addition, the reactions were sampled at the following times: Phenylglycinonitrile: 10 mins; Phenylacetaldehyde cyanohydrin: 1 hour; 2-chloromandelonitrile: 2 hours. Control reactions were performed by incubation of the enzyme in buffer only. The samples were analyzed using non-chiral HPLC methods.

### Example 10

### Confirmation of putative hit enzymes

Following the preliminary characterization experiments, the enzymes which were identified as putative hits were assayed under the optimal conditions determined, in order to evaluate their performance, especially in terms of enantioselectivity, when higher conversions were attained. The enzymes were assayed with 25 mM substrate, under the conditions of pH and temperature noted in the tables included in the text. A standard concentration of 0.6 mg/ml protein was used for each of the enzymes, unless otherwise stated.

### Example 11

### Selected examples of chromatograms from enzyme reactions

In this section, representative examples of chromatograms for each substrate and product combination will be shown, together with a discussion of some of the challenges encountered with the methods and how they were addressed.

### D-Phenylglycine

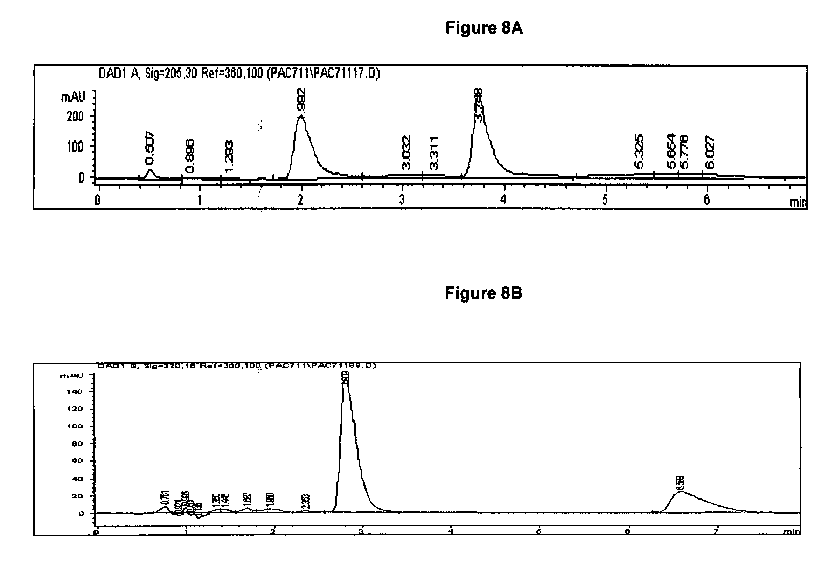

Non-chiral analysis showing the substrate peak eluting at 2.6 min and 3.2 min. See Figures 8A-8E. The two peaks were present in all samples containing higher concentrations of the nitrile; the second peak is thought to be a product associated with the nitrile; it decreased with time and was no longer present once complete conversion to the product had taken place. The chromatogram shown in Figure 8A is a blank control, containing only nitrile and buffer; the samples were all diluted with water and solvent as explained in section 1 above. This was repeated for all samples discussed below. An enzymatic reaction sample is shown in the chromatogram in Figure 8B , with the product eluting at 0.4 min.

Of note in these chromatograms is the small solvent front peak eluting at 0.3 min. Further representation of this peak is given in the chromatogram shown in Figure 8C, in which a negative control consisting of cell lysate in buffer, was run. A very small peak coeluted with the product at 0.4 min. In the initial phase of the project, this peak was regarded as problematic, although the appropriate controls were run with each experiment for in order to maintain accuracy. In these experiments, the peak area resulting from the cell lysate, although it was relatively small, was subtracted from the peak areas of the product in the enzymatic reactions. Improvement of this analysis was obtained by further dilution of the samples and the use of lower injection volumes on the HPLC. Following the implementation of these improvements, interference by this peak was shown to be minimal, as shown in the chromatogram illustrated in Fig. 6C.

The chiral analysis of phenylglycine is shown in chromatogram in Fig. 6D with the L-enantiomer eluting at 6 min and the D-enantiomer at 11 min. Good resolution between the two isomers was obtained. However, the column used was very sensitive and the characteristics of the column appeared to change over time, resulting in changes in the elution times of the acids. While this was easily detected by the use of the proper controls and standards, a greater problem existed in the coelution of the nitrile peak with the D-enantiomer (chromatogram shown in Fig. 6E). The cause of this coelution was unclear; however, it was easily detected by the use of appropriate standards; in addition, the UV spectrum of the acid was very distinctive, making the use of this tool effective in detecting the coelution. The problem was also easily resolved by adjusting the methanol content in the mobile phase.

### (R)-2-chloromandelic acid

The HPLC analysis of chloromandelic acid and chloromandelonitrile offered many of the challenges associated with the analysis of the phenylglycine samples. From the chromatogram shown in Fig. 7A, which contains only chloromandelonitrile in buffer, it is evident that a peak eluted at the same time as the product in the chromatogram shown in Fig. 7B, which represents a chloromandelic acid standard. The contribution of the cell lysate to this peak was found to be small; it would appear that the greatest contribution to this peak was from the chloromandelonitrile, either from a breakdown product or a contaminant in the nitrile preparation. The peak area remained constant throughout each experiment and, using the appropriate controls, it was found that subtraction of the peak area from that of the product yielded sufficient accuracy. Many attempts were made to change the HPLC conditions so that the product peak eluted at a later time; however, these attempts were not successful. Chromatogram shown in Fig. 7C illustrates the appearance of product and the reduction of the substrate peaks.

The chiral analysis of chloromandelic acid was almost problem-free. The elution of a small peak at the same time as the (S)-enantiomer presented some concern (the peak at 2.4 min in chromatogram shown in Fig. 7D). However, once it was established that this peak was present in all the samples at the same level, including the blank control, and that it had a different UV spectrum to that of the chloromandelic acid peak, it was not regarded as a problem. Consequently, it was subtracted from the peak eluting at 2.4 min in each sample. The (R)-enantiomer eluted at 3 minutes.

### (S)-phenyllactic acid

The analysis of phenyllactic acid was initially plagued with the same problems discussed for phenylglycine and 2-chloromandelic acid. However, in this case, adjustment of the solvent concentration in the nonchiral HPLC method led to a shift in the retention time of the acid, so that it no longer coeluted with the cell lysate peak. Following this, no problems were encountered with either the nonchiral or chiral methods. Representative nonchiral chromatograms of the product (1.9 min) and cyanohydrin substrate (3.7 min) are shown in Fig. 8A, while the chiral analysis of the acid is shown in Fig. 8B, with the L-enantiomer eluting at 2 min and the opposite enantiomer at 6 min.

### L-2-methylphenylglycine

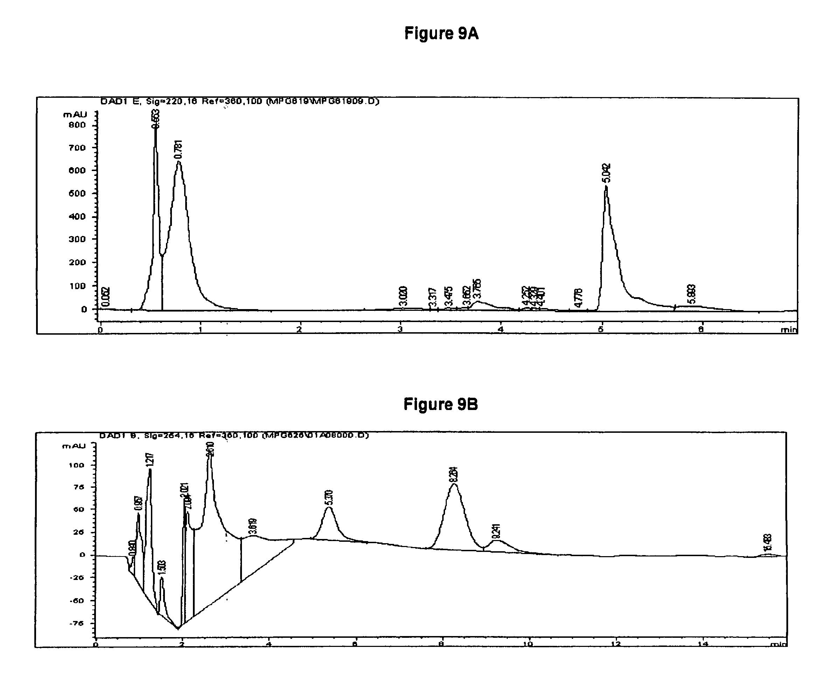

The analysis of methylphenylglycine was unproblematic, although the nonchiral method did not provide baseline separation between a cell lysate peak and the product peak, as shown in the chromatogram illustrated in Fig. 9A. The amino acid standard for this method was provided in the final stages of the project, thus minimizing the time for method development. In the chromatogram shown in Fig. 9A the amino acid elutes at 0.7 min and the aminonitrile at 5.0 min. Sufficient separation between the two initial peaks was obtained to allow the calculation of approximate conversion to product.

The chiral analysis of this compound provided good separation between the two enantiomers, as shown in the chromatogram illustrated in Fig. 9B. The L-enantiomer elutes at 5 min and the D-enantiomer at 8 min.

### L-tert-leucine

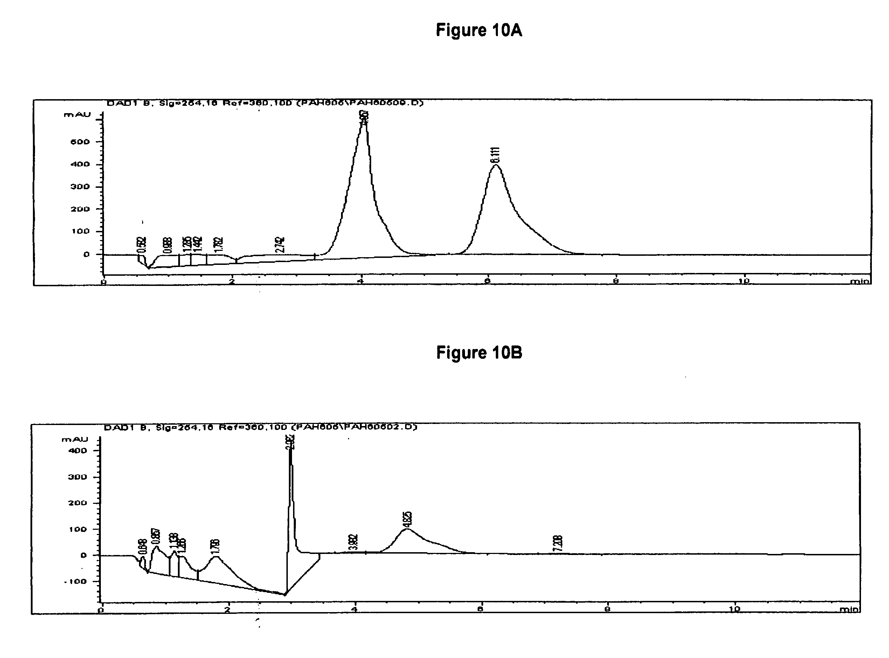

For the nonchiral analysis of *t*-leucine, the cell lysate presented the most serious problem amongst the group of products for this project. This was compounded by the low spectroscopic properties of the amino acid, leading to difficulty in differentiating the product peak from the cell lysate. Good separation of the individual product enantiomers was obtained by chiral analysis as shown in Fig. 10A. During the primary screen, a small peak eluted at the same time as the L-amino acid standard in certain samples (see Fig. 10B) and was thought to be the amino acid. However, further development of the method and the use of the appropriate controls established that this peak was actually a cell lysate peak.

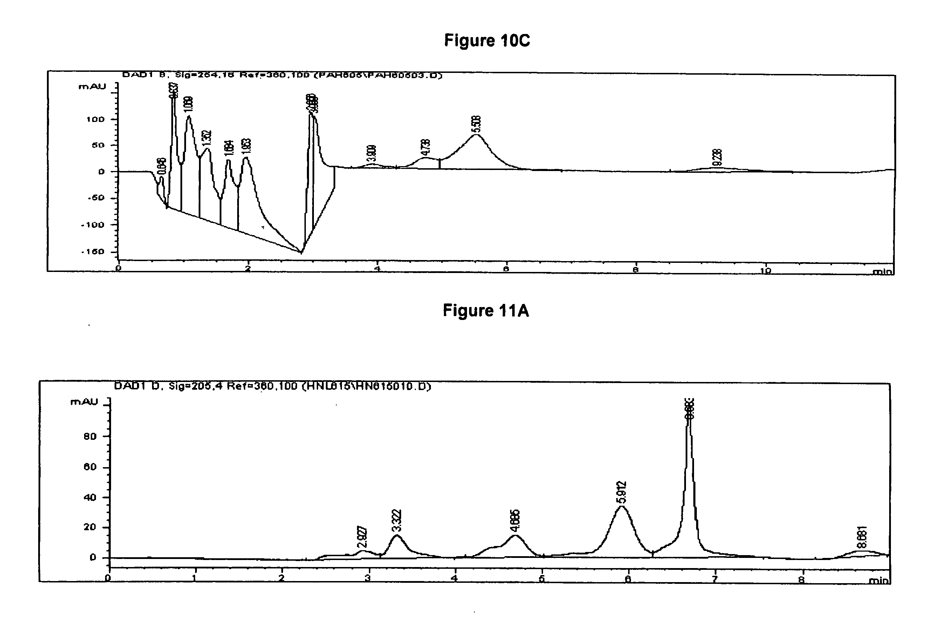

The aminonitrile eluted between the two *t*-leucine peaks, as shown in Fig. 10C; this chromatogram also shows the cell lysate peak at 4.8 min. The UV spectrum of the nitrile was distinct from that of the amino acid, making it easier to differentiate from the acid peaks.

### L-hydroxynorleucine ((S)-2-amino-6-hydroxy hexanoic acid)

The chiral analysis of (S)-2-amino-6-hydroxy hexanoic acid was consistent and reliable. By contrast, the nonchiral method presented many problems, primarily as a result of non-separation between the nitrile and the acid peaks. Towards the latter half of the project, a method was developed and used successfully for the confirmation of activities. Prior to this, most of the analysis was performed using the chiral method; standard curves of the products were run in order to quantify the reactions. A representative chromatogram of (S)-2-amino-6-hydroxy hexanoic acid is shown in Figure 11A, with (S)-2-amino-6-hydroxy hexanoic acid eluting at 6 min. The aminonitrile was not detected by this method.

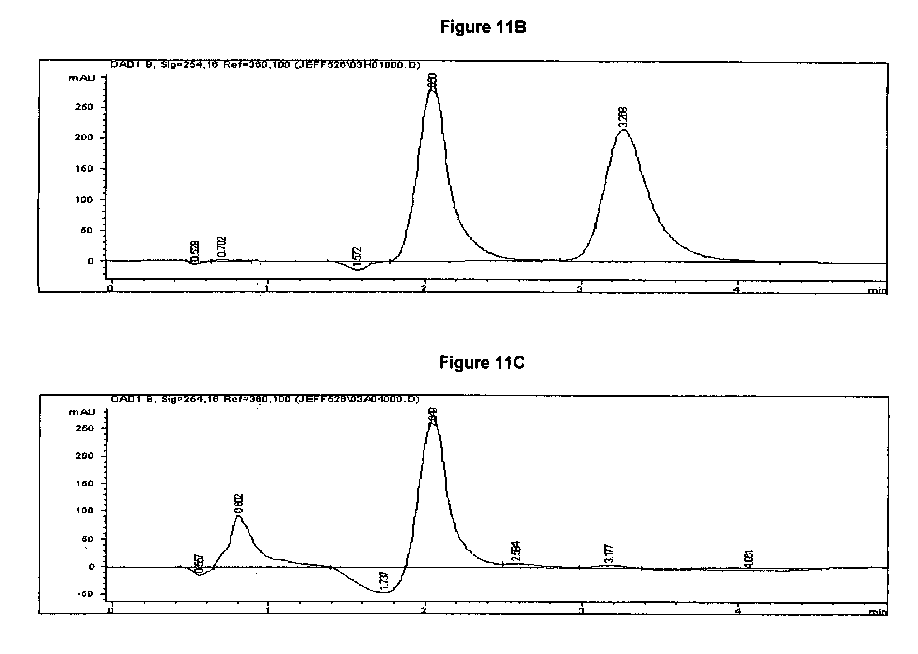

Separation of the individual 2-amino-6-hydroxy hexanoic acid enantiomers is shown in Fig. 11B. The L-enantiomer elutes first, at 2 min, followed by the D-enantiomer at 3 min. In Fig. 11C, an enzymatic sample is represented; the only area of slight concern is the negative peak preceding the elution of the L-enantiomer. However, it did not appear to interfere significantly with the elution of this enantiomer; method development did not eliminate the negative peak.

### 4-methyl-D-leucine and 4-methyl-L-leucine

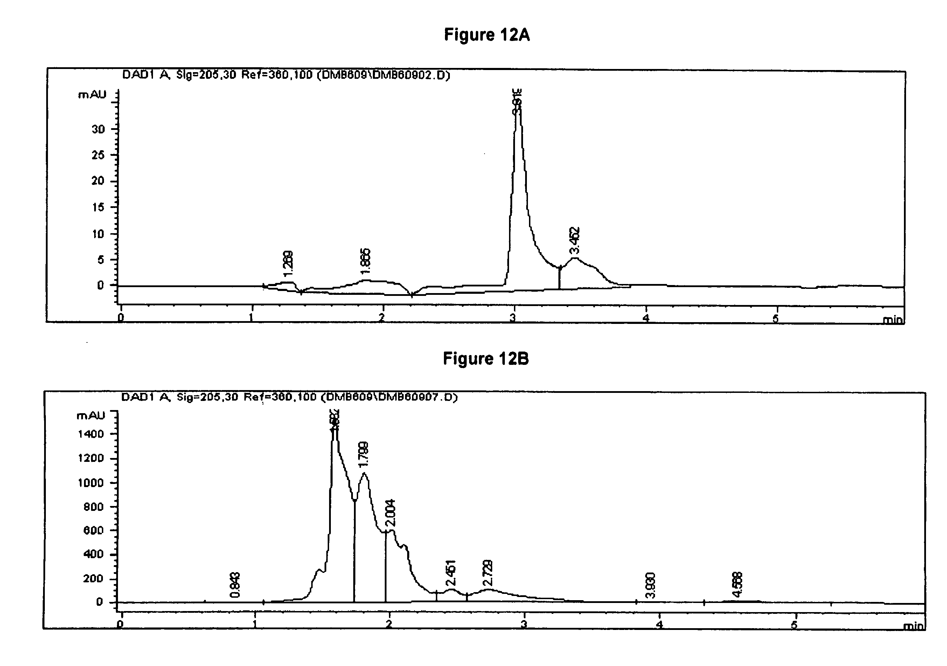

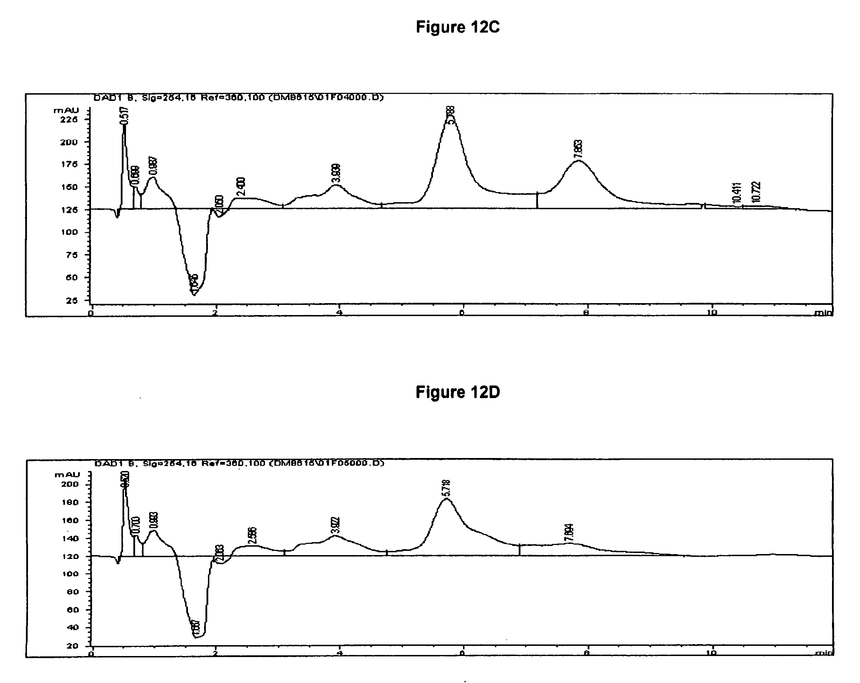

For the detection of 4-methylleucine, the chiral HPLC method again proved more reliable. The combination of low activities, together with the low sensitivity of the method to the compound led to difficulties in detection using nonchiral HPLC. A 2.5 mM standard of the amino acid is shown in Fig. 12A, with a peak height of approximately 40 mAU; this was substantially lower than those detected for the aromatic compounds. Chromatogram in Fig. 12B shows an enzymatic sample, in which conversion was detected using the chiral HPLC method; while it is not clear, it would appear that the 4-methylleucine peak elutes at 2.7 min and is extremely low in both peak height and area. This peak did not appear in samples which were negative by chiral HPLC analysis.

The chiral analysis of 4-methyl-L-leucine and 4-methyl-D-leucine did not present any problems. The L-enantiomer eluted at 5 min and the D-enantiomer at 7 min, although some peak shift did occur, as a result of the sensitivity to the column, described in section (i) for phenylglycine. In chromatograms shown in Figs. 14C-14D, the separation of these amino acids is shown; the first sample represents an enzyme which produced both enantiomers and in the second sample, the enzyme preferentially hydrolyzed the L-enantiomer, with a small amount D-amino acid forming.

### (S)-cyclohexylmandelic acid

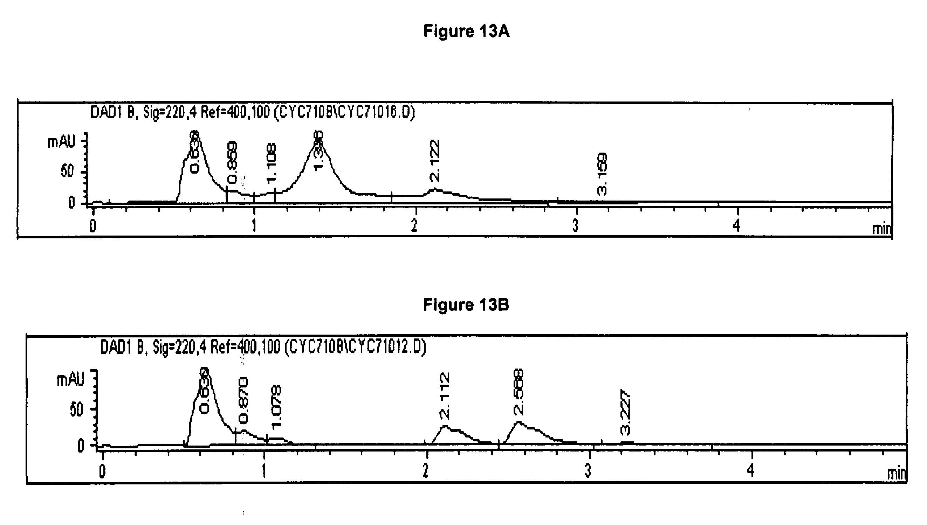

Chromatograms of the standards for cyclohexylmandelic acid (Fig. 13A) and the corresponding nitrile (Fig. 13B) are shown. The acid eluted at 1.3 min, while the cyanohydrin was observed at 2.5 min. The peak eluting at 2.1 min is thought to be the cyclohexylphenylketone, as shown by the elution of a ketone standard at this point.

### Example 12

### An Enzyme Library Approach to Biocatalysis: Development of a Nitrilase Platform for Enantioselective Production of Carboxylic Acid Derivatives

Biocatalytic processes can offer unique advantages in transformations that are challenging to accomplish through conventional chemical methods (Wong, C.-H.; Whitesides, G.M. Enzymes in Synthetic Organic Chemistry; Pergamon, New York, 1994; Drauz, K.; Waldmann, H., Roberts, S.M. Eds. Enzyme Catalysis in Organic Synthesis; VCH: Weinheim, Germany, 2nd ed., 2002). Nitrilases (EC 3.5.5.1) promote the mild hydrolytic conversion of organonitriles directly to the corresponding carboxylic acids (Kobayashi, M.; Shimizu, S. FEMS Microbiol. Lett. 1994, 120, 217; Bunch, A.W. In Biotechnology; Rehm, H.-J.; Reed, G.; Puhler, A.; Stadler, P., Eds.; Wiley-VCH: Weinheim, Germany, Vol. 8a, Chapter 6, pp 277-324; Wieser, M.; Nagasawa, T. In Stereoselective Biocatalysis; Patel, R.N., Ed.; Marcel Dekker: New York, 2000, Chapter 17, pp 461-486.) Fewer than fifteen microbially-derived nitrilases have been characterized and reported to date. (Harper, D.B. Int. J. Biochem. 1985, 17, 677; Levy-Schil, S.; Soubrier, F.; Crutz-Le Coq, A.M.; Faucher, D.; Crouzet, J.; Petre, D. Gene 1995, 161, 15; Yu, F. 1999, US Patent 5872000; Ress-Loschke, M.; Friedrich, T.; Hauer, B.; Mattes, R.; Engels, D. PCT Appl. WO 00/23577, April 2000.). Several nitrilases previously have been explored for the preparation of single-enantiomer carboxylic acids, although little progress has been made in the development of nitrilases as viable synthetic tools. This application describes the discovery of a large and diverse set of nitrilases and herein demonstrate the utility of this nitrilase library for identifying enzymes that catalyze efficient enantioselective production of valuable hydroxy carboxylic acid derivatives.

In an effort to access the most diversified range of enzymes that can be found in Nature, we create large genomic libraries by extracting DNA directly from environmental samples that have been collected from varying global habitats. (For a description of these methods, see: Short, J.M. Nature Biotech. 1997, 15, 1322; Handelsman, J.; Rondon, M.J.; Brady, S.F.; Clardy, J.; Goodman, R.M. Chem. Biol. 1998, 5, R245; Henne, A.; Daniel, R.; Schmitz, R.A.; Gottschalk, G. Appl. Environ. Microbiol. 1999, 65, 3901.). We have established a variety of methods for identifying novel activities through screening mixed populations of uncultured DNA. (Robertson, D.E.; Mathur, E.J.; Swanson, R.V.; Marrs, B.L.; Short, J.M. SIM News 1996, 46, 3; Short, J.M. US Patent 5,958,672, 1999; Short J.M. US Patent 6,030,779, 2000.) Through this approach, nearly 200 new nitrilases have been discovered and characterized. (For a concise description of the studies, see Materials and Methods section below.) All nitrilases were defined as unique at the sequence level and were shown to possess the conserved catalytic triad Glu-Lys-Cys which is characteristic for this enzyme class. (Pace, H.; Brenner, C. Genome Biology 2001, 2, 0001.1-0001.9.) Each nitrilase in our library was overexpressed and stored as a lyophilized cell lysate in order to facilitate rapid evaluation of the library for particular biocatalytic functions.

The initial investigations focused upon the efficacy of nitrilases for production of α-hydroxy acids **2** formed through hydrolysis of cyanohydrins **1.** Cyanohydrins are well-documented to racemize readily under basic conditions through reversible loss of HCN. (Inagaki, M.; Hiratake, J.; Nishioka, T.; Oda, J.; J. Org. Chem 1992, 57, 5643. (b) van Eikeren, P. US Patent 5,241,087, 1993.) Thus, a dynamic kinetic resolution process is possible whereby an enzyme selectively hydrolyzes only one enantiomer of **1,** affording **2** in 100% theoretical yield and with high levels of enantiomeric purity.

One important application of this type involves commercial production of (*R*)-mandelic acid from mandelonitrile. (Ress-Loschke, M.; Friedrich, T.; Hauer, B.; Mattes, R.; Engels, D. PCT Appl. WO 00/23577, April 2000; Yamamoto, K.; Oishi, K.; Fujimatsu, I.; Komatsu, K. Appl. Environ. Microbiol. 1991, 57, 3028; Endo, T.; Tamura, K. US Patent 5,296,373, March 1994.) Mandelic acid and derivatives find broad use as intermediates and resolving agents for production of many pharmaceutical and agricultural products. (Coppola, G.M.; Schuster, H.F. Chiral α-Hydroxy Acids in Enantioselective Synthesis; Wiley-VCH: Weinheim, Germany: 1997.) However, the few known nitrilases derived from cultured organisms have not been found useful for efficient and selective hydrolysis of analogous substrates.

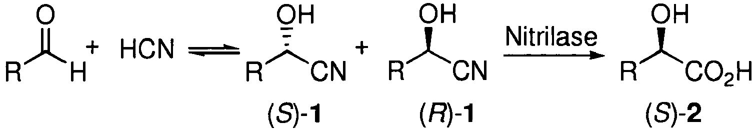

The nitrilase library was screened for activity and enantioselectivity in the hydrolysis of mandelonitrile (3a, Ar = phenyl) to mandelic acid. Preliminary results revealed that 27 enzymes afforded mandelic acid in >90% ee. One enzyme, SEQ ID NOS:385, 386, was studied in greater detail and was found to be very active for hydrolysis of mandelonitrile. Under standard conditions using 25 mM 3a and 0.12 mg/mL enzyme in 10% MeOH (v/v) 0.1 M phosphate buffer at 37oC and pH 8, (R)-mandelic acid was formed quantitatively within 10 min and with 98% *ee.* To confirm synthetic utility, the reaction was performed using 1.0 g 3a (50 mM) and 9 mg nitrilase (0.06 mg/mL nitrilase I); after 3 h (R)-mandelic acid was isolated in high yield (0.93 g, 86%) and again with 98% *ee.*

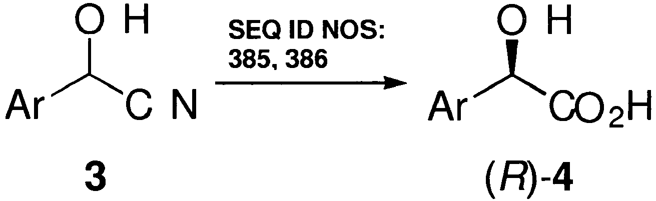

**Table 13. SEQ ID NOS:385, 386-catalyzed production of mandelic acid derivatives and analogues 4^{a}**

| Entry | Ar in 4 | Spec. Act.^{b} | TOF^{c} | % ee^{d} |
|---|---|---|---|---|
| 1 | C₆H₅ | 50 | 28 | 98 |
| 2 | 2-Cl-C₆H₅ | 3 | 1.7 | 97 |
| 3 | 2-Br-C₆H₅ | 10 | 5.6 | 96 |
| 4 | 2-Me-C₆H₅ | 9 | 5.1 | 95 |
| 5 | 3-Cl-C₆H₅ | 6 | 3.4 | 98 |
| 6 | 3-Br-C₆H₅ | 3 | 1.7 | 99 |
| 7 | 4-F-C₆H₅ | 21 | 11.8 | 99 |
| 8 | 1-naphthyl | 5 | 2.8 | 95 |
| 9 | 2-naphthyl | 5 | 2.8 | 98 |
| 10 | 3-pyridyl | 33 | 18.6 | 97 |
| 11 | 3-thienyl | 30 | 16.8 | 95 |

| | | | | |
|---|---|---|---|---|
| (a) Reactions were conducted under standard conditions (see text). Reaction time for complete conversion to **4** was 1-3 h. Entries 8-9 were conducted at pH 9 and 5 mM substrate concentration. (b) Specific activities were measured at 5 min transformation timepoints and are expressed as µmol mg⁻¹ min⁻¹. (c) TOF = turnover frequency, mol product/mol catalyst/sec. (d) Enantioselectivites were determined by chiral HPLC analysis. Hydroxy acids were isolated and absolute configurations were determined to be (*R*) in all cases. | | | | |

The substrate scope of SEQ ID NOS:385, 386 was next explored. As shown in Table 13, a broad range of mandelic acid derivatives as well as aromatic and heteroaromatic analogues (**4**) may be prepared through this method. SEQ ID NOS:385, 386 tolerates aromatic ring substituents in the *ortho-, meta-,* and *para-*positions of mandelonitrile derivatives and products of type **4** were produced with high enantioselectivities. Other larger aromatic groups such as 1-naphthyl and 2-naphthyl also are accommodated within the active site, again affording the acids **4** with high selectivity (Table 13, entries 8-9). Finally, 3-pyridyl and 3-thienyl analogues of mandelic acid were prepared readily using this process (Table 13, entries 10-11). This is the first reported demonstration of a nitrilase that affords a range of mandelic acid derivatives and heteroaromatic analogues of type **4.** High activity on the more sterically encumbered *ortho*-substituted and 1-naphthyl derivatives is particularly noteworthy.

We next examined the preparation of aryllactic acid derivatives **6** through hydrolysis of the corresponding cyanohydrins **5.** Phenyllactic acid and derivatives serve as versatile building blocks for the preparation of numerous biologically active compounds. (Coppola, G.M.; Schuster, H.F. Chiral α-Hydroxy Acids in Enantioselective Synthesis; Wiley-VCH: Weinheim, Germany: 1997.) Upon screening our nitrilase library against the parent cyanohydrin **5a** (Ar = phenyl), we found several enzymes that provided **6a** with high enantiomeric excess. One enzyme, SEQ ID NOS: 103, 104, was further characterized. After optimization, SEQ ID NOS:103, 104, was shown to provide (*S*)-phenyllactic acid (**6a**) with complete conversion (50 mM) and very high enantioselectivity (98% ee) over 6 h. The highest 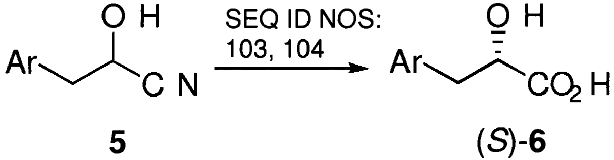 enantioselectivity previously reported for biocatalytic conversion of **5** to **6** was 75% ee achieved through a whole cell transformation using a *Pseudomonas* strain. (Hashimoto, Y.; Kobayashi, E.; Endo, T.; Nishiyama, M.; Horinouchi, S. Biosci. Biotech. Biochem. 1996, 60, 1279.)

**Table 14. Nitrilase II-catalyzed production of aryllactic acid derivatives and analogues 6^{a}**

| Entry | Ar in **6** | Spec. Act.*^{b}* | TOF*^{c}* | % ee*^{d}* |
|---|---|---|---|---|
| 1 | C₆H₅ | 25 | 16 | 99 |
| 2 | 2-Me-C₆H₅ | 160 | 100 | 95 |
| 3 | 2-Br-C₆H₅ | 121 | 76 | 95 |
| 4 | 2-F-C₆H₅ | 155 | 97 | 91 |
| 5 | 3-Me-C₆H₅ | 21 | 13 | 95 |
| 6 | 3-F-C₆H₅ | 22 | 14 | 99 |
| 7 | 1-naphthyl | 64 | 40 | 96 |
| 8 | 2-pyridyl | 10.5 | 6.6 | 99 |
| 9 | 3-pyridyl | 11.6 | 7.2 | 97 |
| 10 | 2-thienyl | 3.4 | 2.1 | 96 |
| 11 | 3-thienyl | 2.3 | 1.4 | 97 |

| | | | | |
|---|---|---|---|---|
| (a) Reaction conditions as in Table 13, except 0.016 mg/mL nitrilase was used. Full conversion to **6** was observed within 6 h. (b)-(d) See Table 13. The absolute configuration was determined to be (*S*) for phenyllactic acid and entries 2-11 were assigned (*S*) based upon identical chiral HPLC peak elution order. | | | | |

*Ortho* and *meta* substituents appear to be tolerated well by nitrilase II, with *ortho* substituted derivatives surprisingly being converted with higher rates relative to the parent substrate **5a.** Novel heteroaromatic derivatives, such as 2-pyridyl-, 3-pyridyl, 2-thienyl- and 3-thienyllactic acids, were prepared with high conversions and enantioselectivities (entries 8-11). Unexpectedly, *para* substituents greatly lowered the rates of these reactions, with full conversion taking over two weeks under these conditions.

The final transformation that we examined was desymmetrization of the readily available prochiral substrate 3-hydroxyglutarylnitrile (7) (Johnson, F.; Panella, J.P.; Carlson, A.A. J. Org. Chem. 1962, 27, 2241) to afford hydroxy acid (*R*)-**8** which, once esterified to (*R*)-**9**, is an intermediate used in the manufacture of the cholesterol-lowering drug LIPITOR^{™}. Previously reported attempts to use enzymes for this process were unsuccessful and **8** was produced with low selectivity (highest: 22% ee) and the undesired (*S*)-configuration. (Crosby, J.A.; Parratt, J.S.; Turner, N.J. Tetrahedron: Asymmetry 1992, 3, 1547; Beard, T.; Cohen, M.A.; Parratt, J.S.; Turner, N.J. Tetrahedron: Asymmetry 1993, 4, 1085; Kakeya, H.; Sakai, N.; Sano, A.; Yokoyama, M.; Sugai, T.; Ohta, H. Chem. Lett. 1991, 1823.)

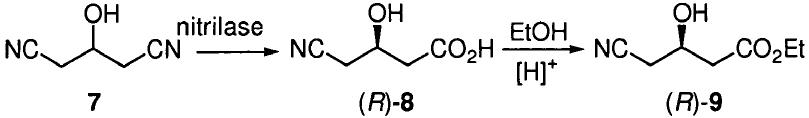

The nitrilase library was screened and unique enzymes were discovered and isolated that provided the required product (*R*)-**8** with high conversion (>95%) and >90% *ee.* Using one of the (*R*)-specific nitrilases, this process was operated on a 1.0 g scale (240 mM **7**, 30 mg enzyme, 22°C, pH 7) and after 22 h, (*R*)-**8** was isolated in 98% yield and 95% *ee.* Interestingly, the same screening program also identified nitrilases that afford the opposite enantiomer (*S*)-**8** with 90-98% ee. Thus, the extensive screen of biodiversity has uncovered enzymes that provide ready access to either enantiomer of the intermediate **8** with high enantioselectivities. Our discovery of the first enzymes that furnish (*R*)-**8** underscores the advantage of having access to a large and diverse library of nitrilases.

### SUMMARY for Nitrilases Activity on HydroxyGlutarylnitrile (Primary Data)

| SEQ ID NO: | **%ee** | **% Conversion** | **LIPITOR™** |
|---|---|---|---|
| 107, 108 | 100 | 79 | S |
| 109, 110 | 100 | 79 | S |
| 111, 112 | 91 | 32 | S |
| 127, 128 | 92 | 106 | S |
| 129, 130 | 100 | 22 | S |
| 133, 134 | 86 | 14 | S |
| 113, 114 | 100 | 108 | S |
| 145, 146 | 100 | 100 | S |
| 101, 102 | 100 | 61 | S |
| 179,180 | 100 | 75 | S |
| 201,202 | 100 | 100 | S |
| 159, 160 | 100 | 71 | S |
| 177,178 | 100 | 11 | S |
| 181, 182 | 100 | 58 | S |
| 183,184 | 100 | 19 | S |
| 185,186 | 100 | 78 | S |
| 191,192 | 100 | 67 | S |
| 57,58 | 100 | 73 | S |
| 197, 198 | 100 | 64 | S |
| 41, 42 | 100 | 16 | R |
| 59, 60 | 100 | 100 | S |
| 207, 208 | 100 | 111 | R |
| 209,210 | 92 | 100 | R |
| 73, 74 | 100 | 3 | R |
| 153,154 | 35 | 39 | |
| 171, 172 | 27 | 33 | |
| 195, 196 | 100 | 87 | R |
| 43, 44 | 100 | 111 | R |
| 67, 68 | 100 | 35 | S |
| 359, 360 | 100 | 87 | S |

By plumbing our environmental genomic libraries created from uncultured DNA, we have discovered a large array of novel nitrilases. This study has revealed specific nitrilases that furnish mandelic and aryllactic acid derivatives, as well as either enantiomer of 4-cyano-3-hydroxybutyric acid in high yield and enantiomeric excess.

### Procedures and Analytical Data:

Hydroxyglutarylnitrile was purchased from TCI America and used as received. Amino acids used for the preparation of aryl lactic acid standards were purchased from PepTech (Cambridge, MA). (*R*)-3-hydroxy-4-cyanobutyric acid was obtained from Gateway Chemical Technology (St. Louis, MO). Both (*R*)- and (*S*)- mandelic acid and (*R*)- and (*S*)- phenyl lactic acid standards were purchased from Sigma Aldrich. All other reagents were purchased from Sigma Aldrich and utilized without further purification. Silica Gel, 70-230 mesh, 60 Å, purchased from Aldrich, was used for chromatographic purifications. All ¹H NMRs and ¹³C NMRs were run on Bruker model AM-500 machines, set at room temperature, 500 MHz and 125MHz respectively for ¹H and ¹³C. Mass analyses and unit mass resolution was achieved by flow injection analysis (FIA) using a Perkin-Elmer Sciex API-4000 TURBOION™ Spray LC/MS/MS system. The LC flow was provided by Schimadzu LC-10Advp pumps, with 0.05% acetic acid and MeOH. Injections were accomplished via a Valco injector valve. The HPLC analysis was done on an Agilent 1100 HPLC with Astec's Chirobiotic R column (100 x 4.6 mm, cat no. 13022 or 150 x 4.6 mm, cat no. 13023) or Daicel's Chiralcel OD column (50 x 4.6 mm, cat no. 14022) and the DAD detector set at 210, 220, 230, and 250 nm. For specific rotations, a Perkin Elmer Model 341 Polarimeter was used, set at 589 nm, Na lamp, at room temperature, with a 100 mm path length cell. Concentrations for specific rotation are reported in grams per 100 mL of solvent. Microbiology techniques were executed in accordance to published protocols. (Sambrook, J. Fritsch, EF, Maniatis, T. (1989) Molecular Cloning: A Laboratory Manual (2nd ed.), Cold Spring Harbor Laboratory Press, Plainview NY.) Glycolic acid products were isolated and absolute configurations were determined to be (R) in all cases by comparison with literature optical rotation data on configurationally defined compounds except for (-)-3-pyridylglycolic acid, which to our knowledge is not known as a single enantiomer. (For mandelic, 2-chloromandelic, 2-methyl mandelic, 3-chloromandelic, 3-bromomandelic and 4-fluoromandelic acid see Hoover, J.R. E.; Dunn, G. L.; Jakas, D.R.; Lam, L.L.; Taggart, J. J.; Guarini, J.R.; Phillips, L. J. Med. Chem. 1974, 17(1), 34-41; For 2-bromo mandelic acid see Collet, A.; Jacques, J.; Bull. Soc. Chem. Fr. 1973, 12, 3330-3331; For 1- and 2-napthylglycolic acid see Takahashi, I; Y. Aoyagi, I. Nakamura, Kitagawa, A., Matsumoto, K., Kitajima, H. Isa, K. Odashima, K. Koga, K. Heterocycles 1999, 51(6), 1371-88; For 3-thienylglycolic acid Gronowitz, S. Ark. Kemi, 1957, 11,519-525.)

For the aryl lactic acid products, absolute configuration was established to be *(S)* for phenyl lactic acid by comparison with literature optical rotation and for all other phenyl lactic acid products, absolute configurations were predicted based upon elution order using chiral HPLC. Absolute configuration for 3-hydroxy-4-cyano-butanoic acid was established by derivatization to (R)-(-)-Methyl (3-O-[benzoyl]-4-cyano)-butanoate and comparison to literature optical rotation data on configurationally defined compound. (3. Beard, T. Cohen, M. A. Parratt, J.S. Turner, N. J. Tetrahedron:Asymm. 4(6), 1993, 1085-1104.)

### Nitrilase Discovery and Characterization Methods:

### 1. Nitrilase Selection.

An *Escherichia coli* screening host strain, SEL700, was optimized for nitrilase selections on a nitrile substrate. An Abs₆₀₀ₙₘ = 1, resuspension of SEL700 screening host in 10 mM MgSO₄ was infected with kanamycin-resistant environmental DNA library for 45 minutes at 37°C, such that complete screening coverage of the library was achieved. Infected cells, now denoted by kanamycin resistance, were plated on kanamycin LB plates and allowed to grow overnight at 30°C. Titer plates were also made to determine infection efficiency. Cells were pooled, washed, and resuspended the next morning with 10 mM MgSO₄. Transformed clones were inoculated into M9 media (without nitrogen) with 10 mM of nitrile substrate. M9 media consisted of 1X M9 salts (NH₄Cl omitted), 0.1mM CaCl₂, 1 mM MgSO₄, 0.2 % glucose, and approximately 10 mM of a nitrile selection substrate. The selection cultures were then incubated at 30°C, shaking at 200 rpm, for up to five weeks. Positive nitrilase cultures were identified by growth, due to positive clone's ability to hydrolyze nitrile substrate. Positive clones were isolated by streaking out a selection culture with growth and subsequent secondary culturing of isolated colonies in the same defined media. The DNA from any positive secondary cultures exhibiting re-growth was then isolated and sequenced to confirm discovery of a nitrilase gene and to establish the unique nature of that gene.

### 2. Nitrilase Biopanning.

Traditional filter lift hybridization screening protocols are limited to libraries with approximately 10⁶ to 10⁷ members. Attempting to screen one library would require approximately 5,000 filter lifts. Therefore, solution phase and other biopanning formats have been developed for ultra high throughput sequence based screening permitting rapid screening of up to 10⁸ member environmental libraries In the solution format, the DNA from a large number of library clones is mixed with tagged molecules of interest under conditions which promote hybridization. The tagged clones and hybridized DNA are then removed from solution and washed at some level of stringency to remove clones which do not have sequence identity with the probe. The hybridized DNA is then eluted and recovered. Clones of interest are sequenced and cloned to provide enzyme activities of interest. This method has been demonstrated to achieve up to 1,000-fold enrichment per round for sequences of interest.

### 3. High Throughput Nitrilase Activity Assay.

Activity assays were conducted using 25 mM (∼3 mg/mL) substrate, 0.1 mg/mL nitrilase in 0.25 mL of assay solution. Assay solutions consisted of 0-10% (v/v) MeOH in 0.1 M sodium phosphate buffer solution at pH 7 to 9 and temperatures 37°C or 22°C. Specific activities were measured at 5 min transformation time point, unless otherwise noted, and are expressed in units µmol mg⁻¹ min⁻¹. Enantiomeric excess and conversion rates were determined by high throughput HPLC analysis comparing enzyme product concentration to standard curves of racemic acid products. Analytical conditions for the products are tabulated below.

### Analytical Methods:

| | **Acid Product** | **Column** | **Liquid Chromatography Method** | **Retention Times of enantiomers (min)** |
|---|---|---|---|---|
| 1.1 | mandelic acid | Chirabiotic R 100 x 4.6 mm | 20%[0.5% AcOH], 80% CH₃CN 1 ml/min | 2.4 (S); 2.9 (R) |
| 1.2 | 2-Cl-mandelic acid | Chirabiotic R 100 x 4.6 mm | 20%[0.5% AcOH], 80% CH₃CN 1 ml/min | 2.3 (*S*); 2.9 *(R)* |
| 1.3 | 2-Br-mandelic acid | Chirabiotic R 100 x 4.6 mm | 20%[0.5% AcOH], 80% CH₃CN 1 ml/min | 2.8; 4.0 |
| 1.4 | 2-CH₃-mandelic acid | Chirabiotic R 100 x 4.6 mm | 20%[0.5% AcOH], 80% CH₃CN 1 ml/min | 3.1; 3.8 |
| 1.5 | 3-Cl-mandelic | Chirabiotic R 100 x 4.6 mm | 10%[0.5% AcOH], 90% CH₃CN 1 ml/min | 3.1; 3.8 |
| 1.6 | 3-Br-mandelic | Chirabiotic R 100 x 4.6 mm | 10%[0.5% AcOH], 90% CH₃CN 1 ml/min | 3.3; 3.9 |
| 1.7 | 4-F-mandelic | Chirabiotic R 150 x 4.6 mm | 20%[0.5% AcOH], 80% CH₃CN 1 ml/min | 3.7; 4.8 |
| 1.8 | 1-napthylglycolic acid | Chirabiotic R 100 x 4.6 mm | 4%[0.5% AcOH], 96% CH₃CN 1 ml/min | 3.1; 3.7 |
| 1.9 | 2-napthylglycolic acid | Chirabiotic R 100 x 4.6 mm | 4%[0.5% AcOH], 96% CH₃CN 1 ml/min | 3.7; 4.7 |
| 1.10 | 3-pyridylglycolic acid | Chirabiotic R 100 x 4.6 mm | 5% [0.5% AcOH], 65% H₂O, 30% CH₃CN, 2 ml/min | 4.4; 5.5 |
| 1.11 | 3-thienylglycolic acid | Chirabiotic R 100 x 4.6 mm | 20%[0.5% AcOH], 80% CH₃CN 2 ml/min | 1.4; 2.5 |
| 2.1 | phenyl lactic acid | Chirabiotic R 150 x 4.6 mm | 20%[0.5% AcOH], 80% CH₃CN 1 ml/min | 2.8 (*S*); 4.0 (R) |
| 2.2 | 2-methylphenyl lactic acid | Chirabiotic R 150 x 4.6 mm | 20%[0.5% AcOH], 80% CH₃CN 1 ml/min | 2.5; 2.8 |
| 2.3 | 2-bromophenyl lactic acid | Chirabiotic R 150 x 4.6 mm | 20%[0.5% AcOH], 80% CH₃CN 1 ml/min | 2.8; 3.2 |
| 2.4 | 2-fluorophenyl lactic acid | Chirabiotic R 150 x 4.6 mm | 20%[0.5% AcOH], 80% CH₃CN 1 ml/min | 2.6; 2.9 |
| 2.5 | 3-methylphenyl lactic acid | Chirabiotic R 150 x 4.6 mm | 20%[0.5% AcOH], 80% CH₃CN 1 ml/min | 2.4; 3.2 |
| 2.6 | 3-fluorophenyl lactic acid | Chirabiotic R 150 x 4.6mm | 20%[0.5% AcOH], 80% CH₃CN 1 ml/min | 2.8; 3.6 |
| 2.7 | 1-napthyllactic acid | Chirabiotic R 150 x 4.6mm | 20%[0.5% AcOH], 80% CH₃CN 1 ml/min | 2.7; 3.1 |
| 2.8 | 2-pyridyllactic acid | Chirabiotic R 150 x 4.6mm | 20%[0.5% AcOH], 80% CH₃CN 1 ml/min | 2.5; 2.9 |
| 2.9 | 3-pyridyllactic acid | Chirabiotic R 150 x 4.6mm | 20%[0.5% AcOH], 80% CH₃CN 1 ml/min | 2.9; 3.6 |
| 2.10 | 2-thienyllactic acid | Chirabiotic R 150 x 4.6mm | 20%[0.5% AcOH], 80% CH₃CN 1 ml/min | 3.6; 4.6 |
| 2.11 | 3-thienyllactic acid | Chirabiotic R 150 x 4.6mm | 20%[0.5% AcOH], 80% CH₃CN 1 ml/min | 3.5; 4.6 |
| | Methyl(3-O [benzoyl]-4-cyano)-butanoate | Daicel OD 50 x 4.6 mm | 5% isopropanol, 95% hexane 1 ml/min | 4.5 (*R*); 5.4(*S*) |

### Cyanohydrin (Substrate) Synthesis:

**Mandelonitrile Synthesis Method A:** Acetone cyanohydrin (685 µL, 7.5 mmol), aldehyde (5 mmol), and catalytic DIEA (13 µL, 0.075 mmol) were mixed at 0 °C. The reactions were stirred on ice for 45 minutes. To drive the equilibrium toward the product, acetone was removed *in vacuo.* Subsequently, crude reactions were acidified with H₂SO₄ (3 µL) and stored at -20°C. TLC was used to monitor reaction progress (3:1 hexane/ethylacetate (EtOAc).
**Mandelonitrile Synthesis Method B:** To a solution of KCN (358 mg, 5.5 mmol) in MeOH (1 mL) at 0°C was added aldehyde (5 mmol) and acetic acid (315 µL, 5.5 mmol). After stirring for one hour on ice, MeOH was removed *in vacuo,* and the crude mixture was partitioned using EtOAc and H₂O. The organic fraction was retained and concentrated *in vacuo.* TLC analysis was used to monitor reaction progress (3:1 Hexanes/EtOAc).
**Aryl Acetaldehyde Cyanohydrin:,** Arylacetic acid (50 mmol) was dissolved in 50 ml anhydrous tetrahydrofuran (THF) in a two-neck 500 ml round-bottom flask under N₂(g) atmosphere. To this solution cooled to 0 °C, under vigorous mixing, was added slowly 105 mmol of thexylchloroborane-dimethyl sulfide (2.55 M in methylene chloride). The reaction was allowed to proceed overnight. Excess acetic acid (10 ml) was added to quench and acidify the reaction followed by the addition 10 ml water. After stirring at room temperature for 1 hour, solvent was removed *in vacuo* and the residue was dissolved in 100 ml water and extracted with 200 ml EtOAc. The EtOAc layer was dried over sodium sulfate, filtered and then concentrated *in vacuo.* Subsequently, 60 mmol of KCN, followed by 100 ml methanol was added to the residue. The solution was then cooled to 0 °C and acetic acid (60 mmol) added. The reaction was stirred for 1-2 hours after all KCN dissolved. Solvents were removed *in vacuo* and residue was dissolved in 100 ml water and 200 ml EtOAc. The aqueous layer was extracted with EtOAc one more time. Combined EtOAc extracts were washed with saturated brine and dried over sodium sulfate, filtered and then concentrated *in vacuo* to obtain crude cyanohydrin product. The cyanohydrin was purified by silica-gel column (hexane/EtOAc), as necessary.
**2-chloro mandelonitrile:** ¹H NMR (CDCl₃, 500 MHz) δ 7.69 (m, 1H), 7.41 (m, 1H), 7.36 (m, 2H), 5.84 (s, 1H), 3.07 (br, 1H). ¹³C NMR (CDCl₃, 125 MHz) δ 132.89, 132.73, 131.22, 130.19, 128.48, 127.84, 118.24, 60.87. MS calc'd for [C₈H₆CINO] 167.01 found 167.9 (LC-MS +).
**2-bromomandelonitrile:** ¹H NMR (CDCl₃, 500 MHz) δ 7.72 (d, 1H, *J*= 6.58), 7.62 (d, 1H, *J=* 8.35), 7.43 (t, 1H, *J=* 8.42), 7.30 (t, 1H, *J=* 7.00), 5.85 (s, 1H). ¹³C NMR (CDCl₃, 125 MHz) δ 134.550, 133.584, 131.564, 128.819, 128.535, 122.565, 118.153,63.379.
**2-methylmandelonitrile:** ¹H NMR (CDCl₃, 500 MHz) δ: 7.60 (d, 1H, *J*= 7.4), 7.23-7.35 (m, 3H), 5.66 (s, 1H), 2.44 (s, 3H). ¹³C NMR (CDCl₃, 298 K, 125 MHz) δ: 136.425, 133.415, 131.450, 130.147,127.204, 126.894, 118.952, 18.916. MS calc'd for [C₉H₉NO] 147.07, found 147.2 (ESI +).
**3-chloromandelonitrile:** ¹H NMR (CDCl₃, 500 MHz) δ 7.55 (s, 1H), 7.43-7.37 (m, 3H), 5.54 (s, 1H). ¹³C NMR (CDCl₃, 125 MHz) 8 137.183, 135.480, 130.718, 130.303,127.047, 124.891, 118.395, 63.156. MS calc'd for [C₈H₆CINO] 167.01 found 167.9 (LC-MS +).
**3-bromomandelonitrile:** ¹H NMR (CDCl₃, 500 MHz) 8 7.69 (s, 1H), 7.56 (d, *J*= 6.2 Hz, 1H), 7.45 (d, *J=* 5.5Hz, 1H), 7.32 (t, *J=* 6.4. Hz, 1H), 5.53 (s, 1H). ¹³C NMR (CDCl₃, 125 MHz) δ 137.376, 133.201, 130.934, 129.208,125.359, 123.380, 118.458, 63.006. MS calc'd for [C₈H₆BrNO] 212.0 found 211.9 (LC-MS +).
**4-fluoromandelonitrile:** ¹H NMR (CDCl₃, 500 MHz) 8 5.54 (s, 1H), 7.13 (m, 2H), 7.51-7.53 (m, 2H). ¹³C NMR (CDCl₃, 125 MHz) δ 63.02, 116.44, 118.97, 128.90, 131.54, 132.51, 162.575.
**4-chloromandelonitrile:** ¹H NMR (CDCl₃, 500 MHz) δ 7.47 (d, *J*= 7.0 Hz , 2H), 7.42 (d, *J*= 7.0 Hz , 2H), 5.53 (s , 1H). ¹³C NMR (CDCl₃, 125 MHz) δ 136.209, 133.845, 129.647, 128.232, 118.630, 63.154. MS calc'd for [C₈H₆CINO] 167.01 found 167.9 (LC-MS +)
**1-naphthyl cyanohydrin :** ¹H NMR (CDCl₃, 500 MHz) 8 8.14 (d, 1H, *J* = 8.5), 7.92 (t, 2H, *J* = 6.1), 7.82 (d, 1H, *J* = 5.7), 7.62 (t, 1H, *J* = 6.1), 7.56 (t, 1H, *J* = 6.1), 7.50 (t, 1H, *J* = 6.1), 6.18 (s, 1H); ¹³C NMR (CDCl₃, 125 MHz) δ 137.0, 135.7, 134.2, 131.1, 129.2, 127.5, 126.7, 125.8, 125.3, 123.1, 119.0, 62.4; MS calc'd for [C₁₂H₉O] 183.21, found 183.2 (ESI +).
**2-naphthyl cyanohydrin:** ¹H NMR (CDCl₃, 500 MHz) 8 8.03 (s, 1H), 7.92 (d, 1H, *J* = 8.6), 7.87-7.91 (m, 2H), 7.61 (dd, 1H, *J* = 6.7, 1.2), 7.55-7.60 (m, 2H), 5.72 (s, 1H); ¹³C NMR (CDCl₃, 125 MHz) δ 134.9, 133.9, 132.7, 129.6, 128.6, 128.0, 127.4, 127.2, 126.4, 123.9, 118.9, 64.1; MS calc'd for [C₁₂H₉O] 183.21, found 183.2 (ESI +).
**3-pyridyl cyanohydrin:** ¹H NMR (CDCl₃, 500 MHz) δ: 8.62 (d, 1H, *J* = 1.8), 8.57 (d, 1H, *J=* 5.1), 7.94 (d, 1H, *J* = 8.1), 7.41 (dd, 1H, *J =* 8.1, 5.1), 5.64 (s, 1H); ¹³C NMR (CDCl₃, 125 MHz) δ 149.921, 147.355, 135.412, 133.044, 124.443, 118.980, 61.085. MS calc'd for [C₇H₆N₂O] 134.05, found 135.2 (ESI +).
**3-thienyl cyanohydrin:** ¹H NMR (CDCl₃, 500 MHz) 8 7.45 (d, *J* = 2.2 Hz 1H), 7.56 (dd, *J* = 6.2 Hz, 1H), 7.45 (d, *J=* 5.5Hz, 1H), 7.32 (t, *J=* 6.4. Hz, 1H), 5.53 (s, 1H). ¹³C NMR (CDCl₃, 125 MHz) 8 137.376, 133.201, 130.934, 129.208,125.359, 123.380, 118.458, 63.006. MS calc'd for [C₆H₅NOS] 139.01 found 139.9 (LC-MS +).
**phenyl acetaldehyde cyanohydrin:** ¹H NMR (CDCl₃, 500 MHz) δ 7.34 (m, 5H), 4.64 (t, *J* = 6.75 Hz, 1H), 3.11 (d, *J* = 6.75 Hz, 2H), 2.75 (br, 1H). ¹³C NMR (CDCl₃, 125 MHz) δ 133.96, 129.91, 129.16, 128.08, 119.47, 62.33, 41.55.
**2-methylphenyl acetaldehyde cyanohydrin:** ¹H NMR (CDCl₃, 500 MHz) δ 7.11 (m, 4H), 4.61 (t, *J* = 6.62 Hz, 1H), 3.12 (d, *J* = 6.62 Hz, 2H), 2.14(s, 3H). ¹³C NMR (CDCl₃, 125 MHz) δ 136.94, 136.47, 132.57, 130.48, 127.61, 125.75, 120.11, 62.95, 44.73 MS calc'd for [C₁₀H₁₁NO]: 161.08, found 162.2 (M+Na, ESI +)
**2-bromophenyl acetaldehyde cyanohydrin:** ¹H NMR (CDCl₃, 500 MHz) δ 7.20 (m, 4H), 4.78 (t, *J* = 6.5 Hz, 1H), 3.26 (d, *J* = 6.5 Hz, 2H). ¹³C NMR (CDCl₃, 100 MHz) δ 133.93, 132.82, 131.72, 129.21, 128.12, 124.86, 119.41, 63.02, 44.89.
**2-fluorophenyl acetaldehyde cyanohydrin:** ¹H NMR (CDCl₃, 500 MHz) δ 7.2 (m, 2H), 7.02 (m, 2H), 4.50 (dd, *J* = 4.62 Hz, *J* = 7.88 Hz, 1H), 3.23(dd, *J=* 4.62 Hz, 1 *J* = 14.12 Hz, 1H), 2.97 (dd, 7.88 Hz, 14.12 Hz, 1H). ¹³C NMR (CDCl₃, 125 MHz) δ 132.18, 131.52, 129.66, 129.03, 128.07, 124.05, 115.8, 63.02, 44.79 MS calc'd for [C₉H₈FNO] 165.06, found 164.2 (ESI +).
**3-methylphenyl acetaldehyde cyanohydrin:** ¹H NMR (CDCl₃, 500 MHz) δ 7.18 (m, 1H), 7.02 (m, 3H), 4.54 (dd, *J* = 4.62 Hz, *J* = 8 Hz, 1H), 3.06 (dd, *J* = 4.62 Hz, *J =* 14.38 Hz, 1H), 2.83(dd, *J* = 8 Hz, *J* = 14.38 Hz, 1H), 2.36 (s, 3H) ¹³C NMR (CDCl₃, 125 MHz) δ 176.25, 138.18, 136.0, 130.97, 128.93, 127.68, 126.58, 76.42, 34.29, 37.69 MS calc'd for [C₁₀H₁₂O_{3]} 180.08, found 180.0 (ESI +).
**3-fluorophenyl acetaldehyde cyanohydrin:** ¹H NMR (CDCl₃, 500 MHz) δ 7.18 (m, 2H), 6.95 (m, 2H), 4.44 (dd, 1H), 3.11(dd, 1H). ¹³C NMR (CDCl₃, 125 MHz) 8 130.40, 125.53, 124.85, 116.92, 114.87, 114.50, 119.77, 61.97, 41.27.
**1-napthyl acetaldehyde cyanohydrin:** ¹H NMR (CDCl₃, 500 MHz) δ 8.07(m, 1H), 7.86(m, 1H), 7.74(m, 1H), 7.41(m, 4H),4.20 (t, *J* = 7 Hz, 1H), 3.33 (d, *J=* 6.8 Hz, 2H) ¹³C NMR (CDCl₃, 125 MHz) δ 177.7, 140.31, 129.74, 129.24, 128.92, 128.26, 127.84, 125.63, 124.53, 124.05, 123.42, 70.58, 38.0 MS calc'd for [C₁₃H₁₁NO] 197.08, found 197.1 (ESI+).
**2-pyridyl acetaldehyde cyanohydrin:** ¹H NMR (CDCl₃, 500 MHz) δ 8.50 (m, 1H), 7.85 (m, 1H), 7.48 (m, 1H), 7.34 (m, 1 H), 4.42 (m, 1H), 3.19 (dd, *J* = 3.5 Hz, J = 13.7 Hz, 2H). ¹³C NMR (CDCl₃, 125 MHz) δ 157.44, 145.69, 140.24, 126.96, 126.16, 122.99, 60.30, 42.60 MS calc'd for [C₈H₈N₂O] 148.06, found 149.1 (ESI +).
**3-pyridyl acetaldehyde cyanohydrin:** ¹H NMR (CDCl₃, 500 MHz) δ 8.62 (d, 1H, *J* = 1.8), 8.57 (d, 1H, *J* = 5.1), 7.94 (d, 1H, *J* = 8.1), 7.41 (dd, 1H, *J* = 8.1, 5.1), 5.64 (s, 1H). ¹³C NMR (CDCl₃, 125 MHz) δ: 149.921, 147.355, 135.412, 133.044, 124.443, 118.980, 61.085. Exact Mass calculated for [C₇H₆N₂O]: 134.05, found: 135.2 (ESI +).
**2-thienyl acetaldehyde cyanohydrin**:¹H NMR (CDCl₃, 500 MHz) δ 7.1 (m, 1H), 6.9 (m, 1H), 6.8 (m, 1H), 4.11 (t, *J* = 7.0Hz, 1H), 2.86 (d, *J* = 7.0Hz, 2H). ¹³C NMR (CDCl₃, 125 MHz) δ 127.68, 127.41, 125.58, 124.60, 118.70, 63.25, 44.84.
**3-thienyl acetaldehyde cyanohydrin:** ¹H NMR (CDCl₃, 500 MHz) δ 7.09 (m, 3H), 4.60 (t, *J=* 6.25Hz, 1H), 3.12 (d, *J=* 6.25Hz, 2H). ¹³C NMR (CDCl₃, 125 MHz) δ 129.05 127.16, 125.27, 122.65, 119.87, 61.58, 44.90.
**Preparation of racemic mandelic acids standards from corresponding cyanohydrins:** (Stoughton, R.W. J. Am. Chem. Soc. 1941, 63, 2376) 2-bromomandelonitrile (230 mg, 1.08 mmol) was dissolved in conc. HCl (1 mL) and stirred at room temperature for 18 h and then at 70 °C for 24 h. After cooling, the reaction mixture was extracted with diethyl ether (4 x 2 mL). Organic extracts were, combined, dried over MgSO₄, filtered and concentrated *in vacuo*. 2-bromomandelic acid was isolated as a colorless powder (180 mg, 0.78 mmol, 70 % yield).
**Preparation of racemic aryllactic acids standards from corresponding amino acids:** Phenylalanine (10 mmol, 1.65g) was dissolved in 30 ml 2N H₂SO₄ at room temperature under N₂ (g) atmosphere. Sodium nitrite (1.4 g in 3 ml aqueous solution, 2 eq) solution was added slowly to the reaction mixture over a period of 3-4 hours with vigorous stirring at room temperature under N₂ (g) atmosphere. The reaction mixture was stirred overnight and the phenyllactic acid product was then extracted into diethylether (3 x 30 ml). Combined ether extracts were dried over MgSO₄ and then filtered and concentrated *in vacuo.* (Kenji, I.; Susumu, A.; Masaru, M.; Yasuyoshi, U.; Koki, Y.; Koichi, K. Patent Number, WO0155074, Publication date: 2001-08-02.)

### General Method for Enzymatic Preparation of α-hydroxy acids:

**(*R*)-(-)-Mandelic Acid** To a solution of mandelonitrile (1.005 g, 7.56 mmol) in 150 mL of sodium phosphate (100 mM) buffer at pH 8 with 10% v/v methanol, that had been N₂ (g) sparged, at 37 °C, was added 9 mg of nitrilase 1 (normalized for nitrilase content). The reaction was conducted under N₂ (g) atmosphere on a rotating platform shaker. Reaction progress was monitored by withdrawing aliquots for HPLC analysis. After 3 h incubation, the reaction mixture was acidified to pH 2 with 1 N HCl and extracted with diethyl ether (4 x 50 ml). Organic fractions were concentrated *in vacuo* and then the residue was taken up in 10% sodium bicarbonate solution. This aqueous solutions was then washed with diethyl ether (3 x 50 ml) and then acidified to pH 2 with 1 N HCl and extracted with diethyl ether (3 x 50 ml). Organic fractions were combined, washed with brine, dried over MgSO₄, filtered and then concentrated *in vacuo*. (*R*)*-*(-)-Mandelic acid (933 mg, 6.22 mmol) was isolated as a colorless powder in 86 % yield. ¹H NMR (DMSO-d₆, 500 MHz) δ 12.6 (br, s, 1H) 7.41 (m, 2H), 7.34 (m, 2H), 7.28 (m, 1H), 5.015 (s, 1H). ¹³C NMR DMSO-d₆, 125 MHz) δ 174.083, 140.216, 128.113, 127.628, 126.628, 72.359. MS calc'd for [C₈H₈O₃] 150.07, found 150.9 (ESI +); *ee* = 98 % [HPLC]. [α]²⁰₅₉₈ = -134.6 (c = 0.5, methanol).
**(-)-2-chloromandelic acid** ¹H NMR (DMSO-d₆, 500 MHz) δ 7.75 (m, 1H), 7.44 (m, 1H), 7.34 (m, 2H), 5.34 (s, 1H). ¹³C NMR (DMSO, 298K, 125MHz) δ 173.07 137.985, 132.105, 129.399, 129.158, 128.705, 127.235. MS calc'd for [C₈H₇ClO₃] 186.0, found 185.0 (LC-MS -). *ee* = 96 % [HPLC]. 92 % yield. [α]²⁰₅₉₈ = -137.6 (c= 0.5, ethanol).
**(-)-2-bromomandelic acid** ¹H NMR (DMSO-d₆, 500 MHz) δ 7.60 (d, *J* = 7.93, 1H), 7.48 (m, 1H), 7.40 (m, 1H), 7.25 (m, 1H), 5.30 (s, 1H). ¹³C NMR DMSO-d₆, 125 MHz) δ 172.994, 139.61, 132.355, 129.652, 128.753, 127.752, 122.681, 71.644. MS calc'd for [C₈H₇BrO₃] 230.0, found 230.9. *ee* =I 96% [HPLC]. 92% yield. [α]²⁰₅₉₈ = - 116.4 (*c*= 0.5, ethanol).
**(-)-2-methylmandelic acid** ¹H NMR (DMSO-d₆, 500 MHz) δ 11.78 (bs, 1H) 7.38 (m, 1H), 7.16-7.38 (m, 3H), 5.18 (s, 1H), 2.35 (s, 3H). ¹³C NMR DMSO-d₆, 125 MHz) δ 174.229, 138.623, 135.649, 130.129, 127.491, 126.990, 125.698, 125.698, 69.733, 18.899. MS calc'd for [C₉H₁₀O₃] 166.1, found 165.2. *ee* = 91 % [HPLC]. 86 % yield. [α]²⁰₅₉₈ = -164.4 (c = 0.5, ethanol).
**(-)-3-chloromandelic acid** ¹H NMR (DMSO-d₆, 500 MHz) δ 7.46 (s, 1H), 7.36 (m, 3H), 5.07 (s, 1H). ¹³C NMR (DMSO, 298K, 125MHz) δ 173.554, 142.685, 132.813, 130.069, 127.568, 126.355, 125.289,71.659. MS calc'd for [C₈H₇ClO₃] 186.0, found 185.34 (MALDI TOF -). *ee* = 98 % [HPLC]. 70 % yield. [α]²⁰₅₉₈ = -120.48 (c= 0.5, methanol).
**(-)-3-bromomandelic acid** ¹H NMR (DMSO-d₆, 500 MHz) δ 7.60 (s, 1H), 7.49 (m, 1H), 7.42 (m, 1H), 7.31 (m, 1H), 5.06 (s, 1H). ¹³C NMR (DMSO, 298K, 125MHz) δ 173.551, 142.917, 130.468, 130.379, 129.237, 125.687, 121.404, 71.605. MS calc'd for [C₈H₇BrO₃] 229.98, found 229.1 (LC-MS). *ee* = 98 % [HPLC]. 82 % yield. [α]²⁰₅₉₈ = -84.8 (c= 0.5, ethanol).
**(-)-4-fluoromandelic acid** ¹H NMR (DMSO, 298K, 500MHz) δ 12.65 (s, 1H), 7.44 (m, 2H), 7.17 (m, 2H), 5.91 (s, 1H), 5.03 (s, 1H) ¹³C NMR (DMSO, 298K, 125MHz) δ 173.93, 162.57, 136.47, 128.61, 128.55, 114.96, 114.80, 71.61. MS calc'd for [C₈H₇FO₃] 170.0, found 168.8. ee =99% [HPLC]. 81% yield. [α]²⁰₅₉₈ = -152.8 (c= 0.5, methanol).
**(-)-1-naphthylglycolic acid** ¹H NMR (DMSO-d₆, 500 MHz) δ 8.28-8.26 (m, 1H), 7.87-7.93 (m, 2H), 7.47-7.58 (m, 4H), 5.66 (s, 1H). ¹³C NMR DMSO-d₆, 125 MHz) δ 174.288, 136.284, 133.423, 130.654, 128.353, 128.192, 125.926, 125.694, 125.613, 125.266, 124.558, 70.940. MS calc'd for [C₁₂H₁₀O₃]: 202.21 found 201.37 (MALDI TOF -). ee = 95% [HPLC]. 90 % yield [α]²⁰₅₉₈ = -115.4 (c = 0.5, ethanol).
**(-)-2-naphthylglycolic acid** ¹H NMR (DMSO-d₆, 500 MHz) δ 12.6 (bm, 1H), 7.88-7.93 (m, 4H), 7.48-7.56 (m, 3H), 5.20 (s, 1H). ¹³C NMR DMSO-d₆, 125 MHz) δ 174.005,137.760,132.644,132.498,127.811, 127.658, 127.506, 127.209, 125.993, 125.334, 124.761, 72.472. MS calc'd for [C₁₂H₁₀O₃] 202.21, found 201.37 (MALDI TOF). *ee* = 98% [HPLC]. 68% yield. [α]²⁰₅₉₈ =-115.4 (c = 0.5, ethanol).
**(-)-3-pyridylglycolic acid** This Reaction was performed in 100 mM ammonium formate buffer at pH 8. To isolate the product, the reaction mixture was filtered through a 10,000 MWCO membrane to remove enzyme and then concentrated *in vacuo*. ¹H NMR (DMSO-d₆, 500 MHz) δ 8.56 (s, 1H), 8.36 (d, *J* = 4.57 Hz, 1H), 8.25 (s, 1H), 7.71 (m, 1H), 7.25 (dd, *J* = 4.98, 4.80 Hz 1H), 5.45 (s, 1H). ¹³C NMR DMSO-d₆, 125 MHz) δ 165.911, 147.862, 147.251, 139.118, 133.381, 122.746, 71.508. MS calc'd for [C₇H₇NO₃] 153.04, found 154.0 ((MALDI TOF). *ee* = 92% [HPLC], 84% yield, [α]²⁰₅₉₈ = -65.2 (*c* = 0.5, H₂O).
**(-)-3-thienylglycolic acid** ¹H NMR (DMSO-d₆, 500 MHz) δ 7.48 (m, 1H), 7.45 (d, *J*= 2.81, 1H,), 7.10 (m, 1H), 5.09 (s, 1H), 3.33 (s, 1H) ¹³C NMR (DMSO, 298K, 125MHz) δ 173.704, 141.109, 126.446, 126.042, 122.247, 68.915 MS calc'd for [C₆H₆O₃S] 158.00, found 157.224 (MALDI TOF). *ee* = 95 % [HPLC]. 70 % yield. [α]²⁰₅₉₈ = -123.28 (c= 0.5, methanol).
**(S)-(-)-phenyllactic acid** ¹H NMR (DMSO-d₆, 500 MHz) δ 7.28(m, 5H), 4.17(dd, *J* = 4.5 Hz, *J* = 8.3 Hz, 1H), 2.98(dd, *J* = 4.5 Hz, *J* = 13.7 Hz, 1H), 2.79 (dd, *J* = 8.3 Hz, *J*= 13.7 Hz, 1H). ¹³C NMR (DMSO, 298K, 125MHz) δ 178.16, 133.4, 129.27, 128.6, 127.3, 70.45, 44.12. *ee* = 97 % [HPLC], 84 % yield. [α]²⁰₅₉₈ = -17.8 (c= 0.5, methanol).
**(-)-2-methylphenyllactic acid** ¹H NMR (DMSO-d₆, 500 MHz) δ 7.16 (m, 4H), 4.47 (dd, *J* = 3.9 Hz, *J* = 8.8 Hz, 1H), 3.25 (dd, *J* = 3.9Hz, 14.3 Hz, 1H), 2.94 (dd, *J* = 8.8 Hz, *J* = 14.3Hz), 2.35(s, 3H). ¹³C NMR (DMSO, 298K, 125MHz) δ 178.61, 137.08, 134.74, 130.80, 130.25, 127.44, 126.34, 70.93, 37.67, 19.79. MS calc'd [C₁₀H₁₂O₃] 180.08, found 180.0 (ESI +). 86 % yield. *ee* = 95 % [HPLC]. [α]²⁰₅₉₈= -13.2 (*c*= 0.5, methanol).
**(-)-2-bromophenyllactic acid** ¹H NMR (DMSO-d₆, 500 MHz) δ 7.28 (m, 4H), 4.60(dd, *J* = 4.0 Hz, *J* = 9.1 Hz, 1H), 3.45 (dd, *J =* 4.0 Hz, *J =* 14.1 Hz, 1H), 3.04(dd, *J* = 8.0 Hz, *J*= 14.1 Hz, 1H). ¹³C NMR (DMSO, 298K, 125MHz) δ 178.70, 136.05, 133.21, 132.10, 128.99, 127.72, 125.0, 70.04, 40.76. MS calc'd for [C₉H₉BrO₃] 243.9, found 243.3 (ESI +). 91 % yield. *ee* = 93 % [HPLC], [α] ²⁰₅₉₈ = -17.6 (c= 0.5, methanol)
**(-)-2-fluorophenyllactic acid** ¹H NMR (DMSO-d₆, 500 MHz) δ 7.10 (m, 4H), 4.64 (t, *J* = 6.8 Hz, 1H), 3.11(d, *J* = 6.8 Hz, 2H). ¹³C NMR (DMSO, 298K, 125MHz) δ 132.18, 131.52, 129.66, 129.03, 128.07, 124.05, 115.8, 63.02, 44.79. MS calc'd for [C₉H₈FNO]: 165.06, found 164.2 (ESI +). 91 % yield. *ee* = 88 % [HPLC]. [α]²⁰₅₉₈ = -14.0 (*c*= 0.5, methanol).
**(-)-3-methylphenyllactic acid** ¹H NMR (DMSO-d₆, 500 MHz) δ 7.18 (m, 1H), 7.02 (m, 3H), 4.54 (dd, *J* = 4.6 Hz, *J* = 8.0 Hz, 1H), 3.06(dd, *J* = 4.54 Hz, *J* = 14.4 Hz, 1H), 2.83(dd, *J* = 8.0 Hz, *J* = 14.4 Hz, 1H), 2.36 (s, 3H). ¹³C NMR (DMSO, 298K, 125MHz) δ 175.88, 163.80, 130.33, 130.09, 125.7, 116.68, 113.75, 71.31, 34.28. MS calc'd for [C₁₀H₁₁NO] 161.08, found 162.2 (ESI +). 80 % yield. *ee* = 98 % [HPLC]. [α]²⁰₅₉₈ = -2.4 (*c*= 0.5, methanol).
**(-)-3-fluorophenyllactic acid** ¹H NMR (DMSO-d₆, 500 MHz) δ 7.2 (m, 1H), 6.9 (m, 3H), 4.56 (dd, 4.5 Hz, *J* = 7.9 Hz, 1H), 3.09(dd, *J* = 4.5 Hz, *J* = 14.1 Hz, 1H), 2.86 (dd, *J* = 7.9 Hz, *J* = 14.1 Hz, 1H). ¹³C NMR (DMSO, 298K, 125MHz) δ 175.88, 163.80, 130.33, 130.09, 125.7, 116.68, 113.75, 71.31, 34.28. MS calc'd for [C₉H₉O₃F] 184.05, found 184.1 (ESI +). 82 % yield. *ee* = 97 % [HPLC]. [α]²⁰₅₉₈= - 5.2 (*c*= 0.5, methanol).
**(-)-1-napthyllactic acid** ¹H NMR (DMSO-d₆, 500 MHz) δ 8.57 (m, 1H), 8.21(m, 1H), 8.08 (m, 1H), 7.61 (m, 4H), 4.64 (dd, 3.5 Hz, 8.5 Hz, 1H), 3.84 (dd, *J* = 3.5 Hz, *J* = 14.5 Hz, 1H), 3.38 (dd, *J* = 8.5 Hz, *J* = 14.5 Hz, 1H) ¹³C NMR (DMSO, 298K, 125MHz) δ 177.7, 140.31, 129.74, 129.24, 128.92, 128.26, 127.84, 125.63, 124.53, 124.05, 123.42, 70.58, 38.0. MS calc'd for [C₁₃H₁₁NO] 197.08, found 197.1(ESI +). 87 % yield. *ee* = 94 % [HPLC]. [α]²⁰₅₉₈ = -16.2 (c= 0.5, methanol).
**(-)-2-pyridyllactic acid** ¹H NMR (DMSO-d₆, 500 MHz) δ 8.49 (m, 1H), 7.62 (m, 1H), 7.21 (m, 2H), 4.50 (t, *J* = 5.0 Hz, 1H), 3.01 (d, *J* = 5.0 Hz, 2H). ¹³C NMR (DMSO, 298K, 125 MHz) δ 178.8, 159.79, 148.84, 136.89, 124.35, 121.75, 71.14, 4.4.09. MS calc'd for [C₈H₉NO3]: 167.06, found 167.0. (ESI +). 62 % yield. *ee* = 94 % [HPLC], [α]²⁰₅₉₈ = -3.6 (*c*= 0.5, methanol).
**(-)-3-pyridyllactic acid** ¹H NMR (DMSO-d₆, 500 MHz) δ 8.43(m, 2H), 7.62(m, 1H), 7.28(m, 1H), 4.57(t, 5.37Hz, 1H), 2.85(d, 5.37Hz, 2H). ¹³C NMR (DMSO, 298K, 125 MHz) δ 176.6, 150.03, 147.12, 136.41, 129.45, 123.26, 61.56, 31.46 MS calc'd for [C₈H₉NO₃] 167.06, found 167.0 (ESI +). 59 % yield. *ee* = 94 % [HPLC]. [α]²⁰₅₉₈ = -4.0 (*c* = 0.5, methanol).
**(-)-2-thienyllactic acid** ¹H NMR (DMSO-d₆, 500 MHz) δ 7.18(m, 1H), 6.94(m, 1H), 6.90 (m, 1H), 4.49 (dd, *J* = 4.1 Hz, *J* = 6.25 Hz, 1H), 3.36 (dd, *J* = 4.1 Hz, *J* = 15.0 Hz, 1H), 3.26(dd, *J* = 6.25 Hz, *J* = 15.0 Hz, 1H). ¹³C NMR (DMSO, 298K, 125MHz) δ 127.68, 127.41, 125.58, 124.60, 118.70, 63.25, 44.84. MS calc'd for [C₇H₇NOS] 153.02, found 153.0 (ESI +). 85 % yield. *ee* = 95 % [HPLC]. [α]²⁰₅₉₈ = - 13.0 (c= 0.5, methanol).
**(-)-3-thienyllactic acid** ¹H NMR (DMSO-d₆, 500 MHz) δ 7.30(m, 1H), 7.13(m, 1H), 7.01(m, 1H), 4.50 (dd, *J* = 4.25 Hz, *J* = 6.5 Hz, 1H), 3.21(dd, *J* = 4.25 Hz, *J* = 15.0 Hz, 1H), 3.10 (dd, *J* = 6.5 Hz, *J* = 15.0 Hz, 1H). ¹³C NMR (DMSO, 298K, 125MHz) δ 127.50, 136.09, 128.83, 126.24, 123.32, 70.65, 34.84. MS calc'd for [C₇H₈O₃S] 172.02, found 172.1 (ESI +). 81 % yield. *ee* = 96 % [HPLC]. [α]²⁰₅₉₈ = - 18.8 (*c*= 0.5, methanol).

### Enzymic Hydrolysis of 3-Hydroxyglutarylnitrile:

3-Hydroxyglutarylnitrile (1.0 g, 9.0 mmol, 240 mM) was suspended in N₂ (g) sparged sodium phosphate buffer (37.5 mL, pH 7, 100 mM) at room temperature. Cell lysate (30 mg, normalized for nitrilase content) was added to bring the concentration to 0.8 mg/ml enzyme and the reaction was at shaken at 100 rpm, room temperature. Reaction progress was monitored by TLC (1:1 EtOAc:Hexanes, R_{f}=0.32, nitrile; R_{f}=0.0, acid) After 22 h, the reaction was acidified with 1M HCl. The reaction mixture was continuously extracted with diethyl ether. The acid product was isolated as a yellow oil (1.15 g, 98 % yield). ¹H NMR (DMSO, 298K, 500MHz) δ 12.32 (s, 1H), 5.52 (s, 1H), 4.10 (m, 1H), 2.70 (dd, 1H, J = 16.8, 4.1 Hz), 2.61 (dd, 1H, J = 16.9, 6.3 Hz), 2.44 (dd, 1H, J = 15.4, 5.3 Hz), 2.37 (dd, 1H, J = 15.6, 7.8 Hz). ¹³C NMR (DMSO, 298K, 125 MHz) δ 171.9, 118.7, 63.4, 41.2, 25.2 MS calc'd for [C₅H₇NO₃]: 129.0, found 130.0 [M+H⁺], (ESI +).

### Preparation of (R)-(-)-Methyl (3-O-[benzoyl]-4-cyano)-butanoate

Benzoyl chloride (0.068 ml, 0.752 mmol) was added to a stirred solution of (*R*)-methyl-(3-hydroxy-4-cyano)-butanoate (71.7 mg, 0.501mmol) in pyridine (2.0 ml), at room temperature. After 19 hours, add an additional 0.5 equivalent of benzoyl chloride (0.023ml, 0.251mmol). Reaction was complete at 23 h, as determined by TLC. Add 1ml H₂O, extract with ether (3 x 10ml). Wash with brine (2 x 10ml). Dry combined aqueous extracts with MgSO₄. Filter off drying agent and remove solvent by rotary evaporation. Purify by column chromatography (hexane:ethyl acetate [2:1]. Rotary evaporation of fractions yielded the product as a yellow oil (46 mg, 0.186 mmol, 37%). ¹H NMR (DMSO, 298K, 500MHz) δ 7.96 (d, 2H, J = 7.8), 7.70 (t, 1H, J = 7.25), 7.56 (t, 2H, J = 7.8), 5.55 (m, 1H), 3.59 (s, 3H), 3.13 (m, 2H), 2.90 (m, 2H). ¹³C NMR (DMSO, 298K, 125MHz) δ 169.6, 164.5, 133.8, 129.3, 128.9, 128.5, 117.3, 66.0, 51.8, 37.5, 22.2 MS calc'd for [C₁₃H₁₃NO₄]: 247.25, found 270.3 [M+Na⁺] *ee* = 95% [HPLC]. [α]²⁰₅₉₈ -32.4 (c = 0.5, CHCl₃).

### Synthesis of (R)-Ethyl-(3-hydroxy-4cyano)-butanoate

A 0.2 M solution of (R)-3-hydroxy-4-cyano-butanoic acid (50 mg, 0.387 mmol) in anhydrous ethanol (1.94 mL) was prepared. The ethanol solution was added dropwise to 1.0 ml of a 50:50 (v/v) mixture of anhydrous 1 M HCl ethereal solution and anhydrous ethanol over sieves. The reaction was stirred overnight at room temperature under N₂ (g) atmosphere. The reaction was monitored by TLC, (1:1 EtOAc:Hexanes, R_{f} = 0.45, ester; R_{f} = 0.0, acid, stained with p-anisaldehyde). After 30 hrs, solvent was removed by rotary evaporation. The crude product was taken up in 25 mL ether, washed with 5 mL saturated bicarbonate and then 5 mL brine. The organic extract was dried over MgSO₄, filtered and then concentrated *in vacuo*, yielding the product as a clear oil. ¹H NMR (DMSO, 298K, 500MHz) δ 5.60 (d, 1H, *J*= 5.58 Hz), 4.12 (m, 1H), 4.07 (q, 2H, *J* = 7.1), 2.66 (m, 2H), 2.47 (m, 2H), 1.87 (t, 3H, *J* = 7.0). ¹³C NMR (DMSO, 298K, 125 MHz) δ 170.21, 118.60, 63.40, 59.98, 41.10, 25.14, 14.02. MS calc'd for [C₇H₁₁NO₃]: 157.1, found 158.2. [M+H⁺]

### Example 13 - Optimization Of Nitrilases For The Enantioselective Production Of (R)-2-Chloromandelic Acid

Chloromandelic acid has the structure:

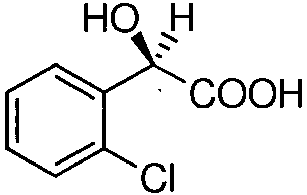

Nitrilases were identified which selectively produced (R)-2-chloromandelic acid from (R,S)-2-chloromandelonitrile. Nitrilases were identified which were useful to improve the enantioselectivity of the enzymes and establishing the effects of process conditions on the enzymes. An examination of the reaction conditions for the enzymatic nitrile hydrolysis was carried out in order to improve the enantiomeric excess of the product. Additionally, further investigation into the effects of process conditions on the enzyme was performed.

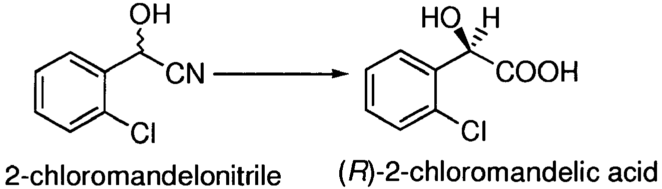

In this embodiment, the enantioselective production of (R)-2-chloromandelic acid was the target. One enzyme, SEQ ID NOS:385, 386, was selected for further confirmation of its enantioselectivity on 2-chloromandelonitrile. SEQ ID NOS:385, 386 was shown to be stable to process components, with a half-life of 8 hours. The enzyme was inhibited by 2-chlorobenzaldehyde and a contaminant in the cyanohydrin substrate, 2-chlorobenzoic acid. The enzymatic reaction was scaled up to a substrate concentration of 45 mM 2-chloromandelonitrile. Over 90% conversion was obtained, with *ee* of 97%. The chiral HPLC method was improved, to remove a contaminating peak that was present in the substrate. Improved accuracy in the determination of enantioselectivity was obtained using this method.

Nitrilases were screened against 2-chloromandelonitrile, with 31 nitrilases exhibiting activity on this substrate. High enantioselectivities were shown by 9 enzymes. The optimization of 5 of these enzymes was undertaken and one of them was identified as a candidate for the next stage of development.

In an effort to improve the enantioselectivity of the selected enzymes for (R)-2-chloromandelic acid, a number of factors that are known to affect this property, together with the activity of the enzymes, were investigated. These included pH, temperature, buffer strength and addition of solvents to the reaction. Initially, 5 nitrilases were selected for these studies, based on the high enantioselectivities obtained by these enzymes. These enzymes were: SEQ ID NOS:385, 386, SEQ ID NOS:197, 198, SEQ ID NOS:217, 218, SEQ ID NOS:55, 56, and SEQ ID NOS:167, 168.

### Effect of pH

The enzymatic reactions were run at a range of pH values, from pH 5 to pH 9. An increase in both activity and enantioselectivity with increasing pH was observed for all of the enzymes. With the exception of SEQ ID NOS:385, 386, pH 9 (0.1 M Tris-HCl buffer) was determined as the optimum for activity and enantioselectivity. The optimum pH for SEQ ID NOS:385, 386 was pH 8 (0.1 M sodium phosphate buffer).

### Effect of temperature

The enzymes exhibited similar temperature profiles, with the highest activities being measured at 37°C and 45°C. Although the latter temperature resulted in higher conversions, the enantioselectivity of most of the enzymes showed a clear preference for the lower temperatures, with ee values being 10-20% lower when the temperature was raised above 37°C. In the case of SEQ ID NOS:385, 386 a slight optimum for enantioselectivity was evident at 37°C. Therefore, this temperature was established as the optimum for hydrolysis of 2-chloromandelonitrile by these enzymes.

### Effect of enzyme concentration

During the concurrent investigation into the enantioselective hydrolysis of phenylacetaldehyde cyanohydrin to L-phenyllactic acid, the concentration of the enzyme in the reaction was found to have a significant effect on the enantioselectivity of the reaction. This provided an indication that the enzymatic hydrolysis rate was faster than the rate of racemization of the remaining cyanohydrin in the reaction. On this basis, the effect of enzyme concentration on the enantioselectivity of the enzymes towards (*R*)-2-chloromandelonitrile was investigated. Enzymatic reactions were performed with the standard concentration of enzyme (0.6 mg protein/ml), half the standard concentration and one-tenth of the standard concentration.

The following Table indicates the highest conversions achieved for the reactions, with the corresponding ee. With the exception of SEQ ID NOS:385, 386, it appears that very little, if any, increased enantioselectivity is observed. Therefore, it appears that the rate of racemization of the remaining chloromandelonitrile is not a limiting factor to obtaining higher enantioselectivities.

### Effect of enzyme concentration on the activity and enantioselectivity of nitrilases for the production of (R)-2-chloromandelic acid.

| | **Enzyme conc (mg protein/ml)** | **Conversion to product (%)** | **Time for highest conversion (h)** | **ee (%)** |
|---|---|---|---|---|
| SEQ ID NOS:385, 386 | 0.06 | 67 | 3 | 92 |
| | 0.3 | 80 | 1 | 82 |
| | 0.6 | 81 | 1 | 82 |
| SEQ ID NOS: 197, 198 | 0.06 | 3 | 5 | 100 |
| | 0.3 | 46 | 4 | 88 |
| | 0.6 | 75 | 3 | 86 |
| SEQ ID | 0.06 | 82 | 4 | 88 |
| NOS:189, 190 | | | | |
| | 0.3 | 80 | 1 | 86 |
| | 0.6 | 76 | 0.5 | 82 |
| SEQ ID NOS:217, 218 | 0.06 | 22 | 5 | ND |
| | 3.3 | 73 | 4 | 82 |
| | 3.6 | 72 | 1.5 | 81 |
| SEQ ID NOS:55, 56 | 0.06 | B1 | 5 | 84 |
| | 0.3 | 73 | 2 | 79 |
| | 0.6 | 67 | 0.5 | 76 |
| SEQ ID NOS:167,168 | 0.06 | 61 | 2 | 81 |
| | 0.3 | 74 | 1.5 | 80 |
| | 0.6 | 74 | 2 | 78 |

### Investigation of other positive enzymes

In addition to the enzymes in the above Table, a number of other nitrilases were screened for their enantioselectivities on 2-chloromandelonitrile. Some of these enzymes were newly discovered enzymes. Some were reinvestigated under conditions that have since been found to be optimal for these enzymes (pH 8 and 37°C). The results of this screening are shown below in the Table.

### Summary of enzymes screened for activity and enantioselectivity on 2-chloromandelonitrile

| **Enzyme** | **Conversion to product (%)** | **Time for highest conversion (h)** | **ee (%)** |
|---|---|---|---|
| SEQ ID NOS:383, 384 | 61 | 6 | 78 |
| SEQ ID NOS:101, 102 | 58 | 3 | 53 |
| SEQ ID NOS:97, 98 | 46 | 3 | 87 |
| SEQ ID NOS:13, 14 | 70 | 3 | 71 |
| SEQIDNOS:5,6 | >95 | 3 | 67 |
| SEQ ID NOS:85, 86 | 50 | 4 | 52 |
| SEQ ID NOS:279, 280 | >95 | 1 | 37 |
| SEQ ID NOS:33, 34 | >95 | 3 | 60 |
| SEQ ID NOS:261, 262 | >95 | 3 | 70 |

### Effect of co-solvent concentration

The addition of methanol as a cosolvent in the enzymatic reactions was shown to enhance the ee. In order to establish the lowest level of methanol that could be added to the reactions, the enzyme reactions were performed at varying concentrations of methanol, ranging from 0-20% (v/v). No significant differences in enantioselectivity were evident between the various methanol concentrations. However, the ee in these reactions was 97-98%, while that of the control reaction, with no added methanol was 95-96%. While this difference in ee is small, the effect of the methanol was shown in more than one set of experiments during the course of this investigation and is therefore regarded as significant.

### Effect of reaction components on activity of SEQ ID NOS:385, 386

A vital part of an investigation into process optimization of an enzyme involves the determination of the effects of any compounds which could be present in the enzymatic reaction. For SEQ ID NOS:385, 386, these components were established as the starting material and equilibrium product of the cyanohydrin, 2-chlorobenzaldehyde; the product, 2-chloromandelic acid and the contaminant detected in the substrate, 2-chlorobenzoic acid. The addition of cyanide to the reaction was found to have no effect on the enzyme activity. The presence of trace amounts of triethylamine was also found to be tolerable to the enzyme.

The effect of the various reaction components on the activity of SEQ ID NOS:385, 386 was assessed by addition of various levels of possible inhibitors to the enzyme reaction. From these experiments, it appeared that both the aldehyde and its oxidation product, 2-chlorobenzoic acid were detrimental to enzyme activity. Approximately 70% and 40% of the activity of SEQ ID NOS:385, 386 was lost upon addition of 5 mM 2-chlorobenzaldehyde or 5 mM 2-chlorobenzoic acid to the reaction, respectively.

### Scale-up hydrolysis of 2-chloromandelonitrile

In order to confirm the conversion and enantioselectivity obtained by SEQ NOS:385, 386 for the production of (R)-2-chloromandelic acid, a larger scale reaction was performed and the product isolated from the aqueous mixture. The reaction was performed in a 20 ml reaction volume, with a substrate concentration of 45 mM 2-chloromandelonitrile. Complete conversion of the cyanohydrin was obtained, with 30 mM product formed. The ee of the product was 97% and the specific activity of the enzyme was 0.13 mmol product/mg nitrilase/h.

It is evident from this experiment, together with the other experiments performed, that the formation of product does not account for the complete loss of substrate. In all experiments, a nitrile-containing control sample was run, in order to determine the extent of breakdown of the cyanohydrin. Overall, it appears that approximately 50% of the substrate is lost over a period of 4 hours at 37°C. It is expected that this breakdown would be to its equilibrium products, cyanide and 2-chlorobenzaldehyde, which could undergo further oxidation. A larger scale reaction was also run at a substrate concentration of 90 mM 2-chloromandelonitrile. However, no product was detected in this reaction. At higher substrate concentrations, it is expected that the concentration of the equilibrium product, 2-chlorobenzaldehyde and the contaminant, 2-chlorobenzoic acid will be present in higher amounts. Based on the results above, it is possible that the enzyme will be completely inhibited under such conditions.

### Reactions under biphasic conditions

The use of biphasic systems can facilitate product recovery following the enzymatic reaction step. These systems can be also be used for the removal of products or by-products which are inhibitory to the enzyme. The nitrilases were shown to be active under biphasic conditions using a variety of solvents. Following the low conversions obtained at the higher substrate concentration above, further investigation of a biphasic system was performed with the hit enzyme, SEQ ID NOS:385, 386. It was important to ascertain whether any inhibitory factors could be removed by the solvent phase and whether any process advantages could be gained by the use of a biphasic system.

Promising results were obtained with hexane as the organic phase. Therefore, further investigations involved the use of this solvent at two different levels: 100% and 70% of the volume of the aqueous phase, with increasing substrate concentrations, up to 90 mM. The substrate was dissolved in the organic phase. The level of hexane did not appear to affect the level of product formation, particularly at the higher concentrations of 2-chloromandelonitrile.

Once again, high conversion was observed in a biphasic system, with a 76% yield of product being observed after 5 hours. The rate of product formation appeared to be slightly lower than in the corresponding monophasic system, where the reaction is complete within 1 hour. Lower enantioselectivity was observed in the biphasic system. Some possibilities which may account for these results are (i) the mass transfer rate is lower than the rate of enzyme activity or (ii) the non-polar solvent directly affects the enzyme.

At a higher substrate concentration, a very low conversion was observed, with 7 mM 2-chloromandelic acid being formed from 90 mM 2-chloromandelonitrile. This level of conversion, albeit low, was higher than that observed in the monophasic system with the same substrate concentration. These results suggest that some of the inhibitory 2-chlorobenzaldehyde or 2-chlorobenzoic acid is retained in the non-polar organic solvent.

### Standard assay conditions:

The following solutions were prepared:
- Substrate stock solution: 50 mM of the cyanohydrin substrate in 0.1 M phosphate buffer (pH 8).
- Enzyme stock solution: 3.33 ml of 0.1 M phosphate buffer (pH 8) to each vial of 20 mg of lyophilized cell lysate (final concentration 6 mg protein/ml)

The reaction volumes varied between the different experiments, depending on the number of time points taken. Unless otherwise noted, all reactions consisted of 25 mM 2-chloromandelonitrile and 10% (v/v) of the enzyme stock solution (final concentration 0.6 mg protein/ml). The reactions were run at 37°C, unless otherwise stated. Controls to monitor the nitrile degradation were run with every experiment. These consisted of 25 mM 2-chloromandelonitrile in 0.1 M phosphate buffer (pH 8).

Sampling of reactions: The reactions were sampled by removing an aliquot from each reaction and diluting these samples by a factor of 8. Duplicate samples were taken for analysis by chiral and achiral HPLC methods. The reactions were sampled at 0.5, 1, 1.5, 2, 3, and 4 hours, unless otherwise shown in the figures above.

### HPLC methods

The achiral HPLC method was run on a SYNERGI-RP™ column (4 µm; 50 x 2 mm) with a mobile phase of 10 mM Na phosphate buffer (pH 2.5). A gradient of methanol was introduced at 3.5 min and increased to 50% over 1.5 min, following which the methanol was decreased to 0%. Elution times for 2-chloromandelic acid and 2-chloromandelonitrile were 2.5 and 6.1 minutes, with another peak appearing with the nitrile at 5.9 minutes.

As described above, the chiral HPLC method was optimized during the course of the investigation, to improve the separation between 2-chlorobenzoic acid and (S)-2-chloromandelic acid. The optimized method was used during the latter half of the investigation and was run on a CHIROBIOTIC-R™ column. The mobile phase was 80% Acetonitrile:20% of 0.5% (v/v) acetic acid. Elution times for (S)-2-chloromandelic acid and (R)-2-chloromandelic acid were 2.4 and 3.5 minutes respectively. A peak for 2-chlorobenzoic acid eluted at 1.9 minutes. For each experiment, a standard curve of the product was included in the HPLC run. The concentration of product in the samples was calculated from the slope of these curves.

### Effect of pH

The effect of pH on the enzyme activity and enantioselectivity was studied by performance of the standard assay in a range of different buffers: 0.1 M Citrate Phosphate pH 5; 0.1 M Citrate Phosphate pH 6; 0.1 M Sodium Phosphate pH 6; 0.1 M Sodium Phosphate pH 7; 0.1 M Sodium Phosphate pH 8; 0.1 M Tris-HCl pH 8; and 0.1 M Tris-HCl pH 9. The standard enzyme concentration was used for all enzymes, with the exception of SEQ ID NOS:385, 386, where half the standard concentration was used (5% v/v of the enzyme stock solution).

### Effect of temperature

The effect of temperature on the activity and enantioselectivity was investigated by performing the standard assay at a range of different temperatures: room temperature, 37°C, 45°C, 50°C and 60°C. The standard enzyme concentration was used for all enzymes, with the exception of SEQ ID NOS:385, 386, where half the standard concentration was used (5% v/v of the enzyme stock solution).

### Effect of enzyme concentration

Reactions were run under standard conditions, with varying enzyme concentrations: 1%, 5% and 10% (v/v) of the enzyme stock solution. The reaction volume was normalized with the appropriate buffer.

### Addition of solvents

The enzyme reactions were performed in the presence of methanol as a cosolvent. Methanol was added to the standard reaction mixture at the following levels: 0, 5, 10, 15 and 20% (v/v).

Biphasic reactions with hexane were also investigated. The aqueous phase contained 10% (v/v) of the enzyme stock solution in 0.1 M phosphate buffer (pH 8). The cyanohydrin was dissolved in the hexane, prior to addition to the reaction. Two levels of organic phase were used: 1 equivalent and 0.7 equivalents of the aqueous phase volume. In addition, a range of nitrile concentrations was investigated: 25, 45 and 90 mM. These reactions were run at room temperature.

Samples from these reactions were taken both from the aqueous and the solvent phase. The hexane was evaporated by centrifugation under vacuum and redissolved in a 50:50 mixture of methanol and water, so that the samples were at the same dilution as the aqueous samples. Analysis of the samples was performed by non-chiral and chiral HPLC.

### Effect of process components

(i) Activity: The effect of the process components on the activity of the enzymes was established by addition of the individual components, 2-chlorobenzaldehyde, 2-chlorobenzoic acid or 2-chloromandelic acid, to the enzymatic reaction. The enzymatic reactions were carried out under standard conditions, in the presence of one of the 2 possible inhibitors as follows: 5, 10, 20 and 25 mM 2-chlorobenzaldehyde; 1.5 and 5 mM 2-chlorobenzoic acid; and 10, 20, 40 and 80 mM 2-chloromandelic acid. Control reactions were performed under standard conditions, with no additive. At each of the sampling times, the samples were diluted to a level of 1 in 10. Control samples containing the reaction components without enzyme were used and diluted to the same level. The samples were analysed by non-chiral HPLC.
(ii) Stability: The stability of the enzymes to process conditions was monitored by incubation of the enzymes in the presence of the reaction components, 2-chlorobenzaldehyde and 2-chloromandelic acid for predetermined time periods, prior to assay of the enzyme activity under standard conditions. In these experiments, the enzymes were incubated at a concentration of 3 mg protein/ml in the presence of each of the following reaction components: 5, 10, 20 and 25 mM 2-chlorobenzaldehyde; and 10, 20, 40 and 80 mM 2-chloromandelic acid. Control reactions were performed by incubation of the enzyme in buffer only.

Assay conditions: At 0, 4, 8 and 24 hours of incubation in the particular additive, 20 µl of the enzyme solution was removed and added to 60 µl of a 41.6 mM substrate stock solution and 20 µl buffer. The enzyme activity was thus assayed under standard conditions. The reactions were sampled 90 minutes after substrate addition and analyzed using the non-chiral HPLC method.

### Scale-up of enzymatic reaction

The enzymatic reactions were run at two difference concentrations: 45 mM and 90 mM substrate. The reactions were run under standard conditions, i.e. pH 8 (0.1 M sodium phosphate buffer), 37°C and 10% (v/v) of the enzyme stock solution. The substrate was dissolved in 10% (v/v/) methanol prior to addition of the buffer. The final reaction volume was 20 ml and the reactions were performed with magnetic stirring.

### Example 14 - Optimization Of Nitrilases For The Enantioselective Production Of L-2-amino-6,6-dimethoxyhexanoic acid

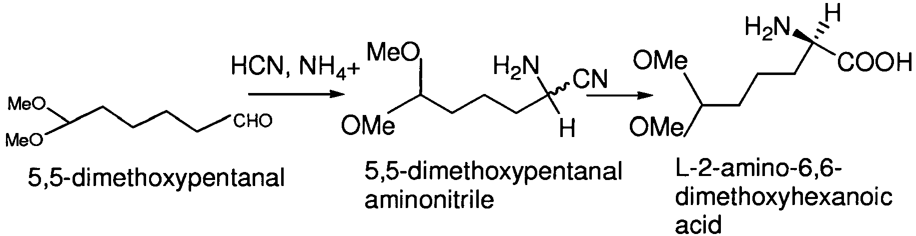

Four of the isolated enzymes were shown to hydrolyze 2-amino-6-hydroxy hexanenitrile to (S)-2-amino-6-hydroxy hexanoic acid, with selectivity towards the L-enantiomer. A new target, with a similar structure to (S)-2-amino-6-hydroxy hexanoic acid was identified. A panel of the isolated nitrilases are screened against the target, 5,5-dimethoxypentanal aminonitrile. The positive enzymes are characterized on this substrate. Laboratory evolution techniques can be used to optimize these nitrilases for improved enantiospecificity towards the specified target. A primary screen is used to identify putative up-mutants, which is confirmed using HPLC.

Optimization of enzymes: GSSM™ and GeneReassembly™ can be performed on selected nitrilases, in order to improve the enantioselectivity and activity of the enzymes for the production of L-2-amino-6,6-dimethoxyhexanoic acid. Four enzymes were identified that can hydrolyze enantioselectively 2-amino-6-hydroxy hexanenitrile to L-(S)-2-amino-6-hydroxy hexanoic acid. However, a slight structural difference is present in the new target molecule, L-2-amino-6,6-dimethoxyhexanoic acid. In order to determine whether this difference affects the activity and enantioselectivity of the enzymes, the complete spectrum of nitrilases is screened against the new target.

An enzyme exhibiting the highest combination of activity and enantioselectivity for the production of L-2-amino-6,6-dimethoxyhexanoic is selected for GSSM™. Following the mutation of the target enzyme, the resulting mutants will be screened on 5,5-dimethoxypentanal aminonitrile, using high throughput screening technology. Following confirmation of the up-mutants by HPLC analysis, the individual up-mutants will be combined in order to further enhance the properties of the mutant enzymes.

In parallel to GSSM™, a GeneReassembly™ can be performed on a combination of parent enzymes, at least one of which can be selected for activity and enantioselectivity on L-2-amino-6,6-dimethoxyhexanoic acid. At least two other nitrilases, with a high degree of homology, can be reassembled with the former enzyme(s); these enzymes will be selected in order to provide diversity to the reassembled sequences.

Crucial to the success of this evolution effort is the development of a high throughput assay for enantioselectivity. Such an assay is a novel enzyme-based enantioselectivity assay that allows for the screening of >30,000 mutants in a significantly shorter time period than the traditionally used method of HPLC.

In one aspect, a non-stochastic method, termed synthetic ligation reassembly, that is related to stochastic shuffling, except that the nucleic acid building blocks are not shuffled or concatenated or chimerized randomly, but rather are assembled non-stochastically, can be used to create variants. This method does not require the presence of high homology between nucleic acids to be shuffled. The ligation reassembly method can be used to non-stochastically generate libraries (or sets) of progeny molecules having at least 10¹⁰⁰ or at least 10¹⁰⁰⁰ different chimeras. The ligation reassembly method provides a non-stochastic method of producing a set of finalized chimeric nucleic acids that have an overall assembly order that is chosen by design, which method is comprised of the steps of generating by design a plurality of specific nucleic acid building blocks having serviceable mutally compatible ligatable ends, as assembling these nucleic acid building blocks, such that a designed overall assembly order is achieved.

The mutually compatible ligatable ends of the nucleic acid building blocks to be assembled are considered to be "serviceable" for this type of ordered assembly if they enable the building blocks to be coupled in predetermined orders. Thus, in one aspect, the overall assembly order in which the nucleic acid building blocks can be coupled is specified by the design of the ligatable ends and, if more than one assembly step is to be used, then the overall assembly order in which the nucleic acid building blocks can be coupled is also specified by the sequential order of the assembly step(s). In a one embodiment of the invention, the annealed building pieces are treated with an enzyme, such as a ligase (*e.g.*, T4 DNA ligase) to achieve covalent bonding of the building pieces.

In a another embodiment, the design of nucleic acid building blocks is obtained upon analysis of the sequences of a set of progenitor nucleic acid templates that serve as a basis for producing a progeny set of finalized chimeric nucleic acid molecules. These progenitor nucleic acid templates thus serve as a source of sequence information that aids in the design of the nucleic acid building blocks that are to be mutagenized, *i.e.* chimerized, recombined or shuffled.

Chimerization may give a family of related genes and their encoded family of related products. In a particular exemplification, the encoded products are nitrilase enzymes. Nucleic acids encoding the nitrilases can be mutagenized in accordance with the methods described herein.

Thus, the sequences of a plurality of progenitor nucleic acid templates encoding nitrilases are aligned in order to select one or more demarcation points, which demarcation points can be located at an area of homology. The demarcation points can be used to delineate the boundaries of nucleic acid building blocks to be generated. Thus, the demarcation points identified and selected in the progenitor molecules serve as potential chimerization points in the assembly of the progeny molecules.

Typically a serviceable demarcation point is an area of homology (comprised of at least one homologous nucleotide base) shared by at least two progenitor templates, but the demarcation point can be an area of homology that is shared by at least half of the progenitor templates, at least two thirds of the progenitor templates, at least three fourths of the progenitor templates, and preferably at almost all of the progenitor templates. Even more preferably still a serviceable demarcation point is an area of homology that is shared by all of the progenitor templates.

In a one embodiment, the ligation reassembly process is performed exhaustively in order to generate an exhaustive library. In other words, all possible ordered combinations of the nucleic acid building blocks are represented in the set of finalized chimeric nucleic acid molecules. At the same time, the assembly order (*i.e.*, the order of assembly of each building block in the 5' to 3' sequence of each finalized chimeric nucleic acid) in each combination is by design (or non-stochastic, non-random). Because of the non-stochastic nature of the method, the possibility of unwanted side products is greatly reduced.

The method provides that, the ligation reassembly process is performed systematically, for example in order to generate a systematically compartmentalized library, with compartments that can be screened systematically, *e.g.*, one by one. Each compartment (or portion) holds chimeras or recombinants with known characteristics. In other words this provides that, through the selective and judicious use of specific nucleic acid building blocks, coupled with the selective and judicious use of sequentially stepped assembly reactions, an experimental design can be achieved where specific sets of progeny products are made in each of several reaction vessels. This allows a systematic examination and screening procedure to be performed. Thus, it allows a potentially very large number of progeny molecules to be examined systematically in smaller groups.

Because of its ability to perform chimerizations in a manner that is highly flexible, yet exhaustive and systematic, particularly when there is a low level of homology among the progenitor molecules, the methods described herein provide for the generation of a library (or set) comprised of a large number of progeny molecules. Because of the non-stochastic nature of the ligation reassembly method, the progeny molecules generated preferably comprise a library of finalized chimeric nucleic acid molecules having an overall assembly order that is chosen by design. In a particularly embodiment, such a generated library is comprised of greater than 10³ to greater than 10¹⁰⁰⁰ different progeny molecular species.

In another exemplification, the synthetic nature of the step in which the building blocks are generated allows the design and introduction of nucleotides (e.g., one or more nucleotides, which may be, for example, codons or introns or regulatory sequences) that can later be optionally removed in an *in vitro* process (*e.g.*, by mutageneis) or in an *in vivo* process (*e.g.*, by utilizing the gene splicing ability of a host organism). It is appreciated that in many instances the introduction of these nucleotides may also be desirable for many other reasons in addition to the potential benefit of creating a serviceable demarcation point.

The synthetic ligation reassembly method utilizes a plurality of nucleic acid building blocks, each of which preferably has two ligatable ends. The two ligatable ends on each nucleic acid building block may be two blunt ends (i.e. each having an overhang of zero nucleotides), or preferably one blunt end and one overhang, or more preferably still two overhangs. On a double-stranded nucleic acid, a useful overhang can be a 3' overhang, or a 5' overhang. A nucleic acid building block can have a 3' overhang, a 5' overhang, two 3' overhangs, or two 5' overhangs. The overall order in which the nucleic acid building blocks are assembled to form a finalized chimeric nucleic acid molecule is determined by purposeful experimental design (*e.g*., by designing sticky ends between building block nucleic acids based on the sequence of the 5' and 3' overhangs) and is not random.

According to one preferred embodiment, a nucleic acid building block is generated by chemical synthesis of two single-stranded nucleic acids (also referred to as single- stranded oligos) and contacting them together under hybridization conditions so as to allow them to anneal to form a double-stranded nucleic acid building block. A double-stranded nucleic acid building block can be of variable size. The sizes of these building blocks can be small or large. Preferred sizes for building block range from 1 base pair (not including any overhangs) to 100,000 base pairs (not including any overhangs). Other preferred size ranges are also provided, which have lower limits of from 1 bp to 10,000 bp (including every integer value in between), and upper limits of from 2 bp to 100, 000 bp (including every integer value in between).

According to one embodiment, a double-stranded nucleic acid building block is generated by first generating two single stranded nucleic acids and allowing them to anneal to form a double-stranded nucleic acid building block. The two strands of a double-stranded nucleic acid building block may be complementary at every nucleotide apart from any that form an overhang; thus containing no mismatches, apart from any overhang(s). The two strands of a double-stranded nucleic acid building block may be complementary at fewer than every nucleotide apart from any that form an overhang. Thus, a double-stranded nucleic acid building block can be used to introduce codon degeneracy. Preferably the codon degeneracy is introduced using the site-saturation mutagenesis described herein, using one or more N,N,GIT cassettes or alternatively using one or more N,N,N cassettes.

### Example 15 - Assays for Evaluation of Nitrilase Activity and Enantioselectivity

An assay method amenable to high throughput automation to increase the screening throughput both of the discovery and evolution efforts for nitrilases is described. The ideal assay is one that permits quantification of both product formation or substrate conversion and also enantiomeric excess. Two achiral and two chiral colorimetric assays that are amenable to high throughput screening were developed.

### Achiral Colorimetic Assays Developed:

OPA assay for residual substrate. The OPA assay is Applicable to α-amino or α-hydroxy nitrile substrates. The lysis of whole cells is not necessary. These results were corroborated by HPLC for 2-chloromandelonitrile and phenyl acetaldehyde cyanohydrin. The assay works best with aromatic nitriles. Aliphatic compounds exhibit a linear standard curve, fluorescence is reduced, reducing the efficacy of the assay.

LDH Assay for quantification and *ee* determination of hydroxyacid formed. The LDH assay is applicable to phenyl lactic acid but not to 2-chloromandelic acid. Use of a resazurin detection system increases sensitivity and reduces background. Background fluorescence of whole cells was overcome either by centrifugation or heat inactivation prior to performing assay.

AAO Assay for quantification and *ee* determination of aminoacid formed. The AAO assay is applicable to phenylalanine and (S)-2-amino-6-hydroxy hexanoic acid. The use of the Amplex Red detection system increases sensitivity. Cell lysis was shown not be necessary. Cells are grown in defined media in order to prevent background fluorescence.

### OPA Assay

The o-phthalaldehyde (OPA) fluorescence based nitrilase assay is used to quantify the amount of α-hydroxynitrile substrate remaining. OPA reacts with the cyanide released from the pH controlled decomposition of α-hydroxynitriles to the corresponding aldehyde and cyanide to yield a fluorescent, quantifiable product. OPA reacts with the cyanide released from the pH controlled decomposition of α-hydroxynitriles to the corresponding aldehyde and cyanide to yield the fluorescent 1-cyano-2-R benzoisoindole.

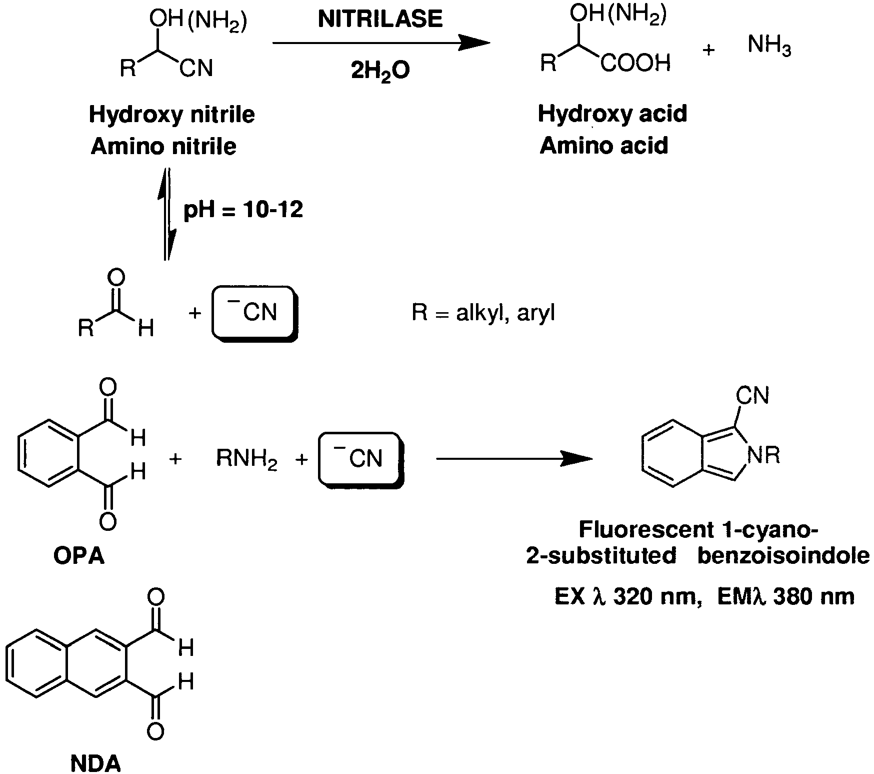

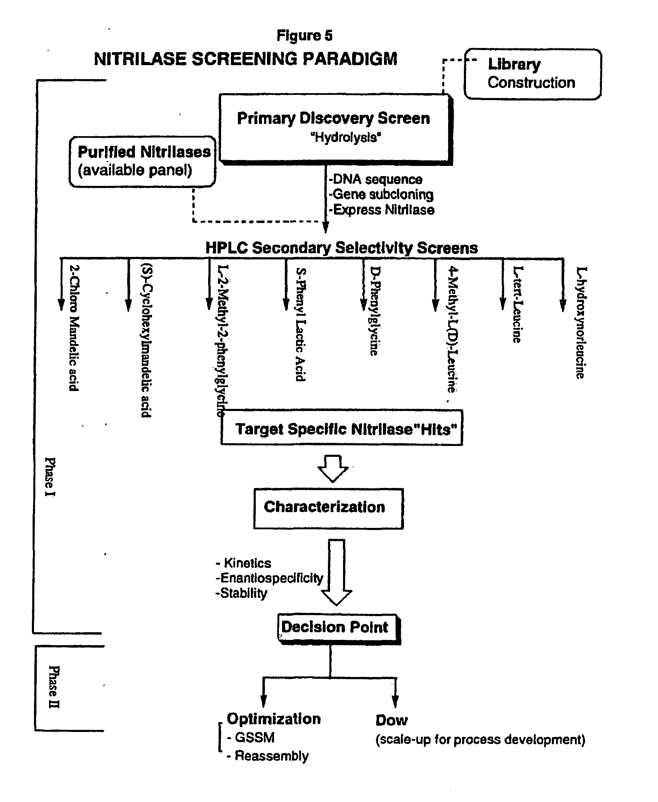

Standard curves were established for the following substrates: 2-Chloromandelonitrile (CMN, 0.998), Cyclohexylmandelonitrile (CHMN, 0.99), Acetophenone aminonitrile (APA, 0.99), and Phenylacetaldehyde cyanohydrin (PAC, 0.97), (Figure 5), (R² values in parentheses). A standard curve for Phenylglycine (PGN, 0.93) was also established. Three of the substrates tested, Dimethylbutanal aminonitrile (DMB) (2-amino-4,4-dimethyl pentanenitrile), Hydroxypivaldehyde aminonitrile (HPA) and Pivaldehyde aminonitrile (PAH), gave very low fluorescence readings and unreliable results under the original assay conditions. For these compounds a number of parameters where adjusted, however the fluorescent signal strength of these compounds was not increased by these manipulations.

In an attempt to increase the fluorescent signal of these three compounds, naphthalene dicarboxaldehyde (NDA) was substituted for OPA. Standard curves for PAH, HPA and DMB with either OPA or NDA were constructed. To determine sensitivity and background fluorescence, a lyophilized nitrilase lysate (SEQ ID NOS:189, 190) with suspected catalytic activity on each of the substrates was added. Hydrolysis was detected in three out of four of the compounds. NDA sharply boosted the signal, often by an order of magnitude, though this reduced linearity is presumably due to signal saturation.

NDA was established as an alternative detection reagent for the aliphatic compounds. However, it is desirable for the assay to utilize the same detection system for all of the substrates since this would facilitate the automated evaluation of multiple nitrilase substrates. The current OPA based assay is effective for the analysis of PAC, CMN, CHMN, APA, MN and PGN. While standard curves have been developed for the aliphatic compounds PAH, HPA, and DMB.

### Whole cell optimization

The effect of addition of lyophilized nitrilase lysate to the assay components, either untreated or heat inactivated, was evaluated. Interfering background fluorescence was not observed in either case. The OPA assay was next evaluated and optimized for nitrilase activity detection in a whole cell format. Both nitrilase expressing whole cells and in-situ lysed cells were evaluated. Lyophilized cell lysates were evaluated alongside their respective whole cell clones as controls. For this optimization study, mandelonitrile (MN) was chosen as a model substrate.

The lyophilized cell lysate of SEQ ID NOS:187, 188 was evaluated alongside whole cells expressing SEQ ID NOS:187, 188 and in *situ lysed* cells expressing SEQ ID NOS:187, 188 The addition of whole cells did not affect fluorescence nor result in fluorescence quenching. Addition of any of the three cell lysis solutions improved permeability (and therefore conversion) of mandelonitrile in the whole cell systems. Three cell lysing solutions were evaluated: B-PER (Pierce), BugBuster (Novagen) and CelLytic B-II (Sigma) and were found not to have a deleterious affect on the OPA assay. The addition of product α-hydroxyacid or α-aminoacid did not affect detection by the OPA assay.

The assay was modified from its original format, which required several liquid transfer steps, into a one plate process, where cell growth, nitrile hydrolysis and OPA assay reaction occurred in the same microtiter plate. Mandelonitrile was tested using this single well format. In this case, the *E. coli.* Gene site-saturation mutagenesis (GSSM^{™}) cell host was evaluated. Three clones were tested: SEQ ID NOS:101, 102, SEQ ID NOS:187, 188, and an empty vector, which was used as a control. Hydrolysis was evaluated at four timepoints, at 10 and 20 mM, and also with a 0 mM control. In an earlier experiment, clone SEQ ID NOS:187, 188 was evaluated against the phenylacetaldehyde cyanohydrin substrate (for which this enzyme does not exhibit activity), and no activity was observed.

The OPA assay was found to detect the presence of both α-hydroxy and α-amino nitrile substrate. Aromatic compounds were readily detectable with the assay, while aliphatic compounds posed some detection challenges. No background issues were evident when using lyophilized cell lysates, *in-situ* lysed whole cells or unlysed whole cells. The assay is amenable to one-plate analysis, where cells are grown, incubated with the substrate, and assayed on the same plate: no liquid transfers are required, easing automation. While all nitriles tested produced a linear response, aliphatic compounds gave a low fluorescent response.

### Chiral LDH Assay

A spectroscopic system based on lactate dehydrogenase (L-LDH) was developed for the analysis of the chiral α-hydroxy acids which are generated by the nitrilase catalyzed hydrolysis of cyanohydrins. The hydroxynitrile substrate is not metabolized by the secondary or detection enzyme and thus starting material does not interfere. Cell lysate which is not heat treated results in background activity for the LDH system; however, heat inactivation or pelleting of the cell lysates eliminates the background activity. (See Figure 4.)

The activity and enantiomeric specificity of commercially available D- and L-lactate dehydrogenases against the nitrilases disclosed herein was evaluated. An LDH was identified which is suitable to both D- and L-phenyl lactic acid analysis. An enzyme suitable for 2-Chloromandelic acid analysis was not found. The chosen LDH enzymes exhibited virtually absolute stereoselectivity. The viability of the assay to detect D- and L-LDH produced from PAC using lyophilized cell lysate was established.

Originally, three colorimetric dyes were evaluated, all of which are tetrazolium salts: NBT (3,3'-dimethoxy-4,4'-biphenylene)bis[2,(4-nitrophenyl) -5-phenyl-2H]-, chloride) MTT (3-(4,5-dimethylthiazol-2-yl)-2,5-diphenyltetrazolium bromide) INT (2-(4-Iodophenyl)-3-(4-nitrophenyl)-5-phenyl- 2H-tetrazolium chloride). The insolubility of the product of these detection system posed an analytical challenge. To address this, another tetrazolium salt with a reportedly soluble product, XTT (2,3-Bis-(2-methoxy-4-nitro-5-sulfophenyl) -2*H*-tetrazolium-5-carboxanilide, was evaluated. While XTT yielded a soluble bright red product, the substrate was insoluble which thus effected the same analytical challenges. As an alternative to the tetrazolium family of dyes, the dual colorimetric/fluorometric dye resazurin was evaluated. Oxidation of resazurin produces resourfin. Both substrate and product are soluble, and the color change can be quantified colorimetrically or fluorimetrically, increasing accuracy. Due to the sensitivity of resazurin, 0.05 mM of lactic acid can be quantified. Optimal results were obtained when using the dye in the same range as the substrate, *e.g.* 0.5 mM resazurin can quantify a range of lactic (and analogs) from 0.05 to 0.5, though the best linearity is at the lower end of this scale. Resourfin was stable over 28 hours, and had a linear fluorescent response.

In the presence of the LDH assay components, lyophilized enzyme gave background fluorescence/absorption. To address this problem the lysate was boiled for 10 minutes and then centrifuged. This resulted in a 90% decrease in background signal. Interestingly, both centrifugation alone (5 minutes @ 14.1 rcf) or boiling followed by centrifugation (5 minutes @ 100°C) reduced the fluorescence to background levels. In a high-throughput format such as 1536 well plates, spinning would be preferable to boiling, as boiling would increase evaporation (8 µl well size) and potentially volatize the nitrile substrates. No background signal resulting from growth media (LB and TB and M9) or cell lytic solutions (B-PER, CelLytic and BugBuster) was noted.

### Chiral AAO Assay

A spectroscopic system based on amino acid oxidase (AAO) was developed for the analysis of the chiral α-amino acids which are generated by the nitrilase catalyzed hydrolysis of amino nitriles.

### Assay Development and Validation

The initial assay validation utilized the 2,2'-azino-di-{3-ethylbenzothiazoline-6-sulfonic acid (ABTS) detection system as outlined above. However, since the color was not stable further investigations utilized the phenol amino antipyrine (PAAP) detection system which is analyzed at λ max 510nm. Enzymes with suitable activity were found for each enantiomer of 4-methyl- leucine, phenylalanine, (S)-2-amino-6-hydroxy hexanoic acid, and *tert*-leucine. The assay is not applicable to methylphenylglycine and does not work well with phenylglycine.

Standard curves were generated for phenylalanine from 0-15 mM. The curve is much more linear when the concentrations remained below 1 mM. The color remains stable for several days as long as it is kept in the dark. Three cell lysing solutions Bug Buster (BB), Bacterial Protein Extracting Reagent (BPER), and Cell Lytic Reagent (CLR) were added to the standard curve and shown to have no affect on color development. The addition of cell lysate (cl) did not exhibit background color formation. Addition of the phenylacetaldehyde aminonitrile sulfate (PAS) starting material also showed no effect on color formation.

The AAO system exhibits greater linearity at up to 1 mM substrate. The concentration of the AAO enzymes and of the acid substrate were adjusted to try to move the intersection of the L-AAO and D-AAO curves closer to the middle of the graph. Premixing the PAAP, the HRP, and the AAO was demonstrated to be effective and caused no change in observed activity establishing that the assay components may be added to the assay in a cocktail format.

A high level of background was observed for the AAO assay of whole cells and this was attributed to the L-amino acids present in the TB and LB growth media. Washing and resuspension of the cells in M9 media eliminated background. For all future experiments cells were grown in M9 media with 0.2% glucose. The lysed cells gave only a slightly better response that unlysed cells. Therefore, cell lysis is not necessary. SEQ ID NOS:187, 188 demonstrated activity on HPA in primary screening based on HPLC analysis.

The use of a fluorescent detection system which would permits implementation of the assay in ultra high throughput fashion such as 1536 well or gigamatrix format was investigated. The fluorescent reagent most applicable to our system is Amplex Red from Molecular Probes which produces the highly fluorescent resorufin (λₑₓ 545 nm; λₑₘ 590nm) Standard curves for phenylalanine and (S)-2-amino-6-hydroxy hexanoic acid were established (0-100 µM).

In preparation for assay automation, nitrilase expressing cells were added into microtiter plate containing M9 0.2% glucose,0.25 mM IPTG media by florescence activated cell sorting (FACS). Three nitrilase expressing subclones, and the empty vector control were evaluated: SEQ ID NOS:101, 102, SEQ ID NOS:187, 188, SEQ ID NOS:29, 30 and the empty vector. The viability of the cells following cell sorting proved to be inconsistent. Thus colony picking is currently being evaluated as an alternative method to add cells into microtiter plates. The evaporative loss from an uncovered 1536-well microtiter plate is approximately 30% per day in the robot incubator (incubator conditions: 37°C at 85% relative humidity (RH)). Incubation in the 95% RH incubator reduced evaporative loss to 1% per day.

The ability of the three subclones to grow in the presence of up to 3.5 mM of nitrile was established using HPA nitrile. Growth rates were only slightly retarded (less that 30%). Subclones grown in the presence of HPA were shown to express a nitrilase that catalyzes the formation of hydroxy norleucine (HNL) as established using the Amplex Red detection system. Only S was evaluated as the enzymes are S-selective. The reaction plate was read at 10 minute intervals, with 40 minutes showing the best linearity. While cell growth is significantly inhibited above 5 mM of HPA when the cells were grown at pH 7, growth was inhibited above 0.1 mM HPA for cells grown at pH 8.

In order to verify the AAO results by HPLC, a reaction was performed using high concentrations of HPA, up to 40 mM (due to HPLC detection challenges for (S)-2-amino-6-hydroxy hexanoic acid) and lyophilized cell lysate SEQ ID NOS:187, 188.

### Comparison AAO and HPLC data for HNL

| | %ee | | %conversion | |
|---|---|---|---|---|
| [HNL] mM | AAO | HPLC | AAO | HPLC |
| 40 | 89% | 100% | 17% | 18% |
| 30 | 89% | 97% | 29% | 36% |
| 20 | 86% | 97% | 21% | 34% |
| 10 | 78% | 98% | 13% | 35% |

In order to determine if conducting the screen at a lower concentration introduces a bias in the results compared to the 20 mM substrate range that was used for HPLC based screens, an experiment was performed with SEQ ID NOS:187, 188 using three concentration ranges. Each experiment was done in triplicate in order to remove any nonsystematic error.

### SEQ ID NOS:187, 188: Observed conversion and ee at multiple concentrations of HPA.

| **Enantiomeric Excess (% *ee)*** | | | | | | | | | |
|---|---|---|---|---|---|---|---|---|---|
| **[HPA] mM** | **10** | **20** | **30** | **1** | **2** | **3** | **0.1** | **0.2** | **0.3** |
| **Trial #1** | 60 | 54 | 72 | 61 | 64 | 63 | 60 | 63 | 60 |
| **Trial #2** | 57 | 62 | 69 | 58 | 57 | 53 | 32 | 51 | 37 |
| **Trial #3** | 53 | 62 | 69 | 60 | 55 | 52 | 79 | 103 | 72 |
| **AVG** | 57% | 59% | 70% | 59% | 59% | 56% | 57% | 72% | 56% |

| **% Conversion** | | | | | | | | | |
|---|---|---|---|---|---|---|---|---|---|
| **[HPA] mM** | **10** | **20** | **30** | **1** | **2** | **3** | **0.1** | **0.2** | **0.3** |
| **Trial #1** | 27% | 27% | 37% | 34% | 29% | 43% | 37% | 29% | 42% |
| **Trial #2** | 42% | 43% | 49% | 48% | 42% | 56% | 46% | 44% | 54% |
| **Trial #3** | 31% | 32% | 43% | 22% | 29% | 27% | 46% | 27% | 35% |
| **AVG** | 33% | 34% | 43% | 35% | 34% | 42% | 43% | 33% | 44% |

The AAO assay can be run on 384 or 1536 well format with cells sorted into an M9 0.2% glucose, 0.25 mM IPTG media. Cells can be grown in the presence of nitrile (in this case HPA), or the cells can be allowed to reach a certain density and the nitrile can then be added. Though cell lytic reagents do not interfere with the assay, when HPA was assayed, addition of the lytic reagents was found to be unnecessary. Either pre- or post- nitrile addition, the mother plate will have to be split into daughter plates, which are then assayed for the respective L- and D- enantiomer content. Incubation times with the AAO/Amplex Red reagents can be adjusted so that the D-and L- plate are read at separate times.

### Example 16 - Identification, Development and Production of Robust, Novel Enzymes Targeted for a Series of High-Value Enantioselective Bioprocesses

The invention provides for the development of nitrilases, through directed evolution, which provide significant technical and commercial advantages for the process manufacturing of the following chemical target:

### L-2-amino-6,6-dimethoxyhexanoic acid

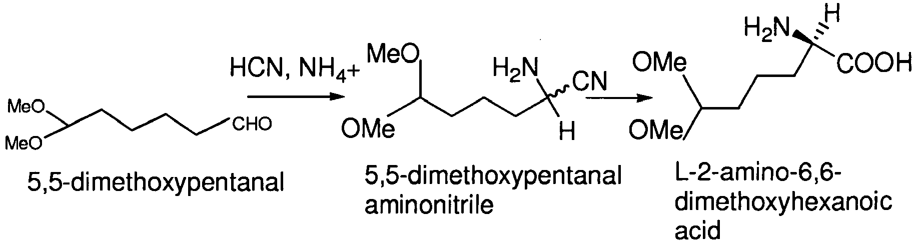

Nitrilase enzymes were shown to hydrolyze 2-amino-6-hydroxy hexanenitrile to (S)-2-amino-6-hydroxy hexanoic acid, with selectivity towards the L-enantiomer. The panel of nitrilases was screened against the target, 5,5-dimethoxypentanal aminonitrile. The positive enzymes were characterized on this substrate. A primary screen is used to identify putative up-mutants, which is then confirmed using HPLC.

GSSM™ and GeneReassembly™ are performed on selected nitrilases, in order to improve the enantioselectivity and activity of the enzymes for the production of L-2-amino-6,6-dimethoxyhexanoic acid. Nitrilases were identified for the enantioselective hydrolysis of 2-amino-6-hydroxy hexanenitrile to L-(S)-2-amino-6-hydroxy hexanoic acid. However, a slight structural difference is presented by the new target molecule, L-2-amino-6,6-dimethoxyhexanoic acid. In order to determine whether this difference affects the activity and enantioselectivity of the enzymes, the complete spectrum of nitrilases was screened against the new target.

First, identification of the correct target gene for GSSM through more detailed characterization of the hit enzymes for the production of L-2-amino-6,6-dimethoxyhexanoic acid was carried out. This effort involves a more extensive investigation of the effects of pH and temperature on activity and enantioselectivity and a more in-depth analysis of the stability of the enzyme to process conditions. Prior to initiation of the screening, the synthesis of a single enantiomer of an alkyl aminonitrile is done; the racemization of this nitrile is studied, in an effort to understand the relationship between this factor and enantioselectivity of the enzymes.

An enzyme exhibiting the highest combination of activity and enantioselectivity for the production of L-2-amino-6,6-dimethoxyhexanoic acid is selected for GSSM. Following the mutation of the target enzyme, the resulting mutants are screened on 5,5-dimethoxypentanal aminonitrile, using high throughput screening technology. Following confirmation of the up-mutants by HPLC analysis a decision point is reached, in order to evaluate the results of the GSSM on the target.

In parallel to GSSM™, a GeneReassembly™ is performed on a combination of parent enzymes, at least one of which is selected for activity and enantioselectivity on L-2-amino-6,6-dimethoxyhexanoic acid. At least two other nitrilases are reassembled with the former enzyme(s); these enzymes are selected in order to provide diversity to the reassembled sequences.

The methods provide for development of racemization conditions for the original substrate aminonitriles. In addition, they provide for the identification of enzymes capable of the conversion of these aminonitriles to the target α-amino acids by dynamic kinetic resolution. They also provide for screening and development of a nitrilase-catalyzed kinetic resolution process for (R)-2-amino-6,6-dimethoxy hexanoic acid (allysine) production. (S)-2-amino-6-hydroxy hexanoic acid will be used as a model substrate for development of the kinetic resolution.

The target α-amino acid products are shown below:
(i) D-4-Fluorophenylglycine 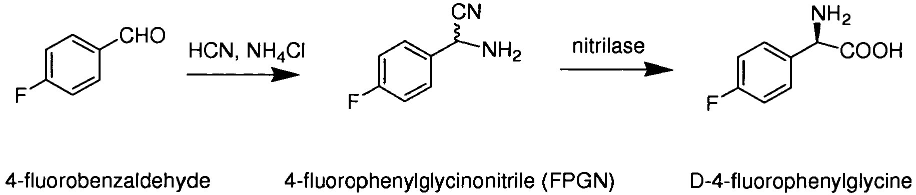
(ii) L-2-Amino-6,6-dimethoxyhexanoic acid (Allysine)

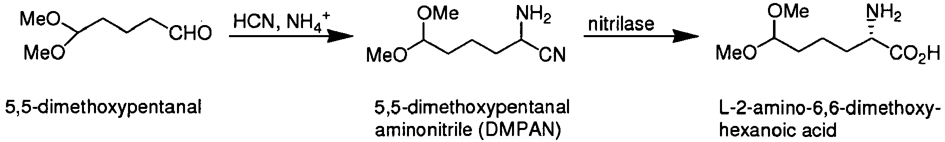

Conditions are developed for the racemization of the aminonitrile substrates for the nitrilase-catalyzed production of D-4-fluorophenylglycine and 2-amino-4,4-dimethyl pentanenitrile (allysine). Two model substrates, phenylglycinonitrile and pentanal aminonitrile are used initially, and racemization is studied in the absence of the enzyme. Concurrently determination of the performance of one or more available nitrilases under a variety of possible racemization conditions is carried out. In addition, the nitrilases are screened against hydroxypentanal aminonitrile for the production of (S)-2-amino-6-hydroxy hexanoic acid, and the promising enzymes are optimized. Once racemization conditions are established, the nitrilases are screened for activity. Further optimization for a kinetic resolution of the product is performed.

A number of enantioselective nitrilases were identified for the hydrolysis of α-aminonitriles to α-amino acids. While these enzymes were shown to have a preference for the required enantiomer of certain aminonitriles, a limiting factor in the further screening, development and comparison of candidate nitrilases is the rate of racemization of the aminonitrile substrates under the reaction conditions.

### Aromatic aminonitrile racemization

The first step is to establish conditions under which aromatic aminonitrile racemization occurs, using the model substrate, phenylglycinonitrile. Racemization strategies include, but are not limited to the list below. Options are roughly prioritized according to their commercial applicability.
(1) Manipulation of the pH of the reaction. Since it has been shown that racemization is rapid at high pH, this approach requires the discovery and optimization of nitrilases which are active and selective at pH>10.
(2) Addition of known chemical racemizing agents, such as aldehydes, ketones, weak bases, resins, metal ions, Lewis acids etc., which can enhance racemization at lower pH.
(3) Synthesis of N-acylated aminonitrile derivatives, *e.g.* N-acetyl phenylglycinonitrile, which may be more easily racemized. In the case of N-acetyl phenylglycinonitrile, a selective D-acylase which removes the acetyl group would enhance the optical purity of the nitrilase product.
(4) Use of a biphasic system in which base-catalyzed racemization occurs in the hydrophobic organic phase and enzymatic hydrolysis in the aqueous phase.
(5) Use of a 2-enzyme system comprised of a nitrilase and an aminonitrile racemase. One amino acid racemase is commercially available at present, and will be tested for activity against phenyl- and fluorophenylglycinonitrile. Gene libraries will be searched for genes showing homology to known amino acid amide racemases, hydantoin racemases or any other racemases which can be identified.

Once conditions for this racemization have been established, they provide the basis for development of conditions for racemization of the target aromatic substrate, 4-fluorophenylglycinonitrile (FPGN). The FPGN is expected to be less stable than the model substrate; thus, it may racemize more quickly, but degradation reactions may be faster as well. The ability of sample enzyme(s) to tolerate and/or function well under them is evaluated. Final optimization of screening methods include the target substrates, sample nitrilases, and substrate racemization conditions.

Investigations carried out have shown that phenylglycinonitrile is easily racemized at pH 10.8. However, it does not appear that any of the existing enzymes can tolerate such harsh conditions of pH. Samples from highly alkaline environments are screened for the presence of nitrilases which are tolerant to such conditions. Once discovered, the enzymes are sequenced and subcloned, and the enzymes are produced as lyophilized cell lysates ready for screening.

### Aliphatic aminonitrile racemization

A model aliphatic aminonitrile, pentanal aminonitrile, is synthesized in its racemic form. However optically enriched samples are prepared using one the following approaches: (i) preparative chiral HPLC; (ii) diastereomeric salt resolution; (iii) diastereomeric derivatization or column chromatography; (iv) synthesis from L-N-BOC norleucine. An HPLC assay is used for the detection of these compounds.

### HPLC Assay

An HPLC assay for the detection of the (S)-2-amino-6-hydroxy hexanoic acid is used. An assay involving pre-column derivatization is used.

### ScreeninglCharacterization:

Nitrilases are screened against 2-amino-6-hydroxy hexanenitrile. For enzymes capable of performing well at greater than 25 mM substrate, scale up reactions are performed. The substrate/product tolerance and stability profiles of the other enzymes are investigated.

The nitrilases are screened, and hits are characterized, focusing on pH and temperature optimum, enantioselectivity and stability under the reaction conditions.

### Enzyme Evolution

A target enzyme exhibiting the desired properties is selected for GSSM™. Following the mutation of the target enzyme, the resulting mutants are screened on the substrate using high throughput screening technology. Once the up-mutants have been confirmed by HPLC analysis, the individual mutations responsible for increased performance may be combined and evaluated for possible additive or synergistic effects.

In addition, a GeneReassembly™ will be performed on a combination of lead enzymes, which are selected for their desirable characteristics, including activity, enantioselectivity and stability in the reaction.

### Example 17

### Optimization of Nitrilases for the Enantioselective

### Production of (S)-Phenyllactic Acid

Nitrilases were identified for the enantioselective hydrolysis of 5 different nitrile substrates. These nitrilases were isolated and optimized for selected targets. The optimization involves process optimization and directed evolution. In particular, enzymes specific for the production of (S)-phenyllactic acid were characterized and optimized. This was aimed primarily at improving the activity of the enzymes, while maintaining a high enantioselectivity. An investigation into the effects of process conditions on the enzymes was also performed.

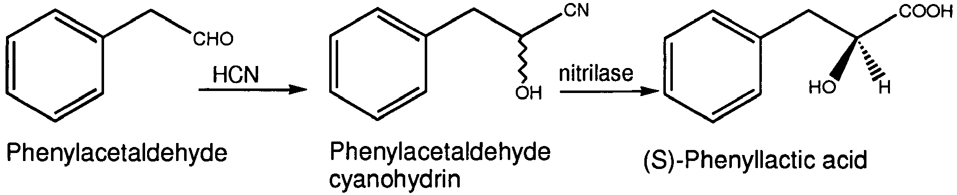

The development of high throughput assays for screening of mutants from potential directed evolution efforts was accomplished. Two achiral and two chiral colorimetric assays that are amenable to high throughput screening were developed and used for nitrilase directed evolution.

SEQ ID NOS:103, 104 was identified as a highly enantioselective nitrilase for the production of (S)- phenyllactic acid. Characterization of SEQ NOS:103, 104 shows the optimum reaction pH and temperature to be pH 8 and 37°C, respectively; the reaction starting material, phenylacetaldehyde, and the product, phenyllactic acid showed no effect on the enzyme activity up to levels of 5 mM and 30 mM, respectively. The scaled-up enzymatic reaction with an enantiomeric excess (*ee*) of 95%.

### Summary of enzymes screened for activity and enantioselectivity on Phenylacetaldehyde cyanohydrin

| **SEQ ID NOS:** | **Enzyme concentration** | **Conversion (%)** | **Time to highest conversion (h)** | ***ee* (%)** |
|---|---|---|---|---|
| | **(mg protein/ml)** | | | |
| 109,110 | 0.15 | 70% | 2 | 71% |
| 115,116 | 0.15 | 70% | 3 | 72% |
| 127, 128 | 0.15 | 66% | 8 | 70% |
| 133,134 | 0.6 | 60% | 3 | 84% |
| 135,136 | 0.15 | 63% | 7 | 87% |
| 125, 126 | 0.6 | 63% | 3 | 83% |

### Example 18 - Directed Evolution of a nucleic acid encoding a nitrilase enzyme.

The *nitB* gene (GenBank Accession No. AX025996, from Alcaligenes faecalis) was subjected to Gene Site Saturated Mutagenesis™ or GSSM™ to generate a library of single amino acid substitution mutants covering the entire enzyme. The sequence of the "parental" *nitB* gene used in the directed evolution is SEQ ID NO: 103, 104. A nitB mutant library was generated from carrying out GSSM™. This *nitB* mutant library was then screened for clones with increased whole cell hydroxymethylthiobutryonitrile (HMTBN, which is a nitrilase substrate) activity. The product of the nitrilase reaction on that substrate is hydroxymethylthiobutyric acid (HMTBA).

Assays were run at 35°C with 100mM HMTBN and 100mM K₃PO₄, pH 7 to approximately 30-40% conversion. Two methods were used to quantitate HMTBN conversion, one being direct measurement of HMTBS produced by HPLC analysis and the other being indirect detection of residual HMTBN using the fluorescent cyanide assay, which has previously been described.

Putative *nitB* up mutants were subjected to a secondary assay to confirm the increased activity. In the secondary assay, up mutants and the wild type control were induced in expression medium in shake flasks. Shake flask cultures are then washed with 100mM K₃PO₄, pH7 and resuspended to the same optical density at 660nm. Kinetic assays were then performed with the normalized cell resuspensions under the same conditions used in the initial assays. Putative up mutants confirmed to have increased HMTBN activity were sequenced and tested for increased activity after transformation back into the same expression strain to ensure that increases in activity are not due to host mutations.

A confirmed *nitB* GSSM™ up-mutant is *nitB* G46P, which contains a glycine (GGT) to proline (CCG) substitution at amino acid 46. The whole cell HMTBN activity of this mutant is approximately 50% greater than that of wild type NitB at both 25°C and 35°C. Upon identification of the beneficial G46P mutation, GSSM™ was used again to generate a pool of double mutants using the *nitB* G46P template. These mutants all contain the G46P mutation and an additional single amino acid substitution at a random site. The double mutants were assayed for HMTBN activity greater than that of *nitB* G46P. Double, triple and quadruple mutants were created in order to speed up the mutation process and identify beneficial mutations more quickly. After the first few beneficial mutations were identified and isolated, they were combined to generate double mutants, the best of which was DM18. DM18 was used as a template to generate triple mutants. The most active triple mutant was TM3 and that was used as a template to generate quadruple mutants. The most active quadruple mutant was QM2. The table summarizes these mutations.

| **mutant** | **mutation 1** | **mutation 2** | **mutation 3** | **mutation 4** |
|---|---|---|---|---|
| DM18 | R (gcg) 29 C(tgt) | Y(tac) 207 M (atg) | | |
| TM3 | R (gcg) 29 C(tgt) | Y(tac) 207 M (atg) | L(ctt) 170 T(act) | |
| QM2 | R (gcg) 29 C(tgt) | Y(tac) 207 M (atg) | L(ctt) 170 T(act) | A(gcg) 197 N9(aat) |

The mutants were characterized first by studying their whole cell HMTBN activity. At 100 mM HMTBN, the HMTBS production rate of QM2 is 1.2 times greater than that of the parental gene. However, at 200 mM HMTBN, the rate of QM2 is 3.6 times that of the parental gene. The productivity of these mutants is increased considerably when the HMTBN concentration is raised from 100 mM to 300 mM. As to conversion rates, TM3 completely converted the substrate after 270 minutes and both DM18 and SM show greater than 75% conversion after this time. To further address the issue of HMTBN concentration effects on activity/productivity of NitB, several mutants were assayed at both 400 mM and 528 mM HMTBN. NitB is essentially inactive at these substrate concentrations, however the mutants retain significant activity at these concentrations. In particular, the activity at these high concentrations were essentially the same as their activity at 200 mM substrate. Therefore, the mutants can be used over a wide substrate concentration range and provide much more flexibility in utility than the NitB parental gene.

The mutants were shown to have higher expression levels than the parental gene and it also appeared tha the QM2 and TM3 mutants contained a greater proportion of soluble enzyme than the wild type as seen in SDS-PAGE analysis. As to stability, all of the enzymes showed essentially the same stability pattern at both 25°C and 35°C.

Finally, the mutants were subjected to codon-optimization. The approach was to optimize the codons and therefore increase the expression levels in the particular host cell. That would, in turn, increase the activity per cell of the enzyme. This resulted in increased whole cell activity in the codon-optimized mutants as compared to controls. The increase in activity was approximately double the activity. An *E. coli* expression system was used.

### Example 20 - Selected Examples of Compounds Produced From a Nitrilase-Catalyzed Reaction

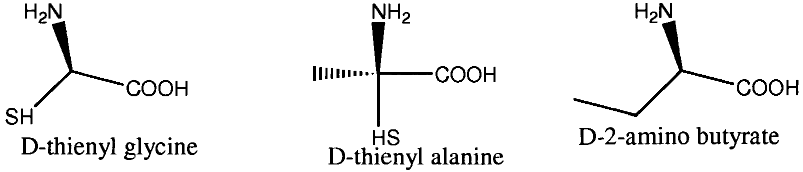

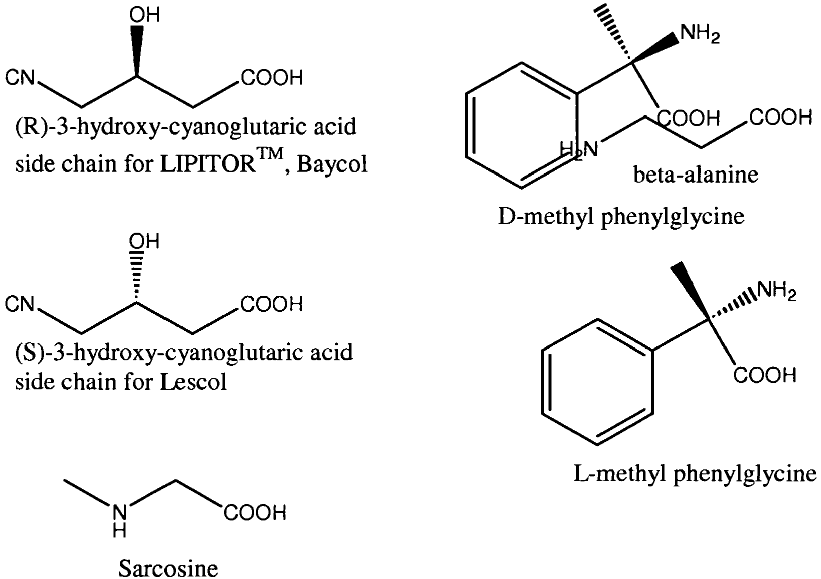

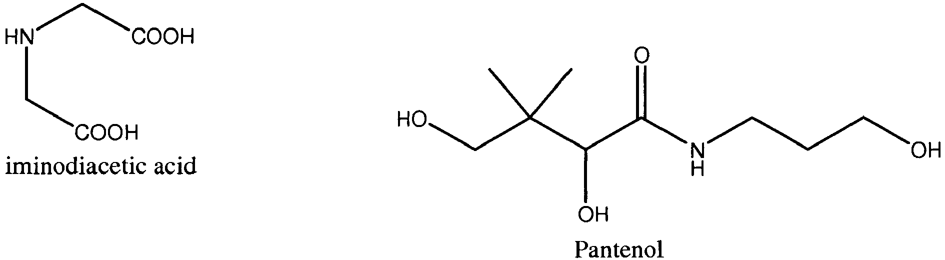

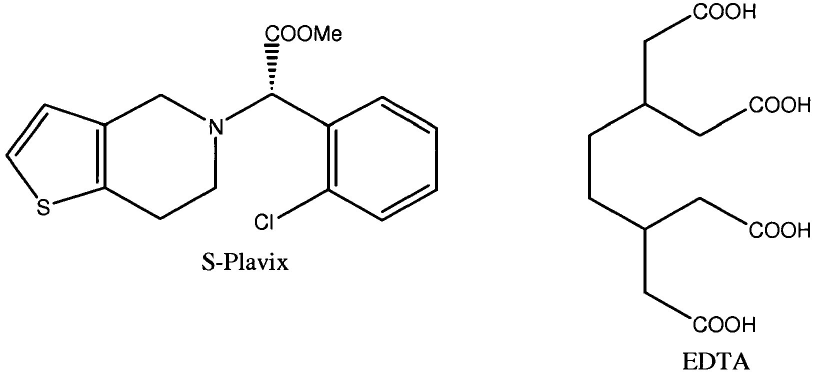

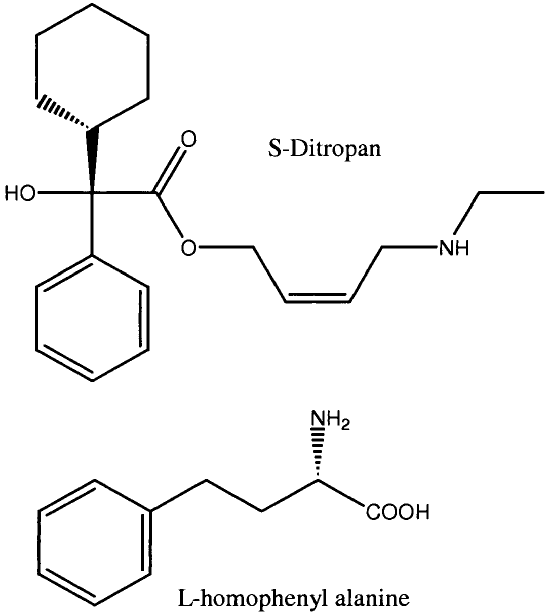

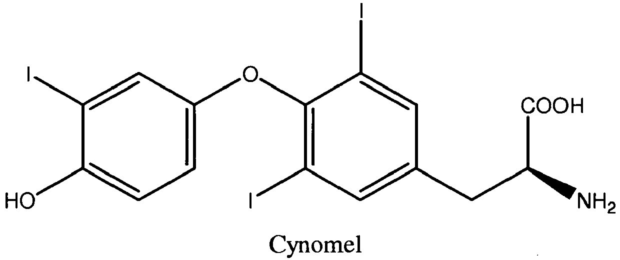

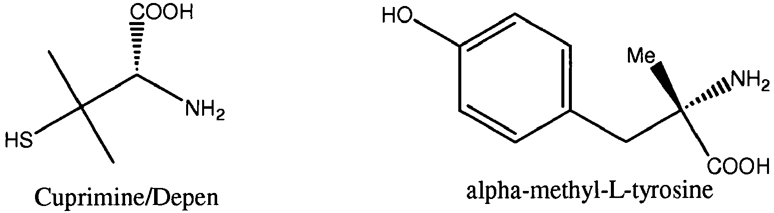

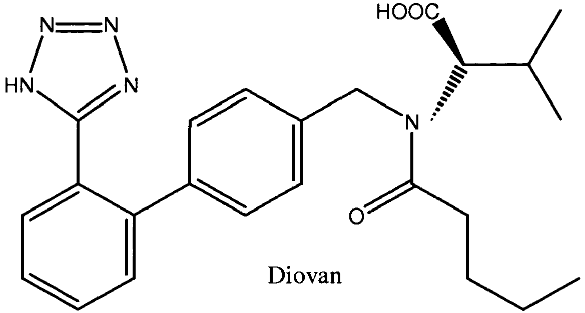

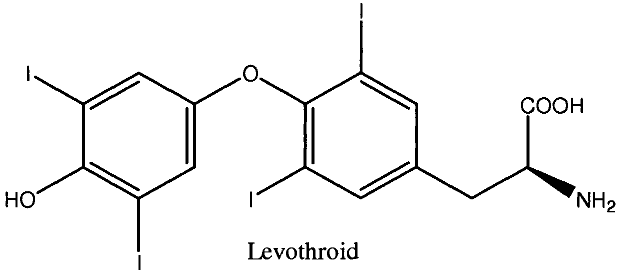

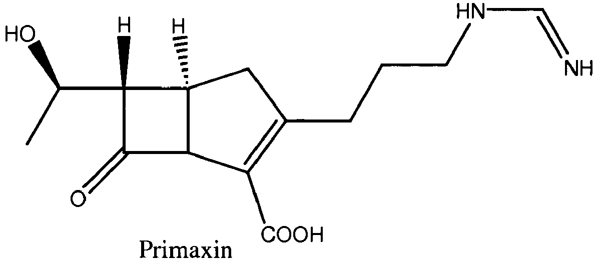

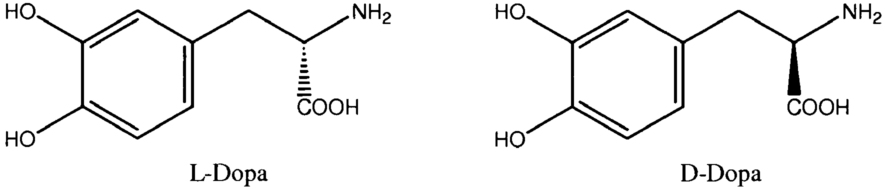

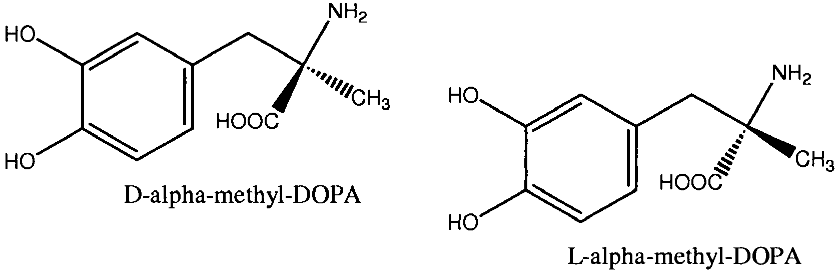

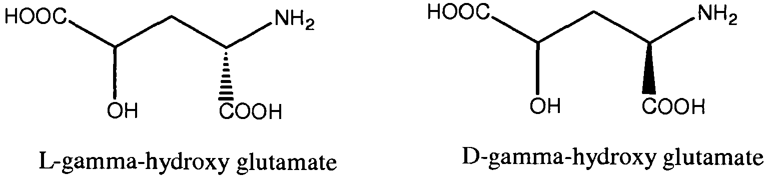

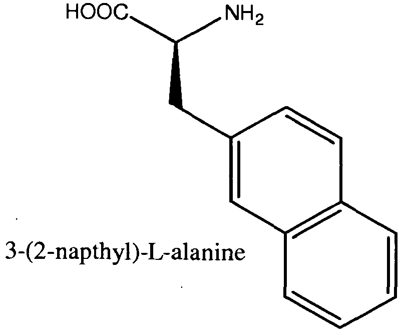

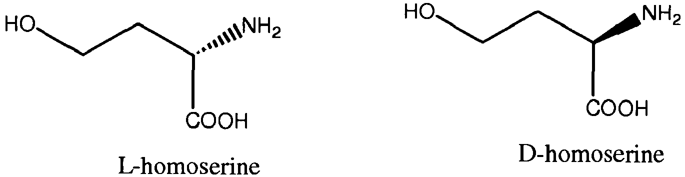

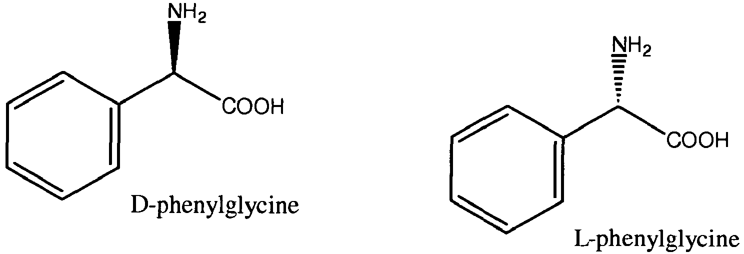

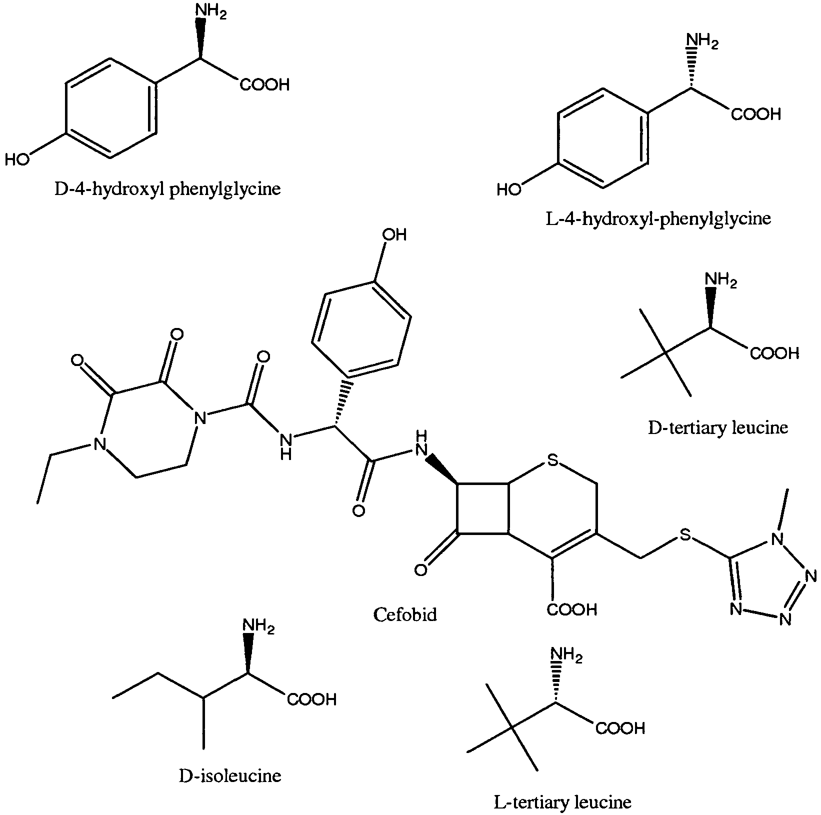

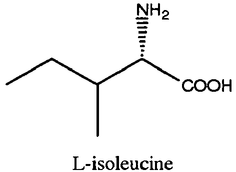

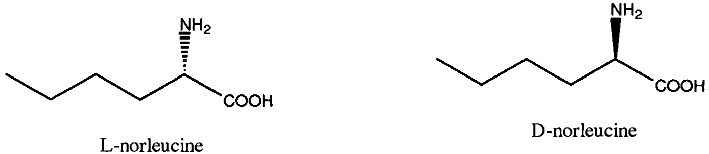

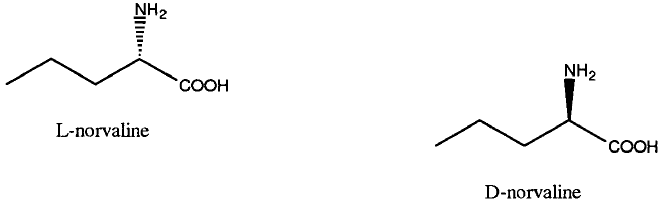

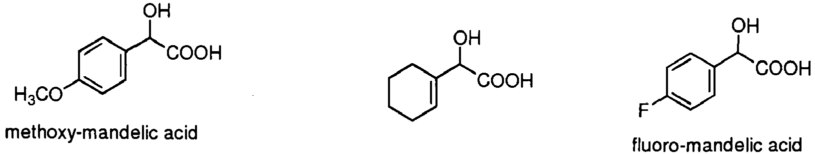

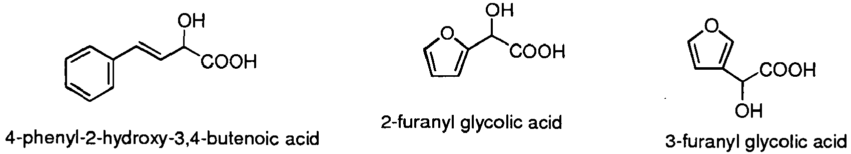

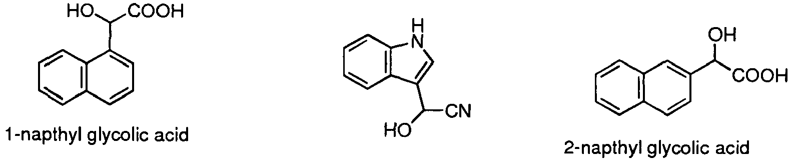

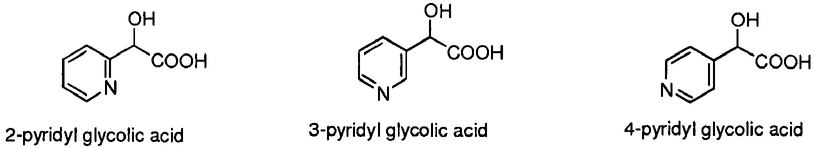

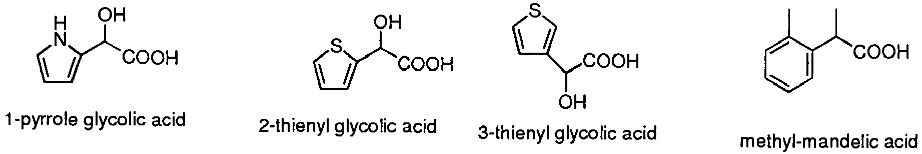

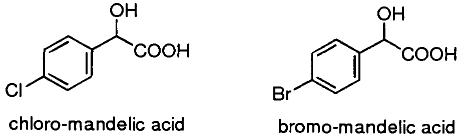

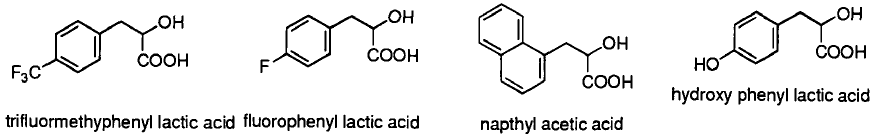

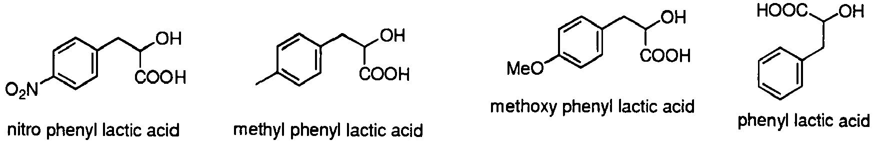

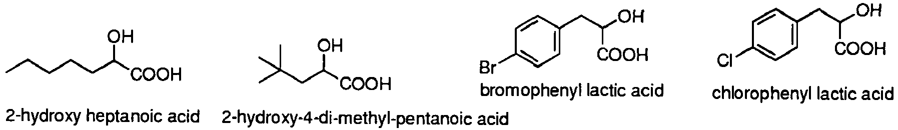

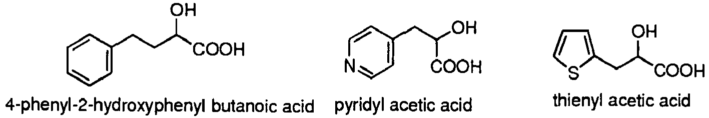

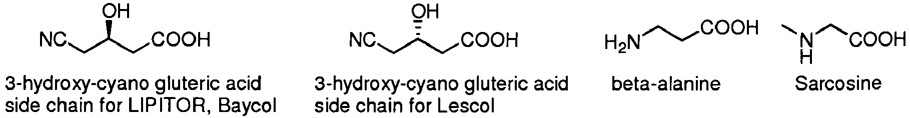

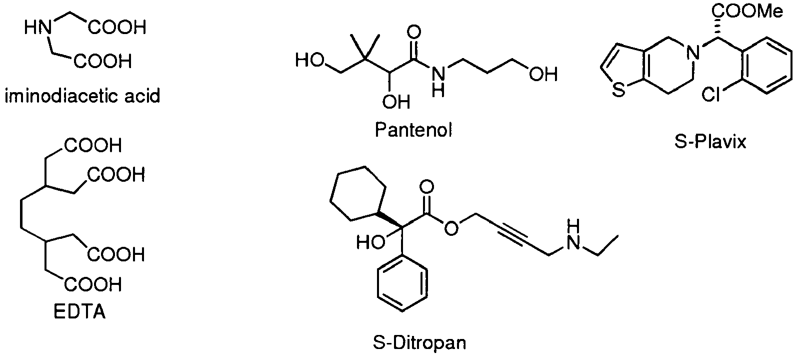

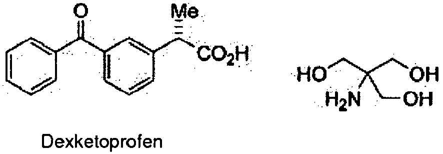

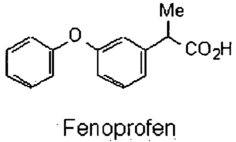

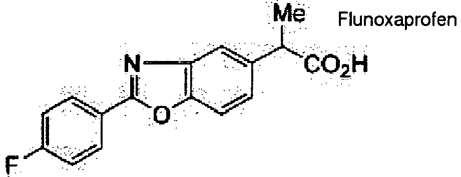

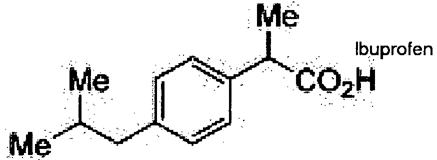

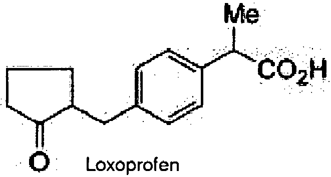

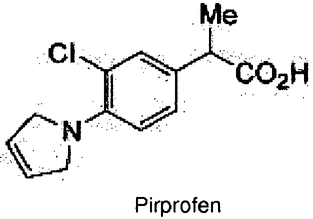

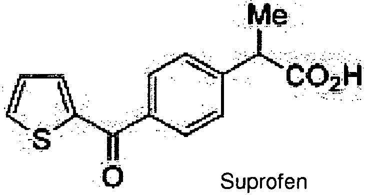

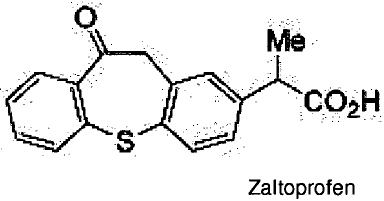

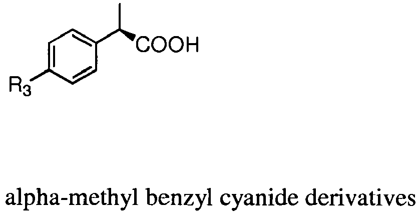

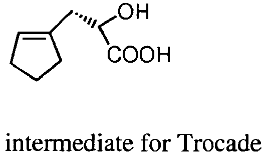

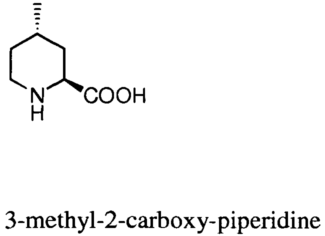

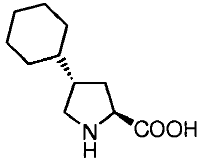

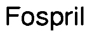

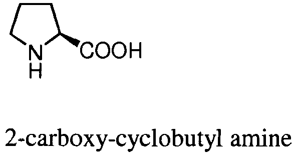

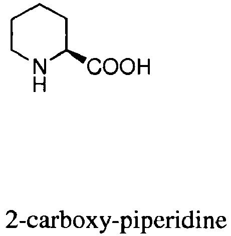

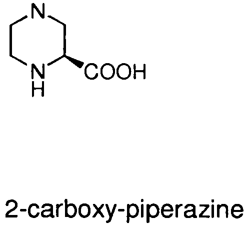

In addition, the following are potential products which can be made via the nitrilase Strecker format. More than 100 amino acids and many new drugs can be produced from their respective aldehydes or ketones utilizing the nitrilase enzymes of the invention. For example, large market drugs which can be synthesized using nitrilases of the invention include homophenylalanine, VASOTECT™, VASOTERIC™, TECZEM™, PRINIVIL™, PRINZIDE™, ZESTRIL™, ZESTORETIC™, RAMACE™, TARKA™, MAVIK™, TRANDOAPRIL™, TRANDOLAPRILAT™, ALTACE™, ODRIK™, UNIRETIC™, LOTENSIN™, LOTREL™, CAPOTEN™, MONOPRIL™, TANATRIL™, ACECOL™, LONGES™, SPIRAPRH™, QUINAPRIL™, and CILAZAPRIL™. Other chiral drugs include DEMSER™ (alpha-methyl-L-Tyrosine), ALDOCHLOR™, LEVOTHROID™, SYNTHROID™, CYTOMEL™, THYOLAR™, HYCODAN™, CUPRIMINE™, DEPEN™, PRIMAXIN™, MIGRANOL™, D.H.E.-45, DIOVAN™, CEFOBID™, L-DOPA, D-DOPA, D-alpha-methyl-DOPA, L-alpha-methyl-DOPA, L-gamma-hydroxyglutamate, D-gamma-hydroxyglutamate, 3-(2-naphthyl)-L-alanine, D-homoserine, and L-homoserine.

Furthermore, the nitrilase enzymes described herein can be useful for synthesizing the following amino acids. Many of these amino acids have pharmaceutical applications. D-phenylglycine, L-phenylglycine, D-hydroxyphenylglycine, L-hydroxyphenylglycine, L-tertiary leucine, D-tertiary leucine, D-isoleucine, L-isoleucine, D-norleucine, L-norleucine, D-norvaline, L-norvaline, D-2-thienylglycine, L-2-thienylglycine, L-2-aminobutyrate, D-2-aminobutyrate, D-cycloleucine, L-cycloleucine, D-2-methylphenylglycine, L-2-methylphenylglycine, L-thienylalanine, and D-thienylalanine.

The nitrilase enzymes can be useful for the synthesis of the following natural amino acids: glycine, L-alanine, L-valine, L-leucine, L-isoleucine, L-phenylalanine, L-tyrosine, L-tryptophan, L-cysteine, L-methionine, L-serine, D-serine, L-threonine, L-lysine, L-arginine, L-histidine, L-aspartate, L-glutamate, L-asparagine, L-glutamine, and L-proline. The following are examples of unnatural amino acids which can be produced using the nitrilase enzymes of the invention. D-alanine, D-valine, D-leucine, D-isoleucine, D-phenylalanine, D-tyrosine, D-tryptophan, D-cysteine, D-methionine, D-threonine, D-lysine, D-arginine, D-histidine, D-aspartate, D-glutamate, D-asparagine, D-glutamine, and D-proline.

Furthermore, nitrilase enzymes can be used in non-Strecker chemical reactions including the synthesis of more chiral drugs such as TAXOTERE™ as well as chiral drugs containing 3-hydroxy-glutaronitrile (a $5.5B market); LIPITOR™, BAYCOL™, and LESCOL™. Chiral product targets that are not drugs include PANTENOL™, L-phosphinothricin, D-phosphinothricin, D-fluorophenylalanine, and L-fluorophenylalanine. Finally, nitrilase can be used to produce unnatural amino acid compounds lacking a chiral center such as sarcosine, iminodiacetic acid, EDTA, alpha-aminobutyrate, and beta-alanine.

The following section includes examples of substrates and products produced by the nitrilases of the disclosure. The chemical structures of the substrates and of the products are shown. Activities, yield and the specific nitrilase shown to be useful in the chemical reactions are included in Tables following the reactions. The chemical reactions shown here are non-limiting examples of activities of the nitrilases.

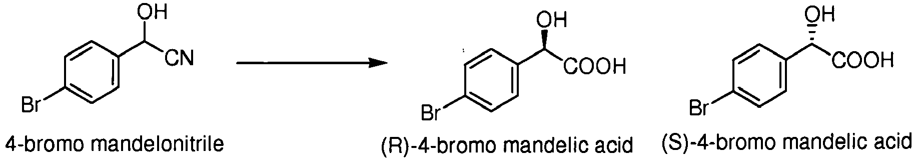

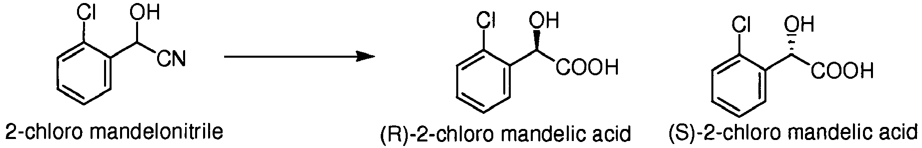

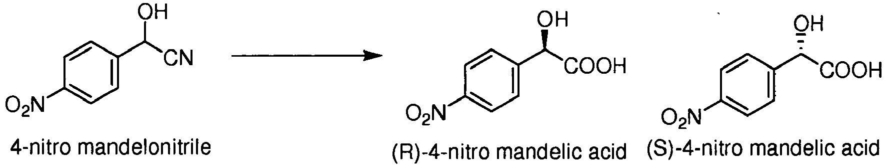

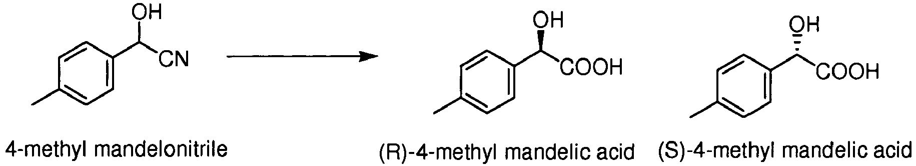

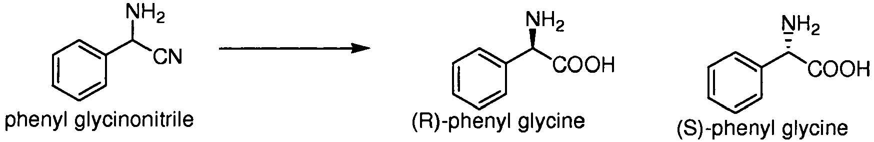

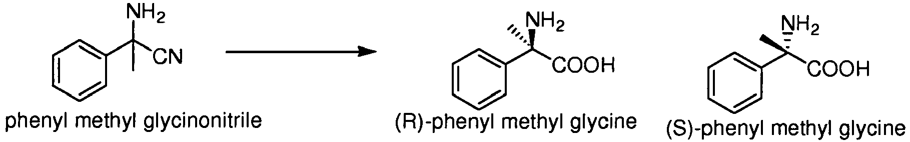

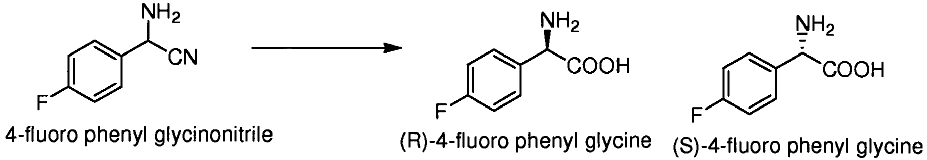

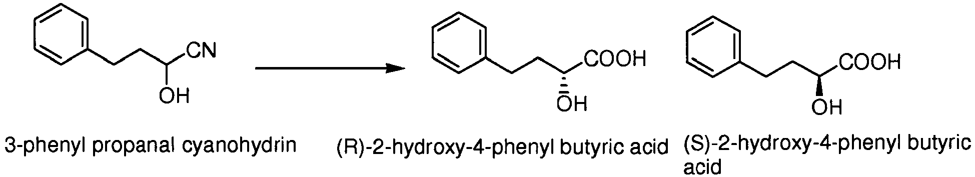

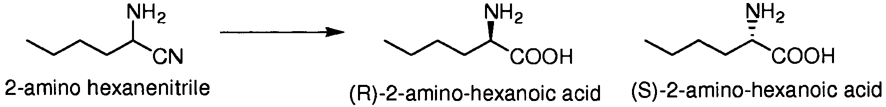

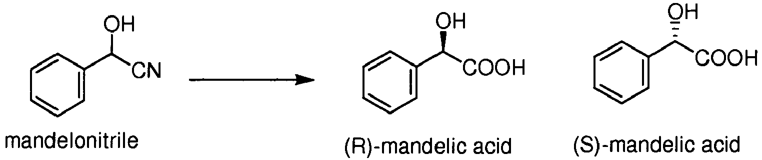

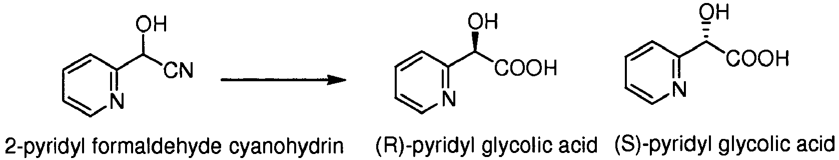

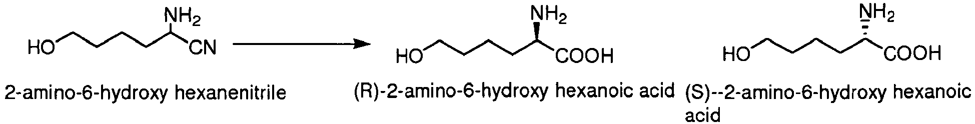

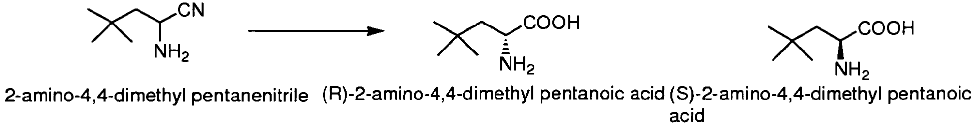

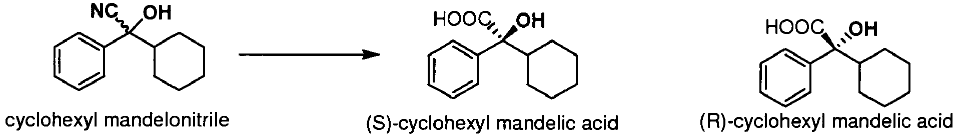

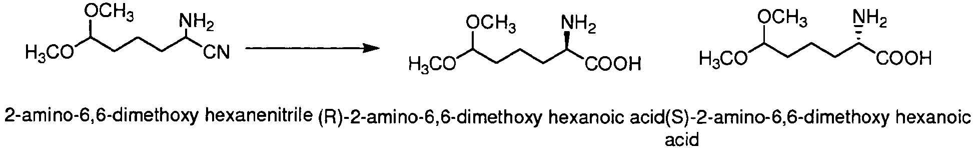

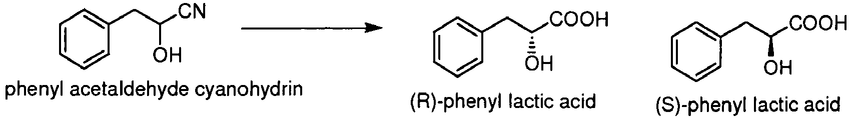

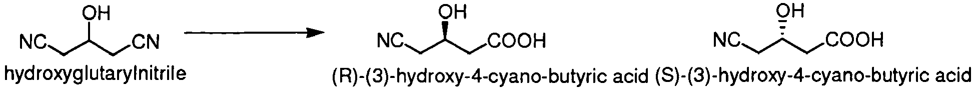

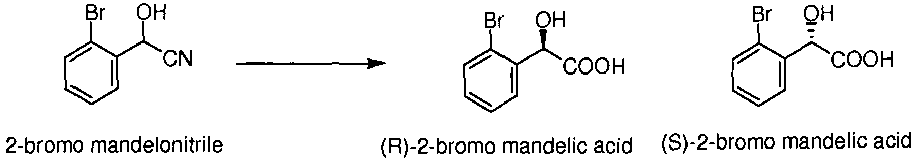

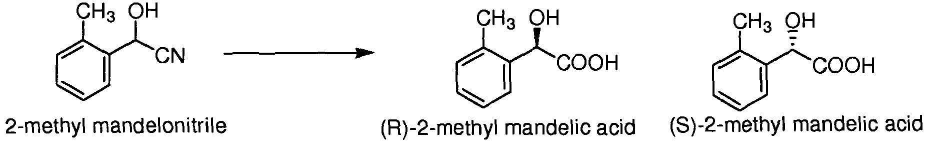

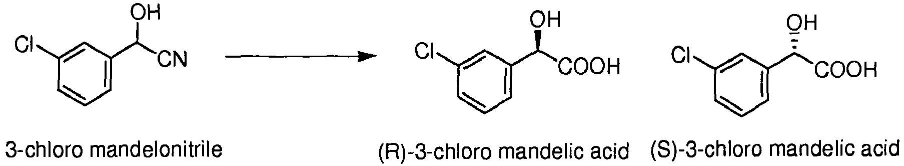

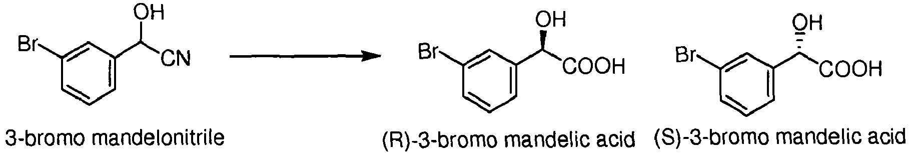

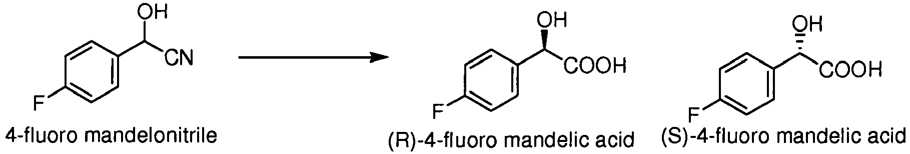

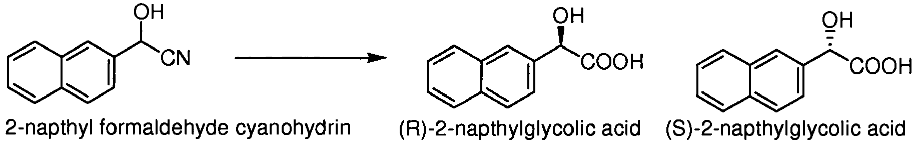

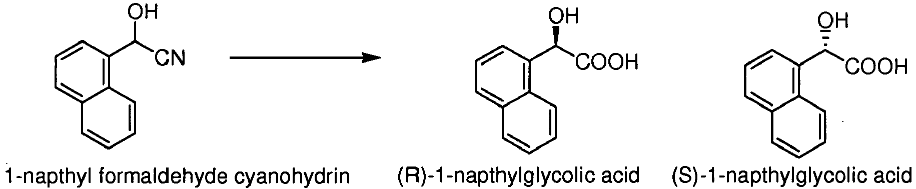

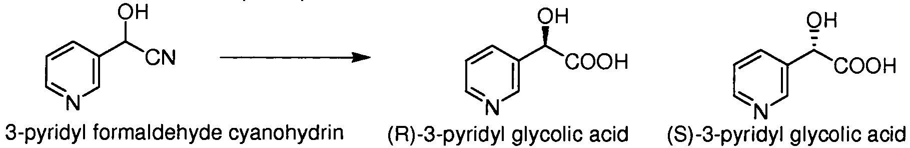

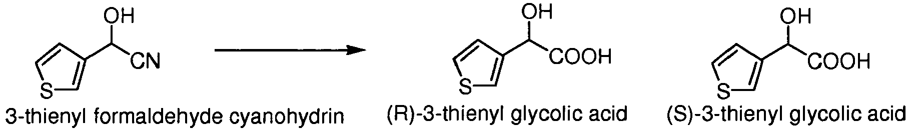

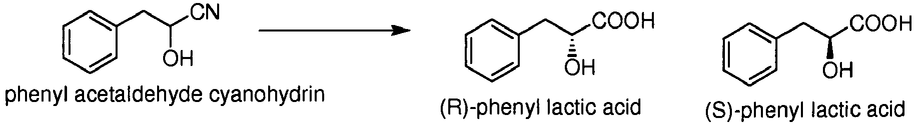

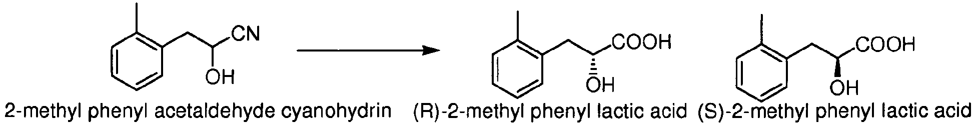

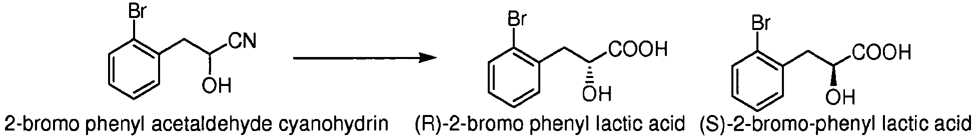

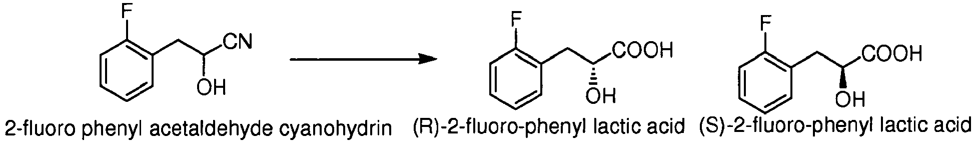

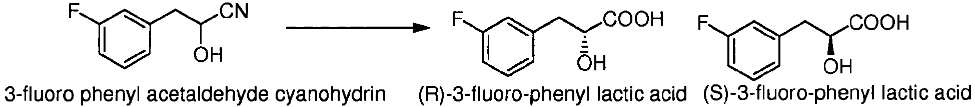

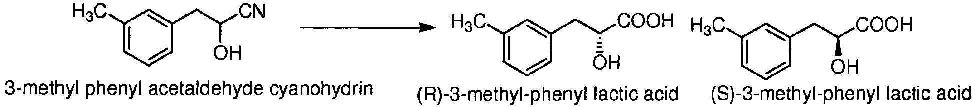

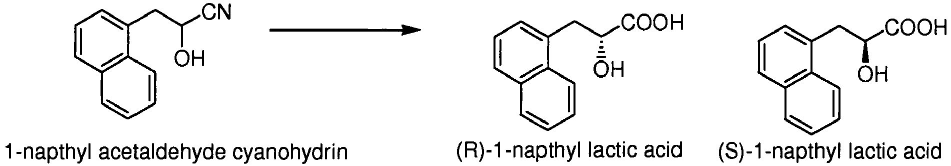

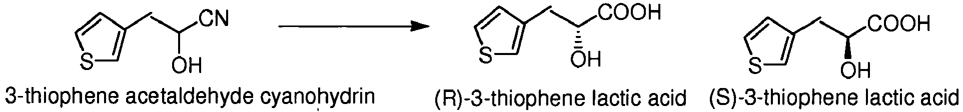

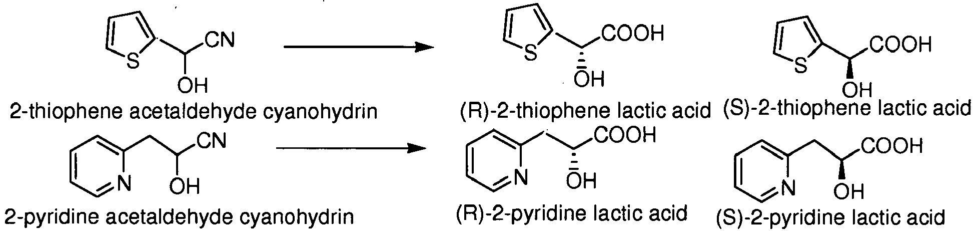

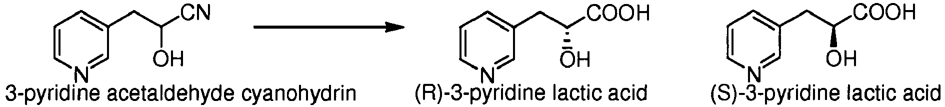

| **Seq ID DNA** | **Seq ID Protein** | | **2-chloromandelic acid** |
|---|---|---|---|
| | | **Yield %** | **ee % (enantiomer)** |
| 5 | 6 | 100% | 68% R |
| 13 | 14 | 100% | 79% R |
| 15 | 16 | 55% | 81% R |
| 33 | 34 | 95% | 61% R |
| 47 | 48 | 43% | 65% R |
| 55 | 56 | 100% | 93% R |
| 85 | 86 | 50% | 52% R |
| 261 | 262 | 100% | 60% R |
| 97 | 98 | 46% | 85% R |
| 279 | 280 | >95% | 40% R |
| 283 | 284 | 70% | 76% R |
| 331 | 332 | 36% | 79% R |
| 291 | 292 | 54% | 74% R |
| 101 | 102 | 53% | 62% R |
| 383 | 384 | 60% | 80% R |
| 385 | 386 | 80% | 97% R |
| 303 | 304 | >95% | 51% R |
| 139 | 140 | 60% | 85% R |
| 167 | 168 | 100% | 88% R |
| 169 | 170 | 72% | 85% R |
| 185 | 186 | 44% | 79% R |
| 187 | 188 | 60% | 78% R |
| 197 | 198 | 100% | 87% R |
| 217 | 218 | 72% | 82% R |

| **Seq ID DNA** | **Seq ID Protein** | | **4-bromo mandelic acid** |
|---|---|---|---|
| | | **Yield %** | **ee % (enantiomer)** |
| 3 | 4 | 12% | 92% R |
| 5 | 6 | 54% | 78% R |
| 333 | 334 | 48% | 93% R |
| 13 | 14 | 55% | 89% R |
| 15 | 16 | 57% | 92% R |
| 19 | 20 | 60% | 92% R |
| 29 | 30 | 58% | 93% R |
| 33 | 34 | 52% | 49% R |
| 47 | 48 | 60% | 92% R |
| 55 | 56 | 56% | 94% R |
| 57 | 58 | 60% | 81% R |
| 85 | 86 | 41% | 84% R |
| 261 | 262 | 48% | 94% R |
| 267 | 268 | 43% | 94% R |
| 97 | 98 | 53% | 91% R |
| 275 | 276 | 37% | 42% R |
| 279 | 280 | 59% | 2% R |
| 283 | 284 | 49% | 94% R |
| 331 | 332 | 42% | 89% R |
| 291 | 292 | 47% | 94% R |
| 101 | 102 | 68% | 77% R |
| 383 | 384 | 67% | 65% R |
| 385 | 386 | 66% | 99% R |
| 303 | 304 | 44% | 6% R |
| 139 | 140 | 61% | 95% R |
| 145 | 146 | 65% | 89% R |
| 167 | 168 | 54% | 92% R |
| 169 | 170 | 53% | 64% R |
| 185 | 186 | 56% | 94% R |
| 187 | 188 | 56% | 59% R |
| 189 | 190 | 59% | 6% R |
| 197 | 198 | 56% | 85% R |
| 215 | 216 | 44% | 65% R |
| 217 | 218 | 57% | 96% R |
| 221 | 222 | 56% | 55% R |
| 223 | 224 | 91% | 1% S |
| 231 | 232 | 8% | 94% R |
| 249 | 250 | 55% | 89% R |

| **Seq ID DNA** | **Seq ID Protein** | | **phenyl glycine** |
|---|---|---|---|
| | | **Yield %** | **ee % (enantiomer)** |
| 5 | 6 | 80% | 10% R |
| 15 | 16 | >90% | no specificity |
| 29 | 30 | 80-100% | < 20% R |
| 47 | 48 | 40-50% | <25% R |
| 55 | 56 | >90% | <20%R |
| 57 | 58 | 60% | 40% R |
| 85 | 86 | 70% | 32% R |
| 101 | 102 | 100% | <20% R |
| 383 | 384 | 75% | 62% R |
| 385 | 386 | 71% | 45% R |
| 139 | 140 | 100% | <20% R |
| 167 | 168 | >90% | <20% R |
| 169 | 170 | 89-90% | <20% R |
| 185 | 186 | ~80% | ~25% R |
| 187 | 188 | 84% | racemic |
| 189 | 190 | ~90% | <10% R |
| 197 | 198 | >90% | 88% R |
| 215 | 216 | 10-20% | < 20% R |
| 217 | 218 | 52% | 86% R |
| 221 | 222 | 20% | R-selective |
| 231 | 232 | 20% | 80% R |

| **Seq ID DNA** | **Seq ID Protein** | | **phenyl methyl glycine** |
|---|---|---|---|
| | | **Yield %** | **ee % (enantiomer)** |
| 189 | 190 | 52% | 45% R |

| **Seq ID DNA** | **Seq ID Protein** | | **4-fluorophenylglycine** |
|---|---|---|---|
| | | **Yield %** | **ee % (enantiomer)** |
| 5 | 6 | 54% | 3% R |
| 333 | 334 | 53% | 2.5% R |
| 13 | 14 | 56% | 2.1% R |
| 33 | 34 | 52.30% | 6.9% R |
| 261 | 262 | 54% | 4.5% R |
| 267 | 268 | 44% | 22% R |
| 97 | 98 | 55% | 2.1% R |
| 279 | 280 | 55% | 7.5% S |
| 283 | 284 | 45% | 0.2% R |
| 291 | 292 | 54% | 0.3% R |
| 303 | 304 | 55% | 3% S |

| **Seq ID DNA** | **Seq ID Protein** | | **2-amino-6-hydroxy-hexanoic acid** |
|---|---|---|---|
| | | **Yield %** | **ee % (enantiomer)** |
| 5 | 6 | 24% | 86% S |
| 333 | 334 | 21% | 85% S |
| 13 | 14 | 16% | 90% S |
| 15 | 16 | <10% | activity observed |
| 29 | 30 | 8% | 78% S |
| 55 | 56 | 80% | 55% S |
| 261 | 262 | 11% | 79% S |
| 361 | 362 | 6% | 72% R |
| 267 | 267 | 25% | 90% S |
| 97 | 98 | 14% | 88% S |
| 279 | 280 | 35% | 93% S |
| 283 | 284 | 14% | 85% S |
| 343 | 344 | 19% | 67% R |
| 101 | 102 | 9% | 83% S |
| 103 | 104 | 30% | 91% S |
| 303 | 304 | 34% | 91% S |
| 145 | 146 | 20% | 85% S |
| 167 | 168 | 35% | 54% S |
| 185 | 186 | 13% | 86% S |
| 187 | 188 | 50% | 60% S |
| 189 | 190 | 5% | 62% S |
| 197 | 198 | 12% | 88% S |
| 217 | 218 | 100% | 52% S |
| 221 | 222 | 32% | 79% S |
| 249 | 250 | 8% | 87% S |

| **Seq ID DNA** | **Seq ID Protein** | | **2-amino-4,4-dimethyl pentanoic acid** |
|---|---|---|---|
| | | **Yield %** | **ee % (enantiomer)** |
| 55 | 56 | ~40% | <20% S |
| 59 | 60 | 30% | >95% S |
| 267 | 268 | 25% | 90% S |
| 103 | 104 | 30% | 91% S |
| 167 | 168 | ~40% | <20% S |
| 221 | 222 | 32% | 79% S |

| **Seq ID DNA** | **Seq ID Protein** | | **phenyl lactic acid** |
|---|---|---|---|
| | | **Yield %** | **ee % (enantiomer)** |
| 321 | 322 | 33% | 56% S |
| 23 | 24 | 20% | 5% S |
| 31 | 32 | 36% | 68% R |
| 39 | 40 | 17% | 5% S |
| 293 | 294 | 100% | 65% S |
| 41 | 42 | 35% | 45% R |
| 43 | 44 | 40% | 85% S |
| 49 | 50 | 75% | 66% S |
| 61 | 62 | 56% | 80% S |
| 73 | 74 | 100% | 5% R |
| 259 | 260 | 95% | 33% S |
| 335 | 336 | 96% | 62% S |
| 83 | 84 | 100% | 49% S |
| 93 | 94 | 80% | 50% S |
| 95 | 96 | 57% | 60% R |
| 271 | 272 | 75% | 60% R |
| 273 | 274 | 100% | 45% S |
| 275 | 276 | 20% | 3% S |
| 99 | 100 | 90% | 82% S |
| 107 | 108 | 80% | 40% S |
| 109 | 110 | 80% | 60% S |
| 115 | 116 | 60% | 63% S |
| 117 | 118 | 20% | 4% S |
| 125 | 126 | 20% | 6% S |
| 127 | 128 | 20% | 8% S |
| 129 | 130 | 20% | 9% S |
| 133 | 134 | 30% | 8% S |
| 135 | 136 | 30% | 7% S |
| 113 | 114 | 20% | 20% S |
| 161 | 162 | 70% | 66% S |
| 171 | 172 | 52% | 60% R |
| 173 | 174 | 20% | 83% S |
| 175 | 176 | 87% | 45% S |
| 183 | 184 | 50% | 57% S |
| 189 | 190 | 20% | 8% S |
| 195 | 196 | 89% | 89% S |
| 205 | 206 | 90% | 73% S |
| 207 | 208 | 76% | 85% S |
| 209 | 210 | 98% | 75% S |
| 213 | 214 | 70% | 86% S |
| 227 | 228 | 99% | 31% S |
| 239 | 240 | 22% | 100% R |
| 241 | 242 | 40% | 62% R |

| **Seq ID DNA** | **Seq ID Protein** | | **Cyclohexylmandelic acid** |
|---|---|---|---|
| | | **Yield %** | **ee % (enantiomer)** |
| 17 | 18 | 60% | Not determined |
| 321 | 322 | 70% | Not determined |
| 49 | 50 | 70% | Not determined |
| 61 | 62 | 70% | Not determined |
| 105 | 106 | >90% | Not determined |
| 115 | 116 | 70% | Not determined |
| 195 | 196 | 55% | Not determined |
| 213 | 214 | 65% | Not determined |
| 237 | 238 | 60% | Not determined |

| **Seq ID DNA** | **Seq ID Protein** | | **mandelic acid** |
|---|---|---|---|
| | | **EE % (enantiomer)** | **YIELD %** |
| 3 | 4 | 1 S | 22 |
| 5 | 6 | 97 R | 100 |
| 333 | 334 | 93 R | 100 |
| 9 | 10 | 8 R | 21 |
| 13 | 14 | 98 R | 100 |
| 15 | 16 | 96 R | 100 |
| 29 | 30 | 99 R | 100 |
| 33 | 34 | 95 R | 100 |
| 35 | 36 | 3 S | 15 |
| 39 | 40 | 35 S | 14 |
| 47 | 48 | 97 R | 100 |
| 55 | 56 | 99 R | 100 |
| 57 | 58 | 96 R | 100 |
| 75 | 76 | 52 R | 56 |
| 257 | 258 | 26 S | 12 |
| 81 | 82 | 16 S | 23 |
| 259 | 260 | 47 S | 15 |
| 83 | 84 | 46 S | 14 |
| 85 | 86 | 83 R | 20 |
| 261 | 262 | 88 R | 100 |
| 361 | 362 | 14 S | 22 |
| 267 | 268 | 96 R | 100 |
| 97 | 98 | 99 R | 100 |
| 277 | 278 | 41 R | 13 |
| 279 | 280 | 28 R | 100 |
| 283 | 284 | 94 R | 100 |
| 331 | 332 | 87 S | 100 |
| 299 | 300 | 68 S | 12 |
| 351 | 352 | 89 R | 100 |
| 317 | 318 | 55 S | 14 |
| 343 | 344 | 21 S | 49 |
| 291 | 292 | 81 R | 100 |
| 287 | 288 | 8 S | 22 |
| 383 | 384 | 80 R | 100 |
| 119 | 120 | 31 R | 14 |
| 385 | 386 | 99 R | 100 |
| 303 | 304 | 45 R | 100 |
| 139 | 140 | 97 R | 100 |
| 145 | 146 | 95 R | 100 |
| 167 | 168 | 99 R | 100 |
| 169 | 170 | 99 R | 100 |
| 185 | 186 | 99 R | 100 |
| 187 | 188 | 99 R | 100 |
| 189 | 190 | 9 R | 100 |
| 197 | 198 | 99 R | 100 |
| 209 | 210 | 15 S | 9 |
| 215 | 216 | 88 R | 100 |
| 217 | 218 | 98 R | 100 |
| 221 | 222 | 85 R | 58 |
| 225 | 226 | 36 S | 14 |
| 231 | 232 | 91 R | 100 |
| 235 | 236 | 87 S | 22 |
| 237 | 238 | 18 S | 8 |
| 239 | 240 | 35 S | 16 |
| 249 | 250 | 98 R | 100 |

| **NOTES** | **Seq ID DNA** | **Seq ID Protein** | | **3-hydroxy-4-cyanobutyric acid** |
|---|---|---|---|---|
| | | | **EE % (enantiomer)** | **YIELD %** |
| | 1 | 2 | not determined | activity observed |
| | 3 | 4 | 4 S | activity observed |
| | 7 | 8 | 6 S | activity observed |
| | 333 | 334 | 6 S | activity observed |
| | 9 | 10 | 16 S | activity observed |
| | 11 | 12 | 8 R | 104 |
| | 15 | 16 | not determined | activity observed |
| | 17 | 18 | 16 R | activity observed |
| | 19 | 20 | 7 R | activity observed |
| | 21 | 22 | 6 S | activity observed |
| | 321 | 322 | 51 R | activity observed |
| | 25 | 26 | 19 S | activity observed |
| | 27 | 28 | 6 S | activity observed |
| | 27 | 28 | 100 S | 111 |
| | 29 | 30 | 16 R | 100 |
| | 31 | 32 | 54 R | 127 |
| | 33 | 34 | 12 S | activity observed |
| | 35 | 36 | 100 S | 35 |
| | 37 | 38 | 100 S | 87 |
| | 39 | 40 | 24 S | activity observed |
| | 293 | 294 | 12 S | 91 |
| | 255 | 256 | 65 S | activity observed |
| | 41 | 42 | 100 R | 16 |
| | 43 | 44 | not determined | activity observed |
| | 47 | 48 | 38 S | 96 |
| | 49 | 50 | 52 S | activity observed |
| | 55 | 56 | 92 R | 122 |
| | 57 | 58 | 100 S | 73 |
| | 59 | 60 | 100 S | 100 |
| | 61 | 62 | 18 R | activity observed |
| | 63 | 64 | 10 R | 9 |
| | 69 | 70 | 2 S | activity observed |
| | 71 | 72 | not determined | activity observed |
| | 73 | 74 | 100 S | 3 |
| | 325 | 326 | 73 R | 4 |
| | 77 | 78 | 100 S | activity observed |
| | 257 | 258 | 3 R | activity observed |
| | 259 | 260 | 55 S | activity observed |
| | 83 | 84 | 34 S | activity observed |
| | 261 | 262 | 22 R | activity observed |
| | 361 | 362 | 4 S | activity observed |
| | 89 | 90 | 31 R | 116 |
| | 297 | 298 | 24 R | activity observed |
| | 91 | 92 | 6 S | activity observed |
| | 267 | 268 | not determined | activity observed |
| | 93 | 94 | 58 S | activity observed |
| | 95 | 96 | 21 R | activity observed |
| | 97 | 98 | 17 R | activity observed |
| | 275 | 276 | 80 S | activity observed |
| | 279 | 280 | 9 S | activity observed |
| | 281 | 282 | not determined | activity observed |
| | 283 | 284 | 23 R | activity observed |
| | 313 | 314 | 12S | activity observed |
| | 351 | 352 | 11 S | activity observed |
| | 309 | 310 | 28 S | activity observed |
| | 291 | 292 | 13 S | activity observed |
| | 287 | 288 | 100 S | activity observed |
| | 99 | 100 | 100 S | activity observed |
| | 101 | 102 | 80 S | 61 |
| | 383 | 384 | 8 R | activity observed |
| | 103 | 104 | 100 R | 98 |
| | 105 | 106 | 13 S | activity observed |
| | 107 | 108 | 100 S | 79 |
| | 109 | 110 | 100 S | 79 |
| | 111 | 112 | 91 S | 32 |
| | 113 | 114 | 100 S | activity observed |
| | 115 | 116 | 66 S | activity observed |
| | 117 | 118 | 22 R | activity observed |
| Different subclone | 119 | 120 | 13S | activity observed |
| Different subclone | 119 | 120 | 100 S | activity observed |
| | 123 | 124 | 20 S | activity observed |
| | 385 | 386 | 13 S | activity observed |
| | 125 | 126 | 25 S | activity observed |
| | 127 | 128 | 92 S | 106 |
| | 129 | 130 | 100 S | 22 |
| | 131 | 132 | 100 S | activity observed |
| | 133 | 134 | 86 S | 14 |
| | 135 | 136 | 22 S | activity observed |
| | 139 | 140 | 16 S | activity observed |
| | 113 | 114 | 100 S | 108 |
| | 143 | 144 | 100 S | activity observed |
| | 145 | 146 | 100 S | 100 |
| | 149 | 150 | 3.2 S | activity observed |
| | 151 | 152 | 8 R | activity observed |
| | 153 | 154 | 35 S | 39 |
| | 155 | 156 | 100 S | activity observed |
| | 157 | 158 | 26 R | activity observed |
| | 159 | 160 | 100 S | 71 |
| | 161 | 162 | 64 R | 122 |
| | 163 | 164 | 10S | activity observed |
| | 167 | 168 | 10R | 106 |
| | 169 | 170 | 14 R | 38 |
| | 171 | 172 | 27 R | 33 |
| | 173 | 174 | 20 R | activity observed |
| | 175 | 176 | 31 S | 58 |
| | 177 | 178 | 100 S | 11 |
| | 179 | 180 | 100 S | 75 |
| | 181 | 182 | 100 S | 58 |
| | 183 | 184 | 100 S | 19 |
| | 185 | 186 | 100 S | 78 |
| | 187 | 188 | 7 S | activity observed |
| | 189 | 190 | 5 S | 104 |
| | 193 | 194 | 7 S | activity observed |
| | 195 | 196 | 95 R | 100 |
| | 197 | 198 | 100 S | 64 |
| | 201 | 202 | 100 S | 132 |
| | 253 | 254 | 4 S | activity observed |
| | 203 | 204 | not determined | activity observed |
| | 205 | 206 | 64 R | activity observed |
| | 207 | 208 | 95 R | 100 |
| | 209 | 210 | 95 R | 100 |
| | 213 | 214 | 25 R | activity observed |
| | 215 | 216 | 100 S | activity observed |
| | 217 | 218 | 11 S | 109 |
| | 219 | 220 | not determined | activity observed |
| | 221 | 222 | 26 R | 100 |
| | 223 | 224 | 5 S | activity observed |
| | 227 | 228 | 52 S | activity observed |
| Clone | 229 | 230 | 31 S | activity observed |
| Subclone | 229 | 230 | 100 S | activity observed |
| | 231 | 232 | 100 S | activity observed |
| | 233 | 234 | 35 S | activity observed |
| | 235 | 236 | 6 S | activity observed |
| | 237 | 238 | 95 R | 100 |
| | 239 | 240 | not determined | activity observed |
| | 241 | 242 | 9R | activity observed |
| | 243 | 244 | 100 S | activity observed |
| | 245 | 246 | 5 S | activity observed |
| | 247 | 248 | not determined | activity observed |
| | 249 | 250 | 21 S | 98 |
| | 251 | 252 | 5 S | activity observed |

The indications of subclones and clones refer to subclones of the originally isolated nucleic acid of the respective SEQ ID NO.

| **Seq ID DNA** | **Seq ID Protein** | | **2-bromo mandelic acid** |
|---|---|---|---|
| | | **EE % (enantiomer)** | **YIELD %** |
| 5 | 6 | 82 R | 63 |
| 97 | 98 | 95 R | 100 |
| 101 | 102 | 92 R | 92 |
| 385 | 386 | 96 R | 100 |
| 185 | 186 | 92 R | 90 |
| 187 | 188 | 90 R | 100 |
| 197 | 198 | 93 R | 74 |
| 217 | 218 | 90 R | 100 |
| 235 | 236 | 91 R | 30 |

| **Seq ID DNA** | **Seq ID Protein** | | **2-methyl mandelic acid** |
|---|---|---|---|
| | | **EE% (enantiomer)** | **YIELD %** |
| 5 | 6 | 85 R | 100 |
| 55 | 56 | 90 R | 100 |
| 97 | 98 | 93 R | 100 |
| 383 | 384 | 97 R | 100 |
| 385 | 386 | 97 R | 100 |
| 139 | 140 | 88 R | 100 |
| 145 | 146 | 93 R | 100 |
| 167 | 168 | 85 R | 100 |
| 185 | 186 | 93 R | 95 |
| 187 | 188 | 96 R | 100 |
| 197 | 198 | 86 R | 100 |
| 217 | 218 | 80 R | 100 |

| **Seq ID Protein** | | **3-chloro mandelic acid** |
|---|---|---|
| | **EE % (enantiomer)** | **YIELD %** |
| 386 | 98 R | 100 |

| **Seq ID DNA** | **Seq ID Protein** | | **3-bromo mandelic acid** |
|---|---|---|---|
| | | **EE% (enantiomer)** | **YIELD %** |
| 385 | 386 | 99 R | 100 |

| **Seq ID DNA** | **Seq ID Protein** | | **4-fluoromandelic acid** |
|---|---|---|---|
| | | **EE% (enantiomer)** | **YIELD %** |
| 5 | 6 | 82 R | 50 |
| 15 | 16 | 92 R | 45 |
| 55 | 56 | 97 R | 45 |
| 85 | 86 | 97 R | 40 |
| 97 | 98 | 98 R | 45 |
| 101 | 102 | 95 R | 50 |
| 385 | 386 | 99 R | 100 |
| 167 | 168 | 97 R | 50 |
| 185 | 186 | 97 R | 50 |
| 187 | 188 | 95 R | 50 |
| 197 | 198 | 79 R | 45 |
| 215 | 216 | 89 R | 40 |
| 217 | 218 | 98 R | 50 |
| 221 | 222 | 68 R | 45 |

| **Seq ID DNA** | **Seq ID Protein** | | **2-napthyl glycolic acid** |
|---|---|---|---|
| | | **EE% (enantiomer)** | **YIELD %** |
| 13 | 14 | 95 R | 85 |
| 97 | 98 | 93 R | 40 |
| 101 | 102 | 96R | 100 |
| 383 | 384 | 98 R | 100 |
| 385 | 386 | 98 R | 100 |
| 125 | 126 | 95 R | 20 |
| 127 | 128 | 75 R | 4 |
| 133 | 134 | 97 R | 20 |
| 145 | 146 | 96R | 100 |
| 169 | 170 | 97 R | 100 |
| 187 | 188 | 95 R | 100 |
| 201 | 202 | 98 R | 9 |

| **Seq ID DNA** | **Seq ID Protein** | | **1- napthyl glycolic acid** |
|---|---|---|---|
| | | **EE % (enantiomer)** | **YIELD %** |
| 13 | 14 | 82 R | 100 |
| 97 | 98 | 84 R | 100 |
| 101 | 102 | 69 R | 100 |
| 383 | 384 | 46 R | 100 |
| 385 | 386 | 95 R | 100 |
| 125 | 126 | 83 R | 16 |
| 127 | 128 | 33 R | 13 |
| 133 | 134 | 42 R | 16 |
| 145 | 146 | 69 R | 100 |
| 169 | 170 | 62 R | 100 |
| 187 | 188 | 55 R | 100 |
| 201 | 202 | 59 R | 15 |

| **Seq ID DNA** | **Seq ID Protein** | | **3-pyridyl glycolic acid** |
|---|---|---|---|
| | | **EE % (enantiomer)** | **YIELD %** |
| 5 | 6 | 94 R | 100 |
| 13 | 14 | 94 R | 100 |
| 29 | 30 | 95 R | 95 |
| 47 | 48 | 95 R | 100 |
| 55 | 56 | 92 R | 85 |
| 57 | 58 | 95 R | 100 |
| 97 | 98 | 96 R | 100 |
| 383 | 384 | 95 R | 100 |
| 385 | 386 | 96 R | 100 |
| 139 | 140 | 90 R | 100 |
| 145 | 146 | 89 R | 100 |
| 167 | 168 | 94 R | 100 |
| 169 | 170 | 91 R | 70 |
| 185 | 186 | 93 R | 90 |
| 187 | 188 | 95 R | 100 |
| 197 | 198 | 93 R | 100 |
| 217 | 218 | 94 R | 100 |
| 249 | 250 | 94 R | 90 |

| **Seq ID DNA** | **Seq ID Protein** | | **3-thienylglycolic acid** |
|---|---|---|---|
| | | **EE% (enantiomer)** | **YIELD %** |
| 385 | 386 | 95 R | 100 |

| **Seq ID DNA** | **Seq ID Protein** | | **2-pyridyl glycolic acid** |
|---|---|---|---|
| | | **EE % (enantiomer)** | **YIELD %** |
| 385 | 386 | 95 R | 100 |

| NOTES | **Seq ID DNA** | **Seq ID Protein** | | **2-hydroxy-4-phenyl butyric acid** |
|---|---|---|---|---|
| | | | **EE % (enantiomer)** | **YIELD %** |
| | 1 | 2 | 66 S | 92 |
| | 3 | 4 | 82 S | 12 |
| | 5 | 6 | 85 S | 100 |
| | 7 | 8 | 81 S | 13 |
| | 333 | 334 | 85 S | 100 |
| | 9 | 10 | 88 S | 13 |
| | 13 | 14 | 82 S | 100 |
| | 15 | 16 | 72 S | 22 |
| | 17 | 18 | 67 S | 100 |
| | 19 | 20 | 90 S | 81 |
| | 21 | 22 | 69 S | 32 |
| | 321 | 322 | 66 S | 100 |
| | 23 | 24 | 71 S | 27 |
| | 25 | 26 | 69 S | 70 |
| | 27 | 28 | 72 S | 20 |
| | 29 | 30 | 91 S | 100 |
| | 31 | 32 | 13 S | 100 |
| | 33 | 34 | 83 S | 38 |
| | 35 | 36 | 69 S | 26 |
| | 37 | 38 | 66 S | 76 |
| | 39 | 40 | 3 S | 110 |
| | 293 | 294 | not determined | 100 |
| | 307 | 308 | 56 S | 54 |
| | 255 | 256 | 74 S | 27 |
| | 41 | 42 | 82 S | 100 |
| | 43 | 44 | 41 S | 100 |
| | 45 | 46 | 85 S | 17 |
| | 47 | 48 | 87 S | 76 |
| | 49 | 50 | 73 S | 17 |
| | 51 | 52 | 70 S | 100 |
| | 53 | 54 | 84 S | 12 |
| | 55 | 56 | 84 S | 100 |
| | 57 | 58 | 91 S | 100 |
| | 59 | 60 | 56 S | 100 |
| | 61 | 62 | 72 S | 65 |
| | 63 | 64 | 87 S | 20 |
| | 359 | 360 | 63 S | 79 |
| | 67 | 68 | 82 S | 19 |
| | 69 | 70 | 66 S | 72 |
| | 71 | 72 | 83 S | 13 |
| | 73 | 74 | 75 S | 26 |
| | 325 | 326 | 87 S | 12 |
| | 75 | 76 | 39 S | 42 |
| | 77 | 78 | 85 S | 23 |
| | 79 | 80 | 85 S | 14 |
| | 257 | 258 | 51 S | 71 |
| | 81 | 82 | 73 S | 51 |
| | 259 | 260 | 51 S | 46 |
| | 335 | 336 | 65 S | 69 |
| | 83 | 84 | 86 S | 10 |
| | 85 | 86 | 66 S | 20 |
| | 261 | 262 | 77 S | 100 |
| | 87 | 88 | 90 S | 16 |
| | 361 | 362 | 67 S | 76 |
| | 89 | 90 | 44 S | 47 |
| | 297 | 298 | 69 S | 100 |
| | 91 | 92 | 65 S | 81 |
| | 267 | 268 | 72 S | 100 |
| | 93 | 94 | 90 S | 17 |
| | 95 | 96 | 66 S | 22 |
| | 271 | 272 | 53 S | 38 |
| | 97 | 98 | 93 S | 100 |
| | 273 | 274 | 84 S | 22 |
| | 275 | 276 | 5 S | 100 |
| | 277 | 278 | 84 S | 20 |
| | 279 | 280 | 75 S | 100 |
| | 281 | 282 | 65 S | 91 |
| | 283 | 284 | 88 S | 100 |
| | 331 | 332 | 21 S | 100 |
| | 311 | 312 | 50 S | 32 |
| | 313 | 314 | 88 S | 100 |
| | 323 | 324 | 67 S | 31 |
| | 329 | 330 | 62 S | 77 |
| | 289 | 290 | 58 S | 70 |
| | 299 | 300 | 71 S | 33 |
| | 351 | 352 | 54 S | 80 |
| | 317 | 318 | 57 S | 18 |
| | 309 | 310 | 58 S | 100 |
| | 343 | 344 | 20 S | 100 |
| | 291 | 292 | 73 S | 27 |
| | 287 | 288 | 41 S | 52 |
| | 99 | 100 | 87 S | 14 |
| | 101 | 102 | 86 S | 100 |
| | 383 | 384 | 70 S | 69 |
| | 103 | 104 | 79 S | 100 |
| | 105 | 106 | 92 S | 74 |
| | 107 | 108 | 87 S | 14 |
| | 109 | 110 | 64 S | 76 |
| | 111 | 112 | 67 S | 81 |
| | 113 | 114 | 63 S | 79 |
| | 115 | 116 | 86 S | 34 |
| | 117 | 118 | 80 S | 21 |
| Different subclone | 119 | 120 | 87 S | 9 |
| Different subclone | 119 | 120 | 7 S | 100 |
| | 121 | 122 | 74 S | 21 |
| | 123 | 124 | 86 S | 12 |
| | 385 | 386 | 63 S | 58 |
| | 125 | 126 | 86 S | 23 |
| | 303 | 304 | 68 S | 100 |
| | 127 | 128 | 73 S | 25 |
| | 129 | 130 | 75 S | 24 |
| | 131 | 132 | 76 S | 22 |
| | 133 | 134 | 88 S | 18 |
| | 135 | 136 | 65 S | 91 |
| | 113 | 114 | 87 S | 20 |
| | 143 | 144 | 60 S | 47 |
| | 145 | 146 | 74 S | 34 |
| | 149 | 150 | 75 S | 100 |
| | 151 | 152 | 74 S | 31 |
| | 153 | 154 | 69 S | 100 |
| | 155 | 156 | 69 S | 74 |
| | 157 | 158 | 62 S | 100 |
| | 159 | 160 | 9 S | 100 |
| | 163 | 164 | 87 S | 21 |
| Different subclone | 165 | 166 | 76 S | 31 |
| Different subclone | 165 | 166 | 59 S | 100 |
| | 167 | 168 | 80 S | 100 |
| | 169 | 170 | 84 S | 23 |
| | 171 | 172 | 8S | 100 |
| | 173 | 174 | 78 S | 67 |
| | 175 | 176 | 84 S | 23 |
| | 177 | 178 | 100 S | 88 |
| | 179 | 180 | 16S | 100 |
| | 183 | 184 | 76 S | 100 |
| | 185 | 186 | 87 S | 100 |
| | 187 | 188 | 80 S | 100 |
| | 193 | 194 | 71 S | 9 |
| | 195 | 196 | 51 S | 100 |
| | 197 | 198 | 73 S | 100 |
| | 201 | 202 | 57 S | 100 |
| | 253 | 254 | 70 S | 32 |
| | 205 | 206 | 83 S | 21 |
| | 209 | 210 | 72 S | 70 |
| | 215 | 216 | 84 S | 19 |
| | 217 | 218 | 91 S | 100 |
| | 219 | 220 | 65 S | 93 |
| | 221 | 222 | 54 S | 100 |
| | 223 | 224 | 69 S | 37 |
| | 225 | 226 | 82 S | 15 |
| | 227 | 228 | 83 S | 23 |
| Clone | 229 | 230 | 78 S | 20 |
| Subclone | 229 | 230 | 74 S | 100 |
| | 231 | 232 | 81 S | 21 |
| | 233 | 234 | 84 S | 19 |
| | 235 | 236 | 61 S | 82 |
| | 237 | 238 | 51 S | 90 |
| | 239 | 240 | 89 S | 16 |
| | 241 | 242 | 24 S | 66 |
| | 243 | 244 | 88 S | 16 |
| | 245 | 246 | 74 S | 23 |
| | 247 | 248 | 72 S | 78 |
| | 249 | 250 | 93 S | 100 |
| | 251 | 252 | 71 S | 17 |

| **Seq ID DNA** | **Seq ID Protein** | **2-amino hexanoic acid** | |
|---|---|---|---|
| | | **Yield** | **EE % (enantiomer)** |
| 5 | 6 | 27% | 11% R |
| 7 | 8 | 9% | 45% S |
| 333 | 334 | 36% | 21% R |
| 11 | 12 | 9% | 58% S |
| 13 | 14 | 23% | 3% S |
| 293 | 294 | 28% | 9% R |
| 47 | 48 | 20% | 15% S |
| 55 | 56 | 27% | 14% R |
| 61 | 62 | 9% | 46% S |
| 261 | 262 | 17% | 39% S |
| 267 | 268 | 11% | 83% S |
| 97 | 98 | 21% | 15% S |
| 283 | 284 | 29% | 20% R |
| 343 | 344 | 21% | 79% R |
| 101 | 102 | 26% | 1% R |
| 103 | 104 | 8% | 91% S |
| 385 | 386 | 20% | 24% R |
| 303 | 304 | 24% | 13% S |
| 145 | 146 | 29% | 10% R |
| 159 | 160 | 14% | 40% S |
| 167 | 168 | 21% | 16% S |
| 169 | 170 | 12% | 53% S |
| 185 | 186 | 37% | 29% S |
| 187 | 188 | 25% | 13% R |
| 189 | 190 | 14% | 68% S |
| 197 | 198 | 28% | 21% R |
| 217 | 218 | 22% | 13% R |
| 221 | 222 | 17% | 54% S |
| 249 | 250 | 20% | 29% S |

While the invention has been described in detail with reference to certain preferred embodiments thereof, it will be understood that modifications and variations are within the scope of that which is described and claimed.

## Claims

1. An isolated, synthetic or recombinant nucleic acid comprising
(a) a nucleic acid sequence at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, or 100% sequence identity to SEQ ID NO:297 and encoding a polypeptide having nitrilase activity, wherein the nucleic acid encodes at least one polypeptide having a nitrilase activity; or
(b) a nucleic acid sequence completely complementary to (a).

2. An expression cassette, a vector or a cloning vehicle comprising a nucleic acid comprising a sequence as set forth in claim 1.

3. A transformed cell comprising
(i) a nucleic acid comprising a sequence as set forth in claim 1 or an expression cassette, a vector or a cloning vehicle as set forth in claim 2;
(ii) the transformed cell of (i), where the cell is
(a) a gram negative bacteria, a gram positive bacteria or a eukaryotic monocellular, organism;
(b) an *Escherichia coli;*
(c) a *Streptomyces diversa, Lactobacihus gasseri, Lactococcus lactis, Lactococcus cremoris,* or a *Bacillus subtilis;* or
(d) a *Saccharomyces cerevisiae, Schizosaccharomyces pombe; Pichia pastoris, Kluyveromyces lactic, Hansenula polymorpha,* or an *Aspergillus niger.*

4. A transgenic non-human organism comprising a sequence as set forth in claim 1 or an expression cassette, a vector or a cloning vehicle as set forth in claim 2.

5. An isolated, synthetic or Recombinant polypeptide comprising:
(a) a sequence comprising at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or more, or 100% sequence identity to SEQ ID NO: 298, wherein the polypeptide has a nitrilase activity; or
(b) an amino acid encoded by the nucleic acid of claim 1 (a).

6. The isolated, synthetic or recombinant polypeptide of claim 5, wherein the nitrilase activity comprises
(a) hydrolyzing a nitrile to a carboxylic acid;
(b) hydrolyzing a cyanhydrin moiety or an aminonitrile moiety of a molecule, wherein optionally the molecule comprises (*S*)-2-amino-40 phenyl bunatoic acid, or the molecule comprises an L-amino acid, or the molecule comprises a food additive or a pharmaceutical drug; or
(c) an enantio-selective nitrilase activity wherein the enantio-selective activity comprises generating a chiral alpha-hydroxy acid molecule or a chiral amino acid molecule by catalyzing the hydrolysis of a molecule having a cyanohydrin moiety or an aminonitrile moiety,
wherein optionally the generated chiral molecule is an (*R)*-enantiomer or an (*S*)-enantiomer.

7. The isolated, synthetic or recombinant polypeptide of claim 5 or claim 6, wherein the nitrilase activity comprises hydrolyzing a hydroxyglutaryl nitrile to generate, an (*R*)-ethyl 4-cyano-3-hydroxybutyric acid,
wherein optionally the polypeptide enantio-selectively hydrolyzes the hydroxyglutaryl nitrile to generate an (*R*)-enantiomer (*R*)-ehtyl 4-cyano-3-hydroxybutyric acid,
wherein optionally the polypeptide produces a product having an enantiomeric purity comprising an *ee* of at least 95%, 96%, 97%, 98% or 99%, and
wherein optionally the hydroxyglutaryle nitrile comprises 1,3-di-cyano-2-hydroxy-propane or a hydoxyglutaronitile, or
wherein optionally the hydroxyglutaronitrile comprise a 3-hydroxyglutaronitrile.

8. The isolated, synthetic or recombinant polypeptide of claim 5 or claim 6, wherein the nitrilase activity comprises hydrolyzing a hydroxyglutaryl nitrile to generate an ethyl 4-cyano-3-hydroxybutyric acid,
wherein optionally the polypeptide enantio-selectively hydrolyzes the hycitoxyglutaryl nitrile to generate an (*S*)-enantiomer (*S*)-methyl 4-cyano-3-hydroxybutyric acid, and
wherein optionally the polypeptide produces a product having an enantiomeric purity comprising an *ee* of 95%, 96%, 97%, 98% or 99%.

9. The isolated, synthetic or recombinant polypeptide of claim 5 or claim 6, wherein the nitrilase activity comprises hydrolyzing a mandelonitrile to generate a mandelic acid or mandelic acid derivative,
wherein optionally the mandelic acid comprises (*R*)-2-chloromandelic acid,
wherein optionally the mandelic acid comprises an aromatic ring substitution in the *ortho-, meta-* or *para-* positions; a 1-paphthyl derivative of (*R*)-mandelic acid, a pyridyl derivative of (*R*)-mandelic acid or thienyl derivative of (*R*)-mandelic acid or a combination thereof,
wherein optionally the mandelic acid comprises (*S*)-methyl benzyl cyanide and the mandelonitrile comprises (*S*)-methoxy-benzyl cyanide, or
wherein optionally the mandelic acid comprises an aromatic ring substitution in the *ortho-, meta-* or *para*- positions; a 1-paphthyl derivative of (*S*)-mandelic acid, a pyridyl derivative of (*S*)-mandelic acid or thienyl derivative of (*S*)-mandelic acid or a combination thereof.

10. The isolated, synthetic or recombinant polypeptide of claim 5 or claim 6, wherein the nitrilase activity comprises hydrolyzing a phenyllactocyanonitrile to generate an (*S*)-phenyl lactic acid derivative or an (*R*)-phenyl lactic acid derivative.

11. An enzyme preparation comprising a polypeptide as set forth in any of claims 5 to 10, wherein the preparation is a liquid or a dry preparation.

12. An industrial process comprising using the isolated, synthetic or recombinant polypeptide as set forth in any of claims 5 to 10, in the hydrolysis of a substrate having a cyanohydrin moiety or an aminonitrile moiety to generate a composition comprising an alpha-hydroxy acid or an amino acid.

13. The industrial process of claim 12, wherein the industrial process further comprises:
(i) the production of a pharmaceutical composition comprising the generated alpha-hydroxy acid or amino acid,
(ii) the production of a chemical comprising the generated alpha-hydroxy acid or amino acid,
(iii) catalyzing the breakdown of a waste, wherein the breakdown comprises hydrolysis of a substrate having a cyanohydrin moiety or an aminonitrile moiety,
(iv) the production of a drug intermediate, wherein the drug intermediate comprises the generated alpha-hydroxy acid or amino acid, and wherein optionally the polypeptide is used to hydrolyze a hydroxygultarylnitrile substrate,
(v) the production of a detergent, or
(vi) the production of a food product.

14. The isolated, synthetic or recombinant polypeptide of claim 5 encoded by a nucleic acid expressed in a host cell, wherein the host cell comprises:
(a) a gram negative bacteria, a gram positive bacteria or a eukaryotic monocellular organism;
(b) an *Escherichia coli;*
(c) a *Streptomyces diversa, Lactobacillus gasseri, Lactococcus lactis, Lactococcus cremoris,* or a *Bacillus subtilis;* or
(d) *a Saccharomyces cerevisiae, Schizosaccharomyces pombe, Pichia pastoris, Kluyveromyces lactis. Hansenula polymorpha,* or an *Aspergillus niger.*

15. A method for making a carboxylic acid comprising hydrolyzing a nitrile using the isolated, synthetic or recombinant polypeptide of claim 5 or claim 6, under aqueous conditions.

16. The polypeptide as set forth in Claim 5, but lacking a signal sequence.

17. The polypeptide as set forth in Claim 5, further comprising a heterologous sequence.

## Patentansprüche

1. Isolierte, synthetische oder rekombinante Nukleinsäure, die Folgendes beinhaltet:
(a) eine Nukleinsäuresequenz, die wenigstens 70 %, 71 %, 72 %, 73 %, 74 %, 75 %, 76 %, 77 %, 78 %, 79 %, 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 % oder mehr oder 100 % Sequenzidentität mit SEQ ID Nr. 297 hat und ein Polypeptid mit Nitrilaseaktivität codiert, wobei die Nukleinsäure wenigstens ein Polypeptid mit Nitrilaseaktivität codiert; oder
(b) eine Nukleinsäuresequenz vollständig komplementär zu (a).

2. Expressionskassette, Vektor oder Klonierungsvehikel, die/der/das eine Nukleinsäure mit einer Sequenz gemäß Anspruch 1 beinhaltet.

3. Transformierte Zelle, die Folgendes beinhaltet:
(i) eine Nukleinsäure, die eine Sequenz nach Anspruch 1 oder eine Expressionskassette, einen Vektor oder ein Klonierungsvehikel nach Anspruch 2 beinhaltet;
(ii) die transformierte Zelle von (i), wobei die Zelle:
(a) ein gramnegatives Bakterium, ein grampositives Bakterium oder ein eukaryotischer einzelliger Organismus ist;
(b) *Escherichia coli* ist;
(c) *Streptomyces diversa, Lactobacillus gasseri, Lactococcus lactis, Lactococcus cremoris* oder *Bacillus subtilis* ist; oder
*(d) Saccharomyces cerevisiae, Schizosaccharomyces pombe, Pichia pastoris, Kluyveromyces lactis, Hansenula polymorpha* oder *Aspergillus niger* ist.

4. Transgener nicht-humaner Organismus, der eine Sequenz nach Anspruch 1 oder eine Expressionskassette, einen Vektor oder ein Klonierungsvehikel nach Anspruch 2 beinhaltet.

5. Isoliertes, synthetisches oder rekombinantes Polypeptid, das Folgendes beinhaltet:
(a) eine Sequenz mit wenigstens 70 %, 71 %, 72 %, 73 %, 74 %, 75 %, 76 %, 77 %, 78 %, 79 %, 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 % oder mehr oder 100 % Sequenzidentität mit SEQ ID Nr. 298, wobei das Polypeptid Nitrilaseaktivität hat, oder
(ii) eine Aminosäure, die mit der Nukleinsäure von Anspruch 1(a) codiert ist.

6. Isoliertes, synthetisches oder rekombinantes Polypeptid nach Anspruch 5, wobei die Nitrilaseaktivität Folgendes beinhaltet:
(a) Hydrolysieren eines Nitrils in eine Carbonsäure;
(b) Hydrolysieren eines Cyanhydrinanteils oder eines Aminonitrilanteils eines Moleküls, wobei das Molekül optional (*S*)-2-Amino-40-phenylbutansäure beinhaltet oder das Molekül eine L-Aminosäure beinhaltet oder das Molekül einen Lebensmittelzusatz oder ein Arzneimittel beinhaltet; oder
(c) eine enantioselektive Nitrilaseaktivität, wobei die enantioselektive Aktivität das Erzeugen eines chiralen Alpha-Hydroxysäuremoleküls oder eines chiralen Aminosäuremoleküls durch Katalysieren der Hydrolyse eines Moleküls mit einem Cyanohydrinanteil oder einem Aminonitrilanteil beinhaltet,
wobei das erzeugte chirale Molekül optional ein (*R*)-Enantiomer oder ein (*S*)-Enantiomer ist.

7. Isoliertes, synthetisches oder rekombinantes Polypeptid nach Anspruch 5 oder Anspruch 6, wobei die Nitrilaseaktivität das Hydrolysieren eines Hydroxyglutarylnitrils zum Erzeugen einer (*R*)-Ethyl 4-cyano-3-hydroxybuttersäure beinhaltet,
wobei das Polypeptid optional das Hydroxyglutarylnitril enantioselektiv hydrolysiert, um eine (*R*)-Enantiomer (*R*)-ethyl 4-cyano-3-hydroxybuttersäure zu erzeugen,
wobei das Polypeptid optional ein Produkt mit einer enantiomeren Reinheit mit einem ee von wenigstens 95 %, 96 %, 97 %, 98 % oder 99 % erzeugt, und
wobei das Hydroxyglutarylnitril optional 1,3-Di-cyano-2-hydroxy-propan oder ein Hydroxyglutaronitril beinhaltet, oder
wobei das Hydroxyglutaronitril optional ein 3-Hydroxyglutaronitril beinhaltet.

8. Isoliertes, synthetisches oder rekombinantes Polypeptid nach Anspruch 5 oder Anspruch 6, wobei die Nitrilaseaktivität das Hydrolysieren eines Hydroxyglutarylnitrils zum Erzeugen einer Ethyl 4-cyano-3-hydroxybuttersäure beinhaltet,
wobei das Polypeptid optional das Hydroxyglutarylnitril enantioselektiv hydrolysiert, um eine (*S*)-Enantiomer (*S*)-ethyl 4-cyano-3-hydroxybuttersäure zu erzeugen, und
wobei das Polypeptid optional ein Produkt mit einer enantiomeren Reinheit mit einem ee von 95 %, 96 %, 97 %, 98 % oder 99 % erzeugt.

9. Isoliertes, synthetisches oder rekombinantes Polypeptid nach Anspruch 5 oder Anspruch 6, wobei die Nitrilaseaktivität das Hydrolysieren eines Mandelonitrils zum Erzeugen einer Mandelsäure oder eines Mandelsäurederivats beinhaltet,
wobei die Mandelsäure optional (*R*)-2-Chlormandelsäure beinhaltet,
wobei die Mandelsäure optional eine aromatische Ringsubstitution in der *ortho-, meta-* oder *para*-Position; ein 1-Paphthylderivat von (*R*)-Mandelsäure, ein Pyridylderivat von (*R*)-Mandelsäure oder ein Thienylderivat von (*R*)-Mandelsäure oder eine Kombination davon beinhaltet,
wobei die Mandelsäure optional (*S*)-Methylbenzylcyanid beinhaltet und das Mandelonitril (*S*)-Methoxy-benzylcyanid beinhaltet, oder
wobei die Mandelsäure optional eine aromatische Ringsubstitution in der *ortho-, meta-* oder *para*-Position; ein 1-Paphthylderivat von (*S*)-Mandelsäure, ein Pyridylderivat von (*S*)-Mandelsäure oder ein Thienylderivat von (*S*)-Mandelsäure oder eine Kombination davon beinhaltet.

10. Isoliertes, synthetisches oder rekombinantes Polypeptid nach Anspruch 5 oder Anspruch 6, wobei die Nitrilaseaktivität das Hydrolysieren eines Phenyllactocyanonitrils zum Erzeugen eines (*S*)-Phenylmilchsäurederivats oder eines (*R*)-Phenylmilchsäurederivats beinhaltet.

11. Enzympräparat, das ein Polypeptid nach einem der Ansprüche 5 bis 10 beinhaltet, wobei das Präparat ein Flüssig- oder ein Trockenpräparat ist.

12. Industrieverfahren, das die Verwendung des isolierten, synthetischen oder rekombinanten Polypeptids nach einem der Ansprüche 5 bis 10 bei der Hydrolyse eines Substrats mit einem Cyanohydrinanteil oder einem Aminonitrilanteil zum Erzeugen einer Zusammensetzung beinhaltet, die eine Alpha-Hydroxysäure oder eine Aminosäure beinhaltet.

13. Industrieverfahren nach Anspruch 12, wobei das Industrieverfahren ferner Folgendes beinhaltet:
(i) Herstellen einer pharmazeutischen Zusammensetzung, die die erzeugte Alpha-Hydroxysäure oder Aminosäure beinhaltet,
(ii) Herstellen einer Chemikalie, die die erzeugte Alpha-Hydroxysäure oder Aminosäure beinhaltet,
(iii) Katalysieren des Abbaus eines Abfallprodukts, wobei der Abbau das Hydrolysieren eines Substrats mit einem Cyanohydrinanteil oder einem Aminonitrilanteil beinhaltet,
(iv) Herstellen eines Arzneimittelintermediats, wobei das Arzneimittelintermediat die erzeugte Alpha-Hydroxysäure oder Aminosäure beinhaltet und wobei das Polypeptid optional zum Hydrolysieren eines Hydroxyglutarylnitrilsubstrats verwendet wird,
(v) Herstellen eines Reinigungsmittels, oder
(vi) Herstellen eines Lebensmittelprodukts.

14. Isoliertes, synthetisches oder rekombinantes Polypeptid nach Anspruch 5, codiert durch eine Nukleinsäure, die in einer Wirtszelle exprimiert ist, wobei die Wirtszelle Folgendes beinhaltet:
(a) ein gramnegatives Bakterium, ein grampositives Bakterium oder einen einzelligen eukaryotischen Organismus;
(b) *Escherichia coli;*
(c) *Streptomyces diversa, Lactobacillus gasseri, Lactococcus lactis, Lactococcus cremoris* oder *Bacillus subtilis;* oder
(d) *Saccharomyces cerevisiae, Schizosaccharomyces pombe, Pichia pastoris, Kluyveromyces lactis, Hansenula polymorpha* oder *Aspergillus niger.*

15. Verfahren zur Herstellung einer Carbonsäure, das das Hydrolysieren eines Nitrils unter Verwendung des isolierten, synthetischen oder rekombinanten Polypeptids nach Anspruch 5 oder Anspruch 6 unter wässrigen Bedingungen beinhaltet.

16. Polypeptid nach Anspruch 5, dem jedoch eine Signalsequenz fehlt.

17. Polypeptid nach Anspruch 5, die ferner eine heterologe Sequenz umfasst.

## Revendications

1. Acide nucléique isolé, synthétique ou recombinant comprenant
(a) une séquence d'acide nucléique d'au moins 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, ou plus, ou de 100% par rapport à SEQ ID n° 297, l'acide nucléique codant au moins un polypeptide ayant une activité nitrilase; ou
(b) une séquence d'acide nucléique complètement complémentaire de la séquence (a).

2. Cassette d'expression, vecteur ou véhicule de clonage comprenant un acide nucléique comprenant une séquence selon la revendication 1.

3. Cellule transformée comprenant
(i) un acide nucléique comprenant une séquence selon la revendication 1 ou une cassette d'expression, un vecteur ou un véhicule de clonage selon la revendication 2 ;
(ii) la cellule transformée de (i), la cellule étant
(a) une bactérie à Gram négatif, une bactérie à Gram positif, ou un organisme eucaryote ;
(b) un *Escherichia coli ;*
(c) un *Streptomyces diversa, Lactobacillus gasseri, Lactococcus lactis, Lactococcus cremoris,* ou un *Bacillus subtilis ;* ou
(d) un *Saccharomyces cerevisiae, Schizosaccharomyces pombe, Pichia pastoris, Kluyveromyces lactis, Hansenula Polymorpha* ou un *Aspergillus niger.*

4. Organisme transgénique comprenant une séquence selon la revendication 1 ou une cassette d'expression, un vecteur ou un véhicule de clonage selon la revendication 2.

5. Polypeptide isolé, synthétique ou recombinant comprenant :
(a) une séquence comprenant d'au moins 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, ou plus, ou de 100% par rapport à SEQ ID n° 298 ; le polypeptide ayant une activité nitrilase, ou
(b) un acide aminé codé par l'acide nucléique selon la revendication 1 (a).

6. Polypeptide isolé, synthétique ou recombinant selon la revendication 5, dans lequel l'activité nitrilase comprend :
(a) l'hydrolyse d'un nitrile en un acide carboxylique ;
(b) l'hydrolyse d'un groupe caractéristique cyanhydrine ou d'un groupe caractéristique aminonitrile d'une molécule, dans lequel optionnellement la molécule comprend un acide (*S*)-2-amino-40 phényl bunatoique, ou la molécule comprend un acide aminé L, ou la molécule comprend un additif alimentaire ou un médicament pharmaceutique ; ou
(c) une activité nitrilase énantiosélective, l'activité nitrilase énantiosélective comprenant la génération d'une molécule chirale d'acide alpha hydroxylé ou d'une molécule chirale d'acide aminé en catalysant l'hydrolyse d'une molécule ayant un groupe caractéristique cyanhydrine ou un groupe caractéristique aminonitrile,
dans lequel optionnellement la molécule chirale générée est un énantiomère (*R*) ou un énantiomère (*S)*.

7. Polypeptide isolé, synthétique ou recombinant selon la revendication 5 ou la revendication 6, dans lequel l'activité nitrilase comprend l'hydrolyse d'un nitrile hydroxyglutaryle pour générer un acide (*R*)-éthyl 4-cyano-3-hydroxybutyrique,
dans lequel optionnellement le polypeptide hydrolyse de manière énantiosélective le nitrile hydroxyglutaryle pour générer un énantiomère (*R*) d'acide (*R*)-éthyl 4-cyano-3-hydroxybutyrique,
dans lequel optionnellement le polypeptide produit un produit ayant une pureté énantiomérique comprenant un ee d'au moins 95%, 96%, 97%, 98% ou 99%, et
dans lequel optionnellement le nitrile hydroxyglutaryle comprend 1,3-di-cyano-2-hydroxy-propane ou un hydroxyglutaronitile, ou
dans lequel optionnellement l'hydroxyglutaronitrile comprend un 3-hydroxyglutaronitrile.

8. Polypeptide isolé, synthétique ou recombinant selon la revendication 5 ou la revendication 6, dans lequel l'activité nitrilase comprend l'hydrolyse d'un nitrile hydroxyglutaryle pour générer un acide éthyl 4-cyano-3-hydroxybutyrique,
dans lequel optionnellement le polypeptide hydrolyse de manière énantiosélective le nitrile hydroxyglutaryle pour générer un énantiomère (*S)* d'acide (*S)*-éthyl 4-cyano-3-hydroxybutyrique, et
dans lequel optionnellement le polypeptide produit un produit ayant une pureté énantiomérique comprenant un ee d'au moins 95%, 96%, 97%, 98% ou 99%.

9. Polypeptide isolé, synthétique ou recombinant selon la revendication 5 ou la revendication 6, dans lequel l'activité nitrilase comprend l'hydrolyse d'un mandélonitrile pour générer un acide mandélique ou un dérivé d'acide mandélique,
dans lequel optionnellement l'acide mandélique comprend un acide (*R*)-2-chloromandélique,
dans lequel optionnellement l'acide mandélique comprend une substitution de cycle aromatique aux positions *ortho-, meta-* ou *para- ;* un dérivé de 1-paphthyl d'acide (*R*)-mandélique, un dérivé de pyridyle d'acide (*R)*-mandélique ou un dérivé de thiényle d'acide (*R)*-mandélique ou une combinaison de ceux-ci,
dans lequel optionnellement l'acide mandélique comprend un cyanure (*S*) méthylbenzylique et le mandélonitrile comprend un cyanure (*S*) méthoxybenzylique, ou
dans lequel optionnellement l'acide mandélique comprend une substitution de cycle aromatique aux positions *ortho-, meta-* ou *para- ;* un dérivé de 1-paphthyl d'acide (*S*)-mandélique, un dérivé de pyridyle d'acide (*S*)-mandélique ou un dérivé de thyényl d'acide (*S*)-mandélique ou une combinaison de ceux-ci.

10. Polypeptide isolé, synthétique ou recombinant selon la revendication 5 ou la revendication 6, dans lequel l'activité nitrilase comprend l'hydrolyse d'un phényllactocyanonitrile pour générer un dérivé d'acide (*S*)-phényllactique ou un dérivé d'acide (*R*)-phényllactique.

11. Préparation enzymatique comprenant un polypeptide selon l'une quelconque des revendications 5 à 10, dans lequel la préparation est une préparation liquide ou sèche.

12. Processus industriel comprenant l'utilisation du polypeptide isolé, synthétique ou recombinant selon l'une quelconque des revendications 5 à 10, dans l'hydrolyse d'un substrat ayant un groupe caractéristique cyanhydrine ou un groupe caractéristique aminonitrile pour générer une composition comprenant un acide alpha-hydroxy ou un acide aminé.

13. Processus industriel selon la revendication 12, le processus industriel comprenant en outre :
(i) la production d'une composition pharmaceutique comprenant l'acide alpha-hydroxy ou l'acide aminé généré,
(ii) la production d'un produit chimique comprenant l'acide alpha-hydroxy ou l'acide aminé généré,
(iii) la catalyse de la décomposition d'un déchet, la décomposition comprenant l'hydrolyse d'un substrat ayant un groupe caractéristique cyanhydrine ou un groupe caractéristique aminonitrile,
(iv) la production d'un médicament intermédiaire, le médicament intermédiaire comprenant l'acide alpha-hydroxy ou l'acide aminé généré, et dans lequel optionnellement le polypeptide est utilisé pour hydrolyser un substrat hydroxygultarylnitrile,
(v) la production d'un détergent, ou
(vi) la production d'un produit alimentaire.

14. Polypeptide isolé, synthétique ou recombinant selon la revendication 5, codé par un acide nucléique exprimé dans une cellule hôte, la cellule hôte comprenant :
(a) une bactérie à Gram négatif, une bactérie à Gram positif, ou un organisme eucaryote ;
(b) un *Escherichia coli ;*
(c) un *Streptomyces diversa, Lactobacillus gasseri, Lactococcus lactis, Lactococcus cremoris,* ou un *Bacillus subtilis ;* ou
(d) un *Saccharomyces cerevisiae, Schizosaccharomyces pombe, Pichia pastoris, Kluyveromyces lactis, Hansenula Polymorpha* ou un *Aspergillus niger.*

15. Procédé de fabrication d'un acide carboxylique comprenant l'hydrolyse d'un nitrile en utilisant le polypeptide isolé, synthétique ou recombinant selon la revendication 5 ou la revendication 6, dans des conditions aqueuses.

16. Polypeptide selon la revendication 5, mais ne comportant pas de séquence signal.

17. Polypeptide selon la revendication 5, comprenant en outre une séquence hétérologue.


### SEQUENCE LISTING

<110> VERENIUM CORPORATION
<120> NITRILASES
<130> P/46800.ep06
<140> divisional of EP 08075753.7
   <141> 2002-05-15
<150> PCT/US2002/015983
   <151> 2002-05-15
<150> US 60/309,006
   <151> 2001-07-30
<150> US 60/351,336
   <151> 2002-01-22
<150> US 60/300,189
   <151> 2001-06-21
<150> US 09/751,299
   <151> 2000-12-28
<150> US 60/254,414
   <151> 2000-12-07
<150> US 60/173,609
   <151> 1999-12-29
<160> 392
<170> FastSEQ for Windows Version 4.0
<210> 1
   <211> 939
   <212> DNA
   <213> Unknown
<220>
   <223> obtained from an environmental sample
<400> 1
   atggaaaagt atattaaagt cgccgcaatt cagatagcta caaaaatagc agattcaccc      60
   gtgaatatag aaaattgcga acgtttggca ttatcggcgg tcaatgaggg tgcgcgttgg     120
   attgctttgc cggagttctt caatacgggc gttagttgga acaaaaaaat tgccttggct     180
   attcagacgc ctgacggcaa ggctgcgatg ttcttacgcg acttttctgc aagacatcat     240
   gtattgatag gaggctcatt tctgtgcagg ttgccggatg gcagtgtgcg caaccgctat     300
   atgtgttatg ccaacggcgc tctcgtgggc aaacatgaca aagacctacc cacgatgtgg     360
   gaaaatgctt tttatgaagg tggggattcc agcgatattg gggtgctggg aacatttgaa      420
   aatacgcgcg ttggtgcagc cgtctgttgg gagttcatgc ggacgatgac tgcccggcgt     480
   cttcgcaatc aggtggatgt catcatgggt ggttcctgct ggtggagcat accgaccaat     540
   ttccccggtt ttgtgcaaaa gctgtgggaa cctggaaata gccgcaacgc gcttgctgcc     600
   atacaggata atgcgcgtct cattggcgtg ccggttgttc atgccgctca ttgcggtgaa     660
   attgagtgtc cgatgccagg attgccgata ggttacaggg ggttctttga gggtaacgcg     720
   gccattgtga atgcagaagg tcaggtgctt gcgcatcggg gtgctggcga gggcgaagga     780
   attgtttgcg cggagatttt accggtagcc aaatcaaaca ggtcggaaat tcccaatcgt     840
   tactggttgc gctgcagagg ctttctacct atttttgcct ggcatcagca acgttggttg     900
   ggaaggcatt ggtatttgcg caatgtgcgc aggacttaa                            939
<210> 2
   <211> 312
   <212> PRT
   <213> Unknown
<220>
   <223> Obtained from an environmental sample
<400> 2
   Met Glu Lys Tyr Ile Lys Val Ala Ala Ile Gln Ile Ala Thr Lys Ile
    1               5                   10                  15
   Ala Asp Ser Pro Val Asn Ile Glu Asn Cys Glu Arg Leu Ala Leu Ser
               20                  25                  30
   Ala Val Asn Glu Gly Ala Arg Trp Ile Ala Leu Pro Glu Phe Phe Asn
           35                  40                  45
   Thr Gly Val Ser Trp Asn Lys Lys Ile Ala Leu Ala Ile Gln Thr Pro
       50                  55                  60
   Asp Gly Lys Ala Ala Met Phe Leu Arg Asp Phe Ser Ala Arg His His
   65                  70                  75                  80
   Val Leu Ile Gly Gly Ser Phe Leu Cys Arg Leu Pro Asp Gly Ser Val
                   85                  90                  95
   Arg Asn Arg Tyr Met Cys Tyr Ala Asn Gly Ala Leu Val Gly Lys His
               100                 105                 110
   Asp Lys Asp Leu Pro Thr Met Trp Glu Asn Ala Phe Tyr Glu Gly Gly
           115                 120                 125
   Asp Ser Ser Asp Ile Gly Val Leu Gly Thr Phe Glu Asn Thr Arg Val
       130                 135                 140
   Gly Ala Ala Val Cys Trp Glu Phe Met Arg Thr Met Thr Ala Arg Arg
   145                 150                 155                 160
   Leu Arg Asn Gln Val Asp Val Ile Met Gly Gly Ser Cys Trp Trp Ser
                   165                 170                 175
   Ile Pro Thr Asn Phe Pro Gly Phe Val Gln Lys Leu Trp Glu Pro Gly
               180                 185                 190
   Asn Ser Arg Asn Ala Leu Ala Ala Ile Gln Asp Asn Ala Arg Leu Ile
           195                 200                 205
   Gly Val Pro Val Val His Ala Ala His Cys Gly Glu Ile Glu Cys Pro
       210                 215                 220
   Met Pro Gly Leu Pro Ile Gly Tyr Arg Gly Phe Phe Glu Gly Asn Ala
   225                 230                 235                 240
   Ala Ile Val Asn Ala Glu Gly Gln Val Leu Ala His Arg Gly Ala Gly
                   245                 250                 255
   Glu Gly Glu Gly Ile Val Cys Ala Glu Ile Leu Pro Val Ala Lys Ser
               260                 265                 270
   Asn Arg Ser Glu Ile Pro Asn Arg Tyr Trp Leu Arg Cys Arg Gly Phe
           275                 280                 285
   Leu Pro Ile Phe Ala Trp His Gln Gln Arg Trp Leu Gly Arg His Trp
       290                 295                 300
   Tyr Leu Arg Asn Val Arg Arg Thr
   305                 310
<210> 3
   <211> 981
   <212> DNA
   <213> Unknown
<220>
   <223> Obtained from an environmental sample
<400> 3
   atggggaatt cgttcaagat cgcggtggta caagcctgtc cggtctttct ggatcgtggc     60
   gcgacagtcg ccaaggcatg ccgcctgatc gcggaggcag ccgcggcggg cgccagcctg    120
   gtggtctttc cggaggcgtt tgtgcccgga tacccactgt gggtctggtt cattccggca    180
   gggcattcgc aaccactgcg ggagttatac gccgaactgg tggggaacgc cgtggcggta    240
   ccgggcgatg ccaccgatcg gctttgcgcg gcagccagag aagccggcgt ggtagtggcg    300
   atcggcatca atgaagtgaa cagcgaagcc agcggcacga cgatttacaa tacgctgctg    360
   tacatcggag cggacggcgc gattctgggc aaacaccgca aagtaatgcc gacgggcgga    420
   gagcgcctgg tctgggcgct tggcgatggg agcgacctgg aggtctacga cctgcctttc    480
   ggccgattgg gtggcctgtt gtgctgggag aactacatgc ccctggcccg gtacgcgatg    540
   tcggcatggg gaaccgagat ctacgtggct ccaacttggg atcgcggaga accgtggctg    600
   tccacaatgc ggcatatcgc gaaagaaggg cgatgctacg tagtgggatg ctgcagttgc    660
   atgaaaattg acgatgtacc cgaccggctg gcgttcaaag ggaagtatct gtcgacggcc    720
   gagggctggc tcaaccccgg cgatagcgta atcgtcgatc cggacggcaa gctgatcgcg    780
   ggcccggcaa gcgagcagga gacgattctg tatgccgatg ccgaccggtc taagatcacc    840
   gggcccaggt ggcagttgga tgtggccggc cactacgcgc ggccggatat cttcgaactg    900
   atcgtgcacc gcgaacctaa gcgatttttg acgatagctc cgcggacgaa ggaggagcgg    960
   gagcctgggc cggaggcctg a                                              981
<210> 4
   <211> 326
   <212> PRT
   <213> Unknown
<220>
   <223> Obtained from an environmental sample
<400> 4
   Met Gly Asn Ser Phe Lys Ile Ala Val Val Gln Ala Cys Pro Val Phe
   1               5                   10                  15
   Leu Asp Arg Gly Ala Thr Val Ala Lys Ala Cys Arg Leu Ile Ala Glu
               20                  25                  30
   Ala Ala Ala Ala Gly Ala Ser Leu Val Val Phe Pro Glu Ala Phe Val
           35                  40                  45
   Pro Gly Tyr Pro Leu Trp Val Trp Phe Ile Pro Ala Gly His Ser Gln
       50                  55                  60
   Pro Leu Arg Glu Leu Tyr Ala Glu Leu Val Gly Asn Ala Val Ala Val
   65                  70                  75                  80
   Pro Gly Asp Ala Thr Asp Arg Leu Cys Ala Ala Ala Arg Glu Ala Gly
                   85                  90                  95
   Val Val Val Ala Ile Gly Ile Asn Glu Val Asn Ser Glu Ala Ser Gly
               100                 105                 110
   Thr Thr Ile Tyr Asn Thr Leu Leu Tyr Ile Gly Ala Asp Gly Ala Ile
           115                 120                 125
   Leu Gly Lys His Arg Lys Val Met Pro Thr Gly Gly Glu Arg Leu Val
       130                 135                 140
   Trp Ala Leu Gly Asp Gly Ser Asp Leu Glu Val Tyr Asp Leu Pro Phe
   145                 150                 155                 160
   Gly Arg Leu Gly Gly Leu Leu Cys Trp Glu Asn Tyr Met Pro Leu Ala
                   165                 170                 175
   Arg Tyr Ala Met Ser Ala Trp Gly Thr Glu Ile Tyr Val Ala Pro Thr
               180                 185                 190
   Trp Asp Arg Gly Glu Pro Trp Leu Ser Thr Met Arg His Ile Ala Lys
           195                 200                 205
   Glu Gly Arg Cys Tyr Val Val Gly Cys Cys Ser Cys Met Lys Ile Asp
       210                 215                 220
   Asp Val Pro Asp Arg Leu Ala Phe Lys Gly Lys Tyr Leu Ser Thr Ala
   225                 230                 235                 240
   Glu Gly Trp Leu Asn Pro Gly Asp Ser Val Ile Val Asp Pro Asp Gly
                   245                 250                 255
   Lys Leu Ile Ala Gly Pro Ala Ser Glu Gln Glu Thr Ile Leu Tyr Ala
               260                 265                 270
   Asp Ala Asp Arg Ser Lys Ile Thr Gly Pro Arg Trp Gln Leu Asp Val
           275                 280                 285
   Ala Gly His Tyr Ala Arg Pro Asp Ile Phe Glu Leu Ile Val His Arg
       290                 295                 300
   Glu Pro Lys Arg Phe Leu Thr Ile Ala Pro Arg Thr Lys Glu Glu Arg
   305                 310                 315                 320
   Glu Pro Gly Pro Glu Ala
                   325
<210> 5
   <211> 1005
   <212> DNA
   <213> Unknown
<220>
   <223> Obtained from an environmental sample
<400> 5
   atgggtacca agcacccggc cttcaaggcc gcagtggtcc aggccgcgcc ggaatggctc        60
   gatctcgacc gcaccgtcga caagaccatc gcgctgatcg aggaggccgc cggcgccggc     120
   gcgaagctca ttgcgttccc ggaaacctgg attcccggct atccgtggca catctgggtc     180
   ggcacgccgg cgtgggcgat cagccgcggc ttcgtgcagc gctacttcga caattcactg     240
   gcctacgaca gcccgcaggc ccagcgcatc gcggacgccg cgaagaagaa caagatcacc     300
   gtggtgctcg gcctgtcgga gcgcgagggt ggcagccttt atatctcgca gtggctgatt     360
   gggccggacg gcgagaccat tgccaagcgc cgcaaactgc gccccaccca cgtcgagcgc     420
   accgtgttcg gcgatggcga cggcagccac atcgcggtgc acgagcgtgc tgacatcggc     480
   cgcctcggcg cgctgtgctg ctgggagcac atccagccgc tgaccaaata cgccatgtat     540
   gcccagaacg agcaggtgca cgtcgccgcc tggccgagct tctcgatgta cgagccgttc     600
   gcccacgcgc tcggctggga agtcaacaat gcggcgagca agatctacgc cgtcgaaggc     660
   tcgtgtttcg tgctcggcgc atgcgcggtg atctcgcagg cgatggtcga cgaaatgtgc     720
   gacaccgagg acaagcgggc gctggtccat gccggcggcg gccacgcggt gatcttcggg     780
   ccggacggca gatcgctggc ggacaagatt ccggagaccc aggaaggcct gctctatgcc     840
   gacatcgacc tcggcgcaat tggcgtggcc aagaacgcgg ccgatccggc ggggcactac     900
   tcgcgcccgg acgtgacgcg gctcctgttc aacaacaagc cggcgcgccg ggtcgagtat     960
   ttctcgctgc cggtcgacgc ggtcgagacg ccgccgcagc cctga                    1005
<210> 6
   <211> 334
   <212> PRT
   <213> Unknown
<220>
   <223> Obtained from an environmental sample
<400> 6
   Met Gly Thr Lys His Pro Ala Phe Lys Ala Ala Val Val Gln Ala Ala
   1               5                   10                  15
   Pro Glu Trp Leu Asp Leu Asp Arg Thr Val Asp Lys Thr Ile Ala Leu
               20                  25                  30
   Ile Glu Glu Ala Ala Gly Ala Gly Ala Lys Leu Ile Ala Phe Pro Glu
           35                  40                  45
   Thr Trp Ile Pro Gly Tyr Pro Trp His Ile Trp Val Gly Thr Pro Ala
       50                  55                  60
   Trp Ala Ile Ser Arg Gly Phe Val Gln Arg Tyr Phe Asp Asn Ser Leu
   65                  70                  75                  80
   Ala Tyr Asp Ser Pro Gln Ala Gln Arg Ile Ala Asp Ala Ala Lys Lys
                   85                  90                  95
   Asn Lys Ile Thr Val Val Leu Gly Leu Ser Glu Arg Glu Gly Gly Ser
               100                 105                 110
   Leu Tyr Ile Ser Gln Trp Leu Ile Gly Pro Asp Gly Glu Thr Ile Ala
           115                 120                 125
   Lys Arg Arg Lys Leu Arg Pro Thr His Val Glu Arg Thr Val Phe Gly
       130                 135                 140
   Asp Gly Asp Gly Ser His Ile Ala Val His Glu Arg Ala Asp Ile Gly
   145                 150                 155                 160
   Arg Leu Gly Ala Leu Cys Cys Trp Glu His Ile Gln Pro Leu Thr Lys
                   165                 170                 175
   Tyr Ala Met Tyr Ala Gln Asn Glu Gln Val His Val Ala Ala Trp Pro
               180                 185                 190
   Ser Phe Ser Met Tyr Glu Pro Phe Ala His Ala Leu Gly Trp Glu Val
           195                 200                 205
   Asn Asn Ala Ala Ser Lys Ile Tyr Ala Val Glu Gly Ser Cys Phe Val
       210                 215                 220
   Leu Gly Ala Cys Ala Val Ile Ser Gln Ala Met Val Asp Glu Met Cys
   225                 230                 235                 240
   Asp Thr Glu Asp Lys Arg Ala Leu Val His Ala Gly Gly Gly His Ala
                   245                 250                 255
   Val Ile Phe Gly Pro Asp Gly Arg Ser Leu Ala Asp Lys Ile Pro Glu
               260                 265                 270
   Thr Gln Glu Gly Leu Leu Tyr Ala Asp Ile Asp Leu Gly Ala Ile Gly
           275                 280                 285
   Val Ala Lys Asn Ala Ala Asp Pro Ala Gly His Tyr Ser Arg Pro Asp
       290                 295                 300
   Val Thr Arg Leu Leu Phe Asn Asn Lys Pro Ala Arg Arg Val Glu Tyr
   305                 310                 315                 320
   Phe Ser Leu Pro Val Asp Ala Val Glu Thr Pro Pro Gln Pro
                   325                 330
<210> 7
   <211> 999
   <212> DNA
   <213> Unknown
<220>
   <223> Obtained from an environmental sample
<400> 7
   atgccgagtg attatcatgc tccattcaaa gtagcagttg tccaggcaac tcccgtcttt      60
   ctcgatcgca gcgcgacgat tgagaaggca tgtgagctaa ttgcctgtgc tggacgtgag     120
   ggcgcacgtc tgatcgtgtt tcctgaagcg ttcattccca cctatcccga ttgggtctgg     180
   accattccac ctggggagat gcggctgctt ggcgaactct acacagagtt gcttgccaat     240
   gcggtcacga tccccagtaa tgcaacggat aggctctgcc aggctgcgaa acgagctgct     300
   gcgtatgtgg tcatgggaat gaacgaacgc aatatcgagg cgagtggaag gagtctctat     360
   aacaccctgt tatacatcga tgctcagggc cagatcatgg gcaaacaccg caagttgata     420
   cccacagccg gtgagcggct catatgggcg caaggagatg ggagtacatt ccaggtctac     480
   gatactcctc tgggcaaact gggagggctc atctgctggg aaaactacat gcctctggct     540
   cgctatgcga tgtatgcctg gggcacgcag atttatgtcg ccccgacatg ggatcgtggc     600
   aacctctggc tctctactct gcggcatatc gctaaggagg gaggcgtcta tgttcttggt     660
   tgtagtatgg tcatgcgcaa gaatgacatt cccgatcact ttgctttcaa agagcagttt     720
   tatgctactg tggacgaatg gatcaacgtt ggtgacagcg ccattgtcca tcccgagggg     780
   aactttcttg cgggaccggt gcgccacaaa gaagagattc tctatgcaga acttgatcca     840
   cgccaatcgt gcggtccggg atggatgctc gatgtggctg ggcactatgc acgccctgat     900
   gtgtttgaat tgattgtcca cacagagatg cgacccatga tgaagcaaga agaggtagga     960
   ggagaaaata catctgaggg aggtgtacga ttcttgtaa                           999
<210> 8
   <211> 332
   <212> PRT
   <213> Unknown
<220>
   <223> Obtained from an environmental sample
<400> 8
   Met Pro Ser Asp Tyr His Ala Pro Phe Lys Val Ala Val Val Gln Ala
    1               5                  10                  15
   Thr Pro Val Phe Leu Asp Arg Ser Ala Thr Ile Glu Lys Ala Cys Glu
               20                  25                  30
   Leu Ile Ala Cys Ala Gly Arg Glu Gly Ala Arg Leu Ile Val Phe Pro
           35                  40                  45
   Glu Ala Phe Ile Pro Thr Tyr Pro Asp Trp Val Trp Thr Ile Pro Pro
       50                  55                  60
   Gly Glu Met Arg Leu Leu Gly Glu Leu Tyr Thr Glu Leu Leu Ala Asn
   65                  70                  75                  80
   Ala Val Thr Ile Pro Ser Asn Ala Thr Asp Arg Leu Cys Gln Ala Ala
                   85                  90                  95
   Lys Arg Ala Ala Ala Tyr Val Val Met Gly Met Asn Glu Arg Asn Ile
               100                 105                 110
   Glu Ala Ser Gly Arg Ser Leu Tyr Asn Thr Leu Leu Tyr Ile Asp Ala
           115                 120                 125
   Gln Gly Gln Ile Met Gly Lys His Arg Lys Leu Ile Pro Thr Ala Gly
       130                 135                 140
   Glu Arg Leu Ile Trp Ala Gln Gly Asp Gly Ser Thr Phe Gln Val Tyr
   145                 150                 155                 160
   Asp Thr Pro Leu Gly Lys Leu Gly Gly Leu Ile Cys Trp Glu Asn Tyr
                   165                 170                 175
   Met Pro Leu Ala Arg Tyr Ala Met Tyr Ala Trp Gly Thr Gln Ile Tyr
               180                 185                 190
   Val Ala Pro Thr Trp Asp Arg Gly Asn Leu Trp Leu Ser Thr Leu Arg
           195                 200                 205
   His Ile Ala Lys Glu Gly Gly Val Tyr Val Leu Gly Cys Ser Met Val
       210                 215                 220
   Met Arg Lys Asn Asp Ile Pro Asp His Phe Ala Phe Lys Glu Gln Phe
   225                 230                 235                 240
   Tyr Ala Thr Val Asp Glu Trp Ile Asn Val Gly Asp Ser Ala Ile Val
                   245                 250                 255
   His Pro Glu Gly Asn Phe Leu Ala Gly Pro Val Arg His Lys Glu Glu
               260                 265                 270
   Ile Leu Tyr Ala Glu Leu Asp Pro Arg Gln Ser Cys Gly Pro Gly Trp
           275                 280                 285
   Met Leu Asp Val Ala Gly His Tyr Ala Arg Pro Asp Val Phe Glu Leu
       290                 295                 300
   Ile Val His Thr Glu Met Arg Pro Met Met Lys Gln Glu Glu Val Gly
   305                 310                 315                 320
   Gly Glu Asn Thr Ser Glu Gly Gly Val Arg Phe Leu
                   325                 330
<210> 9
   <211> 945
   <212> DNA
   <213> Unknown
<220>
   <223> Obtained from an environmental sample
<400> 9
   atggcggctc acaagatcgc ggtggttcag gcgcccagcg ttctcctcga tcgcgagggc      60
   tcggtcgcgc gcgcggtcac gctgctcgac gaggcggcgg cggccggcgc ccgcctggtc     120
   gtgtttccgg aggcctacat ccccggctac ccggactgga tctggcgcct gcgcccctac     180
   ccggacgtca agctggccgc cgagctgcac gaacggctgc tcgccaacgc ggtggatctc     240
   tccaccgacg tgctggcgcc ggtgctggcg gcggcggcgc gtcacgggct caccgtggtc     300
   atgtgcgtgc aggagcgcga cgccggattc agccgcgcca cactttacaa caccgcgctg     360
   gtcatcgacg ccgccggcaa gatcgcgaac cggcaccgca agctcatgcc caccaacccc     420
   gagcgaatgg tgtggggatt cggtgacgcc tcggggctgc gggtggtgag cacgcccgtc     480
   gggcgggtgg gcacgctcct gtgctgggag agctacatgc ccctggcgcg ctgcgcgctc     540
   tacgccgagg gggtcgagat ctacgtgacc ccgacctggg actacggcga aggctggcgc     600
   gccagcatgc agcacatcgc ccgcgagggg cgctgctggg tggtgaccgc ttgcatgtgc     660
   gtgcaggcgc gcgacgtgcc ggccgacttc cccgggcgcg cccagctcta ccccgacgag     720
   gaggagtggt tgaaccccgg cgattcgctg gtggtcgacc ccggcggcaa gatcgtggcc     780
   ggtccgatgt cgcgcgagaa ggggatcttg tacgcggaga tcgatccgga tcgcgtggcg     840
   ggggcgcacc gctcgttcga cgtcgtgggc cactactcgc gtcccgacgt gttccggctg     900
   gaggtcgatc ggacaccggc ggcgccggtg agcttcaaaa aatga                     945
<210> 10
   <211> 314
   <212> PRT
   <213> Unknown
<220>
   <223> Obtained from an environmental sample
<400> 10
   Met Ala Ala His Lys Ile Ala Val Val Gln Ala Pro Ser Val Leu Leu
   1               5                   10                  15
   Asp Arg Glu Gly Ser Val Ala Arg Ala Val Thr Leu Leu Asp Glu Ala
               20                  25                  30
   Ala Ala Ala Gly Ala Arg Leu Val Val Phe Pro Glu Ala Tyr Ile Pro
           35                  40                  45
   Gly Tyr Pro Asp Trp Ile Trp Arg Leu Arg Pro Tyr Pro Asp Val Lys
       50                  55                  60
   Leu Ala Ala Glu Leu His Glu Arg Leu Leu Ala Asn Ala Val Asp Leu
   65                  70                  75                  80
   Ser Thr Asp Val Leu Ala Pro Val Leu Ala Ala Ala Ala Arg His Gly
                   85                  90                  95
   Leu Thr Val Val Met Cys Val Gln Glu Arg Asp Ala Gly Phe Ser Arg
               100                 105                 110
   Ala Thr Leu Tyr Asn Thr Ala Leu Val Ile Asp Ala Ala Gly Lys Ile
           115                 120                 125
   Ala Asn Arg His Arg Lys Leu Met Pro Thr Asn Pro Glu Arg Met Val
       130                 135                 140
   Trp Gly Phe Gly Asp Ala Ser Gly Leu Arg Val Val Ser Thr Pro Val
   145                 150                 155                 160
   Gly Arg Val Gly Thr Leu Leu Cys Trp Glu Ser Tyr Met Pro Leu Ala
                   165                 170                 175
   Arg Cys Ala Leu Tyr Ala Glu Gly Val Glu Ile Tyr Val Thr Pro Thr
               180                 185                 190
   Trp Asp Tyr Gly Glu Gly Trp Arg Ala Ser Met Gln His Ile Ala Arg
           195                 200                 205
   Glu Gly Arg Cys Trp Val Val Thr Ala Cys Met Cys Val Gln Ala Arg
       210                 215                 220
   Asp Val Pro Ala Asp Phe Pro Gly Arg Ala Gln Leu Tyr Pro Asp Glu
   225                 230                 235                 240
   Glu Glu Trp Leu Asn Pro Gly Asp Ser Leu Val Val Asp Pro Gly Gly
                   245                 250                 255
   Lys Ile Val Ala Gly Pro Met Ser Arg Glu Lys Gly Ile Leu Tyr Ala
               260                 265                 270
   Glu Ile Asp Pro Asp Arg Val Ala Gly Ala His Arg Ser Phe Asp Val
           275                 280                 285
   Val Gly His Tyr Ser Arg Pro Asp Val Phe Arg Leu Glu Val Asp Arg
       290                 295                 300
   Thr Pro Ala Ala Pro Val Ser Phe Lys Lys
   305                 310
<210> 11
   <211> 966
   <212> DNA
   <213> Unknown
<220>
   <223> Obtained from an environmental sample
<400> 11
   atgactggat cttatcctaa agacacactg atcgttgggc tagctcaaat cgctcctgtc      60
   tggctggatc gggcggggac actgtcaaag atactggctc aagtccatgc ggcaaatcaa     120
   gcgggttgtc atctcgtagc atttggcgaa gggctgcttc ctggatatcc gttttggatt     180
   gagcgaacaa atggcgcgct gttcaactcg actgtacaaa aggaaatcca cgcgcattat     240
   atggatcagg cggtgcagat cgaagccggt catctcgatc cgctttgtgc aacagccaag     300
   aaatttggaa tcaccgttgt actcggatgc atcgaacgcc cactcgatcg gggcggtcac     360
   agcttgtatg caagtctggt atatattgat tccgagggca gcattcaatc cgtgcatcgc     420
   aaactaatgc caacctacga agaacgactt acctggtcgt caggcgatgg gcacggttta     480
   cgagtgcata ccttaggtgc gtttacggtg ggtggtctca actgttggga aaattggatg     540
   cccttggcgc gcgcagcgat gtatggtcag ggtgaagatt tacatgttgc gatctggcca     600
   ggcggttctc atctcacgca ggatattacc cgctttattg cgctcgaatc acgttcgtac     660
   gtattatctg tctccggtct gatgcgcgca accgattttc caaaagatac tccccatctt     720
   gcctccatcc tagctaaagg tgaagagatt cttgcgaatg gtggttcttg tattgcaggt     780
   cctgacggca agtgggtcgt tgggcctctt gtaggagaag agaagttaat tgtcgcaacc     840
   attgatcact gccgcgtgcg cgaagaacgt cagaatttcg atccttccgg gcattacagc     900
   cggcccgatg tactgcaatt aaaaatcaac agggaacgcc agagcacaat ttcatttagc     960
   gagtag                                                                966
<210> 12
   <211> 321
   <212> PRT
   <213> Unknown
<220>
   <223> Obtained from an environmental sample
<400> 12
   Met Thr Gly Ser Tyr Pro Lys Asp Thr Leu Ile Val Gly Leu Ala Gln
   1               5                   10                  15
   Ile Ala Pro Val Trp Leu Asp Arg Ala Gly Thr Leu Ser Lys Ile Leu
               20                  25                  30
   Ala Gln Val His Ala Ala Asn Gln Ala Gly Cys His Leu Val Ala Phe
           35                  40                  45
   Gly Glu Gly Leu Leu Pro Gly Tyr Pro Phe Trp Ile Glu Arg Thr Asn
       50                  55                  60
   Gly Ala Leu Phe Asn Ser Thr Val Gln Lys Glu Ile His Ala His Tyr
   65                  70                  75                  80
   Met Asp Gln Ala Val Gln Ile Glu Ala Gly His Leu Asp Pro Leu Cys
                   85                  90                  95
   Ala Thr Ala Lys Lys Phe Gly Ile Thr Val Val Leu Gly Cys Ile Glu
               100                 105                 110
   Arg Pro Leu Asp Arg Gly Gly His Ser Leu Tyr Ala Ser Leu Val Tyr
           115                 120                 125
   Ile Asp Ser Glu Gly Ser Ile Gln Ser Val His Arg Lys Leu Met Pro
       130                 135                 140
   Thr Tyr Glu Glu Arg Leu Thr Trp Ser Ser Gly Asp Gly His Gly Leu
   145                 150                 155                 160
   Arg Val His Thr Leu Gly Ala Phe Thr Val Gly Gly Leu Asn Cys Trp
                   165                 170                 175
   Glu Asn Trp Met Pro Leu Ala Arg Ala Ala Met Tyr Gly Gln Gly Glu
               180                 185                 190
   Asp Leu His Val Ala Ile Trp Pro Gly Gly Ser His Leu Thr Gln Asp
           195                 200                 205
   Ile Thr Arg Phe Ile Ala Leu Glu Ser Arg Ser Tyr Val Leu Ser Val
       210                 215                 220
   Ser Gly Leu Met Arg Ala Thr Asp Phe Pro Lys Asp Thr Pro His Leu
   225                 230                 235                 240
   Ala Ser Ile Leu Ala Lys Gly Glu Glu Ile Leu Ala Asn Gly Gly Ser
                   245                 250                 255
   Cys Ile Ala Gly Pro Asp Gly Lys Trp Val Val Gly Pro Leu Val Gly
               260                 265                 270
   Glu Glu Lys Leu Ile Val Ala Thr Ile Asp His Cys Arg Val Arg Glu
           275                 280                 285
   Glu Arg Gln Asn Phe Asp Pro Ser Gly His Tyr Ser Arg Pro Asp Val
       290                 295                 300
   Leu Gln Leu Lys Ile Asn Arg Glu Arg Gln Ser Thr Ile Ser Phe Ser
   305                 310                 315                 320
   Glu
<210> 13
   <211> 1014
   <212> DNA
   <213> Unknown
<220>
   <223> Obtained from an environmental sample
<400> 13
   atgggcattc aacatccgaa atataaggtt gcggtggtgc aggcggcgcc ggcctggctc      60
   gatctcgatg cgtcgatcgc caaatcgatc gcgttgatcg aggaggcggc tgccaatggc     120
   gccaagctga tcgccttccc ggaggcgttc atccctggct atccctggta tatctggctg     180
   gactcgccgg cctgggcgat cggccgcggt tttgtgcagc gctatttcga caactcgctg     240
   gcctatgaca gcccgcaggc cgagaagctg cggctggcgg tgaagaaggc cggcctcacc     300
   gccgtgatcg gcctctccga gcgcgagggc ggcagccttt atctcgcgca atggctgatc     360
   gggcccgatg gcgagaccat cgcaaagcgc cgcaagctgc ggccgaccca tgccgagcgc     420
   accgtctatg gcgaaggcga tggcagcgat ctcgcggtgc atgaccgccc cggcatcggc     480
   cggctcggcg cgctgtgctg ctgggagcat ctgcagccgc tgtcgaaata cgcgatgtat     540
   gcccagaacg agcaggttca tgtcgcggcc tggccgagct tctcgctcta cgacccgttc     600
   gcgccggcgc tcggctggga ggtcaacaat gcggcctcac gcgtctatgc ggtggaaggc     660
   tcgtgcttcg tgctggcgcc ctgcgcgacg gtgtcgaagg cgatgatcga cgagctctgc     720
   gaccgcgacg acaagcacgg gctgctgcat gtcggcgggg gacacgccgc gatctatggg     780
   ccggacggct cttcgattgc ggagaaattg ccgccggagc aggagggcct gctctatgcc     840
   gacatcgatc tcggcgccat cgggattgcc aagaacgccg ccgatccggc cggacattac     900
   tcgcggcccg acgtgacgcg gctgttgctc aacaagaagc cgtcgaagcg tgtcgagcat     960
   ttttcgctgc cggtcgacaa tgtcgagccg gagatcgacg ccgccgccag ctga          1014
<210> 14
   <211> 337
   <212> PRT
   <213> Unknown
<220>
   <223> Obtained from an environmental sample
<400> 14
   Met Gly Ile Gln His Pro Lys Tyr Lys Val Ala Val Val Gln Ala Ala
   1               5                   10                  15
   Pro Ala Trp Leu Asp Leu Asp Ala Ser Ile Ala Lys Ser Ile Ala Leu
               20                  25                  30
   Ile Glu Glu Ala Ala Ala Asn Gly Ala Lys Leu Ile Ala Phe Pro Glu
           35                  40                  45
   Ala Phe Ile Pro Gly Tyr Pro Trp Tyr Ile Trp Leu Asp Ser Pro Ala
       50                  55                  60
   Trp Ala Ile Gly Arg Gly Phe Val Gln Arg Tyr Phe Asp Asn Ser Leu
   65                  70                  75                  80
   Ala Tyr Asp Ser Pro Gln Ala Glu Lys Leu Arg Leu Ala Val Lys Lys
                   85                  90                  95
   Ala Gly Leu Thr Ala Val Ile Gly Leu Ser Glu Arg Glu Gly Gly Ser
               100                 105                 110
   Leu Tyr Leu Ala Gln Trp Leu Ile Gly Pro Asp Gly Glu Thr Ile Ala
           115                 120                 125
   Lys Arg Arg Lys Leu Arg Pro Thr His Ala Glu Arg Thr Val Tyr Gly
       130                 135                 140
   Glu Gly Asp Gly Ser Asp Leu Ala Val His Asp Arg Pro Gly Ile Gly
   145                 150                 155                 160
   Arg Leu Gly Ala Leu Cys Cys Trp Glu His Leu Gln Pro Leu Ser Lys
                   165                 170                 175
   Tyr Ala Met Tyr Ala Gln Asn Glu Gln Val His Val Ala Ala Trp Pro
               180                 185                 190
   Ser Phe Ser Leu Tyr Asp Pro Phe Ala Pro Ala Leu Gly Trp Glu Val
           195                 200                 205
   Asn Asn Ala Ala Ser Arg Val Tyr Ala Val Glu Gly Ser Cys Phe Val
       210                 215                 220
   Leu Ala Pro Cys Ala Thr Val Ser Lys Ala Met Ile Asp Glu Leu Cys
   225                 230                 235                 240
   Asp Arg Asp Asp Lys His Gly Leu Leu His Val Gly Gly Gly His Ala
                   245                 250                 255
   Ala Ile Tyr Gly Pro Asp Gly Ser Ser Ile Ala Glu Lys Leu Pro Pro
               260                 265                 270
   Glu Gln Glu Gly Leu Leu Tyr Ala Asp Ile Asp Leu Gly Ala Ile Gly
           275                 280                 285
   Ile Ala Lys Asn Ala Ala Asp Pro Ala Gly His Tyr Ser Arg Pro Asp
       290                 295                 300
   Val Thr Arg Leu Leu Leu Asn Lys Lys Pro Ser Lys Arg Val Glu His
   305                 310                 315                 320
   Phe Ser Leu Pro Val Asp Asn Val Glu Pro Glu Ile Asp Ala Ala Ala
                   325                 330                 335
   Ser
<210> 15
   <211> 1047
   <212> DNA
   <213> Unknown
<220>
   <223> Obtained from an environmental sample
<400> 15
   atgccaacat caaaacaatt tagagtcgct gcagttcaag ccgccccggt atttcttgac      60
   ctggagggcg caataagcaa aggcatctcc ctcattgagg aggccgcttc caatggagcc     120
   aagctcattg ccttcccgga aacgtggatt cccggctacc cctggtggat ctggctggac     180
   tcacccgctt ggggcatgcg ctttgtccag cgctattttg acaactcgct catgctgggt     240
   agtgagcaag ccaagcgcat gaaccaggct gccgccaata acaagattta cgtggtgatg     300
   ggttatagcg aacgcagtgg cggcagcctc tacatgggcc aatccattat caacgacaag     360
   ggtgaaacga tttttacccg ccgcaaactc aagccaactc atgtcgagcg taccgtgttt     420
   ggggagggag acggcagcca tctttgcgta atggataccg agattggccg cgtcggcgcg     480
   atgtgctgtt gggaacattt gcagccgctc agcaaatatg caatgtattc tcaggatgaa     540
   caaattcaca ttgcctcctg gccgagcttt tcgttatatc ggggggcagc ctatgcactc     600
   ggccccgaac tgaacaacgc cgccagccaa atgtatgcag ccgaaggcca gtgctttgtc     660
   cttgcccctt gcgccaccgt ctcaaaggag atgatcgaaa tgctgataga tgatcccagg     720
   aaagagccgc ttctgctgga aggtggcggg ttcaccatga tttacggccc cgatgggcga     780
   cctctggcta aaccgttgcc tgaaaacgag gaagggctgc tatatgccga tattgacctg     840
   ggcatgattt caatggccaa ggctgccgcc gacccggcag gtcactacgc acgcccggat     900
   gtcactcgcc tactattcaa ttccgcgccc gccaatcgcg tcgagtatat caacccagcg     960
   tcaggcccaa ccgaatcctt aaaagatatg ggaaagatgc aaatggaggc cgaacagcaa    1020
   aaggcggccc tgcgagagat gatctaa                                        1047
<210> 16
   <211> 348
   <212> PRT
   <213> Unknown
<220>
   <223> Obtained from an environmental sample
<400> 16
   Met Pro Thr Ser Lys Gln Phe Arg Val Ala Ala Val Gln Ala Ala Pro
   1 5 10 15
   Val Phe Leu Asp Leu Glu Gly Ala Ile Ser Lys Gly Ile Ser Leu Ile
   20 25 30
   Glu Glu Ala Ala Ser Asn Gly Ala Lys Leu Ile Ala Phe Pro Glu Thr
   35 40 45
   Trp Ile Pro Gly Tyr Pro Trp Trp Ile Trp Leu Asp Ser Pro Ala Trp
   50 55 60
   Gly Met Arg Phe Val Gln Arg Tyr Phe Asp Asn Ser Leu Met Leu Gly
   65 70 75 80
   Ser Glu Gln Ala Lys Arg Met Asn Gln Ala Ala Ala Asn Asn Lys Ile
   85 90 95
   Tyr Val Val Met Gly Tyr Ser Glu Arg Ser Gly Gly Ser Leu Tyr Met
   100 105 110
   Gly Gln Ser Ile Ile Asn Asp Lys Gly Glu Thr Ile Phe Thr Arg Arg
   115 120 125
   Lys Leu Lys Pro Thr His Val Glu Arg Thr Val Phe Gly Glu Gly Asp
   130 135 140
   Gly Ser His Leu Cys Val Met Asp Thr Glu Ile Gly Arg Val Gly Ala
   145 150 155 160
   Met Cys Cys Trp Glu His Leu Gln Pro Leu Ser Lys Tyr Ala Met Tyr
   165 170 175
   Ser Gln Asp Glu Gln Ile His Ile Ala Ser Trp Pro Ser Phe Ser Leu
   180 185 190
   Tyr Arg Gly Ala Ala Tyr Ala Leu Gly Pro Glu Leu Asn Asn Ala Ala
   195 200 205
   Ser Gln Met Tyr Ala Ala Glu Gly Gln Cys Phe Val Leu Ala Pro Cys
   210 215 220
   Ala Thr Val Ser Lys Glu Met Ile Glu Met Leu Ile Asp Asp Pro Arg
   225 230 235 240
   Lys Glu Pro Leu Leu Leu Glu Gly Gly Gly Phe Thr Met Ile Tyr Gly
   245 250 255
   Pro Asp Gly Arg Pro Leu Ala Lys Pro Leu Pro Glu Asn Glu Glu Gly
   260 265 270
   Leu Leu Tyr Ala Asp Ile Asp Leu Gly Met Ile Ser Met Ala Lys Ala
   275 280 285
   Ala Ala Asp Pro Ala Gly His Tyr Ala Arg Pro Asp Val Thr Arg Leu
   290 295 300
   Leu Phe Asn Ser Ala Pro Ala Asn Arg Val Glu Tyr Ile Asn Pro Ala
   305 310 315 320
   Ser Gly Pro Thr Glu Ser Leu Lys Asp Met Gly Lys Met Gln Met Glu
   325 330 335
   Ala Glu Gln Gln Lys Ala Ala Leu Arg Glu Met Ile
   340 345
<210> 17
   <211> 993
   <212> DNA
   <213> Unknown
<220>
   <223> obtained from an environmental sample
<400> 17
   atgagagttg ttaaagccgc agctgtccaa ctgagtcccg tcctctatag ccgcgaggga 60
   acggtcgaga aggtcgtgcg gaagatccat gaacttgccg aagagggagt cgagttcgtc      120
   acctttcctg agaccgtggt gccttattac ccgtactttt cgttcgttca gacgcccttg      180
   cagcaaatct tcggaacaga gtatctgagg ctgctcgacc aggcagtcac cgtgccatcc      240
   gccgccaccg acgcgatcgg cgaggctgcc aggttcgctg gagttgttgt ctcgatcggc      300
   gtcaacgagc gagacggggg aactctgtac aacactcagc ttctcttcga tgccgacgga      360
   agcttaattc agcggcgccg caagatcacg cccacccatt acgagcgcat gatctggggc      420
   cagggtgacg gctcaggtct gcgggccgtt gatagcaagg ccggccgcat tggtcagctg      480
   gcatgctggg agcacaacaa tccactggcg cgctacgcgc tgatagccga cggcgagcag      540
   atccattcgg ccatgtatcc gggctccatg ttcggcgact cgtttgccaa aaagaccgaa      600
   atcaatatcc ggcagcatgc gctggagtct gcgtgcttcg tcgtgaacgc aacggcctgg      660
   ctggacggcg atcaacaggc gcaaatcatg aaggacaccg gctgcagcat cggcccgatc      720
   tccggcggtt gcttcaccac tatcgtggcg ccggacggtt ccctgatcgg cgagcccctc      780
   cgctcgggtg agggcgtggt catcgccgac ctcgacttca cgttaatcga caggcgtaag      840
   caggtgatgg actcgcgagg ccactacagc cggccggagt tgctcagcct cttaatagac      900
   cgcaccccta ccgcgcactt tcacgaacgc gcttcgcacc ccacgacagg agctgagcaa     960
   ggctccgagg atgtgttcga ggctaacatt taa                                   993
<210> 18
   <211> 330
   <212> PRT
   <213> Unknown
<220>
   <223> Obtained from an environmental sample
<400> 18
   Met Arg Val Val Lys Ala Ala Ala Val Gln Leu Ser Pro Val Leu Tyr
   1 5 10 15
   Ser Arg Glu Gly Thr Val Glu Lys Val Val Arg Lys Ile His Glu Leu
   20 25 30
   Ala Glu Glu Gly Val Glu Phe Val Thr Phe Pro Glu Thr Val Val Pro
   35 40 45
   Tyr Tyr Pro Tyr Phe Ser Phe Val Gln Thr Pro Leu Gln Gln Ile Phe
   50 55 60
   Gly Thr Glu Tyr Leu Arg Leu Leu Asp Gln Ala Val Thr Val Pro Ser
   65 70 75 80
   Ala Ala Thr Asp Ala Ile Gly Glu Ala Ala Arg Phe Ala Gly Val Val
   85 90 95
   Val Ser Ile Gly Val Asn Glu Arg Asp Gly Gly Thr Leu Tyr Asn Thr
   100 105 110
   Gln Leu Leu Phe Asp Ala Asp Gly Ser Leu Ile Gln Arg Arg Arg Lys
   115 120 125
   Ile Thr Pro Thr His Tyr Glu Arg Met Ile Trp Gly Gln Gly Asp Gly
   130 135 140
   Ser Gly Leu Arg Ala Val Asp Ser Lys Ala Gly Arg Ile Gly Gln Leu
   145 150 155 160
   Ala Cys Trp Glu His Asn Asn Pro Leu Ala Arg Tyr Ala Leu Ile Ala
   165 170 175
   Asp Gly Glu Gln Ile His Ser Ala Met Tyr Pro Gly Ser Met Phe Gly
   180 185 190
   Asp Ser Phe Ala Lys Lys Thr Glu Ile Asn Ile Arg Gln His Ala Leu
   195 200 205
   Glu Ser Ala Cys Phe Val Val Asn Ala Thr Ala Trp Leu Asp Gly Asp
   210 215 220
   Gln Gln Ala Gln Ile Met Lys Asp Thr Gly Cys Ser Ile Gly Pro Ile
   225 230 235 240
   Ser Gly Gly Cys Phe Thr Thr Ile Val Ala Pro Asp Gly Ser Leu Ile
   245 250 255
   Gly Glu Pro Leu Arg Ser Gly Glu Gly Val Val Ile Ala Asp Leu Asp
   260 265 270
   Phe Thr Leu Ile Asp Arg Arg Lys Gln Val Met Asp Ser Arg Gly His
   275 280 285
   Tyr Ser Arg Pro Glu Leu Leu Ser Leu Leu Ile Asp Arg Thr Pro Thr
   290 295 300
   Ala His Phe His Glu Arg Ala Ser His Pro Thr Thr Gly Ala Glu Gln
   305 310 315 320
   Gly Ser Glu Asp Val Phe Glu Ala Asn Ile
   325 330
<210> 19
   <211> 1050
   <212> DNA
   <213> Unknown
<220>
   <223> Obtained from an environmental sample
<400> 19
   atggaaagca acttccttgc cgcagcagtg caagcagaac cggtttactt caatgctttt      60
   cagacggccg aaaaggccgc gtcattgatt gacgatgccg gtcggcaggg ggctcgctta     120
   gtgacatttc ccgaaacgtg gctgcccggt tacccgtact ggatctggct tggtgccccc     180
   gcctggggaa tgcatcattt catcctaaag taccatcaaa actcgccggt tgcaggagga     240
   ccagaggaac agatcctttg tcaggcggcc cgccgcaacg ggatttttgt cgtcatggga     300
   ctcagcgaga aaatcggggc aagcctctac atggcgcagt ggttcatcag tccagacggc     360
   aaagtggtcg ctcgccgacg caaattgaag cctactcacg tcgaacgttc ggtcttcggg     420
   gaaggggatg gttccgacat tgtcgttctt gatacacccc ttggaaaggt cgggggcctt    480
   tgctgctggg agcacatgca gccactttcg aagtacgcca tgtactcgca aggcgagcag     540
   atccatgctg cttcttggcc gagtgttagc gtctatcgcg ataaaattta cgttctgggg     600
   ccggagctga acggtgccgc caatcagatg tatgcggcag aaggtcagtg tttcgtcctg     660
   gcatcctggg caacggtttc acaagcggct atcgatcttt tttgcgacac gcccgacaag     720
   gccgcgctca tgaaaattgg tggtggtttt tcccagatct atgggccaga cgggtgcccc     780
   ctggcgaagc cgttgccgga ggacgtcgaa ggattggtga ccgctgagat tgacttcaat     840
   gccatcacgc gcgtgaaagc agcggcggac cccgtagggc actatagccg gcccgatgta     900
   ttccgcctgt tgttcaatcg tacgcgccaa gaacgcgtgg tttctgtcaa cacgtttgtg     960
   ccaggtgtca cccagcgaac cgccaagaat gggtcggcgg acgaattggt cggtcacccg    1020
   gagaacgctg tcgcccgggc tgcagagtaa                                     1050
<210> 20
   <211> 349
   <212> PRT
   <213> Unknown
<220>
   <223> Obtained from an environmental sample
<400> 20
   Met Glu Ser Asn Phe Leu Ala Ala Ala Val Gln Ala Glu Pro Val Tyr
   1               5                   10                  15
   Phe Asn Ala Phe Gln Thr Ala Glu Lys Ala Ala Ser Leu Ile Asp Asp
               20                  25                  30
   Ala Gly Arg Gln Gly Ala Arg Leu Val Thr Phe Pro Glu Thr Trp Leu
           35                  40                  45
   Pro Gly Tyr Pro Tyr Trp Ile Trp Leu Gly Ala Pro Ala Trp Gly Met
       50                  55                  60
   His His Phe Ile Leu Lys Tyr His Gln Asn Ser Pro Val Ala Gly Gly
   65                  70                  75                  80
   Pro Glu Glu Gln Ile Leu Cys Gln Ala Ala Arg Arg Asn Gly Ile Phe
                   85                  90                  95
   Val Val Met Gly Leu Ser Glu Lys Ile Gly Ala Ser Leu Tyr Met Ala
               100                 105                 110
   Gln Trp Phe Ile Ser Pro Asp Gly Lys Val Val Ala Arg Arg Arg Lys
           115                 120                 125
   Leu Lys Pro Thr His Val Glu Arg Ser Val Phe Gly Glu Gly Asp Gly
       130                 135                 140
   Ser Asp Ile Val Val Leu Asp Thr Pro Leu Gly Lys Val Gly Gly Leu
   145                 150                 155                 160
   Cys Cys Trp Glu His Met Gln Pro Leu Ser Lys Tyr Ala Met Tyr Ser
                   165                 170                 175
   Gln Gly Glu Gln Ile His Ala Ala Ser Trp Pro Ser Val Ser Val Tyr
               180                 185                 190
   Arg Asp Lys Ile Tyr Val Leu Gly Pro Glu Leu Asn Gly Ala Ala Asn
           195                 200                 205
   Gln Met Tyr Ala Ala Glu Gly Gln Cys Phe Val Leu Ala Ser Trp Ala
       210                 215                 220
   Thr Val Ser Gln Ala Ala Ile Asp Leu Phe Cys Asp Thr Pro Asp Lys
   225                 230                 235                 240
   Ala Ala Leu Met Lys Ile Gly Gly Gly Phe Ser Gln Ile Tyr Gly Pro
                   245                 250                 255
   Asp Gly Cys Pro Leu Ala Lys Pro Leu Pro Glu Asp Val Glu Gly Leu
               260                 265                 270
   Val Thr Ala Glu Ile Asp Phe Asn Ala Ile Thr Arg Val Lys Ala Ala
           275                 280                 285
   Ala Asp Pro Val Gly His Tyr Ser Arg Pro Asp Val Phe Arg Leu Leu
       290                 295                 300
   Phe Asn Arg Thr Arg Gln Glu Arg Val Val Ser Val Asn Thr Phe Val
   305                 310                 315                 320
   Pro Gly Val Thr Gln Arg Thr Ala Lys Asn Gly Ser Ala Asp Glu Leu
                   325                 330                 335
   Val Gly His Pro Glu Asn Ala Val Ala Arg Ala Ala Glu
               340                 345
<210> 21
   <211> 1065
   <212> DNA
   <213> Unknown
<220>
   <223> Obtained from an environmental sample
<400> 21
   atggcactag aacatccgaa gtacgtggcg gccgtggttc aggccgcgcc cgaattcctg      60
   aatctagaca gagggatcga aaagacgatc gcattgatcg acgaagcggg acagaaaggg     120
   gcggccctga ttgcatttcc ggaaacctgg ctgccgggct atccgtttca tgtctggctc     180
   ggtcctcccg catgggcgct tggctcagga ttcgtccagc gctatttcga caactcgatg     240
   acgtacgata gtcctcaggc cgctgcactg agggacgctg ccgcgcgcaa cgggatcacg     300
   gtggtattgg gcttgtcgga gcgatgcggc ggcagcctct atatcgcgca atggatcatc     360
   ggcccggatg gcgcgacggt cgccacgcgc cgcaaattgc ggccgactca tatcgagcgc     420
   accgttttcg gcgatggcga cggcagcgat ctggcagtac acgatctcaa catcggccgc     480
   cttggcgcac tgtgctgctg ggagcacatt cagccgctga ccaagtacgc gatgtatgcg     540
   cagcacgaac aggtgcacgt cgcggcctgg ccgagcttct ccatgtatga attcgcgccc     600
   gcgctcggtc acgaggtgaa caacgcagtc agccgcgtct atgccgttga gggatcgtgc     660
   ttcgtgctcg cgccgtgcgc ggtcatcagc gagcaaatgg tcgacatgtt gtgcgacacg     720
   gcagacaagc gcgcgatgat acgtgccggc ggcgggcacg cagtggcgtt cgggccggac     780
   ggcgaagctc tggtcgagaa actgccggaa aatgaggaag gcttgctgct ggtcgatatc     840
   gatctcggtc gcatctcgct tgcgaaggct gcggccgacc ccgtcggtca ctacgcgcgc     900
   cccgatgtct tgcggctctg gttcgacaag caaccgcggc ggtgcgtcga acatgccggc     960
   gagaacgacg cgtcgcgcag gtcgcacggg tcgtccgggt cacaatcgcc ggcgcaggat    1020
   gggccggcga acgacatggt agaccgtcag gaaaacgtcg attga                   1065
<210> 22
   <211> 354
   <212> PRT
   <213> Unknown
<220>
   <223> Obtained from an environmental sample
<400> 22
   Met Ala Leu Glu His Pro Lys Tyr Val Ala Ala Val Val Gln Ala Ala
   1               5                   10                  15
   Pro Glu Phe Leu Asn Leu Asp Arg Gly Ile Glu Lys Thr Ile Ala Leu
               20                  25                  30
   Ile Asp Glu Ala Gly Gln Lys Gly Ala Ala Leu Ile Ala Phe Pro Glu
           35                  40                  45
   Thr Trp Leu Pro Gly Tyr Pro Phe His Val Trp Leu Gly Pro Pro Ala
       50                  55                  60
   Trp Ala Leu Gly Ser Gly Phe Val Gln Arg Tyr Phe Asp Asn Ser Met
   65                  70                  75                  80
   Thr Tyr Asp Ser Pro Gln Ala Ala Ala Leu Arg Asp Ala Ala Ala Arg
                   85                  90                  95
   Asn Gly Ile Thr Val Val Leu Gly Leu Ser Glu Arg Cys Gly Gly Ser
               100                 105                 110
   Leu Tyr Ile Ala Gln Trp Ile Ile Gly Pro Asp Gly Ala Thr Val Ala
           115                 120                 125
   Thr Arg Arg Lys Leu Arg Pro Thr His Ile Glu Arg Thr Val Phe Gly
       130                 135                 140
   Asp Gly Asp Gly Ser Asp Leu Ala Val His Asp Leu Asn Ile Gly Arg
   145                 150                 155                 160
   Leu Gly Ala Leu Cys Cys Trp Glu His Ile Gln Pro Leu Thr Lys Tyr
                   165                 170                 175
   Ala Met Tyr Ala Gln His Glu Gln Val His Val Ala Ala Trp Pro Ser
               180                 185                 190
   Phe Ser Met Tyr Glu Phe Ala Pro Ala Leu Gly His Glu Val Asn Asn
           195                 200                 205
   Ala Val Ser Arg Val Tyr Ala Val Glu Gly Ser Cys Phe Val Leu Ala
       210                 215                 220
   Pro Cys Ala Val Ile Ser Glu Gln Met Val Asp Met Leu Cys Asp Thr
   225                 230                 235                 240
   Ala Asp Lys Arg Ala Met Ile Arg Ala Gly Gly Gly His Ala Val Ala
                   245                 250                 255
   Phe Gly Pro Asp Gly Glu Ala Leu Val Glu Lys Leu Pro Glu Asn Glu
               260                 265                 270
   Glu Gly Leu Leu Leu Val Asp Ile Asp Leu Gly Arg Ile Ser Leu Ala
           275                 280                 285
   Lys Ala Ala Ala Asp Pro Val Gly His Tyr Ala Arg Pro Asp Val Leu
       290                 295                 300
   Arg Leu Trp Phe Asp Lys Gln Pro Arg Arg Cys Val Glu His Ala Gly
   305                 310                 315                 320
   Glu Asn Asp Ala Ser Arg Arg Ser His Gly Ser Ser Gly Ser Gln Ser
                   325                 330                 335
   Pro Ala Gln Asp Gly Pro Ala Asn Asp Met Val Asp Arg Gln Glu Asn
               340                 345                 350
   Val Asp
<210> 23
   <211> 1005
   <212> DNA
   <213> Unknown
<220>
   <223> Obtained from an environmental sample
<400> 23
   atgaccgtta tcaaagcagc cgccattcaa atcagccccg tgctctacag ccgggcgggg      60
   acagtcgaga aagttgttag gaaggttaga gagctcgggg ccaaaggtgt ccgattcgct     120
   acctttcccg aaaccatcat accgtactac ccgtacttct cgttcgttca gtcggcgttc     180
   gacatgaagc ttgggagtga acatcagcgg ctgctcgacg aatcagtcac aattccttcg     240
   tccgagacgg acgcgatcgc ccaggccgcc aaggaagcgg gcatggtggt gtccgtcggg     300
   gtcaatgagc gcgatgggcg atccatctac aacactcaac ttctgttcga cgctgatggc     360
   acgctcattc agcgtaggcg aaagatcacc ccgacctatc acgagcgcat gatttggggt     420
   caaggcgatg gatccggcct acgcgcggtc gatagcgccg tgggccggat cggccagctt     480
   gcctgctggg agcactacct tcccctggcg cggtacgccc tcatcgcgga cggagagcaa     540
   atccactcgg caatgtatcc aggctcgttc gctggtccgc tatttgccga gcagatagag     600
   gttagtatcc gccagcacgc gcttgagtca gcctgcttcg tcgtcaacgc gaccggatgg     660
   cttagcgccg agcagcaagc tcaaatagtg aaggataccg gatgcgtcgt tggaccaatc     720
   tccggtggct gctttacggc gattgttgat ccggagggtc ggatcatggg ggcgccactc     780
   aaggcaggtg agggggaggt catcgcagat ctcgattttg cgcagattga tttccgcaag      840
   cgtgtgatgg atacgcgagg gcactacagc cgccccgaac ttctaagcct cacgatcgac    900
   cgcagtcagc accatcacat gactgagcga ggcgccgatc accgtgtaga ccacgcaaag     960
   ccaacggtca ccgcagagca gtcggccgtc gagccggcgg aatga                    1005
<210> 24
   <211> 334
   <212> PRT
   <213> Unknown
<220>
   <223> Obtained from an environmental sample
<400> 74
<210> 25
   <211> 939
   <212> DNA
   <213> unknown
<220>
   <223> Obtained from an environmental sample
<400> 25
   gtgtcatcaa ctatcaaagt cgccattatt caggccgctc ctgcttacta cgacctgcag      60
   gcgtcgctgg caaaggccgc cagtctgatc cgcgaggcgg cacgcggcgg cgcgcaattc     120
   gtcgcgttcg gggagacatg gctgccgggc tatccgatgt ggctggattg gtgtcctggc     180
   gcgatcatct gggataaccc cgccaccaaa accgtcttcg cgcgcctcca tgaaaacagc     240
   gtcgccgttc ccggcaggga aacggcattt ctcgccgacc ttgcgatgtc gttaagcatc     300
   gtattatgca tcggcgtcaa tgagaaggtc atgaatgggc cgggacacgg cacgctctac     360
   aacacgctcc tgacgtttga tgcaacgggt gaaatcatca atcatcatcg caagttgatg     420
   ccaacctatg gcgagagatt ggtatggggg ccgggcgacg cagttggcgt gcaagcggtt     480
   gatagtacgg tcgggcgcat cggcgggctg atctgttggg agcactggat gccgctgcca     540
   cgccaactca tgcacaacag cggcgagcag attcacgtct gcgcatggcc gggcgtgcac     600
   gaaatgcacc agatcgcgag ccgtcattat gcattcgagg gccgctgctt tgtgctggcc     660
   gccggattga tcatgcccgc gttcgacctg cccagcgaac tcgaatttcc gcccgaactg     720
   gccgacaagc gcgactatct cctaatgaac ggcggcagcg ccatcatcaa gcccaatggc     780
   aaatatctcg ccgggccggt ttatgacgaa gagactattc tctgcgccga ccttgacctg     840
   actgagaaca tcaaggagca gatgacgctg gacgtgacag ggcattatgc gcgagcggaa     900
   ctgtttgact tgaatgtggt gcggcggcgg aatgcgtag                            939
<210> 26
   <211> 312
   <212> PRT
   <213> Unknown
<220>
   <223> Obtained from an environmental sample
<400> 26
   Val Ser Ser Thr Ile Lys Val Ala Ile Ile Gln Ala Ala Pro Ala Tyr
   1               5                   10                  15
   Tyr Asp Leu Gln Ala Ser Leu Ala Lys Ala Ala Ser Leu Ile Arg Glu
               20                  25                  30
   Ala Ala Arg Gly Gly Ala Gln Phe Val Ala Phe Gly Glu Thr Trp Leu
           35                  40                  45
   Pro Gly Tyr Pro Met Trp Leu Asp Trp Cys Pro Gly Ala Ile Ile Trp
       50                  55                  60
   Asp Asn Pro Ala Thr Lys Thr Val Phe Ala Arg Leu His Glu Asn Ser
   65                  70                  75                  80
   Val Ala Val Pro Gly Arg Glu Thr Ala Phe Leu Ala Asp Leu Ala Met
                   85                  90                  95
   Ser Leu Ser Ile Val Leu Cys Ile Gly Val Asn Glu Lys Val Met Asn
               100                 105                 110
   Gly Pro Gly His Gly Thr Leu Tyr Asn Thr Leu Leu Thr Phe Asp Ala
           115                 120                 125
   Thr Gly Glu Ile Ile Asn His His Arg Lys Leu Met Pro Thr Tyr Gly
       130                 135                 140
   Glu Arg Leu Val Trp Gly Pro Gly Asp Ala Val Gly Val Gln Ala Val
   145                 150                 155                 160
   Asp Ser Thr Val Gly Arg Ile Gly Gly Leu Ile Cys Trp Glu His Trp
                   165                 170                 175
   Met Pro Leu Pro Arg Gln Leu Met His Asn Ser Gly Glu Gln Ile His
               180                 185                 190
   Val Cys Ala Trp Pro Gly Val His Glu Met His Gln Ile Ala Ser Arg
           195                 200                 205
   His Tyr Ala Phe Glu Gly Arg Cys Phe Val Leu Ala Ala Gly Leu Ile
       210                 215                 220
   Met Pro Ala Phe Asp Leu Pro Ser Glu Leu Glu Phe Pro Pro Glu Leu
   225                 230                 235                 240
   Ala Asp Lys Arg Asp Tyr Leu Leu Met Asn Gly Gly Ser Ala Ile Ile
                   245                 250                 255
   Lys Pro Asn Gly Lys Tyr Leu Ala Gly Pro Val Tyr Asp Glu Glu Thr
               260                 265                 270
   Ile Leu Cys Ala Asp Leu Asp Leu Thr Glu Asn Ile Lys Glu Gln Met
           275                 280                 285
   Thr Leu Asp Val Thr Gly His Tyr Ala Arg Ala Glu Leu Phe Asp Leu
       290                 295                 300
   Asn Val Val Arg Arg Arg Asn Ala
   305                 310
<210> 27
   <211> 1056
   <212> DNA
   <213> Unknown
<220>
   <223> Obtained from an environmental sample
<400> 27
   atgccaaccc ccagcgatca tttcaaaatc gccgctgttc aggcctcgcc cgtgtttctg     60
   gaccgggagg ccactgtgga aaaggcctgc cggttgatcg ccgaagccgc aaagcagggc    120
   gcccgcctca tcgtctttcc ggaatctttc atcccgacct acccggattg ggtgtgggcc    180
   gttcccccgg gaagggaaag aatcctgaac cagctgtatt ctgaattcct ggccaatgcc    240
   gtcgatgttc ccggcgcggc gaccgaacaa cttgcccagg ctgcacgaat ggccggcgcc    300
   tatgtgatta tgggcgtcac cgaaagagac acctcggcca gcggggccag cctctacaac    360
   accctgctct acttcagccc cgaaggcatc ctaatgggca aacaccggaa gctggttccc    420
   acggggggcg aacggctggt ctgggcctac ggagacggca gcacgctgga ggtctacgac     480
   actccgctgg gaaagatcgg cgggctgatc tgctgggaga actacatgcc cctggcccgg    540
   tacacgatgt acgcctgggg cacccagatt tacatcgccg ccacctggga ccgcggggaa    600
   ccgtggctct ccaccctgcg gcatatcgcc aaggaaggaa gggtctacgt catcgggtgc    660
   tgcatcgccc tgcgccaggg ggatatcccg gaccggttcg agtacaaggg aaaattttat    720
   tccgggtccc gggagtggat caatgagggc gacagcgcca tcgtgaaccc ggacggggaa    780
   ttcatcgccg ggccggtgcg gatgaaggag gagatcctgt atgccgagat agacccccgg     840
   cagatgcggg gccccaagtg gatgctcgat gtggccggtc attacgcccg gccggatatc    900
   ttcgagctca tcgtccaccg gaatccccac ccgatgatca aaatcgccga agacaggggc    960
   acggggatcg cctcaagttt gattcgcccc cgccctaacc ttcccccatc aagggggagg  1020
   aaatcggcaa gaagcaaacg caagcccaaa aaatga                             1056
<210> 28
   <211> 351
   <212> PRT
   <213> Unknown
<220>
   <223> Obtained from an environmental sample
<400> 28
   Met Pro Thr Pro Ser Asp His Phe Lys Ile Ala Ala Val Gln Ala Ser
   1 5 10 15
   Pro Val Phe Leu Asp Arg Glu Ala Thr Val Glu Lys Ala Cys Arg Leu
   20 25 30
   Ile Ala Glu Ala Ala Lys Gln Gly Ala Arg Leu Ile Val Phe Pro Glu
   35 40 45
   Ser Phe Ile Pro Thr Tyr Pro Asp Trp Val Trp Ala Val Pro Pro Gly
   50 55 60
   Arg Glu Arg Ile Leu Asn Gln Leu Tyr Ser Glu Phe Leu Ala Asn Ala
   65 70 75 80
   Val Asp Val Pro Gly Ala Ala Thr Glu Gln Leu Ala Gln Ala Ala Arg
   85 90 95
   Met Ala Gly Ala Tyr Val Ile Met Gly Val Thr Glu Arg Asp Thr Ser
   100 105 110
   Ala Ser Gly Ala Ser Leu Tyr Asn Thr Leu Leu Tyr Phe Ser Pro Glu
   115 120 125
   Gly Ile Leu Met Gly Lys His Arg Lys Leu Val Pro Thr Gly Gly Glu
   130 135 140
   Arg Leu Val Trp Ala Tyr Gly Asp Gly Ser Thr Leu Glu Val Tyr Asp
   145 150 155 160
   Thr Pro Leu Gly Lys Ile Gly Gly Leu Ile Cys Trp Glu Asn Tyr Met
   165 170 175
   Pro Leu Ala Arg Tyr Thr Met Tyr Ala Trp Gly Thr Gln Ile Tyr Ile
   180 185 190
   Ala Ala Thr Trp Asp Arg Gly Glu Pro Trp Leu Ser Thr Leu Arg His
   195 200 205
   Ile Ala Lys Glu Gly Arg Val Tyr Val Ile Gly Cys Cys Ile Ala Leu
   210 215 220
   Arg Gln Gly Asp Ile Pro Asp Arg Phe Glu Tyr Lys Gly Lys Phe Tyr
   225 230 235 240
   Ser Gly Ser Arg Glu Trp Ile Asn Glu Gly Asp Ser Ala Ile Val Asn
   245 250 255
   Pro Asp Gly Glu Phe Ile Ala Gly Pro Val Arg Met Lys Glu Glu Ile
   260 265 270
   Leu Tyr Ala Glu Ile Asp Pro Arg Gln Met Arg Gly Pro Lys Trp Met
   275 280 285
   Leu Asp Val Ala Gly His Tyr Ala Arg Pro Asp Ile Phe Glu Leu Ile
   290 295 300
   Val His Arg Asn Pro His Pro Met Ile Lys Ile Ala Glu Asp Arg Gly
   305 310 315 320
   Thr Gly Ile Ala Ser Ser Leu Ile Arg Pro Arg Pro Asn Leu Pro Pro
   325 330 335
   Ser Arg Gly Arg Lys Ser Ala Arg Ser Lys Arg Lys Pro Lys Lys
   340 345 350
<210> 29
   <211> 1017
   <212> DNA
   <213> Unknown
<220>
   <223> Obtained from an environmental sample
<400> 29
   atgggcatcg aacatacgaa atacaaggtg gcggtggtgc aggcggcgcc ggcctggctc 60
   gacctcgagg cctcgatcgg caagtccatc ggcctaatca aggaggccgc ggacaagggc 120
   gccaagctga tcgcctttcc ggaggccttc atccccggtt acccctggta tatctggatg     180
   gactcgccgg cctgggcgat cggccgcggc ttcgtccagc gctatttcga caattcgctc     240
   tcctacgaca gtccccaggc cgagcggctg cgtgatgccg tgcgccaggc caagctcacc     300
   gccgtgatcg gcctgtccga acgcgacggc ggcagccttt acctggcgca atggttgatc     360
   gggcccgacg gcgaaaccat tgccaagcgc cgcaagctgc ggccgaccca tgccgagcgc     420
   accgtctatg gcgaaggcga cggcagcgat ctggccgtac atgcccggcc cgacatcggt     480
   cgcttgggcg cgctgtgctg ctgggagcat cttcagccgt tgtcgaagta cgcaatgtac     540
   gcccagaacg agcaggtcca cgtcgctgcc tggccgagct tctcgctcta cgatcccttc     600
   gccccggcgc tcggcgccga ggtcaacaac gctgcctcgc gcgtctatgc ggtggagggc     660
   tcctgcttcg tgctcgcgcc ttgcgcgacg gtgtcgcagg ccatgatcga cgaactctgc     720
   gatcggcccg ataagcatgc gctgctgcat gccggcggag gctttgccgc gatctacggc     780
   cccgacggca gccagatcgg cgagaagctg gcgccggatc aggagggtct gctgatcgcc     840
   gagattgatc tgggcgccat cggtgttgcc aagaacgcgg cagatcccgc cggtcattat     900
   tcacggccgg atgtgacgcg gttgctgctc aacaagaagc ggtaccagcg cgtcgagcaa     960
   tttgccttgc ccgccgacat ggtcgagccc gcggacatag gcgcggcggc gagctga       1017
<210> 30
   <211> 338
   <212> PRT
   <213> Unknown
<220>
   <223> Obtained from an environmental sample
<400> 30
   Met Gly Ile Glu His Thr Lys Tyr Lys Val Ala Val Val Gln Ala Ala
   1               5                   10                  15
   Pro Ala Trp Leu Asp Leu Glu Ala Ser Ile Gly Lys Ser Ile Gly Leu
               20                  25                  30
   Ile Lys Glu Ala Ala Asp Lys Gly Ala Lys Leu Ile Ala Phe Pro Glu
           35                  40                  45
   Ala Phe Ile Pro Gly Tyr Pro Trp Tyr Ile Trp Met Asp Ser Pro Ala
       50                  55                  60
   Trp Ala Ile Gly Arg Gly Phe Val Gln Arg Tyr Phe Asp Asn Ser Leu
   65                  70                  75                  80
   Ser Tyr Asp Ser Pro Gln Ala Glu Arg Leu Arg Asp Ala Val Arg Gln
                   85                  90                  95
   Ala Lys Leu Thr Ala Val Ile Gly Leu Ser Glu Arg Asp Gly Gly Ser
               100                 105                 110
   Leu Tyr Leu Ala Gln Trp Leu Ile Gly Pro Asp Gly Glu Thr Ile Ala
           115                 120                 125
   Lys Arg Arg Lys Leu Arg Pro Thr His Ala Glu Arg Thr Val Tyr Gly
       130                 135                 140
   Glu Gly Asp Gly Ser Asp Leu Ala Val His Ala Arg Pro Asp Ile Gly
   145                 150                 155                 160
   Arg Leu Gly Ala Leu Cys Cys Trp Glu His Leu Gln Pro Leu Ser Lys
                   165                 170                 175
   Tyr Ala Met Tyr Ala Gln Asn Glu Gln Val His Val Ala Ala Trp Pro
               180                 185                 190
   Ser Phe Ser Leu Tyr Asp Pro Phe Ala Pro Ala Leu Gly Ala Glu Val
           195                 200                 205
   Asn Asn Ala Ala Ser Arg Val Tyr Ala Val Glu Gly Ser Cys Phe Val
       210                 215                 220
   Leu Ala Pro Cys Ala Thr Val Ser Gln Ala Met Ile Asp Glu Leu Cys
   225                 230                 235                 240
   Asp Arg Pro Asp Lys His Ala Leu Leu His Ala Gly Gly Gly Phe Ala
                   245                 250                 255
   Ala Ile Tyr Gly Pro Asp Gly Ser Gln Ile Gly Glu Lys Leu Ala Pro
               260                 265                 270
   Asp Gln Glu Gly Leu Leu Ile Ala Glu Ile Asp Leu Gly Ala Ile Gly
           275                 280                 285
   Val Ala Lys Asn Ala Ala Asp Pro Ala Gly His Tyr Ser Arg Pro Asp
       290                 295                 300
   Val Thr Arg Leu Leu Leu Asn Lys Lys Arg Tyr Gln Arg Val Glu Gln
   305                 310                 315                 320
   Phe Ala Leu Pro Ala Asp Met Val Glu Pro Ala Asp Ile Gly Ala Ala
                   325                 330                 335
   Ala Ser
<210> 31
   <211> 933
   <212> DNA
   <213> Unknown
<220>
   <223> Obtained from an environmental sample
<400> 31
   atgaccagaa tagccattat tcagcgaccg cccgtgctgc tcgatcgaag cgccaccatt     60
   gcccgggccg tgcaatcggt cgccgaagcg gcagcgcaag gcgcgaccct gattgtcttg    120
   cccgaatcgt acatccctgg ctatccctca tggatctggc ggctcgcgcc tggcaaagac    180
   ggcgcgatcg tgggccagtt gcatgcgcgc ttgctggcca atgcggtcga cctgagcagc    240
   actgacctcg atgcgcttct tgaagcggcc cgtcagcacg gcgtgaccat tgtttgcggc    300
   atgaacgagt gcgaacggcg tcgcggcggc ggcaccttgt acaacacggt ggtcgtgatc    360
   ggaccggacg gcgtcatgct caaccggcat cgcaaattga tgccgaccaa tcccgagcgc    420
   atggtgcatg gctttggcga tgcatccgga ctgaaagcag ttgatacgcc tgccggccgg    480
   ctgggcacgc tgatctgctg ggagagctac atgccgctgg cacgctatgc cctgtacgag    540
   caaggcatcg agatctacat cgcaccaact tatgacagtg gtgacggctg gatcagcacc    600
   atgcgccaca ttgcactcga agggcgctgc tgggtgattg gcagcggcac ggtcctgaaa    660
   ggcagtgata ttccggacga tttcccggaa cgggcacgcc tgttccctga tccggatgag    720
   tggatcaacg atggtgattc ggtagttatc gatccgcagg gaaagatcgt tgccggtccg    780
   atgcgtaggg aagcaggcat tctatacgcc gatatcgacg tcgcgcgcgt agcaccatca    840
   cgccgcacgc tggatgtcgc ggggcattac gcgcgtccgg acgtcttcga gcttcgggta    900
   caccaggcac cgggggcacg agtaagtaat tga                                 933
<210> 32
   <211> 310
   <212> PRT
   <213> Unknown
<220>
   <223> Obtained from an environmental sample
<400> 32
   Met Thr Arg Ile Ala Ile Ile Gln Arg Pro Pro Val Leu Leu Asp Arg
   1               5                   10                  15
   Ser Ala Thr Ile Ala Arg Ala Val Gln Ser Val Ala Glu Ala Ala Ala
               20                  25                  30
   Gln Gly Ala Thr Leu Ile Val Leu Pro Glu Ser Tyr Ile Pro Gly Tyr
           35                  40                  45
   Pro Ser Trp Ile Trp Arg Leu Ala Pro Gly Lys Asp Gly Ala Ile Val
       50                  55                  60
   Gly Gln Leu His Ala Arg Leu Leu Ala Asn Ala Val Asp Leu Ser Ser
   65                  70                  75                  80
   Thr Asp Leu Asp Ala Leu Leu Glu Ala Ala Arg Gln His Gly Val Thr
                   85                  90                  95
   Ile Val Cys Gly Met Asn Glu Cys Glu Arg Arg Arg Gly Gly Gly Thr
               100                 105                 110
   Leu Tyr Asn Thr Val Val Val Ile Gly Pro Asp Gly Val Met Leu Asn
           115                 120                 125
   Arg His Arg Lys Leu Met Pro Thr Asn Pro Glu Arg Met Val His Gly
       130                 135                 140
   Phe Gly Asp Ala Ser Gly Leu Lys Ala Val Asp Thr Pro Ala Gly Arg
   145                 150                 155                 160
   Leu Gly Thr Leu Ile Cys Trp Glu Ser Tyr Met Pro Leu Ala Arg Tyr
                   165                 170                 175
   Ala Leu Tyr Glu Gln Gly Ile Glu Ile Tyr Ile Ala Pro Thr Tyr Asp
               180                 185                 190
   Ser Gly Asp Gly Trp Ile Ser Thr Met Arg His Ile Ala Leu Glu Gly
           195                 200                 205
   Arg Cys Trp Val Ile Gly Ser Gly Thr Val Leu Lys Gly Ser Asp Ile
       210                 215                 220
   Pro Asp Asp Phe Pro Glu Arg Ala Arg Leu Phe Pro Asp Pro Asp Glu
   225                 230                 235                 240
   Trp Ile Asn Asp Gly Asp Ser Val Val Ile Asp Pro Gln Gly Lys Ile
                   245                 250                 255
   Val Ala Gly Pro Met Arg Arg Glu Ala Gly Ile Leu Tyr Ala Asp Ile
                   260                     265                     270
   Asp Val Ala Arg Val Ala Pro Ser Arg Arg Thr Leu Asp Val Ala Gly
           275                     280                     285
   His Tyr Ala Arg Pro Asp Val Phe Glu Leu Arg Val His Gln Ala Pro
       290                     295                     300
   Gly Ala Arg Val Ser Asn
   305                 310
<210> 33
   <211> 1026
   <212> DNA
   <213> Unknown
<220>
   <223> Obtained from an environmental sample
<400> 33
   atgttaagtc ccgtgacgca gtatcgcgcc gccgcggtgc aggcggcgcc atcttttctc      60
   gatctcgacc gcaccgtcga gaagacgatc gcgatcatcg agcaggcggc cgagcaggat     120
   gtgcgcctga tcgcgtttcc ggaaacctgg attcccggct atccgctctg gatctggctc     180
   ggctcgccgg cctggggcat gcgcttcgtg cagcgctatt tcgagaactc gctggtgcgc     240
   ggcagcaaac agtggaacgc gatcgccgat gcggcgcggc gccaccgcat gaccgtcgtc     300
   gtcggcttca gcgagcgcgc gggaggcagc ctctacatgg gccaggcgat cttcggcccc     360
   gaaggcgagc tcatcgcggc gcgccggaag ctcaagccga cacacgccga gcgaacggtg     420
   ttcggcgagg gcgacggcag ccacttggcc gtttacgaga cgggcgttgg tcgcatcggc     480
   gccctctgct gctgggagca catccagccg ctctcgaaat acgcgatgta tgcggccaac     540
   gaacaggtgc atgtggcctc gtggccgtgc ttcagccttt atcgcggcat ggcctatgcg     600
   ctcgggccgg aggtgaacac cgccgcgagc caggtctacg cggtcgaggg cggctgctac     660
   gtgctggcct cctgtctcgt cgtgacaccc gagatcctga aggtgctgat cgacacgccc     720
   gacaaggagc cgttgctgct cgccggcggg gggttctcga tgatcttcgg ccccgacggc     780
   cgcgcgctcg cccagccgct gccggagacc gaagaggggc tcgtcacggc cgagatcgat     840
   ctcggcgcga tcgcgctcgc caaggccgcg gccgatcccg ccggccatta cgcgcggccc     900
   gacgtgacgc ggttgttgct gaacccgcgc cccgcggcgc gcgtcgaagc gctgggtccg     960
   cgcttcgagg tcgtgcagag cgagcaggcc gagccgccca cgcaaccggc cgaagcggcg    1020
   gattga                                                                1026
<210> 34
   <211> 341
   <212> PRT
   <213> Unknown
<220>
   <223> Obtained from an environmental sample
<400> 34
   Met Leu Ser Pro Val Thr Gln Tyr Arg Ala Ala Ala Val Gln Ala Ala
   1               5                   10                  15
   Pro Ser Phe Leu Asp Leu Asp Arg Thr Val Glu Lys Thr Ile Ala Ile
               20                  25                  30
   Ile Glu Gln Ala Ala Glu Gln Asp Val Arg Leu Ile Ala Phe Pro Glu
           35                  40                  45
   Thr Trp Ile Pro Gly Tyr Pro Leu Trp Ile Trp Leu Gly Ser Pro Ala
       50                  55                  60
   Trp Gly Met Arg Phe Val Gln Arg Tyr Phe Glu Asn Ser Leu Val Arg
   65                  70                  75                  80
   Gly Ser Lys Gln Trp Asn Ala Ile Ala Asp Ala Ala Arg Arg His Arg
                   85                  90                  95
   Met Thr Val Val Val Gly Phe Ser Glu Arg Ala Gly Gly Ser Leu Tyr
               100                 105                 110
   Met Gly Gln Ala Ile Phe Gly Pro Glu Gly Glu Leu Ile Ala Ala Arg
           115                 120                 125
   Arg Lys Leu Lys Pro Thr His Ala Glu Arg Thr Val Phe Gly Glu Gly
       130                 135                 140
   Asp Gly Ser His Leu Ala Val Tyr Glu Thr Gly Val Gly Arg Ile Gly
   145                 150                 155                 160
   Ala Leu Cys Cys Trp Glu His Ile Gln Pro Leu Ser Lys Tyr Ala Met
                   165                 170                 175
   Tyr Ala Ala Asn Glu Gln Val His Val Ala Ser Trp Pro Cys Phe Ser
               180                 185                 190
   Leu Tyr Arg Gly Met Ala Tyr Ala Leu Gly Pro Glu Val Asn Thr Ala
           195                 200                 205
   Ala Ser Gln Val Tyr Ala Val Glu Gly Gly Cys Tyr Val Leu Ala Ser
       210                 215                 220
   Cys Leu Val Val Thr Pro Glu Ile Leu Lys Val Leu Ile Asp Thr Pro
   225                 230                 235                 240
   Asp Lys Glu Pro Leu Leu Leu Ala Gly Gly Gly Phe Ser Met Ile Phe
                   245                 250                 255
   Gly Pro Asp Gly Arg Ala Leu Ala Gln Pro Leu Pro Glu Thr Glu Glu
               260                 265                 270
   Gly Leu Val Thr Ala Glu Ile Asp Leu Gly Ala Ile Ala Leu Ala Lys
           275                 280                 285
   Ala Ala Ala Asp Pro Ala Gly His Tyr Ala Arg Pro Asp Val Thr Arg
       290                 295                 300
   Leu Leu Leu Asn Pro Arg Pro Ala Ala Arg Val Glu Ala Leu Gly Pro
   305                 310                 315                 320
   Arg Phe Glu Val Val Gln Ser Glu Gln Ala Glu Pro Pro Thr Gln Pro
                   325                 330                 335
   Ala Glu Ala Ala Asp
               340
<210> 35
   <211> 942
   <212> DNA
   <213> Unknown
<220>
   <223> Obtained from an environmental sample
<400> 35
   atgacagctc taaaaattgc tgctgttcaa atgtgcgccg aattgggcgc tacagatcga      60
   aacctgagtg cagctggatc attcgtgcgc gacgcatttc gcgaaggtgc ccagtgggta     120
   atcctcccag agttttttac ctcggcaatg gcattcgcac cttcgatggc gcaagcttgg      180
   ttgccactgg aaggaaaggc gctagcgatg atgcgcagcc ttgcgcgtca attcgatggg     240
   gttgttggag gctcatatgt tgccagagag gggaacgact gcgtaaatgc ctttcttctc     300
   gtctttccgg atggaagcta ctaccggcat gacaaagata ttccaacaat gtgggagaac     360
   tgttactaca tcggcggcgt cgacgatggg gtgctggaaa caccaattgg tgcggtggga     420
   gttgcactgt gttgggagtt catccgaaca caaaccgccc gaagactgaa ggatcgcgtt     480
   caattagtgg ttggcggtac ttgctggtgg gattttccga tgcctgtacc tgaacgatat     540
   ctgaggctga ccaggcatat ctccaggaac tttgagcgcg atgctccggc gcggttggcc     600
   agtatgttgg gtgtgcctgt tgtacacgct tcccatgctg gggattttac tgctgtcacc     660
   ccaggcaatg aaacgaagaa ttaccgatcc aactatctgg gagagaccca gatcgtcgat     720
   gccaatggaa atgtgttgaa gcgaatgaca gtggctgatg gtgagggtta cgtcattgct     780
   gacgttcaat tgggggccat atcaaccggt cgaacttcga tccccgacac cttctggacc     840
   tgcaagctaa cgccaggggc acaacaggct tgggatgaac aaaatgcttt tgggtgtggc     900
   tactatgaga acgtcacacg caaacaccta atcggtcgat ga                        942
<210> 36
   <211> 313
   <212> PRT
   <213> Unknown
<220>
   <223> Obtained from an environmental sample
<400> 36
   Met Thr Ala Leu Lys Ile Ala Ala Val Gln Met Cys Ala Glu Leu Gly
   1               5                   10                  15
   Ala Thr Asp Arg Asn Leu Ser Ala Ala Gly Ser Phe Val Arg Asp Ala
               20                  25                  30
   Phe Arg Glu Gly Ala Gln Trp Val Ile Leu Pro Glu Phe Phe Thr Ser
           35                  40                  45
   Ala Met Ala Phe Ala Pro Ser Met Ala Gln Ala Trp Leu Pro Leu Glu
       50                  55                  60
   Gly Lys Ala Leu Ala Met Met Arg Ser Leu Ala Arg Gln Phe Asp Gly
   65                  70                  75                  80
   Val Val Gly Gly Ser Tyr Val Ala Arg Glu Gly Asn Asp Cys Val Asn
                   85                  90                  95
   Ala Phe Leu Leu Val Phe Pro Asp Gly Ser Tyr Tyr Arg His Asp Lys
                           100                     105                     110
   Asp Ile Pro Thr Met Trp Glu Asn Cys Tyr Tyr Ile Gly Gly Val Asp
               115                     120                     125
   Asp Gly Val Leu Glu Thr Pro Ile Gly Ala Val Gly Val Ala Leu Cys
           130                     135                     140
   Trp Glu Phe Ile Arg Thr Gln Thr Ala Arg Arg Leu Lys Asp Arg Val
   145                     150                     155                     160
   Gln Leu Val Val Gly Gly Thr Cys Trp Trp Asp Phe Pro Met Pro Val
                   165                     170                     175
   Pro Glu Arg Tyr Leu Arg Leu Thr Arg His Ile Ser Arg Asn Phe Glu
               180                     185                     190
   Arg Asp Ala Pro Ala Arg Leu Ala Ser Met Leu Gly Val Pro Val Val
           195                     200                     205
   His Ala Ser His Ala Gly Asp Phe Thr Ala Val Thr Pro Gly Asn Glu
       210                     215                     220
   Thr Lys Asn Tyr Arg Ser Asn Tyr Leu Gly Glu Thr Gln Ile Val Asp
   225                     230                     235                     240
   Ala Asn Gly Asn Val Leu Lys Arg Met Thr Val Ala Asp Gly Glu Gly
                   245                     250                     255
   Tyr Val Ile Ala Asp Val Gln Leu Gly Ala Ile Ser Thr Gly Arg Thr
               260                     265                     270
   Ser Ile Pro Asp Thr Phe Trp Thr Cys Lys Leu Thr Pro Gly Ala Gln
           275                     280                     285
   Gln Ala Trp Asp Glu Gln Asn Ala Phe Gly Cys Gly Tyr Tyr Glu Asn
       290                     295                     300
   Val Thr Arg Lys His Leu Ile Gly Arg
   305                     310
<210> 37
   <211> 993
   <212> DNA
   <213> Unknown
<220>
   <223> Obtained from an environmental sample
<400> 37
   atggattcag atatgacgga tacttttaag gcagccatta ttcagcacgc gcctgttttt      60
   ttaaatctcg aagagagtct ggacaaagcc ggcagcctta tagaaaaggc tgccgatcaa     120
   ggcgcgaaag tgatcgcctt tcctgaaaca tggctgcccg gttatcccgt atggctcgac     180
   tactctccaa aagcgggtct gtgggactat cagcctgcaa aatctctcta tcgtctgcta     240
   gtcgataatt cagtcacctt acccggcaaa cacctcgatc aactcctctc catagcgcaa     300
   aagaccggcg catatgttgt aatgggggca cacgaacgag tgggtggaac actctataac     360
   acgacgatct atgttgggat tgatgggaag gagtacaaac ttcatagaaa gctggtgccg     420
   acctataccg aaagattgat ctgggggcgg ggagacggca gcacattgag tgtgttgatg     480
   acggattatg gcgttcttgg aggattgatc tgctgggagc actggatgcc tctggcaaga     540
   gccgcaatgc atgccagata tgaaaccctt catgtggcgc aatggccggc tgtaaaagat     600
   atccatcaga tagcaagcag acattatgct tttgaaggcc ggtgtttcgt gctcgcggca     660
   ggctctgttc tgactcgaag agatataata gaaggattca actcactggc tcgcgccgat     720
   agtgatgcat tggaacttct gaaagctatt tcgggagaag atagtgatct tattttgaat     780
   gggggaagcg cgataattgc gccgaatgga gagtatcttg cgggcccggt ctttaatgaa     840
   ccctccatta tttatgctga aattgatcct gcactgataa gtgagggcca tcttacactg     900
   gatacaagcg gacactactc gcgccctgac atttttcgtc tggagataaa cgatcaacct     960
   caacatgatg taactttcag atcggggcat tag                                  993
<210> 38
   <211> 330
   <212> PRT
   <213> unknown
<220>
   <223> obtained from an environmental sample
<400> 38
   Met Asp Ser Asp Met Thr Asp Thr Phe Lys Ala Ala Ile Ile Gln His
    1               5                  10                  15
   Ala Pro Val Phe Leu Asn Leu Glu Glu Ser Leu Asp Lys Ala Gly Ser
               20                  25                  30
   Leu Ile Glu Lys Ala Ala Asp Gln Gly Ala Lys Val Ile Ala Phe Pro
           35                  40                  45
   Glu Thr Trp Leu Pro Gly Tyr Pro Val Trp Leu Asp Tyr Ser Pro Lys
       50                  55                  60
   Ala Gly Leu Trp Asp Tyr Gln Pro Ala Lys Ser Leu Tyr Arg Leu Leu
   65                  70                  75                  80
   Val Asp Asn Ser Val Thr Leu Pro Gly Lys His Leu Asp Gln Leu Leu
                   85                  90                  95
   Ser Ile Ala Gln Lys Thr Gly Ala Tyr Val Val Met Gly Ala His Glu
               100                 105                 110
   Arg Val Gly Gly Thr Leu Tyr Asn Thr Thr Ile Tyr Val Gly Ile Asp
           115                 120                 125
   Gly Lys Glu Tyr Lys Leu His Arg Lys Leu Val Pro Thr Tyr Thr Glu
       130                 135                 140
   Arg Leu Ile Trp Gly Arg Gly Asp Gly Ser Thr Leu Ser Val Leu Met
   145                 150                 155                 160
   Thr Asp Tyr Gly Val Leu Gly Gly Leu Ile Cys Trp Glu His Trp Met
                   165                 170                 175
   Pro Leu Ala Arg Ala Ala Met His Ala Arg Tyr Glu Thr Leu His Val
               180                 185                 190
   Ala Gln Trp Pro Ala Val Lys Asp Ile His Gln Ile Ala Ser Arg His
           195                 200                 205
   Tyr Ala Phe Glu Gly Arg Cys Phe Val Leu Ala Ala Gly Ser Val Leu
       210                 215                 220
   Thr Arg Arg Asp Ile Ile Glu Gly Phe Asn Ser Leu Ala Arg Ala Asp
   225                 230                 235                 240
   Ser Asp Ala Leu Glu Leu Leu Lys Ala Ile Ser Gly Glu Asp Ser Asp
                   245                 250                 255
   Leu Ile Leu Asn Gly Gly Ser Ala Ile Ile Ala Pro Asn Gly Glu Tyr
               260                 265                 270
   Leu Ala Gly Pro Val Phe Asn Glu Pro Ser Ile Ile Tyr Ala Glu Ile
           275                 280                 285
   Asp Pro Ala Leu Ile Ser Glu Gly His Leu Thr Leu Asp Thr Ser Gly
       290                 295                 300
   His Tyr Ser Arg Pro Asp Ile Phe Arg Leu Glu Ile Asn Asp Gln Pro
   305                 310                 315                 320
   Gln His Asp Val Thr Phe Arg Ser Gly His
                   325                 330
<210> 39
   <211> 1008
   <212> DNA
   <213> Unknown
<220>
   <223> obtained from an environmental sample
<400> 39
   atgaaaaata tcaaaaactc agaaaaaagc agcacagtaa gagtcgctgc ggtacaaatc      60
   agtccggtgt tgtacaaccg cgaagctacc gttcaaaaag tagtcaacaa aatccttgaa     120
   ctaggaaaac aaggggtaca attcgccact tttccggaaa cgatagtgcc ttattatcct     180
   tatttctctt ttattcaggc gccttatgcc atgggcaaag aacacctgcg cttgcttgaa     240
   caatcagtta ctgttccgtc agccgcgacc gatgccataa gtgaggcggc aaaggaagcc     300
   aatatggtag tgtctattgg tgtcaatgaa cgagacggtg gtaccattta caatacgcaa     360
   ctcctttttg atgctgacgg aacattaatt cagcgcagac gtaaacttac accaacgtat      420
   catgaaagaa tgatttgggg acaaggtgac gcttcaggtc ttcgtgccac agacagcgct     480
   gttgggcgta tcgggcagtt ggcttgttgg gaacattaca atccattgtt ccgttatgct     540
   ttgattgctg atggagaaca aatccattct gccatgtatc ccggatcatt tttaggtgcg     600
   ttgcacggtg aacaaaccga aatcaatgta cgccaacacg ctttagaatc ggccagcttc     660
   gtcgtagtgg ctaccggttg gttggatgcc gatcaacaag cacaaattgc gaaagacacc     720
   ggtggaccaa tcggaccaat ttcgggaggt tgttttacag ccgttatagg ccctgacgga     780
   caactaatcg gggaagccct tacatcaggt gaaggggaag tgattgccga tattgatttg      840
   gcacaaattg atgcccgcaa aagattaatg gatgccagtg gtcactacaa ccgtcctgaa     900
   ttgttgagct tgcatatcga tcacactccg actgctccta tgcatgaaag agtagtttac     960
   actgagccgg gattagcaaa aagacaaaat gaaaattcat caaattaa                 1008
<210> 40
   <211> 335
   <212> PRT
   <213> Unknown
<220>
   <223> Obtained from an environmental sample
<400> 40
   Met Lys Asn Ile Lys Asn Ser Glu Lys Ser Ser Thr Val Arg Val Ala
   1               5                   10                  15
   Ala Val Gln Ile Ser Pro Val Leu Tyr Asn Arg Glu Ala Thr Val Gln
               20                  25                  30
   Lys Val Val Asn Lys Ile Leu Glu Leu Gly Lys Gln Gly Val Gln Phe
           35                  40                  45
   Ala Thr Phe Pro Glu Thr Ile Val Pro Tyr Tyr Pro Tyr Phe Ser Phe
       50                  55                  60
   Ile Gln Ala Pro Tyr Ala Met Gly Lys Glu His Leu Arg Leu Leu Glu
   65                  70                  75                  80
   Gln Ser Val Thr Val Pro Ser Ala Ala Thr Asp Ala Ile Ser Glu Ala
                   85                  90                  95
   Ala Lys Glu Ala Asn Met Val Val Ser Ile Gly Val Asn Glu Arg Asp
               100                 105                 110
   Gly Gly Thr Ile Tyr Asn Thr Gln Leu Leu Phe Asp Ala Asp Gly Thr
           115                 120                 125
   Leu Ile Gln Arg Arg Arg Lys Leu Thr Pro Thr Tyr His Glu Arg Met
       130                 135                 140
   Ile Trp Gly Gln Gly Asp Ala Ser Gly Leu Arg Ala Thr Asp Ser Ala
   145                 150                 155                 160
   Val Gly Arg Ile Gly Gln Leu Ala Cys Trp Glu His Tyr Asn Pro Leu
                   165                 170                 175
   Phe Arg Tyr Ala Leu Ile Ala Asp Gly Glu Gln Ile His Ser Ala Met
               180                 185                 190
   Tyr Pro Gly Ser Phe Leu Gly Ala Leu His Gly Glu Gln Thr Glu Ile
           195                 200                 205
   Asn Val Arg Gln His Ala Leu Glu Ser Ala Ser Phe Val Val Val Ala
       210                 215                 220
   Thr Gly Trp Leu Asp Ala Asp Gln Gln Ala Gln Ile Ala Lys Asp Thr
   225                 230                 235                 240
   Gly Gly Pro Ile Gly Pro Ile Ser Gly Gly Cys Phe Thr Ala Val Ile
                   245                 250                 255
   Gly Pro Asp Gly Gln Leu Ile Gly Glu Ala Leu Thr Ser Gly Glu Gly
               260                 265                 270
   Glu Val Ile Ala Asp Ile Asp Leu Ala Gln Ile Asp Ala Arg Lys Arg
           275                 280                 285
   Leu Met Asp Ala Ser Gly His Tyr Asn Arg Pro Glu Leu Leu Ser Leu
       290                 295                 300
   His Ile Asp His Thr Pro Thr Ala Pro Met His Glu Arg Val Val Tyr
   305                 310                 315                 320
   Thr Glu Pro Gly Leu Ala Lys Arg Gln Asn Glu Asn Ser Ser Asn
                   325                 330                 335
<210> 41
   <211> 966
   <212> DNA
   <213> Unknown
<220>
   <223> Obtained from an environmental sample
<400> 41
   atgcccaatg agaataacat cgctacattc aaagttgccg cagtccaggc cacaccggtg     60
   tttcttgatc gtgaagcaac catcgacaaa gcttgcgggt tgattgccac tgccggcaat    120
   gaaggagcgc gcctgattgt gtttccagaa gcgttcatcc caacctatcc tgaatgggtt    180
   tggggtattc cttccggtga gcaaggttta ctcaatgaac tctatgcaga gctgctcacc    240
   aatgcggtca ctattcccag tgacgcgact gacaggctgt gcgaggccgc gcagcttgcg    300
   aatgcctacg tagtgatggg aatgagcgaa cggaatgtcg aggcgagtgg cgcaagccta    360
   tataatacgc tgttgtacat caatgcgcag ggggagattt tagggaaaca tcgaaagctg     420
   gtgccaacgg gcggcgaacg cctggtatgg gcgcagggtg atggcagcac gctgcaggtc    480
   tacgatacgc cattgggaaa actcggtggt ctcatttgct gggaaaatta tatgccgctg    540
   gcacgctatg ctatgtatgc ctgggggaca caaatctatg tcgcggcaac gtgggatcga    600
   ggccaaccct ggctttctac attacggcat atcgccaaag aaggcagggt atacgtgatt    660
   ggttgctgta tcgcgatgcg aaaagacgat attccggata gttactccat gaagcagaaa    720
   taccatgctg aaatggatga atggattaat gttggcgaca gtgtgattgt caatcccgaa    780
   ggacacttta tcgcagggcc tgtgcgcaag caagaagaaa ttctctacgc ggagatcgat    840
   ccacgtatgg tgcaaggccc gaagtggatg ctcgatgtgg cggggcatta tgcgagacca    900
   gatgtgttcc agttgacggt gcatacgaat gtgagagaga tgatgcgggt ggaagatgat    960
   tcataa                                                               966
<210> 42
   <211> 321
   <212> PRT
   <213> Unknown
<220>
   <223> Obtained from an environmental sample
<400> 42
   Met Pro Asn Glu Asn Asn Ile Ala Thr Phe Lys Val Ala Ala Val Gln
   1               5                   10                  15
   Ala Thr Pro Val Phe Leu Asp Arg Glu Ala Thr Ile Asp Lys Ala Cys
               20                  25                  30
   Gly Leu Ile Ala Thr Ala Gly Asn Glu Gly Ala Arg Leu Ile Val Phe
           35                  40                  45
   Pro Glu Ala Phe Ile Pro Thr Tyr Pro Glu Trp Val Trp Gly Ile Pro
       50                  55                  60
   Ser Gly Glu Gln Gly Leu Leu Asn Glu Leu Tyr Ala Glu Leu Leu Thr
   65                  70                  75                  80
   Asn Ala Val Thr Ile Pro Ser Asp Ala Thr Asp Arg Leu Cys Glu Ala
                   85                  90                  95
   Ala Gln Leu Ala Asn Ala Tyr Val Val Met Gly Met Ser Glu Arg Asn
               100                 105                 110
   Val Glu Ala Ser Gly Ala Ser Leu Tyr Asn Thr Leu Leu Tyr Ile Asn
           115                 120                 125
   Ala Gln Gly Glu Ile Leu Gly Lys His Arg Lys Leu Val Pro Thr Gly
       130                 135                 140
   Gly Glu Arg Leu Val Trp Ala Gln Gly Asp Gly Ser Thr Leu Gln Val
   145                 150                 155                 160
   Tyr Asp Thr Pro Leu Gly Lys Leu Gly Gly Leu Ile Cys Trp Glu Asn
                   165                 170                 175
   Tyr Met Pro Leu Ala Arg Tyr Ala Met Tyr Ala Trp Gly Thr Gln Ile
               180                 185                 190
   Tyr Val Ala Ala Thr Trp Asp Arg Gly Gln Pro Trp Leu Ser Thr Leu
           195                 200                 205
   Arg His Ile Ala Lys Glu Gly Arg Val Tyr Val Ile Gly Cys Cys Ile
       210                 215                 220
   Ala Met Arg Lys Asp Asp Ile Pro Asp Ser Tyr Ser Met Lys Gln Lys
   225                 230                 235                 240
   Tyr His Ala Glu Met Asp Glu Trp Ile Asn Val Gly Asp Ser Val Ile
                   245                 250                 255
   Val Asn Pro Glu Gly His Phe Ile Ala Gly Pro Val Arg Lys Gln Glu
               260                 265                 270
   Glu Ile Leu Tyr Ala Glu Ile Asp Pro Arg Met Val Gln Gly Pro Lys
           275                 280                 285
   Trp Met Leu Asp Val Ala Gly His Tyr Ala Arg Pro Asp Val Phe Gln
       290                 295                 300
   Leu Thr Val His Thr Asn Val Arg Glu Met Met Arg Val Glu Asp Asp
   305                 310                 315                 320
   Ser
<210> 43
   <211> 993
   <212> DNA
   <213> Unknown
<220>
   <223> Obtained from an environmental sample
<400> 43
   atgagagtcg ttaaagccgc ggcggtccaa ctgaaacctg tcctctatag ccgagaggga      60
   actgtcgaaa acgtcgtccg taaaatccac gagcttggac agcaaggggt acagttcgcg     120
   acgtttccag agactgtggt gccttactac ccgtactttt cgatcgtgca gtccggctat     180
   caaattcttg gcggtggtga gttcctgaag ctgcttgatc agtcagtaac cgtgccatct     240
   ctcgctacgg aagcgatcgg cgaggcttgc aggcaggcgg gcgtcgttgt ctccatcggc     300
   gtcaacgagc gtgatggagg aactctatac aacacgcaac ttctctttga tgccgacgga     360
   acattgattc aaagacgacg caagatcaca cccacccatt acgagcgcat ggtctggggc     420
   cagggcgatg gctcaggttt acgggccatt gacagcaagg tcgcgcgcat tggtcaactg     480
   gcgtgttttg agcactacaa ccctctcgca cgttacgcga tgatggccga tggcgagcag     540
   atccattctg cgatgttccc cggctccatg ttcggcgata atttttcaga gaaggtggaa     600
   atcaacataa ggcagcatgc aatggagtct gggtgctttg tcgtttgcgc tactgcctgg     660
   ttggatgctg accagcaggc tcaaatcatg aaagacacgg gatgtgagat cggaccgatc     720
   tcaggaggtt gcttcacagc gatcgcggca ccagatggaa gccttatagg tgaacccatc     780
   cgctcaggtg aaggcgtttg tattgccgac ctcgatttca aacttatcga caagcggaag     840
   cacgtagtag acacacgcgg ccattatagc cggccagaat tgctcagcct cctgattgat     900
   cggacgccga cggcccacat acacgaaagg accgagcaac cgagggcggc catcgagaaa     960
   gagtcgcagg atgttttcac cgctgttgct taa                                  993
<210> 44
   <211> 330
   <212> PRT
   <213> Unknown
<220>
   <223> Obtained from an environmental sample
<400> 44
   Met Arg Val Val Lys Ala Ala Ala Val Gln Leu Lys Pro Val Leu Tyr
   1               5                   10                  15
   Ser Arg Glu Gly Thr Val Glu Asn Val Val Arg Lys Ile His Glu Leu
               20                  25                  30
   Gly Gln Gln Gly Val Gln Phe Ala Thr Phe Pro Glu Thr Val Val Pro
           35                  40                  45
   Tyr Tyr Pro Tyr Phe Ser Ile Val Gln Ser Gly Tyr Gln Ile Leu Gly
       50                  55                  60
   Gly Gly Glu Phe Leu Lys Leu Leu Asp Gln Ser Val Thr Val Pro Ser
   65                  70                  75                  80
   Leu Ala Thr Glu Ala Ile Gly Glu Ala Cys Arg Gln Ala Gly Val Val
                   85                  90                  95
   Val Ser Ile Gly Val Asn Glu Arg Asp Gly Gly Thr Leu Tyr Asn Thr
               100                 105                 110
   Gln Leu Leu Phe Asp Ala Asp Gly Thr Leu Ile Gln Arg Arg Arg Lys
           115                 120                 125
   Ile Thr Pro Thr His Tyr Glu Arg Met Val Trp Gly Gln Gly Asp Gly
       130                 135                 140
   Ser Gly Leu Arg Ala Ile Asp Ser Lys Val Ala Arg Ile Gly Gln Leu
   145                 150                 155                 160
   Ala Cys Phe Glu His Tyr Asn Pro Leu Ala Arg Tyr Ala Met Met Ala
                   165                 170                 175
   Asp Gly Glu Gln Ile His Ser Ala Met Phe Pro Gly Ser Met Phe Gly
               180                 185                 190
   Asp Asn Phe Ser Glu Lys Val Glu Ile Asn Ile Arg Gln His Ala Met
           195                 200                 205
   Glu Ser Gly Cys Phe Val Val Cys Ala Thr Ala Trp Leu Asp Ala Asp
       210                 215                 220
   Gln Gln Ala Gln Ile Met Lys Asp Thr Gly Cys Glu Ile Gly Pro Ile
   225                 230                 235                 240
   Ser Gly Gly Cys Phe Thr Ala Ile Ala Ala Pro Asp Gly Ser Leu Ile
                   245                 250                 255
   Gly Glu Pro Ile Arg Ser Gly Glu Gly Val Cys Ile Ala Asp Leu Asp
               260                 265                 270
   Phe Lys Leu Ile Asp Lys Arg Lys His Val Val Asp Thr Arg Gly His
           275                 280                 285
   Tyr Ser Arg Pro Glu Leu Leu Ser Leu Leu Ile Asp Arg Thr Pro Thr
       290                 295                 300
   Ala His Ile His Glu Arg Thr Glu Gln Pro Arg Ala Ala Ile Glu Lys
   305                 310                 315                 320
   Glu Ser Gln Asp Val Phe Thr Ala Val Ala
                   325                 330
<211> 996
   <212> DNA
   <213> Unknown
<220>
   <223> Obtained from an environmental sample
<400> 45
<210> 46
   <211> 331
   <212> PRT
   <213> Unknown
<220>
   <223> Obtained from an environmental sample
<400> 46
   Val Lys Pro Pro Thr Ser Phe Arg Val Ala Ala Val Gln Ala Cys Pro
   1 5 10 15
   Val Tyr Leu Asp Arg Asp Leu Thr Ile Gly Lys Ala Glu Gly Leu Ile
   20 25 30
   Ala Glu Ala Ala Gly Asn Gly Ala Asn Leu Ile Val Phe Pro Glu Ala
   35 40 45
   Phe Val Pro Gly Tyr Pro Val Trp Val Trp Phe Ile Pro Pro Gly Arg
   50 55 60
   Thr Ala Asp Leu Arg Glu Ala Tyr Ser Val Leu His Ala Asn Ser Ile
   65 70 75 80
   Ala Val Pro Ser Gln Ser Thr Glu Arg Leu Cys Ala Ala Ala Arg Arg
   85 90 95
   Ala Gly Val Ala Val Ala Ile Gly Val Asn Glu Arg Asn Ser Glu Ala
   100 105 110
   Ser Gly Gly Ser Leu Phe Asn Thr Leu Leu Tyr Ile Gly Pro Asp Gly
   115 120 125
   Thr Leu Leu Gly Lys His Arg Lys Leu Val Pro Thr Gly Gly Glu Arg
   130 135 140
   Leu Val Trp Ala Ser Gly Asp Gly Ser Asp Leu Ala Val Phe Thr Leu
   145 150 155 160
   Pro Phe Ala Arg Val Gly Gly Leu Ile Cys Trp Glu Asn Tyr Met Pro
   165 170 175
   Leu Ala Arg Tyr Ala Leu Ala Ala Trp Gly Ala Gln Ile His Val Ala
   180 185 190
   Pro Thr Trp Asp Arg Gly Glu Pro Trp Leu Ser Thr Leu Arg His Val
   195 200 205
   Ala Lys Glu Gly Arg Ala Val Thr Ile Gly Cys Cys Gln Ala Val Arg
   210 215 220
   Lys Glu Asp Ile Pro Asp Gly Leu Ala Phe Lys Ser Arg Tyr Leu Ala
   225 230 235 240
   Asp Val Gly Ala Trp Val Asn Pro Gly Gly Ser Val Ile Val Asp Pro
   245 250 255
   Asp Gly Lys Ile Leu Ala Gly Pro Ala Asn Glu Thr Glu Gly Ile Leu
   260 265 270
   Tyr Ala Asp Ile Arg Ala Asp Gln Leu Val Gly Pro Arg Trp Gln Leu
           275                 280                 285
   Asp Ile Ala Gly His Tyr Ala Arg Pro Asp Val Phe Glu Leu Ile Val
       290                 295                 300
   His Arg Arg Ser Thr Pro Met Ile Arg Glu Val Ser Ala Pro Arg Arg
   305                 310                 315                     320
   Arg Ala Arg Thr Gly Lys Arg Pro Arg Arg Arg
                   325                 330
<210> 47
   <211> 1014
   <212> DNA
   <213> Unknown
<220>
   <223> Obtained from an environmental sample
<400> 47
   gtgaaagaag caatcaaagt agcctgtgtg caagcagctc cagtctttct cgacctggac      60
   gccacagtgg acaagaccgt cgccctgatt gaggaggcag cccgtaacgg cgcacgccta     120
   atcgcctttc cagagacctg gattccaggc tacccatggt tcctttggct ggactcacca     180
   gcctggggga tgcaattcgt gcgccgatac cacgagaact cactggtcct cgacagccct     240
   caggccaagc gcatcagtga ggccgcccag cgcgccggta tatacgtcgc gctagggtac     300
   agcgaacgcg tgagcggaac cctctacatg gggcagtggc tcattgacga taagggcgaa     360
   acagctgggc tgcgccgaaa gctgaaacca acccatgtag agcgaaccct cttcggtgaa     420
   ggcgacggat catccctttc cactttcgac acaccgttgg gggtgctggg cggactctgc     480
   tgttgggaac acttacaacc tctttcgaaa tatgcgctct acgcacagaa cgaggaaata     540
   cacttcgccg cctggcctag cttcagcatc taccgtcaag cgacagaagt ccttggacca     600
   gaagtaaatg tcgcagcttc tcggatctac gccgtggaag ggcagtgttt tgttctcgct     660
   tcctgcgcgc tcgtctcgcc agagatgatc gaaatgctct gcactgacga aagcaagcac     720
   agccttcttc aggccggcgg cgggtactcc cgcattatcg gtcccgatgg cagcgaccta     780
   gcgcgcccct tgggcgaaaa cgaggaaggt attctctatg ccactctgga ccctgccgct     840
   cgaatctatg caaagaccgc agctgatcca gccgggcact actccagacc agacgtcact     900
   cggctgctga tcaatcgcag tgccaatcag ccagtcgtag aggttggaag ggaaatacct     960
   gcatcggccc aaggctttga agttgaggcg gcccccgggt acgaaggcga ttga          1014
<210> 48
   <211> 337
   <212> PRT
   <213> Unknown
<220>
   <223> Obtained from an environmental sample
<400> 48
   Val Lys Glu Ala Ile Lys Val Ala Cys Val Gln Ala Ala Pro Val Phe
    1               5                  10                  15
   Leu Asp Leu Asp Ala Thr Val Asp Lys Thr Val Ala Leu Ile Glu Glu
               20                  25                  30
   Ala Ala Arg Asn Gly Ala Arg Leu Ile Ala Phe Pro Glu Thr Trp Ile
           35                  40                  45
   Pro Gly Tyr Pro Trp Phe Leu Trp Leu Asp Ser Pro Ala Trp Gly Met
       50                  55                  60
   Gln Phe Val Arg Arg Tyr His Glu Asn Ser Leu Val Leu Asp Ser Pro
   65                  70                  75                  80
   Gln Ala Lys Arg Ile Ser Glu Ala Ala Gln Arg Ala Gly Ile Tyr Val
                   85                  90                  95
   Ala Leu Gly Tyr Ser Glu Arg Val Ser Gly Thr Leu Tyr Met Gly Gln
               100                 105                 110
   Trp Leu Ile Asp Asp Lys Gly Glu Thr Ala Gly Leu Arg Arg Lys Leu
           115                 120                 125
   Lys Pro Thr His Val Glu Arg Thr Leu Phe Gly Glu Gly Asp Gly Ser
       130                 135                 140
   Ser Leu Ser Thr Phe Asp Thr Pro Leu Gly Val Leu Gly Gly Leu Cys
   145                 150                 155                 160
   Cys Trp Glu His Leu Gln Pro Leu Ser Lys Tyr Ala Leu Tyr Ala Gln
                   165                 170                 175
   Asn Glu Glu Ile His Phe Ala Ala Trp Pro Ser Phe Ser Ile Tyr Arg
               180                 185                 190
   Gln Ala Thr Glu Val Leu Gly Pro Glu Val Asn Val Ala Ala Ser Arg
           195                 200                 205
   Ile Tyr Ala Val Glu Gly Gln Cys Phe Val Leu Ala Ser Cys Ala Leu
       210                 215                 220
   Val Ser Pro Glu Met Ile Glu Met Leu Cys Thr Asp Glu Ser Lys His
   225                 230                 235                 240
   Ser Leu Leu Gln Ala Gly Gly Gly Tyr Ser Arg Ile Ile Gly Pro Asp
                   245                 250                 255
   Gly Ser Asp Leu Ala Arg Pro Leu Gly Glu Asn Glu Glu Gly Ile Leu
               260                 265                 270
   Tyr Ala Thr Leu Asp Pro Ala Ala Arg Ile Tyr Ala Lys Thr Ala Ala
           275                 280                 285
   Asp Pro Ala Gly His Tyr Ser Arg Pro Asp Val Thr Arg Leu Leu Ile
       290                 295                 300
   Asn Arg Ser Ala Asn Gln Pro Val Val Glu Val Gly Arg Glu Ile Pro
   305                 310                 315                 320
   Ala Ser Ala Gln Gly Phe Glu Val Glu Ala Ala Pro Gly Tyr Glu Gly
                   325                 330                 335
   Asp
<210> 49
   <211> 1038
   <212> DNA
   <213> Unknown
<220>
   <223> Obtained from an environmental sample
<400> 49
   atgaacaaag tcgtggctgc tgccgttcag tgcagcccgg tgctttactc ttgcgccgga     60
   actgtaaata aaatttgcga gtggattgca gatttgggca aacaaggggt tgagctggcg    120
   gtgttcgcgg aaaccctggt gccttactac ccgtattttt cttttatcca ggctccttgt    180
   gcgatgggcg cgcaacattt gttgttgatg caagaatcag tagaggttcc ttccatctac    240
   acgcaacaaa ttgccgctgc agcaaaagca gcgaagatgg tggtgtcagt tggtattaac    300
   gaacgcgacg gcggttctat ttataacgcg caattattat ttgatgcggg cggtcagctt    360
   gttcagcacc gccgaaaaat tacgccgaca tttcatgagc gcatggtgtg ggggcagggc     420
   gatggctccg gtttgtgcgc agtggatacg gcagttggtc gtgttggttc gctcgcttgc    480
   tgggaacatt acaacccact cgcgcgttac gcattgatgg cagatcgcga acaaattcac    540
   gtgagtatgt ttcccggttc tttggtcggc gaaatttttg ccgagcaaat tgaagcaact    600
   attcgtcacc acgcattgga gtccggttgc tttgtggtaa atgcgacggg ctggttaacg    660
   ccggaacagc aagctcaaat cgtaaaagat actggtggtc ctatcgctgc cattagcggt    720
   ggttgtttca ccgccattgt ttcaccggaa ggaaaattgc tcggcacgcc attgcgcagt    780
   gattccgggg agggtgcctg tatcgccgaa ctggatttta atctcatcaa taagcgtaag    840
   cgcatgatgg attctgtcgg ccattacagt cgtcctgaat tgctcagttt gctgattgat    900
   aaaacaccga caagtcatac acatccgctt aaaaaacctt tggctcccag tgaaaaaaat    960
   acgccagagg atatcgccac tggtttaaca ctggtcactc ccgtttcaaa tgcaaacctt   1020
   ttcagcgcaa gcaactag                                                 1038
<210> 50
   <211> 345
   <212> PRT
   <213> Unknown
<220>
   <223> Obtained from an environmental sample
<400> 50
   Met Asn Lys Val Val Ala Ala Ala Val Gln Cys Ser Pro Val Leu Tyr
    1               5                  10                  15
   Ser Cys Ala Gly Thr Val Asn Lys Ile Cys Glu Trp Ile Ala Asp Leu
               20                  25                  30
   Gly Lys Gln Gly Val Glu Leu Ala Val Phe Ala Glu Thr Leu Val Pro
           35                  40                  45
   Tyr Tyr Pro Tyr Phe Ser Phe Ile Gln Ala Pro Cys Ala Met Gly Ala
       50                  55                  60
   Gln His Leu Leu Leu Met Gln Glu Ser Val Glu Val Pro Ser Ile Tyr
   65                  70                  75                  80
   Thr Gln Gln Ile Ala Ala Ala Ala Lys Ala Ala Lys Met Val Val Ser
                           85                  90                  95
   Val Gly Ile Asn Glu Arg Asp Gly Gly Ser Ile Tyr Asn Ala Gln Leu
               100                 105                 110
   Leu Phe Asp Ala Gly Gly Gln Leu Val Gln His Arg Arg Lys Ile Thr
           115                 120                 125
   Pro Thr Phe His Glu Arg Met Val Trp Gly Gln Gly Asp Gly Ser Gly
       130                 135                 140
   Leu Cys Ala Val Asp Thr Ala Val Gly Arg Val Gly Ser Leu Ala Cys
   145                 150                 155                 160
   Trp Glu His Tyr Asn Pro Leu Ala Arg Tyr Ala Leu Met Ala Asp Arg
                   165                 170                 175
   Glu Gln Ile His Val Ser Met Phe Pro Gly Ser Leu Val Gly Glu Ile
               180                 185                 190
   Phe Ala Glu Gln Ile Glu Ala Thr Ile Arg His His Ala Leu Glu Ser
           195                 200                 205
   Gly Cys Phe Val Val Asn Ala Thr Gly Trp Leu Thr Pro Glu Gln Gln
       210                 215                 220
   Ala Gln Ile Val Lys Asp Thr Gly Gly Pro Ile Ala Ala Ile Ser Gly
   225                 230                 235                 240
   Gly Cys Phe Thr Ala Ile Val Ser Pro Glu Gly Lys Leu Leu Gly Thr
                   245                 250                 255
   Pro Leu Arg Ser Asp Ser Gly Glu Gly Ala Cys Ile Ala Glu Leu Asp
               260                 265                 270
   Phe Asn Leu Ile Asn Lys Arg Lys Arg Met Met Asp Ser Val Gly His
           275                 280                 285
   Tyr Ser Arg Pro Glu Leu Leu Ser Leu Leu Ile Asp Lys Thr Pro Thr
       290                 295                 300
   Ser His Thr His Pro Leu Lys Lys Pro Leu Ala Pro Ser Glu Lys Asn
   305                 310                 315                 320
   Thr Pro Glu Asp Ile Ala Thr Gly Leu Thr Leu Val Thr Pro Val Ser
                   325                 330                 335
   Asn Ala Asn Leu Phe Ser Ala Ser Asn
               340                 345
<210> 51
   <211> 897
   <212> DNA
   <213> Unknown
<220>
   <223> Obtained from an environmental sample
<400> 51
   gtgaacgtcc gcgtcgcggt ggtgcaggcc acgccggccg tgctcgacgg gccggcgtcg      60
   gtgcggaagg cctgccgcct gatcggcgag gccgcggccg gcggcgcccg cctgatcgcc     120
   ctgcccgagg gcttcgtgcc catcatgccg cgctcctgct gggggcacca cttcgcgctg     180
   atcgcctcgc cgaagtcggc ggccctgcac cggcgcatct gggagaacgc cgtcgacgtc     240
   ggcggcccgc tggcccgcga gctcggcgac gccgcgcgcc gcgcggacgc ctgggtggcc     300
   atcggggtga acgagcgcga cgcccgccgg ccgggcacgc tctggaacac gctgctctgg     360
   ttcgcgcccg acgggagcct ggcccggcgc caccgcaagc tcgtgcccac catgcacgag     420
   cgcacgttct gggggcaggg cgcgggcgac gacctcgagg cgctggccgc ggacttcggc     480
   cgcctgggcg gcctgatctg ctgggagaac ttcatgcccg ccgcgcgccg gcgcctgcac     540
   cgggacgggg tcgacttcta cctggccccc acggcggacg accgggacat ctgggtcgcc     600
   gcgatgcgca cgttcgcctt cgaggccggc gccttcgtcc tctcgccggt gcagtacctg     660
   cggaccgccg acttcccgga ggacttcccg ctgcgcgagg agctcgccga ctgccccgag     720
   gtccagttca ccggggggag cgtgatctgc gacccgtggg gcaacctcct ggcggggccg     780
   gtccacgggg gcgaggagat cctctacgcc gactgcgatc tcgacctcgt cctcgaggcc     840
   cgacgggtgc tcgacacggc cggccactac gaccgcccgg acctcgcctc ggcctga         897
<210> 52
   <211> 298
   <212> PRT
   <213> Unknown
<220>
   <223> obtained from an environmental sample
<400> 52
   Val Asn Val Arg Val Ala Val Val Gln Ala Thr Pro Ala Val Leu Asp
   1               5                   10                  15
   Gly Pro Ala Ser Val Arg Lys Ala Cys Arg Leu Ile Gly Glu Ala Ala
               20                  25                  30
   Ala Gly Gly Ala Arg Leu Ile Ala Leu Pro Glu Gly Phe Val Pro Ile
           35                  40                  45
   Met Pro Arg Ser Cys Trp Gly His His Phe Ala Leu Ile Ala Ser Pro
       50                  55                  60
   Lys Ser Ala Ala Leu His Arg Arg Ile Trp Glu Asn Ala Val Asp Val
   65                  70                  75                  80
   Gly Gly Pro Leu Ala Arg Glu Leu Gly Asp Ala Ala Arg Arg Ala Asp
                   85                  90                  95
   Ala Trp Val Ala Ile Gly Val Asn Glu Arg Asp Ala Arg Arg Pro Gly
               100                 105                 110
   Thr Leu Trp Asn Thr Leu Leu Trp Phe Ala Pro Asp Gly Ser Leu Ala
           115                 120                 125
   Arg Arg His Arg Lys Leu Val Pro Thr Met His Glu Arg Thr Phe Trp
       130                 135                 140
   Gly Gln Gly Ala Gly Asp Asp Leu Glu Ala Leu Ala Ala Asp Phe Gly
   145                 150                 155                 160
   Arg Leu Gly Gly Leu Ile Cys Trp Glu Asn Phe Met Pro Ala Ala Arg
                   165                 170                 175
   Arg Arg Leu His Arg Asp Gly Val Asp Phe Tyr Leu Ala Pro Thr Ala
               180                 185                 190
   Asp Asp Arg Asp Ile Trp Val Ala Ala Met Arg Thr Phe Ala Phe Glu
           195                 200                 205
   Ala Gly Ala Phe Val Leu Ser Pro Val Gln Tyr Leu Arg Thr Ala Asp
       210                 215                 220
   Phe Pro Glu Asp Phe Pro Leu Arg Glu Glu Leu Ala Asp Cys Pro Glu
   225                 230                 235                 240
   Val Gln Phe Thr Gly Gly Ser Val Ile Cys Asp Pro Trp Gly Asn Leu
                   245                 250                 255
   Leu Ala Gly Pro Val His Gly Gly Glu Glu Ile Leu Tyr Ala Asp Cys
               260                 265                 270
   Asp Leu Asp Leu Val Leu Glu Ala Arg Arg Val Leu Asp Thr Ala Gly
           275                 280                 285
   His Tyr Asp Arg Pro Asp Leu Ala Ser Ala
       290                 295
<210> 53
   <211> 954
   <212> DNA
   <213> Unknown
<220>
   <223> Obtained from an environmental sample
<400> 53
   atggtcaatt caaagagtca gattaaaatc gcggcggtgc aggctgcccc ggtctttctg       60
   gaacgggagg cgacgattga caaagcttgc cggttgattg cggaggcagg cgagcagggc      120
   gcgaatctgg tggtcttccc tgagtcattc gtcccggctt atcccgattg ggtctgggcc      180
   gttccggcag gtgaaacaac gctcctgaac acgctctatg ccgaactgct ggccaatgcc      240
   gttgaaattc cgggtccggc gacagagcgg ctgagccagg cagccaacct ggccggggtt      300
   tatgtcgcga ttggcttgac cgagcggaac atcgaggcca gtggggcgag cctgtacaat      360
   actttgctct ttctcgactc agccggcggc atgttaggca agcatcgcaa actgatcccc      420
   accggcggcg agcgcctggt ctgggctcag ggtgatggca gcactctggc ggtgtacgag      480
   actaggtttg gaaaaatggg agggttgatt tgctgggaga attacatgcc cctggcccgt      540
   tatgccttgt atgcctgggg gacgcagatt tacatcgcgg ccacctggga tcgaggcgag      600
   ccgtggctgt caacgctgcg gcatatcgcc gcggaaggcc gggttgttgt cgtcggctgt      660
   ggcatggccc tgcgcaaagc cgacctgccc gaccgctttg aactcaagca gcgattttac      720
   cagaacgccg atgagtggat caatgtcggc gacagcgcga ttgttaaccc tgatggtgaa      780
   ttcatcgccg ggccgctgcg cgagcaggaa ggcatcctct atgctgagat tgatctggcc      840
   cagatgcgcg gccccaaatg gatgctcgac gtggccggcc attacgctcg cccggatgtg      900
   tttgaactca tcgttcatcg ggaggcgcgg cccatgattg cgctaatttc atga            954
<210> 54
   <211> 317
   <212> PRT
   <213> Unknown
<220>
   <223> Obtained from an environmental sample
<400> 54
   Met Val Asn Ser Lys Ser Gln Ile Lys Ile Ala Ala Val Gln Ala Ala
   1               5                   10                  15
   Pro Val Phe Leu Glu Arg Glu Ala Thr Ile Asp Lys Ala Cys Arg Leu
               20                  25                  30
   Ile Ala Glu Ala Gly Glu Gln Gly Ala Asn Leu Val Val Phe Pro Glu
           35                  40                  45
   Ser Phe Val Pro Ala Tyr Pro Asp Trp Val Trp Ala Val Pro Ala Gly
       50                  55                  60
   Glu Thr Thr Leu Leu Asn Thr Leu Tyr Ala Glu Leu Leu Ala Asn Ala
   65                  70                  75                  80
   Val Glu Ile Pro Gly Pro Ala Thr Glu Arg Leu Ser Gln Ala Ala Asn
                   85                  90                  95
   Leu Ala Gly Val Tyr Val Ala Ile Gly Leu Thr Glu Arg Asn Ile Glu
               100                 105                 110
   Ala Ser Gly Ala Ser Leu Tyr Asn Thr Leu Leu Phe Leu Asp Ser Ala
           115                 120                 125
   Gly Gly Met Leu Gly Lys His Arg Lys Leu Ile Pro Thr Gly Gly Glu
       130                 135                 140
   Arg Leu Val Trp Ala Gln Gly Asp Gly Ser Thr Leu Ala Val Tyr Glu
   145                 150                 155                 160
   Thr Arg Phe Gly Lys Met Gly Gly Leu Ile Cys Trp Glu Asn Tyr Met
                   165                 170                 175
   Pro Leu Ala Arg Tyr Ala Leu Tyr Ala Trp Gly Thr Gln Ile Tyr Ile
               180                 185                 190
   Ala Ala Thr Trp Asp Arg Gly Glu Pro Trp Leu Ser Thr Leu Arg His
           195                 200                 205
   Ile Ala Ala Glu Gly Arg Val Val Val Val Gly Cys Gly Met Ala Leu
       210                 215                 220
   Arg Lys Ala Asp Leu Pro Asp Arg Phe Glu Leu Lys Gln Arg Phe Tyr
   225                 230                 235                 240
   Gln Asn Ala Asp Glu Trp Ile Asn Val Gly Asp Ser Ala Ile Val Asn
                   245                 250                 255
   Pro Asp Gly Glu Phe Ile Ala Gly Pro Leu Arg Glu Gln Glu Gly Ile
               260                 265                 270
   Leu Tyr Ala Glu Ile Asp Leu Ala Gln Met Arg Gly Pro Lys Trp Met
           275                 280                 285
   Leu Asp Val Ala Gly His Tyr Ala Arg Pro Asp Val Phe Glu Leu Ile
       290                 295                 300
   Val His Arg Glu Ala Arg Pro Met Ile Ala Leu Ile Ser
   305                 310                 315
<210> 55
   <211> 1017
   <212> DNA
   <213> Unknown
<220>
   <223> Obtained from an environmental sample
<400> 55
   atgggtatcg aacatccgaa gtacaaggtc gccgtggtgc aggcagctcc cgcctggctc      60
   gatctcgacg cgtcgatcga caagtcgatc gcgctgatcg aggaggcggc ccaaaaaggc     120
   gccaagctga tcgcattccc cgaggccttc attcccggct atccctggca catctggatg     180
   gactcgccgg cctgggcgat cggccgcggc tttgtgcagc gctattttga caattcgctc     240
   gcctatgaca gcccgcaggc cgagaagctg cgcgcggcgg tgcgcaaggc aaagctcacc     300
   gccgtgctcg ggctgtccga gcgcgacggc ggcagtctct atctggcgca atggttgatc     360
   gggcccgatg gcgagaccat cgcaaaacgc cgcaagctgc ggccgacaca tgccgagcgc     420
   acggtgtacg gcgagggcga cggcagcgat ctcgcagtcc acaaccgtcc cgatatcggc     480
   cgcctcggcg cgctctgctg ctgggagcat ttgcagccac tgtcgaaata cgcgatgtac     540
   gcgcagaacg agcaggtgca tgtcgcggcc tggccgagct tttcgctcta cgatcccttt     600
   gcggtggcgc tcggcgccga ggtgaacaac gcggcctcgc gcgtctatgc agtcgaaggc     660
   tcctgcttcg tgctggcgcc atgcgccacc gtctcgcagg ccatgatcga cgagctctgc     720
   gaccgaccgg acaagcatac gctgctgcat gtcggcggcg gttttgccgc gatctatggt     780
   cctgacggca gccagatcgg cgacaagctc gcgcccgacc aggaagggct gttgatcgcg     840
   gagatcgacc ttggggccat tggcgtcgcc aagaacgcgg ccgatcccgc cgggcattat     900
   tcgcggcccg acgtgacgcg gctcctgctc aacaagaaac cgtacaagcg cgtcgagcag     960
   ttctcgccac cggccgaggc ggtcgagccc acagatatcg cagcggcggc aagctga       1017
<210> 56
   <211> 338
   <212> PRT
   <213> Unknown
<220>
   <223> Obtained from an environmental sample
<400> 56
   Met Gly Ile Glu His Pro Lys Tyr Lys Val Ala Val Val Gln Ala Ala
   1               5                   10                  15
   Pro Ala Trp Leu Asp Leu Asp Ala Ser Ile Asp Lys Ser Ile Ala Leu
               20                  25                  30
   Ile Glu Glu Ala Ala Gln Lys Gly Ala Lys Leu Ile Ala Phe Pro Glu
           35                  40                  45
   Ala Phe Ile Pro Gly Tyr Pro Trp His Ile Trp Met Asp Ser Pro Ala
       50                  55                  60
   Trp Ala Ile Gly Arg Gly Phe Val Gln Arg Tyr Phe Asp Asn Ser Leu
   65                  70                  75                  80
   Ala Tyr Asp Ser Pro Gln Ala Glu Lys Leu Arg Ala Ala Val Arg Lys
                   85                  90                  95
   Ala Lys Leu Thr Ala Val Leu Gly Leu Ser Glu Arg Asp Gly Gly Ser
               100                 105                 110
   Leu Tyr Leu Ala Gln Trp Leu Ile Gly Pro Asp Gly Glu Thr Ile Ala
           115                 120                 125
   Lys Arg Arg Lys Leu Arg Pro Thr His Ala Glu Arg Thr Val Tyr Gly
       130                 135                 140
   Glu Gly Asp Gly Ser Asp Leu Ala Val His Asn Arg Pro Asp Ile Gly
   145                 150                 155                 160
   Arg Leu Gly Ala Leu Cys Cys Trp Glu His Leu Gln Pro Leu Ser Lys
                   165                 170                 175
   Tyr Ala Met Tyr Ala Gln Asn Glu Gln Val His Val Ala Ala Trp Pro
               180                 185                 190
   Ser Phe Ser Leu Tyr Asp Pro Phe Ala Val Ala Leu Gly Ala Glu Val
           195                 200                 205
   Asn Asn Ala Ala Ser Arg Val Tyr Ala Val Glu Gly Ser Cys Phe Val
       210                 215                 220
   Leu Ala Pro Cys Ala Thr Val Ser Gln Ala Met Ile Asp Glu Leu Cys
   225                 230                 235                 240
   Asp Arg Pro Asp Lys His Thr Leu Leu His Val Gly Gly Gly Phe Ala
                   245                 250                 255
   Ala Ile Tyr Gly Pro Asp Gly Ser Gln Ile Gly Asp Lys Leu Ala Pro
               260                 265                 270
   Asp Gln Glu Gly Leu Leu Ile Ala Glu Ile Asp Leu Gly Ala Ile Gly
           275                 280                 285
   Val Ala Lys Asn Ala Ala Asp Pro Ala Gly His Tyr Ser Arg Pro Asp
       290                 295                 300
   Val Thr Arg Leu Leu Leu Asn Lys Lys Pro Tyr Lys Arg Val Glu Gln
   305                 310                 315                 320
   Phe Ser Pro Pro Ala Glu Ala Val Glu Pro Thr Asp Ile Ala Ala Ala
                   325                 330                 335
   Ala Ser
<210> 57
   <211> 1014
   <212> DNA
   <213> Unknown
<220>
   <223> Obtained from an environmental sample
<400> 57
   gtgaaagaag caatcaaagt agcctgtgtg caagcagctc cagtctttct cgacctggac      60
   gccacagtgg acaagaccgt cgccctgatt gaggaggcag cccgtaacgg cgcacgccta     120
   atcgcctttc cagagacctg gattccaggc tacccatggt tcctttggct ggactcacca     180
   gcctggggga tgcaattcgt gcgccgatac cacgagaact cactggtcct cgacagccct     240
   caggccaagc gcatcagtga ggccgcccag cgcgccggta tatacgtcgc gctagggtac     300
   agcgaacgcg tgagcggaac cctctacatg gggcagtggc tcattgacga taagggcgaa     360
   acagctgggc tgcgccgaaa gctgaaacca acccatgtag agcgaaccct cttcggtgaa     420
   ggcgacggat catccctttc cactttcgac acaccgttgg gggtgctggg cggactctgc     480
   tgttgggaac acttacaacc tctttcgaaa tatgcgctct acgcacagaa cgaggaaata     540
   cacttcgccg cctggcctag cttcagcatc taccgtcaag cgacagaagt ccttggacca     600
   gaagtaaatg tcgcagcttc tcggatctac gccgtggaag ggcagtgttt tgttctcgct     660
   tcctgcgcgc tcgtctcgcc agagatgatc gaaatgctct gcactgacga aagcaagcac     720
   agccttcttc aggccggcgg cgggtactcc cgcattatcg gtcccgatgg cagcgaccta     780
   gcgcgcccct tgggcgaaaa cgaggaaggt attctctatg ccactctgga ccctgccgct     840
   cgaatctatg caaagaccgc agctgatcca gccgggcact actccagacc agacgtcact     900
   cggctgctga tcaatcgcag tgccaatcag ccagtcgtag aggttggacg ggaaatacct     960
   gcatcggccc aaggctttga agttgaggcg gcccccgggt acggaggcga ttga          1014
<210> 58
   <211> 337
   <212> PRT
   <213> Unknown
<220>
   <223> Obtained from an environmental sample
<400> 58
   Val Lys Glu Ala Ile Lys Val Ala Cys Val Gln Ala Ala Pro Val Phe
   1               5                   10                  15
   Leu Asp Leu Asp Ala Thr Val Asp Lys Thr Val Ala Leu Ile Glu Glu
               20                  25                  30
   Ala Ala Arg Asn Gly Ala Arg Leu Ile Ala Phe Pro Glu Thr Trp Ile
           35                  40                  45
   Pro Gly Tyr Pro Trp Phe Leu Trp Leu Asp Ser Pro Ala Trp Gly Met
       50                  55                  60
   Gln Phe Val Arg Arg Tyr His Glu Asn Ser Leu Val Leu Asp Ser Pro
   65                  70                  75                  80
   Gln Ala Lys Arg Ile Ser Glu Ala Ala Gln Arg Ala Gly Ile Tyr Val
                   85                  90                  95
   Ala Leu Gly Tyr Ser Glu Arg Val Ser Gly Thr Leu Tyr Met Gly Gln
               100                 105                 110
   Trp Leu Ile Asp Asp Lys Gly Glu Thr Ala Gly Leu Arg Arg Lys Leu
           115                 120                 125
   Lys Pro Thr His Val Glu Arg Thr Leu Phe Gly Glu Gly Asp Gly Ser
       130                 135                 140
   Ser Leu Ser Thr Phe Asp Thr Pro Leu Gly Val Leu Gly Gly Leu Cys
   145                 150                 155                 160
   Cys Trp Glu His Leu Gln Pro Leu Ser Lys Tyr Ala Leu Tyr Ala Gln
                   165                 170                 175
   Asn Glu Glu Ile His Phe Ala Ala Trp Pro Ser Phe Ser Ile Tyr Arg
               180                 185                 190
   Gln Ala Thr Glu Val Leu Gly Pro Glu Val Asn Val Ala Ala Ser Arg
           195                 200                 205
   Ile Tyr Ala Val Glu Gly Gln Cys Phe Val Leu Ala Ser Cys Ala Leu
       210                 215                 220
   Val Ser Pro Glu Met Ile Glu Met Leu Cys Thr Asp Glu Ser Lys His
   225                 230                 235                 240
   Ser Leu Leu Gln Ala Gly Gly Gly Tyr Ser Arg Ile Ile Gly Pro Asp
                   245                 250                 255
   Gly Ser Asp Leu Ala Arg Pro Leu Gly Glu Asn Glu Glu Gly Ile Leu
               260                 265                 270
   Tyr Ala Thr Leu Asp Pro Ala Ala Arg Ile Tyr Ala Lys Thr Ala Ala
           275                 280                 285
   Asp Pro Ala Gly His Tyr Ser Arg Pro Asp Val Thr Arg Leu Leu Ile
       290                 295                 300
   Asn Arg Ser Ala Asn Gln Pro Val Val Glu Val Gly Arg Glu Ile Pro
   305                 310                 315                 320
   Ala Ser Ala Gln Gly Phe Glu Val Glu Ala Ala Pro Gly Tyr Gly Gly
                   325                 330                 335
   Asp
<210> 59
   <211> 987
   <212> DNA
   <213> Unknown
<220>
   <223> Obtained from an environmental sample
<400> 59
   atgcgagata ggaatttcaa actggcggcc attcaggcgg agccggtttt ctttaatcgc      60
   cgggcctcga cggaaaaggc ctgcagattg atcaaagaag cgggcgcgat gggcgccgat     120
   atcgcgggat tcagcgagac ctggcttccc gggtatccct tttttatctg gggcgcaagc     180
   gccgatccat ccctgctctg gaaggcttct gcggaatacc tggccaatgc cgttcaaata     240
   cccggtcccg agacggatca attatgcgag gcggcgaaaa aggccggcat cgatgtggcg     300
   atcggagtgg ttgaactcga cgagtttacg aagggaacgg cttactgcac gctgctcttc     360
   atcggcaaag aagggaagat cctgggaaag caccgcaaac tcaagccgac gcaccgggag     420
   cgcacggtat ggggagaggg cgatgcgacg ggactcagtg tccatgagcg tccttacggg     480
   cggatcagcg gcctgaactg ctgggagcat aatatggtcc tgcccggcta tgtcctgatg     540
   tctcagggca cgcacattca tatcgcggcc tggccgggtt cggaagggaa agcacctccc     600
   gcgccgtctc cgatgtggga gcgccagctt ctgctctccc gcgctttcgc ttcgcaatcc     660
   gccgcatacg tgattctggt cggaggactc ctgaacccgc agaatattcc ggcgccctac     720
   gatgaacttg ccgtcaagta ccggggagac agtttcatca tcgatccgcg cggggagatc     780
   atcgccgggc cggccaaggg ggaaaccatt ctcatcgccg aaggctcgat ggaacaggtc     840
   ctcgcggcaa agtccgcctt cgatgtcgcg ggacattatt cccgccccga cgtctttcaa     900
   ctctgcgtca accgcaaacc gtaccggcgt gtaagggaaa cttcggagca ggaccaaccc     960
   gcttctgaaa gagaatcgga atcgtaa                                         987
<210> 60
   <211> 328
   <212> PRT
   <213> Unknown
<220>
   <223> Obtained from an environmental sample
<400> 60
   Met Arg Asp Arg Asn Phe Lys Leu Ala Ala Ile Gln Ala Glu Pro Val
   1               5                   10                  15
   Phe Phe Asn Arg Arg Ala Ser Thr Glu Lys Ala Cys Arg Leu Ile Lys
               20                  25                  30
   Glu Ala Gly Ala Met Gly Ala Asp Ile Ala Gly Phe Ser Glu Thr Trp
           35                  40                  45
   Leu Pro Gly Tyr Pro Phe Phe Ile Trp Gly Ala Ser Ala Asp Pro Ser
       50                  55                  60
   Leu Leu Trp Lys Ala Ser Ala Glu Tyr Leu Ala Asn Ala Val Gln Ile
   65                  70                  75                  80
   Pro Gly Pro Glu Thr Asp Gln Leu Cys Glu Ala Ala Lys Lys Ala Gly
                   85                  90                  95
   Ile Asp Val Ala Ile Gly Val Val Glu Leu Asp Glu Phe Thr Lys Gly
               100                 105                 110
   Thr Ala Tyr Cys Thr Leu Leu Phe Ile Gly Lys Glu Gly Lys Ile Leu
           115                 120                 125
   Gly Lys His Arg Lys Leu Lys Pro Thr His Arg Glu Arg Thr Val Trp
       130                 135                 140
   Gly Glu Gly Asp Ala Thr Gly Leu Ser Val His Glu Arg Pro Tyr Gly
   145                 150                 155                 160
   Arg Ile Ser Gly Leu Asn Cys Trp Glu His Asn Met Val Leu Pro Gly
                   165                 170                 175
   Tyr Val Leu Met Ser Gln Gly Thr His Ile His Ile Ala Ala Trp Pro
               180                 185                 190
   Gly Ser Glu Gly Lys Ala Pro Pro Ala Pro Ser Pro Met Trp Glu Arg
           195                 200                 205
   Gln Leu Leu Leu Ser Arg Ala Phe Ala Ser Gln Ser Ala Ala Tyr Val
       210                 215                 220
   Ile Leu Val Gly Gly Leu Leu Asn Pro Gln Asn Ile Pro Ala Pro Tyr
   225                 230                 235                 240
   Asp Glu Leu Ala Val Lys Tyr Arg Gly Asp Ser Phe Ile Ile Asp Pro
                   245                 250                 255
   Arg Gly Glu Ile Ile Ala Gly Pro Ala Lys Gly Glu Thr Ile Leu Ile
                       260                           265                           270
   Ala Glu Gly Ser Met Glu Gln Val Leu Ala Ala Lys Ser Ala Phe Asp
               275                           280                           285
   Val Ala Gly His Tyr Ser Arg Pro Asp Val Phe Gln Leu Cys Val Asn
           290                           295                           300
   Arg Lys Pro Tyr Arg Arg Val Arg Glu Thr Ser Glu Gln Asp Gln Pro
   305                           310                           315                           320
   Ala Ser Glu Arg Glu Ser Glu Ser
                   325
<210> 61
   <211> 966
   <212> DNA
   <213> Unknown
<220>
   <223> Obtained from an environmental sample
<400> 61
   atgactcgat cttacccgaa tgacacactc acggttgggc ttgcgcaaat tgctccagtc      60
   tggttggatc gtacagggac aatttcaaag atattagctc aagtccatgc ggcaaatgaa     120
   gcgggctgtc atcttgtcgc gtttggcgaa ggtctcctcc ccggatatcc gttttggatt     180
   gagcgaacaa atggtgcagt cttcaactcg cccacgcaga aagaaattca cgcgcattat     240
   ctggatcagg ctgtccagat cgaagcaggt catcttgagg cgctttgcga agcagccaag     300
   gaatatgaga tcgcaattgt cctgggatgc attgaacgtc cgcaagatcg tggagggcac     360
   agtctgtatg caagccttgt atatattgat tcagacggca tcatccaatc tgtgcatcga     420
   aagttaatgc caacatatga agaacggctc acctggtcgc caggtgacgg acatggatta     480
   cgggtgcaca aattaggtgc ctttacggtt ggcggcctca actgttggga aaactggatg     540
   cctttggcac gcgcggccat gtatggtcaa ggcgaggatt tgcatattgc catttggccc     600
   ggcggctccc acaatacgca agacattaca cgctttattg cactagaatc gcgttcctat     660
   gttttatctg tgtcaggttt aatgcgctca ggcgattttc caaaagagac cccacatctt     720
   gcatccatcc tggctaaagg tgaggatatt cttgccaacg gtggttcatg tatcgccggt     780
   cctgacggca aatggatcgt tgagccgctt gtaggagaag agaagttaat tgttgcaacg     840
   attgatcatt gtcgtgtgcg cgaagagcgt caaaattttg atccttcagg acattacagc     900
   aggccagatg tattgcaact gaaaataaac aggcaacgcc agagtacaat ctcgtttgga     960
   gagtaa                                                                966
<210> 62
   <211> 321
   <212> PRT
   <213> Unknown
<220>
   <223> Obtained from an environmental sample
<400> 62
   Met Thr Arg Ser Tyr Pro Asn Asp Thr Leu Thr Val Gly Leu Ala Gln
   1               5                   10                  15
   Ile Ala Pro Val Trp Leu Asp Arg Thr Gly Thr Ile Ser Lys Ile Leu
               20                  25                  30
   Ala Gln Val His Ala Ala Asn Glu Ala Gly Cys His Leu Val Ala Phe
           35                  40                  45
   Gly Glu Gly Leu Leu Pro Gly Tyr Pro Phe Trp Ile Glu Arg Thr Asn
       50                  55                  60
   Gly Ala Val Phe Asn Ser Pro Thr Gln Lys Glu Ile His Ala His Tyr
   65                  70                  75                  80
   Leu Asp Gln Ala Val Gln Ile Glu Ala Gly His Leu Glu Ala Leu Cys
                   85                  90                  95
   Glu Ala Ala Lys Glu Tyr Glu Ile Ala Ile Val Leu Gly Cys Ile Glu
               100                 105                 110
   Arg Pro Gln Asp Arg Gly Gly His Ser Leu Tyr Ala Ser Leu Val Tyr
           115                 120                 125
   Ile Asp Ser Asp Gly Ile Ile Gln Ser Val His Arg Lys Leu Met Pro
       130                 135                 140
   Thr Tyr Glu Glu Arg Leu Thr Trp Ser Pro Gly Asp Gly His Gly Leu
   145                 150                 155                 160
   Arg Val His Lys Leu Gly Ala Phe Thr Val Gly Gly Leu Asn Cys Trp
                   165                 170                 175
   Glu Asn Trp Met Pro Leu Ala Arg Ala Ala Met Tyr Gly Gln Gly Glu
               180                 185                 190
   Asp Leu His Ile Ala Ile Trp Pro Gly Gly Ser His Asn Thr Gln Asp
           195                 200                 205
   Ile Thr Arg Phe Ile Ala Leu Glu Ser Arg Ser Tyr Val Leu Ser Val
       210                 215                 220
   Ser Gly Leu Met Arg Ser Gly Asp Phe Pro Lys Glu Thr Pro His Leu
   225                 230                 235                 240
   Ala Ser Ile Leu Ala Lys Gly Glu Asp Ile Leu Ala Asn Gly Gly Ser
                   245                 250                 255
   Cys Ile Ala Gly Pro Asp Gly Lys Trp Ile Val Glu Pro Leu Val Gly
               260                 265                 270
   Glu Glu Lys Leu Ile Val Ala Thr Ile Asp His Cys Arg Val Arg Glu
           275                 280                 285
   Glu Arg Gln Asn Phe Asp Pro Ser Gly His Tyr Ser Arg Pro Asp Val
       290                 295                 300
   Leu Gln Leu Lys Ile Asn Arg Gln Arg Gln Ser Thr Ile Ser Phe Gly
   305                 310                 315                 320
   Glu
<210> 63
   <211> 978
   <212> DNA
   <213> Unknown
<220>
   <223> Obtained from an environmental sample
<400> 63
   atgcaagata gagtaccgat tgtacgagct gcggctatcc aggctgaacc catagtcctt 60
   gattgtgacg cgaccgtgga aaaagcctgc cgattgatcg gtgaagcagc agaaaatggt 120
   gcaaacctga tcgtgtttcc cgaagccttc attcccgttt atcccaatgc ggcgatctgg 180
   ggtcgaggtc tggccacttt tggcggacag cgccagaaat acgtatggac gcgactatgg 240
   aacaattcgg tggaaatccc tggtccggcc accgacaggc tggcaaaggc agcacacgag 300
   gctcgagcca ccgttgtcat gggattgaat gagcgcgcgg tcgataacaa cacgctttac 360
   aacaccctgc tatttattgg gccagacggt cgcttgctgg gcaagcaccg taagctcatg 420
   cccaccaatc acgaacggat gatctggggt atgggagatg ggagcaccct gcgggttttt 480
   gatacaccct gtggaaaagt aggcggtctc atctgctggg aaaactacat gcctctggcg 540
   cgttatgcac tctatggaca gggcgaacaa atccatgtcg cgccgactgc gcacgatggt 600
   gagatcactc tggtcaatgc acgcaatacc gcctatgagg gacgcttatt cgtcatctcc 660
   gtgtgcatga tccttcgcaa gtccagcttt ccccatgatt ttgagctggg cgaggaattg 720
   gcggaggcag atgacttcat aaaatcaggc ggcagcgcga tcgttgggcc agatggcgag 780
   gtgctggcgg gtccattgtg gaatgaagag aatatactgt atgccgatct tgacttgaat 840
   cgaattgtgg atgagagacg agtatttgat gtgacgggcc attattcacg tccagatgtt 900
   ctacgactgc actttaatgc ttcccctcag aaaactattg aaagatatga gcaacctctc 960
   gatccgtctg agggttaa 978
<210> 64
   <211> 325
   <212> PRT
   <213> Unknown
<220>
   <223> Obtained from an environmental sample
<400> 64
   Met Gln Asp Arg Val Pro Ile Val Arg Ala Ala Ala Ile Gln Ala Glu
   1               5                   10                  15
   Pro Ile Val Leu Asp Cys Asp Ala Thr Val Glu Lys Ala Cys Arg Leu
               20                  25                  30
   Ile Gly Glu Ala Ala Glu Asn Gly Ala Asn Leu Ile Val Phe Pro Glu
           35                  40                  45
   Ala Phe Ile Pro Val Tyr Pro Asn Ala Ala Ile Trp Gly Arg Gly Leu
       50                  55                  60
   Ala Thr Phe Gly Gly Gln Arg Gln Lys Tyr Val Trp Thr Arg Leu Trp
   65                  70                  75                  80
   Asn Asn Ser Val Glu Ile Pro Gly Pro Ala Thr Asp Arg Leu Ala Lys
                   85                  90                  95
   Ala Ala His Glu Ala Arg Ala Thr Val Val Met Gly Leu Asn Glu Arg
                       100                     105                     110
   Ala Val Asp Asn Asn Thr Leu Tyr Asn Thr Leu Leu Phe Ile Gly Pro
               115                     120                     125
   Asp Gly Arg Leu Leu Gly Lys His Arg Lys Leu Met Pro Thr Asn His
       130                     135                     140
   Glu Arg Met Ile Trp Gly Met Gly Asp Gly Ser Thr Leu Arg Val Phe
   145                     150                     155                     160
   Asp Thr Pro Cys Gly Lys Val Gly Gly Leu Ile Cys Trp Glu Asn Tyr
                   165                     170                     175
   Met Pro Leu Ala Arg Tyr Ala Leu Tyr Gly Gln Gly Glu Gln Ile His
               180                     185                     190
   Val Ala Pro Thr Ala His Asp Gly Glu Ile Thr Leu Val Asn Ala Arg
           195                     200                     205
   Asn Thr Ala Tyr Glu Gly Arg Leu Phe Val Ile Ser Val Cys Met Ile
       210                     215                     220
   Leu Arg Lys Ser Ser Phe Pro His Asp Phe Glu Leu Gly Glu Glu Leu
   225                     230                     235                     240
   Ala Glu Ala Asp Asp Phe Ile Lys Ser Gly Gly Ser Ala Ile Val Gly
                   245                     250                     255
   Pro Asp Gly Glu Val Leu Ala Gly Pro Leu Trp Asn Glu Glu Asn Ile
               260                     265                     270
   Leu Tyr Ala Asp Leu Asp Leu Asn Arg Ile Val Asp Glu Arg Arg Val
           275                     280                     285
   Phe Asp Val Thr Gly His Tyr Ser Arg Pro Asp Val Leu Arg Leu His
       290                     295                     300
   Phe Asn Ala Ser Pro Gln Lys Thr Ile Glu Arg Tyr Glu Gln Pro Leu
   305                     310                     315                     320
   Asp Pro Ser Glu Gly
                   325
<210> 65
   <211> 1002
   <212> DNA
   <213> Unknown
<220>
   <223> Obtained from an environmental sample
<400> 65
   atgccgaccc ccacttcaaa attcaaaatc ggcgccgtgc aggcatcgcc ggtttttctg      60
   gaccgggaag ccactgcgca aaaagcctgc aaattgattg ccgaagcggg agggcagggc     120
   gcgcggctga tcgttttccc ggagtctttc attcccacct atcctgattg ggtctgggcg     180
   gtcccgccgg gaagaggaaa agtgttaagc gaactttacg ccgagctgct ggccaatgcc     240
   gtggaagtcc ccgggccggt caccgatcag ctgggtgaag cagcccaaaa aacgggcgcc     300
   tatgtcgtca tgggcgtcac ggaaaaggac accgacgcaa gcggcgcgag cctttacaac     360
   acgctcctct atttcaaccc cgcgggggac ctcctgggaa aacaccggaa gcttgttcct     420
   accggcgggg agcggctggt ctgggcgcag ggcgacggca gcaccctgga agtgtacgac     480
   actcccctgg gaaaaatcgg aggcctcatc tgctgggaaa actacatgcc cctcgcccgg     540
   tacacgatgt atgcctgggg gacccagatt tatatcgcgg ccacatggga ccagggggag     600
   acgtggcttg ccaccctgcg gcatatcgct aaggaaggac gggtgtacgt catcggctgc     660
   tgcatcgcgc tgcggcggga cgacatcccc gaccggctgg aatacaagaa gaagttctac     720
   tcggggtcgc gggaatggat caatatgggg gacagcgcca tcgtgaaccc ggaaggcgaa     780
   ttcattgccg gccccgtgcg gatgaaggag gagatcctgt atgccgaggt ggaccccctc     840
   ctgatggcgg gatcgaaatg gatgctcgac gtcgcggggc attacgcgcg ccccgacgtc     900
   tttgaactca tcgtccaccg ccagccccac ccgatgatcc gggtaatcga gaaagaggga     960
   ggggccggaa gaaccgggga cgagaagaag gaaaatgagt ga                       1002
<210> 66
   <211> 333
   <212> PRT
   <213> unknown
<220>
   <223> Obtained from an environmental sample
<400> 66
   Met Pro Thr Pro Thr Ser Lys Phe Lys Ile Gly Ala Val Gln Ala Ser
   1               5                   10                  15
   Pro Val Phe Leu Asp Arg Glu Ala Thr Ala Gln Lys Ala Cys Lys Leu
               20                  25                  30
   Ile Ala Glu Ala Gly Gly Gln Gly Ala Arg Leu Ile Val Phe Pro Glu
           35                  40                  45
   Ser Phe Ile Pro Thr Tyr Pro Asp Trp Val Trp Ala Val Pro Pro Gly
       50                  55                  60
   Arg Gly Lys Val Leu Ser Glu Leu Tyr Ala Glu Leu Leu Ala Asn Ala
   65                  70                  75                  80
   Val Glu Val Pro Gly Pro Val Thr Asp Gln Leu Gly Glu Ala Ala Gln
                   85                  90                  95
   Lys Thr Gly Ala Tyr Val Val Met Gly Val Thr Glu Lys Asp Thr Asp
               100                 105                 110
   Ala Ser Gly Ala Ser Leu Tyr Asn Thr Leu Leu Tyr Phe Asn Pro Ala
           115                 120                 125
   Gly Asp Leu Leu Gly Lys His Arg Lys Leu Val Pro Thr Gly Gly Glu
       130                 135                 140
   Arg Leu Val Trp Ala Gln Gly Asp Gly Ser Thr Leu Glu Val Tyr Asp
   145                 150                 155                 160
   Thr Pro Leu Gly Lys Ile Gly Gly Leu Ile Cys Trp Glu Asn Tyr Met
                   165                 170                 175
   Pro Leu Ala Arg Tyr Thr Met Tyr Ala Trp Gly Thr Gln Ile Tyr Ile
               180                 185                 190
   Ala Ala Thr Trp Asp Gln Gly Glu Thr Trp Leu Ala Thr Leu Arg His
           195                 200                 205
   Ile Ala Lys Glu Gly Arg Val Tyr Val Ile Gly Cys Cys Ile Ala Leu
       210                 215                 220
   Arg Arg Asp Asp Ile Pro Asp Arg Leu Glu Tyr Lys Lys Lys Phe Tyr
   225                 230                 235                 240
   Ser Gly Ser Arg Glu Trp Ile Asn Met Gly Asp Ser Ala Ile Val Asn
                   245                 250                 255
   Pro Glu Gly Glu Phe Ile Ala Gly Pro Val Arg Met Lys Glu Glu Ile
               260                 265                 270
   Leu Tyr Ala Glu Val Asp Pro Leu Leu Met Ala Gly Ser Lys Trp Met
           275                 280                 285
   Leu Asp Val Ala Gly His Tyr Ala Arg Pro Asp Val Phe Glu Leu Ile
       290                 295                 300
   Val His Arg Gln Pro His Pro Met Ile Arg Val Ile Glu Lys Glu Gly
   305                 310                 315                 320
   Gly Ala Gly Arg Thr Gly Asp Glu Lys Lys Glu Asn Glu
                   325                 330
<210> 67
   <211> 936
   <212> DNA
   <213> Unknown
<220>
   <223> Obtained from an environmental sample
<400> 67
   atgccccgtg tggcggtggt ccagcgcccg ccggtgtttc tcgaccgcgc cgcgaccctc      60
   gagaacgccg tggcttcgct cgccgaggcc gcgtcgaacg gggctcgcct cgcggtcttt     120
   ccggaagccc tggttcccgg ctatccggcg tggatgtggc ggctgcggcc cgggcccgac     180
   atggcgctca ccgagcggat tcacgcgcgc ttgcgggcga actcggtgag cctcgccgcc     240
   gacgagctcg cgccgctgcg cgaggcggcc cggcgccacg agctcaccgt agtgtgcggc     300
   ctgcacgagc gcgacgaggc gctcggcggc ggcacgctct ataacaccgt cgtcacgatc     360
   ggcgccgacg gcgcggtgct caaccgccac cggaagctga tgcccaccaa ccccgagcgc     420
   atggtctggg gctgcggcga tgccagcggg ctcaggacgg tccccaccca gtgcgggcgc     480
   gtcggcgccc tgatctgctg ggaaagctac atgccgcttg cacgctacgc gctgtacgcc     540
   cagggaatcg acctctacgt cacgccgacc tacgacagcg gcgagcgggc ggttgcgacc     600
   atgcagcaca ttgcccgcga aggcggctgc tgggtggtga gctgcggctc ggcgtttcag     660
   gcgcgcgacg tcccggacgc gtttccgggg aagagcgagc ttttccgcga caacgacgag     720
   tggatcaacc cgggcgactc ggtcgtggtc gcgccgggcg gcaaggtcgt cgccgggccg     780
   ctgcacaaag aacgcgcgat cctgtacgcc gagatcgacc tcgagcgggt cggcgtggcg     840
   cgccgcagcc tggacgtggt cggccattat gcgcggcccg acctcttcga cctgcacgtg     900
   aacgcccgcc cgcaaagcgt ggttgaattg cgctga                               936
<210> 68
   <211> 311
   <212> PRT
   <213> Unknown
<220>
   <223> Obtained from an environmental sample
<400> 68
   Met Pro Arg Val Ala Val Val Gln Arg Pro Pro Val Phe Leu Asp Arg
   1               5                   10                  15
   Ala Ala Thr Leu Glu Asn Ala Val Ala Ser Leu Ala Glu Ala Ala Ser
               20                  25                  30
   Asn Gly Ala Arg Leu Ala Val Phe Pro Glu Ala Leu Val Pro Gly Tyr
           35                  40                  45
   Pro Ala Trp Met Trp Arg Leu Arg Pro Gly Pro Asp Met Ala Leu Thr
       50                  55                  60
   Glu Arg Ile His Ala Arg Leu Arg Ala Asn Ser Val Ser Leu Ala Ala
   65                  70                  75                  80
   Asp Glu Leu Ala Pro Leu Arg Glu Ala Ala Arg Arg His Glu Leu Thr
                   85                  90                  95
   Val Val Cys Gly Leu His Glu Arg Asp Glu Ala Leu Gly Gly Gly Thr
               100                 105                 110
   Leu Tyr Asn Thr Val Val Thr Ile Gly Ala Asp Gly Ala Val Leu Asn
           115                 120                 125
   Arg His Arg Lys Leu Met Pro Thr Asn Pro Glu Arg Met Val Trp Gly
       130                 135                 140
   Cys Gly Asp Ala Ser Gly Leu Arg Thr Val Pro Thr Gln Cys Gly Arg
   145                 150                 155                 160
   Val Gly Ala Leu Ile Cys Trp Glu Ser Tyr Met Pro Leu Ala Arg Tyr
                   165                 170                 175
   Ala Leu Tyr Ala Gln Gly Ile Asp Leu Tyr Val Thr Pro Thr Tyr Asp
               180                 185                 190
   Ser Gly Glu Arg Ala Val Ala Thr Met Gln His Ile Ala Arg Glu Gly
           195                 200                 205
   Gly Cys Trp Val Val Ser Cys Gly Ser Ala Phe Gln Ala Arg Asp Val
       210                 215                 220
   Pro Asp Ala Phe Pro Gly Lys Ser Glu Leu Phe Arg Asp Asn Asp Glu
   225                 230                 235                 240
   Trp Ile Asn Pro Gly Asp Ser Val Val Val Ala Pro Gly Gly Lys Val
                   245                 250                 255
   Val Ala Gly Pro Leu His Lys Glu Arg Ala Ile Leu Tyr Ala Glu Ile
               260                 265                 270
   Asp Leu Glu Arg Val Gly Val Ala Arg Arg Ser Leu Asp Val Val Gly
           275                 280                 285
   His Tyr Ala Arg Pro Asp Leu Phe Asp Leu His Val Asn Ala Arg Pro
       290                 295                 300
   Gln Ser Val Val Glu Leu Arg
   305                 310
<210> 69
   <211> 939
   <212> DNA
   <213> Unknown
<220>
   <223> Obtained from an environmental sample
<400> 69
   gtgaccgagt ttcggacggt gcgggtcgca gcggtgcagg cgacgccggt gaccctcgac      60
   gccgatgcct cggtcgagaa ggcgatcggg ctgatcggcg aggcggtggc cggtggagcg     120
   cagctcgtcg tgctgcccga ggccttcgtg tcgctctacc cgtcgaacgc gtgggcgcga     180
   gcggccgccg gattcggcgg cttcgacgag ctctgggagc ggatgtgggc cagctcgctc     240
   gacgtcccgg gcccgctggt cgaccggctg gtcgatgcgt gccgcaggca tgacgtggta     300
   tgcgtgatcg gcgtgaacga gcgcgaaagc gaaaggccgg ggtcgcttta caacacgatg     360
   ctgaccctcg gcccgtcggg cctcctgcac cggcaccgca agctcatgcc gacgcaccac     420
   gagcggctgt tccatgggat cggcgacggt caagacctcg gcgttgtgga gaccgacgcg     480
   ggacggatcg ggggactgat ctgctgggag aaccgaatgc cgctcgcgcg ctacgcggtc     540
   taccagggtg gaccgcagat ctgggtcgcg ccgacggccg atgactccga cggctggctc     600
   gcgagcatgc gccacatcgc gatcgagtcg ggcgcgttcg tcgtgtcggt gccgcagttc     660
   atcccggcgt ccgcgttccc cgacgatttc cccgtcgagc taccgccggg caaggaggtg     720
   ttcggccgcg gcggtgcggc gatcgtcgag ccgacctggg gcgaggtaat cgccgggccg     780
   ctctacgatc gggaggggat cgtgttcgcc gactgtgacc tgcgacgcgg cttgcatgcc      840
   aagcgctggt tcgactccgt cggccattac agccgcgcgg aggtgctcga tggcggcgtc      900
   gagcgcgtcc cggcgccggt ggacggcgaa tcgccgtga                             939
<210> 70
   <211> 312
   <212> PRT
   <213> Unknown
<220>
   <223> Obtained from an environmental sample
<400> 70
   Val Thr Glu Phe Arg Thr Val Arg Val Ala Ala Val Gln Ala Thr Pro
   1               5                   10                  15
   Val Thr Leu Asp Ala Asp Ala Ser Val Glu Lys Ala Ile Gly Leu Ile
               20                  25                  30
   Gly Glu Ala Val Ala Gly Gly Ala Gln Leu Val Val Leu Pro Glu Ala
           35                  40                  45
   Phe Val Ser Leu Tyr Pro Ser Asn Ala Trp Ala Arg Ala Ala Ala Gly
       50                  55                  60
   Phe Gly Gly Phe Asp Glu Leu Trp Glu Arg Met Trp Ala Ser Ser Leu
   65                  70                  75                  80
   Asp Val Pro Gly Pro Leu Val Asp Arg Leu Val Asp Ala Cys Arg Arg
                   85                  90                  95
   His Asp Val Val Cys Val Ile Gly Val Asn Glu Arg Glu Ser Glu Arg
               100                 105                 110
   Pro Gly Ser Leu Tyr Asn Thr Met Leu Thr Leu Gly Pro Ser Gly Leu
           115                 120                 125
   Leu His Arg His Arg Lys Leu Met Pro Thr His His Glu Arg Leu Phe
       130                 135                 140
   His Gly Ile Gly Asp Gly Gln Asp Leu Gly Val Val Glu Thr Asp Ala
   145                 150                 155                 160
   Gly Arg Ile Gly Gly Leu Ile Cys Trp Glu Asn Arg Met Pro Leu Ala
                   165                 170                 175
   Arg Tyr Ala Val Tyr Gln Gly Gly Pro Gln Ile Trp Val Ala Pro Thr
               180                 185                 190
   Ala Asp Asp Ser Asp Gly Trp Leu Ala Ser Met Arg His Ile Ala Ile
           195                 200                 205
   Glu Ser Gly Ala Phe Val Val Ser Val Pro Gln Phe Ile Pro Ala Ser
       210                 215                 220
   Ala Phe Pro Asp Asp Phe Pro Val Glu Leu Pro Pro Gly Lys Glu Val
   225                 230                 235                 240
   Phe Gly Arg Gly Gly Ala Ala Ile Val Glu Pro Thr Trp Gly Glu Val
                   245                 250                 255
   Ile Ala Gly Pro Leu Tyr Asp Arg Glu Gly Ile Val Phe Ala Asp Cys
               260                 265                 270
   Asp Leu Arg Arg Gly Leu His Ala Lys Arg Trp Phe Asp Ser Val Gly
           275                 280                 285
   His Tyr Ser Arg Ala Glu Val Leu Asp Gly Gly Val Glu Arg Val Pro
       290                 295                 300
   Ala Pro Val Asp Gly Glu Ser Pro
   305                 310
<210> 71
   <211> 966
   <212> DNA
   <213> Unknown
<220>
   <223> obtained from an environmental sample
<400> 71
   atgccaaacg agaacaccaa cgccacattc aaagttgccg ctgtgcaggc ttcgcctgtg        60
   tttcttgatc gtgccgcaac aatcgacaag gcttgcgatt tgatcgccgc tgctggcggt       120
   gaaggggcac gcttgattgt ctttccagaa gcattcatcc cgtcttatcc tgattgggta       180
   tgggcaattc cttcgggtga agagggcgta ctcaatgagt tgtacgcaga tctgctatcc       240
   aactcggtca cgattcccag tgactcgacg gacaaactgt gcagagcagc caggcttgct       300
   aatgcctacg tggtgatggg tatgagcgaa cgcaatgctg aggcaagcgg cgcgagcatg       360
   tataacacgc tattgtatat tgatgcacag ggggagattc tgggcaagca tcggaagttg      420
   gtgccaacgg gcggcgagcg gctagtctgg gcgcagggcg atggcagtac actgcaggtc       480
   tatgatactc ccttagggaa actcggtggc ttaatttgct gggagaatta tatgccactg       540
   gcccgctata ccatgtatgc ctggggcaca caaatctatg tcgcggcaac gtgggatcgg       600
   ggtcagccct ggctctctac tttacgccac attgccaaag aaggcagggt gtatgtgatt       660
   ggttgttgta tcgcgatgcg taaagacgat atcccagacc attatacaat gaaacagaag       720
   ttttactcag atgcagatga gtggattaat attggcgata gtgcgattgt taatcccgaa       780
   gggcaattta tcgctggacc ggtgcgcaag caggaagaga ttctctatgc ggagattgat       840
   ccgcgcatgg tccaagggcc gaagtggatg ctcgacgtgg cgggacatta tgccaggccg       900
   gatgtgttcg aactgattgt ccacacggat attcgaagga tgatcaaatc ggaaaagaat       960
   tcataa                                                                  966
<210> 72
   <211> 321
   <212> PRT
   <213> Unknown
<220>
   <223> Obtained from an environmental sample
<400> 72
   Met Pro Asn Glu Asn Thr Asn Ala Thr Phe Lys Val Ala Ala Val Gln
    1               5                  10                  15
   Ala Ser Pro Val Phe Leu Asp Arg Ala Ala Thr Ile Asp Lys Ala Cys
               20                  25                  30
   Asp Leu Ile Ala Ala Ala Gly Gly Glu Gly Ala Arg Leu Ile Val Phe
           35                  40                  45
   Pro Glu Ala Phe Ile Pro Ser Tyr Pro Asp Trp Val Trp Ala Ile Pro
       50                  55                  60
   Ser Gly Glu Glu Gly Val Leu Asn Glu Leu Tyr Ala Asp Leu Leu Ser
   65                  70                  75                  80
   Asn Ser Val Thr Ile Pro Ser Asp Ser Thr Asp Lys Leu Cys Arg Ala
                   85                  90                  95
   Ala Arg Leu Ala Asn Ala Tyr Val Val Met Gly Met Ser Glu Arg Asn
               100                 105                 110
   Ala Glu Ala Ser Gly Ala Ser Met Tyr Asn Thr Leu Leu Tyr Ile Asp
           115                 120                 125
   Ala Gln Gly Glu Ile Leu Gly Lys His Arg Lys Leu Val Pro Thr Gly
       130                 135                 140
   Gly Glu Arg Leu Val Trp Ala Gln Gly Asp Gly Ser Thr Leu Gln Val
   145                 150                 155                 160
   Tyr Asp Thr Pro Leu Gly Lys Leu Gly Gly Leu Ile Cys Trp Glu Asn
                   165                 170                 175
   Tyr Met Pro Leu Ala Arg Tyr Thr Met Tyr Ala Trp Gly Thr Gln Ile
               180                 185                 190
   Tyr Val Ala Ala Thr Trp Asp Arg Gly Gln Pro Trp Leu Ser Thr Leu
           195                 200                 205
   Arg His Ile Ala Lys Glu Gly Arg Val Tyr Val Ile Gly Cys Cys Ile
       210                 215                 220
   Ala Met Arg Lys Asp Asp Ile Pro Asp His Tyr Thr Met Lys Gln Lys
   225                 230                 235                 240
   Phe Tyr Ser Asp Ala Asp Glu Trp Ile Asn Ile Gly Asp Ser Ala Ile
                   245                 250                 255
   Val Asn Pro Glu Gly Gln Phe Ile Ala Gly Pro Val Arg Lys Gln Glu
               260                 265                 270
   Glu Ile Leu Tyr Ala Glu Ile Asp Pro Arg Met Val Gln Gly Pro Lys
           275                 280                 285
   Trp Met Leu Asp Val Ala Gly His Tyr Ala Arg Pro Asp Val Phe Glu
       290                 295                 300
   Leu Ile Val His Thr Asp Ile Arg Arg Met Ile Lys Ser Glu Lys Asn
   305                 310                 315                 320
   Ser
<210> 73
   <211> 1035
   <212> DNA
   <213> Unknown
<220>
   <223> Obtained from an environmental sample
<400> 73
   atgacagcaa tagactcaac gtttaaagtc gccgccgttc aggctgcgcc ggtcttcctc     60
   aatcgcgacg caaccgtgga gaaggcgtgc cggctgatca agtccgcggc agagggaggc    120
   gcgcgtctga tcgttttccc ggaagcgttc ataccggcct acccggactg ggtgtggacg    180
   gtccctgccg gtgagcaagg cctgctcaac gacctctacg gccaactcgt cgaccagtcc    240
   gtgacgattc ccagcgacat caccaccgag ttatgtaacg cggcacgggc agcaaacgcc    300
   tatgtcgtga ttggtgtcaa cgagcgcaac gcggaggcaa gcaatggaag cctctacaac    360
   tcgctcctct acatcgacgc aaacggcaaa attctcggta agcaccgcaa gctcgttccc    420
   acaggcggag aacggctcgt gtgggcgcag ggcgatggca gcacgctcga agcctacgac    480
   acggagctgg gcaaactcgg cggtctcatt tgctgggaga actatatgcc gctggcacgc    540
   tacgcgatgt acgcatgggg agtgcagctc tatgtcgccg cgacctggga ccgtggcggc    600
   ccctggactg ccacgctgcg tcatgtcgcc aaggaaggtc agatgtacgt catcgggtgc    660
   tgccaggccc tgcacaagga tgacctgccg gagctagacg ggctgaagga gaagtactac    720
   gccaacgcac gagagtggat caatgttggc gacagcgcta ttgtcggccc ggacggacaa    780
   ttccttgtcg agcccgtccg aatgcgggaa gacatcctct acgccgaggt ggacactcgc    840
   aacttccgcg gcccgaagtg gatgttcgac gcggctggac actacgcgcg tcccgacatt    900
   ttccaactca cagtgaaccg cgagcagcgg ccgatggtcc gcgtcgtcgg tgacagcagt    960
   gaccagaagg agcggccgct cccggacgac ggacggctct ggtacgccta cagcaccaat   1020
   cagcaccacg actga                                                    1035
<210> 74
   <211> 344
   <212> PRT
   <213> Unknown
<220>
   <223> Obtained from an environmental sample
<400> 74
   Met Thr Ala Ile Asp Ser Thr Phe Lys Val Ala Ala Val Gln Ala Ala
    1               5                  10                  15
   Pro Val Phe Leu Asn Arg Asp Ala Thr Val Glu Lys Ala Cys Arg Leu
               20                  25                  30
   Ile Lys Ser Ala Ala Glu Gly Gly Ala Arg Leu Ile Val Phe Pro Glu
           35                  40                  45
   Ala Phe Ile Pro Ala Tyr Pro Asp Trp Val Trp Thr Val Pro Ala Gly
       50                  55                  60
   Glu Gln Gly Leu Leu Asn Asp Leu Tyr Gly Gln Leu Val Asp Gln Ser
   65                  70                  75                  80
   Val Thr Ile Pro Ser Asp Ile Thr Thr Glu Leu Cys Asn Ala Ala Arg
                   85                  90                  95
   Ala Ala Asn Ala Tyr Val Val Ile Gly Val Asn Glu Arg Asn Ala Glu
               100                 105                 110
   Ala Ser Asn Gly Ser Leu Tyr Asn Ser Leu Leu Tyr Ile Asp Ala Asn
           115                 120                 125
   Gly Lys Ile Leu Gly Lys His Arg Lys Leu Val Pro Thr Gly Gly Glu
       130                 135                 140
   Arg Leu Val Trp Ala Gln Gly Asp Gly Ser Thr Leu Glu Ala Tyr Asp
   145                 150                 155                 160
   Thr Glu Leu Gly Lys Leu Gly Gly Leu Ile Cys Trp Glu Asn Tyr Met
                   165                 170                 175
   Pro Leu Ala Arg Tyr Ala Met Tyr Ala Trp Gly Val Gln Leu Tyr Val
               180                 185                 190
   Ala Ala Thr Trp Asp Arg Gly Gly Pro Trp Thr Ala Thr Leu Arg His
           195                 200                 205
   Val Ala Lys Glu Gly Gln Met Tyr Val Ile Gly Cys Cys Gln Ala Leu
       210                 215                 220
   His Lys Asp Asp Leu Pro Glu Leu Asp Gly Leu Lys Glu Lys Tyr Tyr
   225                 230                 235                 240
   Ala Asn Ala Arg Glu Trp Ile Asn Val Gly Asp Ser Ala Ile Val Gly
                   245                 250                 255
   Pro Asp Gly Gln Phe Leu Val Glu Pro Val Arg Met Arg Glu Asp Ile
               260                 265                 270
   Leu Tyr Ala Glu Val Asp Thr Arg Asn Phe Arg Gly Pro Lys Trp Met
           275                 280                 285
   Phe Asp Ala Ala Gly His Tyr Ala Arg Pro Asp Ile Phe Gln Leu Thr
       290                 295                 300
   Val Asn Arg Glu Gln Arg Pro Met Val Arg Val Val Gly Asp Ser Ser
   305                 310                 315                 320
   Asp Gln Lys Glu Arg Pro Leu Pro Asp Asp Gly Arg Leu Trp Tyr Ala
                   325                 330                 335
   Tyr Ser Thr Asn Gln His His Asp
               340
<210> 75
   <211> 1125
   <212> DNA
   <213> Unknown
<220>
   <223> Obtained from an environmental sample
<400> 75
   atgagcacca ttgttaaagc cgctgcggtt caaatcagcc cagtcctcta cagccgcgag     60
   gggacagtcg caaaagttgt gcggaagatc cacgaacttg gccaaaaggg ggtgcggttc    120
   gccacgttcc cggagaccgt ggttccctac tatccatatt tttccgccgt ccagaccccc    180
   attcaactat tgtccggaac cgagtacctg aagttgctcg accaaggcgt gaccgtgccg    240
   tccacgacta ccgacgcaat cggggaggct gcccggaacg ccggcatggt tgtatctatc    300
   ggcgtgaatg agcgtgacgg cgggaccctg tacaacgcgc agttgctctt cgatgcggat    360
   gggaccttga ttcagcgtcg ccgcaagatc actcctacgc attacgagcg catgatctgg    420
   ggccagggag atggttcggg tttgcgggcc gtcaagagcc aggttggtcg tattggccaa    480
   cttgcatgct ttgagcacaa caacccactg gcgcgttacg cgatgatggc cgatggcgag    540
   caaatccatt cggccatgta tccaggttcc gcgttcggcg aggggttcgc ggaaaagatg    600
   gaaatcaata tccgccagca tgcgttggag tccgggtgct tcgttgtgaa tgcaacggcc    660
   tggcttgacg ccagccagca ggcacaaatc atgaatgaca cgggttgcca aatcggtccg    720
   atctcgggcg gttgctttac cacgatcgta acacccgacg gcacgtttct gggcgaacct    780
   ctccggtcgg gtgagggcga ggtcatcgcc gatctcgatt tcaagctgat cgacaaacgc    840
   aagatgttga tggactcgcg cggccactac agtcgcccgg aattgctcag tctgctgatc    900
   gaccgcaccc ccaccgcgca cattcatgag cgaggtgcgc cgcagacgtc aggcgctgtg    960
   caagaggcga cgaaagtggg ttcacacgcg ccgctcctgc gtgacggaca atgggatcag   1020
   ctcaatgcgg gagcgggccg acatacaggg aatggagaag cacagataga aatcatggcc   1080
   gcggcccact cgggcacccg tggaattgaa gcgaagggag cctaa                   1125
<210> 76
   <211> 374
   <212> PRT
   <213> Unknown
<220>
   <223> obtained from an environmental sample
<400> 76
   Met Ser Thr Ile Val Lys Ala Ala Ala Val Gln Ile Ser Pro Val Leu
   1               5                   10                  15
   Tyr Ser Arg Glu Gly Thr Val Ala Lys Val Val Arg Lys Ile His Glu
               20                  25                  30
   Leu Gly Gln Lys Gly Val Arg Phe Ala Thr Phe Pro Glu Thr Val Val
           35                  40                  45
   Pro Tyr Tyr Pro Tyr Phe Ser Ala Val Gln Thr Pro Ile Gln Leu Leu
       50                  55                  60
   Ser Gly Thr Glu Tyr Leu Lys Leu Leu Asp Gln Gly Val Thr Val Pro
   65                  70                  75                  80
   Ser Thr Thr Thr Asp Ala Ile Gly Glu Ala Ala Arg Asn Ala Gly Met
                   85                  90                  95
   Val Val Ser Ile Gly Val Asn Glu Arg Asp Gly Gly Thr Leu Tyr Asn
               100                 105                 110
   Ala Gln Leu Leu Phe Asp Ala Asp Gly Thr Leu Ile Gln Arg Arg Arg
           115                 120                 125
   Lys Ile Thr Pro Thr His Tyr Glu Arg Met Ile Trp Gly Gln Gly Asp
       130                 135                 140
   Gly Ser Gly Leu Arg Ala Val Lys Ser Gln Val Gly Arg Ile Gly Gln
   145                 150                 155                 160
   Leu Ala Cys Phe Glu His Asn Asn Pro Leu Ala Arg Tyr Ala Met Met
                   165                 170                 175
   Ala Asp Gly Glu Gln Ile His Ser Ala Met Tyr Pro Gly Ser Ala Phe
                       180                       185                       190
   Gly Glu Gly Phe Ala Glu Lys Met Glu Ile Asn Ile Arg Gln His Ala
               195                     200                     205
   Leu Glu Ser Gly Cys Phe Val Val Asn Ala Thr Ala Trp Leu Asp Ala
       210                     215                     220
   Ser Gln Gln Ala Gln Ile Met Asn Asp Thr Gly Cys Gln Ile Gly Pro
   225                     230                     235                     240
   Ile Ser Gly Gly Cys Phe Thr Thr Ile Val Thr Pro Asp Gly Thr Phe
                       245                     250                     255
   Leu Gly Glu Pro Leu Arg Ser Gly Glu Gly Glu Val Ile Ala Asp Leu
                   260                     265                     270
   Asp Phe Lys Leu Ile Asp Lys Arg Lys Met Leu Met Asp Ser Arg Gly
               275                     280                     285
   His Tyr Ser Arg Pro Glu Leu Leu Ser Leu Leu Ile Asp Arg Thr Pro
       290                     295                     300
   Thr Ala His Ile His Glu Arg Gly Ala Pro Gln Thr Ser Gly Ala Val
   305                     310                     315                     320
   Gln Glu Ala Thr Lys Val Gly Ser His Ala Pro Leu Leu Arg Asp Gly
                       325                     330                     335
   Gln Trp Asp Gln Leu Asn Ala Gly Ala Gly Arg His Thr Gly Asn Gly
                   340                     345                     350
   Glu Ala Gln Ile Glu Ile Met Ala Ala Ala His Ser Gly Thr Arg Gly
               355                     360                     365
   Ile Glu Ala Lys Gly Ala
       370
<210> 77
   <211> 1056
   <212> DNA
   <213> Unknown
<220>
   <223> Obtained from an environmental sample
<400> 77
   atgccaaccc ccagcgatca tttcaaaatc gccgctgttc aggcctcgcc cgtgtttctg     60
   gaccgggagg ccactgtgga aaaggcctgc cggttgatcg ccgaagccgc aaagcagggc    120
   gtccgcctca tcgtctttcc ggaatcgttc atcccgacct acccggactg ggtatgggcc    180
   gttcccccgg gaagggaaag aatcctgaac cagctgtatt ctgaattcct ggccaatgcc    240
   gtcgatgttc ccggcgcggc gaccgaacaa cttgcccagg ctgcacgaat ggccggcgcc    300
   tatgtgatta tgggcgtcac cgaaagagac acctcggcca gcggggccag cctctacaac    360
   accctgctct acttcagccc cgaaggcatc ctaatgggca aacaccggaa gctggttccc    420
   acggggggcg aacggctggt ctgggcctac ggagacggca gcacgctgga ggtctacgac    480
   actccgctgg gaaagatcgg cgggctgatc tgctgggaga actacatgcc cctggcccgg    540
   tacacgatgt acgcctgggg cacccagatt tacatcgccg ccacctggga ccgcggggaa    600
   ccgtggctct ccaccctgcg gcatatcgca aaggaaggaa gggtctacgt catcgggtgc    660
   tgcatcgccc tgcgccaggg ggatatcccg gaccggttcg agtacaaggg aaaattttat    720
   tccgggtccc gggagtggat caatgagggc gacagcgcca tcgtgaaccc ggacggggaa    780
   ttcatcgccg ggccggtgcg gacgaaggag gagatcctgt atgccgagat agacccccgg    840
   cagatgcggg gccccaagtg gatgctcgat gtggccggtc attacgcccg gccggatatc    900
   ttcgagctca tcgtccaccg gaatccccac ccgatgatca aaatcgccga agacaggggc    960
   acggggatcg cctcaagttt gattcgcccc cgccctaacc ttcccccatc aagggggagg   1020
   aaatcggcaa gaagcaaacg caagcccaaa aaatga                             1056
<210> 78
   <211> 351
   <212> PRT
   <213> Unknown
<220>
   <223> Obtained from an environmental sample
<400> 78
   Met Pro Thr Pro Ser Asp His Phe Lys Ile Ala Ala Val Gln Ala Ser
   1               5                   10                  15
   Pro Val Phe Leu Asp Arg Glu Ala Thr Val Glu Lys Ala Cys Arg Leu
               20                  25                  30
   Ile Ala Glu Ala Ala Lys Gln Gly Val Arg Leu Ile Val Phe Pro Glu
           35                  40                  45
   Ser Phe Ile Pro Thr Tyr Pro Asp Trp Val Trp Ala Val Pro Pro Gly
       50                  55                  60
   Arg Glu Arg Ile Leu Asn Gln Leu Tyr Ser Glu Phe Leu Ala Asn Ala
   65                  70                  75                  80
   Val Asp Val Pro Gly Ala Ala Thr Glu Gln Leu Ala Gln Ala Ala Arg
                   85                  90                  95
   Met Ala Gly Ala Tyr Val Ile Met Gly Val Thr Glu Arg Asp Thr Ser
               100                 105                 110
   Ala Ser Gly Ala Ser Leu Tyr Asn Thr Leu Leu Tyr Phe Ser Pro Glu
           115                 120                 125
   Gly Ile Leu Met Gly Lys His Arg Lys Leu Val Pro Thr Gly Gly Glu
       130                 135                 140
   Arg Leu Val Trp Ala Tyr Gly Asp Gly Ser Thr Leu Glu Val Tyr Asp
   145                 150                 155                 160
   Thr Pro Leu Gly Lys Ile Gly Gly Leu Ile Cys Trp Glu Asn Tyr Met
                   165                 170                 175
   Pro Leu Ala Arg Tyr Thr Met Tyr Ala Trp Gly Thr Gln Ile Tyr Ile
               180                 185                 190
   Ala Ala Thr Trp Asp Arg Gly Glu Pro Trp Leu Ser Thr Leu Arg His
           195                 200                 205
   Ile Ala Lys Glu Gly Arg Val Tyr Val Ile Gly Cys Cys Ile Ala Leu
       210                 215                 220
   Arg Gln Gly Asp Ile Pro Asp Arg Phe Glu Tyr Lys Gly Lys Phe Tyr
   225                 230                 235                 240
   Ser Gly Ser Arg Glu Trp Ile Asn Glu Gly Asp Ser Ala Ile Val Asn
                   245                 250                 255
   Pro Asp Gly Glu Phe Ile Ala Gly Pro Val Arg Thr Lys Glu Glu Ile
               260                 265                 270
   Leu Tyr Ala Glu Ile Asp Pro Arg Gln Met Arg Gly Pro Lys Trp Met
           275                 280                 285
   Leu Asp Val Ala Gly His Tyr Ala Arg Pro Asp Ile Phe Glu Leu Ile
       290                 295                 300
   Val His Arg Asn Pro His Pro Met Ile Lys Ile Ala Glu Asp Arg Gly
   305                 310                 315                 320
   Thr Gly Ile Ala Ser Ser Leu Ile Arg Pro Arg Pro Asn Leu Pro Pro
                   325                 330                 335
   Ser Arg Gly Arg Lys Ser Ala Arg Ser Lys Arg Lys Pro Lys Lys
               340                 345                 350
<210> 79
   <211> 990
   <212> DNA
   <213> Unknown
<220>
   <223> Obtained from an environmental sample
<400> 79
   atgacgaaga aaagcggccg cgattcgttt cgggtcgctg cggtccaggc ctcgtccgtc      60
   tacctggatc gggaacggag catcgagaaa gcgtgccggc tgatcgacga cgcgggacga     120
   aacgacgccg acctcgtcgt gttccccgaa gccttcgtgc ccggataccc actgtgggtg     180
   tggctcgttc cgccggggcg caccgcagac ttgcgctccg cttatgcgac gctccacgcc     240
   aacgcgatca gcattccgga cgactccacc gatcggctgt gcgccgccgc aaaagacgcc     300
   ggcgtcgccg tcgcgatcgg cgtcaacgaa cgcaacaccg aagcgagcgg catgagcctg     360
   ttcaacacgc tgctctatat cggagcggac ggccggattc tcggaaaaca ccggaagctg     420
   gtaccgaccg gcggcgaacg gctcgtctgg gcatctggcg acggcagcga cctcgaggtc     480
   tactcgctgc cgttcggtcg cgtaagcgga ctgatctgct gggagcacta catgccgctc     540
   gcccggtatg cgctcgccgc gtggggcgaa caggtgcacg tcgctccaac ctgggatcgt     600
   ggcgagccgt ggctgtccac gctaaggcac atcgcgaagg aaggccgcgt tctcgtcgtc     660
   ggctgctgtc aagccgtgcg caaggacgac atccctgaca cgctcgcgtt caagtccaaa     720
   tacctcgcag acgtggacgg ctggatcaac ccaggtggca gcgtcatcat caatcctgac     780
   ggcaaggtcg tcgcgggacc ggcgatggaa accgaaactg tactgtacgc ggaccttcgc     840
   accgagcagc tcgtcggacc gcgctggcag ctcgacgtcg gcggacatta cgctcgtccg     900
   gacgtcttcg agctcgtcgt ccatcggcat ccgaagccgt tgattcggac agcgaccggt     960
   gtcaggcgcc gcaagcgtgc acgtcgctaa                                      990
<210> 80
   <211> 329
   <212> PRT
   <213> Unknown
<220>
   <223> Obtained from an environmental sample
<400> 80
   Met Thr Lys Lys Ser Gly Arg Asp Ser Phe Arg Val Ala Ala Val Gln
   1               5                   10                  15
   Ala Ser Ser Val Tyr Leu Asp Arg Glu Arg Ser Ile Glu Lys Ala Cys
               20                  25                  30
   Arg Leu Ile Asp Asp Ala Gly Arg Asn Asp Ala Asp Leu Val Val Phe
           35                  40                  45
   Pro Glu Ala Phe Val Pro Gly Tyr Pro Leu Trp Val Trp Leu Val Pro
       50                  55                  60
   Pro Gly Arg Thr Ala Asp Leu Arg Ser Ala Tyr Ala Thr Leu His Ala
   65                  70                  75                  80
   Asn Ala Ile Ser Ile Pro Asp Asp Ser Thr Asp Arg Leu Cys Ala Ala
                   85                  90                  95
   Ala Lys Asp Ala Gly Val Ala Val Ala Ile Gly Val Asn Glu Arg Asn
               100                 105                 110
   Thr Glu Ala Ser Gly Met Ser Leu Phe Asn Thr Leu Leu Tyr Ile Gly
           115                 120                 125
   Ala Asp Gly Arg Ile Leu Gly Lys His Arg Lys Leu Val Pro Thr Gly
       130                 135                 140
   Gly Glu Arg Leu Val Trp Ala Ser Gly Asp Gly Ser Asp Leu Glu Val
   145                 150                 155                 160
   Tyr Ser Leu Pro Phe Gly Arg Val Ser Gly Leu Ile Cys Trp Glu His
                   165                 170                 175
   Tyr Met Pro Leu Ala Arg Tyr Ala Leu Ala Ala Trp Gly Glu Gln Val
               180                 185                 190
   His Val Ala Pro Thr Trp Asp Arg Gly Glu Pro Trp Leu Ser Thr Leu
           195                 200                 205
   Arg His Ile Ala Lys Glu Gly Arg Val Leu Val Val Gly Cys Cys Gln
       210                 215                 220
   Ala Val Arg Lys Asp Asp Ile Pro Asp Thr Leu Ala Phe Lys Ser Lys
   225                 230                 235                 240
   Tyr Leu Ala Asp Val Asp Gly Trp Ile Asn Pro Gly Gly Ser Val Ile
                   245                 250                 255
   Ile Asn Pro Asp Gly Lys Val Val Ala Gly Pro Ala Met Glu Thr Glu
               260                 265                 270
   Thr Val Leu Tyr Ala Asp Leu Arg Thr Glu Gln Leu Val Gly Pro Arg
           275                 280                 285
   Trp Gln Leu Asp Val Gly Gly His Tyr Ala Arg Pro Asp Val Phe Glu
       290                 295                 300
   Leu Val Val His Arg His Pro Lys Pro Leu Ile Arg Thr Ala Thr Gly
   305                 310                 315                 320
   Val Arg Arg Arg Lys Arg Ala Arg Arg
                   325
<210> 81
   <211> 993
   <212> DNA
   <213> Unknown
<220>
   <223> Obtained from an environmental sample
<400> 81
   atgaaagtcg tcaaagccgc cgctgtccag ttcagcccgg tgctctatag ccgcgaagcg      60
   accgtcgcca aggtcgtccg gaaaatccac gagctcggtc agaaaggcgt gcagttcgcc     120
   acctttcctg aaacggtcgt gccttattac ccttacttcg cggccgtcca gacgggcatc     180
   gagctcttgt cgggcaccga acatctgcgc ctgctcgaac aggccgtgac tgtgccctcc     240
   gctgcgaccg atgcaatcgg cgaagccgcg cgacaggccg gcatggtcgt gtccatcggc     300
   gtcaatgagc gtgacggcgg cacgctttac aacacgcaac tgctcttcga tgccgacggt     360
   acgctgatcc agcgccgccg caagatcacg ccgacccatt tcgaacgcat gatctggggg     420
   cagggagatg gctcgggctt gcgtgcagtc gacagcgcag tcggccgcat cggccagctc     480
   gcatgcttcg agcacaacaa cccgcttgca cgttacgcaa tgatcgccga cggcgagcag     540
   atccattcag cgatgtaccc tggctcggcc tttggcgagg gcttcgccca gcgtatggag     600
   atcaacatcc gccagcatgc gctcgagtcc gccgctttcg tcgtcaacgc aacggcgtgg     660
   cttgacgccg accagcaggc gcaaatcatg aaggacaccg gttgtggaat cggtccgatc     720
   tcgggcggct gcttcaccac gatcgtttct cctgacggta tgctgatggc cgatccgctt     780
   cgctcgggcg aaggcgaagt gattgtcgat ctcgacttca cgcagatcga ccgccgcaag     840
   atgctgatgg actcggccgg ccactacaac cgccctgaac tgctgagtct gatgatcgac     900
   cgtacgccgg ctgcgcatgt tcacgaacgc gcttcgcgcc cgatgaccgt cgacgaccag     960
   agttccggcg atctgcgcac ccaggttgca tga                                   993
<210> 82
   <211> 330
   <212> PRT
   <213> Unknown
<220>
   <223> Obtained from an environmental sample
<400> 82
   Met Lys Val Val Lys Ala Ala Ala Val Gln Phe Ser Pro Val Leu Tyr
   1               5                   10                  15
   Ser Arg Glu Ala Thr Val Ala Lys Val Val Arg Lys Ile His Glu Leu
               20                  25                  30
   Gly Gln Lys Gly Val Gln Phe Ala Thr Phe Pro Glu Thr Val Val Pro
           35                  40                  45
   Tyr Tyr Pro Tyr Phe Ala Ala Val Gln Thr Gly Ile Glu Leu Leu Ser
       50                  55                  60
   Gly Thr Glu His Leu Arg Leu Leu Glu Gln Ala Val Thr Val Pro Ser
   65                  70                  75                  80
   Ala Ala Thr Asp Ala Ile Gly Glu Ala Ala Arg Gln Ala Gly Met Val
                   85                  90                  95
   Val Ser Ile Gly Val Asn Glu Arg Asp Gly Gly Thr Leu Tyr Asn Thr
               100                 105                 110
   Gln Leu Leu Phe Asp Ala Asp Gly Thr Leu Ile Gln Arg Arg Arg Lys
           115                 120                 125
   Ile Thr Pro Thr His Phe Glu Arg Met Ile Trp Gly Gln Gly Asp Gly
       130                 135                 140
   Ser Gly Leu Arg Ala Val Asp Ser Ala Val Gly Arg Ile Gly Gln Leu
   145                 150                 155                 160
   Ala Cys Phe Glu His Asn Asn Pro Leu Ala Arg Tyr Ala Met Ile Ala
                   165                 170                 175
   Asp Gly Glu Gln Ile His Ser Ala Met Tyr Pro Gly Ser Ala Phe Gly
               180                 185                 190
   Glu Gly Phe Ala Gln Arg Met Glu Ile Asn Ile Arg Gln His Ala Leu
           195                 200                 205
   Glu Ser Ala Ala Phe Val Val Asn Ala Thr Ala Trp Leu Asp Ala Asp
       210                 215                 220
   Gln Gln Ala Gln Ile Met Lys Asp Thr Gly Cys Gly Ile Gly Pro Ile
   225                 230                 235                 240
   Ser Gly Gly Cys Phe Thr Thr Ile Val Ser Pro Asp Gly Met Leu Met
                   245                 250                 255
   Ala Asp Pro Leu Arg Ser Gly Glu Gly Glu Val Ile Val Asp Leu Asp
               260                 265                 270
   Phe Thr Gln Ile Asp Arg Arg Lys Met Leu Met Asp Ser Ala Gly His
           275                 280                 285
   Tyr Asn Arg Pro Glu Leu Leu Ser Leu Met Ile Asp Arg Thr Pro Ala
       290                 295                 300
   Ala His Val His Glu Arg Ala Ser Arg Pro Met Thr Val Asp Asp Gln
   305                 310                 315                 320
   Ser Ser Gly Asp Leu Arg Thr Gln Val Ala
                   325                 330
<210> 83
   <211> 1071
   <212> DNA
   <213> Unknown
<220>
   <223> obtained from an environmental sample
<400> 83
   atgatgagtt cagcccgtgt aataaaactc gccgcagcac agctttcacc tgtgctgccg      60
   ggggagtcca caaatagccg cgacggcacc attgccaaag tcgtcgcggc gattgcggag     120
   gctgcgcgcg ccggcgcgca gctgatcgtg tttcccgaaa cggtggtgcc gtattacccg     180
   tatttctcgt tcattacgcc ggcggtgacg atgggggcgg agcatttgcg cttgtacgat     240
   cagtctgtcg tggtgccgag cgccgccact gatactgttg ccgccgctgc aaaaaaacac     300
   agcatggtgg tcgtgctcgg tattaacgaa cgcgatcacg gcacgctcta caacgcgcaa     360
   ttaattttcg atgcgagcgg cgaattatta ttaaaacgcc gaaaaattac cccgacctat     420
   cacgagcgca tggtgtgggg tcagggcgac ggcagcggtt tgaaaaccgt cgacaccgcg     480
   atcggccgtg tcggtgcgct cgcctgctgg gaacattaca acccattggc gcgttacagc     540
   ctgatggccc agcacgaaga aattcattgc agtcaatttc cggggtcatt ggtcgggcca     600
   attttcgccg agcaaatgga agtgacaatg cgccaccacg cgctcgaatc cggttgcttc     660
   gtcgttaatg caacggcgtg gttatcggaa gcgcaaattc aatcgatcag cagcgatccc     720
   gcgatgcaaa aagcactgcg cggcggttgc tacaccgcaa ttatttcgcc cgaaggcaaa     780
   catctgtgcg agccgctacg cgaaggtgaa ggtttgattt ttgccgaagc cgatatggcg     840
   ctcattacca aacgcaaacg catgatggat tcggttggtc attacgcgcg acccgaattg     900
   ctgtcgctgt taatcgacca tcgcgccacc acaccattgc atagcgtcac cgcgagtgat     960
   gccgccgccg taaaaaatac tcggagttcc gctcatgaat cagccgatag tgaaaccatc    1020
   cgcgagtcag ttaataacgg aactccaatc gcacggcttg cgcctagttg a             1071
<210> 84
   <211> 356
   <212> PRT
   <213> Unknown
<220>
   <223> Obtained from an environmental sample
<400> 84
   Met Met Ser Ser Ala Arg Val Ile Lys Leu Ala Ala Ala Gln Leu Ser
    1               5                  10                  15
   Pro Val Leu Pro Gly Glu Ser Thr Asn Ser Arg Asp Gly Thr Ile Ala
               20                  25                  30
   Lys Val Val Ala Ala Ile Ala Glu Ala Ala Arg Ala Gly Ala Gln Leu
           35                  40                  45
   Ile Val Phe Pro Glu Thr Val Val Pro Tyr Tyr Pro Tyr Phe Ser Phe
       50                  55                  60
   Ile Thr Pro Ala Val Thr Met Gly Ala Glu His Leu Arg Leu Tyr Asp
   65                  70                  75                  80
   Gln Ser Val Val Val Pro Ser Ala Ala Thr Asp Thr Val Ala Ala Ala
                   85                  90                  95
   Ala Lys Lys His Ser Met Val Val Val Leu Gly Ile Asn Glu Arg Asp
               100                 105                 110
   His Gly Thr Leu Tyr Asn Ala Gln Leu Ile Phe Asp Ala Ser Gly Glu
           115                 120                 125
   Leu Leu Leu Lys Arg Arg Lys Ile Thr Pro Thr Tyr His Glu Arg Met
       130                 135                 140
   Val Trp Gly Gln Gly Asp Gly Ser Gly Leu Lys Thr Val Asp Thr Ala
   145                 150                 155                 160
   Ile Gly Arg Val Gly Ala Leu Ala Cys Trp Glu His Tyr Asn Pro Leu
                   165                 170                 175
   Ala Arg Tyr Ser Leu Met Ala Gln His Glu Glu Ile His Cys Ser Gln
               180                 185                 190
   Phe Pro Gly Ser Leu Val Gly Pro Ile Phe Ala Glu Gln Met Glu Val
           195                 200                 205
   Thr Met Arg His His Ala Leu Glu Ser Gly Cys Phe Val Val Asn Ala
       210                 215                 220
   Thr Ala Trp Leu Ser Glu Ala Gln Ile Gln Ser Ile Ser Ser Asp Pro
   225                 230                 235                 240
   Ala Met Gln Lys Ala Leu Arg Gly Gly Cys Tyr Thr Ala Ile Ile Ser
                   245                 250                 255
   Pro Glu Gly Lys His Leu Cys Glu Pro Leu Arg Glu Gly Glu Gly Leu
               260                 265                 270
   Ile Phe Ala Glu Ala Asp Met Ala Leu Ile Thr Lys Arg Lys Arg Met
           275                 280                 285
   Met Asp Ser Val Gly His Tyr Ala Arg Pro Glu Leu Leu Ser Leu Leu
       290                 295                 300
   Ile Asp His Arg Ala Thr Thr Pro Leu His Ser Val Thr Ala Ser Asp
   305                 310                 315                 320
   Ala Ala Ala Val Lys Asn Thr Arg Ser Ser Ala His Glu Ser Ala Asp
                   325                 330                 335
   Ser Glu Thr Ile Arg Glu Ser Val Asn Asn Gly Thr Pro Ile Ala Arg
               340                 345                 350
   Leu Ala Pro Ser
           355
<210> 85
   <211> 1014
   <212> DNA
   <213> Unknown
<220>
   <223> Obtained from an environmental sample
<400> 85
   atgggtctgg ttcatcagaa atacaaggtt gcggtggttc aggcggcgcc ggtctttctc      60
   gacctcgatg cgacggtgga caagacgatc gccctgatcg agcaggccgc agcacagggc     120
   gcgaagctga tcgcgtttcc cgagaccttc attcccggat atccgtggca gatctggctt     180
   ggggcgcccg cctgggcgat cggccgtggc ttcgtgcagc gctatttcga taactcgttg     240
   tcatttgaca gcccgcaggc cgaaaaaatt cgcaaggccg tcaagcgcgc caagctgacc     300
   gcggtgatcg gcgtctccga acgcgacggc ggcagcctct atatcggcca atggctgatc     360
   ggtcccgacg gcgagaccat tgcgaagcgc cgcaagctgc ggccgaccca tgccgaacgc     420
   accgtgttcg gcgagggcga cggcagcgac ctcgccgtcc atgatcgcgc cgacgtggga     480
   cggctcggtg caatgtgctg ctgggagcat ctgcagccgc tgtcgaaata cgcgatgtac     540
   gcccagaacg agcaggttca cgtcggcgcc tggccgagct tctcattgta cgacccattc     600
   gcccatgcgc ttggctggga agtaaacaac gcggcgagca aggtttatgc tgtcgagggc     660
   tcatgtttct tcctcggccc gtgcgcggtg gtctcgcagg ccatgatcga cgagctctgc     720
   gattcccccg aaaagcacgc cttcctgcac gctggcggcg gccacgcggt aatctatggg     780
   ccggacggga gttcgcttgc cgacaaactt ccacccgatc aggagggcat tctgtatgcc     840
   gatatcgatc tcggcatgat cggcgtggca aagaacgccg ccgaccccgc aggacactat     900
   tccaggccgg acgtcacgcg gctgctgctc aacacttccc gcgccaatcg cgtcgagcat     960
   ttttcattgc cgatcgatgc cgaggtcatg agcgaaatca gacttcaggc ctga          1014
<210> 86
   <211> 337
   <212> PRT
   <213> Unknown
<220>
   <223> Obtained from an environmental sample
<400> 86
   Met Gly Leu Val His Gln Lys Tyr Lys Val Ala Val Val Gln Ala Ala
   1               5                   10                  15
   Pro Val Phe Leu Asp Leu Asp Ala Thr Val Asp Lys Thr Ile Ala Leu
               20                  25                  30
   Ile Glu Gln Ala Ala Ala Gln Gly Ala Lys Leu Ile Ala Phe Pro Glu
           35                  40                  45
   Thr Phe Ile Pro Gly Tyr Pro Trp Gln Ile Trp Leu Gly Ala Pro Ala
       50                  55                  60
   Trp Ala Ile Gly Arg Gly Phe Val Gln Arg Tyr Phe Asp Asn Ser Leu
   65                  70                  75                  80
   Ser Phe Asp Ser Pro Gln Ala Glu Lys Ile Arg Lys Ala Val Lys Arg
                   85                  90                  95
   Ala Lys Leu Thr Ala Val Ile Gly Val Ser Glu Arg Asp Gly Gly Ser
               100                 105                 110
   Leu Tyr Ile Gly Gln Trp Leu Ile Gly Pro Asp Gly Glu Thr Ile Ala
           115                 120                 125
   Lys Arg Arg Lys Leu Arg Pro Thr His Ala Glu Arg Thr Val Phe Gly
       130                 135                 140
   Glu Gly Asp Gly Ser Asp Leu Ala Val His Asp Arg Ala Asp Val Gly
   145                 150                 155                 160
   Arg Leu Gly Ala Met Cys Cys Trp Glu His Leu Gln Pro Leu Ser Lys
                   165                 170                 175
   Tyr Ala Met Tyr Ala Gln Asn Glu Gln Val His Val Gly Ala Trp Pro
               180                 185                 190
   Ser Phe Ser Leu Tyr Asp Pro Phe Ala His Ala Leu Gly Trp Glu Val
           195                 200                 205
   Asn Asn Ala Ala Ser Lys Val Tyr Ala Val Glu Gly Ser Cys Phe Phe
       210                 215                 220
   Leu Gly Pro Cys Ala Val Val Ser Gln Ala Met Ile Asp Glu Leu Cys
   225                 230                 235                 240
   Asp Ser Pro Glu Lys His Ala Phe Leu His Ala Gly Gly Gly His Ala
                   245                 250                 255
   Val Ile Tyr Gly Pro Asp Gly Ser Ser Leu Ala Asp Lys Leu Pro Pro
               260                 265                 270
   Asp Gln Glu Gly Ile Leu Tyr Ala Asp Ile Asp Leu Gly Met Ile Gly
           275                 280                 285
   Val Ala Lys Asn Ala Ala Asp Pro Ala Gly His Tyr Ser Arg Pro Asp
       290                 295                 300
   Val Thr Arg Leu Leu Leu Asn Thr Ser Arg Ala Asn Arg Val Glu His
   305                 310                 315                 320
   Phe Ser Leu Pro Ile Asp Ala Glu Val Met Ser Glu Ile Arg Leu Gln
                   325                 330                 335
   Ala
<210> 87
   <211> 1062
   <212> DNA
   <213> Unknown
<220>
   <223> Obtained from an environmental sample
<400> 87
   atggcggaat cgaagctgaa ggtcgccgca attcaagttg cgcccgtgtt catggatcgc      60
   gatgccacga tcgcccgcgc ctgcgagcgg atcgccgaag ccgcccgcgc cggcgcggag     120
   ttggtggtct ttcccgaggc attcgtgccc gggtatcccg actggatctg ggtggcgcgg     180
   ccaagccaac gcaaactgct caatgatctt tacgcgcacc tcgtctcgca gtcggtcgac     240
   gtgccgtcgg cctccgtgga tcgtttgcgc gacgcggctc gcgacggcgg ggtcacggtg     300
   gtgatcggcg tcaacgagcg caacaccgaa gcgagcggcg cgagcctcta caacaccgcg     360
   ctcgtgatcg gtccactggg gcagctgatc ggccgccacc gcaagcttgt gccgaccggg     420
   ccggagcgca tggtgtgggc gcagggcgac ggcagcacgc tcgacgtcta cgacacaccc     480
   gtcggcaagc tttcgacgtt gatctgctgg gagaactaca tgccgctcgc gcgctacgcc     540
   atggcggcgt ggggcgcgcg catccacgtc gccggcacgt gggaccgcgg cgagccgtgg     600
   atctcgacca tgcgtcatgt ggcgacggag ggccgcgtat tcgtgattag ctgttgcatg     660
   gcgctgcgca aacgagacat tcccgccgag ctcgagttcg cgatgctcta tcccgacggg     720
   cgcgaatgga tcaacgccgg tgattcgctg gtcgtgaatc ccgctggcca gatcatcgct     780
   gggccgttgc acgagcagga aggaatcctc tacgccgagc tcgagcgcaa tcagatgacc     840
   ggtccgcgtt ggatgttcga cgccgccggc cattacgcgc gaccggacgt cttccaactc     900
   acggtaaacc gctccccgcg cccgatgctg cgggaggcgg gggcaaagac gagtgaggca     960
   aacacgagag atgccgtacc catggacagc acgccctcga gatcgcggcc ccgcgcggtg    1020
   gcgcgaaagg ccgcacgcac cggtcgctcc aagcggcggt ga                       1062
<210> 88
   <211> 353
   <212> PRT
   <213> Unknown
<220>
   <223> Obtained from an environmental sample
<400> 88
   Met Ala Glu Ser Lys Leu Lys Val Ala Ala Ile Gln Val Ala Pro Val
   1 5 10 15
   Phe Met Asp Arg Asp Ala Thr Ile Ala Arg Ala Cys Glu Arg Ile Ala
   20 25 30
   Glu Ala Ala Arg Ala Gly Ala Glu Leu Val Val Phe Pro Glu Ala Phe
   35 40 45
   Val Pro Gly Tyr Pro Asp Trp Ile Trp Val Ala Arg Pro Ser Gln Arg
   50 55 60
   Lys Leu Leu Asn Asp Leu Tyr Ala His Leu Val Ser Gln Ser Val Asp
   65 70 75 80
   Val Pro Ser Ala Ser Val Asp Arg Leu Arg Asp Ala Ala Arg Asp Gly
   85 90 95
   Gly Val Thr Val Val Ile Gly Val Asn Glu Arg Asn Thr Glu Ala Ser
   100 105 110
   Gly Ala Ser Leu Tyr Asn Thr Ala Leu Val Ile Gly Pro Leu Gly Gln
   115 120 125
   Leu Ile Gly Arg His Arg Lys Leu Val Pro Thr Gly Pro Glu Arg Met
   130 135 140
   Val Trp Ala Gln Gly Asp Gly Ser Thr Leu Asp Val Tyr Asp Thr Pro
   145 150 155 160
   Val Gly Lys Leu Ser Thr Leu Ile Cys Trp Glu Asn Tyr Met Pro Leu
   165 170 175
   Ala Arg Tyr Ala Met Ala Ala Trp Gly Ala Arg Ile His Val Ala Gly
   180 185 190
   Thr Trp Asp Arg Gly Glu Pro Trp Ile Ser Thr Met Arg His Val Ala
   195 200 205
   Thr Glu Gly Arg Val Phe Val Ile Ser Cys Cys Met Ala Leu Arg Lys
   210 215 220
   Arg Asp Ile Pro Ala Glu Leu Glu Phe Ala Met Leu Tyr Pro Asp Gly
   225 230 235 240
   Arg Glu Trp Ile Asn Ala Gly Asp Ser Leu Val Val Asn Pro Ala Gly
   245 250 255
   Gln Ile Ile Ala Gly Pro Leu His Glu Gln Glu Gly Ile Leu Tyr Ala
   260 265 270
   Glu Leu Glu Arg Asn Gln Met Thr Gly Pro Arg Trp Met Phe Asp Ala
   275 280 285
   Ala Gly His Tyr Ala Arg Pro Asp Val Phe Gln Leu Thr Val Asn Arg
   290 295 300
   Ser Pro Arg Pro Met Leu Arg Glu Ala Gly Ala Lys Thr Ser Glu Ala
   305 310 315 320
   Asn Thr Arg Asp Ala Val Pro Met Asp Ser Thr Pro Ser Arg Ser Arg
   325 330 335
   Pro Arg Ala Val Ala Arg Lys Ala Ala Arg Thr Gly Arg Ser Lys Arg
   340 345 350
   Arg
<210> 89
   <211> 918
   <212> DNA
   <213> Unknown
<220>
   <223> Obtained from an environmental sample
<400> 89
   atgaatacca aagaagtaaa ggtcgcagcc gctcaatttg ccccacattt tctgaatttg      60
   agcaaaacgg tggaaaaaac ctgcaacttg atttccgaag caggcaaaaa tggagcaaag     120
   ctcattgtat ttccggaagc cttcctctct ggttatcccg attgggtctg gttaattccc     180
   aatggaaatt caacaatgct ggatgattta tatcaggaat tggttgagaa cgctgtaaca     240
   atccctgatt caacaacaca gaaactctgt caggcagcaa aagatgccgg ggtatatgtc     300
   gcagtcggta tccatgaaag aaatgcagaa gcaagtggct tcacactttt caataccctt     360
   ctatacatta atgatcaagg cagcatcatt ggaaaacacc gaaaactgat cccaacaggg     420
   ggcgaacgcc tggtctgggg gcagggtaat ggggatacgc ttgctgcatt cgatacacac     480
   tttggcaaat tgggaggatt gctttgctgg gaaaactaca tgcccctggc tcggcaagct     540
   atgtacgcag ttgggactga agtttatgtt gccccaacct gggactccag tgagaattgg     600
   ttgctgagta tgcgccatat agccagagag ggcggcatgt ttgtgatcaa tgtttgccag     660
   gctgtccgaa aagacgatat tcctgaccgc tatgcattca agcaactcta ttctggtaat     720
   tcagaatgga tcaatagcgg caacagttgc atcatcaatc cgcgcggtga aatcattgcc     780
   ggaccatcct caaacaggca agaaatactc tacgcagatt tagatctgag tttgattaca     840
   aaatctaaac gcatgttcga tgttaccggg cattatgccc ggccggatgt gtttagatat     900
   gaaatcaaaa aaagctag                                                   918
<210> 90
   <211> 305
   <212> PRT
   <213> Unknown
<220>
   <223> Obtained from an environmental sample
<400> 90
   Met Asn Thr Lys Glu Val Lys Val Ala Ala Ala Gln Phe Ala Pro His
   1               5                   10                  15
   Phe Leu Asn Leu Ser Lys Thr Val Glu Lys Thr Cys Asn Leu Ile Ser
               20                  25                  30
   Glu Ala Gly Lys Asn Gly Ala Lys Leu Ile Val Phe Pro Glu Ala Phe
           35                  40                  45
   Leu Ser Gly Tyr Pro Asp Trp Val Trp Leu Ile Pro Asn Gly Asn Ser
       50                  55                  60
   Thr Met Leu Asp Asp Leu Tyr Gln Glu Leu Val Glu Asn Ala Val Thr
   65                  70                  75                  80
   Ile Pro Asp Ser Thr Thr Gln Lys Leu Cys Gln Ala Ala Lys Asp Ala
                   85                  90                  95
   Gly Val Tyr Val Ala Val Gly Ile His Glu Arg Asn Ala Glu Ala Ser
               100                 105                 110
   Gly Phe Thr Leu Phe Asn Thr Leu Leu Tyr Ile Asn Asp Gln Gly Ser
           115                 120                 125
   Ile Ile Gly Lys His Arg Lys Leu Ile Pro Thr Gly Gly Glu Arg Leu
       130                 135                 140
   Val Trp Gly Gln Gly Asn Gly Asp Thr Leu Ala Ala Phe Asp Thr His
   145                 150                 155                 160
   Phe Gly Lys Leu Gly Gly Leu Leu Cys Trp Glu Asn Tyr Met Pro Leu
                   165                 170                 175
   Ala Arg Gln Ala Met Tyr Ala Val Gly Thr Glu Val Tyr Val Ala Pro
               180                 185                 190
   Thr Trp Asp Ser Ser Glu Asn Trp Leu Leu Ser Met Arg His Ile Ala
           195                 200                 205
   Arg Glu Gly Gly Met Phe Val Ile Asn Val Cys Gln Ala Val Arg Lys
       210                 215                 220
   Asp Asp Ile Pro Asp Arg Tyr Ala Phe Lys Gln Leu Tyr Ser Gly Asn
   225                 230                 235                 240
   Ser Glu Trp Ile Asn Ser Gly Asn Ser Cys Ile Ile Asn Pro Arg Gly
                   245                 250                 255
   Glu Ile Ile Ala Gly Pro Ser Ser Asn Arg Gln Glu Ile Leu Tyr Ala
               260                 265                 270
   Asp Leu Asp Leu Ser Leu Ile Thr Lys Ser Lys Arg Met Phe Asp Val
           275                 280                 285
   Thr Gly His Tyr Ala Arg Pro Asp Val Phe Arg Tyr Glu Ile Lys Lys
       290                 295                 300
   Ser
   305
<210> 91
   <211> 939
   <212> DNA
   <213> Unknown
<220>
   <223> obtained from an environmental sample
<400> 91
   atgaccaaaa tcgctgtcat tcaagaacct ccggtctatc tgaatctgag taaatcgatg     60
   gacagagcgg tcgacttgat tgccaatgct gcaagcaagg ggtgtgagtt gattgtgttt    120
   cccgaagcct ggcttgcagg ttaccccacc ttcgtctggc gtcttgcgcc gggcagcgga    180
   atgggaaaaa ctgatgagct ttacgcgcgt ttgctcgcca actcggtcga ccgtagcaaa    240
   gaggggctta gaccattgca ggaggccgca aaggagcatg gcgttgtcat tgtgctgggt    300
   tatcaagagg tggatggcgc gggaagcagc agcacgatct tcaacagctg tgcgattatt    360
   gatgcggacg ggcgactggc caacaatcat cgcaagttga tgcccaccaa tccggagagg    420
   atggtttggg gttttggcga cggttcaggc ctgaacgtcg ttgacaccgc ggtgggcagg    480
   atcggcacgc tgatttgctg ggaaaactac atgccgttag cgcgctacgc gctgtatgtc    540
   caaaacatcg aaatctatgt tgccccgact tgggacagtg gtgccatgtg gcaggcgacc    600
   ctgcagcata tcgcgcgcga aggtggctgc tgggtcatcg gatgtgcaac gtcgctggaa    660
   gcctctgaca tcccggacga cgttccccat cgggatgagc tattcccgaa caaagacgaa    720
   tgggtaaacc ctggcgatgc ggtggtttat aagccatttg gcggcattgt ggccggcccc    780
   atgcatcagg aaaaggggct tctcatcgca gagttggacg tcgccgctgt tcagtcgtca    840
   cgtcggaagt tcgatgcgag cgggcactac gctcgccccg atgtcttcaa actgcatgtg    900
   aatcgcaccg cgatgcggcc agttgatttc acgaattag                           939
<210> 92
   <211> 312
   <212> PRT
   <213> Unknown
<220>
   <223> Obtained from an environmental sample
<400> 92
   Met Thr Lys Ile Ala Val Ile Gln Glu Pro Pro Val Tyr Leu Asn Leu
   1               5                   10                  15
   Ser Lys Ser Met Asp Arg Ala Val Asp Leu Ile Ala Asn Ala Ala Ser
               20                  25                  30
   Lys Gly Cys Glu Leu Ile Val Phe Pro Glu Ala Trp Leu Ala Gly Tyr
           35                  40                  45
   Pro Thr Phe Val Trp Arg Leu Ala Pro Gly Ser Gly Met Gly Lys Thr
       50                  55                  60
   Asp Glu Leu Tyr Ala Arg Leu Leu Ala Asn Ser Val Asp Arg Ser Lys
   65                  70                  75                  80
   Glu Gly Leu Arg Pro Leu Gln Glu Ala Ala Lys Glu His Gly Val Val
                   85                  90                  95
   Ile Val Leu Gly Tyr Gln Glu Val Asp Gly Ala Gly Ser Ser Ser Thr
               100                 105                 110
   Ile Phe Asn Ser Cys Ala Ile Ile Asp Ala Asp Gly Arg Leu Ala Asn
           115                 120                 125
   Asn His Arg Lys Leu Met Pro Thr Asn Pro Glu Arg Met Val Trp Gly
       130                 135                 140
   Phe Gly Asp Gly Ser Gly Leu Asn Val Val Asp Thr Ala Val Gly Arg
   145                 150                 155                 160
   Ile Gly Thr Leu Ile Cys Trp Glu Asn Tyr Met Pro Leu Ala Arg Tyr
                   165                 170                 175
   Ala Leu Tyr Val Gln Asn Ile Glu Ile Tyr Val Ala Pro Thr Trp Asp
               180                 185                 190
   Ser Gly Ala Met Trp Gln Ala Thr Leu Gln His Ile Ala Arg Glu Gly
           195                 200                 205
   Gly Cys Trp Val Ile Gly Cys Ala Thr Ser Leu Glu Ala Ser Asp Ile
       210                 215                 220
   Pro Asp Asp Val Pro His Arg Asp Glu Leu Phe Pro Asn Lys Asp Glu
   225                 230                 235                 240
   Trp Val Asn Pro Gly Asp Ala Val Val Tyr Lys Pro Phe Gly Gly Ile
                   245                 250                 255
   Val Ala Gly Pro Met His Gln Glu Lys Gly Leu Leu Ile Ala Glu Leu
               260                 265                 270
   Asp Val Ala Ala Val Gln Ser Ser Arg Arg Lys Phe Asp Ala Ser Gly
           275                 280                 285
   His Tyr Ala Arg Pro Asp Val Phe Lys Leu His Val Asn Arg Thr Ala
       290                 295                 300
   Met Arg Pro Val Asp Phe Thr Asn
   305                 310
<210> 93
   <211> 978
   <212> DNA
   <213> Unknown
<220>
   <223> Obtained from an environmental sample
<400> 93
   atgcccatca tcaaagccgc tgccgtgcaa atcagcccgg tgctttacag tcgcgaaggc      60
   accgtggaca aggtctgtca acagatcatc gacctcggtc ggcaaggcgt gcagttcgcc     120
   gtctttccgg aaacggtggt gccttactac ccgtactttt cgtttgtgca gccggccttt     180
   gccatgggcg cacagcacct caagttgctg gatcaatcgg tgacagtgcc gtcggccgcc     240
   accttggcca tcggtgaagc ttgcaagcaa gcagggatag tggtgtccat cggcgtcaac     300
   gaacgcgatg gcggtacgat ctacaacgcg caattactct tcgatgccga cggcagcctg     360
   attcagcatc gccgcaaaat caccccgacc tatcacgaac gcatggtctg ggggcaaggc      420
   gatggttccg gcctgcgcgc catcgacagt gcagtggggc gcattggctc cctggcctgt     480
   tgggagcatt acaacccgct ggctcgttat gccttgatgg ccgatggcga gcagatccac     540
   gccgcgatgt ttcccggctc gctggtgggc gacatttttg ccgagcagat cgaagtcacc     600
   atccgccatc acgccttgga gtccggctgt ttcgtggtca acgccaccgc ctggctggac     660
   gccgatcagc agggccaaat catgcaagac accggttgca gcctcggccc gatctcgggt     720
   ggctgcttca ccgccatcgt ttcccctgaa ggcaagttgc tcggtgagcc gctgcgttcc     780
   ggcgaagggg tggtgatcgc cgatctcgat ctggcactga tcgataagcg taaacggatg     840
   atggattcgg tcgggcatta cagtcgcccg gaactgctca gcctgttgat cgaccgcacg     900
   cccacagcgc atgtgcatga acgcagcgcg cacctggtgg ctgtcgctac cgaggagttc     960
   gatcatgcaa accaatga                                                   978
<210> 94
   <211> 325
   <212> PRT
   <213> Unknown
<220>
   <223> Obtained from an environmental sample
<400> 94
   Met Pro Ile Ile Lys Ala Ala Ala Val Gln Ile Ser Pro Val Leu Tyr
   1               5                   10                  15
   Ser Arg Glu Gly Thr Val Asp Lys Val Cys Gln Gln Ile Ile Asp Leu
               20                  25                  30
   Gly Arg Gln Gly Val Gln Phe Ala Val Phe Pro Glu Thr Val Val Pro
           35                  40                  45
   Tyr Tyr Pro Tyr Phe Ser Phe Val Gln Pro Ala Phe Ala Met Gly Ala
       50                  55                  60
   Gln His Leu Lys Leu Leu Asp Gln Ser Val Thr Val Pro Ser Ala Ala
   65                  70                  75                  80
   Thr Leu Ala Ile Gly Glu Ala Cys Lys Gln Ala Gly Ile Val Val Ser
                   85                  90                  95
   Ile Gly Val Asn Glu Arg Asp Gly Gly Thr Ile Tyr Asn Ala Gln Leu
               100                 105                 110
   Leu Phe Asp Ala Asp Gly Ser Leu Ile Gln His Arg Arg Lys Ile Thr
           115                 120                 125
   Pro Thr Tyr His Glu Arg Met Val Trp Gly Gln Gly Asp Gly Ser Gly
       130                 135                 140
   Leu Arg Ala Ile Asp Ser Ala Val Gly Arg Ile Gly Ser Leu Ala Cys
   145                 150                 155                 160
   Trp Glu His Tyr Asn Pro Leu Ala Arg Tyr Ala Leu Met Ala Asp Gly
                   165                 170                 175
   Glu Gln Ile His Ala Ala Met Phe Pro Gly Ser Leu Val Gly Asp Ile
               180                 185                 190
   Phe Ala Glu Gln Ile Glu Val Thr Ile Arg His His Ala Leu Glu Ser
           195                 200                 205
   Gly Cys Phe Val Val Asn Ala Thr Ala Trp Leu Asp Ala Asp Gln Gln
       210                 215                 220
   Gly Gln Ile Met Gln Asp Thr Gly Cys Ser Leu Gly Pro Ile Ser Gly
   225                 230                 235                 240
   Gly Cys Phe Thr Ala Ile Val Ser Pro Glu Gly Lys Leu Leu Gly Glu
                   245                 250                 255
   Pro Leu Arg Ser Gly Glu Gly Val Val Ile Ala Asp Leu Asp Leu Ala
               260                 265                 270
   Leu Ile Asp Lys Arg Lys Arg Met Met Asp Ser Val Gly His Tyr Ser
           275                 280                 285
   Arg Pro Glu Leu Leu Ser Leu Leu Ile Asp Arg Thr Pro Thr Ala His
       290                 295                 300
   Val His Glu Arg Ser Ala His Leu Val Ala Val Ala Thr Glu Glu Phe
   305                 310                 315                 320
   Asp His Ala Asn Gln
                   325
<210> 95
   <211> 966
   <212> DNA
   <213> Unknown
<220>
   <223> Obtained from an environmental sample
<400> 95
   atgtccaacg agaataacat tgctacattc aaagttgccg cagtccaggc cacacctgtg      60
   tttcttgatc gtgaagcaac catcgacaaa gcttgcgcgt tgattgccac tgctggcagt     120
   gaaggagcgc gcctgattgt gtttccagaa gcattcatcc caacttatcc tgaatgggta     180
   tggggtattc cctccggtga gcaaggttta ctcaacgaac tctatgcaga gttgctcacc     240
   aatgcggtca ccattcccag cgatgcgact gacaggctgt gcgaggctgc gcagcttgcg     300
   aatgcctacg tagtgatggg catgagcgaa cggaacgtcg aggcgagtgg cgcaagcctg     360
   tataatacgc tgttgtacat aaatgcgcag ggggagattt tagggaaaca tcgaaagctg      420
   gtgccaacgg gcggcgaacg cctggtatgg gcgcagggtg atggcagtac gctgcaggtc     480
   tacgatactc cattgggaaa actcggtggc ttaatttgct gggaaaatta tatgccgctg     540
   gcacggtatg ctatgtatgc ctggggaaca caaatctatg tcgcggcaac gtgggatcgc     600
   ggtcaaccct ggctttctac attaaggcat atcgccaaag aaggcagggt atacgtgatt     660
   ggttgctgta tcgcgatgcg taaagacgat attccagatc gttacaccat gaagcaaaaa     720
   tattatgctg aaatggatga atggatgaat gttggtgaca gtgtgattgt caatcccgag     780
   gggcacttta ttgccgggcc tgtgcgcaag caggaagaaa ttctctacgc ggagattgat     840
   cctcgcatgg tgcaaggccc gaagtggatg ctcgatgtgg cagggcatta tgcgagaccg     900
   gatgtgttcc agttgacggt gcatacggat gtgaggcgga tgatgcgggt ggaagatgat     960
   tcataa                                                                966
<210> 96
   <211> 321
   <212> PRT
   <213> Unknown
<220>
   <223> Obtained from an environmental sample
<400> 96
   Met Ser Asn Glu Asn Asn Ile Ala Thr Phe Lys Val Ala Ala Val Gln
   1               5                   10                  15
   Ala Thr Pro Val Phe Leu Asp Arg Glu Ala Thr Ile Asp Lys Ala Cys
               20                  25                  30
   Ala Leu Ile Ala Thr Ala Gly Ser Glu Gly Ala Arg Leu Ile Val Phe
           35                  40                  45
   Pro Glu Ala Phe Ile Pro Thr Tyr Pro Glu Trp Val Trp Gly Ile Pro
       50                  55                  60
   Ser Gly Glu Gln Gly Leu Leu Asn Glu Leu Tyr Ala Glu Leu Leu Thr
   65                  70                  75                  80
   Asn Ala Val Thr Ile Pro Ser Asp Ala Thr Asp Arg Leu Cys Glu Ala
                   85                  90                  95
   Ala Gln Leu Ala Asn Ala Tyr Val Val Met Gly Met Ser Glu Arg Asn
               100                 105                 110
   Val Glu Ala Ser Gly Ala Ser Leu Tyr Asn Thr Leu Leu Tyr Ile Asn
           115                 120                 125
   Ala Gln Gly Glu Ile Leu Gly Lys His Arg Lys Leu Val Pro Thr Gly
       130                 135                 140
   Gly Glu Arg Leu Val Trp Ala Gln Gly Asp Gly Ser Thr Leu Gln Val
   145                 150                 155                 160
   Tyr Asp Thr Pro Leu Gly Lys Leu Gly Gly Leu Ile Cys Trp Glu Asn
                   165                 170                 175
   Tyr Met Pro Leu Ala Arg Tyr Ala Met Tyr Ala Trp Gly Thr Gln Ile
               180                 185                 190
   Tyr Val Ala Ala Thr Trp Asp Arg Gly Gln Pro Trp Leu Ser Thr Leu
           195                 200                 205
   Arg His Ile Ala Lys Glu Gly Arg Val Tyr Val Ile Gly Cys Cys Ile
       210                 215                 220
   Ala Met Arg Lys Asp Asp Ile Pro Asp Arg Tyr Thr Met Lys Gln Lys
   225                 230                 235                 240
   Tyr Tyr Ala Glu Met Asp Glu Trp Met Asn Val Gly Asp Ser Val Ile
                   245                 250                 255
   Val Asn Pro Glu Gly His Phe Ile Ala Gly Pro Val Arg Lys Gln Glu
               260                 265                 270
   Glu Ile Leu Tyr Ala Glu Ile Asp Pro Arg Met Val Gln Gly Pro Lys
           275                 280                 285
   Trp Met Leu Asp Val Ala Gly His Tyr Ala Arg Pro Asp Val Phe Gln
       290                 295                 300
   Leu Thr Val His Thr Asp Val Arg Arg Met Met Arg Val Glu Asp Asp
   305                 310                 315                 320
   Ser
<210> 97
   <211> 1017
   <212> DNA
   <213> Unknown
<220>
   <223> Obtained from an environmental sample
<400> 97
   atgggcatcg aacatccgaa atacaaggtc gccgtggtgc aagctgcgcc cgcctggctc      60
   gacctcgacg cgtcgatcga caagacgatc gggctgatcg aggaggcggc gaagaaaggc     120
   gccaagctga tcgctttccc cgaagccttc attcccggct acccttggca catctggctc     180
   gactcacccg cctgggcgat cggccgcggt ttcgtgcagc gctatttcga caattcgctc     240
   gcctacgaca gcccacaggc ggaaaggctg cgacaggccg tgcggaaggc caagctcacc     300
   gccgtgatcg gcctgtccga gcgcgacggc ggcagcctct atctcgcgca gtggctgatc     360
   gggcccgacg gtgagaccat cgcaaagcgc cgcaagctgc ggccgaccca tgccgagcgc     420
   accgtctatg gcgaaggcga cggcagcgat ctcgccgtcc atgagcgggc cgacatcggc     480
   cggctcggcg cgctgtgctg ctgggagcat ctgcagccgc tgtcgaaatt cgccatgtac     540
   gcccagaacg agcaggtaca tgtcgcggcc tggccgagct tctcgctcta cgatcccttc     600
   gcgcctgcgc tgggcgcgga ggtgaacaac gccgcctccc gcatctatgc ggtggaaggc     660
   tcctgcttcg tgctcgcacc gtgcgcgacg gtctcgcagg ccatgatcga cgagctctgc     720
   gatcggccgg acaagcacgc gctgctgcat gccggcggcg gcttcgccgc gatctacggg     780
   cccgacggca gccagatcgg cgacaagctg ccgcccgagc aggagggcct gctgatcgcc     840
   gagatcgatc tgggcgcgat cggcgtcgcc aagaacgcgg ccgatcccgc cgggcattat     900
   tcgcggcccg acgtcacgcg gctcctgctc aacaggaagc cgaacaagcg cgtggagcag     960
   ttcgcgctgc ccgtcgacac ggtcgagccc gtcgacgtcg cggcggcagc aagctga       1017
<210> 98
   <211> 338
   <212> PRT
   <213> Unknown
<220>
   <223> Obtained from an environmental sample
<400> 98
   Met Gly Ile Glu His Pro Lys Tyr Lys Val Ala Val Val Gln Ala Ala
   1               5                   10                  15
   Pro Ala Trp Leu Asp Leu Asp Ala Ser Ile Asp Lys Thr Ile Gly Leu
               20                  25                  30
   Ile Glu Glu Ala Ala Lys Lys Gly Ala Lys Leu Ile Ala Phe Pro Glu
           35                  40                  45
   Ala Phe Ile Pro Gly Tyr Pro Trp His Ile Trp Leu Asp Ser Pro Ala
       50                  55                  60
   Trp Ala Ile Gly Arg Gly Phe Val Gln Arg Tyr Phe Asp Asn Ser Leu
   65                  70                  75                  80
   Ala Tyr Asp Ser Pro Gln Ala Glu Arg Leu Arg Gln Ala Val Arg Lys
                   85                  90                  95
   Ala Lys Leu Thr Ala Val Ile Gly Leu Ser Glu Arg Asp Gly Gly Ser
               100                 105                 110
   Leu Tyr Leu Ala Gln Trp Leu Ile Gly Pro Asp Gly Glu Thr Ile Ala
           115                 120                 125
   Lys Arg Arg Lys Leu Arg Pro Thr His Ala Glu Arg Thr Val Tyr Gly
       130                 135                 140
   Glu Gly Asp Gly Ser Asp Leu Ala Val His Glu Arg Ala Asp Ile Gly
   145                 150                 155                 160
   Arg Leu Gly Ala Leu Cys Cys Trp Glu His Leu Gln Pro Leu Ser Lys
                   165                 170                 175
   Phe Ala Met Tyr Ala Gln Asn Glu Gln Val His Val Ala Ala Trp Pro
               180                 185                 190
   Ser Phe Ser Leu Tyr Asp Pro Phe Ala Pro Ala Leu Gly Ala Glu Val
           195                 200                 205
   Asn Asn Ala Ala Ser Arg Ile Tyr Ala Val Glu Gly Ser Cys Phe Val
       210                 215                 220
   Leu Ala Pro Cys Ala Thr Val Ser Gln Ala Met Ile Asp Glu Leu Cys
   225                 230                 235                 240
   Asp Arg Pro Asp Lys His Ala Leu Leu His Ala Gly Gly Gly Phe Ala
                   245                 250                 255
   Ala Ile Tyr Gly Pro Asp Gly Ser Gln Ile Gly Asp Lys Leu Pro Pro
               260                 265                 270
   Glu Gln Glu Gly Leu Leu Ile Ala Glu Ile Asp Leu Gly Ala Ile Gly
           275                 280                 285
   Val Ala Lys Asn Ala Ala Asp Pro Ala Gly His Tyr Ser Arg Pro Asp
       290                 295                 300
   Val Thr Arg Leu Leu Leu Asn Arg Lys Pro Asn Lys Arg Val Glu Gln
   305                 310                 315                 320
   Phe Ala Leu Pro Val Asp Thr Val Glu Pro Val Asp Val Ala Ala Ala
                   325                 330                 335
   Ala Ser
<210> 99
   <211> 1014
   <212> DNA
   <213> Unknown
<220>
   <223> Obtained from an environmental sample
<400> 99
   atgcctgaca agagaatcgt ccgcgccgcc gcggtccaga tagcaccgga cctcgaacgg      60
   cccggtggca cgctcgagaa ggtcctcgag acgatcgacg acgccgcacg ccagggcgtg     120
   cagctcatcg tcttccccga gaccttcctg ccctactacc cgtacttttc gttcgtgcgg     180
   gcgccggtgg catcgggtgc agagcacatg cggctctatg acgaagcggt ggtcgtgccc     240
   gggccggtga cgcatgcggt ggccgagcgg gcacggcggc acggcatggt cgtcgtgctc     300
   ggcgtgaacg agcgcgatca cggcagctta tacaacgcac aactgatctt cgataccgac     360
   ggcgagctgc tgctcaagcg ccgcaagatc acgccgacgt ttcacgaacg gatgatctgg     420
   ggcatgggcg acgcagccgg cctgaaggta gcggaaacgc gtatcggccg ggtgggtgca     480
   ctcgcttgct gggaacacta caacccgctt gcacgttatg cactgatgac ccagcacgaa     540
   gagattcatt gcagccagtt tcccggctcg ctggtcggac ccatcttcgg tgaacagatc     600
   gaagtgacca tccggcatca cgcactggaa tccggctgct tcgtgatcaa ttccaccggc     660
   tggctgaccg agccgcagat cgagtcgatc acgaaagatc cgggcctgca gaaggcgctt     720
   cgcggcggct gcaacacggc gatcatctcg cccgaaggcc agcatctcgc cccgccgctg     780
   cgtgagggcg agggcatggt catcgctgac ctggacatgt cgctgatcac caaacgcaaa     840
   cgcatgatgg attctgtcgg ccactacgcg cggcccgaac tgctgagcct cgccatcaac     900
   gaccggccgg cggtcacgtc ggcacccatg aacagcttct catcttcaac cgggggattg     960
   caccttgaac gcgaacgaga ccttgtcggc cgtgagccgg caattgatga ctga          1014
<210> 100
   <211> 337
   <212> PRT
   <213> Unknown
<220>
   <223> Obtained from an environmental sample
<400> 100
   Met Pro Asp Lys Arg Ile Val Arg Ala Ala Ala Val Gln Ile Ala Pro
   1               5                   10                  15
   Asp Leu Glu Arg Pro Gly Gly Thr Leu Glu Lys Val Leu Glu Thr Ile
               20                  25                  30
   Asp Asp Ala Ala Arg Gln Gly Val Gln Leu Ile Val Phe Pro Glu Thr
           35                  40                  45
   Phe Leu Pro Tyr Tyr Pro Tyr Phe Ser Phe Val Arg Ala Pro Val Ala
       50                  55                  60
   Ser Gly Ala Glu His Met Arg Leu Tyr Asp Glu Ala Val Val Val Pro
   65                  70                  75                  80
   Gly Pro Val Thr His Ala Val Ala Glu Arg Ala Arg Arg His Gly Met
                   85                  90                  95
   Val Val Val Leu Gly Val Asn Glu Arg Asp His Gly Ser Leu Tyr Asn
               100                 105                 110
   Ala Gln Leu Ile Phe Asp Thr Asp Gly Glu Leu Leu Leu Lys Arg Arg
           115                 120                 125
   Lys Ile Thr Pro Thr Phe His Glu Arg Met Ile Trp Gly Met Gly Asp
       130                 135                 140
   Ala Ala Gly Leu Lys Val Ala Glu Thr Arg Ile Gly Arg Val Gly Ala
   145                 150                 155                 160
   Leu Ala Cys Trp Glu His Tyr Asn Pro Leu Ala Arg Tyr Ala Leu Met
                   165                 170                 175
   Thr Gln His Glu Glu Ile His Cys Ser Gln Phe Pro Gly Ser Leu Val
               180                 185                 190
   Gly Pro Ile Phe Gly Glu Gln Ile Glu Val Thr Ile Arg His His Ala
           195                 200                 205
   Leu Glu Ser Gly Cys Phe Val Ile Asn Ser Thr Gly Trp Leu Thr Glu
       210                 215                 220
   Pro Gln Ile Glu Ser Ile Thr Lys Asp Pro Gly Leu Gln Lys Ala Leu
   225                 230                 235                 240
   Arg Gly Gly Cys Asn Thr Ala Ile Ile Ser Pro Glu Gly Gln His Leu
                   245                 250                 255
   Ala Pro Pro Leu Arg Glu Gly Glu Gly Met Val Ile Ala Asp Leu Asp
               260                 265                 270
   Met Ser Leu Ile Thr Lys Arg Lys Arg Met Met Asp Ser Val Gly His
           275                 280                 285
   Tyr Ala Arg Pro Glu Leu Leu Ser Leu Ala Ile Asn Asp Arg Pro Ala
       290                 295                 300
   Val Thr Ser Ala Pro Met Asn Ser Phe Ser Ser Ser Thr Gly Gly Leu
   305                 310                 315                 320
   His Leu Glu Arg Glu Arg Asp Leu Val Gly Arg Glu Pro Ala Ile Asp
                   325                 330                 335
   Asp
<210> 101
   <211> 1065
   <212> DNA
   <213> Unknown
<220>
   <223> Obtained from an environmental sample
<400> 101
   atggcgacag tccatccgaa atttaaagta gccgccgtcc aggcggcccc ggcctttctc      60
   gacctcgacg cgtcggtgga aaaagcggtg cgcctgattg atgaagccgg cgccgctggt     120
   gcccggctca tcgcgtttcc agagactttt atccccggtt atccgtggtg gatctggctc     180
   ggtgctccgg cctgggcgat catgcgcggc ttcgtctccc gctatttcga caactcgctg     240
   cagtacggca ccccggaagc cgaccggctg cgggcagccg ccaaacgcaa caaaatgttc     300
   gtcgcgctcg gactgtcaga gcgcgacggc ggcagtctct acatcgccca atggattatc     360
   ggacccgacg gcgagacggt cgcaacgcgc cgcaagctca agcctactca cgccgagcgg     420
   acggtgttcg gcgaaggcga tggctcgcac cttgcggtcc acgaacttga tatcgggcgg     480
   gtcggtgcgc tgtgctgttg ggagcacctg cagccactgt cgaagtacgc gatgtatgcg     540
   cagaacgagc aagttcatat cgcggcgtgg ccgagctttt cgctttacga tccgttcgcg     600
   catgcgcttg gcgccgaggt caacaacgcg gcgagcaaga tctacgcggt cgaaggctca     660
   tgctttgtga ttgcgccatg cgcgaccgtt tcccaggcga tgatcgacga attgtgtgac     720
   tcgcccgaga agcatcagtt cctgcacgtc ggcggcggtt tcgccgtgat ctatggtccc     780
   gacggcgcgc cactcgccaa gccactggcg cccgatcagg agggtctcct ttacgcggat     840
   atcgacctcg gcatgatttc ggtcgcgaaa gcggcggccg atccggctgg acattacgcg     900
   cgcccggacg tgacccgtct gttgttcaac aatcgtcctg ggaaccgggt ggagacactc     960
   gcgctgccgg tcgaccagga ggcagaggcg ggagcaggcg gcaaacctgc gcccaagtca    1020
   ccgagtgtcg ctgcgttcac actgacgcag gcggcagccg agtag                    1065
<210> 102
   <211> 354
   <212> PRT
   <213> Unknown
<220>
   <223> Obtained from an environmental sample
<400> 102
   Met Ala Thr Val His Pro Lys Phe Lys Val Ala Ala Val Gln Ala Ala
   1               5                   10                  15
   Pro Ala Phe Leu Asp Leu Asp Ala Ser Val Glu Lys Ala Val Arg Leu
               20                  25                  30
   Ile Asp Glu Ala Gly Ala Ala Gly Ala Arg Leu Ile Ala Phe Pro Glu
           35                  40                  45
   Thr Phe Ile Pro Gly Tyr Pro Trp Trp Ile Trp Leu Gly Ala Pro Ala
       50                  55                  60
   Trp Ala Ile Met Arg Gly Phe Val Ser Arg Tyr Phe Asp Asn Ser Leu
   65                  70                  75                  80
   Gln Tyr Gly Thr Pro Glu Ala Asp Arg Leu Arg Ala Ala Ala Lys Arg
                   85                  90                  95
   Asn Lys Met Phe Val Ala Leu Gly Leu Ser Glu Arg Asp Gly Gly Ser
               100                 105                 110
   Leu Tyr Ile Ala Gln Trp Ile Ile Gly Pro Asp Gly Glu Thr Val Ala
           115                 120                 125
   Thr Arg Arg Lys Leu Lys Pro Thr His Ala Glu Arg Thr Val Phe Gly
       130                 135                 140
   Glu Gly Asp Gly Ser His Leu Ala Val His Glu Leu Asp Ile Gly Arg
   145                 150                 155                 160
   Val Gly Ala Leu Cys Cys Trp Glu His Leu Gln Pro Leu Ser Lys Tyr
                   165                 170                 175
   Ala Met Tyr Ala Gln Asn Glu Gln Val His Ile Ala Ala Trp Pro Ser
               180                 185                 190
   Phe Ser Leu Tyr Asp Pro Phe Ala His Ala Leu Gly Ala Glu Val Asn
           195                 200                 205
   Asn Ala Ala Ser Lys Ile Tyr Ala Val Glu Gly Ser Cys Phe Val Ile
       210                 215                 220
   Ala Pro Cys Ala Thr Val Ser Gln Ala Met Ile Asp Glu Leu Cys Asp
   225                 230                 235                 240
   Ser Pro Glu Lys His Gln Phe Leu His Val Gly Gly Gly Phe Ala Val
                   245                 250                 255
   Ile Tyr Gly Pro Asp Gly Ala Pro Leu Ala Lys Pro Leu Ala Pro Asp
               260                 265                 270
   Gln Glu Gly Leu Leu Tyr Ala Asp Ile Asp Leu Gly Met Ile Ser Val
           275                 280                 285
   Ala Lys Ala Ala Ala Asp Pro Ala Gly His Tyr Ala Arg Pro Asp Val
       290                 295                 300
   Thr Arg Leu Leu Phe Asn Asn Arg Pro Gly Asn Arg Val Glu Thr Leu
   305                 310                 315                 320
   Ala Leu Pro Val Asp Gln Glu Ala Glu Ala Gly Ala Gly Gly Lys Pro
                   325                 330                 335
   Ala Pro Lys Ser Pro Ser Val Ala Ala Phe Thr Leu Thr Gln Ala Ala
               340                 345                 350
   Ala Glu
<210> 103
   <211> 945
   <212> DNA
   <213> Unknown
<220>
   <223> Obtained from an environmental sample
<400> 103
   atgggcgagt tcggcgaggt gacgctgggg gtggcgcagg cggcgccggt gtacttcgac      60
   cgggaggcgt cgacggagaa ggctcgcggc ctgatccggg aggcggggga gaagggcgtc     120
   gacctgttgg cgttcgggga gacgtggctg acggggtacc cgtactggaa ggatgcgccg     180
   tggtctcggg agtacaacga cctgcgcgcg cggtacgtgg cgaatggcgt gatgataccg     240
   gggccggaga cggacgcgct atgccaggca gcggcggaag cgggggtgga cgtggcaatc     300
   ggcgtggtgg agctggagcc ggggagcctt tcgagcgtgt attgcacgtt gctgttcatc     360
   tcgcgcgagg gcgagatcct ggggcggcac cggaagctga agccgacgga ttcggaacgg     420
   cggtactggt cagagggtga tgcgacgggg ctgcgggtgt acgagcggcc atatggccgg     480
   ttgagcggat tgaactgctg ggaacacctt atgatgttgc cggggtacgc gctggcggca     540
   caggggacgc agtttcatgt ggcagcgtgg ccgaacatgg cgagctcggc gagcgagctg     600
   ctgtcgcggg cgtatgcgta ccaggccgga tgctacgtgt tgtgcgcggg cggctcgggg     660
   cctgcgccgg gagagctacc ggacggcatc gcggcggagt cgctggacca cctgacgggc     720
   gagagctgca tcatcgaccc gtggggaaaa gtgatcgcgg ggccggtgtc gtgcgaggag     780
   acgctgatta cggcgcgggt atcgaccgcg tcaatctacc ggcgcaagtc gctgacggac     840
   gtgggtggcc actactcgcg accggacgtg ttccggttcg aggtggatag gtcggagcgc     900
   ccgcgagtgg tgtttcggga tggggatgtg gacgaccggg ggtaa                     945
<210> 104
   <211> 314
   <212> PRT
   <213> Unknown
<220>
   <223> obtained from an environmental sample
<210> 105
   <211> 975
   <212> DNA
   <213> Unknown
<220>
   <223> Obtained from an environmental sample
<400> 105
   atgaccattg tcaaagccgc tgccgtccag attgcccccg ttctctacag ccgtgaaggc      60
   actgtagaaa aggtcgttaa caagattcgc gaactcggcg agaagggcgt gcagttcgcc     120
   gttttccctg aaaccgtcgt accgtactac ccgtactttt cctttgtgca gagccctttc     180
   aaaatgggtt ccgagcacta caaattgctc gaccaggccg ttgtcgtgcc gtcggcgacc     240
   accgatgcca tcggcaaagc ggccaaggaa gccaacatgg tggtgtccat cggcgtcaac     300
   gaacgcgatg gcagcaccct ctacaacacg cagttgctgt ttgatgccga cggcactttg     360
   attcaggccc gtcgcaagat ttcaccgacc taccacgaac gcatgatctg gggcatgggc      420
   gacggttccg gcctgcgcgc caccgacagc gcggtcgggc gcatcggaca attggcctgc     480
   tgggaacatt acaatccgct ggcgcgttac gccttgatcg aagacggcga acagatccac     540
   gcctcgatgt acccgggctc gttcgcaggt cctttattca ctcgccagat ggaagtcagc     600
   atccgcatgc atgccctgga atcggcgtgc ttcgtggtca actcgaccgc gtggttgtac     660
   ccggaacagc aagcccagat catggccgac accggttgcg agatcgggcc gatctccggc     720
   ggctgctaca ccgcgatcat cgacccacag ggtgaagtcg tcggcgcact gaccgaaggc     780
   gagggcgaag tgattgccga catcgatctg ttccagatcg aaatccgtaa acgtcagatg     840
   gacggccgtg gtcactacag ccgtccggaa atcctgagcc tgaacatcga ccgtacgccg     900
   catcgccatg ttcacgaacg caacgaccag cagaaaccgg gtgtgatcga cactgctgaa     960
   gaaaccgggc gttga                                                      975
<210> 106
   <211> 324
   <212> PRT
   <213> Unknown
<220>
   <223> Obtained from an environmental sample
<400> 106
   Met Thr Ile Val Lys Ala Ala Ala Val Gln Ile Ala Pro Val Leu Tyr
   1               5                   10                  15
   Ser Arg Glu Gly Thr Val Glu Lys Val Val Asn Lys Ile Arg Glu Leu
               20                  25                  30
   Gly Glu Lys Gly Val Gln Phe Ala Val Phe Pro Glu Thr Val Val Pro
           35                  40                  45
   Tyr Tyr Pro Tyr Phe Ser Phe Val Gln Ser Pro Phe Lys Met Gly Ser
       50                  55                  60
   Glu His Tyr Lys Leu Leu Asp Gln Ala Val Val Val Pro Ser Ala Thr
   65                  70                  75                  80
   Thr Asp Ala Ile Gly Lys Ala Ala Lys Glu Ala Asn Met Val Val Ser
                   85                  90                  95
   Ile Gly Val Asn Glu Arg Asp Gly Ser Thr Leu Tyr Asn Thr Gln Leu
               100                 105                 110
   Leu Phe Asp Ala Asp Gly Thr Leu Ile Gln Ala Arg Arg Lys Ile Ser
           115                 120                 125
   Pro Thr Tyr His Glu Arg Met Ile Trp Gly Met Gly Asp Gly Ser Gly
       130                 135                 140
   Leu Arg Ala Thr Asp Ser Ala Val Gly Arg Ile Gly Gln Leu Ala Cys
   145                 150                 155                 160
   Trp Glu His Tyr Asn Pro Leu Ala Arg Tyr Ala Leu Ile Glu Asp Gly
                   165                 170                 175
   Glu Gln Ile His Ala Ser Met Tyr Pro Gly Ser Phe Ala Gly Pro Leu
               180                 185                 190
   Phe Thr Arg Gln Met Glu Val Ser Ile Arg Met His Ala Leu Glu Ser
           195                 200                 205
   Ala Cys Phe Val Val Asn Ser Thr Ala Trp Leu Tyr Pro Glu Gln Gln
       210                 215                 220
   Ala Gln Ile Met Ala Asp Thr Gly Cys Glu Ile Gly Pro Ile Ser Gly
   225                 230                 235                 240
   Gly Cys Tyr Thr Ala Ile Ile Asp Pro Gln Gly Glu Val Val Gly Ala
                   245                 250                 255
   Leu Thr Glu Gly Glu Gly Glu Val Ile Ala Asp Ile Asp Leu Phe Gln
               260                 265                 270
   Ile Glu Ile Arg Lys Arg Gln Met Asp Gly Arg Gly His Tyr Ser Arg
           275                 280                 285
   Pro Glu Ile Leu Ser Leu Asn Ile Asp Arg Thr Pro His Arg His Val
       290                 295                 300
   His Glu Arg Asn Asp Gln Gln Lys Pro Gly Val Ile Asp Thr Ala Glu
   305                 310                 315                 320
   Glu Thr Gly Arg
<210> 107
   <211> 981
   <212> DNA
   <213> Unknown
<220>
   <223> Obtained from an environmental sample
<400> 107
<210> 108
   <211> 326
   <212> PRT
   <213> Unknown
<220>
   <223> Obtained from an environmental sample
<400> 108
   Met Ala Ile Ile Arg Ala Ala Ala Val Gln Ile Ser Pro Val Leu Tyr
    1               5                  10                  15
   Ser Arg Glu Gly Thr Val Asp Lys Val Cys Gln Gln Ile Ile Thr Leu
               20                  25                  30
   Gly Lys Gln Gly Val Gln Phe Ala Val Phe Pro Glu Thr Val Val Pro
           35                  40                  45
   Tyr Tyr Pro Tyr Phe Ser Phe Val Gln Pro Ala Phe Ala Met Gly Ala
       50                  55                  60
   Gln His Leu Lys Leu Leu Asp Gln Ser Val Thr Val Pro Ser Ala Ala
   65                  70                  75                  80
   Thr Leu Ala Ile Gly Glu Ala Cys Lys Gln Ala Gly Met Val Val Ser
                   85                  90                  95
   Ile Gly Val Asn Glu Arg Asp Gly Gly Thr Ile Tyr Asn Ala Gln Leu
               100                 105                 110
   Leu Phe Asp Ala Asp Gly Thr Leu Ile Gln His Arg Arg Lys Ile Thr
           115                 120                 125
   Pro Thr Tyr His Glu Arg Met Val Trp Gly Gln Gly Asp Gly Ser Gly
       130                 135                 140
   Leu Arg Ala Ile Asp Ser Ala Val Gly Arg Ile Gly Ser Leu Ala Cys
   145                 150                 155                 160
   Trp Glu His Tyr Asn Pro Leu Ala Arg Tyr Ala Leu Met Ala Asp Gly
                   165                 170                 175
   Glu Gln Ile His Ala Ala Met Phe Pro Gly Ser Leu Val Gly Asp Ile
               180                 185                 190
   Phe Ala Glu Gln Ile Glu Val Thr Ile Arg His His Ala Leu Glu Ser
           195                 200                 205
   Gly Cys Phe Val Val Asn Ala Thr Ala Trp Leu Asp Ala Asp Gln Gln
       210                 215                 220
   Gly Gln Ile Met Gln Asp Thr Gly Cys Gly Leu Gly Pro Ile Ser Gly
   225                 230                 235                 240
   Gly Cys Phe Thr Ala Ile Val Ser Pro Glu Gly Lys Leu Leu Gly Glu
                   245                 250                 255
   Pro Leu Arg Ser Gly Glu Gly Val Val Ile Ala Asp Leu Asp Thr Ala
               260                 265                 270
   Leu Ile Asp Lys Arg Lys Arg Met Met Asp Ser Val Gly His Tyr Ser
           275                 280                 285
   Arg Pro Glu Leu Leu Ser Leu Leu Ile Asp Arg Ser Pro Thr Ala His
       290                 295                 300
   Val His Glu Arg Ala Gly Phe Val Ser Ser Asn Ala Gly Leu Gln Glu
   305                 310                 315                 320
   Val Ala His Ala Asp Gln
                   325
<210> 109
   <211> 1092
   <212> DNA
   <213> Unknown
<220>
   <223> Obtained from an environmental sample
<400> 109
   atggccatca ttcgcgcagc agccgtacag atcagcccgg ttctttacag ccgcgaaggc      60
   accgtggaca gggtctgcca gcagatcatc acccttggca aacaaggtgt gcagttcgcc     120
   gtgttcccgg aaacggtggt gccgtactac ccctattttt cctttgtgca gccggcattt     180
   gcgatgggtg cacaacacct caaattgctc gatcaatctg taaccgtgcc atcggccgcc     240
   accctggcga ttggcgaagc gtgcaagcaa gcaggaatgg tcgtttccat cggcgtcaat     300
   gaacgcgatg gcggtacgat ttacaacgcg caattactct tcgatgctga cggcactctg     360
   attcagcatc ggcgcaaaat caccccgacc taccacgagc gcatggtctg ggggcagggc      420
   gatggttccg gtctgcgcgc catcgacagc gcggtcgggc gcatcggctc cctggcatgc     480
   tgggaacatt acaacccgct ggcccgttac gccttgatgg cagacggcga acagatccac     540
   gccgcgatgt ttcccggttc cctggtgggt gacatcttcg ccgagcagat cgaggtcacc     600
   atccgccatc acgcattgga atcaggatgc ttcgtggtca atgcaacagc ttggctggat     660
   gcggatcagc agggccaaat aatgcaggac acaggttgcg gccttggtcc catctcgggc     720
   ggctgcttca ccgcgatcgt atcgccggaa gggaagctac ttggagagcc gcttcgctca     780
   ggggaaggcg tagtgattgc cgacctcgat atggccttga tcgacaagcg caaacggatg     840
   atggattcag taggtcatta cagtcgtccc gagctgctca gcctattgat cgatcgatcg     900
   ccgactgcgc attttcatga acgcgccggg ctttgttccg agcgacgccg gtttgcagga     960
   ggtcgcgcat gcagaccaat gaattgctcg ctgacctgca aatccaaggc ctgcgttggc    1020
   cggccgcgca aatggcttgt cgcgccaagg cggcgccggt ccttcagacc acaaggcgct    1080
   gagcctaggt aa                                                        1092
<210> 110
   <211> 363
   <212> PRT
   <213> Unknown
<220>
   <223> Obtained from an environmental sample
<400> 110
   Met Ala Ile Ile Arg Ala Ala Ala Val Gln Ile Ser Pro Val Leu Tyr
   1 5 10 15
   Ser Arg Glu Gly Thr Val Asp Arg Val Cys Gln Gln Ile Ile Thr Leu
   20 25 30
   Gly Lys Gln Gly Val Gln Phe Ala Val Phe Pro Glu Thr Val Val Pro
   35 40 45
   Tyr Tyr Pro Tyr Phe Ser Phe Val Gln Pro Ala Phe Ala Met Gly Ala
   50 55 60
   Gln His Leu Lys Leu Leu Asp Gln Ser Val Thr Val Pro Ser Ala Ala
   65 70 75 80
   Thr Leu Ala Ile Gly Glu Ala Cys Lys Gln Ala Gly Met Val Val Ser
   85 90 95
   Ile Gly Val Asn Glu Arg Asp Gly Gly Thr Ile Tyr Asn Ala Gln Leu
   100 105 110
   Leu Phe Asp Ala Asp Gly Thr Leu Ile Gln His Arg Arg Lys Ile Thr
   115 120 125
   Pro Thr Tyr His Glu Arg Met Val Trp Gly Gln Gly Asp Gly Ser Gly
   130 135 140
   Leu Arg Ala Ile Asp Ser Ala Val Gly Arg Ile Gly Ser Leu Ala Cys
   145 150 155 160
   Trp Glu His Tyr Asn Pro Leu Ala Arg Tyr Ala Leu Met Ala Asp Gly
   165 170 175
   Glu Gln Ile His Ala Ala Met Phe Pro Gly Ser Leu Val Gly Asp Ile
   180 185 190
   Phe Ala Glu Gln Ile Glu Val Thr Ile Arg His His Ala Leu Glu Ser
   195 200 205
   Gly Cys Phe Val Val Asn Ala Thr Ala Trp Leu Asp Ala Asp Gln Gln
   210 215 220
   Gly Gln Ile Met Gln Asp Thr Gly Cys Gly Leu Gly Pro Ile Ser Gly
   225 230 235 240
   Gly Cys Phe Thr Ala Ile Val Ser Pro Glu Gly Lys Leu Leu Gly Glu
   245 250 255
   Pro Leu Arg Ser Gly Glu Gly Val Val Ile Ala Asp Leu Asp Met Ala
   260 265 270
   Leu Ile Asp Lys Arg Lys Arg Met Met Asp Ser Val Gly His Tyr Ser
   275 280 285
   Arg Pro Glu Leu Leu Ser Leu Leu Ile Asp Arg Ser Pro Thr Ala His
   290 295 300
   Phe His Glu Arg Ala Gly Leu Cys Ser Glu Arg Arg Arg Phe Ala Gly
   305 310 315 320
   Gly Arg Ala Cys Arg Pro Met Asn Cys Ser Leu Thr Cys Lys Ser Lys
   325 330 335
   Ala Cys Val Gly Arg Pro Arg Lys Trp Leu Val Ala Pro Arg Arg Arg
   340 345 350
   Arg Ser Phe Arg Pro Gln Gly Ala Glu Pro Arg
   355 360
<210> 111
   <211> 990
   <212> DNA
   <213> Unknown
<220>
   <223> Obtained from an environmental sample
<400> 111
   atgcccaaaa cagtacgtgc cgcagcagtc cagatcgcgc ccgacctgac gtcacgcgcc     60
   ggcaccgtcg agcgggtcct caatgcaatc gccgaagctg ctgacaaagg cgccgagctg    120
   atcgtatttc ccgagacctt cgtgccctgg tatccctatt tcagtttcgt tctgccacct    180
   gtccagcaag gccctgagca tcttcgtctt tatgaggaag cagtcacggt accatcagca    240
   gaaacacggg ccgtcgcgga cgccgcgcgc aaacgcaatg cggttatcgt ccttggcgtc    300
   aatgagcgcg accacggctc gctctataac actcagctga tcttcgacgc ggatggcagc    360
   ctgaaactca agcgtcgcaa gatcacgccg acctatcacg aacggatgat ctggggccaa    420
   ggcgatggcg ccggcctgaa ggttgtcgac actgccgtcg gtcgcgtggg tgccctggca    480
   tgctgggagc attacaatcc tctggcccgc tatactttga tggcccagca tgaggaaatt    540
   cacgcctctc atttcccggg ctcactggtc ggcccgatat tcggcgagca aatcgaagtc    600
   accatgcgcc accacgcgtt ggaatcgggc tgtttcgtgg tcaatgccac cggctggctg    660
   agcgaggagc agatcgcatc tattcatccg gaccccgcct tgcaaaaggg cctgcgcgat    720
   ggctgcatga cctgcatcat cacgccggaa ggacgccatg tcgtaccgcc gctgacctcg    780
   ggcgaaggca tcctgatcgg cgatctggac atgcggctca ttaccaagcg caagcggatg    840
   atggattcgg tcggacacta tgctcggcct gaactgctgc accttgtcca tgacacgacg    900
   cccgcacgcg cacgcgagca ggtcggcctt tcaggcgatt ttcccgatgc ggagcaagac    960
   aagctatttg aggaggttca taatgcgtga                                     990
<210> 112
   <211> 329
   <212> PRT
   <213> Unknown
<220>
   <223> Obtained from an environmental sample
<400> 112
   Met Pro Lys Thr Val Arg Ala Ala Ala Val Gln Ile Ala Pro Asp Leu
   1               5                   10                  15
   Thr Ser Arg Ala Gly Thr Val Glu Arg Val Leu Asn Ala Ile Ala Glu
               20                  25                  30
   Ala Ala Asp Lys Gly Ala Glu Leu Ile Val Phe Pro Glu Thr Phe Val
           35                  40                  45
   Pro Trp Tyr Pro Tyr Phe Ser Phe Val Leu Pro Pro Val Gln Gln Gly
       50                  55                  60
   Pro Glu His Leu Arg Leu Tyr Glu Glu Ala Val Thr Val Pro Ser Ala
   65                  70                  75                  80
   Glu Thr Arg Ala Val Ala Asp Ala Ala Arg Lys Arg Asn Ala Val Ile
                   85                  90                  95
   Val Leu Gly Val Asn Glu Arg Asp His Gly Ser Leu Tyr Asn Thr Gln
               100                 105                 110
   Leu Ile Phe Asp Ala Asp Gly Ser Leu Lys Leu Lys Arg Arg Lys Ile
           115                 120                 125
   Thr Pro Thr Tyr His Glu Arg Met Ile Trp Gly Gln Gly Asp Gly Ala
       130                 135                 140
   Gly Leu Lys Val Val Asp Thr Ala Val Gly Arg Val Gly Ala Leu Ala
   145                 150                 155                 160
   Cys Trp Glu His Tyr Asn Pro Leu Ala Arg Tyr Thr Leu Met Ala Gln
                   165                 170                 175
   His Glu Glu Ile His Ala Ser His Phe Pro Gly Ser Leu Val Gly Pro
                       180                     185                     190
   Ile Phe Gly Glu Gln Ile Glu Val Thr Met Arg His His Ala Leu Glu
                   195                 200                 205
   Ser Gly Cys Phe Val Val Asn Ala Thr Gly Trp Leu Ser Glu Glu Gln
       210                 215                 220
   Ile Ala Ser Ile His Pro Asp Pro Ala Leu Gln Lys Gly Leu Arg Asp
   225                 230                 235                 240
   Gly Cys Met Thr Cys Ile Ile Thr Pro Glu Gly Arg His Val Val Pro
                   245                 250                 255
   Pro Leu Thr Ser Gly Glu Gly Ile Leu Ile Gly Asp Leu Asp Met Arg
               260                 265                 270
   Leu Ile Thr Lys Arg Lys Arg Met Met Asp Ser Val Gly His Tyr Ala
           275                 280                 285
   Arg Pro Glu Leu Leu His Leu Val His Asp Thr Thr Pro Ala Arg Ala
       290                 295                 300
   Arg Glu Gln Val Gly Leu Ser Gly Asp Phe Pro Asp Ala Glu Gln Asp
   305                 310                 315                 320
   Lys Leu Phe Glu Glu Val His Asn Ala
                   325
<210> 113
   <211> 993
   <212> DNA
   <213> Unknown
<220>
   <223> Obtained from an environmental sample
<400> 113
   atgacgaagg aacgcgccgc gcgcagcctg cgcgcagctg ccatacagct tgaagccgaa      60
   gtcggcgaca tcgccgccaa tctcgcacgc atcgaggcga tggtcgagga ggctgcgggc     120
   aagggcgccg aactgatcgc cattccggag ttctgcacct cccgcatgcc cttcgatgca     180
   cgcgtgcacg acgccgtgct gccgccggac aacttcgtgg tcgatgcctt tcgccgcatg     240
   gcagcgacgc acaactgccg gctcggcggc tccatgctca ttgccgacgg tggcgagatc     300
   tacaaccgct accacttcgt cgaacccgac ggcagcgtgc atctgcacga caaggatctg     360
   ccgacgatgt gggagaacgc cttctacacc ggcggctccg acgacggcgt cttcgacacc     420
   ggcatcggcg gcgtcggcgc cgcggtgtgc tgggaactgg tacgcaccgg caccgtgcga     480
   cgcatgctcg gtcgcgtcga cgtcgccatg accggcacgc attggtggac gatgccgcac     540
   aactggggca gcgccgtcgc gcgcacgctg gccgcgatga cgcagtacaa ccgctacatg     600
   tccgagaatg cacccaccga attcgcccgc cgcctgggtg tgccggtgct gcaggcctcg     660
   cactgcggaa gcttccgcac cggtttcttg ctgctgccag gcagcgggcg tgcactgccc     720
   tatgacaccg agtacgtcgg cgccacacag atcgtcgatg ccgatggcca catcctcgcc     780
   caccgtcgca cgcaggaagg ccccggtgtc gtcgtcgccg acatcacgct cggtgcccgc     840
   acgcccgagc tgccactgga agaccgcttc tggattcccg agctgccgct cttcctcaag     900
   gcctactggc accaccagaa cctgtgcggc aagtcctact accgtcgcgt cggccgcgat     960
   gccggcctgg cggcggcgga gcgttcggca tga                                  993
<210> 114
   <211> 330
   <212> PRT
   <213> Unknown
<220>
   <223> Obtained from an environmental sample
<400> 114
   Met Thr Lys Glu Arg Ala Ala Arg Ser Leu Arg Ala Ala Ala Ile Gln
   1 5 10 15
   Leu Glu Ala Glu Val Gly Asp Ile Ala Ala Asn Leu Ala Arg Ile Glu
   20 25 30
   Ala Met Val Glu Glu Ala Ala Gly Lys Gly Ala Glu Leu Ile Ala Ile
   35 40 45
   Pro Glu Phe Cys Thr Ser Arg Met Pro Phe Asp Ala Arg Val His Asp
   50 55 60
   Ala Val Leu Pro Pro Asp Asn Phe Val Val Asp Ala Phe Arg Arg Met
   65 70 75 80
   Ala Ala Thr His Asn Cys Arg Leu Gly Gly Ser Met Leu Ile Ala Asp
   85 90 95
   Gly Gly Glu Ile Tyr Asn Arg Tyr His Phe Val Glu Pro Asp Gly Ser
   100 105 110
   Val His Leu His Asp Lys Asp Leu Pro Thr Met Trp Glu Asn Ala Phe
   115 120 125
   Tyr Thr Gly Gly Ser Asp Asp Gly Val Phe Asp Thr Gly Ile Gly Gly
   130 135 140
   Val Gly Ala Ala Val Cys Trp Glu Leu Val Arg Thr Gly Thr Val Arg
   145 150 155 160
   Arg Met Leu Gly Arg Val Asp Val Ala Met Thr Gly Thr His Trp Trp
   165 170 175
   Thr Met Pro His Asn Trp Gly Ser Ala Val Ala Arg Thr Leu Ala Ala
   180 185 190
   Met Thr Gln Tyr Asn Arg Tyr Met Ser Glu Asn Ala Pro Thr Glu Phe
   195 200 205
   Ala Arg Arg Leu Gly Val Pro Val Leu Gln Ala Ser His Cys Gly Ser
   210 215 220
   Phe Arg Thr Gly Phe Leu Leu Leu Pro Gly Ser Gly Arg Ala Leu Pro
   225 230 235 240
   Tyr Asp Thr Glu Tyr Val Gly Ala Thr Gln Ile Val Asp Ala Asp Gly
   245 250 255
   His Ile Leu Ala His Arg Arg Thr Gln Glu Gly Pro Gly Val Val Val
   260 265 270
   Ala Asp Ile Thr Leu Gly Ala Arg Thr Pro Glu Leu Pro Leu Glu Asp
   275 280 285
   Arg Phe Trp Ile Pro Glu Leu Pro Leu Phe Leu Lys Ala Tyr Trp His
   290 295 300
   His Gln Asn Leu Cys Gly Lys Ser Tyr Tyr Arg Arg Val Gly Arg Asp
   305 310 315 320
   Ala Gly Leu Ala Ala Ala Glu Arg Ser Ala
   325 330
<210> 115
   <211> 993
   <212> DNA
   <213> Unknown
<220>
   <223> Obtained from an environmental sample
<400> 115
   atgaaccaaa tcattaaagc ggcggcagtt caatgtagcc ctgtgttgta tagccaagcg      60
   ggtacagtca agaaaatctg tgacacgatt ttggagttgg ggcagcaagg tgtgcaattt     120
   gccgtatttc ctgaaactgt tgtgccttat tacccttatt tttcttttgt gcaaccaccg     180
   tttgccatgg gtaaagaaca tttaaagcta ttgcatgaat cggttgtcgt gccatcggca     240
   gcaacaactt taattggaca ggcatgcaaa gaagcgaaca tggtggtttc tattggtatt     300
   aatgagcgtg caggcggcac gatttataac gctcaattgt tgtttgatgc ggatggttcg     360
   attattcagc atcgccgtaa aattacccca acgtatcatg aacgtatggt gtgggggcaa     420
   ggcgatggca gtggtttacg tgcgatagat tctgctgtag gacgtattgg gtcgctggca     480
   tgttgggagc attacaaccc tttggctcgg tttgctttga tggcggatgg tgagcaaatt     540
   catgcggcga tgtttccggg atcactcgtg gggcagattt ttgcagatca gatcagtgcc     600
   accattcagc accatgcttt agagtcgggc tgttttgtgg tgaatgccac agcatggctt     660
   gacccagagc aacaacaaca aattatgcaa gatacaggct gtgaactcgg tccaatttcg     720
   gggggatgtt ttacggccat cgtttctcca gaaggcaaat ttttgtctga accgatcaca     780
   caaggcgaag gttatgtgat tgccgattta gacttttcct taatcgaaaa acgtaaacgg     840
   atgatggatt ctgttgggca ttatagtcgt ccagaattac tcagtttgtt gattgatcgt     900
   cgtcctacct cagttttgca tgagttaaaa ctagagaatc catcgaataa cagcatcgaa     960
   aaagtgtctg aatttgccga ggtacacgca tag                                 993
<210> 116
   <211> 330
   <212> PRT
   <213> Unknown
<220>
   <223> Obtained from an environmental sample
<400> 116
   Met Asn Gln Ile Ile Lys Ala Ala Ala Val Gln Cys Ser Pro Val Leu
   1               5                   10                  15
   Tyr Ser Gln Ala Gly Thr Val Lys Lys Ile Cys Asp Thr Ile Leu Glu
                       20                  25                  30
   Leu Gly Gln Gln Gly Val Gln Phe Ala Val Phe Pro Glu Thr Val Val
               35                  40                  45
   Pro Tyr Tyr Pro Tyr Phe Ser Phe Val Gln Pro Pro Phe Ala Met Gly
       50                  55                  60
   Lys Glu His Leu Lys Leu Leu His Glu Ser Val Val Val Pro Ser Ala
   65                  70                  75                  80
   Ala Thr Thr Leu Ile Gly Gln Ala Cys Lys Glu Ala Asn Met Val Val
                   85                  90                  95
   Ser Ile Gly Ile Asn Glu Arg Ala Gly Gly Thr Ile Tyr Asn Ala Gln
               100                 105                 110
   Leu Leu Phe Asp Ala Asp Gly Ser Ile Ile Gln His Arg Arg Lys Ile
           115                 120                 125
   Thr Pro Thr Tyr His Glu Arg Met Val Trp Gly Gln Gly Asp Gly Ser
       130                 135                 140
   Gly Leu Arg Ala Ile Asp Ser Ala Val Gly Arg Ile Gly Ser Leu Ala
   145                 150                 155                 160
   Cys Trp Glu His Tyr Asn Pro Leu Ala Arg Phe Ala Leu Met Ala Asp
                   165                 170                 175
   Gly Glu Gln Ile His Ala Ala Met Phe Pro Gly Ser Leu Val Gly Gln
               180                 185                 190
   Ile Phe Ala Asp Gln Ile Ser Ala Thr Ile Gln His His Ala Leu Glu
           195                 200                 205
   Ser Gly Cys Phe Val Val Asn Ala Thr Ala Trp Leu Asp Pro Glu Gln
       210                 215                 220
   Gln Gln Gln Ile Met Gln Asp Thr Gly Cys Glu Leu Gly Pro Ile Ser
   225                 230                 235                 240
   Gly Gly Cys Phe Thr Ala Ile Val Ser Pro Glu Gly Lys Phe Leu Ser
                   245                 250                 255
   Glu Pro Ile Thr Gln Gly Glu Gly Tyr Val Ile Ala Asp Leu Asp Phe
               260                 265                 270
   Ser Leu Ile Glu Lys Arg Lys Arg Met Met Asp Ser Val Gly His Tyr
           275                 280                 285
   Ser Arg Pro Glu Leu Leu Ser Leu Leu Ile Asp Arg Arg Pro Thr Ser
       290                 295                 300
   Val Leu His Glu Leu Lys Leu Glu Asn Pro Ser Asn Asn Ser Ile Glu
   305                 310                 315                 320
   Lys Val Ser Glu Phe Ala Glu Val His Ala
                   325                 330
<210> 117
   <211> 957
   <212> DNA
   <213> Unknown
<220>
   <223> Obtained from an environmental sample
<400> 117
   atgactcaat ccaggataat tcgtgctgcg gcagcgcaga tcgctccgga tttgcaggtt      60
   ccaggtaaca cgatcgacaa agtttgccgc accatcagcg aggcggccgc aaaaggcgta     120
   cagattattg ttttccctga aaccttggtg ccttattacc cttacttctc ttacatttca     180
   ccgcccattc aacagggcaa agaacatttg cggctgtatg accatgcagt ggttgtgccc     240
   ggctcggaaa ccgaggcaat ttcagctctt gccgcccaac acaatatggt ggtggttttg     300
   ggtgtgaacg agcgcgatca cggcacactt tacaacgcac aaattatttt caacagcgac     360
   ggaaagattc tgttgaagcg ccgaaaaatt acaccaactt atcacgagcg gatggtgtgg     420
   gggcagggtg acgcttcagg cttgaaggtg gttgattccg cagtgggccg tgtgggtgca     480
   ttggcctgtt gggaacacta caaccccttg gctcgctatt gtttgatggc ccagcacgaa     540
   gaaattcact gtgcgcagtt tcccggttca ttggtggggc aagtttttgc cgaccaaatg     600
   gaagtgacca ttcgtcacca cgcacttgag tcgggctgtt ttgtcatcaa cagcaccgct     660
   tggctttctg aagaacaggt tcaaagtatt tcatccgaca gcgcattgca gaaagggctt     720
   agaggcggtt gtttcacggc cattgtcagc cctgagggaa agctgttggc tgagccgctc     780
   accgagggtg agggcatggt gatcgccgac ctcgacatgg cgttggttac gaaacgcaaa     840
   cgcatgatgg attcagtggg ccattatgcg cgccccgagt tgttgagttt gctggttcgg     900
   gatgaggctt caagccccat gaaaaaaatt cagggagttc aacatgctga gtactga        957
<210> 118
   <211> 318
   <212> PRT
   <213> Unknown
<220>
   <223> Obtained from an environmental sample
<400> 118
   Met Thr Gln Ser Arg Ile Ile Arg Ala Ala Ala Ala Gln Ile Ala Pro
   1               5                   10                  15
   Asp Leu Gln Val Pro Gly Asn Thr Ile Asp Lys Val Cys Arg Thr Ile
               20                  25                  30
   Ser Glu Ala Ala Ala Lys Gly Val Gln Ile Ile Val Phe Pro Glu Thr
           35                  40                  45
   Leu Val Pro Tyr Tyr Pro Tyr Phe Ser Tyr Ile Ser Pro Pro Ile Gln
       50                  55                  60
   Gln Gly Lys Glu His Leu Arg Leu Tyr Asp His Ala Val Val Val Pro
   65                  70                  75                  80
   Gly Ser Glu Thr Glu Ala Ile Ser Ala Leu Ala Ala Gln His Asn Met
                   85                  90                  95
   Val Val Val Leu Gly Val Asn Glu Arg Asp His Gly Thr Leu Tyr Asn
               100                 105                 110
   Ala Gln Ile Ile Phe Asn Ser Asp Gly Lys Ile Leu Leu Lys Arg Arg
           115                 120                 125
   Lys Ile Thr Pro Thr Tyr His Glu Arg Met Val Trp Gly Gln Gly Asp
       130                 135                 140
   Ala Ser Gly Leu Lys Val Val Asp Ser Ala Val Gly Arg Val Gly Ala
   145                 150                 155                 160
   Leu Ala Cys Trp Glu His Tyr Asn Pro Leu Ala Arg Tyr Cys Leu Met
                   165                 170                 175
   Ala Gln His Glu Glu Ile His Cys Ala Gln Phe Pro Gly Ser Leu Val
               180                 185                 190
   Gly Gln Val Phe Ala Asp Gln Met Glu Val Thr Ile Arg His His Ala
           195                 200                 205
   Leu Glu Ser Gly Cys Phe Val Ile Asn Ser Thr Ala Trp Leu Ser Glu
       210                 215                 220
   Glu Gln Val Gln Ser Ile Ser Ser Asp Ser Ala Leu Gln Lys Gly Leu
   225                 230                 235                 240
   Arg Gly Gly Cys Phe Thr Ala Ile Val Ser Pro Glu Gly Lys Leu Leu
                   245                 250                 255
   Ala Glu Pro Leu Thr Glu Gly Glu Gly Met Val Ile Ala Asp Leu Asp
               260                 265                 270
   Met Ala Leu Val Thr Lys Arg Lys Arg Met Met Asp Ser Val Gly His
           275                 280                 285
   Tyr Ala Arg Pro Glu Leu Leu Ser Leu Leu Val Arg Asp Glu Ala Ser
       290                 295                 300
   Ser Pro Met Lys Lys Ile Gln Gly Val Gln His Ala Glu Tyr
   305                 310                 315
<210> 119
   <211> 984
   <212> DNA
   <213> Unknown
<220>
   <223> Obtained from an environmental sample
<400> 119
   atggatacac tcaaagttgg attggttcag atggccccca tctggttgaa ccgggataaa      60
   accctgatca aagttgagga atacatgcag aaagcaggca aacagggctg caacctggta     120
   gcttttggtg aagcgctggt tcccggctac cccttctggg tggaacgcac agagggcgcc     180
   agattcaatt ccaaagtcca gaaagaactc tttgcacatt accttgatca ggcggtgcag     240
   atcgaagccg gccaccttga tcctctccag gcattagccc aacaatacaa gatggctgtg     300
   tacgtgggga cgattgaacg cccgcctgag cggagcggcc acagcctgta ctgctcccta     360
   atatttatag acccagaagg cgagatcggc tcggttcacc gcaagttgat gcccacccat     420
   gaggaacgcc tggtctggtc aactggcgat gggcacggcc tgcgaacaca ttctctgggc     480
   gcctttaccg ttggcggact caactgctgg gaaaactgga tgccgctctc ccgcacagct     540
   ctttatgcca tgggagagga tcttcatgtt gctgcctggc ccgggagtca gcgcaatact     600
   tatgatataa ccaaattcat tgccaaggaa tctcgctctt atgtgatctc cgtatccggg     660
   atgatgaaaa aagaaaatat cctctctgaa attccccaca gccaattgat gctggaaaat     720
   agcgaggata ttatggctga tggcggatcc tgtctggctg gaccagatgg agaatggatc     780
   atcgagccca tcgtcggaga ggaaaccctg gtaactgctg aactatcaca tcagcgggtc     840
   agagaagaaa gacagaattt cgacccaaca ggtcactaca gtcggcctga tgtgacccgc     900
   ctggtagtcg accgcaggcg ccagcagatc ctggagatca ccccggacga aaaaggaaga     960
   tcggatgaaa atcaatccct ttaa                                            984
<210> 120
   <211> 327
   <212> PRT
   <213> Unknown
<220>
   <223> Obtained from an environmental sample
<400> 120
   Met Asp Thr Leu Lys Val Gly Leu Val Gln Met Ala Pro Ile Trp Leu
   1               5                   10                  15
   Asn Arg Asp Lys Thr Leu Ile Lys Val Glu Glu Tyr Met Gln Lys Ala
               20                  25                  30
   Gly Lys Gln Gly Cys Asn Leu Val Ala Phe Gly Glu Ala Leu Val Pro
           35                  40                  45
   Gly Tyr Pro Phe Trp Val Glu Arg Thr Glu Gly Ala Arg Phe Asn Ser
       50                  55                  60
   Lys Val Gln Lys Glu Leu Phe Ala His Tyr Leu Asp Gln Ala Val Gln
   65                  70                  75                  80
   Ile Glu Ala Gly His Leu Asp Pro Leu Gln Ala Leu Ala Gln Gln Tyr
                   85                  90                  95
   Lys Met Ala Val Tyr Val Gly Thr Ile Glu Arg Pro Pro Glu Arg Ser
               100                 105                 110
   Gly His Ser Leu Tyr Cys Ser Leu Ile Phe Ile Asp Pro Glu Gly Glu
           115                 120                 125
   Ile Gly Ser Val His Arg Lys Leu Met Pro Thr His Glu Glu Arg Leu
       130                 135                 140
   Val Trp Ser Thr Gly Asp Gly His Gly Leu Arg Thr His Ser Leu Gly
   145                 150                 155                 160
   Ala Phe Thr Val Gly Gly Leu Asn Cys Trp Glu Asn Trp Met Pro Leu
                   165                 170                 175
   Ser Arg Thr Ala Leu Tyr Ala Met Gly Glu Asp Leu His Val Ala Ala
               180                 185                 190
   Trp Pro Gly Ser Gln Arg Asn Thr Tyr Asp Ile Thr Lys Phe Ile Ala
           195                 200                 205
   Lys Glu Ser Arg Ser Tyr Val Ile Ser Val Ser Gly Met Met Lys Lys
       210                 215                 220
   Glu Asn Ile Leu Ser Glu Ile Pro His Ser Gln Leu Met Leu Glu Asn
   225                 230                 235                 240
   Ser Glu Asp Ile Met Ala Asp Gly Gly Ser Cys Leu Ala Gly Pro Asp
                   245                 250                 255
   Gly Glu Trp Ile Ile Glu Pro Ile Val Gly Glu Glu Thr Leu Val Thr
               260                 265                 270
   Ala Glu Leu Ser His Gln Arg Val Arg Glu Glu Arg Gln Asn Phe Asp
           275                 280                 285
   Pro Thr Gly His Tyr Ser Arg Pro Asp Val Thr Arg Leu Val Val Asp
       290                 295                 300
   Arg Arg Arg Gln Gln Ile Leu Glu Ile Thr Pro Asp Glu Lys Gly Arg
   305                 310                 315                 320
   Ser Asp Glu Asn Gln Ser Leu
                   325
<210> 121
   <211> 1158
   <212> DNA
   <213> Unknown
<220>
   <223> Obtained from an environmental sample
<400> 121
   atgagcaaaa aagttctagg cggcagagaa aaagtaaaag ttgcagtagt tcaggctgcg      60
   cccgttttca tggacaagga gaagacgatt gaaaaggctt gcaagctaat aaaagaagcg     120
   gggagaaatc gagccgagct catagcgttc tcagagtcat tcatccccgt ctatcctgca     180
   tactataccg tcggctatga aaccccttct caagaatgga gagattacgt gattgcgcta    240
   caggataact ccgtgctgat tccgagcgag gataccgagg tactcggaca ggctgcaaag    300
   gaggcagggg cttatgcagt aataggatgc agcgagatgg acgaccgtcc gggaagccga    360
   acagtttaca acacgctcct cttcatcggc aaagacggca aggtcatggg aaggcataga    420
   aaactcaaac ccacgttcac ggagagaata tactggggag agggagatgc tggagacata    480
   aaggtttttg ataccgagat cggcaggatc ggaggcctcg tatgctggga gaaccatatg    540
   actctagtca gggccgcgat gatacacagg ggagaggagt ttcatatcgc ggtctggccg    600
   ggaaactgga agggtgcgga aaacaagctt ctccaagcag ataatagccc aggaggcgcc    660
   ctctgcaacc ttcaatctct cattaaagta cacgcctttg aggccggggc gtttgtgctg    720
   agcgcttgcg gctttttgac gccagaggat ttcccggaaa ggtggcatta tataagggat    780
   ggtaaccata ttaactgcga ctgggcactg ggcggaagct caatcgtcaa tcccgccggc    840
   cgttatctcg tcgagcctaa ctttgagaag gatgcaatcc tctatgcgga ttgttatgca    900
   aaccagataa aagcagtaaa agcggttttt gattcccttg gccactattc ccgctgggat    960
   attgcccaac tggcgataag gcaggaagcc tggaatccag aggtttcttt gatcgattcc   1020
   tcttcgactg aagttgagct tccggcagac gagcttcgaa ggatttcgga gaagtttgaa   1080
   gtaactgcgg ataagttgga atctttgctt gaggaaattg gaaagattaa aaagcccagg   1140
   aaacaagccg gttcctaa                                                 1158
<210> 122
   <211> 385
   <212> PRT
   <213> Unknown
<220>
   <223> Obtained from an environmental sample
<400> 122
   Met Ser Lys Lys Val Leu Gly Gly Arg Glu Lys Val Lys Val Ala Val
   1               5                   10                  15
   Val Gln Ala Ala Pro Val Phe Met Asp Lys Glu Lys Thr Ile Glu Lys
               20                  25                  30
   Ala Cys Lys Leu Ile Lys Glu Ala Gly Arg Asn Arg Ala Glu Leu Ile
           35                  40                  45
   Ala Phe Ser Glu Ser Phe Ile Pro Val Tyr Pro Ala Tyr Tyr Thr Val
       50                  55                  60
   Gly Tyr Glu Thr Pro Ser Gln Glu Trp Arg Asp Tyr Val Ile Ala Leu
   65                  70                  75                  80
   Gln Asp Asn Ser Val Leu Ile Pro Ser Glu Asp Thr Glu Val Leu Gly
                   85                  90                  95
   Gln Ala Ala Lys Glu Ala Gly Ala Tyr Ala Val Ile Gly Cys Ser Glu
               100                 105                 110
   Met Asp Asp Arg Pro Gly Ser Arg Thr Val Tyr Asn Thr Leu Leu Phe
           115                 120                 125
   Ile Gly Lys Asp Gly Lys Val Met Gly Arg His Arg Lys Leu Lys Pro
       130                 135                 140
   Thr Phe Thr Glu Arg Ile Tyr Trp Gly Glu Gly Asp Ala Gly Asp Ile
   145                 150                 155                 160
   Lys Val Phe Asp Thr Glu Ile Gly Arg Ile Gly Gly Leu Val Cys Trp
                   165                 170                 175
   Glu Asn His Met Thr Leu Val Arg Ala Ala Met Ile His Arg Gly Glu
               180                 185                 190
   Glu Phe His Ile Ala Val Trp Pro Gly Asn Trp Lys Gly Ala Glu Asn
           195                 200                 205
   Lys Leu Leu Gln Ala Asp Asn Ser Pro Gly Gly Ala Leu Cys Asn Leu
       210                 215                 220
   Gln Ser Leu Ile Lys Val His Ala Phe Glu Ala Gly Ala Phe Val Leu
   225                 230                 235                 240
   Ser Ala Cys Gly Phe Leu Thr Pro Glu Asp Phe Pro Glu Arg Trp His
                   245                 250                 255
   Tyr Ile Arg Asp Gly Asn His Ile Asn Cys Asp Trp Ala Leu Gly Gly
               260                 265                 270
   Ser Ser Ile Val Asn Pro Ala Gly Arg Tyr Leu Val Glu Pro Asn Phe
           275                 280                 285
   Glu Lys Asp Ala Ile Leu Tyr Ala Asp Cys Tyr Ala Asn Gln Ile Lys
       290                 295                 300
   Ala Val Lys Ala Val Phe Asp Ser Leu Gly His Tyr Ser Arg Trp Asp
   305                 310                 315                 320
   Ile Ala Gln Leu Ala Ile Arg Gln Glu Ala Trp Asn Pro Glu Val Ser
                   325                 330                 335
   Leu Ile Asp Ser Ser Ser Thr Glu Val Glu Leu Pro Ala Asp Glu Leu
               340                 345                 350
   Arg Arg Ile Ser Glu Lys Phe Glu Val Thr Ala Asp Lys Leu Glu Ser
           355                 360                 365
   Leu Leu Glu Glu Ile Gly Lys Ile Lys Lys Pro Arg Lys Gln Ala Gly
       370                 375                 380
   Ser
   385
<210> 123
   <211> 990
   <212> DNA
   <213> Unknown
<220>
   <223> Obtained from an environmental sample
<400> 123
   atgtcaactt tcaagatcgc taccgtgcag agtgcaccag tatttatgga ccgcgaagct      60
   accattgaca agacttgcga gctgatcgcc gaagcagcac aagatgacga cgttcgccta     120
   gtggtcttcc ccgaggcctt tatccccacc tatccggact gggtatggcg tatccctccc     180
   ggacagcacc agatgcttgc cgacctgtac ggggagttgc tcgagcagtc ggtgacgata     240
   cccagtctgg ctaccgagcg gctctgtcag gctgcaaaga aagcgggcgt ttatgtagct     300
   gtgggcctta acgaacgcaa tacagaggcc agcaacgcta ccctgtacaa caccctgctc     360
   tacattgacg ccgagggcaa cttgctaggt aagcaccgaa agctggtacc gaccgctccc     420
   gaacgcatgg tctgggcaca gggagatggc agtacccttg aggtctacga gacctccttc     480
   ggaaaactca gcggactaat ctgttgggag aactacatgc ctctcgctcg ttatgccctg     540
   tatgcctggg gagtacagct ctatttggct cctacttggg atcgaggcga gccctggctt     600
   tccactctgc ggcacattgc caaggaagga cgagtatacg tggtcggctg ctctatcgcc     660
   ttacgtaagg aagacatccc cgaccgattc gaattcaagg cgaagtacta cgcagaggca     720
   ggagagtgga taaacaaagg tgacagcgtc atcgtcggtc ccgatggcga gctcatcgcc     780
   gggcctctac ataaggaaca ggggatactc tatgctgagc tggacacaag gcagatgcac     840
   gcccccaagt ggaacctgga tgtagccgga cactacgcgc gcccggacgt gtttcggctg     900
   accgtgagca aggatggcca tccgatgctc ggcgttgccc aagggcccaa gcatgagccg     960
   caagataaga ccgaagtatt agagggctag                                      990
<210> 124
   <211> 329
   <212> PRT
   <213> Unknown
<220>
   <223> Obtained from an environmental sample
<400> 124
   Met Ser Thr Phe Lys Ile Ala Thr Val Gln Ser Ala Pro Val Phe Met
   1               5                   10                  15
   Asp Arg Glu Ala Thr Ile Asp Lys Thr Cys Glu Leu Ile Ala Glu Ala
               20                  25                  30
   Ala Gln Asp Asp Asp Val Arg Leu Val Val Phe Pro Glu Ala Phe Ile
           35                  40                  45
   Pro Thr Tyr Pro Asp Trp Val Trp Arg Ile Pro Pro Gly Gln His Gln
       50                  55                  60
   Met Leu Ala Asp Leu Tyr Gly Glu Leu Leu Glu Gln Ser Val Thr Ile
   65                  70                  75                  80
   Pro Ser Leu Ala Thr Glu Arg Leu Cys Gln Ala Ala Lys Lys Ala Gly
                   85                  90                  95
   Val Tyr Val Ala Val Gly Leu Asn Glu Arg Asn Thr Glu Ala Ser Asn
               100                 105                 110
   Ala Thr Leu Tyr Asn Thr Leu Leu Tyr Ile Asp Ala Glu Gly Asn Leu
           115                 120                 125
   Leu Gly Lys His Arg Lys Leu Val Pro Thr Ala Pro Glu Arg Met Val
       130                 135                 140
   Trp Ala Gln Gly Asp Gly Ser Thr Leu Glu Val Tyr Glu Thr Ser Phe
   145                 150                 155                 160
   Gly Lys Leu Ser Gly Leu Ile Cys Trp Glu Asn Tyr Met Pro Leu Ala
                   165                 170                 175
   Arg Tyr Ala Leu Tyr Ala Trp Gly Val Gln Leu Tyr Leu Ala Pro Thr
               180                 185                 190
   Trp Asp Arg Gly Glu Pro Trp Leu Ser Thr Leu Arg His Ile Ala Lys
           195                 200                 205
   Glu Gly Arg Val Tyr Val Val Gly Cys Ser Ile Ala Leu Arg Lys Glu
       210                 215                 220
   Asp Ile Pro Asp Arg Phe Glu Phe Lys Ala Lys Tyr Tyr Ala Glu Ala
   225                 230                 235                 240
   Gly Glu Trp Ile Asn Lys Gly Asp Ser Val Ile Val Gly Pro Asp Gly
                   245                 250                 255
   Glu Leu Ile Ala Gly Pro Leu His Lys Glu Gln Gly Ile Leu Tyr Ala
               260                 265                 270
   Glu Leu Asp Thr Arg Gln Met His Ala Pro Lys Trp Asn Leu Asp Val
           275                 280                 285
   Ala Gly His Tyr Ala Arg Pro Asp Val Phe Arg Leu Thr Val Ser Lys
       290                 295                 300
   Asp Gly His Pro Met Leu Gly Val Ala Gln Gly Pro Lys His Glu Pro
   305                 310                 315                 320
   Gln Asp Lys Thr Glu Val Leu Glu Gly
                   325
<210> 125
   <211> 1050
   <212> DNA
   <213> Unknown
<220>
   <223> Obtained from an environmental sample
<400> 125
   atgacaactg taaaaaagac ggtacgcgca gcagcgatcc agatcgcacc tgacctcgac      60
   agtgcaggcg gtacgctgga caaggtttgc acggccattc aaaaggcggc ggcacaaggc     120
   gcggagctgg tggtttttcc cgaaaccttc ttgccctact atccttactt ttcattcgtg     180
   cggccgccct tcgcatccgg cccggaacac ttgctgctat atgaacgcgc agtggcggtg     240
   ccaggcccgg tgaccgatgc cgtctctgcc gtcgcgcgca gccacggcgt ggtggtggta     300
   ctcggcgtca atgaacgcga ccatggcacg ctgtacaaca cccaactggt gttcgacgcg     360
   aatggcgaac tggtgttgaa acgcagaaaa atcacgccga cttatcacga gcggatgatc     420
   tggggtcaag gcgacggcag cggactcaaa gtagtgcaaa cggcggtcgg ccggctaggc     480
   gcgctagcct gttgggaaca ctacaaccca ctggcccgtt atgcattgat ggcgcaacac     540
   gaagaaatcc attgcgccca gtttcccggg tccatggtcg ggcaaatatt cgccgaccag     600
   atggaagtga cgatacgcca tcacgctctc gagtcggctt gcttcgtggt gaatgccaca     660
   ggctggctga ccgatgcgca aatcacatcg atcacgccgg accccgcgct acaaaaggca     720
   ttacgtggcg gttgctgcac cgccatcgtc tcgccggaag gtgtgctcct ggcagagccg     780
   ctacgcagcg gcgaaggcat ggtgatcgcc gatctcgata tggcactcat caccaaacgc     840
   aaacggatga tggattcggt cggccactat gcgcggcccg aattgttaag cctgcttgtc     900
   gacgaccggc gcaaggtacc ggtatccgcg ctatttgccg acagcaaccc tgccaacggg     960
   cacacagttt tcaccccatc cgacatacca acccttggga gcgcacatca tgcaaacagt    1020
   taccaaaccg aaccagcaac tgatcactga                                     1050
<210> 126
   <211> 349
   <212> PRT
   <213> Unknown
<220>
   <223> Obtained from an environmental sample
<400> 126
   Met Thr Thr Val Lys Lys Thr Val Arg Ala Ala Ala Ile Gln Ile Ala
   1 5 10 15
   Pro Asp Leu Asp Ser Ala Gly Gly Thr Leu Asp Lys Val Cys Thr Ala
   20 25 30
   Ile Gln Lys Ala Ala Ala Gln Gly Ala Glu Leu Val Val Phe Pro Glu
   35 40 45
   Thr Phe Leu Pro Tyr Tyr Pro Tyr Phe Ser Phe Val Arg Pro Pro Phe
   50 55 60
   Ala Ser Gly Pro Glu His Leu Leu Leu Tyr Glu Arg Ala Val Ala Val
   65 70 75 80
   Pro Gly Pro Val Thr Asp Ala Val Ser Ala Val Ala Arg Ser His Gly
   85 90 95
   Val Val Val Val Leu Gly Val Asn Glu Arg Asp His Gly Thr Leu Tyr
   100 105 110
   Asn Thr Gln Leu Val Phe Asp Ala Asn Gly Glu Leu Val Leu Lys Arg
   115 120 125
   Arg Lys Ile Thr Pro Thr Tyr His Glu Arg Met Ile Trp Gly Gln Gly
   130 135 140
   Asp Gly Ser Gly Leu Lys Val Val Gln Thr Ala Val Gly Arg Leu Gly
   145 150 155 160
   Ala Leu Ala Cys Trp Glu His Tyr Asn Pro Leu Ala Arg Tyr Ala Leu
   165 170 175
   Met Ala Gln His Glu Glu Ile His Cys Ala Gln Phe Pro Gly Ser Met
   180 185 190
   Val Gly Gln Ile Phe Ala Asp Gln Met Glu Val Thr Ile Arg His His
   195 200 205
   Ala Leu Glu Ser Ala Cys Phe Val Val Asn Ala Thr Gly Trp Leu Thr
   210 215 220
   Asp Ala Gln Ile Thr Ser Ile Thr Pro Asp Pro Ala Leu Gln Lys Ala
   225 230 235 240
   Leu Arg Gly Gly Cys Cys Thr Ala Ile Val Ser Pro Glu Gly Val Leu
   245 250 255
   Leu Ala Glu Pro Leu Arg Ser Gly Glu Gly Met Val Ile Ala Asp Leu
   260 265 270
   Asp Met Ala Leu Ile Thr Lys Arg Lys Arg Met Met Asp Ser Val Gly
   275 280 285
   His Tyr Ala Arg Pro Glu Leu Leu Ser Leu Leu Val Asp Asp Arg Arg
   290 295 300
   Lys Val Pro Val Ser Ala Leu Phe Ala Asp Ser Asn Pro Ala Asn Gly
   305 310 315 320
   His Thr Val Phe Thr Pro Ser Asp Ile Pro Thr Leu Gly Ser Ala His
   325 330 335
   His Ala Asn Ser Tyr Gln Thr Glu Pro Ala Thr Asp His
   340 345
<210> 127
   <211> 1005
   <212> DNA
   <213> Unknown
<220>
   <223> Obtained from an environmental sample
<400> 127
   atgatagcac ggaagacaat aagggcggcg gcggtgcaga tagcgcctgt gatggaagat      60
   cggaaggcga cgaccgacaa ggtgtgcgcc tacattcagg aagcaggcga gaatggagcc     120
   gaaattgtgg tgtttcctga aaccttcatt cccaattatc cctatttctc ttttgtaaaa     180
   cctcccgtgt tggcaggtaa ggatcacctt accttgtatg accaagcggt ggaaatccct     240
   agccctacta ccgaccaagt ggggtctatg gccaaaaaat ggggaatcgt agtggtgttg     300
   ggcgtgaacg aaagaagcca cggcactttg tacaatgccc aaattgtctt tgacgctact     360
   ggtgatattg tattggtgag acgcaaaatc acccctacct atcatgaacg gatgatctgg     420
   ggacagggag atggcagtgg attaaaagca gtagacacag ctgtgggaag agtgggcgct     480
   ttggcgtgtt gggaacacta taatccactt gcgcgctacg cccttatggt agaccatgag     540
   gaaattcatt gcagccaatt ccctggctct atggtcggcc ccattttcgg tgaccagata     600
   gaagtgacga ttcgccacca tgcgttggaa tcgggttgtt ttgtcatcaa ttccacaggt     660
   tggctgtttg aagagcaaat ccaagccatc accgatgatc cgaaactgca caaagcattg     720
   aaagacggct gtatgaccgc cattatttct cccgaaggcg tgcatttgac caaaccctta     780
   acagaaggcg aaggcatcat ctacgcctat ctggacatga aactcataga caagcggaaa     840
   cggatgatgg actcggtagg acactatgca cgtccagagt tgctctcttt gcatatcaac     900
   aatgcagagc aaaaaccagc cgtttacacc tctcctctta ccaaaacgga aaccaaagaa     960
   gacgtaaaaa gctatgatcg caacaaagaa cagcttatcg tctga                    1005
<210> 128
   <211> 334
   <212> PRT
   <213> Unknown
<220>
   <223> Obtained from an environmental sample
<400> 128
   Met Ile Ala Arg Lys Thr Ile Arg Ala Ala Ala Val Gln Ile Ala Pro
                                        1
   1               5                   10                  15
   Val Met Glu Asp Arg Lys Ala Thr Thr Asp Lys Val Cys Ala Tyr Ile
               20                  25                  30
   Gln Glu Ala Gly Glu Asn Gly Ala Glu Ile Val Val Phe Pro Glu Thr
           35                  40                  45
   Phe Ile Pro Asn Tyr Pro Tyr Phe Ser Phe Val Lys Pro Pro Val Leu
       50                  55                  60
   Ala Gly Lys Asp His Leu Thr Leu Tyr Asp Gln Ala Val Glu Ile Pro
   65                  70                  75                  80
   Ser Pro Thr Thr Asp Gln Val Gly Ser Met Ala Lys Lys Trp Gly Ile
                   85                  90                  95
   Val Val Val Leu Gly Val Asn Glu Arg Ser His Gly Thr Leu Tyr Asn
               100                 105                 110
   Ala Gln Ile Val Phe Asp Ala Thr Gly Asp Ile Val Leu Val Arg Arg
           115                 120                 125
   Lys Ile Thr Pro Thr Tyr His Glu Arg Met Ile Trp Gly Gln Gly Asp
       130                 135                 140
   Gly Ser Gly Leu Lys Ala Val Asp Thr Ala Val Gly Arg Val Gly Ala
   145                 150                 155                 160
   Leu Ala Cys Trp Glu His Tyr Asn Pro Leu Ala Arg Tyr Ala Leu Met
                   165                 170                 175
   Val Asp His Glu Glu Ile His Cys Ser Gln Phe Pro Gly Ser Met Val
               180                 185                 190
   Gly Pro Ile Phe Gly Asp Gln Ile Glu Val Thr Ile Arg His His Ala
           195                 200                 205
   Leu Glu Ser Gly Cys Phe Val Ile Asn Ser Thr Gly Trp Leu Phe Glu
       210                 215                 220
   Glu Gln Ile Gln Ala Ile Thr Asp Asp Pro Lys Leu His Lys Ala Leu
   225                 230                 235                 240
   Lys Asp Gly Cys Met Thr Ala Ile Ile Ser Pro Glu Gly Val His Leu
                   245                 250                 255
   Thr Lys Pro Leu Thr Glu Gly Glu Gly Ile Ile Tyr Ala Tyr Leu Asp
               260                 265                 270
   Met Lys Leu Ile Asp Lys Arg Lys Arg Met Met Asp Ser Val Gly His
           275                 280                 285
   Tyr Ala Arg Pro Glu Leu Leu Ser Leu His Ile Asn Asn Ala Glu Gln
       290                 295                 300
   Lys Pro Ala Val Tyr Thr Ser Pro Leu Thr Lys Thr Glu Thr Lys Glu
   305                 310                 315                 320
   Asp Val Lys Ser Tyr Asp Arg Asn Lys Glu Gln Leu Ile Val
                   325                 330
<210> 129
   <211> 1011
   <212> DNA
   <213> Unknown
<220>
   <223> Obtained from an environmental sample
<400> 129
   atgtcagaaa agcgaattat taaagcggct gcagttcaaa tcacaccaga ttttgaatcg     60
   catgatggaa ccgtaaagaa ggtttgtaat gtaattgatg aagcgggtgc taaaggtgta    120
   cagatcattg tattccctga aacctttatt ccatattacc catatttttc tttcatcact    180
   ccaccagtga ctgctggcgc ggagcatttg cggctctatg aaaaaagtgt cgtgatacct    240
   ggtcccgtta ctcaagccat ttccgaacgt gcacgcatga ataatatggt tgttgtactt    300
   ggtgtaaatg agcgtgataa cggcagtcta tataacaccc agattatttt tgatgctacc    360
   ggtgagatgc ttctgaagag aagaaaaatc acacctacct atcatgagcg catgatttgg    420
   gggcaaggag atgcttcagg cctgaaggtc gtcgatacgg ctattgggcg agtcggagca    480
   ttggcatgct gggagcacta taaccctttg gctagataca gcctcatgac acagcatgaa    540
   gaaattcact gtgctcaatt tccaggctcc atggttggtc agatcttcgc agatcaaatg    600
   gatgtcacga ttcgtcatca tgccttggag tcaggttgct tcgtcatcaa ctccactggc    660
   tggttaactg atgatcagat caaatctatc accgacgatc ccaaaatgca gaaagcttta    720
   agaggtggtt gcaacacggc cattatttct ccagaaggga atcatttaac cgagcctttg    780
   cgagaaggtg aaggcatggt gattgctgat cttgatatgg cactcatcac caaacgaaaa    840
   agaatgatgg actcagttgg ccactacgcc agaccagaac tgttgagctt agcgatcaat    900
   gatgctccgg ctactccttc attccagatg aacgaacatc gtcttaaatc agtgcaatta    960
   cctatcgcag aggagcttaa aaatgacaac aagcttagca gtggacagta a            1011
<210> 130
   <211> 336
   <212> PRT
   <213> Unknown
<220>
   <223> Obtained from an environmental sample
<400> 130
   Met Ser Glu Lys Arg Ile Ile Lys Ala Ala Ala Val Gln Ile Thr Pro
   1               5                   10                  15
   Asp Phe Glu Ser His Asp Gly Thr Val Lys Lys Val Cys Asn Val Ile
               20                  25                  30
   Asp Glu Ala Gly Ala Lys Gly Val Gln Ile Ile Val Phe Pro Glu Thr
           35                  40                  45
   Phe Ile Pro Tyr Tyr Pro Tyr Phe Ser Phe Ile Thr Pro Pro Val Thr
       50                  55                  60
   Ala Gly Ala Glu His Leu Arg Leu Tyr Glu Lys Ser Val Val Ile Pro
   65                  70                  75                  80
   Gly Pro Val Thr Gln Ala Ile Ser Glu Arg Ala Arg Met Asn Asn Met
                   85                  90                  95
   Val Val Val Leu Gly Val Asn Glu Arg Asp Asn Gly Ser Leu Tyr Asn
               100                 105                 110
   Thr Gln Ile Ile Phe Asp Ala Thr Gly Glu Met Leu Leu Lys Arg Arg
           115                 120                 125
   Lys Ile Thr Pro Thr Tyr His Glu Arg Met Ile Trp Gly Gln Gly Asp
       130                 135                 140
   Ala Ser Gly Leu Lys Val Val Asp Thr Ala Ile Gly Arg Val Gly Ala
   145                 150                 155                 160
   Leu Ala Cys Trp Glu His Tyr Asn Pro Leu Ala Arg Tyr Ser Leu Met
                   165                 170                 175
   Thr Gln His Glu Glu Ile His Cys Ala Gln Phe Pro Gly Ser Met Val
               180                 185                 190
   Gly Gln Ile Phe Ala Asp Gln Met Asp Val Thr Ile Arg His His Ala
           195                 200                 205
   Leu Glu Ser Gly Cys Phe Val Ile Asn Ser Thr Gly Trp Leu Thr Asp
       210                 215                 220
   Asp Gln Ile Lys Ser Ile Thr Asp Asp Pro Lys Met Gln Lys Ala Leu
   225                 230                 235                 240
   Arg Gly Gly Cys Asn Thr Ala Ile Ile Ser Pro Glu Gly Asn His Leu
                   245                 250                 255
   Thr Glu Pro Leu Arg Glu Gly Glu Gly Met Val Ile Ala Asp Leu Asp
               260                 265                 270
   Met Ala Leu Ile Thr Lys Arg Lys Arg Met Met Asp Ser Val Gly His
           275                 280                 285
   Tyr Ala Arg Pro Glu Leu Leu Ser Leu Ala Ile Asn Asp Ala Pro Ala
       290                 295                 300
   Thr Pro Ser Phe Gln Met Asn Glu His Arg Leu Lys Ser Val Gln Leu
   305                 310                 315                 320
   Pro Ile Ala Glu Glu Leu Lys Asn Asp Asn Lys Leu Ser Ser Gly Gln
                   325                 330                 335
<210> 131
   <211> 1011
   <212> DNA
   <213> Unknown
<220>
   <223> Obtained from an environmental sample
<400> 131
   atgtcagaaa agcgaattat taaagcggct gcagttcaaa tcacaccaga ttttgaatcg      60
   catgatggaa ccgtaaagaa ggtttgtaat gtaattgatg aagcgggtgc taaaggtgta     120
   cagatcattg tattccctga aacctttatt ccatattacc catatttttc tttcatcact     180
   ccaccagtga ctgctggcgc ggagcatttg cggctctatg aaaaaagtgt cgtgatacct     240
   ggtcccgtta ctcaagacat ttccgaacgt gcacgcatga ataatatggt tgttgtactt     300
   ggtgtaaatg agcgtgataa cggcagtcta tataacaccc agattatttt tgatgctacc     360
   ggtgagatgc ttctgaagag aagaaaaatc acacctacct atcatgagcg catgatttgg     420
   gggcaaggag atgcttcagg cctgaaggtc gtcgatacgg ctattgggcg agtcggagca     480
   ttggcatgct gggagcacta taaccctttg gctagataca gcctcatgac acagcatgaa      540
   gaaattcact gtgctcaatt tccaggctcc atggttggtc agatcttcgc agatcaaatg      600
   gatgtcacga ttcgtcatca tgccttggag tcaggttgct tcgtcatcaa ctccactggc      660
   tggttaactg atgatcagat caaatctatc accgacgatc ccaaaatgca gaaagcttta      720
   agaggtggtt gcaacacggc cattatttct ccagaaggga atcatttaac cgagcctttg      780
   cgagaaggtg aaggcatggt gattgctgat cttgatatgg cactcatcac caaacgaaaa      840
   agaatgatgg actcagttgg ccactacgcc agaccagaac tgttgagctt agcgatcaat      900
   gatgctccgg ctactccttc attccagatg aacgaacatc gtcttaaatc agtgcaatta      960
   cctatcgcag aggagcttaa aaatgacaac aagcttagca gtggacagta a              1011
<210> 132
   <211> 336
   <212> PRT
   <213> Unknown
<220>
   <223> Obtained from an environmental sample
<400> 132
   Met Ser Glu Lys Arg Ile Ile Lys Ala Ala Ala Val Gln Ile Thr Pro
   1               5                   10                  15
   Asp Phe Glu Ser His Asp Gly Thr Val Lys Lys Val Cys Asn Val Ile
               20                  25                  30
   Asp Glu Ala Gly Ala Lys Gly Val Gln Ile Ile Val Phe Pro Glu Thr
           35                  40                  45
   Phe Ile Pro Tyr Tyr Pro Tyr Phe Ser Phe Ile Thr Pro Pro Val Thr
       50                  55                  60
   Ala Gly Ala Glu His Leu Arg Leu Tyr Glu Lys Ser Val Val Ile Pro
   65                  70                  75                  80
   Gly Pro Val Thr Gln Asp Ile Ser Glu Arg Ala Arg Met Asn Asn Met
                   85                  90                  95
   Val Val Val Leu Gly Val Asn Glu Arg Asp Asn Gly Ser Leu Tyr Asn
               100                 105                 110
   Thr Gln Ile Ile Phe Asp Ala Thr Gly Glu Met Leu Leu Lys Arg Arg
           115                 120                 125
   Lys Ile Thr Pro Thr Tyr His Glu Arg Met Ile Trp Gly Gln Gly Asp
       130                 135                 140
   Ala Ser Gly Leu Lys Val Val Asp Thr Ala Ile Gly Arg Val Gly Ala
   145                 150                 155                 160
   Leu Ala Cys Trp Glu His Tyr Asn Pro Leu Ala Arg Tyr Ser Leu Met
                   165                 170                 175
   Thr Gln His Glu Glu Ile His Cys Ala Gln Phe Pro Gly Ser Met Val
               180                 185                 190
   Gly Gln Ile Phe Ala Asp Gln Met Asp Val Thr Ile Arg His His Ala
           195                 200                 205
   Leu Glu Ser Gly Cys Phe Val Ile Asn Ser Thr Gly Trp Leu Thr Asp
       210                 215                 220
   Asp Gln Ile Lys Ser Ile Thr Asp Asp Pro Lys Met Gln Lys Ala Leu
   225                 230                 235                 240
   Arg Gly Gly Cys Asn Thr Ala Ile Ile Ser Pro Glu Gly Asn His Leu
                   245                 250                 255
   Thr Glu Pro Leu Arg Glu Gly Glu Gly Met Val Ile Ala Asp Leu Asp
               260                 265                 270
   Met Ala Leu Ile Thr Lys Arg Lys Arg Met Met Asp Ser Val Gly His
           275                 280                 285
   Tyr Ala Arg Pro Glu Leu Leu Ser Leu Ala Ile Asn Asp Ala Pro Ala
       290                 295                 300
   Thr Pro Ser Phe Gln Met Asn Glu His Arg Leu Lys Ser Val Gln Leu
   305                 310                 315                 320
   Pro Ile Ala Glu Glu Leu Lys Asn Asp Asn Lys Leu Ser Ser Gly Gln
                   325                 330                 335
<210> 133
   <211> 1026
   <212> DNA
   <213> Unknown
<220>
   <223> Obtained from an environmental sample
<400> 133
   atgtcgacca agcggatcgt acgcgccgct gccgttcagc tggcaccgga tctggagcgg      60
   ccggagggca cactggagaa ggtttgcgcg gccatcgaca aggcggcggg ggacggtgtg     120
   cagctcatcg tcttccccga gaccttcgta ccgtactacc cgtacttctc tttcgtgcgt     180
   gcgccggtcg cgatgggtgc cgagcacatg cggttatacg agcgcgcggt agcggtgccc     240
   ggtccagtaa cggccaccgt ggcggagcgg gcaaaagcgc acgcgatggt cgtcgtgctg     300
   ggtgtaaacg agcgcgatca cggctcactg tataacgcgc aactgatctt cgacgagacc     360
   ggccgtctcg tcctcaaacg ccgcaagatc actccgacct atcacgagcg catggtgtgg     420
   gggcagggcg acggcagcgg ccttaaggtt gtagacaccg gtatcggcag gatcggagcc     480
   ctcgcctgct gggagcacta caacccgctc gcgcgctatg cgctcatggc gcagcacgaa     540
   gagattcatt gcgcgcagtt tccgggctcg atggtggggc cgatcttcgc ggatcagatc     600
   gaggtcacga tccgccatca cgcgctggag tcgggctgct tcgtcgtcaa tgcgaccggc     660
   tggctgacac ccgaacagat cgcgtcgatc acaccggacg cgggtctgca aaaggcaatc     720
   agcgggggct gcaacaccgc gatcatctcg ccggagggcg tgcacctggc cccgccgttg     780
   cgagaaggtg agggcatggt cgtggccgac ctcgacatgg cgctcatcac caaacgcaaa     840
   cgcatgatgg attcggtggg tcactacgct cgcccggagt tgctcagcct gcgcatcgat     900
   agccgcgccg cttcgccgat gtcgtcacaa atggaaatac ccgggagctt gcatgaaatc     960
   accagccacg atgtccagcc agcaactgat gaccgagctc cagtcctccg gcttgaggtt    1020
   ggctga                                                               1026
<210> 134
   <211> 341
   <212> PRT
   <213> Unknown
<220>
   <223> Obtained from an environmental sample
<400> 134
   Met Ser Thr Lys Arg Ile Val Arg Ala Ala Ala Val Gln Leu Ala Pro
   1               5                   10                  15
   Asp Leu Glu Arg Pro Glu Gly Thr Leu Glu Lys Val Cys Ala Ala Ile
               20                  25                  30
   Asp Lys Ala Ala Gly Asp Gly Val Gln Leu Ile Val Phe Pro Glu Thr
           35                  40                  45
   Phe Val Pro Tyr Tyr Pro Tyr Phe Ser Phe Val Arg Ala Pro Val Ala
       50                  55                  60
   Met Gly Ala Glu His Met Arg Leu Tyr Glu Arg Ala Val Ala Val Pro
   65                  70                  75                  80
   Gly Pro Val Thr Ala Thr Val Ala Glu Arg Ala Lys Ala His Ala Met
                   85                  90                  95
   Val Val Val Leu Gly Val Asn Glu Arg Asp His Gly Ser Leu Tyr Asn
               100                 105                 110
   Ala Gln Leu Ile Phe Asp Glu Thr Gly Arg Leu Val Leu Lys Arg Arg
           115                 120                 125
   Lys Ile Thr Pro Thr Tyr His Glu Arg Met Val Trp Gly Gln Gly Asp
       130                 135                 140
   Gly Ser Gly Leu Lys Val Val Asp Thr Gly Ile Gly Arg Ile Gly Ala
   145                 150                 155                 160
   Leu Ala Cys Trp Glu His Tyr Asn Pro Leu Ala Arg Tyr Ala Leu Met
                   165                 170                 175
   Ala Gln His Glu Glu Ile His Cys Ala Gln Phe Pro Gly Ser Met Val
               180                 185                 190
   Gly Pro Ile Phe Ala Asp Gln Ile Glu Val Thr Ile Arg His His Ala
           195                 200                 205
   Leu Glu Ser Gly Cys Phe Val Val Asn Ala Thr Gly Trp Leu Thr Pro
       210                 215                 220
   Glu Gln Ile Ala Ser Ile Thr Pro Asp Ala Gly Leu Gln Lys Ala Ile
   225                 230                 235                 240
   Ser Gly Gly Cys Asn Thr Ala Ile Ile Ser Pro Glu Gly Val His Leu
                   245                 250                 255
   Ala Pro Pro Leu Arg Glu Gly Glu Gly Met Val Val Ala Asp Leu Asp
               260                 265                 270
   Met Ala Leu Ile Thr Lys Arg Lys Arg Met Met Asp Ser Val Gly His
           275                 280                 285
   Tyr Ala Arg Pro Glu Leu Leu Ser Leu Arg Ile Asp Ser Arg Ala Ala
       290                 295                 300
   Ser Pro Met Ser Ser Gln Met Glu Ile Pro Gly Ser Leu His Glu Ile
   305                 310                 315                 320
   Thr Ser His Asp Val Gln Pro Ala Thr Asp Asp Arg Ala Pro Val Leu
                   325                 330                 335
   Arg Leu Glu Val Gly
               340
<210> 135
   <211> 1011
   <212> DNA
   <213> Unknown
<220>
   <223> Obtained from an environmental sample
<400> 135
   atgtcagaca agcgaatcat taaagcggct gcagttcaaa tcactcctga ctttgactca      60
   gcagatggaa ccgttaagaa agtgtgcaag gtaatcgatg aagcaggtgc aaagggagtt     120
   caaattattg tattcccgga aaccttcatc ccttactacc catacttttc attcattaca     180
   cctccagtca ctgctggcgc tgagcattta aagctttatg agaaaagtgt cgtgatacct     240
   ggcccggtta cccaagcgat tgccgagcga gccagggtta atcagatggt tgtcgtgctt     300
   ggtgtcaacg agcgagataa cggtagcctc tacaacacac aattgatctt tgataccaac     360
   ggcgaactgc tacttaaaag aagaaaaatc acccctacct accatgaacg tatgatctgg     420
   gggcaaggtg atgcatcagg tctcaaagta gttgaaacag agatcgcccg agtaggtgcc     480
   ttggcttgtt gggaacacta caacccactg gccagatatg cactcatgac acagcatgaa     540
   gaaattcact gtgcgcaatt cccaggctct atggttggcc agatatttgc cgatcagatg     600
   gatgtcacta tccgacatca cgccttagag tcaggctgct tcgtcatcaa cgccactggc     660
   tggctcaccg acgcgcaaat ccaatcgatt actgatgacc caaaaatgca aaaagcatta     720
   cgtggcggct gcaacacagc catcatctcc cccgaagggg tgcacttaac agagccacta     780
   cgtgaaggag aaggcatggt gattgccaat cttgatatgg cactcatcac aaaacgaaaa     840
   agaatgatgg attcggtagg ccattattca agaccagaat tattaagcct ggcaattaac     900
   gacaaaccag caactacaac attttcaatg actgaggggc gtactcaaac agagccattt     960
   cgaatcgcag aggagttgaa aaatgacgac aagcttagca ctggaaacta a             1011
<210> 136
   <211> 336
   <212> PRT
   <213> Unknown
<220>
   <223> Obtained from an environmental sample
<400> 136
   Met Ser Asp Lys Arg Ile Ile Lys Ala Ala Ala Val Gln Ile Thr Pro
   1               5                   10                  15
   Asp Phe Asp Ser Ala Asp Gly Thr Val Lys Lys Val Cys Lys Val Ile
               20                  25                  30
   Asp Glu Ala Gly Ala Lys Gly Val Gln Ile Ile Val Phe Pro Glu Thr
           35                  40                  45
   Phe Ile Pro Tyr Tyr Pro Tyr Phe Ser Phe Ile Thr Pro Pro Val Thr
       50                  55                  60
   Ala Gly Ala Glu His Leu Lys Leu Tyr Glu Lys Ser Val Val Ile Pro
   65                  70                  75                  80
   Gly Pro Val Thr Gln Ala Ile Ala Glu Arg Ala Arg Val Asn Gln Met
                   85                  90                  95
   Val Val Val Leu Gly Val Asn Glu Arg Asp Asn Gly Ser Leu Tyr Asn
               100                 105                 110
   Thr Gln Leu Ile Phe Asp Thr Asn Gly Glu Leu Leu Leu Lys Arg Arg
           115                 120                 125
   Lys Ile Thr Pro Thr Tyr His Glu Arg Met Ile Trp Gly Gln Gly Asp
       130                 135                 140
   Ala Ser Gly Leu Lys Val Val Glu Thr Glu Ile Ala Arg Val Gly Ala
   145                 150                 155                 160
   Leu Ala Cys Trp Glu His Tyr Asn Pro Leu Ala Arg Tyr Ala Leu Met
                   165                 170                 175
   Thr Gln His Glu Glu Ile His Cys Ala Gln Phe Pro Gly Ser Met Val
               180                 185                 190
   Gly Gln Ile Phe Ala Asp Gln Met Asp Val Thr Ile Arg His His Ala
           195                 200                 205
   Leu Glu Ser Gly Cys Phe Val Ile Asn Ala Thr Gly Trp Leu Thr Asp
                   210                     215                     220
   Ala Gln Ile Gln Ser Ile Thr Asp Asp Pro Lys Met Gln Lys Ala Leu
   225                 230                 235                 240
   Arg Gly Gly Cys Asn Thr Ala Ile Ile Ser Pro Glu Gly Val His Leu
                   245                 250                 255
   Thr Glu Pro Leu Arg Glu Gly Glu Gly Met Val Ile Ala Asn Leu Asp
               260                 265                 270
   Met Ala Leu Ile Thr Lys Arg Lys Arg Met Met Asp Ser Val Gly His
           275                 280                 285
   Tyr Ser Arg Pro Glu Leu Leu Ser Leu Ala Ile Asn Asp Lys Pro Ala
       290                 295                 300
   Thr Thr Thr Phe Ser Met Thr Glu Gly Arg Thr Gln Thr Glu Pro Phe
   305                 310                 315                 320
   Arg Ile Ala Glu Glu Leu Lys Asn Asp Asp Lys Leu Ser Thr Gly Asn
                   325                 330                 335
<210> 137
   <211> 978
   <212> DNA
   <213> Unknown
<220>
   <223> Obtained from an environmental sample
<400> 137
   atggctattg tcaaggccgc ggcggtgcag atcagtccgg tgctctacag tcgcgccggc      60
   acagtggaca aggtcgtcgc gaagatccgc gagctgggcc gacgaggggt cgagttcgcc     120
   gtcttccccg agaccgtcat tccctactat ccctacttct ctttcgtgca gcccccctac     180
   acccaggcca ccgaacacct gcgcctgctc gaggaatcgg tgaccgtgcc ctccgccgaa     240
   accgacgcga tcgccaaggc cgctcgcgag gcgggcatgg tcgtctccat cggcgtcaac     300
   gagcgcgacg gcggaaccat ctacaacacc caactcctct tcgacgccga cggcactctc     360
   atccagcgcc gccgcaagat cacccccacc tatcacgaac gcatggtctg gggcagggga     420
   gacggctcag gtctgcgcgc cgtcgacagt gcggtcggcc gcatcggcca gctcgcctgc     480
   tgggagcact accagccact ggcccggtac gccctcatcg ctgacggcga gcagatccac     540
   gccgcgatgt accccggcgc cttcggcggc gatctgttcg ccgagcagat cgaggtcaac     600
   atccgccagc acgccctgga atccgccagc ttcgtcgtca acgccaccgc ctggctcgac     660
   gccgatcagc aggcccagat cgccaaggac accggaggcc cggtcccggc cttctccggt     720
   ggcttcttca ccgccatcgt cgaccccgaa ggccgtatca tcggcgaccc cctcaccagc     780
   ggcgaaggcg aagtgatcgc cgacctcgat ctcgctctca tcaaccgccg caagcgcctc     840
   atggacgcca gtggacacta ccagccgccc gaaattctta gcttcacatt gaccggtgca     900
   ccggcgcctt atgtcaagag cgcggcgtgc cggggaaccc cgggtacgac cgtggccgag     960
   gagggacggt ccgcttag                                                   978
<210> 138
   <211> 325
   <212> PRT
   <213> Unknown
<220>
   <223> Obtained from an environmental sample
<400> 138
   Met Ala Ile Val Lys Ala Ala Ala Val Gln Ile Ser Pro Val Leu Tyr
    1               5                   10                  15
   Ser Arg Ala Gly Thr Val Asp Lys Val Val Ala Lys Ile Arg Glu Leu
               20                  25                  30
   Gly Arg Arg Gly Val Glu Phe Ala Val Phe Pro Glu Thr Val Ile Pro
           35                  40                  45
   Tyr Tyr Pro Tyr Phe Ser Phe Val Gln Pro Pro Tyr Thr Gln Ala Thr
       50                  55                  60
   Glu His Leu Arg Leu Leu Glu Glu Ser Val Thr Val Pro Ser Ala Glu
   65                  70                  75                  80
   Thr Asp Ala Ile Ala Lys Ala Ala Arg Glu Ala Gly Met Val Val Ser
                   85                  90                  95
   Ile Gly Val Asn Glu Arg Asp Gly Gly Thr Ile Tyr Asn Thr Gln Leu
               100                 105                 110
   Leu Phe Asp Ala Asp Gly Thr Leu Ile Gln Arg Arg Arg Lys Ile Thr
           115                 120                 125
   Pro Thr Tyr His Glu Arg Met Val Trp Gly Gln Gly Asp Gly Ser Gly
       130                 135                 140
   Leu Arg Ala Val Asp Ser Ala Val Gly Arg Ile Gly Gln Leu Ala Cys
   145                 150                 155                 160
   Trp Glu His Tyr Gln Pro Leu Ala Arg Tyr Ala Leu Ile Ala Asp Gly
                   165                 170                 175
   Glu Gln Ile His Ala Ala Met Tyr Pro Gly Ala Phe Gly Gly Asp Leu
               180                 185                 190
   Phe Ala Glu Gln Ile Glu Val Asn Ile Arg Gln His Ala Leu Glu Ser
           195                 200                 205
   Ala Ser Phe Val Val Asn Ala Thr Ala Trp Leu Asp Ala Asp Gln Gln
       210                 215                 220
   Ala Gln Ile Ala Lys Asp Thr Gly Gly Pro Val Pro Ala Phe Ser Gly
   225                 230                 235                 240
   Gly Phe Phe Thr Ala Ile Val Asp Pro Glu Gly Arg Ile Ile Gly Asp
                   245                 250                 255
   Pro Leu Thr Ser Gly Glu Gly Glu Val Ile Ala Asp Leu Asp Leu Ala
               260                 265                 270
   Leu Ile Asn Arg Arg Lys Arg Leu Met Asp Ala Ser Gly His Tyr Gln
           275                 280                 285
   Pro Pro Glu Ile Leu Ser Phe Thr Leu Thr Gly Ala Pro Ala Pro Tyr
       290                 295                 300
   Val Lys Ser Ala Ala Cys Arg Gly Thr Pro Gly Thr Thr Val Ala Glu
   305                 310                 315                 320
   Glu Gly Arg Ser Ala
                   325
<210> 139
   <211> 999
   <212> DNA
   <213> Unknown
<220>
   <223> Obtained from an environmental sample
<400> 139
   atgaaaacaa cggttaccgt tgcctgcgtt caggccgccc ccgtatttat ggatttagaa      60
   ggcaccgtag ataaaacaat caccctcatc tctgaagccg cacagaaagg cgcggagctc     120
   atcgcttttc cggagacctg gatacccggt tacccgtggt tcttatggct gaactcgccc     180
   gccacaaata tgcccctggt ttatcagtat catcagaact ctctggtgct ggacagtacc     240
   caggcgaagc gaattgcgga tgcggcacgg cagaataaca tcactgtcgc tctgggcttc     300
   agcgaacgcg atcatggaag cctctatatc gcacagtggc tgattggcag cgacggggag     360
   accattggca tccggcgcaa gctcaaggcc acgcacgtgg agcgtacgct gttcggcgaa     420
   agcgacggct cctccctgac cacctgggag acacctctgg gtaacgtcgg ggccctctgc     480
   tgctgggagc acctgcagcc gctgtcccgc tatgcaatgt attcccagca tgaggagatc     540
   cacatcgctg cctggcccag tttcagtctc tacaccagtg caacggccgc actgggtcct     600
   gacgtcaata cggcggcttc acgcctctat gccgcggagg ggcagtgctt cgtgatagcc     660
   ccgtgtgccg tggtttctga tgaaatgatt gatttactct gtcctgatga tgaccggaga     720
   gcgttactca gtgccggagg gggacatgcc cgtatttacg gcccggacgg aagagaactc     780
   gtcacccctc tcggggaaaa tgaggaagga ctgcttatcg ctgagctcga ctctgctgcg     840
   attacctttg ccaaactggc ggcagacccg gttggccact attcccgtcc tgacgtgacc     900
   cgcctccttt ttaatccttc agccaacaag actgtgatta aacgacattc gcctcctgag     960
   ttaattgccg agcagactgc agaagaagag gaggagtag                            999
<210> 140
   <211> 332
   <212> PRT
   <213> Unknown
<220>
   <223> Obtained from an environmental sample
<400> 140
   Met Lys Thr Thr Val Thr Val Ala Cys Val Gln Ala Ala Pro Val Phe
    1               5                  10                  15
   Met Asp Leu Glu Gly Thr Val Asp Lys Thr Ile Thr Leu Ile Ser Glu
               20                  25                  30
   Ala Ala Gln Lys Gly Ala Glu Leu Ile Ala Phe Pro Glu Thr Trp Ile
           35                  40                  45
   Pro Gly Tyr Pro Trp Phe Leu Trp Leu Asn Ser Pro Ala Thr Asn Met
               50                  55                  60
   Pro Leu Val Tyr Gln Tyr His Gln Asn Ser Leu Val Leu Asp Ser Thr
   65                  70                  75                  80
   Gln Ala Lys Arg Ile Ala Asp Ala Ala Arg Gln Asn Asn Ile Thr Val
                   85                  90                  95
   Ala Leu Gly Phe Ser Glu Arg Asp His Gly Ser Leu Tyr Ile Ala Gln
               100                 105                 110
   Trp Leu Ile Gly Ser Asp Gly Glu Thr Ile Gly Ile Arg Arg Lys Leu
           115                 120                 125
   Lys Ala Thr His Val Glu Arg Thr Leu Phe Gly Glu Ser Asp Gly Ser
       130                 135                 140
   Ser Leu Thr Thr Trp Glu Thr Pro Leu Gly Asn Val Gly Ala Leu Cys
   145                 150                 155                 160
   Cys Trp Glu His Leu Gln Pro Leu Ser Arg Tyr Ala Met Tyr Ser Gln
                   165                 170                 175
   His Glu Glu Ile His Ile Ala Ala Trp Pro Ser Phe Ser Leu Tyr Thr
               180                 185                 190
   Ser Ala Thr Ala Ala Leu Gly Pro Asp Val Asn Thr Ala Ala Ser Arg
           195                 200                 205
   Leu Tyr Ala Ala Glu Gly Gln Cys Phe Val Ile Ala Pro Cys Ala Val
       210                 215                 220
   Val Ser Asp Glu Met Ile Asp Leu Leu Cys Pro Asp Asp Asp Arg Arg
   225                 230                 235                 240
   Ala Leu Leu Ser Ala Gly Gly Gly His Ala Arg Ile Tyr Gly Pro Asp
                   245                 250                 255
   Gly Arg Glu Leu Val Thr Pro Leu Gly Glu Asn Glu Glu Gly Leu Leu
               260                 265                 270
   Ile Ala Glu Leu Asp Ser Ala Ala Ile Thr Phe Ala Lys Leu Ala Ala
           275                 280                 285
   Asp Pro Val Gly His Tyr Ser Arg Pro Asp Val Thr Arg Leu Leu Phe
       290                 295                 300
   Asn Pro Ser Ala Asn Lys Thr Val Ile Lys Arg His Ser Pro Pro Glu
   305                 310                 315                 320
   Leu Ile Ala Glu Gln Thr Ala Glu Glu Glu Glu Glu
                   325                 330
<210> 141
   <211> 1026
   <212> DNA
   <213> Unknown
<220>
   <223> Obtained from an environmental sample
<400> 141
   atggtgttca aggcagcgac tgttcatgca gctccggtat tcatggacaa ggaagcgtcg      60
   atagataagg ctatcgacct catcaagaag gccggtcagg aagggattaa gcttctggtt     120
   tttccggaaa cgtttattcc gggctatccg tattttatcg aatgctatcc gccgcttgcg     180
   caggtggaag cgctcgccca gtacactgac gcttccgtgg agatcgacgg cccggaagtc     240
   acccggcttc agcaggtagc caaggcggca ggcgttgcag tcgtcatggg catcagcgaa     300
   cgaatggctg agacccgaac ctgcttcaac tcgcaggtgt tcattgacgt cgacggcacg     360
   ctgctcggcg tgcatcgcaa gctgcagccg acttatgccg agcgcaaggt atgggcacag     420
   ggcggtggtt atacgctgag gacctacaag agctcgcttg gcgtgctcgg cggtcttgcc     480
   tgctgggagc acacgatgaa cctcgcgcgg caggccctga tcatgcagag cgagcagatc     540
   catgcggctg catggcccgg actatcgacg atgcgaggtt tcgagcccgt ggccgatatc     600
   cagatcgacg ccatgatgaa gactcacgcg cttaccgcac agtgctgggt gctttcggcc     660
   ggcaatcccg tcgaccggac ctgcctcgac tggatggaaa agaacatcgg accgcaggat     720
   tacgtcaccg agggcggcgg atggagcgcc gttatccatc cgttcaacag ctatctcggc     780
   ggccctcaca cgggccttga ggaaaagctg gtcgtcggcg agatcaatct ggacgatctc     840
   aagttcgtca aagtctggct cgacagcaaa gggcactatg ctcggccgga aatcctgaaa     900
   cttggcgtca accaaaagca gatttggcct gatgaacatt tgctggcgcg gcaggatgtg     960
   accgagttgc tggaggcgga tatcatcgaa taccccttgc aactgttgca agaccgcgcg    1020
   caatag                                                               1026
<210> 142
   <211> 341
   <212> PRT
   <213> Unknown
<220>
   <223> Obtained from an environmental sample
<400> 142
   Met Val Phe Lys Ala Ala Thr Val His Ala Ala Pro Val Phe Met Asp
   1               5                   10                  15
   Lys Glu Ala Ser Ile Asp Lys Ala Ile Asp Leu Ile Lys Lys Ala Gly
               20                  25                  30
   Gln Glu Gly Ile Lys Leu Leu Val Phe Pro Glu Thr Phe Ile Pro Gly
           35                  40                  45
   Tyr Pro Tyr Phe Ile Glu Cys Tyr Pro Pro Leu Ala Gln Val Glu Ala
       50                  55                  60
   Leu Ala Gln Tyr Thr Asp Ala Ser Val Glu Ile Asp Gly Pro Glu Val
   65                  70                  75                  80
   Thr Arg Leu Gln Gln Val Ala Lys Ala Ala Gly Val Ala Val Val Met
                   85                  90                  95
   Gly Ile Ser Glu Arg Met Ala Glu Thr Arg Thr Cys Phe Asn Ser Gln
               100                 105                 110
   Val Phe Ile Asp Val Asp Gly Thr Leu Leu Gly Val His Arg Lys Leu
           115                 120                 125
   Gln Pro Thr Tyr Ala Glu Arg Lys Val Trp Ala Gln Gly Gly Gly Tyr
       130                 135                 140
   Thr Leu Arg Thr Tyr Lys Ser Ser Leu Gly Val Leu Gly Gly Leu Ala
   145                 150                 155                 160
   Cys Trp Glu His Thr Met Asn Leu Ala Arg Gln Ala Leu Ile Met Gln
                   165                 170                 175
   Ser Glu Gln Ile His Ala Ala Ala Trp Pro Gly Leu Ser Thr Met Arg
               180                 185                 190
   Gly Phe Glu Pro Val Ala Asp Ile Gln Ile Asp Ala Met Met Lys Thr
           195                 200                 205
   His Ala Leu Thr Ala Gln Cys Trp Val Leu Ser Ala Gly Asn Pro Val
       210                 215                 220
   Asp Arg Thr Cys Leu Asp Trp Met Glu Lys Asn Ile Gly Pro Gln Asp
   225                 230                 235                 240
   Tyr Val Thr Glu Gly Gly Gly Trp Ser Ala Val Ile His Pro Phe Asn
                   245                 250                 255
   Ser Tyr Leu Gly Gly Pro His Thr Gly Leu Glu Glu Lys Leu Val Val
               260                 265                 270
   Gly Glu Ile Asn Leu Asp Asp Leu Lys Phe Val Lys Val Trp Leu Asp
           275                 280                 285
   Ser Lys Gly His Tyr Ala Arg Pro Glu Ile Leu Lys Leu Gly Val Asn
       290                 295                 300
   Gln Lys Gln Ile Trp Pro Asp Glu His Leu Leu Ala Arg Gln Asp Val
   305                 310                 315                 320
   Thr Glu Leu Leu Glu Ala Asp Ile Ile Glu Tyr Pro Leu Gln Leu Leu
                   325                 330                 335
   Gln Asp Arg Ala Gln
               340
<210> 143
   <211> 1122
   <212> DNA
   <213> Unknown
<220>
   <223> Obtained from an environmental sample
<400> 143
   atgacgatca ttgcaggcgc ggttcatgcg gcgccggtat tcatggatgt cgatgccact      60
   atcgacaagg catgcgaaat cattcgcaag gcaggcaaag acggaatcga gcttctcgtc     120
   ttccctgagg ttttcgtacc cggctacccc tacttcatcg agtgctatcc gaccttgaac     180
   caaaccgctg cgctggccgc ctatacggat gcctcgatcg aggttccagg cccggaagtc     240
   cggcgcttgc aggtggccgc acatcaggcc ggcgtgatgg ttgtgatggg cgtgagcgag     300
   cgtctgcgcg gatctcgcac ctgcttcaac agccaggtgt tcatcgaccg tgacggcacc     360
   ttgctgggcg tgcaccgcaa actccagccg acctatgtcg agcgcatcgt ctggggccag     420
   ggcggcggac acaccctcaa ggtattcgac agcacactgg gcaaggtggg cggactggcc     480
   tgctgggagc acacgatgaa cctcgcgcgc catgcgttga tcgcccaggg tatccagatc     540
   catgccgccg cctggcctgg gctttcgaca atggccgggt tcgaagcggt ggctgacgtc     600
   cagatcgacg cgatgatgaa aactcatgcg ttgagcgcgc aatgctttgt cgtatcggcc     660
   gcaaaccctg tggatcagac ctgcctggag tggatggaga aacacctcgg cccgcagcaa     720
   ctcgttaccg ccggcggagg ctggtcggca atcgtccatc ctttctgtgg ttatatcgcc     780
   gcccctcaca ccggtgccga ggagaaggtt ctggtaggcg aaatcaatct ggacgacctc     840
   aagcaggtca aggtatgggt tgattccgca ggtcattatg cgcgcccgga agtcgtgcaa     900
   ttgcgcgacg ccctggagag ccgtggcaat tatcgcgttg cgctgacccg cgacgccgac     960
   accttcgtgc cgctggaaga ccgcgtgcgc tttgcgcgcc agcagaacgc cgacctcttc    1020
   atctcgatcc acgccgacgc caacgccaac cacgatgcgc gcggggctgg cttcacttcg    1080
   aaggttgaaa acctttccac gggcatttta ccaggcgatt ga                      1122
<210> 144
   <211> 373
   <212> PRT
   <213> Unknown
<220>
   <223> Obtained from an environmental sample
<400> 144
   Met Thr Ile Ile Ala Gly Ala Val His Ala Ala Pro Val Phe Met Asp
   1               5                   10                  15
   Val Asp Ala Thr Ile Asp Lys Ala Cys Glu Ile Ile Arg Lys Ala Gly
               20                  25                  30
   Lys Asp Gly Ile Glu Leu Leu Val Phe Pro Glu Val Phe Val Pro Gly
           35                  40                  45
   Tyr Pro Tyr Phe Ile Glu Cys Tyr Pro Thr Leu Asn Gln Thr Ala Ala
       50                  55                  60
   Leu Ala Ala Tyr Thr Asp Ala Ser Ile Glu Val Pro Gly Pro Glu Val
   65                  70                  75                  80
   Arg Arg Leu Gln Val Ala Ala His Gln Ala Gly Val Met Val Val Met
                   85                  90                  95
   Gly Val Ser Glu Arg Leu Arg Gly Ser Arg Thr Cys Phe Asn Ser Gln
               100                 105                 110
   Val Phe Ile Asp Arg Asp Gly Thr Leu Leu Gly Val His Arg Lys Leu
           115                 120                 125
   Gln Pro Thr Tyr Val Glu Arg Ile Val Trp Gly Gln Gly Gly Gly His
       130                 135                 140
   Thr Leu Lys Val Phe Asp Ser Thr Leu Gly Lys Val Gly Gly Leu Ala
   145                 150                 155                 160
   Cys Trp Glu His Thr Met Asn Leu Ala Arg His Ala Leu Ile Ala Gln
                   165                 170                 175
   Gly Ile Gln Ile His Ala Ala Ala Trp Pro Gly Leu Ser Thr Met Ala
               180                 185                 190
   Gly Phe Glu Ala Val Ala Asp Val Gln Ile Asp Ala Met Met Lys Thr
           195                 200                 205
   His Ala Leu Ser Ala Gln Cys Phe Val Val Ser Ala Ala Asn Pro Val
       210                 215                 220
   Asp Gln Thr Cys Leu Glu Trp Met Glu Lys His Leu Gly Pro Gln Gln
   225                 230                 235                 240
   Leu Val Thr Ala Gly Gly Gly Trp Ser Ala Ile Val His Pro Phe Cys
                   245                 250                 255
   Gly Tyr Ile Ala Ala Pro His Thr Gly Ala Glu Glu Lys Val Leu Val
               260                 265                 270
   Gly Glu Ile Asn Leu Asp Asp Leu Lys Gln Val Lys Val Trp Val Asp
           275                 280                 285
   Ser Ala Gly His Tyr Ala Arg Pro Glu Val Val Gln Leu Arg Asp Ala
       290                 295                 300
   Leu Glu Ser Arg Gly Asn Tyr Arg Val Ala Leu Thr Arg Asp Ala Asp
   305                 310                 315                 320
   Thr Phe Val Pro Leu Glu Asp Arg Val Arg Phe Ala Arg Gln Gln Asn
                   325                 330                 335
   Ala Asp Leu Phe Ile Ser Ile His Ala Asp Ala Asn Ala Asn His Asp
               340                 345                 350
   Ala Arg Gly Ala Gly Phe Thr Ser Lys Val Glu Asn Leu Ser Thr Gly
           355                 360                 365
   Ile Leu Pro Gly Asp
       370
<210> 145
   <211> 1014
   <212> DNA
   <213> Unknown
<220>
   <223> Obtained from an environmental sample
<400> 145
   atgggcatca cccacccgaa ctacaaggtc gcagtggtcc aggctgcgcc ggtctggttg      60
   aacctcgagg caacggtcga gaagacaatc aggtatattg aagaggcggc caaggctgga     120
   gcgaagctga tagcgtttcc ggaaacctgg attccgggct atccatggca catttggatc     180
   ggaacgcccg catgggcaat cggtaagggc ttcgtccagc gctatttcga caactcgctc     240
   agctatgaca gcccgctcgc gcggcagatc gctgacgccg cagcaaagag caagatcacg     300
   gttgttctcg gcctctccga gcgcgacggt ggaagcctat acatcgcgca atggctgatc     360
   ggaccagatg gcgagaccat cgcgaagcgg cgcaagctgc gtccgaccca cgtcgagcgc     420
   acggtgttcg gtgacggtga cggcagccac atcgccgtgc atgaccgatc cgatctgggc     480
   cggctcgggg cgttgtgctg ctgggagcac gtgcagccgt tgacgaaatt cgcgatgtac     540
   gcgcagaacg agcaggttca cgtggcagca tggccgagct tctcgatgta cgaacccttt     600
   gcgcatgcgc tgggttggga gacgaacaac gcggtcagca aggtctacgc ggtcgaggga     660
   tcgtgcttcg tgctcgctcc ctgtgccgtt atttcgcaag cgatggtgga cgagatgtgc     720
   gacactcccg acaagcgcga gcttgttcac gccggcggcg gccacgcggt gatttacggc     780
   cctgacggaa gcccgctcgc agaaaagctc ggggaaaacg aagaggggct tctctacgcg     840
   acggtcaatc ttgctgcgat cggggttgcc aagaatgccg cggatccggc cgggcactat     900
   tcgcgtccgg acgttctaag gctgctattc aacaagagcc cggcccgaag agtggagcat     960
   tttgcgctgc cgcacgagca gctcgagatc ggggcaggcc cgtctggcga ctga          1014
<210> 146
   <211> 337
   <212> PRT
   <213> Unknown
<220>
   <223> Obtained from an environmental sample
<400> 146
   Met Gly Ile Thr His Pro Asn Tyr Lys Val Ala Val Val Gln Ala Ala
   1               5                   10                  15
   Pro Val Trp Leu Asn Leu Glu Ala Thr Val Glu Lys Thr Ile Arg Tyr
               20                  25                  30
   Ile Glu Glu Ala Ala Lys Ala Gly Ala Lys Leu Ile Ala Phe Pro Glu
           35                  40                  45
   Thr Trp Ile Pro Gly Tyr Pro Trp His Ile Trp Ile Gly Thr Pro Ala
       50                  55                  60
   Trp Ala Ile Gly Lys Gly Phe Val Gln Arg Tyr Phe Asp Asn Ser Leu
   65                  70                  75                  80
   Ser Tyr Asp Ser Pro Leu Ala Arg Gln Ile Ala Asp Ala Ala Ala Lys
                   85                  90                  95
   Ser Lys Ile Thr Val Val Leu Gly Leu Ser Glu Arg Asp Gly Gly Ser
               100                 105                 110
   Leu Tyr Ile Ala Gln Trp Leu Ile Gly Pro Asp Gly Glu Thr Ile Ala
           115                 120                 125
   Lys Arg Arg Lys Leu Arg Pro Thr His Val Glu Arg Thr Val Phe Gly
       130                 135                 140
   Asp Gly Asp Gly Ser His Ile Ala Val His Asp Arg Ser Asp Leu Gly
   145                 150                 155                 160
   Arg Leu Gly Ala Leu Cys Cys Trp Glu His Val Gln Pro Leu Thr Lys
                   165                 170                 175
   Phe Ala Met Tyr Ala Gln Asn Glu Gln Val His Val Ala Ala Trp Pro
               180                 185                 190
   Ser Phe Ser Met Tyr Glu Pro Phe Ala His Ala Leu Gly Trp Glu Thr
           195                 200                 205
   Asn Asn Ala Val Ser Lys Val Tyr Ala Val Glu Gly Ser Cys Phe Val
       210                 215                 220
   Leu Ala Pro Cys Ala Val Ile Ser Gln Ala Met Val Asp Glu Met Cys
   225                 230                 235                 240
   Asp Thr Pro Asp Lys Arg Glu Leu Val His Ala Gly Gly Gly His Ala
                   245                 250                 255
   Val Ile Tyr Gly Pro Asp Gly Ser Pro Leu Ala Glu Lys Leu Gly Glu
               260                 265                 270
   Asn Glu Glu Gly Leu Leu Tyr Ala Thr Val Asn Leu Ala Ala Ile Gly
               275                     280                     285
   Val Ala Lys Asn Ala Ala Asp Pro Ala Gly His Tyr Ser Arg Pro Asp
       290                     295                     300
   Val Leu Arg Leu Leu Phe Asn Lys Ser Pro Ala Arg Arg Val Glu His
   305                     310                     315                     320
   Phe Ala Leu Pro His Glu Gln Leu Glu Ile Gly Ala Gly Pro Ser Gly
                       325                     330                     335
   Asp
<210> 147
   <211> 1098
   <212> DNA
   <213> Unknown
<220>
   <223> Obtained from an environmental sample
<400> 147
   atgacccagc acgagaccac tgcccggagg ctggcagctg tgcatgccgc gcctgtgttc      60
   atggacaccg acgcgaccat cgacaaggtg atcggcttcg tcgaacaggc cggccgcgaa     120
   ggcatcgaac tcctggtgtt ccccgagacc ttcgtgcctg gttaccccta ctggatcgag     180
   tgctatccgc cgctgcagca ggtggccgcc aacgcgcagt acacggacgc ctccgtcgag     240
   gtgcctggtc cggagatcaa gcgggtgcag gcggcctgtg cccgcgctgg cgtcgaagtc     300
   gtcctcggcg tcagcgagcg actcaggggt accaggacat gcttcaactc ccaggtgttc     360
   atcgacgccg acgggagcct gctcggcgtg caccgcaagc tgcagccgac gtacgtggag     420
   cgcatcgtgt gggcccaggg cggaggcgcg accctgtcgg tgttcggctc ccgctccggc     480
   cggatcggcg gtctggcctg ctgggagcac acgatgaacc tggctcgtca ggcactgctt     540
   gagcaggagc agcagatcca cgcggcggcg tggcctgccc tgtcgacgat ggcggggttc     600
   gagaccgtcg cggacgccca gatcgaggcc atgatgaaga cccatgcgct cacggcacag     660
   gtgttcgtca tctgcgcgtc caacccggtc gacggcactt gcctggaatg gatgcgggac     720
   aacctcggtg aacagaagtt cgtgaccgcc ggagggggct ggtccgcggt catccacccc     780
   ttcaactcct tcctcggcgg gccgcatacc ggtttggagg agaagctcgt cagcgcgacg     840
   atcgacttct ccgacatccg cttggtcaag gcctgggttg attcgaaggg gcactacgcg     900
   cggcccgagg tcctgcgact cgcggtcgac cgcaagccac tgtggcacga cgagtgcgag     960
   gtgccgggac aggcgcaggt acgcacccgc gctgcttctc tggcagtgca ggagcacccg    1020
   gtggtgctgc ctcagggggc ggcgcggccc gctccgcaag actgggacac ctctgcggcg    1080
   caggagctga cttcctga                                                  1098
<210> 148
   <211> 365
   <212> PRT
   <213> Unknown
<220>
   <223> Obtained from an environmental sample
<400> 148
   Met Thr Gln His Glu Thr Thr Ala Arg Arg Leu Ala Ala Val His Ala
    1               5                  10                  15
   Ala Pro Val Phe Met Asp Thr Asp Ala Thr Ile Asp Lys Val Ile Gly
               20                  25                  30
   Phe Val Glu Gln Ala Gly Arg Glu Gly Ile Glu Leu Leu Val Phe Pro
           35                  40                  45
   Glu Thr Phe Val Pro Gly Tyr Pro Tyr Trp Ile Glu Cys Tyr Pro Pro
       50                  55                  60
   Leu Gln Gln Val Ala Ala Asn Ala Gln Tyr Thr Asp Ala Ser Val Glu
   65                  70                  75                  80
   Val Pro Gly Pro Glu Ile Lys Arg Val Gln Ala Ala Cys Ala Arg Ala
                   85                  90                  95
   Gly Val Glu Val Val Leu Gly Val Ser Glu Arg Leu Arg Gly Thr Arg
               100                 105                 110
   Thr Cys Phe Asn Ser Gln Val Phe Ile Asp Ala Asp Gly Ser Leu Leu
           115                 120                 125
   Gly Val His Arg Lys Leu Gln Pro Thr Tyr Val Glu Arg Ile Val Trp
       130                 135                 140
   Ala Gln Gly Gly Gly Ala Thr Leu Ser Val Phe Gly Ser Arg Ser Gly
   145                 150                 155                 160
   Arg Ile Gly Gly Leu Ala Cys Trp Glu His Thr Met Asn Leu Ala Arg
                   165                 170                 175
   Gln Ala Leu Leu Glu Gln Glu Gln Gln Ile His Ala Ala Ala Trp Pro
               180                 185                 190
   Ala Leu Ser Thr Met Ala Gly Phe Glu Thr Val Ala Asp Ala Gln Ile
           195                 200                 205
   Glu Ala Met Met Lys Thr His Ala Leu Thr Ala Gln Val Phe Val Ile
       210                 215                 220
   Cys Ala Ser Asn Pro Val Asp Gly Thr Cys Leu Glu Trp Met Arg Asp
   225                 230                 235                 240
   Asn Leu Gly Glu Gln Lys Phe Val Thr Ala Gly Gly Gly Trp Ser Ala
                   245                 250                 255
   Val Ile His Pro Phe Asn Ser Phe Leu Gly Gly Pro His Thr Gly Leu
               260                 265                 270
   Glu Glu Lys Leu Val Ser Ala Thr Ile Asp Phe Ser Asp Ile Arg Leu
           275                 280                 285
   Val Lys Ala Trp Val Asp Ser Lys Gly His Tyr Ala Arg Pro Glu Val
       290                 295                 300
   Leu Arg Leu Ala Val Asp Arg Lys Pro Leu Trp His Asp Glu Cys Glu
   305                 310                 315                 320
   Val Pro Gly Gln Ala Gln Val Arg Thr Arg Ala Ala Ser Leu Ala Val
                   325                 330                 335
   Gln Glu His Pro Val Val Leu Pro Gln Gly Ala Ala Arg Pro Ala Pro
               340                 345                 350
   Gln Asp Trp Asp Thr Ser Ala Ala Gln Glu Leu Thr Ser
           355                 360                 365
<210> 149
   <211> 942
   <212> DNA
   <213> Unknown
<220>
   <223> Obtained from an environmental sample
<400> 149
   atgacgaagc ttgagaaggt ggtcgcggcg gcggtccagg cgacgccgga gttcctcgac      60
   cgcgaggcga ccgtcgagaa ggccgtgcgg ctgatcaagg aagcggccgg ggagggcgcc     120
   ggcctgatcg tgttccccga gacgttcatc ccgacgtacc cggactgggt ctggcgcgcg     180
   ccggcctggg acggcccatc cgcggacctg tacgcaatgc tgctggagaa cgcggtggag     240
   atccccgggc cggtgacgga gaccctgggg aaggcggcga agcaggccaa ggccttcgtg     300
   tcgatgggcg tcaacgagcg cgagccgggc ggcgggacga tctacaacac gcaggtcacg     360
   ttcggacccg acgggagcgt gctcggcaag caccgcaagc tgatgccgac cggcggcgag     420
   cgcctggtgt gggggatggg cgacgggtcg atgctccagg tctatgacac gccgttcggc     480
   cgcctgggcg ggctgatctg ctgggagaac tacatgccgc tcgcgcgcta ctcgatgtac     540
   gccaagggcg tggacgtcta cgttgcgccg acgtgggaca acagcgacat gtgggtggcg     600
   acgctccgcc acatcgccaa ggaggggcgg ctgtacgtga tcggcgtggc gccgctgctg     660
   cgcgggtcgg acgtccccga cgacgtgccg gggaaggccg agctgtgggg cggcgatgac     720
   gactggatgt cgcgcggctt ctccaccatc gtcgcgccgg gcggcgaggt gctggccggt     780
   ccgctgacgg aggaggaagg catcctctac gcggagatcg acccggcgag agcccgttcg     840
   tcacggcacc agttcgatcc ggtggggcac tactcgcgcc ccgacgtgtt tcggctcgtc     900
   gtggacgagt cgcccaagcc ccagacgtcc ggcccgggct ag                        942
<210> 150
   <211> 313
   <212> PRT
   <213> Unknown
<220>
   <223> Obtained from an environmental sample
<400> 150
   Met Thr Lys Leu Glu Lys Val Val Ala Ala Ala Val Gln Ala Thr Pro
   1               5                   10                  15
   Glu Phe Leu Asp Arg Glu Ala Thr Val Glu Lys Ala Val Arg Leu Ile
               20                  25                  30
   Lys Glu Ala Ala Gly Glu Gly Ala Gly Leu Ile Val Phe Pro Glu Thr
           35                  40                  45
   Phe Ile Pro Thr Tyr Pro Asp Trp Val Trp Arg Ala Pro Ala Trp Asp
       50                  55                  60
   Gly Pro Ser Ala Asp Leu Tyr Ala Met Leu Leu Glu Asn Ala Val Glu
   65                  70                  75                  80
   Ile Pro Gly Pro Val Thr Glu Thr Leu Gly Lys Ala Ala Lys Gln Ala
                   85                  90                  95
   Lys Ala Phe Val Ser Met Gly Val Asn Glu Arg Glu Pro Gly Gly Gly
               100                 105                 110
   Thr Ile Tyr Asn Thr Gln Val Thr Phe Gly Pro Asp Gly Ser Val Leu
           115                 120                 125
   Gly Lys His Arg Lys Leu Met Pro Thr Gly Gly Glu Arg Leu Val Trp
       130                 135                 140
   Gly Met Gly Asp Gly Ser Met Leu Gln Val Tyr Asp Thr Pro Phe Gly
   145                 150                 155                 160
   Arg Leu Gly Gly Leu Ile Cys Trp Glu Asn Tyr Met Pro Leu Ala Arg
                   165                 170                 175
   Tyr Ser Met Tyr Ala Lys Gly Val Asp Val Tyr Val Ala Pro Thr Trp
               180                 185                 190
   Asp Asn Ser Asp Met Trp Val Ala Thr Leu Arg His Ile Ala Lys Glu
           195                 200                 205
   Gly Arg Leu Tyr Val Ile Gly Val Ala Pro Leu Leu Arg Gly Ser Asp
       210                 215                 220
   Val Pro Asp Asp Val Pro Gly Lys Ala Glu Leu Trp Gly Gly Asp Asp
   225                 230                 235                 240
   Asp Trp Met Ser Arg Gly Phe Ser Thr Ile Val Ala Pro Gly Gly Glu
                   245                 250                 255
   Val Leu Ala Gly Pro Leu Thr Glu Glu Glu Gly Ile Leu Tyr Ala Glu
               260                 265                 270
   Ile Asp Pro Ala Arg Ala Arg Ser Ser Arg His Gln Phe Asp Pro Val
           275                 280                 285
   Gly His Tyr Ser Arg Pro Asp Val Phe Arg Leu Val Val Asp Glu Ser
       290                 295                 300
   Pro Lys Pro Gln Thr Ser Gly Pro Gly
   305                 310
<210> 151
   <211> 993
   <212> DNA
   <213> Unknown
<220>
   <223> Obtained from an environmental sample
<400> 151
   atgagagtcg ttaaagccgc cgcggtccaa ctgaaaccag tcctttatag ccgtgaggga     60
   acagtcgata acgtcgtcaa gaagatccac gagctgggcc aacaaggagt gcagttcgca    120
   acgttcccgg aaaccgtggt gccttactat ccgtactttt cgatcgtgca gtccggctat    180
   caaatccttg ccggcggtga gttcctaaag ctgcttgatc agtcagtgac cgtgccatct    240
   cttgccaccg aagcgatcgg cgaggcctgc aggcaagcgg gcgtcgttgt ctccatcggc    300
   gtcaacgagc gtgacggggg aactctgtac aatacgcaac ttctctttga tgccgacggc    360
   acgttgattc aaagacgacg caagatcacg cccacccatt acgagcgcat ggtctggggc    420
   cagggcgatg gctcaggttt acgggcggtt gacagcaagg tcgcgcgcat tggtcaactg    480
   gcttgttttg agcactacaa cccgcttgcg cgttacgcca tgatggccga tggcgagcaa    540
   atccactctg cgatgttccc gggctccatg ttcggcgatg cgttttcaga gaaggtggaa    600
   atcaacgtaa ggcagcatgc aatggagtct ggatgctttg tcgtctgcgc tacggcctgg    660
   ctggatgccg accaacaggc acaaatcatg aaggacacag gctgcgagat cggtccgatc    720
   tcgggcggtt gcttcaccgc tatcgtgaca cccgacggga cgctgatagg cgaacccatc    780
   cactcgggcg aaggcgtttg tattgccgac ctcgatttca agctcatcga caagcggaag    840
   cacgtggtgg acacgcgcgg ccactacagc cggccagaat tgctcagcct cctaattgat    900
   cggactccca cggcacacat acacgaacgg aacgagcaac cgaagtcggc cgttgagcaa    960
   gactcgcaga atgtattcac cgctattgct taa                                 993
<210> 152
   <211> 330
   <212> PRT
   <213> Unknown
<220>
   <223> Obtained from an environmental sample
<400> 152
   Met Arg Val Val Lys Ala Ala Ala Val Gln Leu Lys Pro Val Leu Tyr
   1               5                   10                  15
   Ser Arg Glu Gly Thr Val Asp Asn Val Val Lys Lys Ile His Glu Leu
               20                  25                  30
   Gly Gln Gln Gly Val Gln Phe Ala Thr Phe Pro Glu Thr Val Val Pro
           35                  40                  45
   Tyr Tyr Pro Tyr Phe Ser Ile Val Gln Ser Gly Tyr Gln Ile Leu Ala
       50                  55                  60
   Gly Gly Glu Phe Leu Lys Leu Leu Asp Gln Ser Val Thr Val Pro Ser
   65                  70                  75                  80
   Leu Ala Thr Glu Ala Ile Gly Glu Ala Cys Arg Gln Ala Gly Val Val
                   85                  90                  95
   Val Ser Ile Gly Val Asn Glu Arg Asp Gly Gly Thr Leu Tyr Asn Thr
               100                 105                 110
   Gln Leu Leu Phe Asp Ala Asp Gly Thr Leu Ile Gln Arg Arg Arg Lys
           115                 120                 125
   Ile Thr Pro Thr His Tyr Glu Arg Met Val Trp Gly Gln Gly Asp Gly
       130                 135                 140
   Ser Gly Leu Arg Ala Val Asp Ser Lys Val Ala Arg Ile Gly Gln Leu
   145                 150                 155                 160
   Ala Cys Phe Glu His Tyr Asn Pro Leu Ala Arg Tyr Ala Met Met Ala
                   165                 170                 175
   Asp Gly Glu Gln Ile His Ser Ala Met Phe Pro Gly Ser Met Phe Gly
               180                 185                 190
   Asp Ala Phe Ser Glu Lys Val Glu Ile Asn Val Arg Gln His Ala Met
           195                 200                 205
   Glu Ser Gly Cys Phe Val Val Cys Ala Thr Ala Trp Leu Asp Ala Asp
       210                 215                 220
   Gln Gln Ala Gln Ile Met Lys Asp Thr Gly Cys Glu Ile Gly Pro Ile
   225                 230                 235                 240
   Ser Gly Gly Cys Phe Thr Ala Ile Val Thr Pro Asp Gly Thr Leu Ile
                   245                 250                 255
   Gly Glu Pro Ile His Ser Gly Glu Gly Val Cys Ile Ala Asp Leu Asp
               260                 265                 270
   Phe Lys Leu Ile Asp Lys Arg Lys His Val Val Asp Thr Arg Gly His
           275                 280                 285
   Tyr Ser Arg Pro Glu Leu Leu Ser Leu Leu Ile Asp Arg Thr Pro Thr
       290                 295                 300
   Ala His Ile His Glu Arg Asn Glu Gln Pro Lys Ser Ala Val Glu Gln
   305                 310                 315                 320
   Asp Ser Gln Asn Val Phe Thr Ala Ile Ala
                   325                 330
<210> 153
   <211> 1074
   <212> DNA
   <213> Unknown
<220>
   <223> Obtained from an environmental sample
<400> 153
   atgccaaacg caagaaagat tgttggagcc gtggcccaag ttgcacagga attcttcgac 60
   actgaagcga atctcggtaa agcgatagcg gcgattcaca atgctgcgaa gcaaggcgca 120
   gatatcgtcg tcttcgccga atgctatttg ggccaatatc catattgggc gcaattttac 180
   gacaactctg ccaagaacta ttccaaggtt tggacggccc tgtacgacgg tgcgatcact 240
   gtgggtggcg atgaatgccg ggctattgct gctgcggcta gacagtccaa gattcatgtc 300
   gtcatgggtt gcaatgagct atccgaccga gccggcggcg caacgttata caacagcctc 360
   ttgtttttcg accgaaaggg cgagttgatc ggtcgacacc ggaaattgat gccgtcgatg 420
   cacgagcggt tgatccatgg cacaggcgac ggaagagact tgaatgttta cgataccgat 480
   atcggtatgt tgggtgggtt gatttgctgg gagcaccata tgtcgctctc gaagtatgcc 540
   atggcgacta tgggtgaaga agttcatgtt gcaagctggc ctgggatgtg gcgcggagga 600
   gacgcggcaa tcggtgagag gatggtcgaa gcggatcttg gggcgccgtt tgtttgtgac 660
   gccgaatttg cgatccgaga atatgcggca gagacaggaa atttcgttct aagcgcgtct 720
   ggatattttc cgaaggacaa tatatccgat gagtggcgcg aagcgattcc aaaccttcaa 780
   gcgcagtggg ctgtgggcgg gagttctatc gtggcaccgg ggggctccta tctggtccca 840
   ccactcatta atgaggagaa gatcctctgc gccgaactcg atttcaatct caggcgtctt 900
   tggaaagcct ggatcgatcc gattggtcac tattcgcgtc ccgatgttta tagcctgcaa 960
   ctgcataacg ttgctgggcg tgagtattcc tatcaggccg tagatttgaa gcgcacgcca 1020
   aagccccaat cgctgtgggt agatgcgtcc gaggaagacg gtgcgctgaa ttga 1074
<210> 154
   <211> 357
   <212> PRT
   <213> Unknown
<220>
   <223> Obtained from an environmental sample
<400> 154
   Met Pro Asn Ala Arg Lys Ile Val Gly Ala Val Ala Gln Val Ala Gln
   1               5                   10                  15
   Glu Phe Phe Asp Thr Glu Ala Asn Leu Gly Lys Ala Ile Ala Ala Ile
               20                  25                  30
   His Asn Ala Ala Lys Gln Gly Ala Asp Ile Val Val Phe Ala Glu Cys
           35                  40                  45
   Tyr Leu Gly Gln Tyr Pro Tyr Trp Ala Gln Phe Tyr Asp Asn Ser Ala
       50                  55                  60
   Lys Asn Tyr Ser Lys Val Trp Thr Ala Leu Tyr Asp Gly Ala Ile Thr
   65                  70                  75                  80
   Val Gly Gly Asp Glu Cys Arg Ala Ile Ala Ala Ala Ala Arg Gln Ser
                   85                  90                  95
   Lys Ile His Val Val Met Gly Cys Asn Glu Leu Ser Asp Arg Ala Gly
               100                 105                 110
   Gly Ala Thr Leu Tyr Asn Ser Leu Leu Phe Phe Asp Arg Lys Gly Glu
           115                 120                 125
   Leu Ile Gly Arg His Arg Lys Leu Met Pro Ser Met His Glu Arg Leu
       130                 135                 140
   Ile His Gly Thr Gly Asp Gly Arg Asp Leu Asn Val Tyr Asp Thr Asp
   145                 150                 155                 160
   Ile Gly Met Leu Gly Gly Leu Ile Cys Trp Glu His His Met Ser Leu
                   165                 170                 175
   Ser Lys Tyr Ala Met Ala Thr Met Gly Glu Glu Val His Val Ala Ser
               180                 185                 190
   Trp Pro Gly Met Trp Arg Gly Gly Asp Ala Ala Ile Gly Glu Arg Met
           195                 200                 205
   Val Glu Ala Asp Leu Gly Ala Pro Phe Val Cys Asp Ala Glu Phe Ala
       210                 215                 220
   Ile Arg Glu Tyr Ala Ala Glu Thr Gly Asn Phe Val Leu Ser Ala Ser
   225                 230                 235                 240
   Gly Tyr Phe Pro Lys Asp Asn Ile Ser Asp Glu Trp Arg Glu Ala Ile
                   245                 250                 255
   Pro Asn Leu Gln Ala Gln Trp Ala Val Gly Gly Ser Ser Ile Val Ala
               260                 265                 270
   Pro Gly Gly Ser Tyr Leu Val Pro Pro Leu Ile Asn Glu Glu Lys Ile
           275                 280                 285
   Leu Cys Ala Glu Leu Asp Phe Asn Leu Arg Arg Leu Trp Lys Ala Trp
       290                 295                 300
   Ile Asp Pro Ile Gly His Tyr Ser Arg Pro Asp Val Tyr Ser Leu Gln
   305                 310                 315                 320
   Leu His Asn Val Ala Gly Arg Glu Tyr Ser Tyr Gln Ala Val Asp Leu
                   325                 330                 335
   Lys Arg Thr Pro Lys Pro Gln Ser Leu Trp Val Asp Ala Ser Glu Glu
               340                 345                 350
   Asp Gly Ala Leu Asn
           355
<210> 155
   <211> 1041
   <212> DNA
   <213> Unknown
<220>
   <223> Obtained from an environmental sample
<400> 155
   atgggcatcg aacatccgaa atacaaggtg gccgtggtgc aggccgcgcc ggcctggctc      60
   gatctcgacg gctcgatcaa gaaggcgatt gcgctgatcg aggaagcggc cgccaagggc     120
   gctaagctga tcgctttccc cgaaaccttc attcccggct atccctggca catctggctg      180
   gactcgccgg cctgggcgat cggccgcggc tttgtgcagc gctacttcga taactcgctg      240
   gcctacgaca gcccgcaagc cgaaaagctg cgcgccgcgg tcaagaaggc caagctcact      300
   gccgtgattg gcctgtcgga gcgcgacggc ggcagcctct atatagcgca atggctgatt      360
   ggccctgatg gcgagaccat cgcaaaacgc agaaagctgc ggccaacgca cgcggaacgc      420
   accgtttttg gcgagggtga cggcagcgac cttgccgtgc acgaccggcc cggaatcggg      480
   cggctgggag cgctgtgctg ctgggagcac ctgcaaccgc tttcgaaata cgcgatgtat      540
   gcgcagaacg aacaggtcca tgtcgcgtca tggccgagct tctcgctcta cgaccccttc      600
   gcgccggcgc tcggcgccga ggtcaacaat gcggcttccc gcgtctacgc ggtcgagggc      660
   tcgtgcttcg tgctggcgcc gtgcgccacg gtttcgcaag ccatgatcga cgagctgtgt      720
   gaccggccgg acaagcatgc gctgttgcac gccggtggcg gacacgccgc gatttacggc      780
   ccggacggca gctcgatcgc ggagaagctg ccgcaggacg cggagggcct gttgatcgcc      840
   gagatcgatc tcggggcgat cggggttgcc aagaatgcag ccgacccggc cggtcattat      900
   tcgcggccgg acgtgacgcg actcctgctg aacaagaacc ggatgcgaag ggtcgaggag      960
   tttgcgctgc cggtcgatcc ggtcgcaacg accgaggagg agcaagtcgc gacgccgtcg     1020
   aggcccagcc aggccgcgta a                                               1041
<210> 156
   <211> 346
   <212> PRT
   <213> Unknown
<220>
   <223> Obtained from an environmental sample
<400> 156
   Met Gly Ile Glu His Pro Lys Tyr Lys Val Ala Val Val Gln Ala Ala
   1               5                   10                  15
   Pro Ala Trp Leu Asp Leu Asp Gly Ser Ile Lys Lys Ala Ile Ala Leu
               20                  25                  30
   Ile Glu Glu Ala Ala Ala Lys Gly Ala Lys Leu Ile Ala Phe Pro Glu
           35                  40                  45
   Thr Phe Ile Pro Gly Tyr Pro Trp His Ile Trp Leu Asp Ser Pro Ala
       50                  55                  60
   Trp Ala Ile Gly Arg Gly Phe Val Gln Arg Tyr Phe Asp Asn Ser Leu
   65                  70                  75                  80
   Ala Tyr Asp Ser Pro Gln Ala Glu Lys Leu Arg Ala Ala Val Lys Lys
                   85                  90                  95
   Ala Lys Leu Thr Ala Val Ile Gly Leu Ser Glu Arg Asp Gly Gly Ser
               100                 105                 110
   Leu Tyr Ile Ala Gln Trp Leu Ile Gly Pro Asp Gly Glu Thr Ile Ala
           115                 120                 125
   Lys Arg Arg Lys Leu Arg Pro Thr His Ala Glu Arg Thr Val Phe Gly
       130                 135                 140
   Glu Gly Asp Gly Ser Asp Leu Ala Val His Asp Arg Pro Gly Ile Gly
   145                 150                 155                 160
   Arg Leu Gly Ala Leu Cys Cys Trp Glu His Leu Gln Pro Leu Ser Lys
                   165                 170                 175
   Tyr Ala Met Tyr Ala Gln Asn Glu Gln Val His Val Ala Ser Trp Pro
               180                 185                 190
   Ser Phe Ser Leu Tyr Asp Pro Phe Ala Pro Ala Leu Gly Ala Glu Val
           195                 200                 205
   Asn Asn Ala Ala Ser Arg Val Tyr Ala Val Glu Gly Ser Cys Phe Val
       210                 215                 220
   Leu Ala Pro Cys Ala Thr Val Ser Gln Ala Met Ile Asp Glu Leu Cys
   225                 230                 235                 240
   Asp Arg Pro Asp Lys His Ala Leu Leu His Ala Gly Gly Gly His Ala
                   245                 250                 255
   Ala Ile Tyr Gly Pro Asp Gly Ser Ser Ile Ala Glu Lys Leu Pro Gln
               260                 265                 270
   Asp Ala Glu Gly Leu Leu Ile Ala Glu Ile Asp Leu Gly Ala Ile Gly
           275                 280                 285
   Val Ala Lys Asn Ala Ala Asp Pro Ala Gly His Tyr Ser Arg Pro Asp
       290                 295                 300
   Val Thr Arg Leu Leu Leu Asn Lys Asn Arg Met Arg Arg Val Glu Glu
   305                 310                 315                 320
   Phe Ala Leu Pro Val Asp Pro Val Ala Thr Thr Glu Glu Glu Gln Val
                   325                 330                 335
   Ala Thr Pro Ser Arg Pro Ser Gln Ala Ala
                       340                 345
<210> 157
   <211> 1011
   <212> DNA
   <213> Unknown
<220>
   <223> Obtained from an environmental sample
<400> 157
   atgagagtcg ttaaagctgc tgcggtccaa ctgagtcccg tgctgtatag ccgtgaggga      60
   acagtagaaa aggtcgttcg gaagatccac gagcttggcg atcaaggagt cgagttcgcc     120
   acgttcccgg agaccgtagt gccctactat ccgtacttct cggccgtcca gacgccgatt     180
   cagaacatgc acggcccgga gcacctgaag ttgctcgagc aatcggtgac cgtcccgtcg     240
   cccgccaccg acgcgatcgg cgacgcctgc cgccacgccg gcgtcgtcgt ctcgatcggc     300
   gtcaacgaac gcgatggcgg cacgatctac aacacgcagc tcctgttcga cgccgacggc     360
   accttgatcc agcgccggcg aaagatcacg ccgaccttct acgaacgaat ggtctgggga     420
   cagggtgacg gttcggggct gcgcgccgtc gacagccgcg taggacgcat cggccagctc     480
   gcctgtttcg agcactacaa cccgctggcg cgctacgcca tgatggccga cggcgagcag     540
   attcactccg cgatgtaccc cggctccatc tttggagacg cattcgcgca gaaaatcgag     600
   atcaacatcc gccagcacgc gctcgagtcc ggtgcgttcg tcgtcaacgc caccgcctgg     660
   ctcgatgccg accagcaggc gcggatcatg aaggataccg gctgcaccat cgaaccgatc     720
   tcgggcggtt gcttcaccgc catcgtcacc ccggacggga ccctgctggg cgaagcgata     780
   cgttcggggg agggagtggt ggtcgccgat ctcgacttca cgctgatcga caggcgcaag     840
   caagtgatgg actctcgtgg tcactacagt cggccggagt tgctcagcct tctgatcgac     900
   cgcacaccca ccgcacacct acacgaacgc gaagcgcacc ccagagcaag tgaggactgg     960
   caaggttccg agagtctgcg cgccatgcag gcctcggcac cgaaggtctg a             1011
<210> 158
   <211> 336
   <212> PRT
   <213> Unknown
<220>
   <223> Obtained from an environmental sample
<400> 158
   Met Arg Val Val Lys Ala Ala Ala Val Gln Leu Ser Pro Val Leu Tyr
   1               5                   10                  15
   Ser Arg Glu Gly Thr Val Glu Lys Val Val Arg Lys Ile His Glu Leu
               20                  25                  30
   Gly Asp Gln Gly Val Glu Phe Ala Thr Phe Pro Glu Thr Val Val Pro
           35                  40                  45
   Tyr Tyr Pro Tyr Phe Ser Ala Val Gln Thr Pro Ile Gln Asn Met His
       50                  55                  60
   Gly Pro Glu His Leu Lys Leu Leu Glu Gln Ser Val Thr Val Pro Ser
   65                  70                  75                  80
   Pro Ala Thr Asp Ala Ile Gly Asp Ala Cys Arg His Ala Gly Val Val
                   85                  90                  95
   Val Ser Ile Gly Val Asn Glu Arg Asp Gly Gly Thr Ile Tyr Asn Thr
               100                 105                 110
   Gln Leu Leu Phe Asp Ala Asp Gly Thr Leu Ile Gln Arg Arg Arg Lys
           115                 120                 125
   Ile Thr Pro Thr Phe Tyr Glu Arg Met Val Trp Gly Gln Gly Asp Gly
       130                 135                 140
   Ser Gly Leu Arg Ala Val Asp Ser Arg Val Gly Arg Ile Gly Gln Leu
   145                 150                 155                 160
   Ala Cys Phe Glu His Tyr Asn Pro Leu Ala Arg Tyr Ala Met Met Ala
                   165                 170                 175
   Asp Gly Glu Gln Ile His Ser Ala Met Tyr Pro Gly Ser Ile Phe Gly
               180                 185                 190
   Asp Ala Phe Ala Gln Lys Ile Glu Ile Asn Ile Arg Gln His Ala Leu
           195                 200                 205
   Glu Ser Gly Ala Phe Val Val Asn Ala Thr Ala Trp Leu Asp Ala Asp
       210                 215                 220
   Gln Gln Ala Arg Ile Met Lys Asp Thr Gly Cys Thr Ile Glu Pro Ile
   225                 230                 235                 240
   Ser Gly Gly Cys Phe Thr Ala Ile Val Thr Pro Asp Gly Thr Leu Leu
                   245                 250                 255
   Gly Glu Ala Ile Arg Ser Gly Glu Gly Val Val Val Ala Asp Leu Asp
               260                 265                 270
   Phe Thr Leu Ile Asp Arg Arg Lys Gln Val Met Asp Ser Arg Gly His
           275                 280                 285
   Tyr Ser Arg Pro Glu Leu Leu Ser Leu Leu Ile Asp Arg Thr Pro Thr
       290                 295                 300
   Ala His Leu His Glu Arg Glu Ala His Pro Arg Ala Ser Glu Asp Trp
   305                 310                 315                 320
   Gln Gly Ser Glu Ser Leu Arg Ala Met Gln Ala Ser Ala Pro Lys Val
                   325                 330                 335
<210> 159
   <211> 930
   <212> DNA
   <213> Unknown
<220>
   <223> Obtained from an environmental sample
<400> 159
   atgtcatcaa ccgtgacggt tgccattatt caggcagcac ccgtgtatta tgacctgcct     60
   gccacgctgg acaaagccgc caaactggtg gcggatgcgg cggcacaggg cgcaacgctg    120
   attgtcttcg gcgagacatg gtttccgggg tatccggcat ggctggatta ctgccccaat    180
   gtcgcgctgt ggaatcatcc cccgaccaag caggtatttg agcgcctgca tcgcaacagc    240
   atcgctgtgc caagcaagga actcgatttt ctgggggcgc tggcacgcaa gcatcaggtg    300
   gtgctggtgt tgagcattaa tgaacgtgtg gagcagggcg cggggcatgg cacgctgtat    360
   aacacgctgc tcacgattga cgccgatggc acgctggcaa atcatcatcg caaactgatg    420
   ccgacctata ccgagcgcat ggtgtggggc atgggcgacg gggtggggtt gcaagcggtg     480
   gatactgccg tcgggcgcgt aggcggctta atctgctggg aacactggat gccgttggca    540
   cgccagacca tgcacatcag cggcgaacag attcatattt ccgtcttccc aaccgtccat    600
   gagatgcacc agattgccag ccgccagtat gcctttgaag ggcggacgtt tgtgctgacc    660
   gttggcggca ttcttgcggc acaggacttg cccgccgaac tggaacgccc cgccgatttg    720
   ccgcccacgc agcttgtcca gcgcggcggc agcgccatta tcgcgccgga tggtcgttat    780
   ctggcgggtc cagtctataa tgaggaaacc atcctgaccg caacgctgga tttgggcgag    840
   atcatccgcg agagcatgac gctggatgtc accggacatt atgcccgccc ggatgttttt    900
   gacctgaccg tgaagcgcag ccgaccatga                                     930
<210> 160
   <211> 309
   <212> PRT
   <213> Unknown
<220>
   <223> Obtained from an environmental sample
<400> 160
   Met Ser Ser Thr Val Thr Val Ala Ile Ile Gln Ala Ala Pro Val Tyr
    1               5                  10                  15
   Tyr Asp Leu Pro Ala Thr Leu Asp Lys Ala Ala Lys Leu Val Ala Asp
               20                  25                  30
   Ala Ala Ala Gln Gly Ala Thr Leu Ile Val Phe Gly Glu Thr Trp Phe
           35                  40                  45
   Pro Gly Tyr Pro Ala Trp Leu Asp Tyr Cys Pro Asn Val Ala Leu Trp
       50                  55                  60
   Asn His Pro Pro Thr Lys Gln Val Phe Glu Arg Leu His Arg Asn Ser
   65                  70                  75                  80
   Ile Ala Val Pro Ser Lys Glu Leu Asp Phe Leu Gly Ala Leu Ala Arg
                   85                  90                  95
   Lys His Gln Val Val Leu Val Leu Ser Ile Asn Glu Arg Val Glu Gln
               100                 105                 110
   Gly Ala Gly His Gly Thr Leu Tyr Asn Thr Leu Leu Thr Ile Asp Ala
           115                 120                 125
   Asp Gly Thr Leu Ala Asn His His Arg Lys Leu Met Pro Thr Tyr Thr
       130                 135                 140
   Glu Arg Met Val Trp Gly Met Gly Asp Gly Val Gly Leu Gln Ala Val
   145                 150                 155                 160
   Asp Thr Ala Val Gly Arg Val Gly Gly Leu Ile Cys Trp Glu His Trp
                   165                 170                 175
   Met Pro Leu Ala Arg Gln Thr Met His Ile Ser Gly Glu Gln Ile His
               180                 185                 190
   Ile Ser Val Phe Pro Thr Val His Glu Met His Gln Ile Ala Ser Arg
           195                 200                 205
   Gln Tyr Ala Phe Glu Gly Arg Thr Phe Val Leu Thr Val Gly Gly Ile
       210                 215                 220
   Leu Ala Ala Gln Asp Leu Pro Ala Glu Leu Glu Arg Pro Ala Asp Leu
   225                 230                 235                 240
   Pro Pro Thr Gln Leu Val Gln Arg Gly Gly Ser Ala Ile Ile Ala Pro
                   245                 250                 255
   Asp Gly Arg Tyr Leu Ala Gly Pro Val Tyr Asn Glu Glu Thr Ile Leu
               260                 265                 270
   Thr Ala Thr Leu Asp Leu Gly Glu Ile Ile Arg Glu Ser Met Thr Leu
           275                 280                 285
   Asp Val Thr Gly His Tyr Ala Arg Pro Asp Val Phe Asp Leu Thr Val
       290                 295                 300
   Lys Arg Ser Arg Pro
   305
<210> 161
   <211> 1008
   <212> DNA
   <213> Unknown
<220>
   <223> Obtained from an environmental sample
<400> 161
   atgaccacca tccgcgccgc cgccgtgcag tttagcccgg tgctgtactc gcgccaggcc      60
   accgtcgaca agctgtgccg caccctgctg gaactgggcc gcgaaggggt gcagttcgcg     120
   gtattcccgg aaaccgtggt gccgtactac ccatattttt ccttcgtgca gccaccgttc     180
   gccatgggca aacaacacct gttgctgctc gagcaatccg tcactgtgcc ctctgacgtc     240
   acccggcaga tcggtgaggc ctgccgggaa gcggggatcg tcgccagcat cggcgtcaac     300
   gaacgcgacg gcggcactat ttataacgcg cagttgctgt tcgatgccga cggcagcctg     360
   attcagcagc ggcgcaagat caccccgacc tatcacgaac gcatggtctg ggggcagggc     420
   gatggttccg gcctgcgcgc cgtggacagt gcggtggggc gtatcggttc cctggcctgc     480
   tgggaacatt acaaccccct ggcgcgctac gcgctgatgg ccgatggcga acagattcat     540
   gtggcgatgt ttcccggctc cctggtcggc gacatctttg ccgagcagat cgaagtcacc     600
   atccgccacc acgccctgga aagcggctgc ttcgtggtca acgccacggc ttggctggat     660
   gccgaccagc agggccggat catgcaggac accggctgcg agttggggcc gatttccggc     720
   ggctgtttta ccgcgatcat ttccccggag ggcaaggttc tcggcgagcc gctgcgcagc     780
   ggcgaagggg tggtcattgc tgacctcgac ctggccctga tcgacaagcg caaacgcatg     840
   atggattcgg tcggtcacta cagccgcccg gaactgctca gcctgcttat cgaccgcagc     900
   ccgaccgccc acgtgcatga acttgccgcc gcgcttaatc ctgccaggga gtctgatcca     960
   ctagtgtcga cctgcaggcg cgcgagctcc agcttttgtt ccctttag                1008
<210> 162
   <211> 335
   <212> PRT
   <213> Unknown
<220>
   <223> Obtained from an environmental sample
<400> 162
   Met Thr Thr Ile Arg Ala Ala Ala Val Gln Phe Ser Pro Val Leu Tyr
   1               5                   10                  15
   Ser Arg Gln Ala Thr Val Asp Lys Leu Cys Arg Thr Leu Leu Glu Leu
               20                  25                  30
   Gly Arg Glu Gly Val Gln Phe Ala Val Phe Pro Glu Thr Val Val Pro
           35                  40                  45
   Tyr Tyr Pro Tyr Phe Ser Phe Val Gln Pro Pro Phe Ala Met Gly Lys
       50                  55                  60
   Gln His Leu Leu Leu Leu Glu Gln Ser Val Thr Val Pro Ser Asp Val
   65                  70                  75                  80
   Thr Arg Gln Ile Gly Glu Ala Cys Arg Glu Ala Gly Ile Val Ala Ser
                   85                  90                  95
   Ile Gly Val Asn Glu Arg Asp Gly Gly Thr Ile Tyr Asn Ala Gln Leu
               100                 105                 110
   Leu Phe Asp Ala Asp Gly Ser Leu Ile Gln Gln Arg Arg Lys Ile Thr
           115                 120                 125
   Pro Thr Tyr His Glu Arg Met Val Trp Gly Gln Gly Asp Gly Ser Gly
       130                 135                 140
   Leu Arg Ala Val Asp Ser Ala Val Gly Arg Ile Gly Ser Leu Ala Cys
   145                 150                 155                 160
   Trp Glu His Tyr Asn Pro Leu Ala Arg Tyr Ala Leu Met Ala Asp Gly
                   165                 170                 175
   Glu Gln Ile His Val Ala Met Phe Pro Gly Ser Leu Val Gly Asp Ile
               180                 185                 190
   Phe Ala Glu Gln Ile Glu Val Thr Ile Arg His His Ala Leu Glu Ser
           195                 200                 205
   Gly Cys Phe Val Val Asn Ala Thr Ala Trp Leu Asp Ala Asp Gln Gln
       210                 215                 220
   Gly Arg Ile Met Gln Asp Thr Gly Cys Glu Leu Gly Pro Ile Ser Gly
   225                 230                 235                 240
   Gly Cys Phe Thr Ala Ile Ile Ser Pro Glu Gly Lys Val Leu Gly Glu
                   245                 250                 255
   Pro Leu Arg Ser Gly Glu Gly Val Val Ile Ala Asp Leu Asp Leu Ala
               260                 265                 270
   Leu Ile Asp Lys Arg Lys Arg Met Met Asp Ser Val Gly His Tyr Ser
           275                 280                 285
   Arg Pro Glu Leu Leu Ser Leu Leu Ile Asp Arg Ser Pro Thr Ala His
       290                 295                 300
   Val His Glu Leu Ala Ala Ala Leu Asn Pro Ala Arg Glu Ser Asp Pro
   305                 310                 315                 320
   Leu Val Ser Thr Cys Arg Arg Ala Ser Ser Ser Phe Cys Ser Leu
                   325                 330                 335
<210> 163
   <211> 978
   <212> DNA
   <213> Unknown
<220>
   <223> Obtained from an environmental sample
<400> 163
   gtgaccatca tcaaagccgc cgcagtgcag atcagccccg tgctttacag ccgggaagcc      60
   accgtcgaaa aggtcgttcg cgagacccgc gaactcggcc agaagggcgt gcagttcgca     120
   acgtttccgg aaaccgtggt gccgtactac ccatacttct ccgccgtcca gacgggcatc     180
   gaactgctgt ccggcaaaga gcacctgcga ctgctggagc aggccgtgac tgttccttcc     240
   cccgccactg atgcgattgc ccaggcggca cgcgaggccg gcatggtggt gtcgatcggc     300
   gtcaacgagc gtgacggcgg caccatctac aacacgcagc tgctctttga tgccgacggc     360
   acgctggtgc agcgccgccg caagatcacg ccgacgcatt tcgagcgcat ggtgtggggc     420
   cagggcgacg gttcgggcct gcgcgcagtg gataccaagg tcggccgcat tggccagctg     480
   gcctgcttcg agcacaacaa cccgctcgcg cgctacgcaa tgatggccga tggcgagcag     540
   atccattcct ccatgtaccc gggctccgcc ttcggcgacg gattcgcgca gcgcatggag     600
   atcaacattc gccaacacgc cctggagtcg ggttgcttcg tggtgaatgc caccgcgtgg     660
   ctcgacgccg accagcaggc gcagatcatg aaggacacgg gctgcgccat cgggccgatc     720
   tctggcggct gcttcacgac catcgtcacg ccggacggca tgctgatcgg cgaacccctc     780
   cgcgagggcg agggcgagat catcgccgac ctcgatttca ccctgatcga ccgccgcaag     840
   ctgctgatgg actcggtcgg ccactacaac cgtccggagc tgctgagcct gctgatcgac     900
   cgcacacccg cggcgaactt ccatgagcgc agtacgcatc cggccgtcga tgccgccagc     960
   ggcctcgaaa tcctctaa                                                   978
<210> 164
   <211> 325
   <212> PRT
   <213> unknown
<220>
   <223> obtained from an environmental sample
<400> 164
   Val Thr Ile Ile Lys Ala Ala Ala Val Gln Ile Ser Pro Val Leu Tyr
   1               5                   10                  15
   Ser Arg Glu Ala Thr Val Glu Lys Val Val Arg Glu Thr Arg Glu Leu
               20                  25                  30
   Gly Gln Lys Gly Val Gln Phe Ala Thr Phe Pro Glu Thr Val Val Pro
           35                  40                  45
   Tyr Tyr Pro Tyr Phe Ser Ala Val Gln Thr Gly Ile Glu Leu Leu Ser
       50                  55                  60
   Gly Lys Glu His Leu Arg Leu Leu Glu Gln Ala Val Thr Val Pro Ser
   65                  70                  75                  80
   Pro Ala Thr Asp Ala Ile Ala Gln Ala Ala Arg Glu Ala Gly Met Val
                   85                  90                  95
   Val Ser Ile Gly Val Asn Glu Arg Asp Gly Gly Thr Ile Tyr Asn Thr
               100                 105                 110
   Gln Leu Leu Phe Asp Ala Asp Gly Thr Leu Val Gln Arg Arg Arg Lys
           115                 120                 125
   Ile Thr Pro Thr His Phe Glu Arg Met Val Trp Gly Gln Gly Asp Gly
       130                 135                 140
   Ser Gly Leu Arg Ala Val Asp Thr Lys Val Gly Arg Ile Gly Gln Leu
   145                 150                 155                 160
   Ala Cys Phe Glu His Asn Asn Pro Leu Ala Arg Tyr Ala Met Met Ala
                   165                 170                 175
   Asp Gly Glu Gln Ile His Ser Ser Met Tyr Pro Gly Ser Ala Phe Gly
               180                 185                 190
   Asp Gly Phe Ala Gln Arg Met Glu Ile Asn Ile Arg Gln His Ala Leu
           195                 200                 205
   Glu Ser Gly Cys Phe Val Val Asn Ala Thr Ala Trp Leu Asp Ala Asp
       210                 215                 220
   Gln Gln Ala Gln Ile Met Lys Asp Thr Gly Cys Ala Ile Gly Pro Ile
   225                 230                 235                 240
   Ser Gly Gly Cys Phe Thr Thr Ile Val Thr Pro Asp Gly Met Leu Ile
                   245                 250                 255
   Gly Glu Pro Leu Arg Glu Gly Glu Gly Glu Ile Ile Ala Asp Leu Asp
               260                 265                 270
   Phe Thr Leu Ile Asp Arg Arg Lys Leu Leu Met Asp Ser Val Gly His
           275                 280                 285
   Tyr Asn Arg Pro Glu Leu Leu Ser Leu Leu Ile Asp Arg Thr Pro Ala
       290                 295                 300
   Ala Asn Phe His Glu Arg Ser Thr His Pro Ala Val Asp Ala Ala Ser
   305                 310                 315                 320
   Gly Leu Glu Ile Leu
                   325
<210> 165
   <211> 1008
   <212> DNA
   <213> Unknown
<220>
   <223> Obtained from an environmental sample
<400> 165
   atggccaatt tcaaattcaa ggcggcggcg gtgcaggccg cgcccgcttt cctcgatctc     60
   gaggctagca tcgccaagtc gatcgccctg atcgaacaag ccgccgccaa cggcgccaag    120
   ctgatcgcct ttcccgaagt cttcattccc ggctacccct ggcacatctg gctcgacagt    180
   cccgcctggg cgatcgggcg cggcttcgtc tcgcgctatt tcgagaactc gctggactac    240
   aacagccccg aggccgagcg cctcaggctc gccgtcaaga aggcgggcct gacggcggtg    300
   atcggcctct ccgagcgcga cggcggcagc ctctacatcg cgcaatggat catcggccct    360
   gacggcgaga ccgttgcgaa acggcgtaag ctccggccga cccattgcga gcgcacggtc    420
   tatggagaag gcgacggcag cgacctcgcg gttcacgacg tatctggcat cggccgtctc    480
   ggcgcgctct gctgctggga gcatatccag ccgctgtcga aattcgcgat gtattcgcaa    540
   aatgagcaag tgcacgtcgc gtcctggccg agcttctcgc tctacgaccc gttcgcgccg    600
   gcgctgggcg ccgaggtcaa caacgcagcc tcgcggatct atgcggtcga aggctcatgc    660
   ttcgtcattg cgccctgcgc gaccgtttcg cctgcaatga tcgaggaact gtgcgacgcg    720
   ccaaacaaac atgcgcttct gcacgcgggc ggcggcttcg cgcgcatcta tgggccggac    780
   ggcgcttcga tcgccgagac gctgccgcca gatcaggaag gcttgatcta cgccgacatc    840
   gacctcaccg cgatcggcgt cgccaaggcc gccgccgatc ccgccggcca ttattcgcgc    900
   cccgacgtca cgcgcctgct cttcaacaag aagcccgctc ggcgagtcga aacttttgct    960
   ttgcccgtcg atgcgccggc gccggagacg cagaccgccg cgagctga                1008
<210> 166
   <211> 335
   <212> PRT
   <213> Unknown
<220>
   <223> Obtained from an environmental sample
<400> 166
   Met Ala Asn Phe Lys Phe Lys Ala Ala Ala Val Gln Ala Ala Pro Ala
   1               5                   10                  15
   Phe Leu Asp Leu Glu Ala Ser Ile Ala Lys Ser Ile Ala Leu Ile Glu
               20                  25                  30
   Gln Ala Ala Ala Asn Gly Ala Lys Leu Ile Ala Phe Pro Glu Val Phe
           35                  40                  45
   Ile Pro Gly Tyr Pro Trp His Ile Trp Leu Asp Ser Pro Ala Trp Ala
       50                  55                  60
   Ile Gly Arg Gly Phe Val Ser Arg Tyr Phe Glu Asn Ser Leu Asp Tyr
   65                  70                  75                  80
   Asn Ser Pro Glu Ala Glu Arg Leu Arg Leu Ala Val Lys Lys Ala Gly
                   85                  90                  95
   Leu Thr Ala Val Ile Gly Leu Ser Glu Arg Asp Gly Gly Ser Leu Tyr
               100                 105                 110
   Ile Ala Gln Trp Ile Ile Gly Pro Asp Gly Glu Thr Val Ala Lys Arg
           115                 120                 125
   Arg Lys Leu Arg Pro Thr His Cys Glu Arg Thr Val Tyr Gly Glu Gly
       130                 135                 140
   Asp Gly Ser Asp Leu Ala Val His Asp Val Ser Gly Ile Gly Arg Leu
   145                 150                 155                 160
   Gly Ala Leu Cys Cys Trp Glu His Ile Gln Pro Leu Ser Lys Phe Ala
                   165                 170                 175
   Met Tyr Ser Gln Asn Glu Gln Val His Val Ala Ser Trp Pro Ser Phe
               180                 185                 190
   Ser Leu Tyr Asp Pro Phe Ala Pro Ala Leu Gly Ala Glu Val Asn Asn
           195                 200                 205
   Ala Ala Ser Arg Ile Tyr Ala Val Glu Gly Ser Cys Phe Val Ile Ala
       210                 215                 220
   Pro Cys Ala Thr Val Ser Pro Ala Met Ile Glu Glu Leu Cys Asp Ala
   225                 230                 235                 240
   Pro Asn Lys His Ala Leu Leu His Ala Gly Gly Gly Phe Ala Arg Ile
                   245                 250                 255
   Tyr Gly Pro Asp Gly Ala Ser Ile Ala Glu Thr Leu Pro Pro Asp Gln
               260                 265                 270
   Glu Gly Leu Ile Tyr Ala Asp Ile Asp Leu Thr Ala Ile Gly Val Ala
           275                 280                 285
   Lys Ala Ala Ala Asp Pro Ala Gly His Tyr Ser Arg Pro Asp Val Thr
       290                 295                 300
   Arg Leu Leu Phe Asn Lys Lys Pro Ala Arg Arg Val Glu Thr Phe Ala
   305                 310                 315                 320
   Leu Pro Val Asp Ala Pro Ala Pro Glu Thr Gln Thr Ala Ala Ser
                   325                 330                 335
<210> 167
   <211> 1017
   <212> DNA
   <213> Unknown
<220>
   <223> Obtained from an environmental sample
<400> 167
   atgggtattg aacatccgaa gtacagggtt gccgtggtgc aggccgcacc ggcctggctc      60
   gatcttgacg cgtcgatcga caagtcgatc gcgctgatcg aggaggctgc ccagaaaggc     120
   gccaagctga tcgcattccc cgaggccttc atccccggct acccctggca tatctggatg     180
   gactcgcccg cctgggcgat tggccgcggt tttgtgcagc gctacttcga caattcgctg     240
   gcctatgaca gcccgcaggc cgagaagctg cgcgcggccg tgcgcaaggc aaaactcacg     300
   gccgtgatcg gcttgtcgga gcgtgacggc ggcagccttt atctcgcaca atggctgatc     360
   ggccccgacg gcgagaccat cgcaaaacgg cgcaagctgc ggccgacaca tgccgagcgc     420
   actgtgtacg gcgagggcga cggcagcgac cttgcggtcc acaatcgtcc ggacatcggc     480
   aggctcggtg cgctctgctg ctgggagcat cttcagccac tgtcgaaata cgcgatgtac     540
   gcgcagaacg agcaggtgca cgtcgcggcc tggccgagct tttcgctcta cgatcccttc     600
   gccgtggcgc tcggcgccga ggtgaacaac gcggcctccc gcgtctatgc ggtcgaaggc     660
   tcctgcttcg tgctggcgcc gtgcgcgaca gtctcgcaag ccatgatcga cgagctctgc     720
   gatcggccgg acaagcacgc gctgctgcat gtcggcggcg gctttgccgc gatctacggg     780
   cccgacggca gccagatcgg cgacaagctc gcccccgacc aggagggcct gttgatcgcc     840
   gagatcgatc tcggcgccat aggtgtcgcc aagaacgccg cggatcccgc cgggcactat     900
   tcgcggcccg acgtgacgcg gctgttgctc aacaagaaac cgtacaagcg cgtcgaacag     960
   ttctcgccgc cgtcggaggc ggttgaaccc acggatatcg cggcggcggc aagctga       1017
<210> 168
   <211> 338
   <212> PRT
   <213> Unknown
<220>
   <223> Obtained from an environmental sample
<400> 168
   Met Gly Ile Glu His Pro Lys Tyr Arg Val Ala Val Val Gln Ala Ala
   1               5                   10                  15
   Pro Ala Trp Leu Asp Leu Asp Ala Ser Ile Asp Lys Ser Ile Ala Leu
               20                  25                  30
   Ile Glu Glu Ala Ala Gln Lys Gly Ala Lys Leu Ile Ala Phe Pro Glu
           35                  40                  45
   Ala Phe Ile Pro Gly Tyr Pro Trp His Ile Trp Met Asp Ser Pro Ala
       50                  55                  60
   Trp Ala Ile Gly Arg Gly Phe Val Gln Arg Tyr Phe Asp Asn Ser Leu
   65                  70                  75                  80
   Ala Tyr Asp Ser Pro Gln Ala Glu Lys Leu Arg Ala Ala Val Arg Lys
                   85                  90                  95
   Ala Lys Leu Thr Ala Val Ile Gly Leu Ser Glu Arg Asp Gly Gly Ser
               100                 105                 110
   Leu Tyr Leu Ala Gln Trp Leu Ile Gly Pro Asp Gly Glu Thr Ile Ala
           115                 120                 125
   Lys Arg Arg Lys Leu Arg Pro Thr His Ala Glu Arg Thr Val Tyr Gly
       130                 135                 140
   Glu Gly Asp Gly Ser Asp Leu Ala Val His Asn Arg Pro Asp Ile Gly
   145                 150                 155                 160
   Arg Leu Gly Ala Leu Cys Cys Trp Glu His Leu Gln Pro Leu Ser Lys
                   165                 170                 175
   Tyr Ala Met Tyr Ala Gln Asn Glu Gln Val His Val Ala Ala Trp Pro
               180                 185                 190
   Ser Phe Ser Leu Tyr Asp Pro Phe Ala Val Ala Leu Gly Ala Glu Val
           195                 200                 205
   Asn Asn Ala Ala Ser Arg Val Tyr Ala Val Glu Gly Ser Cys Phe Val
       210                 215                 220
   Leu Ala Pro Cys Ala Thr Val Ser Gln Ala Met Ile Asp Glu Leu Cys
   225                 230                 235                 240
   Asp Arg Pro Asp Lys His Ala Leu Leu His Val Gly Gly Gly Phe Ala
                   245                 250                 255
   Ala Ile Tyr Gly Pro Asp Gly Ser Gln Ile Gly Asp Lys Leu Ala Pro
               260                 265                 270
   Asp Gln Glu Gly Leu Leu Ile Ala Glu Ile Asp Leu Gly Ala Ile Gly
           275                 280                 285
   Val Ala Lys Asn Ala Ala Asp Pro Ala Gly His Tyr Ser Arg Pro Asp
       290                 295                 300
   Val Thr Arg Leu Leu Leu Asn Lys Lys Pro Tyr Lys Arg Val Glu Gln
   305                 310                 315                 320
   Phe Ser Pro Pro Ser Glu Ala Val Glu Pro Thr Asp Ile Ala Ala Ala
                   325                 330                 335
   Ala Ser
<210> 169
   <211> 1077
   <212> DNA
   <213> Unknown
<220>
   <223> Obtained from an environmental sample
<210> 170
   <211> 358
   <212> PRT
   <213> Unknown
<220>
   <223> Obtained from an environmental sample
<400> 170
   Met Ala Leu Thr His Pro Lys Leu Lys Val Ala Ala Val Gln Ala Ala
   1 5 10 15
   Pro Ala Phe Leu Asp Val Asp Ala Ala Val Asp Lys Ala Val Arg Leu
   20 25 30
   Ile Asp Glu Ala Ala Ala Asn Gly Ser Ser Leu Val Ala Phe Pro Glu
   35 40 45
   Thr Trp Ile Pro Gly Tyr Pro Phe Trp Ile Trp Leu Gly Ser Pro Ala
   50 55 60
   Trp Ala Ile Met Arg Gly Phe Val Ser Arg Tyr Phe Asp Asn Ser Leu
   65 70 75 80
   Ser Tyr Asp Ser Arg Gln Ala Glu Arg Leu Arg Asp Ala Ala Lys Arg
   85 90 95
   His Lys Leu Thr Val Val Met Gly Leu Ser Glu Arg Ala Gly Gly Ser
   100 105 110
   Leu Tyr Ile Ala Gln Trp Ile Ile Gly Pro Asn Gly Glu Thr Val Ala
   115 120 125
   Gln Arg Arg Lys Leu Lys Pro Thr His Ala Glu Arg Thr Val Phe Gly
   130 135 140
   Glu Gly Asp Gly Ser His Leu Ala Val His Asn Leu Pro Ile Gly Arg
   145 150 155 160
   Leu Gly Ala Leu Cys Cys Trp Glu His Leu Gln Pro Leu Ser Lys Tyr
   165 170 175
   Ala Met Tyr Ala Gln Asn Glu Glu Ile His Val Ala Ala Trp Pro Ser
   180 185 190
   Phe Ser Leu Tyr Asp Pro Phe Ala His Ala Leu Gly Ala Glu Val Asn
   195 200 205
   Asn Ala Ala Ser Gln Ile Tyr Ala Val Glu Gly Ser Cys Phe Val Val
   210 215 220
   Ala Pro Cys Ala Val Ile Ser Gln Glu Met Ile Asp Leu Met Cys Asp
   225 230 235 240
   Thr Pro Asp Lys His Gln Leu Ile His Val Gly Gly Gly Phe Thr Val
   245 250 255
   Ile Tyr Gly Pro Asp Gly Ala Arg Ile Gly Asp Lys Leu Ala Pro Asp
   260 265 270
   Gln Glu Gly Ile Val Tyr Ala Asp Ile Asp Leu Gly Met Ile Pro Ile
   275 280 285
   Ala Lys Ala Ala Ala Asp Pro Ala Gly His Tyr Ala Arg Pro Asp Val
   290 295 300
   Thr Arg Leu Leu Phe Asn Asn Arg Pro Ala Asn Arg Val Glu Thr Leu
   305 310 315 320
   Val Leu Pro Val Asp Gln Val Arg Asp Ile Asp Ala Arg Val Glu Ala
   325 330 335
   Ala Ala Pro Gln Ala Arg Pro Ala Thr Gly Asn Glu Asp Pro Ala Ala
   340 345 350
   Lys Pro Met Ala Ala Glu
   355
<210> 171
   <211> 1011
   <212> DNA
   <213> Unknown
<220>
   <223> Obtained from an environmental sample
<400> 171
   atgactaagg gaacagtgaa agtcgcggcg gcgcaggtca cccccgtgtt catggatcgt      60
   aaagccacga tcgtcaaggc ctgcgacacg atcgccgagg cgggcaagaa cggcgcgcgg     120
   ctggtggtgt tcccggagac gtttgttcct ggctacccgg actgggtctg gacggcgacg     180
   gctgggacgc atcgcgatat ccaccaggcg atgtacgcgg aactgctgga ccaggctgtc     240
   tcgattccga gcccggcgac ggacgccctc tgccgtgctg caaagaaggc gggcgtctac     300
   gtcgtcatcg gcgtcaatga gctgagtggg ccgggcggaa gcctgtacaa cacgctgatc     360
   tacatcgatg acgaaggcga gatcatgggc cgccaccgca agctggtccc cacgatgggc     420
   gagcgcctgg tctgggcacc cggcgacggc agcacgctgg aggcgtacga gacatcgatc     480
   ggcaggctgg gcggactgat ctgctgggag aactacatgc cgctggcccg ctacgccatg     540
   tacgcctggg gcgtgcagat ctacgtcgcg ccgacgtggg acagctcgga cgggtgggtt     600
   ggcagcatgc agcacatcgc ccgcgaaggg cggacggcgg tgatcggctg ctgcatggcg     660
   atccgtcgca gcgacatccc ggacaagtac gagttcaaga agctgtaccc gccgagcaag     720
   agcaaagacg aagaatgggt gaacgatggc aacagcgtca tcgtcgcacc cggtggacga     780
   atactcgccg ggccggtcgc caaagaggag acgatcctct acgccgatct ggacccggca     840
   gccgagcgcg gttcaaagtt ctcgttagat gtggcagggc actacgcgcg gccggacgtc     900
   ttccagctga cggtgaatcg cggtccggca gaactggtga atgtggccgg tgatatcgca     960
   ccggcaacca acggcaaagt caaaacaccg gcgaaattac gccgcaagta a             1011
<210> 172
   <211> 336
   <212> PRT
   <213> Unknown
<220>
   <223> Obtained from an environmental sample
<400> 172
   Met Thr Lys Gly Thr Val Lys Val Ala Ala Ala Gln Val Thr Pro Val
   1               5                   10                  15
   Phe Met Asp Arg Lys Ala Thr Ile Val Lys Ala Cys Asp Thr Ile Ala
               20                  25                  30
   Glu Ala Gly Lys Asn Gly Ala Arg Leu Val Val Phe Pro Glu Thr Phe
           35                  40                  45
   Val Pro Gly Tyr Pro Asp Trp Val Trp Thr Ala Thr Ala Gly Thr His
       50                  55                  60
   Arg Asp Ile His Gln Ala Met Tyr Ala Glu Leu Leu Asp Gln Ala Val
   65                  70                  75                  80
   Ser Ile Pro Ser Pro Ala Thr Asp Ala Leu Cys Arg Ala Ala Lys Lys
                   85                  90                  95
   Ala Gly Val Tyr Val Val Ile Gly Val Asn Glu Leu Ser Gly Pro Gly
               100                 105                 110
   Gly Ser Leu Tyr Asn Thr Leu Ile Tyr Ile Asp Asp Glu Gly Glu Ile
           115                 120                 125
   Met Gly Arg His Arg Lys Leu Val Pro Thr Met Gly Glu Arg Leu Val
       130                 135                 140
   Trp Ala Pro Gly Asp Gly Ser Thr Leu Glu Ala Tyr Glu Thr Ser Ile
   145                 150                 155                 160
   Gly Arg Leu Gly Gly Leu Ile Cys Trp Glu Asn Tyr Met Pro Leu Ala
                   165                 170                 175
   Arg Tyr Ala Met Tyr Ala Trp Gly Val Gln Ile Tyr Val Ala Pro Thr
               180                 185                 190
   Trp Asp Ser Ser Asp Gly Trp Val Gly Ser Met Gln His Ile Ala Arg
           195                 200                 205
   Glu Gly Arg Thr Ala Val Ile Gly Cys Cys Met Ala Ile Arg Arg Ser
       210                 215                 220
   Asp Ile Pro Asp Lys Tyr Glu Phe Lys Lys Leu Tyr Pro Pro Ser Lys
   225                 230                 235                 240
   Ser Lys Asp Glu Glu Trp Val Asn Asp Gly Asn Ser Val Ile Val Ala
                   245                 250                 255
   Pro Gly Gly Arg Ile Leu Ala Gly Pro Val Ala Lys Glu Glu Thr Ile
               260                 265                 270
   Leu Tyr Ala Asp Leu Asp Pro Ala Ala Glu Arg Gly Ser Lys Phe Ser
           275                 280                 285
   Leu Asp Val Ala Gly His Tyr Ala Arg Pro Asp Val Phe Gln Leu Thr
       290                 295                 300
   Val Asn Arg Gly Pro Ala Glu Leu Val Asn Val Ala Gly Asp Ile Ala
   305                 310                 315                 320
   Pro Ala Thr Asn Gly Lys Val Lys Thr Pro Ala Lys Leu Arg Arg Lys
                   325                 330                 335
<210> 173
   <211> 993
   <212> DNA
   <213> Unknown
<220>
   <223> Obtained from an environmental sample
<400> 173
   atgaaagttg ttaaagccgc cgcagtccaa ctgagtcccg tcctctatag ccgcgaggga      60
   acggtcgaga gggtcgttcg gaagatccac gagcttggcc ggcagggggt acagttcgcc     120
   accttcccgg agaccgtagt gccttactac ccgtactttt ccttcgtcca gacgccctta     180
   cagattatag ccggacctga gcatctaaag ctgctcgacc aggcagtgac cgtgccgtcc     240
   cctgccaccg acgctatcag cgaggctgcc aggcaggcgg gagttgtggt gtccataggc     300
   gtcaacgagc gtgacggcgg aaccctgtac aacacgcagc tgctcttcga tgccgatggc     360
   gccttgatcc agcgccgccg caagattacg cccactcatt tcgagcgcat gatctggggc     420
   cagggcgacg ggtcgggcct gcgcgctgtc gacagcaagg tcggtcgcat tggccagctc     480
   gcatgctggg agcacaacaa ccccctggcg cgctacgcga tgatagccga cggcgagcag     540
   atccattcgg caatgtatcc gggctccatg ttcggcgacc cgtttgccca gaagacggaa     600
   atcaatatcc ggcagcatgc attggagtct gcgtgcttcg tcgtgtgcgc cacggcctgg     660
   ctggacgccg atcagcaggc gcaaatctgc aaggacactg gctgcgacat cggcccgatc     720
   tccggcggtt gcttcaccgc gatcgtggcg cctgatggaa ccttgctggg cgagcccatc     780
   cgctcgggcg aaggcatggt catcgtcgac ctcgacttca cgctcatcga caagcgcaag     840
   caggtgatgg actcgcgcgg ccactacaac cggccggaat tgctcagtct cctgatcgac     900
   cgcacaccca ctgcgcatgt tcacgaccgc gctgtgcgcc ccgagtcagc cgcggagcaa     960
   cgttcggagg aacttctcgc tacggctgtc taa                                  993
<210> 174
   <211> 330
   <212> PRT
   <213> Unknown
<220>
   <223> Obtained from an environmental sample
<400> 174
   Met Lys Val Val Lys Ala Ala Ala Val Gln Leu Ser Pro Val Leu Tyr
   1               5                   10                  15
   Ser Arg Glu Gly Thr Val Glu Arg Val Val Arg Lys Ile His Glu Leu
               20                  25                  30
   Gly Arg Gln Gly Val Gln Phe Ala Thr Phe Pro Glu Thr Val Val Pro
           35                  40                  45
   Tyr Tyr Pro Tyr Phe Ser Phe Val Gln Thr Pro Leu Gln Ile Ile Ala
       50                  55                  60
   Gly Pro Glu His Leu Lys Leu Leu Asp Gln Ala Val Thr Val Pro Ser
   65                  70                  75                  80
   Pro Ala Thr Asp Ala Ile Ser Glu Ala Ala Arg Gln Ala Gly Val Val
                   85                  90                  95
   Val Ser Ile Gly Val Asn Glu Arg Asp Gly Gly Thr Leu Tyr Asn Thr
               100                 105                 110
   Gln Leu Leu Phe Asp Ala Asp Gly Ala Leu Ile Gln Arg Arg Arg Lys
           115                 120                 125
   Ile Thr Pro Thr His Phe Glu Arg Met Ile Trp Gly Gln Gly Asp Gly
       130                 135                 140
   Ser Gly Leu Arg Ala Val Asp Ser Lys Val Gly Arg Ile Gly Gln Leu
   145                 150                 155                 160
   Ala Cys Trp Glu His Asn Asn Pro Leu Ala Arg Tyr Ala Met Ile Ala
                   165                 170                 175
   Asp Gly Glu Gln Ile His Ser Ala Met Tyr Pro Gly Ser Met Phe Gly
               180                 185                 190
   Asp Pro Phe Ala Gln Lys Thr Glu Ile Asn Ile Arg Gln His Ala Leu
           195                 200                 205
   Glu Ser Ala Cys Phe Val Val Cys Ala Thr Ala Trp Leu Asp Ala Asp
       210                 215                 220
   Gln Gln Ala Gln Ile Cys Lys Asp Thr Gly Cys Asp Ile Gly Pro Ile
   225                 230                 235                 240
   Ser Gly Gly Cys Phe Thr Ala Ile Val Ala Pro Asp Gly Thr Leu Leu
                   245                 250                 255
   Gly Glu Pro Ile Arg Ser Gly Glu Gly Met Val Ile Val Asp Leu Asp
               260                 265                 270
   Phe Thr Leu Ile Asp Lys Arg Lys Gln Val Met Asp Ser Arg Gly His
           275                 280                 285
   Tyr Asn Arg Pro Glu Leu Leu Ser Leu Leu Ile Asp Arg Thr Pro Thr
       290                 295                 300
   Ala His Val His Asp Arg Ala Val Arg Pro Glu Ser Ala Ala Glu Gln
   305                 310                 315                 320
   Arg Ser Glu Glu Leu Leu Ala Thr Ala Val
                   325                 330
<210> 175
   <211> 945
   <212> DNA
   <213> Unknown
<220>
   <223> Obtained from an environmental sample
<400> 175
   atgaccacct acgggcagtt tagacttgcc gcgatcaatg ccgcgcctgt ctacttcgac      60
   agggaagcat ccaccgaaaa agcttgccgg ctcattctcg aagcgggggc atcaggggcg     120
   acgctggcgg cgtttggcga gacgtggctt cccggctatc cattccacat ctggagggaa     180
   gttccgactg ctctccgagt ggaatacatc gctaatgccg tcgagattcc cagcccaacg     240
   accgaccgat tgtgcgcggc ggctcgtcag gcgaacatcg atgttgtgat cggcgttgtc     300
   gaactggatg cgcagacaca cgggacggtc tactgtacgc tcttgttcat tggcagcgat     360
   ggctcaattc tgggacgtca tcgaaagatt aaaccgactt tcgtggagcg aaccgcatgg     420
   ggggaaggtg acggcagcag cctgatcgtc tacgagcgcc cgtatggcaa gatcagtggt     480
   ctgtgttgct gggaacacaa tatggttctg ccgggctacg cgctgatggc gcaggggacg     540
   cagattcata tcgccgcatg gcccggctgg gaaagcactc gccatctgct cttatcaaga     600
   gcattcgctt ctcaggcagc ggcgtatgtg attgatgtag gcgctatcgt caatcgtgac     660
   gaccttcggg aagattacca ggctttgatt gctggaagct actggggcgg aagttgcatc     720
   atcaacccag aaggcgaggt catcgctggt ccagcgaaat cggagaccat tctggttgca     780
   gattgctcaa ccgagcagat ctttagctca aaagtgctct gtgatgtggg cgggcattat     840
   tctcgcccgg atatttttca gctccatgtc aatcgaaagc catatcaacg tatcgtcgag     900
   acgaacaacc cacaccccgc tccgattgag ttcgattacc gttga                     945
<210> 176
   <211> 314
   <212> PRT
   <213> Unknown
<220>
   <223> Obtained from an environmental sample
<400> 176
   Met Thr Thr Tyr Gly Gln Phe Arg Leu Ala Ala Ile Asn Ala Ala Pro
   1               5                   10                  15
   Val Tyr Phe Asp Arg Glu Ala Ser Thr Glu Lys Ala Cys Arg Leu Ile
               20                  25                  30
   Leu Glu Ala Gly Ala Ser Gly Ala Thr Leu Ala Ala Phe Gly Glu Thr
           35                  40                  45
   Trp Leu Pro Gly Tyr Pro Phe His Ile Trp Arg Glu Val Pro Thr Ala
                   50                      55                      60
   Leu Arg Val Glu Tyr Ile Ala Asn Ala Val Glu Ile Pro Ser Pro Thr
   65                      70                      75                      80
   Thr Asp Arg Leu Cys Ala Ala Ala Arg Gln Ala Asn Ile Asp Val Val
                       85                      90                      95
   Ile Gly Val Val Glu Leu Asp Ala Gln Thr His Gly Thr Val Tyr Cys
                   100                     105                     110
   Thr Leu Leu Phe Ile Gly Ser Asp Gly Ser Ile Leu Gly Arg His Arg
               115                     120                     125
   Lys Ile Lys Pro Thr Phe Val Glu Arg Thr Ala Trp Gly Glu Gly Asp
       130                     135                     140
   Gly Ser Ser Leu Ile Val Tyr Glu Arg Pro Tyr Gly Lys Ile Ser Gly
   145                     150                     155                     160
   Leu Cys Cys Trp Glu His Asn Met Val Leu Pro Gly Tyr Ala Leu Met
                       165                     170                     175
   Ala Gln Gly Thr Gln Ile His Ile Ala Ala Trp Pro Gly Trp Glu Ser
                   180                     185                     190
   Thr Arg His Leu Leu Leu Ser Arg Ala Phe Ala Ser Gln Ala Ala Ala
               195                     200                     205
   Tyr Val Ile Asp Val Gly Ala Ile Val Asn Arg Asp Asp Leu Arg Glu
       210                     215                     220
   Asp Tyr Gln Ala Leu Ile Ala Gly Ser Tyr Trp Gly Gly Ser Cys Ile
   225                     230                     235                     240
   Ile Asn Pro Glu Gly Glu Val Ile Ala Gly Pro Ala Lys Ser Glu Thr
                       245                     250                     255
   Ile Leu Val Ala Asp Cys Ser Thr Glu Gln Ile Phe Ser Ser Lys Val
                   260                     265                     270
   Leu Cys Asp Val Gly Gly His Tyr Ser Arg Pro Asp Ile Phe Gln Leu
               275                     280                     285
   His Val Asn Arg Lys Pro Tyr Gln Arg Ile Val Glu Thr Asn Asn Pro
       290                     295                     300
   His Pro Ala Pro Ile Glu Phe Asp Tyr Arg
   305                     310
<210> 177
   <211> 948
   <212> DNA
   <213> Unknown
<220>
   <223> obtained from an environmental sample
<400> 177
   atggactcac ttaccgttgg tctcgcgcaa atcgcgcccg tttggttgaa tcgggcgggg     60
   acgttgtcaa agatgttgga acaggttcga gcggcgaaag aggcgggctg tcagcttgtt    120
   gtgtttggtg aggcgttgct ccccggttat ccattttgga tcgaactgac gaacggcgca    180
   gtcttcaatt cgccgatgca aaaggaaatc cacgcgcact acatggatca agctgtgcag    240
   atcgaagcag ggcatcttga tccattgtgc ggcgcggcaa aagcgcacgg catcaccgtg    300
   gtcgcgggca tcatcgagcg tccgttggat cgcggcggac atagtttata tgcgagtctg    360
   gtgtatatcg atttgaacgg tgtcatccaa tcggtgcatc gcaaactgat gcccacctat    420
   gaagaacgac tcacctggtc gcctggcgat ggtcatgggt tacgcgtgca tacactgggc    480
   gcttttacgg ttggcaaact caattgttgg gaaaactgga tgccgctgcc gcgcgcggct    540
   ctgtatgcgc aaggcgaaga tctgcacgtt gctgtctggc ccgggtccgt gcgcaacaca    600
   caggatatta cgcgctttat cgcaatggag tcgcgatcgt ttgtcgtttc ggtttcgagt    660
   ttgatgcgca agagtgactt cccacaagat acgcctcatc tctccgccat tcttgaatct    720
   gcacccgatc cactcgccaa cggaggttcg tgtctggctg gacctgacgg taaatggatc    780
   gttgaaccgg ttgcggatga agagaagttg atcgtcgcca ccattgacca tgcccgtgta    840
   cgtgaagaac gccagaactt tgatccatcc ggtcattaca gccgaccaga tgtgacacaa    900
   ttgagagtca accgccagcg acaaagcgtt atcgcttttg atgagtag                 948
<210> 178
   <211> 315
   <212> PRT
   <213> Unknown
<220>
   <223> Obtained from an environmental sample
<400> 178
   Met Asp Ser Leu Thr Val Gly Leu Ala Gln Ile Ala Pro Val Trp Leu
   1               5                   10                  15
   Asn Arg Ala Gly Thr Leu Ser Lys Met Leu Glu Gln Val Arg Ala Ala
               20                  25                  30
   Lys Glu Ala Gly Cys Gln Leu Val Val Phe Gly Glu Ala Leu Leu Pro
           35                  40                  45
   Gly Tyr Pro Phe Trp Ile Glu Leu Thr Asn Gly Ala Val Phe Asn Ser
       50                  55                  60
   Pro Met Gln Lys Glu Ile His Ala His Tyr Met Asp Gln Ala Val Gln
   65                  70                  75                  80
   Ile Glu Ala Gly His Leu Asp Pro Leu Cys Gly Ala Ala Lys Ala His
                   85                  90                  95
   Gly Ile Thr Val Val Ala Gly Ile Ile Glu Arg Pro Leu Asp Arg Gly
               100                 105                 110
   Gly His Ser Leu Tyr Ala Ser Leu Val Tyr Ile Asp Leu Asn Gly Val
           115                 120                 125
   Ile Gln Ser Val His Arg Lys Leu Met Pro Thr Tyr Glu Glu Arg Leu
       130                 135                 140
   Thr Trp Ser Pro Gly Asp Gly His Gly Leu Arg Val His Thr Leu Gly
   145                 150                 155                 160
   Ala Phe Thr Val Gly Lys Leu Asn Cys Trp Glu Asn Trp Met Pro Leu
                   165                 170                 175
   Pro Arg Ala Ala Leu Tyr Ala Gln Gly Glu Asp Leu His Val Ala Val
               180                 185                 190
   Trp Pro Gly Ser Val Arg Asn Thr Gln Asp Ile Thr Arg Phe Ile Ala
           195                 200                 205
   Met Glu Ser Arg Ser Phe Val Val Ser Val Ser Ser Leu Met Arg Lys
       210                 215                 220
   Ser Asp Phe Pro Gln Asp Thr Pro His Leu Ser Ala Ile Leu Glu Ser
   225                 230                 235                 240
   Ala Pro Asp Pro Leu Ala Asn Gly Gly Ser Cys Leu Ala Gly Pro Asp
                   245                 250                 255
   Gly Lys Trp Ile Val Glu Pro Val Ala Asp Glu Glu Lys Leu Ile Val
               260                 265                 270
   Ala Thr Ile Asp His Ala Arg Val Arg Glu Glu Arg Gln Asn Phe Asp
           275                 280                 285
   Pro Ser Gly His Tyr Ser Arg Pro Asp Val Thr Gln Leu Arg Val Asn
       290                 295                 300
   Arg Gln Arg Gln Ser Val Ile Ala Phe Asp Glu
   305                 310                 315
<210> 179
   <211> 915
   <212> DNA
   <213> Unknown
<220>
   <223> Obtained from an environmental sample
<400> 179
   atgaccacca aagcagccat cattcaggcg cgccccatat actacgatct ggcggcgtgt      60
   gtcgataagg cgcttgccct catcaccgag gcggcggcac gcggcgcgaa catcgtcacg     120
   ctcggcgaga cgtggctgcc gggctatccc gcgtggctgg atgtgtgcgt cgagatgggg     180
   ctgtgggatc acgcgccgac caaagccgtc tttcagcggc tccatgccaa cagcgtcacc     240
   atccccggcg cggagatcag ccagttctgc gacatcgccc gccgccttag catcgtgctg     300
   gtgctcagcg tcaacgagcg cgtccgcaac accttgttca acaccctgct cacgattgac     360
   gagcgcggcg acatccgcaa ccaccaccgc aagctgatgc cgacctacac tgagcgcatc     420
   gtctgggggc agggcgacgg cgcgggctta caggcggtcg agacggcaac cgggcgcgtc      480
   ggcgggctga tctgctggga acactggatg ccgctggcac ggcaggcgct gcacaacgcc     540
   ggggagcaaa ttcacgtttc ggtcttcccg accgtcaacg acccgcgcca ccaagtcgcc     600
   agccgccagt acgctttcga ggggcgctgc ttcgtgctga ccgccggcag catccagcgc     660
   gccgacgacc taccgccgga actgaccgtc aaggcgggca tcgcgccgga tgatctggtg     720
   cagggcggcg gcagcgccat catcgcgccg gacatgcgct acctcgccgg accctgcttc     780
   gacgaggaaa ccatcctcta cgccgacctc gacctgagcg agacgatccg cgagagcatg     840
   acgctggacg tgagcgggca ttactcgcgc cccgacgtgt tcaccttcga ggttaatcgg     900
   cagcggaaaa tttag                                                      915
<212> PRT
   <213> Unknown
<220>
   <223> Obtained from an environmental sample
<400> 180
<210> 181
   <211> 990
   <212> DNA
   <213> Unknown
<220>
   <223> Obtained from an environmental sample
<400> 181
   atgcccaaga ccgtacgcgc agccgcagtc cagatcgcgc ccgacctgac gtcacgcgct      60
   ggcaccgtcg agcgggtcct gaatgcgatt gccgaagcct ctgacaaagg cgcggagctg     120
   attgtatttc cagaaacctt tgtgccttgg tatccgtatt tcagcttcgt tctgccgcct     180
   gtccagcagg gacccgagca cctgcggctt tatgaagaag cggtgacggt accatcagca     240
   gaaacacggg ccgtcgccga cgccgcgcgc aaacgcaatg cggtcatcgt ccttggcgtc     300
   aatgagcgcg accacggctc gctctacaac acccagctga tcttcgacgc ggatggcagc     360
   ctgaaactca agcgccgcaa gatcacgccc acctatcacg agcggatgat ctggggacag     420
   ggcgatggcg ccggtctaaa agtggtcgaa actgccatcg gccgcatggg cgcattggcg     480
   tgctgggagc actacaaccc cctcgcccga tacgcgctga tggctcagca tgaggaaatt     540
   cacgcctctc attttccggg ctcactggtc ggcccgatat tcggcgagca gatcgaagtc     600
   acgatgcgcc accacgcgtt ggaatcgggc tgtttcgtgg tcaatgccac cggctggtta     660
   agcgaggagc agatcgcgtc cattcaccca gatcccagcc tgcagaaggg tcttcgagat     720
   ggctgcatga cctgcatcat aaccccggaa ggccgccacg tcgttcctcc tctgacatcg     780
   ggtgaaggaa tcctgattgg cgacctggac atgcggctca tcaccaagcg caagcgaatg     840
   atggattccg tcggacacta tgcacgtcct gagctgctgc accttgtcca tgacacgacg     900
   cccgcacgcg cacgcgagca ggtgggcctt tcaggcgatt tttccgatgc agggcaagac     960
   aagctatttg aggaggttca agatgcgtga                                      990
<210> 182
   <211> 329
   <212> PRT
   <213> Unknown
<220>
   <223> Obtained from an environmental sample
<400> 182
   Met Pro Lys Thr Val Arg Ala Ala Ala Val Gln Ile Ala Pro Asp Leu
   1               5                   10                  15
   Thr Ser Arg Ala Gly Thr Val Glu Arg Val Leu Asn Ala Ile Ala Glu
               20                  25                  30
   Ala Ser Asp Lys Gly Ala Glu Leu Ile Val Phe Pro Glu Thr Phe Val
           35                  40                  45
   Pro Trp Tyr Pro Tyr Phe Ser Phe Val Leu Pro Pro Val Gln Gln Gly
       50                  55                  60
   Pro Glu His Leu Arg Leu Tyr Glu Glu Ala Val Thr Val Pro Ser Ala
   65                  70                  75                  80
   Glu Thr Arg Ala Val Ala Asp Ala Ala Arg Lys Arg Asn Ala Val Ile
                   85                  90                  95
   Val Leu Gly Val Asn Glu Arg Asp His Gly Ser Leu Tyr Asn Thr Gln
               100                 105                 110
   Leu Ile Phe Asp Ala Asp Gly Ser Leu Lys Leu Lys Arg Arg Lys Ile
           115                 120                 125
   Thr Pro Thr Tyr His Glu Arg Met Ile Trp Gly Gln Gly Asp Gly Ala
       130                 135                 140
   Gly Leu Lys Val Val Glu Thr Ala Ile Gly Arg Met Gly Ala Leu Ala
   145                 150                 155                 160
   Cys Trp Glu His Tyr Asn Pro Leu Ala Arg Tyr Ala Leu Met Ala Gln
                   165                 170                 175
   His Glu Glu Ile His Ala Ser His Phe Pro Gly Ser Leu Val Gly Pro
               180                 185                 190
   Ile Phe Gly Glu Gln Ile Glu Val Thr Met Arg His His Ala Leu Glu
           195                 200                 205
   Ser Gly Cys Phe Val Val Asn Ala Thr Gly Trp Leu Ser Glu Glu Gln
       210                 215                 220
   Ile Ala Ser Ile His Pro Asp Pro Ser Leu Gln Lys Gly Leu Arg Asp
   225                 230                 235                 240
   Gly Cys Met Thr Cys Ile Ile Thr Pro Glu Gly Arg His Val Val Pro
                   245                 250                 255
   Pro Leu Thr Ser Gly Glu Gly Ile Leu Ile Gly Asp Leu Asp Met Arg
               260                 265                 270
   Leu Ile Thr Lys Arg Lys Arg Met Met Asp Ser Val Gly His Tyr Ala
           275                 280                 285
   Arg Pro Glu Leu Leu His Leu Val His Asp Thr Thr Pro Ala Arg Ala
       290                 295                 300
   Arg Glu Gln Val Gly Leu Ser Gly Asp Phe Ser Asp Ala Gly Gln Asp
   305                 310                 315                 320
   Lys Leu Phe Glu Glu Val Gln Asp Ala
                   325
<210> 183
   <211> 1002
   <212> DNA
   <213> Unknown
<220>
   <223> Obtained from an environmental sample
<400> 183
   atggctgagt cacgcattat tcgtgccgcc gccgcccaga ttgcgccaga tctccatgag      60
   gccagcaaaa cgctggcccg ggtgctggac gcgatcgatc aggcagccgc acagggggca     120
   gagatcatcg tctttcccga gacctttgtg ccttattacc cctacttctc gtttatcacg     180
   cccgcgatga ccgccggagc ggcccatctg aaattgtatg accaggcggt ggtggtgccc     240
   ggcccgatca cccatgcggt gggcgaacgc gcccgcctgc gcaacatcgt cgtggtgctg 300
   ggggtgaatg aacgtgacca cggcacgctc tacaacaccc aactggtatt tgatgccagc 360
   ggggaactgg tgctgaaacg ccgcaaaatc accccgacct atcacgaacg gatgatctgg 420
   ggacagggag acggtgccgg attaaaggtg gtggactcgg cggttgggcg catcggggct 480
   ttagcctgct gggagcacta caacccactg gcgcgctaca gcctgatgac tcagcacgag 540
   gagatccatt gcagccagtt ccctggttca ctggtggggc cgatttttgc cgagcagatg 600
   gacgtcacca ttcgccatca tgcactggag tccggttgct ttgtcatcaa tgccaccggc 660
   tggctgaccg aggagcagat caacgagctg accagcgacc cggcgttaca aaaggggctg 720
   cgtggtggct gcaacaccgc catcatctcg ccggaaggcc gccatctggt gccgccactg 780
   accgaaggtg aggggatttt gattgccgat ctggacatgg ccctgatcac caaacgcaaa 840
   cgcatgatgg attctgtcgg ccactatgcc cgaccggaat tactcagcct gcgcctcgat 900
   gcgacgcctg cccgttatgt ggtggcgcgt gataatgagt ccgaaaccgg aggaggcaac 960
   gatgcagaac gtaccgtcta cgcgccagca gctgatcact ga 1002
<210> 184
   <211> 333
   <212> PRT
   <213> Unknown
<220>
   <223> Obtained from an environmental sample
<400> 184
   Met Ala Glu Ser Arg Ile Ile Arg Ala Ala Ala Ala Gln Ile Ala Pro
    1               5                   10                  15
   Asp Leu His Glu Ala Ser Lys Thr Leu Ala Arg Val Leu Asp Ala Ile
               20                  25                  30
   Asp Gln Ala Ala Ala Gln Gly Ala Glu Ile Ile Val Phe Pro Glu Thr
           35                  40                  45
   Phe Val Pro Tyr Tyr Pro Tyr Phe Ser Phe Ile Thr Pro Ala Met Thr
       50                  55                  60
   Ala Gly Ala Ala His Leu Lys Leu Tyr Asp Gln Ala Val Val Val Pro
   65                  70                  75                  80
   Gly Pro Ile Thr His Ala Val Gly Glu Arg Ala Arg Leu Arg Asn Ile
                   85                  90                  95
   Val Val Val Leu Gly Val Asn Glu Arg Asp His Gly Thr Leu Tyr Asn
               100                 105                 110
   Thr Gln Leu Val Phe Asp Ala Ser Gly Glu Leu Val Leu Lys Arg Arg
           115                 120                 125
   Lys Ile Thr Pro Thr Tyr His Glu Arg Met Ile Trp Gly Gln Gly Asp
       130                 135                 140
   Gly Ala Gly Leu Lys Val Val Asp Ser Ala Val Gly Arg Ile Gly Ala
   145                 150                 155                 160
   Leu Ala Cys Trp Glu His Tyr Asn Pro Leu Ala Arg Tyr Ser Leu Met
                   165                 170                 175
   Thr Gln His Glu Glu Ile His Cys Ser Gln Phe Pro Gly Ser Leu Val
               180                 185                 190
   Gly Pro Ile Phe Ala Glu Gln Met Asp Val Thr Ile Arg His His Ala
           195                 200                 205
   Leu Glu Ser Gly Cys Phe Val Ile Asn Ala Thr Gly Trp Leu Thr Glu
       210                 215                 220
   Glu Gln Ile Asn Glu Leu Thr Ser Asp Pro Ala Leu Gln Lys Gly Leu
   225                 230                 235                 240
   Arg Gly Gly Cys Asn Thr Ala Ile Ile Ser Pro Glu Gly Arg His Leu
                   245                 250                 255
   Val Pro Pro Leu Thr Glu Gly Glu Gly Ile Leu Ile Ala Asp Leu Asp
               260                 265                 270
   Met Ala Leu Ile Thr Lys Arg Lys Arg Met Met Asp Ser Val Gly His
           275                 280                 285
   Tyr Ala Arg Pro Glu Leu Leu Ser Leu Arg Leu Asp Ala Thr Pro Ala
       290                 295                 300
   Arg Tyr Val Val Ala Arg Asp Asn Glu Ser Glu Thr Gly Gly Gly Asn
   305                 310                 315                 320
   Asp Ala Glu Arg Thr Val Tyr Ala Pro Ala Ala Asp His
                   325                 330
<210> 185
   <211> 1017
   <212> DNA
   <213> Unknown
<220>
   <223> Obtained from an environmental sample
<400> 185
   atgggcattg aacatccgaa atacaaggtc gcggtggtgc aggcggcccc cgcctggctc      60
   gatctcgacg gctcggtcga taagtcgatc gcgctgatca aggaggcggc cgagaagggg     120
   gcgaagctga tcgcctttcc cgaggccttc atccccggtt acccctggca tatctggatg     180
   gactcgccgg cctgggcgat cggccgcggc ttcgtgcagc gttatttcga caattcgctg     240
   tcctatgaca gtccgcaggc cgagcggctg cgcgatgcgg tgaagaaggc gaagctcacc     300
   gccgtgttcg gactgtccga gcgcgacggc ggcagcctct acctcgcgca atggctgatc     360
   gggcccgatg gcgagaccat cgccaagcgc cgcaagctgc ggccgaccca cgccgaacgt     420
   accgtctatg gcgaaggcga cggcagcgat cttgccgtgc atgcgcgcgc cgacatcggc     480
   cggatcggcg cgctctgctg ctgggagcat ctgcagccac tgtcgaaata cgcgatgtac     540
   gcccagaacg aacaggtcca tgtcgcagcc tggcccagct tctcgctgta cgaccccttc     600
   gcgccggcgt taggggccga ggtcaacaac gcggcctccc gcgtctatgc ggtggaaggc     660
   tcctgcttcg tgctcgcgcc gtgcgcgacg gtgtcgcagg cgatgatcga cgagctctgc     720
   gaccggcccg acaagaacgc gctgctgcac gtcggcggcg gctttgccgc gatctatggc     780
   cccgacggca gccagatcgg cgacaagctg gcgccggacc aggaggggct gctgatcgcc     840
   gagatcgacc ttggcgccat cggtgtcgcc aagaacgccg ccgatcccgc cgggcactat     900
   tcgcgtcccg acgtgacgcg gttgctgctc aacaagaagc gataccagcg cgtcgagcag     960
   ttcgcgctgc cggtcgacac cgtcgagccg gcggatatcg gcgcagcggc gagctga       1017
<210> 186
   <211> 338
   <212> PRT
   <213> Unknown
<220>
   <223> Obtained from an environmental sample
<400> 186
   Met Gly Ile Glu His Pro Lys Tyr Lys Val Ala Val Val Gln Ala Ala
   1               5                   10                  15
   Pro Ala Trp Leu Asp Leu Asp Gly Ser Val Asp Lys Ser Ile Ala Leu
               20                  25                  30
   Ile Lys Glu Ala Ala Glu Lys Gly Ala Lys Leu Ile Ala Phe Pro Glu
           35                  40                  45
   Ala Phe Ile Pro Gly Tyr Pro Trp His Ile Trp Met Asp Ser Pro Ala
       50                  55                  60
   Trp Ala Ile Gly Arg Gly Phe Val Gln Arg Tyr Phe Asp Asn Ser Leu
   65                  70                  75                  80
   Ser Tyr Asp Ser Pro Gln Ala Glu Arg Leu Arg Asp Ala Val Lys Lys
                   85                  90                  95
   Ala Lys Leu Thr Ala Val Phe Gly Leu Ser Glu Arg Asp Gly Gly Ser
               100                 105                 110
   Leu Tyr Leu Ala Gln Trp Leu Ile Gly Pro Asp Gly Glu Thr Ile Ala
           115                 120                 125
   Lys Arg Arg Lys Leu Arg Pro Thr His Ala Glu Arg Thr Val Tyr Gly
       130                 135                 140
   Glu Gly Asp Gly Ser Asp Leu Ala Val His Ala Arg Ala Asp Ile Gly
   145                 150                 155                 160
   Arg Ile Gly Ala Leu Cys Cys Trp Glu His Leu Gln Pro Leu Ser Lys
                   165                 170                 175
   Tyr Ala Met Tyr Ala Gln Asn Glu Gln Val His Val Ala Ala Trp Pro
               180                 185                 190
   Ser Phe Ser Leu Tyr Asp Pro Phe Ala Pro Ala Leu Gly Ala Glu Val
           195                 200                 205
   Asn Asn Ala Ala Ser Arg Val Tyr Ala Val Glu Gly Ser Cys Phe Val
       210                 215                 220
   Leu Ala Pro Cys Ala Thr Val Ser Gln Ala Met Ile Asp Glu Leu Cys
   225                 230                 235                 240
   Asp Arg Pro Asp Lys Asn Ala Leu Leu His Val Gly Gly Gly Phe Ala
                   245                 250                 255
   Ala Ile Tyr Gly Pro Asp Gly Ser Gln Ile Gly Asp Lys Leu Ala Pro
               260                 265                 270
   Asp Gln Glu Gly Leu Leu Ile Ala Glu Ile Asp Leu Gly Ala Ile Gly
           275                 280                 285
   Val Ala Lys Asn Ala Ala Asp Pro Ala Gly His Tyr Ser Arg Pro Asp
       290                 295                 300
   Val Thr Arg Leu Leu Leu Asn Lys Lys Arg Tyr Gln Arg Val Glu Gln
   305                 310                 315                 320
   Phe Ala Leu Pro Val Asp Thr Val Glu Pro Ala Asp Ile Gly Ala Ala
                   325                 330                 335
   Ala Ser
<210> 187
   <211> 1059
   <212> DNA
   <213> Unknown
<220>
   <223> Obtained from an environmental sample
<400> 187
   atgggcatca atcatcccaa gtacaaagtg gccgtcgtgc aagcggcacc tgtctggctc      60
   gacctggatg gaacagtcga caagtgcatt cggctgatag gcgaggccgc tgagaaggga     120
   tgcaagctca ttgcatttcc cgagacgttc atcccggggt acccctggca catctggatg     180
   ggagctccgg cctggacgat cgggcgcgga ttcgtgcagc gatacttcga caattcgctt     240
   gcgtacgaca gtccgcaggc aaacaagctt cgcgccgcgg tgaagcgcgc cggagtgacg     300
   gcagttctcg gcttgtcgga gcgccgcgga ggctccctgt acatcgccca gtggctcatc     360
   ggacctgatg gcgagaccat cgctcaacgg cgaaagctgc gccccaccca tgcggagcgc     420
   accgtcttcg gcgagggcga tggcagcgat ttggcggtgc acagccgccc cgacatcggc     480
   cgactgggtg ccctttgctg ctgggaacat ctccagcctt tgaccaagta cgcgatgtac     540
   gcgcaagacg agcaagtgca cgtcgctgca tggccgagct tctcgatgta cgagcctttc     600
   gcgcacgccc tggggtggga gacgaacaac gcggtgagca aggtgtacgc ggtcgaaggt     660
   tcgtgctacg tcctggcccc ctgcgccatc atctctcagg cgatggtgga cgaactcgtc     720
   gacagcgagg acaagaagcc gctggttcat gccggcgggg ggcatgcggt gatctatggt     780
   cccgatggca ccctgcttac tcccaagctt gcagaagacg aggagggcct actgatcgcg     840
   gagatcgatc tgggggcaat cggggtcgcc aagaacgcgg cagaccccgc cggccactac     900
   tcgcggcccg atgtcacccg cctgctcttc aacaaccggc cggccaagcg cgtggagacg     960
   atgctgctcc cggtcgacgc ggcagaagtc gtggagccgg cggacggagc gctcaatgcg    1020
   tccgagggac gccagcgaca gttcaagctg cccgcctag                            1059
<210> 188
   <211> 352
   <212> PRT
   <213> Unknown
<220>
   <223> Obtained from an environmental sample
<400> 188
   Met Gly Ile Asn His Pro Lys Tyr Lys Val Ala Val Val Gln Ala Ala
   1 5 10 15
   Pro Val Trp Leu Asp Leu Asp Gly Thr Val Asp Lys Cys Ile Arg Leu
   20 25 30
   Ile Gly Glu Ala Ala Glu Lys Gly Cys Lys Leu Ile Ala Phe Pro Glu
   35 40 45
   Thr Phe Ile Pro Gly Tyr Pro Trp His Ile Trp Met Gly Ala Pro Ala
   50 55 60
   Trp Thr Ile Gly Arg Gly Phe Val Gln Arg Tyr Phe Asp Asn Ser Leu
   65 70 75 80
   Ala Tyr Asp Ser Pro Gln Ala Asn Lys Leu Arg Ala Ala Val Lys Arg
   85 90 95
   Ala Gly Val Thr Ala Val Leu Gly Leu Ser Glu Arg Arg Gly Gly Ser
   100 105 110
   Leu Tyr Ile Ala Gln Trp Leu Ile Gly Pro Asp Gly Glu Thr Ile Ala
   115 120 125
   Gln Arg Arg Lys Leu Arg Pro Thr His Ala Glu Arg Thr Val Phe Gly
   130 135 140
   Glu Gly Asp Gly Ser Asp Leu Ala Val His Ser Arg Pro Asp Ile Gly
   145 150 155 160
   Arg Leu Gly Ala Leu Cys Cys Trp Glu His Leu Gln Pro Leu Thr Lys
   165 170 175
   Tyr Ala Met Tyr Ala Gln Asp Glu Gln Val His Val Ala Ala Trp Pro
   180 185 190
   Ser Phe Ser Met Tyr Glu Pro Phe Ala His Ala Leu Gly Trp Glu Thr
   195 200 205
   Asn Asn Ala Val Ser Lys Val Tyr Ala Val Glu Gly Ser Cys Tyr Val
   210 215 220
   Leu Ala Pro Cys Ala Ile Ile Ser Gln Ala Met Val Asp Glu Leu Val
   225 230 235 240
   Asp Ser Glu Asp Lys Lys Pro Leu Val His Ala Gly Gly Gly His Ala
   245 250 255
   Val Ile Tyr Gly Pro Asp Gly Thr Leu Leu Thr Pro Lys Leu Ala Glu
   260 265 270
   Asp Glu Glu Gly Leu Leu Ile Ala Glu Ile Asp Leu Gly Ala Ile Gly
   275 280 285
   Val Ala Lys Asn Ala Ala Asp Pro Ala Gly His Tyr Ser Arg Pro Asp
   290 295 300
   Val Thr Arg Leu Leu Phe Asn Asn Arg Pro Ala Lys Arg Val Glu Thr
   305 310 315 320
   Met Leu Leu Pro Val Asp Ala Ala Glu Val Val Glu Pro Ala Asp Gly
   325 330 335
   Ala Leu Asn Ala Ser Glu Gly Arg Gln Arg Gln Phe Lys Leu Pro Ala
   340 345 350
<210> 189
   <211> 1005
   <212> DNA
   <213> Unknown
<220>
   <223> Obtained from an environmental sample
<400> 189
   atgcaagaca cgaaattcaa agttgcagtc gtccaggccg cgccggtatt catggatgcg      60
   ccagcctccg tggccaaggc gatcggtttc atccaggagg cgggcgcagc cggggcgaag     120
   ctgctggcgt tcccggaggt ctggattccg ggctaccctt ggtggctttg gctcgggacg     180
   ccggcgtggg gaatgcagtt tgtgccgcgc tatcacgcca attcgctgcg tgctgatgga     240
   cccgaaatcc tcgctctttg tgcggccgcc gccgaagcga agatcaacgt cgtgatgggc     300
   ttctccgaaa tcgacggagg aacgctctac ctaagtcagg ttttcatcag cgatgcgggc     360
   aagatcatct tcaagcgccg aaagctcaag ccgacccacg tcgaacgtac gctatttggt     420
   gaaggagatg ggtctgattt ccgagtcgtc gacagcagcg tcgggcgcct cggagccctg     480
   tgctgtgccg aacacattca gccgttgtcg aaatacgcca tgtacgcgat gaacgagcaa     540
   attcatgtgg cgtcgtggcc atctttcacg ctctatcgcg gcaaagccta cgctttgggt     600
   catgaggtga atcttgccgc cagccaaatc tacgcgctcg aaggaggttg cttcgtcttg     660
   catgccacgg caattaccgg tcaggatatg ttcgacatgc tttgcgacac tccggaaagg     720
   gcggatttgc tgaatgcgga gggagcaaag ccgggtggag gctattcgat gatttttggt     780
   cccgatggtc agccgatgtg cgagcatctg ccgcaggaca aggaaggcat cctctatgcc     840
   ggcgtagacc tgtcgatgat tgcgatcgcc aaagcggcct acgatcctac ggggcactac     900
   gcccgcggtg atgtcgtccg tctcatggtc aaccgcagcc cccgtcgcac gagcgtcagc     960
   ttcagcgaag acgagaacgc ggcggtcact ttcaccgaga cctga                    1005
<210> 190
   <211> 334
   <212> PRT
   <213> Unknown
<220>
   <223> Obtained from an environmental sample
<400> 190
   Met Gln Asp Thr Lys Phe Lys Val Ala Val Val Gln Ala Ala Pro Val
   1               5                   10                  15
   Phe Met Asp Ala Pro Ala Ser Val Ala Lys Ala Ile Gly Phe Ile Gln
               20                  25                  30
   Glu Ala Gly Ala Ala Gly Ala Lys Leu Leu Ala Phe Pro Glu Val Trp
           35                  40                  45
   Ile Pro Gly Tyr Pro Trp Trp Leu Trp Leu Gly Thr Pro Ala Trp Gly
       50                  55                  60
   Met Gln Phe Val Pro Arg Tyr His Ala Asn Ser Leu Arg Ala Asp Gly
   65                  70                  75                  80
   Pro Glu Ile Leu Ala Leu Cys Ala Ala Ala Ala Glu Ala Lys Ile Asn
                       85                      90                      95
   Val Val Met Gly Phe Ser Glu Ile Asp Gly Gly Thr Leu Tyr Leu Ser
               100                     105                     110
   Gln Val Phe Ile Ser Asp Ala Gly Lys Ile Ile Phe Lys Arg Arg Lys
           115                     120                     125
   Leu Lys Pro Thr His Val Glu Arg Thr Leu Phe Gly Glu Gly Asp Gly
       130                     135                     140
   Ser Asp Phe Arg Val Val Asp Ser Ser Val Gly Arg Leu Gly Ala Leu
   145                     150                     155                     160
   Cys Cys Ala Glu His Ile Gln Pro Leu Ser Lys Tyr Ala Met Tyr Ala
                   165                     170                     175
   Met Asn Glu Gln Ile His Val Ala Ser Trp Pro Ser Phe Thr Leu Tyr
               180                     185                     190
   Arg Gly Lys Ala Tyr Ala Leu Gly His Glu Val Asn Leu Ala Ala Ser
           195                     200                     205
   Gln Ile Tyr Ala Leu Glu Gly Gly Cys Phe Val Leu His Ala Thr Ala
       210                     215                     220
   Ile Thr Gly Gln Asp Met Phe Asp Met Leu Cys Asp Thr Pro Glu Arg
   225                     230                     235                     240
   Ala Asp Leu Leu Asn Ala Glu Gly Ala Lys Pro Gly Gly Gly Tyr Ser
                   245                     250                     255
   Met Ile Phe Gly Pro Asp Gly Gln Pro Met Cys Glu His Leu Pro Gln
               260                     265                     270
   Asp Lys Glu Gly Ile Leu Tyr Ala Gly Val Asp Leu Ser Met Ile Ala
           275                     280                     285
   Ile Ala Lys Ala Ala Tyr Asp Pro Thr Gly His Tyr Ala Arg Gly Asp
       290                     295                     300
   Val Val Arg Leu Met Val Asn Arg Ser Pro Arg Arg Thr Ser Val Ser
   305                     310                     315                     320
   Phe Ser Glu Asp Glu Asn Ala Ala Val Thr Phe Thr Glu Thr
                   325                     330
<210> 191
   <211> 945
   <212> DNA
   <213> Unknown
<220>
   <223> Obtained from an environmental sample
<400> 191
   atgaaaaagt tagctgtggt tcaacgtgcg tcaggatttt tagataagca gcagagcatc      60
   gcgttggcgg tggaaagtat tcagtctgct gcgaataatg gcgcagagct tgttgttttt     120
   acggaagcct ttattcctgg ttatcctgtc tggttatggc gtctgcgccc tggcaaagac     180
   tgggggacaa cagacagtct ttatcaacgc ttaataagca acgcggttga tttaagctca     240
   tcggatttgg atccgattta tgaagcggca aaacgtcatc acgtcacggt tgtatgcggc     300
   attaatgaac gcgactccag cgtcagccga acaacgctat acaacactta catcacggtt     360
   tgtcatgagg gcaatctcat caatgttcat cgaaaactga tgccgaccaa cccagagaga     420
   atggtgtggg gctttggtga tgcgactgga ttaagggtag tagacactcc tgtcggaagg     480
   attggctcac tcgtttgctg ggagaactac atgccgttgg cacgctatgc actttatgct     540
   cagggcgtcg aaatttacat tgcgcctact tatgacagtg gctcggactg gactgaaagc     600
   ttgcgccata tcgccagaga gggcagatgc tacgttgtcg gcagcggtaa cttgttgaga     660
   gccagcgacc tgcctgatga ttttccagaa aaagaaaccc tctatcctga taaagacgag     720
   tggattaacg gcggagactc taccgttatc gctcccggcg gtgaaacatt agttgctccg     780
   ctgcatgcag aggaaggcat actgtattgc gatattgata ctgataaagt ggcggcggct     840
   cggcgttctt tcgacgttgc aggccattac tctcgcccag acatatttac actcaacgta     900
   aatcgagcgc cgcaaacatc tctgcgtatc agggaagccg agtaa                     945
<210> 192
   <211> 314
   <212> PRT
   <213> Unknown
<220>
   <223> Obtained from an environmental sample
<400> 192
   Met Lys Lys Leu Ala Val Val Gln Arg Ala Ser Gly Phe Leu Asp Lys
    1              5                   10                  15
   Gln Gln Ser Ile Ala Leu Ala Val Glu Ser Ile Gln Ser Ala Ala Asn
               20                  25                  30
   Asn Gly Ala Glu Leu Val Val Phe Thr Glu Ala Phe Ile Pro Gly Tyr
           35                  40                  45
   Pro Val Trp Leu Trp Arg Leu Arg Pro Gly Lys Asp Trp Gly Thr Thr
       50                  55                  60
   Asp Ser Leu Tyr Gln Arg Leu Ile Ser Asn Ala Val Asp Leu Ser Ser
   65                  70                  75                  80
   Ser Asp Leu Asp Pro Ile Tyr Glu Ala Ala Lys Arg His His Val Thr
                   85                  90                  95
   Val Val Cys Gly Ile Asn Glu Arg Asp Ser Ser Val Ser Arg Thr Thr
               100                 105                 110
   Leu Tyr Asn Thr Tyr Ile Thr Val Cys His Glu Gly Asn Leu Ile Asn
           115                 120                 125
   Val His Arg Lys Leu Met Pro Thr Asn Pro Glu Arg Met Val Trp Gly
       130                 135                 140
   Phe Gly Asp Ala Thr Gly Leu Arg Val Val Asp Thr Pro Val Gly Arg
   145                 150                 155                 160
   Ile Gly Ser Leu Val Cys Trp Glu Asn Tyr Met Pro Leu Ala Arg Tyr
                   165                 170                 175
   Ala Leu Tyr Ala Gln Gly Val Glu Ile Tyr Ile Ala Pro Thr Tyr Asp
               180                 185                 190
   Ser Gly Ser Asp Trp Thr Glu Ser Leu Arg His Ile Ala Arg Glu Gly
           195                 200                 205
   Arg Cys Tyr Val Val Gly Ser Gly Asn Leu Leu Arg Ala Ser Asp Leu
       210                 215                 220
   Pro Asp Asp Phe Pro Glu Lys Glu Thr Leu Tyr Pro Asp Lys Asp Glu
   225                 230                 235                 240
   Trp Ile Asn Gly Gly Asp Ser Thr Val Ile Ala Pro Gly Gly Glu Thr
                   245                 250                 255
   Leu Val Ala Pro Leu His Ala Glu Glu Gly Ile Leu Tyr Cys Asp Ile
               260                 265                 270
   Asp Thr Asp Lys Val Ala Ala Ala Arg Arg Ser Phe Asp Val Ala Gly
           275                 280                 285
   His Tyr Ser Arg Pro Asp Ile Phe Thr Leu Asn Val Asn Arg Ala Pro
       290                 295                 300
   Gln Thr Ser Leu Arg Ile Arg Glu Ala Glu
   305                 310
<210> 193
   <211> 966
   <212> DNA
   <213> Unknown
<220>
   <223> Obtained from an environmental sample
<400> 193
   atgtcaaacg agaaccacaa ccaaacattc aaagttgccg cggtgcaggc cacacctgta     60
   ttcctcgatc gtgaagcgac catcgacaaa gcttgtgagt tgattgctgc agccggcaat    120
   gaaggagcgc ggctggttgt cttcccggag gcattcatcc catcctaccc agattgggta    180
   tgggcaatcc caccgggcga agaaggcgtg ctcaatgagt tgtacgcgga actgctctcc    240
   aattcggtca cgattcccag tgatgtgacg gatagactgt gccgagccgc gagacttgcc    300
   aatgcctacg tagtgatggg gatgagcgaa cgcaatgccg aggccagtgg cgcaagcctg    360
   tataacacgc tgttgtacat cgatgcgcag ggcgagattc tgggcaaaca tcgaaagctg    420
   gtgcccacag gcggcgaacg gctggtgtgg gcgcagggcg atggcagcac gctgcaggtc    480
   tacgatactc cactgggtaa actcggcggt ttgatttgct gggagaatta tatgccgctg    540
   gcccgctaca ccatgtacgc atggggcaca caaatctatg ttgcggcgac atgggatcgc    600
   gggcaaccct ggctctccac tttacggcat atcgccaaag aaggcagggt gtacgtgatc    660
   ggctgctgta tcgtgatgcg caaagacgat atcccagatc gttacccgat gaagcagaag    720
   ttttacgcgg aggccgatga gtggatcaac ataggggaca gcgcaatcgt caatcctgaa    780
   gggcagttta gcgccgggcc ggtacgcaaa caggaagaga ttctctacgc ggaaattgat    840
   ccgcgcatgg tgcaaggccc gaagtggatg ctcgacgtag cagggcacta cgcgaggccg    900
   gacgtattcc agttgacggt gcatacggat gcgaggcaga tgatcaggtt ggaacacgat    960
   gtttaa                                                               966
<210> 194
   <211> 321
   <212> PRT
   <213> Unknown
<220>
   <223> Obtained from an environmental sample
<400> 194
   Met Ser Asn Glu Asn His Asn Gln Thr Phe Lys Val Ala Ala Val Gln
   1               5                   10                  15
   Ala Thr Pro Val Phe Leu Asp Arg Glu Ala Thr Ile Asp Lys Ala Cys
               20                  25                  30
   Glu Leu Ile Ala Ala Ala Gly Asn Glu Gly Ala Arg Leu Val Val Phe
           35                  40                  45
   Pro Glu Ala Phe Ile Pro Ser Tyr Pro Asp Trp Val Trp Ala Ile Pro
       50                  55                  60
   Pro Gly Glu Glu Gly Val Leu Asn Glu Leu Tyr Ala Glu Leu Leu Ser
   65                  70                  75                  80
   Asn Ser Val Thr Ile Pro Ser Asp Val Thr Asp Arg Leu Cys Arg Ala
                   85                  90                  95
   Ala Arg Leu Ala Asn Ala Tyr Val Val Met Gly Met Ser Glu Arg Asn
               100                 105                 110
   Ala Glu Ala Ser Gly Ala Ser Leu Tyr Asn Thr Leu Leu Tyr Ile Asp
           115                 120                 125
   Ala Gln Gly Glu Ile Leu Gly Lys His Arg Lys Leu Val Pro Thr Gly
       130                 135                 140
   Gly Glu Arg Leu Val Trp Ala Gln Gly Asp Gly Ser Thr Leu Gln Val
   145                 150                 155                 160
   Tyr Asp Thr Pro Leu Gly Lys Leu Gly Gly Leu Ile Cys Trp Glu Asn
                   165                 170                 175
   Tyr Met Pro Leu Ala Arg Tyr Thr Met Tyr Ala Trp Gly Thr Gln Ile
               180                 185                 190
   Tyr Val Ala Ala Thr Trp Asp Arg Gly Gln Pro Trp Leu Ser Thr Leu
           195                 200                 205
   Arg His Ile Ala Lys Glu Gly Arg Val Tyr Val Ile Gly Cys Cys Ile
       210                 215                 220
   Val Met Arg Lys Asp Asp Ile Pro Asp Arg Tyr Pro Met Lys Gln Lys
   225                 230                 235                 240
   Phe Tyr Ala Glu Ala Asp Glu Trp Ile Asn Ile Gly Asp Ser Ala Ile
                   245                 250                 255
   Val Asn Pro Glu Gly Gln Phe Ser Ala Gly Pro Val Arg Lys Gln Glu
               260                 265                 270
   Glu Ile Leu Tyr Ala Glu Ile Asp Pro Arg Met Val Gln Gly Pro Lys
           275                 280                 285
   Trp Met Leu Asp Val Ala Gly His Tyr Ala Arg Pro Asp Val Phe Gln
       290                 295                 300
   Leu Thr Val His Thr Asp Ala Arg Gln Met Ile Arg Leu Glu His Asp
   305                 310                 315                 320
   Val
<210> 195
   <211> 993
   <212> DNA
   <213> Unknown
<220>
   <223> Obtained from an environmental sample
<400> 195
   atgaaagtcg tcaaagccgc cgcggtacag ttcagcccgg tgctctatag ccgtgaggca      60
   accgtcgcca aggtcgtgca gaagatccac gaactcggcc agaaaggcgt gcagttcgcc     120
   accttccccg aaacggtcgt gccttattac ccttactttg cggccgtcca gacgggcatc     180
   gagcttctct cgggcaccga acatctgcgc ctgctcgaac aggctgtgac tgtcccgtcc     240
   gctgctaccg acgcgatcgg cgaagccgcg cgaaaggccg gcatggtcgt gtccattggc     300
   gtcaatgagc gcgatggcgg cacgctgtac aacgcacaac tgctcttcga tgccgacggt     360
   acgctgatcc agcgccgccg caagatcacg ccgacgcatt tcgaacgcat gatctggggc     420
   cagggagatg gctcgggctt gcgtgcagtc gacagcgccg tcggccgcgt cggccagctc     480
   gcatgtttcg agcacaacaa cccgctcgcc cgctacgcaa tgatcgccga cggcgagcag     540
   atccattcgg cgatgtaccc tggctcggcc tttggcgagg gcttcgccca gcgtatggaa     600
   atcaacatcc gccagcatgc gctcgagtcc gccgctttcg tcgtcaacgc aacagcgtgg     660
   ctggacgccg accagcaggc gcaaatcatg aaggacaccg gttgtggaat cggtccgatc      720
   acgggcggct gcttcaccac gatcgtctct cctgacggca tgctgatggc cgagccgctt      780
   cgctcgggtg aaggcgaagt gatcgtcgat ctcgacttca cgcagatcga ccgccgcaag      840
   atgctgatgg actcggccgg ccactacaac cgccctgaac tgctgagtct gatgatcgac      900
   cgtacgccga ccgcgcatgt tcacgaacgc gcttcgcacc cgatgatcgt caacgaccag      960
   ggttccgacg atctgcgcac ccaggctgca tga                                   993
<210> 196
   <211> 330
   <212> PRT
   <213> Unknown
<220>
   <223> Obtained from an environmental sample
<400> 196
   Met Lys Val Val Lys Ala Ala Ala Val Gln Phe Ser Pro Val Leu Tyr
   1               5                   10                  15
   Ser Arg Glu Ala Thr Val Ala Lys Val Val Gln Lys Ile His Glu Leu
               20                  25                  30
   Gly Gln Lys Gly Val Gln Phe Ala Thr Phe Pro Glu Thr Val Val Pro
           35                  40                  45
   Tyr Tyr Pro Tyr Phe Ala Ala Val Gln Thr Gly Ile Glu Leu Leu Ser
       50                  55                  60
   Gly Thr Glu His Leu Arg Leu Leu Glu Gln Ala Val Thr Val Pro Ser
   65                  70                  75                  80
   Ala Ala Thr Asp Ala Ile Gly Glu Ala Ala Arg Lys Ala Gly Met Val
                   85                  90                  95
   Val Ser Ile Gly Val Asn Glu Arg Asp Gly Gly Thr Leu Tyr Asn Ala
               100                 105                 110
   Gln Leu Leu Phe Asp Ala Asp Gly Thr Leu Ile Gln Arg Arg Arg Lys
           115                 120                 125
   Ile Thr Pro Thr His Phe Glu Arg Met Ile Trp Gly Gln Gly Asp Gly
       130                 135                 140
   Ser Gly Leu Arg Ala Val Asp Ser Ala Val Gly Arg Val Gly Gln Leu
   145                 150                 155                 160
   Ala Cys Phe Glu His Asn Asn Pro Leu Ala Arg Tyr Ala Met Ile Ala
                   165                 170                 175
   Asp Gly Glu Gln Ile His Ser Ala Met Tyr Pro Gly Ser Ala Phe Gly
               180                 185                 190
   Glu Gly Phe Ala Gln Arg Met Glu Ile Asn Ile Arg Gln His Ala Leu
           195                 200                 205
   Glu Ser Ala Ala Phe Val Val Asn Ala Thr Ala Trp Leu Asp Ala Asp
       210                 215                 220
   Gln Gln Ala Gln Ile Met Lys Asp Thr Gly Cys Gly Ile Gly Pro Ile
   225                 230                 235                 240
   Thr Gly Gly Cys Phe Thr Thr Ile Val Ser Pro Asp Gly Met Leu Met
                   245                 250                 255
   Ala Glu Pro Leu Arg Ser Gly Glu Gly Glu Val Ile Val Asp Leu Asp
               260                 265                 270
   Phe Thr Gln Ile Asp Arg Arg Lys Met Leu Met Asp Ser Ala Gly His
           275                 280                 285
   Tyr Asn Arg Pro Glu Leu Leu Ser Leu Met Ile Asp Arg Thr Pro Thr
       290                 295                 300
   Ala His Val His Glu Arg Ala Ser His Pro Met Ile Val Asn Asp Gln
   305                 310                 315                 320
   Gly Ser Asp Asp Leu Arg Thr Gln Ala Ala
                   325                 330
<210> 197
   <211> 1017
   <212> DNA
   <213> Unknown
<220>
   <223> Obtained from an environmental sample
<400> 197
   atgggcatcg aacatccaaa ataccgcgtt gccgccgtgc aggctgcgcc ggcctggctc      60
   gacctcgatc gctcgatcga caaggctatt gcactgatcg aggaggccgc cgcgaacggc     120
   gccagattga tcgcattccc ggaggtcttc atccccggct acccctggca tatctggctc     180
   gactcgccgg cctgggcgat cggccgcggc ttcgtgcagc gttatttcga caattcgctc     240
   gcttatgata gtccgcaggc cgagcggctc cgcgcagcgg tccgcaaggc gcgcctgacc     300
   gccgtgatcg gcctttcgga gcggagcggc ggcagcctct acatcgcgca atggctcgtt     360
   ggccccgacg gcgagaccat cgcgaagcgc cgcaagctcc gtccgacgca tgccgagcgc     420
   acggtctatg gcgagggcga cggcagcgat ctggcggtcc atgaccggcc cgatatcgga     480
   cggctcggcg cgctgtgctg ctgggaacat ctgcaaccgt tgtcgaaata tgcgatgtat     540
   gcccagaacg agcaggtcca tgtggcgtca tggccgagtt tttcgctcta cgatcccttt     600
   gccccggcgc tcggcgcgga ggtgaacaat gcggcctccc gggtctatgc ggtcgaaggc     660
   tcctgcttcg tgctggcgcc gtgcgcgacc gtctcgcagg ccatgatcga tgagctgtgc     720
   gaccggcccg acaagcacgc gctgctccat gccggcggtg gctttgccgc gatctacggg     780
   cccgacggca gttcgctggc cgaaaagctc gcgccggacc aggagggcct gctttacgcc     840
   gacatcgatc tcggcgcgat cggcgtcgcg aagaacgccg ccgacccggc agggcattat     900
   tcgcggcccg atgtcacgcg gctgctgctg aacaacaagc cctacaagcg cgtggagcat     960
   tttgctttgc ccggcgatac cgtggcgcct gccgatgtgg atgcggcggc gagctga       1017
<210> 198
   <211> 338
   <212> PRT
   <213> Unknown
<220>
   <223> Obtained from an environmental sample
<400> 198
   Met Gly Ile Glu His Pro Lys Tyr Arg Val Ala Ala Val Gln Ala Ala
   1               5                   10                  15
   Pro Ala Trp Leu Asp Leu Asp Arg Ser Ile Asp Lys Ala Ile Ala Leu
               20                  25                  30
   Ile Glu Glu Ala Ala Ala Asn Gly Ala Arg Leu Ile Ala Phe Pro Glu
           35                  40                  45
   Val Phe Ile Pro Gly Tyr Pro Trp His Ile Trp Leu Asp Ser Pro Ala
       50                  55                  60
   Trp Ala Ile Gly Arg Gly Phe Val Gln Arg Tyr Phe Asp Asn Ser Leu
   65                  70                  75                  80
   Ala Tyr Asp Ser Pro Gln Ala Glu Arg Leu Arg Ala Ala Val Arg Lys
                   85                  90                  95
   Ala Arg Leu Thr Ala Val Ile Gly Leu Ser Glu Arg Ser Gly Gly Ser
               100                 105                 110
   Leu Tyr Ile Ala Gln Trp Leu Val Gly Pro Asp Gly Glu Thr Ile Ala
           115                 120                 125
   Lys Arg Arg Lys Leu Arg Pro Thr His Ala Glu Arg Thr Val Tyr Gly
       130                 135                 140
   Glu Gly Asp Gly Ser Asp Leu Ala Val His Asp Arg Pro Asp Ile Gly
   145                 150                 155                 160
   Arg Leu Gly Ala Leu Cys Cys Trp Glu His Leu Gln Pro Leu Ser Lys
                   165                 170                 175
   Tyr Ala Met Tyr Ala Gln Asn Glu Gln Val His Val Ala Ser Trp Pro
               180                 185                 190
   Ser Phe Ser Leu Tyr Asp Pro Phe Ala Pro Ala Leu Gly Ala Glu Val
           195                 200                 205
   Asn Asn Ala Ala Ser Arg Val Tyr Ala Val Glu Gly Ser Cys Phe Val
       210                 215                 220
   Leu Ala Pro Cys Ala Thr Val Ser Gln Ala Met Ile Asp Glu Leu Cys
   225                 230                 235                 240
   Asp Arg Pro Asp Lys His Ala Leu Leu His Ala Gly Gly Gly Phe Ala
                   245                 250                 255
   Ala Ile Tyr Gly Pro Asp Gly Ser Ser Leu Ala Glu Lys Leu Ala Pro
               260                 265                 270
   Asp Gln Glu Gly Leu Leu Tyr Ala Asp Ile Asp Leu Gly Ala Ile Gly
           275                 280                 285
   Val Ala Lys Asn Ala Ala Asp Pro Ala Gly His Tyr Ser Arg Pro Asp
       290                 295                 300
   Val Thr Arg Leu Leu Leu Asn Asn Lys Pro Tyr Lys Arg Val Glu His
   305                 310                 315                 320
   Phe Ala Leu Pro Gly Asp Thr Val Ala Pro Ala Asp Val Asp Ala Ala
                   325                 330                 335
   Ala Ser
<210> 199
   <211> 993
   <212> DNA
   <213> Unknown
<220>
   <223> Obtained from an environmental sample
<400> 199
   atgtcaaacg cactgaagcc gttcaaaatc gccgccgttc aggcgacgcc ggtttttctg      60
   aaccgggaag ccacggcgga gaaggccgcg gctttgatcc gcgaagcggg aagcgccgga     120
   gccaagctca tcgttttccc ggaatcgttt attccggcct atccggactg ggtctgggtg     180
   gtcccctcgg ggagggatcg ccttctcagc ggcctctacg gggagatgct cgaaaacgcc     240
   gtggaaatcc ccggcccggc cacggggcat atcggccggg cggcgaagga atcgggcgct     300
   tatgtcgtca tgggcgtgac cgagcgggac acggaggcga gcggagccag tttgttcaac     360
   accttgattt atttcggtcc gaccggggaa attttgggca aacaccggaa gctggttccc     420
   accgggggcg aacggatcgt ctgggcccag ggggacggaa gcaccctgga ggtctacgat     480
   acgcccctgg gaaaactggg cgggctgatc tgctgggaaa actacatgcc cctggcccgg     540
   tacgccatgt acgcctgggg aacccagctt tacgtggccg ccacctggga ccgaggcgaa     600
   ccctggcttt cgacgcttcg gcatatcgcc aaggaagggc gggtgtatgt catcgggtgc     660
   tgcatcgcca tgcggaaagg ggatatcccg gatcggttcg aacacaaggg gctctacgcc     720
   cccgaccggg actggatcaa ccccggcgac agcgcgatcg tcaaccccca gggggagatg     780
   atcgccgggc ccgcttccaa taaggaagag atcctttatg cggaagtcga cccgcagatg     840
   atgcgcgggc ccaaatggat gctcgatgtg gccggccatt acgcgcggcc cgatgtcttc     900
   gagctcaccg tccgccggga accgcggccg atgatccgcg tggcgggagg cgcgggcggg     960
   accgaaccca aagagaagaa gaccgccggc tga                                  993
<210> 200
   <211> 330
   <212> PRT
   <213> Unknown
<220>
   <223> Obtained from an environmental sample
<400> 200
   Met Ser Asn Ala Leu Lys Pro Phe Lys Ile Ala Ala Val Gln Ala Thr
   1               5                   10                  15
   Pro Val Phe Leu Asn Arg Glu Ala Thr Ala Glu Lys Ala Ala Ala Leu
               20                  25                  30
   Ile Arg Glu Ala Gly Ser Ala Gly Ala Lys Leu Ile Val Phe Pro Glu
           35                  40                  45
   Ser Phe Ile Pro Ala Tyr Pro Asp Trp Val Trp Val Val Pro Ser Gly
       50                  55                  60
   Arg Asp Arg Leu Leu Ser Gly Leu Tyr Gly Glu Met Leu Glu Asn Ala
   65                  70                  75                  80
   Val Glu Ile Pro Gly Pro Ala Thr Gly His Ile Gly Arg Ala Ala Lys
                   85                  90                  95
   Glu Ser Gly Ala Tyr Val Val Met Gly Val Thr Glu Arg Asp Thr Glu
               100                 105                 110
   Ala Ser Gly Ala Ser Leu Phe Asn Thr Leu Ile Tyr Phe Gly Pro Thr
           115                 120                 125
   Gly Glu Ile Leu Gly Lys His Arg Lys Leu Val Pro Thr Gly Gly Glu
       130                 135                 140
   Arg Ile Val Trp Ala Gln Gly Asp Gly Ser Thr Leu Glu Val Tyr Asp
   145                 150                 155                 160
   Thr Pro Leu Gly Lys Leu Gly Gly Leu Ile Cys Trp Glu Asn Tyr Met
                   165                 170                 175
   Pro Leu Ala Arg Tyr Ala Met Tyr Ala Trp Gly Thr Gln Leu Tyr Val
               180                 185                 190
   Ala Ala Thr Trp Asp Arg Gly Glu Pro Trp Leu Ser Thr Leu Arg His
           195                 200                 205
   Ile Ala Lys Glu Gly Arg Val Tyr Val Ile Gly Cys Cys Ile Ala Met
       210                 215                 220
   Arg Lys Gly Asp Ile Pro Asp Arg Phe Glu His Lys Gly Leu Tyr Ala
   225                 230                 235                 240
   Pro Asp Arg Asp Trp Ile Asn Pro Gly Asp Ser Ala Ile Val Asn Pro
                           245                     250                     255
   Gln Gly Glu Met Ile Ala Gly Pro Ala Ser Asn Lys Glu Glu Ile Leu
                   260                     265                     270
   Tyr Ala Glu Val Asp Pro Gln Met Met Arg Gly Pro Lys Trp Met Leu
           275                     280                     285
   Asp Val Ala Gly His Tyr Ala Arg Pro Asp Val Phe Glu Leu Thr Val
       290                     295                     300
   Arg Arg Glu Pro Arg Pro Met Ile Arg Val Ala Gly Gly Ala Gly Gly
   305                     310                     315                     320
   Thr Glu Pro Lys Glu Lys Lys Thr Ala Gly
                   325                     330
<210> 201
   <211> 930
   <212> DNA
   <213> Unknown
<220>
   <223> Obtained from an environmental sample
<400> 201
   atgaccacga agagcatccg catcgcggcc gtacaggcgg ctcccgcgtt tctcgacctg      60
   gctggtacgc tggaccggct cgaggcctgg gcccgcaagg ccgccgccac cggtgcccgc     120
   gtcatcgcgt tccccgagac ctggctgccg ggctacccgg cgtggatcga ctcgtcgccg     180
   gaggccgcga tctggggcca tcccggctcg cgcgacctgc accagcgcct gatggagaat     240
   gccgtcgagg tcccgggccc cgcgaccgcg cgcatcgcga agctcgccgg cgagctcggc     300
   gtgacgatcg tggtcggcgc gcacgagcgg gcggggaaca ccctctacaa cacggcgctg     360
   acgttcgggc ccgagggcag gctgctcaat caccaccgga agctggtgcc gacctacagc     420
   gaacggctgc tgtggggcta cggcgacggc gctggactgg tggcgccggc ggtggacggt     480
   gtgaaggtcg gggcgctggt gtgctgggag cactggatgc cgctcacccg ccaggcgatg     540
   cacgacgtcg gcgagcacgt gcacgtcgcc ctgtggcccg gcgtccacga gatgcaccag     600
   gtggcctcgc ggcactatgc gttcgagggc cgctgtttcg tgatcgcggt cgggagcatc     660
   ctgcgcgtgg accagatgcc gaagcagctg ccgccgctgg agaagtacgc gaagagcgcc     720
   aaggggctga tgatcgcggg cggcagcgcc atcatcgcgc cgaacggccg ctacgtcgcg     780
   gcgccggtgt acgacgagga gacgatcgtc accgccgact gcgacctcgg cgagatcccg     840
   cgcgaggcgc agacgctcga tgtctcgggc cactacagcc ggccggacgt gttcagcttc     900
   ggggtggtca gacaccggcc gcgtgcgtaa                                      930
<210> 202
   <211> 309
   <212> PRT
   <213> Unknown
<220>
   <223> Obtained from an environmental sample
<400> 202
   Met Thr Thr Lys Ser Ile Arg Ile Ala Ala Val Gln Ala Ala Pro Ala
   1               5                   10                  15
   Phe Leu Asp Leu Ala Gly Thr Leu Asp Arg Leu Glu Ala Trp Ala Arg
               20                  25                  30
   Lys Ala Ala Ala Thr Gly Ala Arg Val Ile Ala Phe Pro Glu Thr Trp
           35                  40                  45
   Leu Pro Gly Tyr Pro Ala Trp Ile Asp Ser Ser Pro Glu Ala Ala Ile
       50                  55                  60
   Trp Gly His Pro Gly Ser Arg Asp Leu His Gln Arg Leu Met Glu Asn
   65                  70                  75                  80
   Ala Val Glu Val Pro Gly Pro Ala Thr Ala Arg Ile Ala Lys Leu Ala
                   85                  90                  95
   Gly Glu Leu Gly Val Thr Ile Val Val Gly Ala His Glu Arg Ala Gly
               100                 105                 110
   Asn Thr Leu Tyr Asn Thr Ala Leu Thr Phe Gly Pro Glu Gly Arg Leu
           115                 120                 125
   Leu Asn His His Arg Lys Leu Val Pro Thr Tyr Ser Glu Arg Leu Leu
       130                 135                 140
   Trp Gly Tyr Gly Asp Gly Ala Gly Leu Val Ala Pro Ala Val Asp Gly
   145                 150                 155                 160
   Val Lys Val Gly Ala Leu Val Cys Trp Glu His Trp Met Pro Leu Thr
                   165                 170                 175
   Arg Gln Ala Met His Asp Val Gly Glu His Val His Val Ala Leu Trp
               180                 185                 190
   Pro Gly Val His Glu Met His Gln Val Ala Ser Arg His Tyr Ala Phe
           195                 200                 205
   Glu Gly Arg Cys Phe Val Ile Ala Val Gly Ser Ile Leu Arg Val Asp
       210                 215                 220
   Gln Met Pro Lys Gln Leu Pro Pro Leu Glu Lys Tyr Ala Lys Ser Ala
   225                 230                 235                 240
   Lys Gly Leu Met Ile Ala Gly Gly Ser Ala Ile Ile Ala Pro Asn Gly
                   245                 250                 255
   Arg Tyr Val Ala Ala Pro Val Tyr Asp Glu Glu Thr Ile Val Thr Ala
               260                 265                 270
   Asp Cys Asp Leu Gly Glu Ile Pro Arg Glu Ala Gln Thr Leu Asp Val
           275                 280                 285
   Ser Gly His Tyr Ser Arg Pro Asp Val Phe Ser Phe Gly Val Val Arg
       290                 295                 300
   His Arg Pro Arg Ala
   305
<210> 203
   <211> 966
   <212> DNA
   <213> Unknown
<220>
   <223> Obtained from an environmental sample
<400> 203
   atgtcaagct tacccacatc cgcgttcacc gtcgccgccg cgcaagcgtc gccagtgttc      60
   ctcgaccgcg acgcgacgct gcagaaggct tgcgggctga tcgccgacgc cgggcgcgcg     120
   ggcgcgcgcc tgatcgtctt ccccgaagcc ttcatcccgg cctaccccga ttgggtgtgg     180
   gcggtcccag ctggcgaaga ggggatgctg agcgagctct acgccgagct ggtcgcgaat     240
   tcgctggcta ttccgagcga cgcgaccgat cggctatgtc gcgcggcgca ggccgcgcat     300
   atcaatgtgg tcgtggggtt gagcgagcgc aatgtcgagg ccagcggcgc cagcctctac     360
   aacacgctgc tgtatatcga cgcggcggga acgatcctgg gtaaacaccg caagcttgtg     420
   ccgaccggcg gggagcgcct ggtctgggcg cagggcgacg gcagcacgct cgatgtgtac     480
   gacaccgcgc tcggcaagct cggcggcctg atctgttggg aaaactacat gccgctggca     540
   cgctacgcgc tgtacgcctg gggtgtgcaa atctatgtcg cggccacctg ggatcgcggc      600
   gagccctggc tttctactct gcgacatatc gccaaggaag gccgtgtcta cgtgatcggc     660
   tgtggcatgg cgctgcgcag agatgatatt cccgatcgct tcgctttcaa gcagcgcttc     720
   tatgcccagg ccggcgaatg gatcaacgtc ggcgacagcg cgatcgtcaa cccgagcggc     780
   gagtttattg ccggacctgt gcgcgaacgc gaggagattc tgtacgcgga ggtcgacccg     840
   gagcagatga gcgggccaaa gtggatgctc gacgtggccg ggcactacgg gcggccggat     900
   gtcttccggc tcagcgtcaa ccgggcgccg caccagatga tccagacgga gaaccgggag     960
   acctga                                                                966
<210> 204
   <211> 321
   <212> PRT
   <213> Unknown
<220>
   <223> Obtained from an environmental sample
<400> 204
   Met Ser Ser Leu Pro Thr Ser Ala Phe Thr Val Ala Ala Ala Gln Ala
   1               5                   10                  15
   Ser Pro Val Phe Leu Asp Arg Asp Ala Thr Leu Gln Lys Ala Cys Gly
               20                  25                  30
   Leu Ile Ala Asp Ala Gly Arg Ala Gly Ala Arg Leu Ile Val Phe Pro
           35                  40                  45
   Glu Ala Phe Ile Pro Ala Tyr Pro Asp Trp Val Trp Ala Val Pro Ala
       50                  55                  60
   Gly Glu Glu Gly Met Leu Ser Glu Leu Tyr Ala Glu Leu Val Ala Asn
   65                  70                  75                  80
   Ser Leu Ala Ile Pro Ser Asp Ala Thr Asp Arg Leu Cys Arg Ala Ala
                   85                  90                  95
   Gln Ala Ala His Ile Asn Val Val Val Gly Leu Ser Glu Arg Asn Val
               100                 105                 110
   Glu Ala Ser Gly Ala Ser Leu Tyr Asn Thr Leu Leu Tyr Ile Asp Ala
           115                 120                 125
   Ala Gly Thr Ile Leu Gly Lys His Arg Lys Leu Val Pro Thr Gly Gly
       130                 135                 140
   Glu Arg Leu Val Trp Ala Gln Gly Asp Gly Ser Thr Leu Asp Val Tyr
   145                 150                 155                 160
   Asp Thr Ala Leu Gly Lys Leu Gly Gly Leu Ile Cys Trp Glu Asn Tyr
                   165                 170                 175
   Met Pro Leu Ala Arg Tyr Ala Leu Tyr Ala Trp Gly Val Gln Ile Tyr
               180                 185                 190
   Val Ala Ala Thr Trp Asp Arg Gly Glu Pro Trp Leu Ser Thr Leu Arg
           195                 200                 205
   His Ile Ala Lys Glu Gly Arg Val Tyr Val Ile Gly Cys Gly Met Ala
       210                 215                 220
   Leu Arg Arg Asp Asp Ile Pro Asp Arg Phe Ala Phe Lys Gln Arg Phe
   225                 230                 235                 240
   Tyr Ala Gln Ala Gly Glu Trp Ile Asn Val Gly Asp Ser Ala Ile Val
                   245                 250                 255
   Asn Pro Ser Gly Glu Phe Ile Ala Gly Pro Val Arg Glu Arg Glu Glu
               260                 265                 270
   Ile Leu Tyr Ala Glu Val Asp Pro Glu Gln Met Ser Gly Pro Lys Trp
           275                 280                 285
   Met Leu Asp Val Ala Gly His Tyr Gly Arg Pro Asp Val Phe Arg Leu
       290                 295                 300
   Ser Val Asn Arg Ala Pro His Gln Met Ile Gln Thr Glu Asn Arg Glu
   305                 310                 315                 320
   Thr
<210> 205
   <211> 969
   <212> DNA
   <213> Unknown
<220>
   <223> Obtained from an environmental sample
<400> 205
   atgaccaccg taaaagccgc cgcagtacag atcagccccg tgctctatag ccgggaggcc      60
   accgtagaca aggtcgttcg caagatccgc gagctcggcc aaaagggagt gcagttcgcc     120
   accttcccgg aaaccgtagt gccgtactac ccctacttcg ctgcagtcca gacaggcatc     180
   gaactgttgt ccggcaagga acacatgcgc ctgctggagc aggccgttac cgtcccctcg     240
   cccgccacgg atgcgattgc tcaggcggcg cgcgaagcca atatggtggt gtccatcggc     300
   gtcaacgagc gcgacggcgg caccatctac aacacgcagc tgctcttcga tgccgacggc     360
   acgctcgtgc agcgccgccg caagataacg ccaacgcact tcgagcgcat ggtctggggc     420
   cagggcgatg gttcgggatt gcgcgccgcg gacaccaagg ttggccgcat cggccagttg     480
   gcctgcttcg agcacaacaa cccgctcgcc cgttacgcca tgatggccga tggcgagcag     540
   atccactccg ccatgtaccc gggctcggcc ttcggcgagg gcttcgcgca gcgcatggag     600
   atcaacatcc gccagcatgc cctggagtct ggctgcttcg tggtgaatgc gaccgcctgg     660
   ctcgatgccg accaacaggc gcagatcatg aaggacaccg gttgctcgat cggcccgatc     720
   tccggcggct gcttcacgac catcgtcacg cctgagggca tgctgattgg cgagccgctc     780
   cgcgagggcg aaggcgaaat catcgccgac ctcgatttct cgatgatcga tcgccgcaag     840
   ctgctgatgg actcggtcgg tcactacaac cgtccggagc ttctgagcct cctgatcgat     900
   cgcacgcctg ccgcgaactt ccatgaacgt accgcgagcc aggcgaacgc cggcgtcgaa     960
   atcctctga                                                             969
<210> 206
   <211> 322
   <212> PRT
   <213> Unknown
<220>
   <223> Obtained from an environmental sample
<400> 206
   Met Thr Thr Val Lys Ala Ala Ala Val Gln Ile Ser Pro Val Leu Tyr
   1               5                   10                  15
   Ser Arg Glu Ala Thr Val Asp Lys Val Val Arg Lys Ile Arg Glu Leu
               20                  25                  30
   Gly Gln Lys Gly Val Gln Phe Ala Thr Phe Pro Glu Thr Val Val Pro
           35                  40                  45
   Tyr Tyr Pro Tyr Phe Ala Ala Val Gln Thr Gly Ile Glu Leu Leu Ser
       50                  55                  60
   Gly Lys Glu His Met Arg Leu Leu Glu Gln Ala Val Thr Val Pro Ser
   65                  70                  75                  80
   Pro Ala Thr Asp Ala Ile Ala Gln Ala Ala Arg Glu Ala Asn Met Val
                   85                  90                  95
   Val Ser Ile Gly Val Asn Glu Arg Asp Gly Gly Thr Ile Tyr Asn Thr
               100                 105                 110
   Gln Leu Leu Phe Asp Ala Asp Gly Thr Leu Val Gln Arg Arg Arg Lys
           115                 120                 125
   Ile Thr Pro Thr His Phe Glu Arg Met Val Trp Gly Gln Gly Asp Gly
       130                 135                 140
   Ser Gly Leu Arg Ala Ala Asp Thr Lys Val Gly Arg Ile Gly Gln Leu
   145                 150                 155                 160
   Ala Cys Phe Glu His Asn Asn Pro Leu Ala Arg Tyr Ala Met Met Ala
                   165                 170                 175
   Asp Gly Glu Gln Ile His Ser Ala Met Tyr Pro Gly Ser Ala Phe Gly
               180                 185                 190
   Glu Gly Phe Ala Gln Arg Met Glu Ile Asn Ile Arg Gln His Ala Leu
           195                 200                 205
   Glu Ser Gly Cys Phe Val Val Asn Ala Thr Ala Trp Leu Asp Ala Asp
       210                 215                 220
   Gln Gln Ala Gln Ile Met Lys Asp Thr Gly Cys Ser Ile Gly Pro Ile
   225                 230                 235                 240
   Ser Gly Gly Cys Phe Thr Thr Ile Val Thr Pro Glu Gly Met Leu Ile
                   245                 250                 255
   Gly Glu Pro Leu Arg Glu Gly Glu Gly Glu Ile Ile Ala Asp Leu Asp
               260                 265                 270
   Phe Ser Met Ile Asp Arg Arg Lys Leu Leu Met Asp Ser Val Gly His
           275                 280                 285
   Tyr Asn Arg Pro Glu Leu Leu Ser Leu Leu Ile Asp Arg Thr Pro Ala
       290                 295                 300
   Ala Asn Phe His Glu Arg Thr Ala Ser Gln Ala Asn Ala Gly Val Glu
   305                 310                 315                 320
   Ile Leu
<210> 207
   <211> 966
   <212> DNA
   <213> Unknown
<220>
   <223> Obtained from an environmental sample
<400> 207
   atgtcaaacg agaacaacaa cgctacattc aaagttgccg cagtacaggc tacacctgtt       60
   tttctcgatc gtgaagcgac tctcgacaag gcttgcgatt tgatcgccgc cgccggaggt      120
   gaaggggcac gattggttgt ctttccagaa gccttcatac cggcctatcc ggattgggta      180
   tgggcaatcc caccgggtga agagggcgta cttaatgagt tgtacgcaga gctgctctcc      240
   aactcggtca cgattcccag tgacgcgacg gacagactgt gccgggccgc gaggcttgct      300
   aatgcttacg tggtgatggg gataagcgaa cgcaatgtcg aggcgagtgg agcaagcctg      360
   tataacacgc tgttgtacat cgatgcgcag ggtgagattc taggcaaaca tcgaaagcta      420
   gtgccaacgg gcggcgagcg gctggtgtgg gcgcagggcg atggcagcac actgcaggtc      480
   tacgatactc cactgggaaa actcggcggt ttaatttgct gggagaatta tatgccgctg      540
   gcccgctata ccatgtatgc ctggggcaca caaatctatg tcgccgctac gtgggatcgc      600
   gggcaaccct ggctctccac tttgcggcat atcgccaaag aaggcagggt gtacgtgatt      660
   ggttgttgta tcgcgatgcg caaagacgat atccctgatc gttacgcaat gaagcagaag      720
   ttttacgcgg aggcagatga gtggatcaat ataggtgaca gcgcgattgt caatcctgaa      780
   gggcaattta tcgcagggcc agtacgcaag caggaagaga ttctctacgc agagattgat      840
   ccgcgcatgg tacaagggcc gaagtggatg ctcgacgtgg cggggcacta tgccaggccg      900
   gatgtgttcc agttgacggt gcatacggat gtgcgacaga tgattcggat ggaacacgat      960
   tcttaa                                                                 966
<210> 208
   <211> 321
   <212> PRT
   <213> Unknown
<220>
   <223> Obtained from an environmental sample
<400> 208
   Met Ser Asn Glu Asn Asn Asn Ala Thr Phe Lys Val Ala Ala Val Gln
   1               5                   10                  15
   Ala Thr Pro Val Phe Leu Asp Arg Glu Ala Thr Leu Asp Lys Ala Cys
               20                  25                  30
   Asp Leu Ile Ala Ala Ala Gly Gly Glu Gly Ala Arg Leu Val Val Phe
           35                  40                  45
   Pro Glu Ala Phe Ile Pro Ala Tyr Pro Asp Trp Val Trp Ala Ile Pro
       50                  55                  60
   Pro Gly Glu Glu Gly Val Leu Asn Glu Leu Tyr Ala Glu Leu Leu Ser
   65                  70                  75                  80
   Asn Ser Val Thr Ile Pro Ser Asp Ala Thr Asp Arg Leu Cys Arg Ala
                   85                  90                  95
   Ala Arg Leu Ala Asn Ala Tyr Val Val Met Gly Ile Ser Glu Arg Asn
               100                 105                 110
   Val Glu Ala Ser Gly Ala Ser Leu Tyr Asn Thr Leu Leu Tyr Ile Asp
           115                 120                 125
   Ala Gln Gly Glu Ile Leu Gly Lys His Arg Lys Leu Val Pro Thr Gly
       130                 135                 140
   Gly Glu Arg Leu Val Trp Ala Gln Gly Asp Gly Ser Thr Leu Gln Val
   145                 150                 155                 160
   Tyr Asp Thr Pro Leu Gly Lys Leu Gly Gly Leu Ile Cys Trp Glu Asn
                   165                 170                 175
   Tyr Met Pro Leu Ala Arg Tyr Thr Met Tyr Ala Trp Gly Thr Gln Ile
               180                 185                 190
   Tyr Val Ala Ala Thr Trp Asp Arg Gly Gln Pro Trp Leu Ser Thr Leu
           195                 200                 205
   Arg His Ile Ala Lys Glu Gly Arg Val Tyr Val Ile Gly Cys Cys Ile
       210                 215                 220
   Ala Met Arg Lys Asp Asp Ile Pro Asp Arg Tyr Ala Met Lys Gln Lys
   225                 230                 235                 240
   Phe Tyr Ala Glu Ala Asp Glu Trp Ile Asn Ile Gly Asp Ser Ala Ile
                   245                 250                 255
   Val Asn Pro Glu Gly Gln Phe Ile Ala Gly Pro Val Arg Lys Gln Glu
               260                 265                 270
   Glu Ile Leu Tyr Ala Glu Ile Asp Pro Arg Met Val Gln Gly Pro Lys
           275                 280                 285
   Trp Met Leu Asp Val Ala Gly His Tyr Ala Arg Pro Asp Val Phe Gln
       290                 295                 300
   Leu Thr Val His Thr Asp Val Arg Gln Met Ile Arg Met Glu His Asp
   305                 310                 315                 320
   Ser
<210> 209
   <211> 993
   <212> DNA
   <213> Unknown
<220>
   <223> Obtained from an environmental sample
<400> 209
   atgaaagtcg tcaaagccgc cgctgtccag atcagtcccg ttctctacag ccgtgaggca     60
   accgtcgaaa aggtcgtgaa gaaaattcac gagcttggcc aactgggcgt gcagttcgcc    120
   acctttcccg agaccgtagt gccttactac ccgtactttt ccgccgtcca gacaggcatt    180
   gagcttctgt ccggaactga gcatctgcgg ctgctcgatc aggccgtgac ggtaccgtct    240
   cccgctaccg atgcgatcgg agaggcggcc cgcaaggcgg gcatggtggt gtccatcggc    300
   gtgaatgaac gcgacggcgg caccttgtac aacacacagt tgctcttcga tgccgatggc    360
   accttgatcc agcgccgccg caagatcacg cccacccact tcgaacggat gatctggggc    420
   cagggggacg gctcgggcct gcgcgccgtc gacagcaagg ttggtcgcat tggtcagctt    480
   gcctgcttcg agcacaacaa cccgctggcc cgctacgcgc tgattgccga cggcgagcag    540
   atccattccg ccatgtatcc gggttctgct ttcggcgaag gctttgccca aaggatggaa    600
   atcaatatcc gccagcatgc gctggagtct ggtgcctttg tcgtcaacgc aacggcctgg    660
   ctggatgctg accagcaggc gcaaatcatc aaggacaccg gctgtgggat tggcccgatc      720
   tcgggcggct gcttcaccac gatcgtggca cccgacggca tgctgatggc cgaacctctg      780
   cgttcgggcg agggtgaggt catcgtggat ctcgacttca cgctgatcga ccgacgcaag      840
   atgttgatgg actcggcggg ccactataac cgtccagaac tgctcagtct catgattgac      900
   cgtaccgcga cggcgcatgt tcacgaacgc gctgcgcatc cggtgtcggg cgcggagcag      960
   ggtccggagg atctgcgcac tccggccgcg tga                                   993
<210> 210
   <211> 330
   <212> PRT
   <213> Unknown
<220>
   <223> Obtained from an environmental sample
<400> 210
   Met Lys Val Val Lys Ala Ala Ala Val Gln Ile Ser Pro Val Leu Tyr
   1               5                   10                  15
   Ser Arg Glu Ala Thr Val Glu Lys Val Val Lys Lys Ile His Glu Leu
               20                  25                  30
   Gly Gln Leu Gly Val Gln Phe Ala Thr Phe Pro Glu Thr Val Val Pro
           35                  40                  45
   Tyr Tyr Pro Tyr Phe Ser Ala Val Gln Thr Gly Ile Glu Leu Leu Ser
       50                  55                  60
   Gly Thr Glu His Leu Arg Leu Leu Asp Gln Ala Val Thr Val Pro Ser
   65                  70                  75                  80
   Pro Ala Thr Asp Ala Ile Gly Glu Ala Ala Arg Lys Ala Gly Met Val
                   85                  90                  95
   Val Ser Ile Gly Val Asn Glu Arg Asp Gly Gly Thr Leu Tyr Asn Thr
               100                 105                 110
   Gln Leu Leu Phe Asp Ala Asp Gly Thr Leu Ile Gln Arg Arg Arg Lys
           115                 120                 125
   Ile Thr Pro Thr His Phe Glu Arg Met Ile Trp Gly Gln Gly Asp Gly
       130                 135                 140
   Ser Gly Leu Arg Ala Val Asp Ser Lys Val Gly Arg Ile Gly Gln Leu
   145                 150                 155                 160
   Ala Cys Phe Glu His Asn Asn Pro Leu Ala Arg Tyr Ala Leu Ile Ala
                   165                 170                 175
   Asp Gly Glu Gln Ile His Ser Ala Met Tyr Pro Gly Ser Ala Phe Gly
               180                 185                 190
   Glu Gly Phe Ala Gln Arg Met Glu Ile Asn Ile Arg Gln His Ala Leu
           195                 200                 205
   Glu Ser Gly Ala Phe Val Val Asn Ala Thr Ala Trp Leu Asp Ala Asp
       210                 215                 220
   Gln Gln Ala Gln Ile Ile Lys Asp Thr Gly Cys Gly Ile Gly Pro Ile
   225                 230                 235                 240
   Ser Gly Gly Cys Phe Thr Thr Ile Val Ala Pro Asp Gly Met Leu Met
                   245                 250                 255
   Ala Glu Pro Leu Arg Ser Gly Glu Gly Glu Val Ile Val Asp Leu Asp
               260                 265                 270
   Phe Thr Leu Ile Asp Arg Arg Lys Met Leu Met Asp Ser Ala Gly His
           275                 280                 285
   Tyr Asn Arg Pro Glu Leu Leu Ser Leu Met Ile Asp Arg Thr Ala Thr
       290                 295                 300
   Ala His Val His Glu Arg Ala Ala His Pro Val Ser Gly Ala Glu Gln
   305                 310                 315                 320
   Gly Pro Glu Asp Leu Arg Thr Pro Ala Ala
                   325                 330
<210> 211
   <211> 1062
   <212> DNA
   <213> unknown
<220>
   <223> Obtained from an environmental sample
<400> 211
   atgggcgtcg cacatccgaa atacaaagtg gccgccgtgc aggcagcgcc cgcttttctc      60
   gacctggacg cctcggtcga aaaggccgtc cgtttcatcg acgaagccgg cgccgccggc     120
   gcccgcctca tcgcctttcc ggagacctgg atacccggtt acccctggtg gatctggcta     180
   ggcgcgccgg cctgggctat catgcgcggc ttcgtctcgc gctatttcga caactcgctc     240
   agctacgaca gcccgcaggc cgagaagctc cgcgccgccg ccaagcgcaa caagatggtg     300
   gtggtgctcg gcctctccga gcgcgacggc ggcagccttt acatcgcgca atggatcatc     360
   ggcccggacg gcgaaaccat cgccaagcgc cgcaagctca agccgaccca cgcggagcgg     420
   accgtgttcg gcgaaggcga cggctcgcat cttgcggtgc acgagcttga tgttggccgg     480
   ctcggcgcgc tgtgctgctg ggaacacctg cagccgctgt ccaaatacgc catgtatgcg     540
   cagaacgaac aggtgcatgt cgcggcctgg ccgagctttt cgctttacga tccgttcgcg     600
   cacgcgctcg gcgcggaagt gaacaatgcg gcgagcaaaa tctatgcggt cgagggctcg     660
   tgtttcgtca tcgcgccgtg cgcgaccgtt tcgcaggcga tgatcgacga actctgcgat     720
   acgccggaga agcatcagtt cctgcatgcc ggcggcggct ttgccgtgat ttacggcccc     780
   gacggcgccc cgctcgcggc gccgctgccg cccgacaagg aaggcttgct ctacgccgac     840
   atcgatctcg ggatgatttc ggttgccaaa gcggcagccg atccggccgg gcattatgca     900
   cgccccgacg tcacccggct tctgttcaac aatcggcctg ggtatcgggt cgagaccatg     960
   gcgttgccga tcgatgcgga gaccaaggcg gaagcaccgg ctaagccgga acccaaggca    1020
   ccgaacgtgg cgccgttcgc gccggtgcaa gcggccgagt ga                       1062
<210> 212
   <211> 353
   <212> PRT
   <213> Unknown
<220>
   <223> Obtained from an environmental sample
<400> 212
   Met Gly Val Ala His Pro Lys Tyr Lys Val Ala Ala Val Gln Ala Ala
   1               5                   10                  15
   Pro Ala Phe Leu Asp Leu Asp Ala Ser Val Glu Lys Ala Val Arg Phe
               20                  25                  30
   Ile Asp Glu Ala Gly Ala Ala Gly Ala Arg Leu Ile Ala Phe Pro Glu
           35                  40                  45
   Thr Trp Ile Pro Gly Tyr Pro Trp Trp Ile Trp Leu Gly Ala Pro Ala
       50                  55                  60
   Trp Ala Ile Met Arg Gly Phe Val Ser Arg Tyr Phe Asp Asn Ser Leu
   65                  70                  75                  80
   Ser Tyr Asp Ser Pro Gln Ala Glu Lys Leu Arg Ala Ala Ala Lys Arg
                   85                  90                  95
   Asn Lys Met Val Val Val Leu Gly Leu Ser Glu Arg Asp Gly Gly Ser
               100                 105                 110
   Leu Tyr Ile Ala Gln Trp Ile Ile Gly Pro Asp Gly Glu Thr Ile Ala
           115                 120                 125
   Lys Arg Arg Lys Leu Lys Pro Thr His Ala Glu Arg Thr Val Phe Gly
       130                 135                 140
   Glu Gly Asp Gly Ser His Leu Ala Val His Glu Leu Asp Val Gly Arg
   145                 150                 155                 160
   Leu Gly Ala Leu Cys Cys Trp Glu His Leu Gln Pro Leu Ser Lys Tyr
                   165                 170                 175
   Ala Met Tyr Ala Gln Asn Glu Gln Val His Val Ala Ala Trp Pro Ser
               180                 185                 190
   Phe Ser Leu Tyr Asp Pro Phe Ala His Ala Leu Gly Ala Glu Val Asn
           195                 200                 205
   Asn Ala Ala Ser Lys Ile Tyr Ala Val Glu Gly Ser Cys Phe Val Ile
       210                 215                 220
   Ala Pro Cys Ala Thr Val Ser Gln Ala Met Ile Asp Glu Leu Cys Asp
   225                 230                 235                 240
   Thr Pro Glu Lys His Gln Phe Leu His Ala Gly Gly Gly Phe Ala Val
                   245                 250                 255
   Ile Tyr Gly Pro Asp Gly Ala Pro Leu Ala Ala Pro Leu Pro Pro Asp
               260                 265                 270
   Lys Glu Gly Leu Leu Tyr Ala Asp Ile Asp Leu Gly Met Ile Ser Val
           275                 280                 285
   Ala Lys Ala Ala Ala Asp Pro Ala Gly His Tyr Ala Arg Pro Asp Val
       290                 295                 300
   Thr Arg Leu Leu Phe Asn Asn Arg Pro Gly Tyr Arg Val Glu Thr Met
   305                 310                 315                 320
   Ala Leu Pro Ile Asp Ala Glu Thr Lys Ala Glu Ala Pro Ala Lys Pro
                   325                 330                 335
   Glu Pro Lys Ala Pro Asn Val Ala Pro Phe Ala Pro Val Gln Ala Ala
               340                 345                 350
   Glu
<210> 213
   <211> 993
   <212> DNA
   <213> Unknown
<220>
   <223> Obtained from an environmental sample
<400> 213
   atgagagttg ttaaagccgc tgctgtccaa ctgagtcccg tcctctatag tcgcgaggga     60
   acggtcgaaa aggtcgtgcg gaagatccat gaacttgccg aagagggagt cgagttcgcc    120
   acctttcctg agaccgtggt gccttactac ccgtactttt ccttcgttca gacgcccttg    180
   gagcaaatct tcggaacaga gtatctgagg ctgctcgacc aggcagtcac cgtgccatcc    240
   cctgccaccg acgcgatcgg cgaggcagcc aggttcgctg gagttgttgt ctcgatcggc    300
   gtcaacgagc gagacggggg aactctatac aacactcagc ttctcttcga tgccgacggc    360
   aggataattc agcggcgccg caagatcacg cccacccatt acgagcgcat gatctggggc    420
   cagggcgacg gctcaggtct gcgggccgtt gatagcaagg ccggccgtat tggtcagctg    480
   gcatgctggg agcacaacaa tccactggcg cgctacgcgc tgatggccga cggcgagcag    540
   atccattccg ccatgtatcc gggctccatg ttcggcgact cgtttgccca gaagaccgaa    600
   atcaatatcc ggcagcatgc cctagagtct gggtgcttcg tcgtgaacgc aacggcctgg    660
   ctggacggcg atcagcaggc gcatatcatg aaggacaccg gctgcagcat cggcccgatc    720
   tccggcggtt gcttcactgc gatcgtcgca cccgatggta gcctgctggg cgaacccatc    780
   cgttccggtg agggcgtggt catcgccgac ctcgacttca cgttgatcga caggcgtaag    840
   caggtgatgg actcgcgagg ccattacagc cggccggagt tgctcagcct cttaatagac    900
   cgcaccccta ccgcgcactt tcacgaacgc gcttcgcccc ccacgacaga agctgagcaa    960
   ggctccgagg atgtgttcga ggctcgcatt taa                                 993
<210> 214
   <211> 330
   <212> PRT
   <213> Unknown
<220>
   <223> obtained from an environmental sample
<400> 214
   Met Arg Val Val Lys Ala Ala Ala Val Gln Leu Ser Pro Val Leu Tyr
   1               5                   10                  15
   Ser Arg Glu Gly Thr Val Glu Lys Val Val Arg Lys Ile His Glu Leu
               20                  25                  30
   Ala Glu Glu Gly Val Glu Phe Ala Thr Phe Pro Glu Thr Val Val Pro
           35                  40                  45
   Tyr Tyr Pro Tyr Phe Ser Phe Val Gln Thr Pro Leu Glu Gln Ile Phe
       50                  55                  60
   Gly Thr Glu Tyr Leu Arg Leu Leu Asp Gln Ala Val Thr Val Pro Ser
   65                  70                  75                  80
   Pro Ala Thr Asp Ala Ile Gly Glu Ala Ala Arg Phe Ala Gly Val Val
                   85                  90                  95
   Val Ser Ile Gly Val Asn Glu Arg Asp Gly Gly Thr Leu Tyr Asn Thr
               100                 105                 110
   Gln Leu Leu Phe Asp Ala Asp Gly Arg Ile Ile Gln Arg Arg Arg Lys
           115                 120                 125
   Ile Thr Pro Thr His Tyr Glu Arg Met Ile Trp Gly Gln Gly Asp Gly
       130                 135                 140
   Ser Gly Leu Arg Ala Val Asp Ser Lys Ala Gly Arg Ile Gly Gln Leu
   145                 150                 155                 160
   Ala Cys Trp Glu His Asn Asn Pro Leu Ala Arg Tyr Ala Leu Met Ala
                   165                 170                 175
   Asp Gly Glu Gln Ile His Ser Ala Met Tyr Pro Gly Ser Met Phe Gly
               180                 185                 190
   Asp Ser Phe Ala Gln Lys Thr Glu Ile Asn Ile Arg Gln His Ala Leu
           195                 200                 205
   Glu Ser Gly Cys Phe Val Val Asn Ala Thr Ala Trp Leu Asp Gly Asp
       210                 215                 220
   Gln Gln Ala His Ile Met Lys Asp Thr Gly Cys Ser Ile Gly Pro Ile
   225                 230                 235                 240
   Ser Gly Gly Cys Phe Thr Ala Ile Val Ala Pro Asp Gly Ser Leu Leu
                   245                 250                 255
   Gly Glu Pro Ile Arg Ser Gly Glu Gly Val Val Ile Ala Asp Leu Asp
               260                 265                 270
   Phe Thr Leu Ile Asp Arg Arg Lys Gln Val Met Asp Ser Arg Gly His
           275                 280                 285
   Tyr Ser Arg Pro Glu Leu Leu Ser Leu Leu Ile Asp Arg Thr Pro Thr
       290                 295                 300
   Ala His Phe His Glu Arg Ala Ser Pro Pro Thr Thr Glu Ala Glu Gln
   305                 310                 315                 320
   Gly Ser Glu Asp Val Phe Glu Ala Arg Ile
                   325                 330
<210> 215
   <211> 1008
   <212> DNA
   <213> Unknown
<220>
   <223> Obtained from an environmental sample
<400> 215
   atgagtatta cccaccccaa atttaaagct gctgttgttc aagctgcccc agtatttcta      60
   gacctagatg ggtctgttaa taaggcgatt aatctcattg atgaagctgc cgctgccgga     120
   gccaagctca ttgccttccc tgaaaccttc attccaggct atccatggtg gatttggctg     180
   ggatcgccgg cgtgggctct gggccggggg ttcgttcagc gttacttcga caattccctg     240
   cagtacgaca gcccgcaggc ggatcgctta cgcgaggcgg cacgacgcaa cagcattacg     300
   gtcgtgctgg gcttgtccga gcgtgatggc ggttctctct atatcgcaca gtggctgatc     360
   ggcccggatg gcgaaaccat cgcgcagcgg cgcaagcttc gtcctactca tggggagcgc     420
   acggtattcg gtgaagggga tggcagcgat ctggtggttc atcaaaccga actgggccgt     480
   cttggcgcgc ttaactgctg ggagaacatc ctgtctctga acaaatatgt gatgtactcc     540
   cagcatgaac aggtccatgt agcatcctgg cccagtttct cgacgtatga accgttcgcg     600
   catgcgctcg gctatgaggt aaacaacgca attagccagg tctatgcggt ggaaggcggg     660
   tgcttcgtgt tggccccgtg ctctaccatc tctgaagaaa tgattgccga actgtgcgat     720
   acacccgata aattcgagct gacgcatgct ggtggcggcc acgcaatcat ctatggtccg     780
   gacggtcgtg ctctgtgcga aaagctgccc gagaaccagg agggcctgct gtacgcggaa     840
   atcgatctgg gggtgatttc tatggcaaaa agtgccatgg atcctgtcgg ccattactct     900
   cgccccgatg tctaccgtgt gctgttcaat aagatcccgg caaagcgtat cgagcacttc     960
   aatttgccgt tggatgagca agcaggggaa gagccaccag ctgattaa                 1008
<210> 216
   <211> 335
   <212> PRT
   <213> Unknown
<220>
   <223> Obtained from an environmental sample
<400> 216
   Met Ser Ile Thr His Pro Lys Phe Lys Ala Ala Val Val Gln Ala Ala
   1 5 10 15
   Pro Val Phe Leu Asp Leu Asp Gly Ser Val Asn Lys Ala Ile Asn Leu
   20 25 30
   Ile Asp Glu Ala Ala Ala Ala Gly Ala Lys Leu Ile Ala Phe Pro Glu
   35 40 45
   Thr Phe Ile Pro Gly Tyr Pro Trp Trp Ile Trp Leu Gly Ser Pro Ala
   50 55 60
   Trp Ala Leu Gly Arg Gly Phe Val Gln Arg Tyr Phe Asp Asn Ser Leu
   65 70 75 80
   Gln Tyr Asp Ser Pro Gln Ala Asp Arg Leu Arg Glu Ala Ala Arg Arg
   85 90 95
   Asn Ser Ile Thr Val Val Leu Gly Leu Ser Glu Arg Asp Gly Gly Ser
   100 105 110
   Leu Tyr Ile Ala Gln Trp Leu Ile Gly Pro Asp Gly Glu Thr Ile Ala
   115 120 125
   Gln Arg Arg Lys Leu Arg Pro Thr His Gly Glu Arg Thr Val Phe Gly
   130 135 140
   Glu Gly Asp Gly Ser Asp Leu Val Val His Gln Thr Glu Leu Gly Arg
   145 150 155 160
   Leu Gly Ala Leu Asn Cys Trp Glu Asn Ile Leu Ser Leu Asn Lys Tyr
   165 170 175
   Val Met Tyr Ser Gln His Glu Gln Val His Val Ala Ser Trp Pro Ser
   180 185 190
   Phe Ser Thr Tyr Glu Pro Phe Ala His Ala Leu Gly Tyr Glu Val Asn
   195 200 205
   Asn Ala Ile Ser Gln Val Tyr Ala Val Glu Gly Gly Cys Phe Val Leu
   210 215 220
   Ala Pro Cys Ser Thr Ile Ser Glu Glu Met Ile Ala Glu Leu Cys Asp
   225 230 235 240
   Thr Pro Asp Lys Phe Glu Leu Thr His Ala Gly Gly Gly His Ala Ile
   245 250 255
   Ile Tyr Gly Pro Asp Gly Arg Ala Leu Cys Glu Lys Leu Pro Glu Asn
   260 265 270
   Gln Glu Gly Leu Leu Tyr Ala Glu Ile Asp Leu Gly Val Ile Ser Met
   275 280 285
   Ala Lys Ser Ala Met Asp Pro Val Gly His Tyr Ser Arg Pro Asp Val
   290 295 300
   Tyr Arg Val Leu Phe Asn Lys Ile Pro Ala Lys Arg Ile Glu His Phe
   305 310 315 320
   Asn Leu Pro Leu Asp Glu Gln Ala Gly Glu Glu Pro Pro Ala Asp
   325 330 335
<210> 217
   <211> 1011
   <212> DNA
   <213> Unknown
<220>
   <223> Obtained from an environmental sample
<400> 217
   gtgggtatca gccatccgaa attcaaggct gcggtcgtac aggccgggcc tgccttcctc      60
   gacctcgacg gcggcgtcga acgagccgtg tcgctcatcg gccaagcagc ggccgaaggg     120
   gcacagctga ttgcctttcc tgaaacgtgg attcccggtt acccgtggca cacctggctt     180
   ggcagcccgg cgtgggcaat ggaaaaaggc tttgtccaac gatatttcga caacgcgttg     240
   cggcatggtt ctccgcaagc cgagcgaatc tccggggctg cggcggagca caagattatg     300
   gtgtcgcttg ggtttgcgga acgcgatgga ggcacgcttt atatcgcgca gtggctcatc     360
   ggacccgacg gccaaactat ctcacgacgg cggaagctta agccgactca cgtcgagcgc     420
   actgtatttg gcgagggaga cggaagcgat ctctccgtgc atgatacggc gcttggacgt     480
   atcggctcac tttgttgctg ggagcatttg caaccgttgt cgaaatacgc aatgtacgcc     540
   cagaatgaac agattcacat tggcgcatgg cccagctttt cgctatacca gccatttgcg     600
   aatgcgctga gtcccgaagt caatatcgca gtaagccgcg tgtacgccgt ggaaggccag     660
   tgtttcttcc tcgcgccgtg cgcgacggtt tcggacgcca tgatcgaaac actgtgcgat     720
   acgcccgaaa agcagggact gattcgggcg ggtggcgggc acgccgcgat cttcggccca     780
   gatggaagtc tgctgacgcc tacggtagcg gatacttacg agggcctgct gtatgcagaa     840
   ctcgacctcg gcgtcatttc gatcgccaag agtgcagcgg accccgccgg ccactattcg     900
   cggccagatg tcacacgcct tctattgaat cagacgcctt cgaagcgcgt tcagaatatg     960
   gtgttaccac tggagacggt cacggagccc gaaggcccgg ttcagcccta g             1011
<210> 218
   <211> 336
   <212> PRT
   <213> Unknown
<220>
   <223> obtained from an environmental sample
<400> 218
   Val Gly Ile Ser His Pro Lys Phe Lys Ala Ala Val Val Gln Ala Gly
   1               5                   10                  15
   Pro Ala Phe Leu Asp Leu Asp Gly Gly Val Glu Arg Ala Val Ser Leu
               20                  25                  30
   Ile Gly Gln Ala Ala Ala Glu Gly Ala Gln Leu Ile Ala Phe Pro Glu
           35                  40                  45
   Thr Trp Ile Pro Gly Tyr Pro Trp His Thr Trp Leu Gly Ser Pro Ala
       50                  55                  60
   Trp Ala Met Glu Lys Gly Phe Val Gln Arg Tyr Phe Asp Asn Ala Leu
   65                  70                  75                  80
   Arg His Gly Ser Pro Gln Ala Glu Arg Ile Ser Gly Ala Ala Ala Glu
                   85                  90                  95
   His Lys Ile Met Val Ser Leu Gly Phe Ala Glu Arg Asp Gly Gly Thr
               100                 105                 110
   Leu Tyr Ile Ala Gln Trp Leu Ile Gly Pro Asp Gly Gln Thr Ile Ser
           115                 120                 125
   Arg Arg Arg Lys Leu Lys Pro Thr His Val Glu Arg Thr Val Phe Gly
       130                 135                 140
   Glu Gly Asp Gly Ser Asp Leu Ser Val His Asp Thr Ala Leu Gly Arg
   145                 150                 155                 160
   Ile Gly Ser Leu Cys Cys Trp Glu His Leu Gln Pro Leu Ser Lys Tyr
                   165                 170                 175
   Ala Met Tyr Ala Gln Asn Glu Gln Ile His Ile Gly Ala Trp Pro Ser
               180                 185                 190
   Phe Ser Leu Tyr Gln Pro Phe Ala Asn Ala Leu Ser Pro Glu Val Asn
           195                 200                 205
   Ile Ala Val Ser Arg Val Tyr Ala Val Glu Gly Gln Cys Phe Phe Leu
       210                 215                 220
   Ala Pro Cys Ala Thr Val Ser Asp Ala Met Ile Glu Thr Leu Cys Asp
   225                 230                 235                 240
   Thr Pro Glu Lys Gln Gly Leu Ile Arg Ala Gly Gly Gly His Ala Ala
                   245                 250                 255
   Ile Phe Gly Pro Asp Gly Ser Leu Leu Thr Pro Thr Val Ala Asp Thr
               260                 265                 270
   Tyr Glu Gly Leu Leu Tyr Ala Glu Leu Asp Leu Gly Val Ile Ser Ile
           275                 280                 285
   Ala Lys Ser Ala Ala Asp Pro Ala Gly His Tyr Ser Arg Pro Asp Val
       290                 295                 300
   Thr Arg Leu Leu Leu Asn Gln Thr Pro Ser Lys Arg Val Gln Asn Met
   305                 310                 315                 320
   Val Leu Pro Leu Glu Thr Val Thr Glu Pro Glu Gly Pro Val Gln Pro
                   325                 330                 335
<210> 219
   <211> 996
   <212> DNA
   <213> Unknown
<220>
   <223> Obtained from an environmental sample
<400> 219
   atgaaaatcg tcaaagctgc cgccgttcaa attagtcccg tgctttatag ccgcgatgga      60
   acggtggaaa aagttgtgcg gaagatccat gagctcggcc aacagggagt gcagttcgca     120
   accttccccg agaccgtgat tccgtactat ccctattttt cctttctgca acccgcctac     180
   cagatcgcgg ccgggcagga gcacctcaag ttgctcgacc aggcggtgac ggtgccgtcc     240
   gctgccacgc acgcgatcgg ccaggcatgc aaacaggcag gagtggttgt ttcgatcggc     300
   atcaatgagc gggacaatgg aacgatctac aacacccagc ttctcttcga ctccgacgga     360
   accctgctgc agcggcgccg caagatctca cccacttacc acgaacggat gatctggggc     420
   cagggcgacg gttccggcct gcgcgccgtg gatagcaagg ccggccggat tgggcagctc     480
   gcctgttggg agcactacaa ccctttggcg cgctacgcga tgatggccga cggcgagcag     540
   atccattccg ccatgtaccc tggctccatc ttcggcgatc tgtttgccga acagacccag     600
   atcaacgtcc ggcagcacgc gttggagtcc ggctgtttcg tagtctgctc caccgcctgg     660
   ttggatcccg atcaacaagc gcaaatcgtc aaggacacag gcggcgccat tggtgcgatc     720
   tcgggcggct gtttcacggc gattgtggcc cccgatagca ctctgctcgg agagccgatc     780
   cgctccggcg aaggcgtggt cattgcggat ctcgacttca cccggatcga caagcgtaaa     840
   cagttgatgg attcccgagg tcattacagc cggccggaat tgctgagtct gctgattgat     900
   cgcacttcca ccgcacatgt gcaaaaccgc gtgccggccg atccattcgg cgccggctgt     960
   gttgcagaac caggagtaga aacatgccca cgatag                               996
<210> 220
   <211> 331
   <212> PRT
   <213> Unknown
<220>
   <223> Obtained from an environmental sample
<400> 220
   Met Lys Ile Val Lys Ala Ala Ala Val Gln Ile Ser Pro Val Leu Tyr
   1               5                   10                  15
   Ser Arg Asp Gly Thr Val Glu Lys Val Val Arg Lys Ile His Glu Leu
               20                  25                  30
   Gly Gln Gln Gly Val Gln Phe Ala Thr Phe Pro Glu Thr Val Ile Pro
           35                  40                  45
   Tyr Tyr Pro Tyr Phe Ser Phe Leu Gln Pro Ala Tyr Gln Ile Ala Ala
       50                  55                  60
   Gly Gln Glu His Leu Lys Leu Leu Asp Gln Ala Val Thr Val Pro Ser
   65                  70                  75                  80
   Ala Ala Thr His Ala Ile Gly Gln Ala Cys Lys Gln Ala Gly Val Val
                   85                  90                  95
   Val Ser Ile Gly Ile Asn Glu Arg Asp Asn Gly Thr Ile Tyr Asn Thr
               100                 105                 110
   Gln Leu Leu Phe Asp Ser Asp Gly Thr Leu Leu Gln Arg Arg Arg Lys
           115                 120                 125
   Ile Ser Pro Thr Tyr His Glu Arg Met Ile Trp Gly Gln Gly Asp Gly
       130                 135                 140
   Ser Gly Leu Arg Ala Val Asp Ser Lys Ala Gly Arg Ile Gly Gln Leu
   145                 150                 155                 160
   Ala Cys Trp Glu His Tyr Asn Pro Leu Ala Arg Tyr Ala Met Met Ala
                   165                 170                 175
   Asp Gly Glu Gln Ile His Ser Ala Met Tyr Pro Gly Ser Ile Phe Gly
               180                 185                 190
   Asp Leu Phe Ala Glu Gln Thr Gln Ile Asn Val Arg Gln His Ala Leu
           195                 200                 205
   Glu Ser Gly Cys Phe Val Val Cys Ser Thr Ala Trp Leu Asp Pro Asp
       210                 215                 220
   Gln Gln Ala Gln Ile Val Lys Asp Thr Gly Gly Ala Ile Gly Ala Ile
   225                 230                 235                 240
   Ser Gly Gly Cys Phe Thr Ala Ile Val Ala Pro Asp Ser Thr Leu Leu
                   245                 250                 255
   Gly Glu Pro Ile Arg Ser Gly Glu Gly Val Val Ile Ala Asp Leu Asp
               260                 265                 270
   Phe Thr Arg Ile Asp Lys Arg Lys Gln Leu Met Asp Ser Arg Gly His
           275                 280                 285
   Tyr Ser Arg Pro Glu Leu Leu Ser Leu Leu Ile Asp Arg Thr Ser Thr
       290                 295                 300
   Ala His Val Gln Asn Arg Val Pro Ala Asp Pro Phe Gly Ala Gly Cys
   305                 310                 315                 320
   Val Ala Glu Pro Gly Val Glu Thr Cys Pro Arg
                   325                 330
<210> 221
   <211> 1146
   <212> DNA
   <213> Unknown
<220>
   <223> Obtained from an environmental sample
<400> 221
   atgttgacct acaaaggcgt gttcaaagca gcgacagttc aggcggagcc ggtgtggatg      60
   gacgctgacg ccaccattac caaagccatc cggatcatcg aagaggcggc tgataacggg     120
   gcgaagtttg tcgcattccc ggaggtgttc atccctggat acccttggtg gatctggctg     180
   ggcactgcga tgtggggagc caaattcgtc gtgcccttcc acgaaaattg ccttgagctt     240
   ggcgacaagc ggatgcagcg cattcaagct gcggcaaaac aaaatggcat tgcactcgtc     300
   atgggttacg gtgaacgtga cggaggtagc cgctacatga gccaggtatt catcgacgat     360
   agcggaaaaa ttgtggcgaa ccgccgcaag ctcaaaccga cgcacgaaga gcgaacgatt     420
   ttcggcgagg gaaacggatc cgatttcatc acccatgact tcccatttgc gagagtcgga     480
   ggcttcaact gctgggaaca ccttcagccc ctgagcaaat acatgatgta cagcctgcag     540
   gaacaggtgc atgtcgcgtc gtggccggcc atgtgcacct atcagcctga cgtgccccag     600
   ttaggtgcag gcgctaacga ggcggtcacc cgctcttacg cgatcgaagg cgcatgttat     660
   gtgctgggag cgacactcgt tatcggcaag gcagcccatg atgcattctg cgacactgaa     720
   gagcatcaca agctgcttgg catgggcgga gggtgggccc gaattttcgg cccggacggt     780
   gagtatctcg cagaatcgct cgctcatgac gcggagggca tcctgtacgc tgacattgat     840
   ctgagcaaga tcctgctcgc caaagcaaac acggacacag ttggccacta cgcgcgacct     900
   gacgtcttgt cattgctcgt caacacacac aatcccgggc ctgtacgcta tcttgacgaa     960
   gagggccggc aggtttcaac gtcaattcgc aggcacgaaa aactggaagg ccagagcctc    1020
   gacctcgagg ttacgccagc gacaccagcg accctggaca tcgcgagcct ggtacaacag    1080
   gctaagccat cgaccgttaa gagcgagagt aacgcgagca caaaacaacc ggatctcgcg    1140
   gtctga                                                               1146
<210> 222
   <211> 381
   <212> PRT
   <213> Unknown
<220>
   <223> Obtained from an environmental sample
<400> 222
   Met Leu Thr Tyr Lys Gly Val Phe Lys Ala Ala Thr Val Gln Ala Glu
   1               5                   10                  15
   Pro Val Trp Met Asp Ala Asp Ala Thr Ile Thr Lys Ala Ile Arg Ile
               20                  25                  30
   Ile Glu Glu Ala Ala Asp Asn Gly Ala Lys Phe Val Ala Phe Pro Glu
           35                  40                  45
   Val Phe Ile Pro Gly Tyr Pro Trp Trp Ile Trp Leu Gly Thr Ala Met
       50                  55                  60
   Trp Gly Ala Lys Phe Val Val Pro Phe His Glu Asn Cys Leu Glu Leu
   65                  70                  75                  80
   Gly Asp Lys Arg Met Gln Arg Ile Gln Ala Ala Ala Lys Gln Asn Gly
                   85                  90                  95
   Ile Ala Leu Val Met Gly Tyr Gly Glu Arg Asp Gly Gly Ser Arg Tyr
               100                 105                 110
   Met Ser Gln Val Phe Ile Asp Asp Ser Gly Lys Ile Val Ala Asn Arg
           115                 120                 125
   Arg Lys Leu Lys Pro Thr His Glu Glu Arg Thr Ile Phe Gly Glu Gly
       130                 135                 140
   Asn Gly Ser Asp Phe Ile Thr His Asp Phe Pro Phe Ala Arg Val Gly
   145                 150                 155                 160
   Gly Phe Asn Cys Trp Glu His Leu Gln Pro Leu Ser Lys Tyr Met Met
                   165                 170                 175
   Tyr Ser Leu Gln Glu Gln Val His Val Ala Ser Trp Pro Ala Met Cys
               180                 185                 190
   Thr Tyr Gln Pro Asp Val Pro Gln Leu Gly Ala Gly Ala Asn Glu Ala
           195                 200                 205
   Val Thr Arg Ser Tyr Ala Ile Glu Gly Ala Cys Tyr Val Leu Gly Ala
       210                 215                 220
   Thr Leu Val Ile Gly Lys Ala Ala His Asp Ala Phe Cys Asp Thr Glu
   225                 230                 235                 240
   Glu His His Lys Leu Leu Gly Met Gly Gly Gly Trp Ala Arg Ile Phe
                   245                 250                 255
   Gly Pro Asp Gly Glu Tyr Leu Ala Glu Ser Leu Ala His Asp Ala Glu
               260                 265                 270
   Gly Ile Leu Tyr Ala Asp Ile Asp Leu Ser Lys Ile Leu Leu Ala Lys
           275                 280                 285
   Ala Asn Thr Asp Thr Val Gly His Tyr Ala Arg Pro Asp Val Leu Ser
       290                 295                 300
   Leu Leu Val Asn Thr His Asn Pro Gly Pro Val Arg Tyr Leu Asp Glu
   305                 310                 315                 320
   Glu Gly Arg Gln Val Ser Thr Ser Ile Arg Arg His Glu Lys Leu Glu
                   325                 330                 335
   Gly Gln Ser Leu Asp Leu Glu Val Thr Pro Ala Thr Pro Ala Thr Leu
               340                 345                 350
   Asp Ile Ala Ser Leu Val Gln Gln Ala Lys Pro Ser Thr Val Lys Ser
           355                 360                 365
   Glu Ser Asn Ala Ser Thr Lys Gln Pro Asp Leu Ala Val
       370                 375                 380
<210> 223
   <211> 996
   <212> DNA
   <213> Unknown
<220>
   <223> Obtained from an environmental sample
<400> 223
   atgagagttg ttaaagccag cgcagttcaa ctgaagcctg tactttatag ccgcgagggg      60
   acggtcgaaa aggtcgtagc gaagattcat gagctagggc agcagggtgt gcagttcgcc     120
   gcgttccctg agaccgtggt gccttattac ccgtactttt cgatcgtgca gtccggctat     180
   caaatccttc gcggcggtga gttcgtcaag ctcctcgatc agtccgtgac ggtgccatct     240
   tatagcactg aagccatcgg cgaagcctgc aggcaggagg agatggttgt ctccataggc     300
   gtcaacgagc gtgatggcgg aacgatctac aacgcgcagt tactctttga ttccgacggc     360
   acgttgatcc aaagacgacg caagatcacc cccacccatt acgaacgcat gatctggggc     420
   cagggcgatg gctcaggtct gcgcgctgtt gacagcaatg tcgcacgcat tggtcaactg     480
   gcatgctttg agcactacaa ccctcttgcg cggtacgcga tgatggccga tggcgagcag     540
   atccattccg ccatgttccc cggttccatg ttcggcgatg gttttgcgga gaggacggaa     600
   attgccgtaa ggcaacatgc gatggagtcc gggtgctttg tcgtttgcgc tacggcctgg     660
   ctcgatcccg gccagcaggc tcagatcgcc aacgacaccg gtatcaccga catcggcccg     720
   atctccgggg gttgcttcac tgcgatcatc gcacccgatg ggagcctgct gggccaacct     780
   atccgctcgg gtgaaggcga agtcatcgtc gacctcgatt tcacgttaat tgacaagcgg     840
   aaacatattg tcgactcgag aggacattac agccggccag aattgctgag cctgctgatc     900
   gatcgcactc ccaccgcgca ccttcacgac cgcgctgtgc agcacaatgc cggatcggaa     960
   ggagcgtcgg aacatcttcg cgaagacgcc gcctga                                996
<210> 224
   <211> 331
   <212> PRT
   <213> Unknown
<220>
   <223> Obtained from an environmental sample
<400> 224
   Met Arg Val Val Lys Ala Ser Ala Val Gln Leu Lys Pro Val Leu Tyr
   1 5 10 15
   Ser Arg Glu Gly Thr Val Glu Lys Val Val Ala Lys Ile His Glu Leu
   20 25 30
   Gly Gln Gln Gly Val Gln Phe Ala Ala Phe Pro Glu Thr Val Val Pro
   35 40 45
   Tyr Tyr Pro Tyr Phe Ser Ile Val Gln Ser Gly Tyr Gln Ile Leu Arg
   50 55 60
   Gly Gly Glu Phe Val Lys Leu Leu Asp Gln Ser Val Thr Val Pro Ser
   65 70 75 80
   Tyr Ser Thr Glu Ala Ile Gly Glu Ala Cys Arg Gln Glu Glu Met Val
   85 90 95
   Val Ser Ile Gly Val Asn Glu Arg Asp Gly Gly Thr Ile Tyr Asn Ala
   100 105 110
   Gln Leu Leu Phe Asp Ser Asp Gly Thr Leu Ile Gln Arg Arg Arg Lys
   115 120 125
   Ile Thr Pro Thr His Tyr Glu Arg Met Ile Trp Gly Gln Gly Asp Gly
   130 135 140
   Ser Gly Leu Arg Ala Val Asp Ser Asn Val Ala Arg Ile Gly Gln Leu
   145 150 155 160
   Ala Cys Phe Glu His Tyr Asn Pro Leu Ala Arg Tyr Ala Met Met Ala
   165 170 175
   Asp Gly Glu Gln Ile His Ser Ala Met Phe Pro Gly Ser Met Phe Gly
   180 185 190
   Asp Gly Phe Ala Glu Arg Thr Glu Ile Ala Val Arg Gln His Ala Met
   195 200 205
   Glu Ser Gly Cys Phe Val Val Cys Ala Thr Ala Trp Leu Asp Pro Gly
   210 215 220
   Gln Gln Ala Gln Ile Ala Asn Asp Thr Gly Ile Thr Asp Ile Gly Pro
   225 230 235 240
   Ile Ser Gly Gly Cys Phe Thr Ala Ile Ile Ala Pro Asp Gly Ser Leu
   245 250 255
   Leu Gly Gln Pro Ile Arg Ser Gly Glu Gly Glu Val Ile Val Asp Leu
   260 265 270
   Asp Phe Thr Leu Ile Asp Lys Arg Lys His Ile Val Asp Ser Arg Gly
   275 280 285
   His Tyr Ser Arg Pro Glu Leu Leu Ser Leu Leu Ile Asp Arg Thr Pro
   290 295 300
   Thr Ala His Leu His Asp Arg Ala Val Gln His Asn Ala Gly Ser Glu
   305 310 315 320
   Gly Ala Ser Glu His Leu Arg Glu Asp Ala Ala
   325 330
<210> 225
   <211> 951
   <212> DNA
   <213> Unknown
<220>
   <223> Obtained from an environmental sample
<400> 225
   atgaccaccg tcaaagccgc cgctgtccag atgagtcccg tgctttacag tcgcgacgat      60
   accatcgaga aaatttgtcg acagatcatc gagcttggcc gacagggagt gcagttcgcc     120
   acgtttccag agacagttat tccgtattat ccatatttcg ccttcgtgca gcggccttac     180
   gaaatgtcgg cccaatatca tcagttgctt gatcaagcgg tgaccgtgcc ttccggttca     240
   acgcacgcca ttggggccgc ctgtaaacaa gccggaattg ttgtctcaat cggcgtcaac     300
   gagcgggagg gcggcacgct ctatggcaca cagttgctgt ttgatgcaga cggcctcttg     360
   atccagcgtc gccgcaagat cactccgacc taccacgagc ggatgatttg gggacagggt     420
   gacggttccg ggctgcgagc cgtagatagc gcggtcggtc gaatcggaca gttggcgtgt     480
   tgggaacacc acaatccgct ggctcgttat gctttagcgg ccgatggcga acaaattcac     540
   gcggcgatgt accctggctc gatcttgggt gaactatttg ccgagcagat tcaggtcaac     600
   atccggcagc acgccatgga atctggttgc ttcgtcgtga acgccacggc ctggctaagc     660
   gaggaacagc aagcccgaat catgaaggac accggatcat tcgatagccc aatcaccggt     720
   ggttgcttta ccgccattgt cgcgcccaac gggcagataa tcggtgaacc gctgcgcatc     780
   ggcgaaggcg tcgtgattgc cgatttggac ttcgctttga ttgatgagag gaagcggctg     840
   atggactcac gcggcctcta tagccgccct gagttgctaa gcttgttaat cgacagaatg     900
   cctacatccc atgtgcatga acgggttgag cgtagcatgg cgatggcatg a              951
<210> 226
   <211> 316
   <212> PRT
   <213> Unknown
<220>
   <223> Obtained from an environmental sample
<400> 226
   Met Thr Thr Val Lys Ala Ala Ala Val Gln Met Ser Pro Val Leu Tyr
   1               5                   10                  15
   Ser Arg Asp Asp Thr Ile Glu Lys Ile Cys Arg Gln Ile Ile Glu Leu
               20                  25                  30
   Gly Arg Gln Gly Val Gln Phe Ala Thr Phe Pro Glu Thr Val Ile Pro
           35                  40                  45
   Tyr Tyr Pro Tyr Phe Ala Phe Val Gln Arg Pro Tyr Glu Met Ser Ala
       50                  55                  60
   Gln Tyr His Gln Leu Leu Asp Gln Ala Val Thr Val Pro Ser Gly Ser
   65                  70                  75                  80
   Thr His Ala Ile Gly Ala Ala Cys Lys Gln Ala Gly Ile Val Val Ser
                   85                  90                  95
   Ile Gly Val Asn Glu Arg Glu Gly Gly Thr Leu Tyr Gly Thr Gln Leu
               100                 105                 110
   Leu Phe Asp Ala Asp Gly Leu Leu Ile Gln Arg Arg Arg Lys Ile Thr
           115                 120                 125
   Pro Thr Tyr His Glu Arg Met Ile Trp Gly Gln Gly Asp Gly Ser Gly
       130                 135                 140
   Leu Arg Ala Val Asp Ser Ala Val Gly Arg Ile Gly Gln Leu Ala Cys
   145                 150                 155                 160
   Trp Glu His His Asn Pro Leu Ala Arg Tyr Ala Leu Ala Ala Asp Gly
                   165                 170                 175
   Glu Gln Ile His Ala Ala Met Tyr Pro Gly Ser Ile Leu Gly Glu Leu
               180                 185                 190
   Phe Ala Glu Gln Ile Gln Val Asn Ile Arg Gln His Ala Met Glu Ser
           195                 200                 205
   Gly Cys Phe Val Val Asn Ala Thr Ala Trp Leu Ser Glu Glu Gln Gln
       210                 215                 220
   Ala Arg Ile Met Lys Asp Thr Gly Ser Phe Asp Ser Pro Ile Thr Gly
   225                 230                 235                 240
   Gly Cys Phe Thr Ala Ile Val Ala Pro Asn Gly Gln Ile Ile Gly Glu
                   245                 250                 255
   Pro Leu Arg Ile Gly Glu Gly Val Val Ile Ala Asp Leu Asp Phe Ala
               260                 265                 270
   Leu Ile Asp Glu Arg Lys Arg Leu Met Asp Ser Arg Gly Leu Tyr Ser
           275                 280                 285
   Arg Pro Glu Leu Leu Ser Leu Leu Ile Asp Arg Met Pro Thr Ser His
       290                 295                 300
   Val His Glu Arg Val Glu Arg Ser Met Ala Met Ala
   305                 310                 315
<210> 227
   <211> 1035
   <212> DNA
   <213> Unknown
<220>
   <223> Obtained from an environmental sample
<400> 227
   atgtccgatc gacgcatcgt tcgcgcggca gccattcaga tcgccccgga tctcgagcgc      60
   tgcagtgtaa cgctcgagaa ggtttgctcg gcgatcgacg aagccgcggg caaaggcgct     120
   caactgagcg tctttcccga gaccttcgtc ccgtactacc cctatttctc gtttgtccgc     180
   cctccggtcg catcgggggc cgatcacatg cggctatacg aggaagcggt ggtggtgccg     240
   ggtcccgtga cgcaggctgt ttccgaacgt gcgcgcatgc atgggatggt ggtggtgctc     300
   ggcgtcaacg agcgcgacca cgggagcctc tacaacaccc aactgatttt cgattgcgat     360
   ggtcggctcg ctctgaaacg ccgcaagatc acgccgacgt ttcacgagcg catgatctgg     420
   ggccagggcg acgccagtgg actgaaagtc gcgcgcacgg gtatcgggcg ggtcggcgcg     480
   ctcgcgtgct gggagcatta caacccgctc gcgcgctacg cgctgatgac ccagcacgaa     540
   gagatccatt gcagtcaatt tccaggctcg ctcgtcggtc cgatcttctc cgagcagatg     600
   gacgtgacga tccgccatca cgccctcgaa tcggggtgct tcgtcgtgaa cgcaaccggt     660
   tggctcacgg acgcgcagat cgcctcgatc accgatgacc cgaagcttca acgggcgctg     720
   cgcggtggct gcaacacggc gatcgtttca ccggaaggcc agcatctggc ggagccgttg     780
   cgcgaaggcg aggggatggt ggtcgcggac ctcgacatgt cgctcatcac caagcgcaag     840
   cgaatgatgg attcggtcgg ccactatgcg cggccggaac tgttgagcct cgcgatcaac     900
   gatcgtcccg cagccccttt cggccggatg tgcgctgccg aagcaatgcg gggagccgac     960
   gacgtcgtca cagtaggagc atttcatgag cgccagcgag aacgtgtcgg cgaagagccg    1020
   gcaattgatg actga                                                     1035
<210> 228
   <211> 344
   <212> PRT
   <213> unknown
<220>
   <223> Obtained from an environmental sample
<400> 228
   Met Ser Asp Arg Arg Ile Val Arg Ala Ala Ala Ile Gln Ile Ala Pro
   1 5 10 15
   Asp Leu Glu Arg Cys Ser Val Thr Leu Glu Lys Val Cys Ser Ala Ile
   20 25 30
   Asp Glu Ala Ala Gly Lys Gly Ala Gln Leu Ser Val Phe Pro Glu Thr
   35 40 45
   Phe Val Pro Tyr Tyr Pro Tyr Phe Ser Phe Val Arg Pro Pro Val Ala
   50 55 60
   Ser Gly Ala Asp His Met Arg Leu Tyr Glu Glu Ala Val Val Val Pro
   65 70 75 80
   Gly Pro Val Thr Gln Ala Val Ser Glu Arg Ala Arg Met His Gly Met
   85 90 95
   Val Val Val Leu Gly Val Asn Glu Arg Asp His Gly Ser Leu Tyr Asn
   100 105 110
   Thr Gln Leu Ile Phe Asp Cys Asp Gly Arg Leu Ala Leu Lys Arg Arg
   115 120 125
   Lys Ile Thr Pro Thr Phe His Glu Arg Met Ile Trp Gly Gln Gly Asp
   130 135 140
   Ala Ser Gly Leu Lys Val Ala Arg Thr Gly Ile Gly Arg Val Gly Ala
   145 150 155 160
   Leu Ala Cys Trp Glu His Tyr Asn Pro Leu Ala Arg Tyr Ala Leu Met
   165 170 175
   Thr Gln His Glu Glu Ile His Cys Ser Gln Phe Pro Gly Ser Leu Val
   180 185 190
   Gly Pro Ile Phe Ser Glu Gln Met Asp Val Thr Ile Arg His His Ala
   195 200 205
   Leu Glu Ser Gly Cys Phe Val Val Asn Ala Thr Gly Trp Leu Thr Asp
   210 215 220
   Ala Gln Ile Ala Ser Ile Thr Asp Asp Pro Lys Leu Gln Arg Ala Leu
   225 230 235 240
   Arg Gly Gly Cys Asn Thr Ala Ile Val Ser Pro Glu Gly Gln His Leu
   245 250 255
   Ala Glu Pro Leu Arg Glu Gly Glu Gly Met Val Val Ala Asp Leu Asp
   260 265 270
   Met Ser Leu Ile Thr Lys Arg Lys Arg Met Met Asp Ser Val Gly His
   275 280 285
   Tyr Ala Arg Pro Glu Leu Leu Ser Leu Ala Ile Asn Asp Arg Pro Ala
   290 295 300
   Ala Pro Phe Gly Arg Met Cys Ala Ala Glu Ala Met Arg Gly Ala Asp
   305 310 315 320
   Asp Val Val Thr Val Gly Ala Phe His Glu Arg Gln Arg Glu Arg Val
   325 330 335
   Gly Glu Glu Pro Ala Ile Asp Asp
   340
<210> 229
   <211> 975
   <212> DNA
   <213> Unknown
<220>
   <223> Obtained from an environmental sample
<400> 229
   atgcctgaaa acaaagtcaa agttgccctc gttcagcatc cgcctgtttt tctgaatttg      60
   ccgaaaacgt tggaaaaagt agaaggtttg gcgcgagagt gcgccgccaa tgaagcgaaa     120
   atcgttgtct ttcctgaaac ctggctgacc ggctatccgg tctggcttga tgaagcgccg     180
   agagccgcgc tgtgggatta tccgcccgcc aagcgtttgt atcaatacct cacggaaaat     240
   tcattgcaga ttccgagcgc tgagtttgaa tctttgcgcg aaatcgctaa gaaaaattcc     300
   ctttatttag tcgttggagt tcacgaacga agcggcggaa cgctctacaa tacgataatt     360
   tatctcacgc ccgacggcag ttataaaact caccgcaaat tggttccaac ttacacggaa     420
   agacttgtct ggggcgcagg cgacggaagc ggtctaaatg ttgtggaaac gccttacggg     480
   attctcggag gtttgatttg ctgggaacat tggatgcctc tggctagggc ggcaatgcat     540
   tcaaaaaatg aagcgattca cgtttgccaa tttcccacgg ttcacgagcg acatcaaatc     600
   gccagccgtc attacgcctt cgaagggcag tgttttgtct tgacttccgg ttgcgcgatg     660
   acgaaaacgg atgttttgga aggttttgaa tcgctcgaaa caaacgacca cgaagttttc    720
   gggcttttgg attcgataga aaaggaagaa ctgatgcgtg gcggaagcgc gattattgcg     780
   cccgatttga gctattcggt cgagccggtt tttgacgaaa aaacgattgt ttacggcgaa     840
   ttaaatctcg atttaaccaa gcagggacat ctgtttttgg ataccgacgg acattattcg     900
   cgtcccgatg ttttcgagtt gcgcgtcaac gataaagcga accgaaacgt ccgttttgca     960
   tccgaaacag tatag                                                      975
<210> 230
   <211> 324
   <212> PRT
   <213> Unknown
<220>
   <223> Obtained from an environmental sample
<400> 230
   Met Pro Glu Asn Lys Val Lys Val Ala Leu Val Gln His Pro Pro Val
   1               5                   10                  15
   Phe Leu Asn Leu Pro Lys Thr Leu Glu Lys Val Glu Gly Leu Ala Arg
               20                  25                  30
   Glu Cys Ala Ala Asn Glu Ala Lys Ile Val Val Phe Pro Glu Thr Trp
           35                  40                  45
   Leu Thr Gly Tyr Pro Val Trp Leu Asp Glu Ala Pro Arg Ala Ala Leu
       50                  55                  60
   Trp Asp Tyr Pro Pro Ala Lys Arg Leu Tyr Gln Tyr Leu Thr Glu Asn
   65                  70                  75                  80
   Ser Leu Gln Ile Pro Ser Ala Glu Phe Glu Ser Leu Arg Glu Ile Ala
                   85                  90                  95
   Lys Lys Asn Ser Leu Tyr Leu Val Val Gly Val His Glu Arg Ser Gly
               100                 105                 110
   Gly Thr Leu Tyr Asn Thr Ile Ile Tyr Leu Thr Pro Asp Gly Ser Tyr
           115                 120                 125
   Lys Thr His Arg Lys Leu Val Pro Thr Tyr Thr Glu Arg Leu Val Trp
       130                 135                 140
   Gly Ala Gly Asp Gly Ser Gly Leu Asn Val Val Glu Thr Pro Tyr Gly
   145                 150                 155                 160
   Ile Leu Gly Gly Leu Ile Cys Trp Glu His Trp Met Pro Leu Ala Arg
                   165                 170                 175
   Ala Ala Met His Ser Lys Asn Glu Ala Ile His Val Cys Gln Phe Pro
               180                 185                 190
   Thr Val His Glu Arg His Gln Ile Ala Ser Arg His Tyr Ala Phe Glu
           195                 200                 205
   Gly Gln Cys Phe Val Leu Thr Ser Gly Cys Ala Met Thr Lys Thr Asp
       210                 215                 220
   Val Leu Glu Gly Phe Glu Ser Leu Glu Thr Asn Asp His Glu Val Phe
   225                 230                 235                 240
   Gly Leu Leu Asp Ser Ile Glu Lys Glu Glu Leu Met Arg Gly Gly Ser
                   245                 250                 255
   Ala Ile Ile Ala Pro Asp Leu Ser Tyr Ser Val Glu Pro Val Phe Asp
               260                 265                 270
   Glu Lys Thr Ile Val Tyr Gly Glu Leu Asn Leu Asp Leu Thr Lys Gln
           275                 280                 285
   Gly His Leu Phe Leu Asp Thr Asp Gly His Tyr Ser Arg Pro Asp Val
       290                 295                 300
   Phe Glu Leu Arg Val Asn Asp Lys Ala Asn Arg Asn Val Arg Phe Ala
   305                 310                 315                 320
   Ser Glu Thr Val
<210> 231
   <211> 1062
   <212> DNA
   <213> Unknown
<220>
   <223> Obtained from an environmental sample
<400> 231
   atgagtcaga tccatccaaa acttaaggtc gcggccgtgc aggctgcccc cgcctttctc      60
   gatctcgatg catcgatcga aaagacaata cgctatgtcg acgaagcggc tgcggccggg     120
   gcgaagttga ttgcgtttcc ggaaacctgg attcccggct acccatggtg gatctggctc     180
   ggcgctccgg cctgggcgat catgcgtggc ttcgtctcgc gctatttcga caactcgctg     240
   caatatggca gtccggaagc tgaacggctg cgggacgccg ccaggcgcaa caagatatac     300
   atcgccctcg gcctgtccga gcgcgacggg ggcagtcttt atatcgcgca atggatcatc     360
   gggcctggcg gcgaaacggt tgcacaacgc cgcaagctca agcccacgca cgccgagcgc     420
   actgtattcg gcgaaggcga tggttcacat ctggccgtgc atgatctcga tattggaaga     480
   ttgggcgcgc tttgttgctg ggaacatctg caaccgttgt cgaaatatgc aatgtacgcc     540
   cagaacgagc aaattcacgt cgccgcctgg ccgagcttct cgctatacga tccctttgca     600
   cacgcactcg gcgccgaggt caataacgct gcgagcaaga tctatgcggt cgagggatcg     660
   tgcttcgtca ttgcgccgtg cgcaacggtt tcgcaggtga tgatcgatga gctctgcgat     720
   acccccgaaa agcatcaatt ccttcacgtc ggcggcggct tcgccgtcat ttacggtccc     780
   gacggctcgc cactggccaa acctcttccg ccagaccagg agggacttct ctatgccgac     840
   atcgatctcg gcatgatctc ggtcgccaag gccgcagccg atcccgccgg acattatgca     900
   cgtcccgatg taactcgcct gctgttcaac aatcgcccgg caaaccgcgt cgagaagctg     960
   gcgttgccgg tcgatcagga ggccgaagtg gatagtccgc tgaaggctcc cgacgcatct    1020
   cccaaagtga cggcgctcaa gccgtcgcag gctgcggagt ag                       1062
<210> 232
   <211> 353
   <212> PRT
   <213> Unknown
<220>
   <223> Obtained from an environmental sample
<400> 232
   Met Ser Gln Ile His Pro Lys Leu Lys Val Ala Ala Val Gln Ala Ala
    1               5                   10                  15
   Pro Ala Phe Leu Asp Leu Asp Ala Ser Ile Glu Lys Thr Ile Arg Tyr
               20                  25                  30
   Val Asp Glu Ala Ala Ala Ala Gly Ala Lys Leu Ile Ala Phe Pro Glu
           35                  40                  45
   Thr Trp Ile Pro Gly Tyr Pro Trp Trp Ile Trp Leu Gly Ala Pro Ala
       50                  55                  60
   Trp Ala Ile Met Arg Gly Phe Val Ser Arg Tyr Phe Asp Asn Ser Leu
   65                  70                  75                  80
   Gln Tyr Gly Ser Pro Glu Ala Glu Arg Leu Arg Asp Ala Ala Arg Arg
                   85                  90                  95
   Asn Lys Ile Tyr Ile Ala Leu Gly Leu Ser Glu Arg Asp Gly Gly Ser
               100                 105                 110
   Leu Tyr Ile Ala Gln Trp Ile Ile Gly Pro Gly Gly Glu Thr Val Ala
           115                 120                 125
   Gln Arg Arg Lys Leu Lys Pro Thr His Ala Glu Arg Thr Val Phe Gly
       130                 135                 140
   Glu Gly Asp Gly Ser His Leu Ala Val His Asp Leu Asp Ile Gly Arg
   145                 150                 155                 160
   Leu Gly Ala Leu Cys Cys Trp Glu His Leu Gln Pro Leu Ser Lys Tyr
                   165                 170                 175
   Ala Met Tyr Ala Gln Asn Glu Gln Ile His Val Ala Ala Trp Pro Ser
               180                 185                 190
   Phe Ser Leu Tyr Asp Pro Phe Ala His Ala Leu Gly Ala Glu Val Asn
           195                 200                 205
   Asn Ala Ala Ser Lys Ile Tyr Ala Val Glu Gly Ser Cys Phe Val Ile
       210                 215                 220
   Ala Pro Cys Ala Thr Val Ser Gln Val Met Ile Asp Glu Leu Cys Asp
   225                 230                 235                 240
   Thr Pro Glu Lys His Gln Phe Leu His Val Gly Gly Gly Phe Ala Val
                   245                 250                 255
   Ile Tyr Gly Pro Asp Gly Ser Pro Leu Ala Lys Pro Leu Pro Pro Asp
               260                 265                 270
   Gln Glu Gly Leu Leu Tyr Ala Asp Ile Asp Leu Gly Met Ile Ser Val
           275                 280                 285
   Ala Lys Ala Ala Ala Asp Pro Ala Gly His Tyr Ala Arg Pro Asp Val
       290                 295                 300
   Thr Arg Leu Leu Phe Asn Asn Arg Pro Ala Asn Arg Val Glu Lys Leu
   305                 310                 315                 320
   Ala Leu Pro Val Asp Gln Glu Ala Glu Val Asp Ser Pro Leu Lys Ala
                   325                 330                 335
   Pro Asp Ala Ser Pro Lys Val Thr Ala Leu Lys Pro Ser Gln Ala Ala
               340                 345                 350
   Glu
<210> 233
   <211> 1002
   <212> DNA
   <213> Unknown
<220>
   <223> Obtained from an environmental sample
<400> 233
   gtgagcacca tcgtcaaagc cgcggccgtg cagatcagcc ccgtgctgta cagccgcgag      60
   ggcaccgtcg agcgggtcgt gaagaagatc cgggagctgg gcgaaaaggg cgtccagttc     120
   gccaccttcc ccgagaccgt catcccttac tacccgtact tttccttcgt tcagacgccc     180
   ttgcagatcc tcgccggccc cgagcatctg aagctgctcg accagtcggt gaccgtgccg     240
   tcccccgcca cggacgcgat cggccaggcc gcccggcagg caggaatggt ggtgtccatc     300
   ggcgtcaacg agcgtgacgg cggcaccctg tacaacacgc agctgctctt cgacgcggac     360
   ggcgcgctga tccagcgtcg ccgcaagatc aagcccaccc actacgagcg catgatctgg     420
   ggcgagggcg acggctccgg cctgcgcgcc gtcgacagcc aggtcggtcg tatcggccag     480
   ctggcctgct gggagcacaa caaccccctg gcgcgctacg ccatgatggc cgacggcgag    540
   cagatccatt cggccatgta tccgggctcg atgttcggcg acccgttcgc ccagaagacg     600
   gaaatcaaca tccggcagca tgcgctggaa tccggatgct tcgtcgtctg ctcgacggcc     660
   tggttggacg ccgatcagca ggcgcaaatc atgcaggaca cgggctgcgc catcggcccg     720
   atctcgggcg gctgcctcac ggcgatcgtg gcgcccgacg gcacgttcct gggcgaaccg     780
   ctcacgtcgg gcgagggcga ggtcatcgcc gacctcgatt tcaagctgat cgacaagcgc     840
   aagcagacga tggactcgcg cggccactac aaccgccccg aactgctcag cctgctgatc     900
   gatcgaacgc cgacgtcgaa cgtccatgag cgcgccgcgc acccgaaggt cgaggcgtca     960
   caaacggctg gcgacacgga gcggacccgc gaggtcctgt aa                      1002
<210> 234
   <211> 333
   <212> PRT
   <213> Unknown
<220>
   <223> Obtained from an environmental sample
<400> 234
   Val Ser Thr Ile Val Lys Ala Ala Ala Val Gln Ile Ser Pro Val Leu
   1               5                   10                  15
   Tyr Ser Arg Glu Gly Thr Val Glu Arg Val Val Lys Lys Ile Arg Glu
               20                  25                  30
   Leu Gly Glu Lys Gly Val Gln Phe Ala Thr Phe Pro Glu Thr Val Ile
           35                  40                  45
   Pro Tyr Tyr Pro Tyr Phe Ser Phe Val Gln Thr Pro Leu Gln Ile Leu
       50                  55                  60
   Ala Gly Pro Glu His Leu Lys Leu Leu Asp Gln Ser Val Thr Val Pro
   65                  70                  75                  80
   Ser Pro Ala Thr Asp Ala Ile Gly Gln Ala Ala Arg Gln Ala Gly Met
                   85                  90                  95
   Val Val Ser Ile Gly Val Asn Glu Arg Asp Gly Gly Thr Leu Tyr Asn
               100                 105                 110
   Thr Gln Leu Leu Phe Asp Ala Asp Gly Ala Leu Ile Gln Arg Arg Arg
           115                 120                 125
   Lys Ile Lys Pro Thr His Tyr Glu Arg Met Ile Trp Gly Glu Gly Asp
       130                 135                 140
   Gly Ser Gly Leu Arg Ala Val Asp Ser Gln Val Gly Arg Ile Gly Gln
   145                 150                 155                 160
   Leu Ala Cys Trp Glu His Asn Asn Pro Leu Ala Arg Tyr Ala Met Met
                   165                 170                 175
   Ala Asp Gly Glu Gln Ile His Ser Ala Met Tyr Pro Gly Ser Met Phe
               180                 185                 190
   Gly Asp Pro Phe Ala Gln Lys Thr Glu Ile Asn Ile Arg Gln His Ala
           195                 200                 205
   Leu Glu Ser Gly Cys Phe Val Val Cys Ser Thr Ala Trp Leu Asp Ala
       210                 215                 220
   Asp Gln Gln Ala Gln Ile Met Gln Asp Thr Gly Cys Ala Ile Gly Pro
   225                 230                 235                 240
   Ile Ser Gly Gly Cys Leu Thr Ala Ile Val Ala Pro Asp Gly Thr Phe
                   245                 250                 255
   Leu Gly Glu Pro Leu Thr Ser Gly Glu Gly Glu Val Ile Ala Asp Leu
               260                 265                 270
   Asp Phe Lys Leu Ile Asp Lys Arg Lys Gln Thr Met Asp Ser Arg Gly
           275                 280                 285
   His Tyr Asn Arg Pro Glu Leu Leu Ser Leu Leu Ile Asp Arg Thr Pro
       290                 295                 300
   Thr Ser Asn Val His Glu Arg Ala Ala His Pro Lys Val Glu Ala Ser
   305                 310                 315                 320
   Gln Thr Ala Gly Asp Thr Glu Arg Thr Arg Glu Val Leu
                   325                 330
<210> 235
   <211> 993
   <212> DNA
   <213> Unknown
<220>
   <223> Obtained from an environmental sample
<400> 235
   atgaaaattg ttaaagcggc agcagttcag ctgagccccg tcctctatag ccgcgagggg     60
   acggtcgaaa gagtagtgcg gaagattcat caacttggtc aacagggagt gcagtttgcc    120
   accttcccgg aaacagtggt gccttactac ccgtattttt cgatcgtgca gtccgggtat    180
   caaatccttc gcggcggtga gttcgtgaag ctgctcgatc agtcagtgac ggtgccatct    240
   cttgccaccg aagcgatcgc cgaggcctgc aggcaggcgg gcgtcgttgt ctccatcggc    300
   gtcaatgagc gtgacggcgg aactatatac aatgcgcagc ttctgtttga ttcggacggc    360
   acattgattc agaggcgacg caagatcacg cccacccact acgagcgcat gatctggggc    420
   cagggcgatg gctcgggtct gcgggctgtg gacagcaagg tggcacgtat tggtcaactg    480
   gcgtgctttg agcattacaa ccccctcgca cgatacgcga tgatcgccga tggcgagcag    540
   atccactctg caatgtttcc cggttccatg ttcggcgatg gtttcgcgga gaggaccgag    600
   atcgcggtca ggcagcatgc gcaggagtcc ggatgctttg tagtttgtgc tacggcgtgg    660
   ctggatgccg accagcaggc tcaaattgcc gcggacacag gcatcaccga cctgggaccg    720
   atctccggcg gttgcttcac tgcgatcatt gcacctgatg ggagcctgct gggtcaacca    780
   atccgctcgg gcgaaggtga cgtcattgtc gatctcgatt tcactctgat cgacaggcgg    840
   aagcatgttg tggactcgag aggtcactac agccggccgg aattgctaag cctgctgatc    900
   gaccgtactc ccacagcgca cgttcacgaa cgggccgcgc actctcactt ggccgccgag    960
   caatgcttgg aggatcttaa cgcgcttgct taa                                 993
<210> 236
   <211> 330
   <212> PRT
   <213> Unknown
<220>
   <223> Obtained from an environmental sample
<400> 236
   Met Lys Ile Val Lys Ala Ala Ala Val Gln Leu Ser Pro Val Leu Tyr
   1               5                   10                  15
   Ser Arg Glu Gly Thr Val Glu Arg Val Val Arg Lys Ile His Gln Leu
               20                  25                  30
   Gly Gln Gln Gly Val Gln Phe Ala Thr Phe Pro Glu Thr Val Val Pro
           35                  40                  45
   Tyr Tyr Pro Tyr Phe Ser Ile Val Gln Ser Gly Tyr Gln Ile Leu Arg
       50                  55                  60
   Gly Gly Glu Phe Val Lys Leu Leu Asp Gln Ser Val Thr Val Pro Ser
   65                  70                  75                  80
   Leu Ala Thr Glu Ala Ile Ala Glu Ala Cys Arg Gln Ala Gly Val Val
                   85                  90                  95
   Val Ser Ile Gly Val Asn Glu Arg Asp Gly Gly Thr Ile Tyr Asn Ala
               100                 105                 110
   Gln Leu Leu Phe Asp Ser Asp Gly Thr Leu Ile Gln Arg Arg Arg Lys
           115                 120                 125
   Ile Thr Pro Thr His Tyr Glu Arg Met Ile Trp Gly Gln Gly Asp Gly
       130                 135                 140
   Ser Gly Leu Arg Ala Val Asp Ser Lys Val Ala Arg Ile Gly Gln Leu
   145                 150                 155                 160
   Ala Cys Phe Glu His Tyr Asn Pro Leu Ala Arg Tyr Ala Met Ile Ala
                   165                 170                 175
   Asp Gly Glu Gln Ile His Ser Ala Met Phe Pro Gly Ser Met Phe Gly
               180                 185                 190
   Asp Gly Phe Ala Glu Arg Thr Glu Ile Ala Val Arg Gln His Ala Gln
           195                 200                 205
   Glu Ser Gly Cys Phe Val Val Cys Ala Thr Ala Trp Leu Asp Ala Asp
       210                 215                 220
   Gln Gln Ala Gln Ile Ala Ala Asp Thr Gly Ile Thr Asp Leu Gly Pro
   225                 230                 235                 240
   Ile Ser Gly Gly Cys Phe Thr Ala Ile Ile Ala Pro Asp Gly Ser Leu
                   245                 250                 255
   Leu Gly Gln Pro Ile Arg Ser Gly Glu Gly Asp Val Ile Val Asp Leu
               260                 265                 270
   Asp Phe Thr Leu Ile Asp Arg Arg Lys His Val Val Asp Ser Arg Gly
           275                 280                 285
   His Tyr Ser Arg Pro Glu Leu Leu Ser Leu Leu Ile Asp Arg Thr Pro
       290                 295                 300
   Thr Ala His Val His Glu Arg Ala Ala His Ser His Leu Ala Ala Glu
   305                 310                 315                 320
   Gln Cys Leu Glu Asp Leu Asn Ala Leu Ala
                   325                 330
<210> 237
   <211> 993
   <212> DNA
   <213> Unknown
<220>
   <223> Obtained from an environmental sample
<400> 237
   atgaaagtcg tcaaagccgc cgcagtgcag atcagtcctg ttctctacag ccgagaggca      60
   accgtcgcca aggtcgtgca gaagatccac gaactcggcc agaaaggcgt gcagttcgcc     120
   acctttccag agaccgtagt gccttactac ccgtactttt ccgccgtcca gacaggcatc     180
   gagcttctgt ccggcacgga gcatctccgg ctgctcgatc aggccgtgac ggtgccgtct     240
   gccgctaccg acgcgatcgg agaggcagcc cggaaggcag gcatggtggt gtcgatcggc     300
   gtcaatgaac gcgatggcgg caccttgtac aacacacagt tgctcttcga tgccgatggc     360
   accttgatcc agcgccgccg caagatcacg ccgacccact tcgaacgcat gatctggggc     420
   cagggggacg gttcgggcct gcgcgctgtc gacagcaagg tcggtcgcat tggccagttg     480
   gcctgcttcg agcacaacaa cccgctggcc cgctacgcgt tgattgccga cggcgagcag     540
   atccattccg ccatgtatcc gggttctgct tttggcgaag gatttgccca aaggatggaa     600
   atcaatatcc gccagcatgc gctggagtcg ggtgcgttcg tcgtcaacgc aacggcctgg     660
   ctggatgctg accagcaggc gcagatcatg aaggacaccg gctgcgggat tggcccgatc     720
   tcgggcggtt gcttcaccac gatcgtgtca cccgacggca tgctgatggc cgaacccctg     780
   cgctcgggcg agggtgaggt catcgtcgat ctcgacttca cgctgatcga ccgtcgcaag     840
   atgttgatgg actcggcggg ccactataac cgcccggaac tgctcagtct catgatcgac     900
   cgcaccccga ccgcgcacgt tcatgaacgc gctgcgcgtc cggtgtcggg cgttgagcag     960
   aacccggagg aacttcgcat cccggccgcg tga                                  993
<210> 238
   <211> 330
   <212> PRT
   <213> Unknown
<220>
   <223> Obtained from an environmental sample
<400> 238
   Met Lys Val Val Lys Ala Ala Ala Val Gln Ile Ser Pro Val Leu Tyr
   1               5                   10                  15
   Ser Arg Glu Ala Thr Val Ala Lys Val Val Gln Lys Ile His Glu Leu
               20                  25                  30
   Gly Gln Lys Gly Val Gln Phe Ala Thr Phe Pro Glu Thr Val Val Pro
           35                  40                  45
   Tyr Tyr Pro Tyr Phe Ser Ala Val Gln Thr Gly Ile Glu Leu Leu Ser
       50                  55                  60
   Gly Thr Glu His Leu Arg Leu Leu Asp Gln Ala Val Thr Val Pro Ser
   65                  70                  75                  80
   Ala Ala Thr Asp Ala Ile Gly Glu Ala Ala Arg Lys Ala Gly Met Val
                   85                  90                  95
   Val Ser Ile Gly Val Asn Glu Arg Asp Gly Gly Thr Leu Tyr Asn Thr
               100                 105                 110
   Gln Leu Leu Phe Asp Ala Asp Gly Thr Leu Ile Gln Arg Arg Arg Lys
           115                 120                 125
   Ile Thr Pro Thr His Phe Glu Arg Met Ile Trp Gly Gln Gly Asp Gly
       130                 135                 140
   Ser Gly Leu Arg Ala Val Asp Ser Lys Val Gly Arg Ile Gly Gln Leu
   145                 150                 155                 160
   Ala Cys Phe Glu His Asn Asn Pro Leu Ala Arg Tyr Ala Leu Ile Ala
                   165                 170                 175
   Asp Gly Glu Gln Ile His Ser Ala Met Tyr Pro Gly Ser Ala Phe Gly
               180                 185                 190
   Glu Gly Phe Ala Gln Arg Met Glu Ile Asn Ile Arg Gln His Ala Leu
           195                 200                 205
   Glu Ser Gly Ala Phe Val Val Asn Ala Thr Ala Trp Leu Asp Ala Asp
       210                 215                 220
   Gln Gln Ala Gln Ile Met Lys Asp Thr Gly Cys Gly Ile Gly Pro Ile
   225                 230                 235                 240
   Ser Gly Gly Cys Phe Thr Thr Ile Val Ser Pro Asp Gly Met Leu Met
                   245                 250                 255
   Ala Glu Pro Leu Arg Ser Gly Glu Gly Glu Val Ile Val Asp Leu Asp
               260                 265                 270
   Phe Thr Leu Ile Asp Arg Arg Lys Met Leu Met Asp Ser Ala Gly His
           275                 280                 285
   Tyr Asn Arg Pro Glu Leu Leu Ser Leu Met Ile Asp Arg Thr Pro Thr
       290                 295                 300
   Ala His Val His Glu Arg Ala Ala Arg Pro Val Ser Gly Val Glu Gln
   305                 310                 315                 320
   Asn Pro Glu Glu Leu Arg Ile Pro Ala Ala
                   325                 330
<210> 239
   <211> 969
   <212> DNA
   <213> Unknown
<220>
   <223> Obtained from an environmental sample
<400> 239
   atgtccaatg agaataagta tgacacattt aaagttgctg cagtccaggc cacacctgtg     60
   tttcttgatc gtgaagcaac catcgacaaa gcttgcgagt tgattgctac tgcaggtcgt    120
   gaaggtgctc gcctgattgt ctttccagaa gcgttcatcc catcctatcc cgagtgggta    180
   tggggtattc cctctggtga gcaaggttta ctcaacgaac tttattcaga gttgctcacc    240
   aatgcggtta ccatacccag cgacgcgact gacagattgt gcgaggcggc aaagcttgcg    300
   aatgcctatg tagtgatggg aatgagcgaa cggaatgtcg aagcgagtgg tgcaagcctg    360
   tataacacga tgttgtatat agatgcacag ggggagattt tagggaaaca tcggaagctg    420
   gtgccaacgg gtggtgaacg cctggtatgg gcgcaaggtg atggcagcac gctgcaggtc    480
   tacgatactc cattgggaaa acttggtggg ttaatttgct gggaaaatta tatgccgctg    540
   gcacgctata cgatgtatgc ctggggaaca caaatctatg ttgcagcaac gtgggattgc    600
   ggccaaccct ggctctcaac gatacggcat attgctaaag aaggcagggt atacgtggtt    660
   ggttgctgta tcgcgatgcg taaagatgat attccagatc gttactctat gaagcagaaa    720
   tattatgctg aaatggatga atggataaat gttggggata gcgcgattgt caatcccgaa    780
   ggacatttta ttgcagggcc tgtgcgcaag caagaagaaa ttctctatgc ggagattgat    840
   ccacgtatga tgcaaggccc gaagtggatg cttgacgtgg cgggacatta tgcaagacca    900
   gatgtgttcc agttgacggt gcatacggat gtgaggcaga tgatacgggt ggaagatgat    960
   tctcaatga                                                            969
<210> 240
   <211> 322
   <212> PRT
   <213> unknown
<220>
   <223> Obtained from an environmental sample
<400> 240
   Met Ser Asn Glu Asn Lys Tyr Asp Thr Phe Lys Val Ala Ala Val Gln
   1               5                   10                  15
   Ala Thr Pro Val Phe Leu Asp Arg Glu Ala Thr Ile Asp Lys Ala Cys
               20                  25                  30
   Glu Leu Ile Ala Thr Ala Gly Arg Glu Gly Ala Arg Leu Ile Val Phe
           35                  40                  45
   Pro Glu Ala Phe Ile Pro Ser Tyr Pro Glu Trp Val Trp Gly Ile Pro
       50                  55                  60
   Ser Gly Glu Gln Gly Leu Leu Asn Glu Leu Tyr Ser Glu Leu Leu Thr
   65                  70                  75                  80
   Asn Ala Val Thr Ile Pro Ser Asp Ala Thr Asp Arg Leu Cys Glu Ala
                   85                  90                  95
   Ala Lys Leu Ala Asn Ala Tyr Val Val Met Gly Met Ser Glu Arg Asn
               100                 105                 110
   Val Glu Ala Ser Gly Ala Ser Leu Tyr Asn Thr Met Leu Tyr Ile Asp
           115                 120                 125
   Ala Gln Gly Glu Ile Leu Gly Lys His Arg Lys Leu Val Pro Thr Gly
       130                 135                 140
   Gly Glu Arg Leu Val Trp Ala Gln Gly Asp Gly Ser Thr Leu Gln Val
   145                 150                 155                 160
   Tyr Asp Thr Pro Leu Gly Lys Leu Gly Gly Leu Ile Cys Trp Glu Asn
                   165                 170                 175
   Tyr Met Pro Leu Ala Arg Tyr Thr Met Tyr Ala Trp Gly Thr Gln Ile
               180                 185                 190
   Tyr Val Ala Ala Thr Trp Asp Cys Gly Gln Pro Trp Leu Ser Thr Ile
           195                 200                 205
   Arg His Ile Ala Lys Glu Gly Arg Val Tyr Val Val Gly Cys Cys Ile
       210                 215                 220
   Ala Met Arg Lys Asp Asp Ile Pro Asp Arg Tyr Ser Met Lys Gln Lys
   225                 230                 235                 240
   Tyr Tyr Ala Glu Met Asp Glu Trp Ile Asn Val Gly Asp Ser Ala Ile
                   245                 250                 255
   Val Asn Pro Glu Gly His Phe Ile Ala Gly Pro Val Arg Lys Gln Glu
               260                 265                 270
   Glu Ile Leu Tyr Ala Glu Ile Asp Pro Arg Met Met Gln Gly Pro Lys
           275                 280                 285
   Trp Met Leu Asp Val Ala Gly His Tyr Ala Arg Pro Asp Val Phe Gln
       290                 295                 300
   Leu Thr Val His Thr Asp Val Arg Gln Met Ile Arg Val Glu Asp Asp
   305                 310                 315                 320
   Ser Gln
<210> 241
   <211> 972
   <212> DNA
   <213> Unknown
<220>
   <223> Obtained from an environmental sample
<400> 241
   atgatatcca acgagcataa caatactcca ttcaaggttg ctgctgtgca ggctacacct      60
   gtgtttcttg atcgcgaagc aacgatcgat aaagcgtgtg aactgatcgc tactgccggt     120
   catgaaggcg ctcgtttgat tgtttttcca gaagcgttca tcccatccta tcccgagtgg     180
   gtatggggaa ttccctctgg cgagcaaggt ttgctcaacg atctgtatgc agagttactc     240
   accaattcag ttacgatacc cagcaacgca actgacaggc tttgtagagc cgcgaagctt     300
   gctaatgcct acgtggtgat ggggatgagc gaacggaata tcgaagcgag cggcgcaagc     360
   ctgtacaata cgatgttata tatagatgca cagggtgaga ttttgggcaa acatcgaaag     420
   ctggtgccaa cgggcggaga acgcctggta tgggcacaag gagatggaag cacgctgcag     480
   gtttacgata cacctctagg aaagcttggt ggtttaattt gctgggaaaa ttatatgccg     540
   ctggcacgct acgctatgta tgcctgggga actcaaatct acgtcgcggc aacgtgggat     600
   cgcggccaac cctggctctc aacgatacgg catatcgcta aagagggcag ggtatacgta     660
   atcggttgct gtatcgcgat gcgtaaagac gatattccag ataggtactc catgaagcag     720
   aagtattatg cggagatgga tgaatggatc aacgtaggtg acagcgcgat tgtcaatcct     780
   gagggggact tcattgcggg gcctgtgagc aagcaggagg aaattctcta tgcggagatt     840
   gatccgcgga tggtgcaagg tccgaagtgg atgctggatg tggcggggca ttacgcgagg     900
   cctgatgtgt tcgagttgac ggtgcatacg gatgtgaggg agatgatgcg ggtggagcat     960
   gattatcaat ga                                                         972
<210> 242
   <211> 323
   <212> PRT
   <213> Unknown
<220>
   <223> Obtained from an environmental sample
<400> 242
   Met Ile Ser Asn Glu His Asn Asn Thr Pro Phe Lys Val Ala Ala Val
   1               5                   10                  15
   Gln Ala Thr Pro Val Phe Leu Asp Arg Glu Ala Thr Ile Asp Lys Ala
               20                  25                  30
   Cys Glu Leu Ile Ala Thr Ala Gly His Glu Gly Ala Arg Leu Ile Val
           35                  40                  45
   Phe Pro Glu Ala Phe Ile Pro Ser Tyr Pro Glu Trp Val Trp Gly Ile
       50                  55                  60
   Pro Ser Gly Glu Gln Gly Leu Leu Asn Asp Leu Tyr Ala Glu Leu Leu
   65                  70                  75                  80
   Thr Asn Ser Val Thr Ile Pro Ser Asn Ala Thr Asp Arg Leu Cys Arg
                   85                  90                  95
   Ala Ala Lys Leu Ala Asn Ala Tyr Val Val Met Gly Met Ser Glu Arg
               100                 105                 110
   Asn Ile Glu Ala Ser Gly Ala Ser Leu Tyr Asn Thr Met Leu Tyr Ile
           115                 120                 125
   Asp Ala Gln Gly Glu Ile Leu Gly Lys His Arg Lys Leu Val Pro Thr
       130                 135                 140
   Gly Gly Glu Arg Leu Val Trp Ala Gln Gly Asp Gly Ser Thr Leu Gln
   145                 150                 155                 160
   Val Tyr Asp Thr Pro Leu Gly Lys Leu Gly Gly Leu Ile Cys Trp Glu
                   165                 170                 175
   Asn Tyr Met Pro Leu Ala Arg Tyr Ala Met Tyr Ala Trp Gly Thr Gln
               180                 185                 190
   Ile Tyr Val Ala Ala Thr Trp Asp Arg Gly Gln Pro Trp Leu Ser Thr
           195                 200                 205
   Ile Arg His Ile Ala Lys Glu Gly Arg Val Tyr Val Ile Gly Cys Cys
       210                 215                 220
   Ile Ala Met Arg Lys Asp Asp Ile Pro Asp Arg Tyr Ser Met Lys Gln
   225                 230                 235                 240
   Lys Tyr Tyr Ala Glu Met Asp Glu Trp Ile Asn Val Gly Asp Ser Ala
                   245                 250                 255
   Ile Val Asn Pro Glu Gly Asp Phe Ile Ala Gly Pro Val Ser Lys Gln
               260                 265                 270
   Glu Glu Ile Leu Tyr Ala Glu Ile Asp Pro Arg Met Val Gln Gly Pro
           275                 280                 285
   Lys Trp Met Leu Asp Val Ala Gly His Tyr Ala Arg Pro Asp Val Phe
       290                 295                 300
   Glu Leu Thr Val His Thr Asp Val Arg Glu Met Met Arg Val Glu His
   305                 310                 315                 320
   Asp Tyr Gln
<210> 243
   <211> 999
   <212> DNA
   <213> Unknown
<220>
   <223> Obtained from an environmental sample
<400> 243
   atgaagcaaa ctcgagtagc gatcattcag gcggaacctg tatatctcaa tttgcaagcg      60
   agtgttgcga gggctatcga tcttgccgga cgcgccgcga agcaaggagc gcgtctgata     120
   gtgtttggag agacctggtt gccgggttat cccgcgtggc tggattactg tcccggcatg     180
   gcgttctggg atcaccggcc gacaaaagaa gtgtttgccc ggacccgcga gaacagtgtt     240
   gtaattccgg gaaaggaaat cgaacagctc tgtaaaactg cggcggagct gggagttgta     300
   atttcgatcg gtgtaaacga aaaaattctg gaaggccccg gaaacggcac gctctacaat     360
   tctcttttgc tgattgatga atcaggaaaa ctggccggcc atcaccgcaa actggttccg     420
   acttatacgg aacggatggt gtggggaatg ggtgatggag gtggaatgga agccatatcg     480
   actgcagccg gcagggttgg cggattgatt tgctgggagc actggatgcc attgagccgg     540
   caggtgctgc acatgtcggg tgaggaaatt catgtggcag tgtggcccac ggttcatgag     600
   gtgcaccagc ttgcatcacg ccattatgca tttgaagggc gttgttttgt gctcgcagcc     660
   ggattgttga tgaaggtccg ggatattcct ccggagctgg aattgccttc tcagatgtcg     720
   cgtgaatccg aagactggct tctgcgcggc gggagcgccg tcattggtcc ggatggaaag     780
   tacattgtgg agccgttgtt tgatcgagag gcgattctca cagccgatct tgaattagcc     840
   gcatgcgatc gtgaaaaaat gacgctggac gtaacgggac attattcccg ccccgatctt     900
   tttcacctgg aattcaggaa acagcaatcc ggccatattg cgggagcagg aacgatcagc     960
   cggcaaaaat cagcgccgga ccgcgcggac gatcactaa                           999
<210> 244
   <211> 332
   <212> PRT
   <213> Unknown
<220>
   <223> Obtained from an environmental sample
<400> 244
   Met Lys Gln Thr Arg Val Ala Ile Ile Gln Ala Glu Pro Val Tyr Leu
   1               5                   10                  15
   Asn Leu Gln Ala Ser Val Ala Arg Ala Ile Asp Leu Ala Gly Arg Ala
               20                  25                  30
   Ala Lys Gln Gly Ala Arg Leu Ile Val Phe Gly Glu Thr Trp Leu Pro
           35                  40                  45
   Gly Tyr Pro Ala Trp Leu Asp Tyr Cys Pro Gly Met Ala Phe Trp Asp
       50                  55                  60
   His Arg Pro Thr Lys Glu Val Phe Ala Arg Thr Arg Glu Asn Ser Val
   65                  70                  75                  80
   Val Ile Pro Gly Lys Glu Ile Glu Gln Leu Cys Lys Thr Ala Ala Glu
                   85                  90                  95
   Leu Gly Val Val Ile Ser Ile Gly Val Asn Glu Lys Ile Leu Glu Gly
               100                 105                 110
   Pro Gly Asn Gly Thr Leu Tyr Asn Ser Leu Leu Leu Ile Asp Glu Ser
           115                 120                 125
   Gly Lys Leu Ala Gly His His Arg Lys Leu Val Pro Thr Tyr Thr Glu
       130                 135                 140
   Arg Met Val Trp Gly Met Gly Asp Gly Gly Gly Met Glu Ala Ile Ser
   145                 150                 155                 160
   Thr Ala Ala Gly Arg Val Gly Gly Leu Ile Cys Trp Glu His Trp Met
                   165                 170                 175
   Pro Leu Ser Arg Gln Val Leu His Met Ser Gly Glu Glu Ile His Val
               180                 185                 190
   Ala Val Trp Pro Thr Val His Glu Val His Gln Leu Ala Ser Arg His
           195                 200                 205
   Tyr Ala Phe Glu Gly Arg Cys Phe Val Leu Ala Ala Gly Leu Leu Met
       210                 215                 220
   Lys Val Arg Asp Ile Pro Pro Glu Leu Glu Leu Pro Ser Gln Met Ser
   225                 230                 235                 240
   Arg Glu Ser Glu Asp Trp Leu Leu Arg Gly Gly Ser Ala Val Ile Gly
                   245                 250                 255
   Pro Asp Gly Lys Tyr Ile Val Glu Pro Leu Phe Asp Arg Glu Ala Ile
               260                 265                 270
   Leu Thr Ala Asp Leu Glu Leu Ala Ala Cys Asp Arg Glu Lys Met Thr
           275                 280                 285
   Leu Asp Val Thr Gly His Tyr Ser Arg Pro Asp Leu Phe His Leu Glu
       290                 295                 300
   Phe Arg Lys Gln Gln Ser Gly His Ile Ala Gly Ala Gly Thr Ile Ser
   305                 310                 315                 320
   Arg Gln Lys Ser Ala Pro Asp Arg Ala Asp Asp His
                   325                 330
<210> 245
   <211> 999
   <212> DNA
   <213> unknown
<220>
   <223> Obtained from an environmental sample
<400> 245
   atgggtgaga atgccaactt caccgtcgca gctgtccagg caacgccggt cttcttagac      60
   cgggatgcga cggtcgagaa ggcttgcgag ctcatcgctg aagccgggcg aaacggagcg     120
   cgcctggccg tttttcccga ggcgtttgtg ccggcttacc cggactgggt ctgggctgtt     180
   ccgccgggcg attcaaggct gctgcacgag ctctacggtg agctgatcca gaactctgtc     240
   acgattccca gcgagtcgac ggagaaactc tgccgggccg cccgcggggc caaagtctgc     300
   gtggcgatcg gcatcaacga gaggaatgcg gaggcaagcg ggggtagcct ctacaacagc     360
   ctcctgtaca tcagcccgga cggccaggtc ctcgggaagc accgcaagct cgttcccacc     420
   ggagcggagc ggcttgtctg ggcgcagggc gacggcagca ctatcgacgt gtttgagttg     480
   cctttctgtc gtttgggtgg cctcatctgt tgggagaact acatgccgct ggcccgttat     540
   gcgatgtacg cctggggcac gcaggtctac gtcgcggcaa cgtgggacca cggcgaacct     600
   tggctctcaa ccttgaggca tatcgccagg gaggggcgtg catatgtcat tggcgtttgc     660
   atgccgatgc gcatgagcga catcccggac cgatacgagt tcaagcgcaa gtactatggc     720
   gggcgcgact ggatcaatac tggtgacagc gccatcgtgg gtccggacgg aaacttcatc     780
   gccggccccc tgagcgagcg cgaagagatc ctgtacgccg atatagacct gaatcggctt     840
   gcgaactcga agtggatgct ggacgtcgcc gggcactatg cacggccgga cgtcttccag     900
   ttgaccgtta accgcgagcc gaacccgatg atctctgagg atgggcacaa gacggttccc     960
   acgctaccga aacgtgcggg gaagagtagg acgagatga                             999
<210> 246
   <211> 332
   <212> PRT
   <213> Unknown
<220>
   <223> Obtained from an environmental sample
<400> 246
   Met Gly Glu Asn Ala Asn Phe Thr Val Ala Ala Val Gln Ala Thr Pro
   1               5                   10                  15
   Val Phe Leu Asp Arg Asp Ala Thr Val Glu Lys Ala Cys Glu Leu Ile
               20                  25                  30
   Ala Glu Ala Gly Arg Asn Gly Ala Arg Leu Ala Val Phe Pro Glu Ala
           35                  40                  45
   Phe Val Pro Ala Tyr Pro Asp Trp Val Trp Ala Val Pro Pro Gly Asp
       50                  55                  60
   Ser Arg Leu Leu His Glu Leu Tyr Gly Glu Leu Ile Gln Asn Ser Val
   65                  70                  75                  80
   Thr Ile Pro Ser Glu Ser Thr Glu Lys Leu Cys Arg Ala Ala Arg Gly
                   85                  90                  95
   Ala Lys Val Cys Val Ala Ile Gly Ile Asn Glu Arg Asn Ala Glu Ala
               100                 105                 110
   Ser Gly Gly Ser Leu Tyr Asn Ser Leu Leu Tyr Ile Ser Pro Asp Gly
           115                 120                 125
   Gln Val Leu Gly Lys His Arg Lys Leu Val Pro Thr Gly Ala Glu Arg
       130                 135                 140
   Leu Val Trp Ala Gln Gly Asp Gly Ser Thr Ile Asp Val Phe Glu Leu
   145                 150                 155                 160
   Pro Phe Cys Arg Leu Gly Gly Leu Ile Cys Trp Glu Asn Tyr Met Pro
                   165                 170                 175
   Leu Ala Arg Tyr Ala Met Tyr Ala Trp Gly Thr Gln Val Tyr Val Ala
               180                 185                 190
   Ala Thr Trp Asp His Gly Glu Pro Trp Leu Ser Thr Leu Arg His Ile
           195                 200                 205
   Ala Arg Glu Gly Arg Ala Tyr Val Ile Gly Val Cys Met Pro Met Arg
       210                 215                 220
   Met Ser Asp Ile Pro Asp Arg Tyr Glu Phe Lys Arg Lys Tyr Tyr Gly
   225                 230                 235                 240
   Gly Arg Asp Trp Ile Asn Thr Gly Asp Ser Ala Ile Val Gly Pro Asp
                   245                 250                 255
   Gly Asn Phe Ile Ala Gly Pro Leu Ser Glu Arg Glu Glu Ile Leu Tyr
               260                 265                 270
   Ala Asp Ile Asp Leu Asn Arg Leu Ala Asn Ser Lys Trp Met Leu Asp
           275                 280                 285
   Val Ala Gly His Tyr Ala Arg Pro Asp Val Phe Gln Leu Thr Val Asn
       290                 295                 300
   Arg Glu Pro Asn Pro Met Ile Ser Glu Asp Gly His Lys Thr Val Pro
   305                 310                 315                 320
   Thr Leu Pro Lys Arg Ala Gly Lys Ser Arg Thr Arg
                   325                 330
<210> 247
   <211> 990
   <212> DNA
   <213> Unknown
<220>
   <223> Obtained from an environmental sample
<400> 247
   atgccgaccc ccaaggaaaa gtttagaatc gccgccgttc aggcttgccc cgtttttctg      60
   gaccgggggg agacggtaaa gaaggcctgc cggctggcgg ccgaggccgg gggccagggc     120
   gcccggctga tcgtgtttcc ggagtccttc atccccgctt acccggactg ggtgtgggcc     180
   gttccgccgg ggagggagaa gctcctgaat gaaatgtacg ccgaattcct ggccggcgcg     240
   gtggaagtcc cggggccggt gacggaggaa ttgggccggg cggcggaaag ggccggcgct     300
   tacctggtca tgggggtcac cgagcgggac accgaggcca gcggggcaag cctgtacaac     360
   accctcctct atttcggtcc ccagggaagc ctgctgggaa aacaccgcaa actggtgccc     420
   acgggagggg aacggaccgt ctgggcccgg ggggacggca gcacgctgca ggtgtacgat     480
   acccccctgg gaaagatcgg cggcctgatc tgctgggaga actacatgcc cctggcccgc     540
   tacgccatgt atgcgtgggg cactcagatt tacctggccc ccacctggga ccggggggaa 600
   ccctggcttt caaccctgcg gcacatcgcc aaggaagggc gggtttacgt ggtggggtgc 660
   tgcatggcta tgcaaaaagg ggacatcccg gatcgcttcg aatacaagca aaaatactat 720
   cccgcagccc gggagtggat caacacgggc gacagcgcca tcctgaaccc ggagggggaa 780
   ttcatcgccg ggccggcggg aaagaaggaa gagatcctgt acgctgaaat agacccccgg 840
   cagatggggg ggcccaagtg gatgctggac gtggccggcc attacgcccg gccggatgtc 900
   ttcgaactga tcgttcaccg ggaggcccgg ccgatgatcc gggtgacgga agcgccgtct 960
   ccgggagaga aagaaacggg tgaaggatag 990
<210> 248
   <211> 329
   <212> PRT
   <213> Unknown
<220>
   <223> Obtained from an environmental sample
<400> 248
   Met Pro Thr Pro Lys Glu Lys Phe Arg Ile Ala Ala Val Gln Ala Cys
   1 5 10 15
   Pro Val Phe Leu Asp Arg Gly Glu Thr Val Lys Lys Ala Cys Arg Leu
   20 25 30
   Ala Ala Glu Ala Gly Gly Gln Gly Ala Arg Leu Ile Val Phe Pro Glu
   35 40 45
   Ser Phe Ile Pro Ala Tyr Pro Asp Trp Val Trp Ala Val Pro Pro Gly
   50 55 60
   Arg Glu Lys Leu Leu Asn Glu Met Tyr Ala Glu Phe Leu Ala Gly Ala
   65 70 75 80
   Val Glu Val Pro Gly Pro Val Thr Glu Glu Leu Gly Arg Ala Ala Glu
   85 90 95
   Arg Ala Gly Ala Tyr Leu Val Met Gly Val Thr Glu Arg Asp Thr Glu
   100 105 110
   Ala Ser Gly Ala Ser Leu Tyr Asn Thr Leu Leu Tyr Phe Gly Pro Gln
   115 120 125
   Gly Ser Leu Leu Gly Lys His Arg Lys Leu Val Pro Thr Gly Gly Glu
   130 135 140
   Arg Thr Val Trp Ala Arg Gly Asp Gly Ser Thr Leu Gln Val Tyr Asp
   145 150 155 160
   Thr Pro Leu Gly Lys Ile Gly Gly Leu Ile Cys Trp Glu Asn Tyr Met
   165 170 175
   Pro Leu Ala Arg Tyr Ala Met Tyr Ala Trp Gly Thr Gln Ile Tyr Leu
   180 185 190
   Ala Pro Thr Trp Asp Arg Gly Glu Pro Trp Leu Ser Thr Leu Arg His
   195 200 205
   Ile Ala Lys Glu Gly Arg Val Tyr Val Val Gly Cys Cys Met Ala Met
   210 215 220
   Gln Lys Gly Asp Ile Pro Asp Arg Phe Glu Tyr Lys Gln Lys Tyr Tyr
   225 230 235 240
   Pro Ala Ala Arg Glu Trp Ile Asn Thr Gly Asp Ser Ala Ile Leu Asn
   245 250 255
   Pro Glu Gly Glu Phe Ile Ala Gly Pro Ala Gly Lys Lys Glu Glu Ile
   260 265 270
   Leu Tyr Ala Glu Ile Asp Pro Arg Gln Met Gly Gly Pro Lys Trp Met
   275 280 285
   Leu Asp Val Ala Gly His Tyr Ala Arg Pro Asp Val Phe Glu Leu Ile
   290 295 300
   Val His Arg Glu Ala Arg Pro Met Ile Arg Val Thr Glu Ala Pro Ser
   305 310 315 320
   Pro Gly Glu Lys Glu Thr Gly Glu Gly
   325
<210> 249
   <211> 1017
   <212> DNA
   <213> Unknown
<220>
   <223> Obtained from an environmental sample
<400> 249
   atgggcattc aacatccgaa atatcgcgtc gcggtggtgc aggcggcacc ggcctggctc      60
   gacctcgagg cgtcggtcag caagagcatc gcgctgatag aggaggccgc cgccaagggc     120
   gccaagctga tcgcgttccc cgaggccttc atccccggct atccctggta catctggctg     180
   gactcgccgg cctgggcgat cggccgcggc ttcgtgcagc gctatttcga caattcgctc     240
   agctatgaca gcccgcaggc ggagcgcctg aggctcgcag tgaagaaggc cggcatgacc     300
   gcagtgctcg gcctgtccga gcgcgacggc ggcagcctct atctcgcgca atggttgatc     360
   ggacccgacg gcgagaccat cgcaaagcgg cgcaagctgc ggccgaccca tgccgagcgc     420
   accgtctacg gcgagggcga cggcagcgac cttgcggtgc atgaccgccc cggcatcggc     480
   cggctcggtg cgctgtgctg ctgggagcat ctgcagccgc tgtcgaaata cgcgatgtac     540
   gcccagaacg agcaggtgca tgtcgcggcc tggccgagct tctcgctgta cgatccgttc     600
   gcgccggcgc tcggctggga ggtcaacaat gcggcctcgc gcgtctatgc cgtcgagggc     660
   tcctgcttcg tgctggcgcc ctgcgccacc gtctcgcagg cgatgatcga cgagctctgc     720
   gaccgcgacg acaagcatgc gctgctgcat gttggcggcg gccatgccgc gatcttcggc     780
   cccgacggca gcgcgatcgc ggacaagctt ccgtccgacc aggagggcct cctgttcgcc     840
   gacatcgatc tcggcgcgat cgggatcgcg aagaatgccg ctgatccggc cgggcactat     900
   tcgcgcccgg acgtgacgcg gctgctgctc aacaagaagc cctcgaagcg cgtcgagcac     960
   ttcgcgctgc cgctcgacac gctcgcgggc gaggagatcg acgcggccgc aagctaa       1017
<210> 250
   <211> 338
   <212> PRT
   <213> Unknown
<220>
   <223> Obtained from an environmental sample
<400> 250
   Met Gly Ile Gln His Pro Lys Tyr Arg Val Ala Val Val Gln Ala Ala
   1 5 10 15
   Pro Ala Trp Leu Asp Leu Glu Ala Ser Val Ser Lys Ser Ile Ala Leu
   20 25 30
   Ile Glu Glu Ala Ala Ala Lys Gly Ala Lys Leu Ile Ala Phe Pro Glu
   35 40 45
   Ala Phe Ile Pro Gly Tyr Pro Trp Tyr Ile Trp Leu Asp Ser Pro Ala
   50 55 60
   Trp Ala Ile Gly Arg Gly Phe Val Gln Arg Tyr Phe Asp Asn Ser Leu
   65 70 75 80
   Ser Tyr Asp Ser Pro Gln Ala Glu Arg Leu Arg Leu Ala Val Lys Lys
   85 90 95
   Ala Gly Met Thr Ala Val Leu Gly Leu Ser Glu Arg Asp Gly Gly Ser
   100 105 110
   Leu Tyr Leu Ala Gln Trp Leu Ile Gly Pro Asp Gly Glu Thr Ile Ala
   115 120 125
   Lys Arg Arg Lys Leu Arg Pro Thr His Ala Glu Arg Thr Val Tyr Gly
   130 135 140
   Glu Gly Asp Gly Ser Asp Leu Ala Val His Asp Arg Pro Gly Ile Gly
   145 150 155 160
   Arg Leu Gly Ala Leu Cys Cys Trp Glu His Leu Gln Pro Leu Ser Lys
   165 170 175
   Tyr Ala Met Tyr Ala Gln Asn Glu Gln Val His Val Ala Ala Trp Pro
   180 185 190
   Ser Phe Ser Leu Tyr Asp Pro Phe Ala Pro Ala Leu Gly Trp Glu Val
   195 200 205
   Asn Asn Ala Ala Ser Arg Val Tyr Ala Val Glu Gly Ser Cys Phe Val
   210 215 220
   Leu Ala Pro Cys Ala Thr Val Ser Gln Ala Met Ile Asp Glu Leu Cys
   225 230 235 240
   Asp Arg Asp Asp Lys His Ala Leu Leu His Val Gly Gly Gly His Ala
   245 250 255
   Ala Ile Phe Gly Pro Asp Gly Ser Ala Ile Ala Asp Lys Leu Pro Ser
   260 265 270
   Asp Gln Glu Gly Leu Leu Phe Ala Asp Ile Asp Leu Gly Ala Ile Gly
   275 280 285
   Ile Ala Lys Asn Ala Ala Asp Pro Ala Gly His Tyr Ser Arg Pro Asp
   290 295 300
   Val Thr Arg Leu Leu Leu Asn Lys Lys Pro Ser Lys Arg Val Glu His
   305 310 315 320
   Phe Ala Leu Pro Leu Asp Thr Leu Ala Gly Glu Glu Ile Asp Ala Ala
   325 330 335
   Ala Ser
<210> 251
   <211> 978
   <212> DNA
   <213> Unknown
<220>
   <223> Obtained from an environmental sample
<400> 251
   gtgaccatcg tgagagctgc cgccgtgcag atcagtcccg tgctctacag ccgggaagcc      60
   accgtagaaa aagtcgttcg caagatccgc gaactgggaa gaaacggcgt gcagttcgcc     120
   accttcccgg aaaccctggt gccctactac ccgtacttcg cggccgtgca gacgggcatc     180
   gaactgctgt ccggcaagga gcacctgcga ctgctggaac aatccgtaac ggttccctcg     240
   cccgccaccg atgccattgc ccaggcggca cgcgaagccg gcatggtggt gtccatcggt     300
   gtcaacgagc gcgacggagg caccatctac aacacgcagc tgctgtttga cgccgacggc     360
   acgctggtac agcgccgccg caagatcacg ccgacgcatt tcgagcgcat ggtctggggc     420
   cagggcgacg gctcgggcct gcgagccgtg gacaccaagg ccggccgcat cggtcagctc     480
   gcctgcttcg agcacaacaa cccgctggcg cgctacgcca tgatcgccga cggtgagcag     540
   atccattcgg ccatgtaccc gggctctgcc ttcggcgagg gcttcgcgca gcgcatggaa     600
   atcaacatac gccagcacgc cctggagtct ggctgcttcg tggtgaatgc gaccgcgtgg     660
   ctggatgccg accagcaggc gcagatcatg aaggatacgg gctgcggcat cggcccgatc     720
   tccggcggct gcttcacgac catcgtcacg ccggacggca tgctgatcgg tgaacccctc     780
   cgcgaaggcg aaggcgaagt catcgccgac ctcgatttca ccctgatcga ccggcgcaag     840
   ctgctggtgg actcggtggg ccactacaac cgtccggagc tgctgagcct gctgatcgat     900
   cgcacccctg cggcgaactt ccatgagcgc aatgcgcttc cgtccgtcaa caccgccagc     960
   agcctcgaaa tcgtctga                                                   978
<210> 252
   <211> 325
   <212> PRT
   <213> Unknown
<220> -
   <223> Obtained from an environmental sample
<400> 252
   Val Thr Ile Val Arg Ala Ala Ala Val Gln Ile Ser Pro Val Leu Tyr
   1               5                   10                  15
   Ser Arg Glu Ala Thr Val Glu Lys Val Val Arg Lys Ile Arg Glu Leu
               20                  25                  30
   Gly Arg Asn Gly Val Gln Phe Ala Thr Phe Pro Glu Thr Leu Val Pro
           35                  40                  45
   Tyr Tyr Pro Tyr Phe Ala Ala Val Gln Thr Gly Ile Glu Leu Leu Ser
       50                  55                  60
   Gly Lys Glu His Leu Arg Leu Leu Glu Gln Ser Val Thr Val Pro Ser
   65                  70                  75                  80
   Pro Ala Thr Asp Ala Ile Ala Gln Ala Ala Arg Glu Ala Gly Met Val
                   85                  90                  95
   Val Ser Ile Gly Val Asn Glu Arg Asp Gly Gly Thr Ile Tyr Asn Thr
               100                 105                 110
   Gln Leu Leu Phe Asp Ala Asp Gly Thr Leu Val Gln Arg Arg Arg Lys
           115                 120                 125
   Ile Thr Pro Thr His Phe Glu Arg Met Val Trp Gly Gln Gly Asp Gly
       130                 135                 140
   Ser Gly Leu Arg Ala Val Asp Thr Lys Ala Gly Arg Ile Gly Gln Leu
   145                 150                 155                 160
   Ala Cys Phe Glu His Asn Asn Pro Leu Ala Arg Tyr Ala Met Ile Ala
                   165                 170                 175
   Asp Gly Glu Gln Ile His Ser Ala Met Tyr Pro Gly Ser Ala Phe Gly
               180                 185                 190
   Glu Gly Phe Ala Gln Arg Met Glu Ile Asn Ile Arg Gln His Ala Leu
           195                 200                 205
   Glu Ser Gly Cys Phe Val Val Asn Ala Thr Ala Trp Leu Asp Ala Asp
       210                 215                 220
   Gln Gln Ala Gln Ile Met Lys Asp Thr Gly Cys Gly Ile Gly Pro Ile
   225                     230                     235                     240
   Ser Gly Gly Cys Phe Thr Thr Ile Val Thr Pro Asp Gly Met Leu Ile
                       245                     250                     255
   Gly Glu Pro Leu Arg Glu Gly Glu Gly Glu Val Ile Ala Asp Leu Asp
                   260                     265                     270
   Phe Thr Leu Ile Asp Arg Arg Lys Leu Leu Val Asp Ser Val Gly His
               275                     280                     285
   Tyr Asn Arg Pro Glu Leu Leu Ser Leu Leu Ile Asp Arg Thr Pro Ala
           290                     295                     300
   Ala Asn Phe His Glu Arg Asn Ala Leu Pro Ser Val Asn Thr Ala Ser
   305                     310                     315                     320
   Ser Leu Glu Ile Val
                       325
<210> 253
   <211> 924
   <212> DNA
   <213> Unknown
<220>
   <223> obtained from an environmental sample
<400> 253
   atgtcaaacg agaaccacaa ccaaacattc aaagttgccg cggtgcaggc cacacctgta      60
   ttcctcgatc gtgaagcgac catcgacaaa gcttgcgagt tgattgctgc agccggcaat     120
   gaaggggcga ggctggttgt cttcccggag gcattcatcc cgtcctatcc agattgggta     180
   tgggcaatcc caccgggcga agaaggcgtg ctcaatgagt tgtacgcgga actgctctcc     240
   aattcggtca cgattcccag tgacgtgacg gatagactgt gccgggccgc gaggcttgcc     300
   aatgcctacg tagtgatggg gatgagcgaa tgcaatgccg aggccagtgg cgcaagcctg     360
   tataacacgc tattgtacat cgatgcgaag ggtgaaatcc tgggtaaaca tcgaaagttg     420
   gtgccaactg gcggcgagcg actggtgtgg gcacagggcg atggcagcac gctgcaggtc     480
   tacgatactc cactgggtaa actcggcggt ttaatttgct gggagaatta tatgccgctg     540
   gcccgctaca ccatgtacgc ctggggcaca caaatctata tcgcagcgac atgggatcgc     600
   gggcaaccct ggctctccac cttgcggcat atcgccaaag aaggcagggt gtacgtgatc     660
   ggctgttgta tcgcgatgcg caaagatgat atcccagagc gttacccaat gaagcagaag     720
   ttttacgcgg aggccgatga gtggatcaat ataggcgaca gcgcgatcgt caatcctgaa     780
   gggcagttta tcgcggggcc ggtacgcaaa caggaagaga ttctctacgc ggagattaat     840
   ccgcgcatgg tgcaaggccc gaagtggatg ctcgacgtgg cagggcacta cgccaggccg     900
   gacgtattcc agttgacagt gtaa                                            924
<210> 254
   <211> 307
   <212> PRT
   <213> Unknown
<220>
   <223> Obtained from an environmental sample
<400> 254
   Met Ser Asn Glu Asn His Asn Gln Thr Phe Lys Val Ala Ala Val Gln
   1               5                   10                  15
   Ala Thr Pro Val Phe Leu Asp Arg Glu Ala Thr Ile Asp Lys Ala Cys
               20                  25                  30
   Glu Leu Ile Ala Ala Ala Gly Asn Glu Gly Ala Arg Leu Val Val Phe
           35                  40                  45
   Pro Glu Ala Phe Ile Pro Ser Tyr Pro Asp Trp Val Trp Ala Ile Pro
       50                  55                  60
   Pro Gly Glu Glu Gly Val Leu Asn Glu Leu Tyr Ala Glu Leu Leu Ser
   65                  70                  75                  80
   Asn Ser Val Thr Ile Pro Ser Asp Val Thr Asp Arg Leu Cys Arg Ala
                   85                  90                  95
   Ala Arg Leu Ala Asn Ala Tyr Val Val Met Gly Met Ser Glu Cys Asn
               100                 105                 110
   Ala Glu Ala Ser Gly Ala Ser Leu Tyr Asn Thr Leu Leu Tyr Ile Asp
           115                 120                 125
   Ala Lys Gly Glu Ile Leu Gly Lys His Arg Lys Leu Val Pro Thr Gly
       130                 135                 140
   Gly Glu Arg Leu Val Trp Ala Gln Gly Asp Gly Ser Thr Leu Gln Val
   145                 150                 155                 160
   Tyr Asp Thr Pro Leu Gly Lys Leu Gly Gly Leu Ile Cys Trp Glu Asn
                   165                 170                 175
   Tyr Met Pro Leu Ala Arg Tyr Thr Met Tyr Ala Trp Gly Thr Gln Ile
               180                 185                 190
   Tyr Ile Ala Ala Thr Trp Asp Arg Gly Gln Pro Trp Leu Ser Thr Leu
           195                 200                 205
   Arg His Ile Ala Lys Glu Gly Arg Val Tyr Val Ile Gly Cys Cys Ile
       210                 215                 220
   Ala Met Arg Lys Asp Asp Ile Pro Glu Arg Tyr Pro Met Lys Gln Lys
   225                 230                 235                 240
   Phe Tyr Ala Glu Ala Asp Glu Trp Ile Asn Ile Gly Asp Ser Ala Ile
                   245                 250                 255
   Val Asn Pro Glu Gly Gln Phe Ile Ala Gly Pro Val Arg Lys Gln Glu
               260                 265                 270
   Glu Ile Leu Tyr Ala Glu Ile Asn Pro Arg Met Val Gln Gly Pro Lys
           275                 280                 285
   Trp Met Leu Asp Val Ala Gly His Tyr Ala Arg Pro Asp Val Phe Gln
       290                 295                 300
   Leu Thr Val
   305
<210> 255
   <211> 1005
   <212> DNA
   <213> Unknown
<220>
   <223> Obtained from an environmental sample
<400> 255
   atgacaatgt ctaagaccaa atttagggtt gcagctgtgc aggcggcgcc ggttttcctt      60
   gatcgggaag cgacgttgga taaagcttgt ggattgattg aggaggcggg ccgcaacggc     120
   gccagcctcg tcgtcttccc tgagtcattc attccggcct accccgattg ggtttgggct     180
   gtgccggcgg gcgaagaagc tttactcaat gaactgtacg cacaactgtt ggccaacgcc     240
   gttgaaattc ccggcccggc cactcaacgt ttgagccagg cggctaaaaa ggctaaggtt     300
   cacctggcta tgggcctgac cgaacgcaac agcgaggcca gcggcggcag cctttacaac     360
   accttgctct atcttgaccc gcagggccac attctgggca agcatcgtaa gctggtgccc     420
   accggcggtg agcggctggt ttgggcccag ggcgacggca gcactttgca agtttacgat     480
   acgcctctgg gtaaactcag cggcctgatt tgctgggaaa attatatgcc gctggcgcgc     540
   tacgcgctgt atgcctgggg tacgcaaatt tatattgcgg ccacctggga tcggggtgag     600
   ccgtggcttt cgacgttgcg gcatattgcc aaagagggcc gggtgttggt catcggttgc     660
   ggtatggcct tgcgcaaggc tgatattcct gatcattttg aattcaagca gcgcttttat     720
   caaaacgccg ccgagtggat caacgggggc gacagcgcca ttgtcaaccc tgatggtgaa     780
   tttattgctg gccccttaag cgagcaggaa ggcattttgt acgccgagat tgatccggcc     840
   cagatgggcg ggccaaagtg gatgctcgac gtggccgggc attacgctcg cccggatgtg     900
   tttgaactga cggtccatac cgccgcccga cccatgatca cctcgaaaaa ggatggccta     960
   acacccgccg aggccgttac gcaagtaacg aaagcattat tgtaa                    1005
<210> 256
   <211> 334
   <212> PRT
   <213> Unknown
<220>
   <223> Obtained from an environmental sample
<400> 256
   Met Thr Met Ser Lys Thr Lys Phe Arg Val Ala Ala Val Gln Ala Ala
   1               5                   10                  15
   Pro Val Phe Leu Asp Arg Glu Ala Thr Leu Asp Lys Ala Cys Gly Leu
               20                  25                  30
   Ile Glu Glu Ala Gly Arg Asn Gly Ala Ser Leu Val Val Phe Pro Glu
           35                  40                  45
   Ser Phe Ile Pro Ala Tyr Pro Asp Trp Val Trp Ala Val Pro Ala Gly
       50                  55                  60
   Glu Glu Ala Leu Leu Asn Glu Leu Tyr Ala Gln Leu Leu Ala Asn Ala
   65                  70                  75                  80
   Val Glu Ile Pro Gly Pro Ala Thr Gln Arg Leu Ser Gln Ala Ala Lys
                   85                  90                  95
   Lys Ala Lys Val His Leu Ala Met Gly Leu Thr Glu Arg Asn Ser Glu
               100                 105                 110
   Ala Ser Gly Gly Ser Leu Tyr Asn Thr Leu Leu Tyr Leu Asp Pro Gln
           115                 120                 125
   Gly His Ile Leu Gly Lys His Arg Lys Leu Val Pro Thr Gly Gly Glu
       130                 135                 140
   Arg Leu Val Trp Ala Gln Gly Asp Gly Ser Thr Leu Gln Val Tyr Asp
   145                 150                 155                 160
   Thr Pro Leu Gly Lys Leu Ser Gly Leu Ile Cys Trp Glu Asn Tyr Met
                   165                 170                 175
   Pro Leu Ala Arg Tyr Ala Leu Tyr Ala Trp Gly Thr Gln Ile Tyr Ile
               180                 185                 190
   Ala Ala Thr Trp Asp Arg Gly Glu Pro Trp Leu Ser Thr Leu Arg His
           195                 200                 205
   Ile Ala Lys Glu Gly Arg Val Leu Val Ile Gly Cys Gly Met Ala Leu
       210                 215                 220
   Arg Lys Ala Asp Ile Pro Asp His Phe Glu Phe Lys Gln Arg Phe Tyr
   225                 230                 235                 240
   Gln Asn Ala Ala Glu Trp Ile Asn Gly Gly Asp Ser Ala Ile Val Asn
                   245                 250                 255
   Pro Asp Gly Glu Phe Ile Ala Gly Pro Leu Ser Glu Gln Glu Gly Ile
               260                 265                 270
   Leu Tyr Ala Glu Ile Asp Pro Ala Gln Met Gly Gly Pro Lys Trp Met
           275                 280                 285
   Leu Asp Val Ala Gly His Tyr Ala Arg Pro Asp Val Phe Glu Leu Thr
       290                 295                 300
   Val His Thr Ala Ala Arg Pro Met Ile Thr Ser Lys Lys Asp Gly Leu
   305                 310                 315                 320
   Thr Pro Ala Glu Ala Val Thr Gln Val Thr Lys Ala Leu Leu
                   325                 330
<210> 257
   <211> 942
   <212> DNA
   <213> Unknown
<220>
   <223> Obtained from an environmental sample
<400> 257
   atgaacaaga tcgcgatcat tcagcgccca cccgtgctac tcgaccgcat cgccacgctg     60
   gcggttgcgg tggagtcgat cgacgaagct gccgcagccg gtgcctcact gatcgttctt    120
   ccagaaacct tcatccccgg ctacccgtcc tggatctggc gtctcgcgcc ggggagggat    180
   ggtgcgctca ttgcccagtt gcatgcccga ctgctcgcca acgcggtcga tcttgcggct    240
   ggagatctgg atgccctgtg tgaagttgcg cacggccacc gggtgaccgt ggtgtgcggc    300
   ctcaacgaat gcgagcgcag tcgcggcggg ggcactctct acaacacggt cgtcgtgatc    360
   gaccccgacg gcaagctgtg caatcgccac cgcaagctga tgccgaccaa cccggaacgc    420
   atggtgcacg gtctgggtga tgcatcgggc ctgcgcgccg tcgacacccc ggtgggtcga    480
   gtgggcgcac tcatctgctg ggaaaactat atgccgctgg cacgctacgc actttacgcc    540
   gagggggtgg aagtctacgt ggcgcccacc tatgacagcg gcgatggctg gatcagtacg    600
   atgcgtcata ttgcgcttga gggacgctgc tgggtgctgg gtagcggaac cgtactgcgt    660
   ggcagcgacg tcccagaaga ctttccgtca cacctggacc tgtttcccga cgcggaggaa    720
   tggatcaatc cgggcgactc ggtggtcgtc gatcctcagg gcaagatcgt cgcaggcccg    780
   atgcgacgtg agacaggcat tctctacgca gaaatcgacg ccgaacgggt cgcgccttcg    840
   cgccgcacgc tcgatgtcgc cggacactac gcccgcccgg atattttcga gctccatgtc    900
   cgacgtacgc cggcgatgcc ggtccacgcc gttgatgcat ga                       942
<210> 258
   <211> 313
   <212> PRT
   <213> Unknown
<220>
   <223> Obtained from an environmental sample
<400> 258
   Met Asn Lys Ile Ala Ile Ile Gln Arg Pro Pro Val Leu Leu Asp Arg
    1               5                  10                  15
   Ile Ala Thr Leu Ala Val Ala Val Glu Ser Ile Asp Glu Ala Ala Ala
               20                  25                  30
   Ala Gly Ala Ser Leu Ile Val Leu Pro Glu Thr Phe Ile Pro Gly Tyr
           35                  40                  45
   Pro Ser Trp Ile Trp Arg Leu Ala Pro Gly Arg Asp Gly Ala Leu Ile
       50                  55                  60
   Ala Gln Leu His Ala Arg Leu Leu Ala Asn Ala Val Asp Leu Ala Ala
   65                  70                  75                  80
   Gly Asp Leu Asp Ala Leu Cys Glu Val Ala His Gly His Arg Val Thr
                   85                  90                  95
   Val Val Cys Gly Leu Asn Glu Cys Glu Arg Ser Arg Gly Gly Gly Thr
               100                 105                 110
   Leu Tyr Asn Thr Val Val Val Ile Asp Pro Asp Gly Lys Leu Cys Asn
           115                 120                 125
   Arg His Arg Lys Leu Met Pro Thr Asn Pro Glu Arg Met Val His Gly
       130                 135                 140
   Leu Gly Asp Ala Ser Gly Leu Arg Ala Val Asp Thr Pro Val Gly Arg
   145                 150                 155                 160
   Val Gly Ala Leu Ile Cys Trp Glu Asn Tyr Met Pro Leu Ala Arg Tyr
                   165                 170                 175
   Ala Leu Tyr Ala Glu Gly Val Glu Val Tyr Val Ala Pro Thr Tyr Asp
               180                 185                 190
   Ser Gly Asp Gly Trp Ile Ser Thr Met Arg His Ile Ala Leu Glu Gly
           195                 200                 205
   Arg Cys Trp Val Leu Gly Ser Gly Thr Val Leu Arg Gly Ser Asp Val
       210                 215                 220
   Pro Glu Asp Phe Pro Ser His Leu Asp Leu Phe Pro Asp Ala Glu Glu
   225                 230                 235                 240
   Trp Ile Asn Pro Gly Asp Ser Val Val Val Asp Pro Gln Gly Lys Ile
                   245                 250                 255
   Val Ala Gly Pro Met Arg Arg Glu Thr Gly Ile Leu Tyr Ala Glu Ile
               260                 265                 270
   Asp Ala Glu Arg Val Ala Pro Ser Arg Arg Thr Leu Asp Val Ala Gly
           275                 280                 285
   His Tyr Ala Arg Pro Asp Ile Phe Glu Leu His Val Arg Arg Thr Pro
       290                 295                 300
   Ala Met Pro Val His Ala Val Asp Ala
   305                 310
<210> 259
   <211> 981
   <212> DNA
   <213> Unknown
<220>
   <223> Obtained from an environmental sample
<400> 259
   atggccatca tcaaagccgc cgccgtccag atcagtccgg tgctgtatag ccgcgaggga      60
   accgtggaca aggtctgcca gcagatcatc gccctcggtc agcaaggcgt gcaatttgcg     120
   gtctttccgg aaacggtggt gccgtactac ccctatttct ccttcgtcca gccgccgttc     180
   gccatgggca aggaacacct gaaactgttg gaacaatcgg tgattgtgcc gtcggctgcc     240
   accttggcga tcggcgaagc gtgcaaacaa gcgggaatgg tggtgtctat cggcgtcaat     300
   gagcgcgatg gcggcacgat ctacaacgcc cagttgctgt ttgacgctga tggcagcttg     360
   attcagcacc gtcgcaaaat aaccccgacg taccacgagc ggatgatctg gggtcaaggc     420
   gacggctccg ggttgcgcgc catcgacagc gcggtcgggc gtattggctc gctggcctgc     480
   tgggaacatt acaacccctt ggcccgctac gccctgatgg ccgacggcga gcagattcac     540
   gcggctatgt ttcccggctc tctggtgggt gacatttttg ccgatcagat agaggtcact     600
   attcgtcatc acgccttgga gtccggctgc ttcgtggtca actccaccgc gtggcttgat     660
   gctgatcagc aaggccaaat catgcaggac accggttgca gcattggccc aatctcgggt     720
   ggctgcttca cggccatcgt ttccccggaa ggcaaattac tcggcgaacc gctgcgttca     780
   ggtgagggcg cagtcatcgc cgacctggac atggcattga tcgacaagcg caaacggatg     840
   atggattccg tcggccatta cagccgccca gaactgctca gtttattgat cgaccgcacg     900
   cccaccgctc atgtgcatga gcgcggcgcc catcaccttg ccgtagcctc tatcggggag     960
   cttgaccatg caaaccaatg a                                                981
<210> 260
   <211> 326
   <212> PRT
   <213> Unknown
<220>
   <223> Obtained from an environmental sample
<400> 260
   Met Ala Ile Ile Lys Ala Ala Ala Val Gln Ile Ser Pro Val Leu Tyr
   1               5                   10                  15
   Ser Arg Glu Gly Thr Val Asp Lys Val Cys Gln Gln Ile Ile Ala Leu
               20                  25                  30
   Gly Gln Gln Gly Val Gln Phe Ala Val Phe Pro Glu Thr Val Val Pro
           35                  40                  45
   Tyr Tyr Pro Tyr Phe Ser Phe Val Gln Pro Pro Phe Ala Met Gly Lys
       50                  55                  60
   Glu His Leu Lys Leu Leu Glu Gln Ser Val Ile Val Pro Ser Ala Ala
   65                  70                  75                  80
   Thr Leu Ala Ile Gly Glu Ala Cys Lys Gln Ala Gly Met Val Val Ser
                   85                  90                  95
   Ile Gly Val Asn Glu Arg Asp Gly Gly Thr Ile Tyr Asn Ala Gln Leu
               100                 105                 110
   Leu Phe Asp Ala Asp Gly Ser Leu Ile Gln His Arg Arg Lys Ile Thr
           115                 120                 125
   Pro Thr Tyr His Glu Arg Met Ile Trp Gly Gln Gly Asp Gly Ser Gly
       130                 135                 140
   Leu Arg Ala Ile Asp Ser Ala Val Gly Arg Ile Gly Ser Leu Ala Cys
   145                 150                 155                 160
   Trp Glu His Tyr Asn Pro Leu Ala Arg Tyr Ala Leu Met Ala Asp Gly
                   165                 170                 175
   Glu Gln Ile His Ala Ala Met Phe Pro Gly Ser Leu Val Gly Asp Ile
               180                 185                 190
   Phe Ala Asp Gln Ile Glu Val Thr Ile Arg His His Ala Leu Glu Ser
           195                 200                 205
   Gly Cys Phe Val Val Asn Ser Thr Ala Trp Leu Asp Ala Asp Gln Gln
       210                 215                 220
   Gly Gln Ile Met Gln Asp Thr Gly Cys Ser Ile Gly Pro Ile Ser Gly
   225                 230                 235                 240
   Gly Cys Phe Thr Ala Ile Val Ser Pro Glu Gly Lys Leu Leu Gly Glu
                   245                 250                 255
   Pro Leu Arg Ser Gly Glu Gly Ala Val Ile Ala Asp Leu Asp Met Ala
               260                 265                 270
   Leu Ile Asp Lys Arg Lys Arg Met Met Asp Ser Val Gly His Tyr Ser
           275                 280                 285
   Arg Pro Glu Leu Leu Ser Leu Leu Ile Asp Arg Thr Pro Thr Ala His
       290                 295                 300
   Val His Glu Arg Gly Ala His His Leu Ala Val Ala Ser Ile Gly Glu
   305                 310                 315                 320
   Leu Asp His Ala Asn Gln
                   325
<210> 261
   <211> 1014
   <212> DNA
   <213> Unknown
<220>
   <223> Obtained from an environmental sample
<400> 261
   atgggtctgg ttcatcagaa atacaaggta gcggtggttc aggcggcacc ggtttttctc      60
   gatctcgatg caaccgtgga caagaccatt gcgctgatcg aggaagcttc cgcgcaaggc     120
   gcaaaactgg tcgcgtttcc cgagaccttc attcccggat atccgtggca gatctggctc     180
   ggcgcgccgg cctgggcgat cggccgcggc tttgtgcagc gctacttcga caattcgctg     240
   ggcttcgaca gcccgcaggc ggaaaaaatc cgccaggccg tgaagcgcgc caagctgacc     300
   gcggtgcttg ggctatccga acgcgacggc ggcagcctct atatcgcgca gtggctgatc     360
   ggccctgacg gcgagaccat cgccaagcgc cggaaactgc gtccgaccca tgccgaacgc     420
   accgtgttcg gcgaaggcga cggcagcgat cttgcggtcc acgatcgcgc cgacgtcggg     480
   cgcttgggcg cactgtgctg ctgggagcac ctgcagccgc tatcgaaata cgcgatgtac     540
   gcccagaacg aacaggtgca tgtcggcgcc tggccgagct tttcgctgta cgatccgttc     600
   gcacatgcgc tcggccatga ggtcaacaac gccgccagca aggtctatgc ggtcgaaggc     660
   tcctgcttct tcctgggtcc ctgcgctgtc gtctcgcagg cgatgatcga cgagctctgc     720
   gactctcccg agaaacatgc cttcctgcat gtcggcggcg gccacgcggt gatctacggc     780
   ccggacggca gttcgctggc cgagaaactc ccgcccgacc aggaaggcat tctgtacgcc     840
   gatatcgatc tcggcatgat cggggtcgcg aagaacgccg ccgatccggc cgggcattat     900
   tcgcggcccg acgtcacgcg gctgttgctc aacaccacgc gcgccaaccg cgtcgagcat     960
   ttctcgcttc ccgtcgatgc cgaggtcatg agcgaaatca ggctgcaggc ctga          1014
<210> 262
   <211> 337
   <212> PRT
   <213> Unknown
<220>
   <223> Obtained from an environmental sample
<400> 262
   Met Gly Leu Val His Gln Lys Tyr Lys Val Ala Val Val Gln Ala Ala
    1               5                  10                  15
   Pro Val Phe Leu Asp Leu Asp Ala Thr Val Asp Lys Thr Ile Ala Leu
               20                  25                  30
   Ile Glu Glu Ala Ser Ala Gln Gly Ala Lys Leu Val Ala Phe Pro Glu
           35                  40                  45
   Thr Phe Ile Pro Gly Tyr Pro Trp Gln Ile Trp Leu Gly Ala Pro Ala
       50                  55                  60
   Trp Ala Ile Gly Arg Gly Phe Val Gln Arg Tyr Phe Asp Asn Ser Leu
   65                  70                  75                  80
   Gly Phe Asp Ser Pro Gln Ala Glu Lys Ile Arg Gln Ala Val Lys Arg
                   85                  90                  95
   Ala Lys Leu Thr Ala Val Leu Gly Leu Ser Glu Arg Asp Gly Gly Ser
               100                 105                 110
   Leu Tyr Ile Ala Gln Trp Leu Ile Gly Pro Asp Gly Glu Thr Ile Ala
           115                 120                 125
   Lys Arg Arg Lys Leu Arg Pro Thr His Ala Glu Arg Thr Val Phe Gly
       130                 135                 140
   Glu Gly Asp Gly Ser Asp Leu Ala Val His Asp Arg Ala Asp Val Gly
   145                 150                 155                 160
   Arg Leu Gly Ala Leu Cys Cys Trp Glu His Leu Gln Pro Leu Ser Lys
                   165                 170                 175
   Tyr Ala Met Tyr Ala Gln Asn Glu Gln Val His Val Gly Ala Trp Pro
               180                 185                 190
   Ser Phe Ser Leu Tyr Asp Pro Phe Ala His Ala Leu Gly His Glu Val
           195                 200                 205
   Asn Asn Ala Ala Ser Lys Val Tyr Ala Val Glu Gly Ser Cys Phe Phe
       210                 215                 220
   Leu Gly Pro Cys Ala Val Val Ser Gln Ala Met Ile Asp Glu Leu Cys
   225                 230                 235                 240
   Asp Ser Pro Glu Lys His Ala Phe Leu His Val Gly Gly Gly His Ala
                   245                 250                 255
   Val Ile Tyr Gly Pro Asp Gly Ser Ser Leu Ala Glu Lys Leu Pro Pro
               260                 265                 270
   Asp Gln Glu Gly Ile Leu Tyr Ala Asp Ile Asp Leu Gly Met Ile Gly
           275                 280                 285
   Val Ala Lys Asn Ala Ala Asp Pro Ala Gly His Tyr Ser Arg Pro Asp
       290                 295                 300
   Val Thr Arg Leu Leu Leu Asn Thr Thr Arg Ala Asn Arg Val Glu His
   305                 310                 315                 320
   Phe Ser Leu Pro Val Asp Ala Glu Val Met Ser Glu Ile Arg Leu Gln
                   325                 330                 335
   Ala
<210> 263
   <211> 1014
   <212> DNA
   <213> Unknown
<220>
   <223> Obtained from an environmental sample
<400> 263
   atgaaagtcg tcaaagcggc agcggttcag ttgagccctg tcctctacag ccgcgaggca      60
   accgtcgcga aggtcgtgcg gaagatccac gagcttgggc agcagggcgt gcagttcgcc     120
   accttcccgg aaaccgttgt gccgtactac ccgtatttct ccgcggtcca gacgccgatg     180
   cagcttctgg ctggaaccga gcatctgaaa ttgctcgacc aggccgtgac ggtgccgtct     240
   cccgcgaccg acgcgatcgg cgaggcagcc cggaaggcgg gcatggtggt gtccatcggc     300
   gtcaacgagc gtgatggtgg aaccctgtac aacacccaat tgctcttcga cgccgatggc     360
   accctgatcc agcgccgccg caagatcacg cccacccatt tcgagcgcat gatctggggc     420
   cagggtgacg ggtcgggcct gcgtgccgtc gacagcaagg tcggccgcat tggccagctg     480
   gcatgcttcg agcacaacaa tcctctggcg cgctacgcga tgatggccga cggcgagcag     540
   atccattcgg ccatgtatcc gggttctgcc ttcggcgagg gctttgccca gaggatggaa     600
   atcaatatcc gccagcacgc actggagtcc gggtgcttcg tcgtgaacgc gacggcctgg     660
   ctggatgccg accagcaggc gcaaatcatg aaagacacgg gctgcgggat cggtccgatc     720
   tcgggcggtt gcttcaccac gatcgtggca cccgacggca cgctgctggg ggaacctctg     780
   cgctcgggcg agggcgaggt catcgccgat ctcgatttca cggagatcga ccggcgcaag     840
   atgctgatgg actcggcagg ccactacaac cgtccggaac tgctcagtct gctgatcgac     900
   cgcacgccga ccgcaaacgt gcacgaacgg atggcgcatc cccaagcgag cacgaagcag     960
   ccgcgctccg gcgatctgcc cgctgcgctg gctggcgcgc aggagatcct gtga          1014
<210> 264
   <211> 337
   <212> PRT
   <213> Unknown
<220>
   <223> Obtained from an environmental sample
<400> 264
   Met Lys Val Val Lys Ala Ala Ala Val Gln Leu Ser Pro Val Leu Tyr
   1               5                   10                  15
   Ser Arg Glu Ala Thr Val Ala Lys Val Val Arg Lys Ile His Glu Leu
               20                  25                  30
   Gly Gln Gln Gly Val Gln Phe Ala Thr Phe Pro Glu Thr Val Val Pro
           35                  40                  45
   Tyr Tyr Pro Tyr Phe Ser Ala Val Gln Thr Pro Met Gln Leu Leu Ala
       50                  55                  60
   Gly Thr Glu His Leu Lys Leu Leu Asp Gln Ala Val Thr Val Pro Ser
   65                  70                  75                  80
   Pro Ala Thr Asp Ala Ile Gly Glu Ala Ala Arg Lys Ala Gly Met Val
                   85                  90                  95
   Val Ser Ile Gly Val Asn Glu Arg Asp Gly Gly Thr Leu Tyr Asn Thr
               100                 105                 110
   Gln Leu Leu Phe Asp Ala Asp Gly Thr Leu Ile Gln Arg Arg Arg Lys
           115                 120                 125
   Ile Thr Pro Thr His Phe Glu Arg Met Ile Trp Gly Gln Gly Asp Gly
       130                 135                 140
   Ser Gly Leu Arg Ala Val Asp Ser Lys Val Gly Arg Ile Gly Gln Leu
   145                 150                 155                 160
   Ala Cys Phe Glu His Asn Asn Pro Leu Ala Arg Tyr Ala Met Met Ala
                   165                 170                 175
   Asp Gly Glu Gln Ile His Ser Ala Met Tyr Pro Gly Ser Ala Phe Gly
               180                 185                 190
   Glu Gly Phe Ala Gln Arg Met Glu Ile Asn Ile Arg Gln His Ala Leu
           195                 200                 205
   Glu Ser Gly Cys Phe Val Val Asn Ala Thr Ala Trp Leu Asp Ala Asp
       210                 215                 220
   Gln Gln Ala Gln Ile Met Lys Asp Thr Gly Cys Gly Ile Gly Pro Ile
   225                 230                 235                 240
   Ser Gly Gly Cys Phe Thr Thr Ile Val Ala Pro Asp Gly Thr Leu Leu
                   245                 250                 255
   Gly Glu Pro Leu Arg Ser Gly Glu Gly Glu Val Ile Ala Asp Leu Asp
               260                 265                 270
   Phe Thr Glu Ile Asp Arg Arg Lys Met Leu Met Asp Ser Ala Gly His
           275                 280                 285
   Tyr Asn Arg Pro Glu Leu Leu Ser Leu Leu Ile Asp Arg Thr Pro Thr
       290                 295                 300
   Ala Asn Val His Glu Arg Met Ala His Pro Gln Ala Ser Thr Lys Gln
   305                 310                 315                 320
   Pro Arg Ser Gly Asp Leu Pro Ala Ala Leu Ala Gly Ala Gln Glu Ile
                   325                 330                 335
   Leu
<210> 265
   <211> 999
   <212> DNA
   <213> unknown
<220>
   <223> Obtained from an environmental sample
<400> 265
   atgcttaatc taggcatagt ccagatgaac gcagagccgc tcaacgtgga aggcaacctg      60
   ctcaaggcgg agcgctatgt cgcgaagtgc gccgcggacg gcgcccaact cgtggtgctg     120
   ccggagatgt tcaacgtcgg cttccacctc ggcgagtccc tgatgatggt cgccgagccc     180
   ctggacggca agaccgtgca gtggctgcaa cggcaggcgt ccacccataa catatatatc     240
   accgggagct tatacgagcg ttacgacgag catttctaca acaccatggt catggtggga     300
   tacgacggca gcgtgcagta ctaccgcaag cgcaatccta cctggtccga gtcggcggtg     360
   tggcgccgca gcgaggtgcc aggccccggt atattcgata ccccgttcgg gcgcatcggg     420
   ggcgtcatct gcttcgattc cttcgcgcgc gagacccacg agggcttcaa gcagagcggg     480
   gtcgaggcgg tggtaatcat cgccctgtgg ggcgccaacc gtgcgcgggc attcttctgg     540
   cgcccggacc tcctgctaag ccgggaaggg ctggtccgtt ggtcccggct ggcctcggag     600
   gacgtccccc gaaatcacgc gaaagagctc ggggtcccgg tcgccttcgt caaccagagt     660
   ggcaccatcc gcatgaccag ccccatccct ttccccgact ggccggtgca gagctccttc     720
   tacgacttca tcggcaagtc ccacgtccgg gacgcatccg gagaggtgat cgcgagggtg     780
   gacgaggggg agatcgactc ctgcctggtg gtcccggtag aggtcgagca ggcgcagagc     840
   aggccggaga tcaggaagtc aaatatatcg cccggctacc tcggcaagga ttactatttc     900
   gtggagccgc cgcttatctg caagctcttc caggtctggt tcctcagcgg cctggtgccc     960
   accgaatacg aggcgcggcg tctgcgccac ctgttctga                            999
<210> 266
   <211> 332
   <212> PRT
   <213> Unknown
<220>
   <223> Obtained from an environmental sample
<400> 266
   Met Leu Asn Leu Gly Ile Val Gln Met Asn Ala Glu Pro Leu Asn Val
   1               5                   10                  15
   Glu Gly Asn Leu Leu Lys Ala Glu Arg Tyr Val Ala Lys Cys Ala Ala
               20                  25                  30
   Asp Gly Ala Gln Leu Val Val Leu Pro Glu Met Phe Asn Val Gly Phe
           35                  40                  45
   His Leu Gly Glu Ser Leu Met Met Val Ala Glu Pro Leu Asp Gly Lys
       50                  55                  60
   Thr Val Gln Trp Leu Gln Arg Gln Ala Ser Thr His Asn Ile Tyr Ile
   65                  70                  75                  80
   Thr Gly Ser Leu Tyr Glu Arg Tyr Asp Glu His Phe Tyr Asn Thr Met
                   85                  90                  95
   Val Met Val Gly Tyr Asp Gly Ser Val Gln Tyr Tyr Arg Lys Arg Asn
               100                 105                 110
   Pro Thr Trp Ser Glu Ser Ala Val Trp Arg Arg Ser Glu Val Pro Gly
           115                 120                 125
   Pro Gly Ile Phe Asp Thr Pro Phe Gly Arg Ile Gly Gly Val Ile Cys
       130                 135                 140
   Phe Asp Ser Phe Ala Arg Glu Thr His Glu Gly Phe Lys Gln Ser Gly
   145                 150                 155                 160
   Val Glu Ala Val Val Ile Ile Ala Leu Trp Gly Ala Asn Arg Ala Arg
                   165                 170                 175
   Ala Phe Phe Trp Arg Pro Asp Leu Leu Leu Ser Arg Glu Gly Leu Val
               180                 185                 190
   Arg Trp Ser Arg Leu Ala Ser Glu Asp Val Pro Arg Asn His Ala Lys
           195                 200                 205
   Glu Leu Gly Val Pro Val Ala Phe Val Asn Gln Ser Gly Thr Ile Arg
       210                 215                 220
   Met Thr Ser Pro Ile Pro Phe Pro Asp Trp Pro Val Gln Ser Ser Phe
   225                 230                 235                 240
   Tyr Asp Phe Ile Gly Lys Ser His Val Arg Asp Ala Ser Gly Glu Val
                   245                 250                 255
   Ile Ala Arg Val Asp Glu Gly Glu Ile Asp Ser Cys Leu Val Val Pro
               260                 265                 270
   Val Glu Val Glu Gln Ala Gln Ser Arg Pro Glu Ile Arg Lys Ser Asn
           275                 280                 285
   Ile Ser Pro Gly Tyr Leu Gly Lys Asp Tyr Tyr Phe Val Glu Pro Pro
       290                 295                 300
   Leu Ile Cys Lys Leu Phe Gln Val Trp Phe Leu Ser Gly Leu Val Pro
   305                 310                 315                 320
   Thr Glu Tyr Glu Ala Arg Arg Leu Arg His Leu Phe
                   325                 330
<210> 267
   <211> 1038
   <212> DNA
   <213> Unknown
<220>
   <223> Obtained from an environmental sample
<400> 267
   atgggcatta cgcatccgaa gttcaaggcc gcggcggtac aggcggcgcc gggctttctc      60
   gacagcgagg ccaccgtcga caagacgatc cgcctgatgc aggaagcggc ggaccacggc     120
   gcctcgctga tcgtctttcc ggaagcctgg ctgcccggtt atccgtggtg gatctggctc     180
   ggtccgcccg cctggggcat gcagttcgtg cagcgctact tcgacaattc gccgagcgtc     240
   ggcgatgatc ttttccgccg gatcgagcgc gcggccgcca aggcgaagat cgaagtggtc     300
   ctcggtctca gcgagcgcgc tgccggctcg ctgtacctcg cgcaggcgtt catctcctca     360
   acgggcgaga cgcgcgcagt gcgccgcaag ttgcgaccaa cgcacgtcga gcgaaccgtt     420
   ttcggcgagg gcgatggcag cgacttcaag gtgttcgaca ctccgctggg ccgcgtcggt     480
   ggtctcttgt gctgggaaca cctgcaaccg ctgtcgcgct acgcgatgtt ctcgatgaac     540
   gagcaggtgc acgccgccgc ctggccgacg ttcagcctct acacggattt tgcccatgcc     600
   ctcggccacg aactgaatct cgcagccagc gctacttacg cggctgaagg gcagtgctac     660
   gtgattgccg cctgtggcgt ggtcacgcag gagatgctgg atctgatgaa ggcgccgtgc     720
   cccccggaat atctgcgggt cggcggcgga tacgccatga tctttgcgcc cgacggacgg     780
   cgcattgcgg cggcgctgcc gccggaacaa gaagggctga tttacgccga catcgatctt     840
   tcgatgatct ctctcgccaa ggcggctgcc gatcccaccg gtcactactc gcggccggat     900
   gtcgtgcggc ttatgctgaa taccgaaccg atgcagcggg tcgaaaagct gcagccgccg     960
   ctggactcag ccgcgcgccg tgagaatgaa ccggcacgcg agaccgcagc ggcgaccgag    1020
   agccgccagc cccagtaa                                                  1038
<210> 268
   <211> 344
   <212> PRT
   <213> Unknown
<220>
   <223> Obtained from an environmental sample
<400> 268
   Met Gly Ile Thr His Pro Lys Phe Lys Ala Ala Ala Val Gln Ala Ala
   1               5                   10                  15
   Pro Gly Phe Leu Asp Ser Glu Ala Thr Val Asp Lys Thr Ile Arg Leu
               20                  25                  30
   Met Gln Glu Ala Ala Asp His Gly Ala Ser Leu Ile Val Phe Pro Glu
           35                  40                  45
   Ala Trp Leu Pro Gly Tyr Pro Trp Trp Ile Trp Leu Gly Pro Pro Ala
       50                  55                  60
   Trp Gly Met Gln Phe Val Gln Arg Tyr Phe Asp Asn Ser Pro Ser Val
   65                  70                  75                  80
   Gly Asp Asp Leu Phe Arg Arg Ile Glu Arg Ala Ala Ala Lys Ala Lys
                   85                  90                  95
   Ile Glu Val Val Leu Gly Leu Ser Glu Arg Ala Ala Gly Ser Leu Tyr
               100                 105                 110
   Leu Ala Gln Ala Phe Ile Ser Ser Thr Gly Glu Thr Arg Ala Val Arg
           115                 120                 125
   Arg Lys Leu Arg Pro Thr His Val Glu Arg Thr Val Phe Gly Glu Gly
       130                 135                 140
   Asp Gly Ser Asp Phe Lys Val Phe Asp Thr Pro Leu Gly Arg Val Gly
   145                 150                 155                 160
   Gly Leu Leu Cys Trp Glu His Leu Gln Pro Leu Ser Arg Tyr Ala Met
                   165                 170                 175
   Phe Ser Met Asn Glu Gln Val His Ala Ala Ala Trp Pro Thr Phe Ser
               180                 185                 190
   Leu Tyr Thr Asp Phe Ala His Ala Leu Gly His Glu Leu Asn Leu Ala
           195                 200                 205
   Ala Ser Ala Thr Tyr Ala Ala Glu Gly Gln Cys Tyr Val Ile Ala Ala
       210                 215                 220
   Cys Gly Val Val Thr Gln Glu Met Leu Asp Leu Met Lys Ala Pro Cys
   225                 230                 235                 240
   Pro Pro Glu Tyr Leu Arg Val Gly Gly Gly Tyr Ala Met Ile Phe Ala
                   245                 250                 255
   Pro Asp Gly Arg Arg Ile Ala Ala Ala Leu Pro Pro Glu Gln Glu Gly
               260                 265                 270
   Leu Ile Tyr Ala Asp Ile Asp Leu Ser Met Ile Ser Leu Ala Lys Ala
           275                 280                 285
   Ala Ala Asp Pro Thr Gly His Tyr Ser Arg Pro Asp Val Val Arg Leu
       290                 295                 300
   Met Leu Asn Thr Glu Pro Met Gln Arg Val Glu Lys Leu Gln Pro Pro
   305                 310                 315                 320
   Leu Asp Ser Ala Ala Arg Arg Glu Asn Glu Pro Ala Arg Glu Thr Ala
                   325                 330                 335
   Ala Ala Thr Glu Ser Arg Gln Pro
               340
<210> 269
   <211> 1014
   <212> DNA
   <213> Unknown
<220>
   <223> Obtained from an environmental sample
<400> 269
   atgtctgaaa cagccttcaa gatcgcggtc gtacaggcgg ctccggtttt tctcgacgca      60
   aaggcgacgg tggacaaggc gatcggtctg atggccgaag ccggcgccaa gggcgccaag     120
   ctgctcgcat tcccggaagt attcatcccc ggctaccctt ggtggctgtg gctgggcaca     180
   ccggcatggg gcatgcagtt tgttgccaag tatcacgcga actcgcttcg tgcagacggg     240
   cctgaattgg cagccctcgc ggcggcggcg gcgaagtccg atatcaatgc cgtcatcggc     300
   ttctcggaga tcgacggcgg ttccctctac atcagccagg cgctcatcag cgacaagggc     360
   gagataatgt tcaaacggcg caagctgaag ccgacgcacg tcgaacgcac gttgttcggc     420
   gaaggggacg ggtccgactt ccaggtcgtg gacacgagcg tcggcaggct cggtgccttg     480
   tgttgcgccg aacacataca gccgctgtcg aagtacgcga tgtactccat gcacgaacag     540
   gtgcacgtcg cctcctggcc gtcatttact ttgtaccgcg gcacggcata tgccttgggc     600
   cacgaggtca atctggccgc gagccagatt tatgcgctcg agggaggctg tttcgtcctt     660
   catgcgagcg ccatcaccgg ccaggacatg tttgacgtgc tgtgcgacac tccggagagg     720
   acgcaactgc tgaactccga cggcggcaag gtcggcggcg gctactcgat gatcttcggt     780
   cccgatggcc agccccttgt tgggcatctg cctcaagaca ccgagggaat actctacgca     840
   gatattgacc tggcgaacat ttccgttgcc aaagcggcct acgacccgtc cggacactat     900
   gcgcgcggag acgtggtgcg cttgatggtc aatcgcaacc cgcggcatac gagtgttgcg     960
   ttcggcgggg gcgccggcga ggcagcaacc tggacggaag caaaagcgga gtga          1014
<210> 270
   <211> 337
   <212> PRT
   <213> Unknown
<220>
   <223> Obtained from an environmental sample
<400> 270
   Met Ser Glu Thr Ala Phe Lys Ile Ala Val Val Gln Ala Ala Pro Val
   1               5                   10                  15
   Phe Leu Asp Ala Lys Ala Thr Val Asp Lys Ala Ile Gly Leu Met Ala
               20                  25                  30
   Glu Ala Gly Ala Lys Gly Ala Lys Leu Leu Ala Phe Pro Glu Val Phe
           35                  40                  45
   Ile Pro Gly Tyr Pro Trp Trp Leu Trp Leu Gly Thr Pro Ala Trp Gly
               50                  55                  60
   Met Gln Phe Val Ala Lys Tyr His Ala Asn Ser Leu Arg Ala Asp Gly
   65                  70                  75                  80
   Pro Glu Leu Ala Ala Leu Ala Ala Ala Ala Ala Lys Ser Asp Ile Asn
                   85                  90                  95
   Ala Val Ile Gly Phe Ser Glu Ile Asp Gly Gly Ser Leu Tyr Ile Ser
               100                 105                 110
   Gln Ala Leu Ile Ser Asp Lys Gly Glu Ile Met Phe Lys Arg Arg Lys
           115                 120                 125
   Leu Lys Pro Thr His Val Glu Arg Thr Leu Phe Gly Glu Gly Asp Gly
       130                 135                 140
   Ser Asp Phe Gln Val Val Asp Thr Ser Val Gly Arg Leu Gly Ala Leu
   145                 150                 155                 160
   Cys Cys Ala Glu His Ile Gln Pro Leu Ser Lys Tyr Ala Met Tyr Ser
                   165                 170                 175
   Met His Glu Gln Val His Val Ala Ser Trp Pro Ser Phe Thr Leu Tyr
               180                 185                 190
   Arg Gly Thr Ala Tyr Ala Leu Gly His Glu Val Asn Leu Ala Ala Ser
           195                 200                 205
   Gln Ile Tyr Ala Leu Glu Gly Gly Cys Phe Val Leu His Ala Ser Ala
       210                 215                 220
   Ile Thr Gly Gln Asp Met Phe Asp Val Leu Cys Asp Thr Pro Glu Arg
   225                 230                 235                 240
   Thr Gln Leu Leu Asn Ser Asp Gly Gly Lys Val Gly Gly Gly Tyr Ser
                   245                 250                 255
   Met Ile Phe Gly Pro Asp Gly Gln Pro Leu Val Gly His Leu Pro Gln
               260                 265                 270
   Asp Thr Glu Gly Ile Leu Tyr Ala Asp Ile Asp Leu Ala Asn Ile Ser
           275                 280                 285
   Val Ala Lys Ala Ala Tyr Asp Pro Ser Gly His Tyr Ala Arg Gly Asp
       290                 295                 300
   Val Val Arg Leu Met Val Asn Arg Asn Pro Arg His Thr Ser Val Ala
   305                 310                 315                 320
   Phe Gly Gly Gly Ala Gly Glu Ala Ala Thr Trp Thr Glu Ala Lys Ala
                   325                 330                 335
   Glu
<210> 271
   <211> 966
   <212> DNA
   <213> Unknown
<220>
   <223> Obtained from an environmental sample
<400> 271
   atgtccagcg agaataacaa cgctacattc aaagttgccg cagttcaggc cacacctgtg     60
   tatcttgatc gtgaagcaac catcgacaag gcttgcgagt tgatcgctac tgctggcagc    120
   gaaggagctc gcctgattat ctttccagaa gcgttcatcc caacctatcc tgagtgggta    180
   tggggtattc cttctggtga gcaaggttta ctcaacgagc tctattcaga gttgctcacc    240
   aattcggtca cgattcccag cgacgcgact gacagactgt gcgaggccgc gaagcttgct    300
   aatgcctacg tggtgatggg aatgagtgaa cggaatgtcg aagcgagtgg tgcaagcctg    360
   tataatacgc tcttgtacat agatgcgcag ggggagattt tagggaaaca tcgaaagttg     420
   gtaccaacgg gcggtgagcg cctggtatgg gcgcaaggtg atggcagcac gctgcaggtc    480
   tacgatactc cattgggaaa actcggtggt ttaatttgct gggaaaatta tatgccactg    540
   gcacgctacg ctatgtatgc ctggggtaca caaatctatg tcgcagcaac gtgggatcgc    600
   ggccaaccct ggctctcaac gttacggcat attgccaaag aaggcagggt atacgtaatt    660
   ggttgctgta ttgcgatgcg taaagatgat attccagatc gttactccat gaagcagaag    720
   tattacgctg aaatggagga atggattaat attggtgaca gcgcgattgt caatcccgaa    780
   ggacacttta ttgcagggcc tgtgcgcaag caagaagaaa ttctttacgc ggagatcgat    840
   ccacgcatgg tgcaaggccc gaagtggatg ctcgatgtgg ctgggcacta tgcgagacca    900
   gatgtgttcc agttgacggt gcatacggat gtgaggcaga tgattcgggt ggaacatgat    960
   tcataa                                                               966
<210> 272
   <211> 321
   <212> PRT
   <213> Unknown
<220>
   <223> Obtained from an environmental sample
<400> 272
   Met Ser Ser Glu Asn Asn Asn Ala Thr Phe Lys Val Ala Ala Val Gln
    1               5                  10                  15
   Ala Thr Pro Val Tyr Leu Asp Arg Glu Ala Thr Ile Asp Lys Ala Cys
               20                  25                  30
   Glu Leu Ile Ala Thr Ala Gly Ser Glu Gly Ala Arg Leu Ile Ile Phe
           35                  40                  45
   Pro Glu Ala Phe Ile Pro Thr Tyr Pro Glu Trp Val Trp Gly Ile Pro
       50                  55                  60
   Ser Gly Glu Gln Gly Leu Leu Asn Glu Leu Tyr Ser Glu Leu Leu Thr
   65                  70                  75                  80
   Asn Ser Val Thr Ile Pro Ser Asp Ala Thr Asp Arg Leu Cys Glu Ala
                   85                  90                  95
   Ala Lys Leu Ala Asn Ala Tyr Val Val Met Gly Met Ser Glu Arg Asn
               100                 105                 110
   Val Glu Ala Ser Gly Ala Ser Leu Tyr Asn Thr Leu Leu Tyr Ile Asp
           115                 120                 125
   Ala Gln Gly Glu Ile Leu Gly Lys His Arg Lys Leu Val Pro Thr Gly
       130                 135                 140
   Gly Glu Arg Leu Val Trp Ala Gln Gly Asp Gly Ser Thr Leu Gln Val
   145                 150                 155                 160
   Tyr Asp Thr Pro Leu Gly Lys Leu Gly Gly Leu Ile Cys Trp Glu Asn
                   165                 170                 175
   Tyr Met Pro Leu Ala Arg Tyr Ala Met Tyr Ala Trp Gly Thr Gln Ile
               180                 185                 190
   Tyr Val Ala Ala Thr Trp Asp Arg Gly Gln Pro Trp Leu Ser Thr Leu
           195                 200                 205
   Arg His Ile Ala Lys Glu Gly Arg Val Tyr Val Ile Gly Cys Cys Ile
       210                 215                 220
   Ala Met Arg Lys Asp Asp Ile Pro Asp Arg Tyr Ser Met Lys Gln Lys
   225                 230                 235                 240
   Tyr Tyr Ala Glu Met Glu Glu Trp Ile Asn Ile Gly Asp Ser Ala Ile
                   245                 250                 255
   Val Asn Pro Glu Gly His Phe Ile Ala Gly Pro Val Arg Lys Gln Glu
               260                 265                 270
   Glu Ile Leu Tyr Ala Glu Ile Asp Pro Arg Met Val Gln Gly Pro Lys
           275                 280                 285
   Trp Met Leu Asp Val Ala Gly His Tyr Ala Arg Pro Asp Val Phe Gln
       290                 295                 300
   Leu Thr Val His Thr Asp Val Arg Gln Met Ile Arg Val Glu His Asp
   305                 310                 315                 320
   Ser
<210> 273
   <211> 1023
   <212> DNA
   <213> Unknown
<220>
   <223> Obtained from an environmental sample
<400> 273
   atgaataact caccaccaac catccgcgct gctgccattc agctcagtcc agttctgttt      60
   agtcgggatg ggactaccga gaaggtgttg caggcgatcg ctagtgctgc caaggaaggg     120
   gcacaactgg ttgtttttcc agaaactttc atcccctact acccctattt ctcattcatt     180
   caaccccctg tgttgatggg caaagagcac atgcggctct atgaggaagc cgtgacggtc    240
   ccgggtccgg tgacagatgc ggtcagtcga gcagcccgtt cttacggcat ggtggtagtg     300
   ctgggggtga atgagcgaga tggtggctca atttacaata cacagttgat tttcgatgct     360
   gacggcacat tgttgctgaa gcgacgcaaa atcaccccta cctatcatga gcgcatggtc     420
   tgggggcagg gagacggtgc tggattgaag gtattggata cagcagtcgg taaggtgggt     480
   gcgctggcat gttgggaaca ttacaatccc ctggcacgat ttgcgctgat ggcacagcat     540
   gagcagattc actgcgctca gttccccggt tctctggtgg gacaaatttt cactgatcag     600
   attgaggtaa cgattcggca tcatgcgttg gaatcgggtt gttttgtggt gaatgctact     660
   ggctggctct ctccagaaca ggtggcacaa atcaccacgg atgaaaagtt gcaacgggtg     720
   ctgagtggcg ggtgtaatac cgccattatt ggacctgaag gcaatcatct ctgtcctccc     780
   attaccgatg gtgagggcat agcgatcgcc gatctcgact tctcactaat caccaaacgc     840
   aaacgcatga tggattgcgt cggtcactac tcccgccctg acttgttgaa gctgcaactc     900
   aatgcaacgg catggtcggt gctggctggg gagcaggggg caggtgccag ggagcagggg     960
   ctaggtgtgc cggatgccat gctgtctacg cctaagccag aatactcaac actggatcag    1020
   tag                                                                  1023
<210> 274
   <211> 340
   <212> PRT
   <213> Unknown
<220>
   <223> Obtained from an environmental sample
<400> 274
   Met Asn Asn Ser Pro Pro Thr Ile Arg Ala Ala Ala Ile Gln Leu Ser
   1               5                   10                  15
   Pro Val Leu Phe Ser Arg Asp Gly Thr Thr Glu Lys Val Leu Gln Ala
               20                  25                  30
   Ile Ala Ser Ala Ala Lys Glu Gly Ala Gln Leu Val Val Phe Pro Glu
           35                  40                  45
   Thr Phe Ile Pro Tyr Tyr Pro Tyr Phe Ser Phe Ile Gln Pro Pro Val
       50                  55                  60
   Leu Met Gly Lys Glu His Met Arg Leu Tyr Glu Glu Ala Val Thr Val
   65                  70                  75                  80
   Pro Gly Pro Val Thr Asp Ala Val Ser Arg Ala Ala Arg Ser Tyr Gly
                   85                  90                  95
   Met Val Val Val Leu Gly Val Asn Glu Arg Asp Gly Gly Ser Ile Tyr
               100                 105                 110
   Asn Thr Gln Leu Ile Phe Asp Ala Asp Gly Thr Leu Leu Leu Lys Arg
           115                 120                 125
   Arg Lys Ile Thr Pro Thr Tyr His Glu Arg Met Val Trp Gly Gln Gly
       130                 135                 140
   Asp Gly Ala Gly Leu Lys Val Leu Asp Thr Ala Val Gly Lys Val Gly
   145                 150                 155                 160
   Ala Leu Ala Cys Trp Glu His Tyr Asn Pro Leu Ala Arg Phe Ala Leu
                   165                 170                 175
   Met Ala Gln His Glu Gln Ile His Cys Ala Gln Phe Pro Gly Ser Leu
               180                 185                 190
   Val Gly Gln Ile Phe Thr Asp Gln Ile Glu Val Thr Ile Arg His His
           195                 200                 205
   Ala Leu Glu Ser Gly Cys Phe Val Val Asn Ala Thr Gly Trp Leu Ser
       210                 215                 220
   Pro Glu Gln Val Ala Gln Ile Thr Thr Asp Glu Lys Leu Gln Arg Val
   225                 230                 235                 240
   Leu Ser Gly Gly Cys Asn Thr Ala Ile Ile Gly Pro Glu Gly Asn His
                   245                 250                 255
   Leu Cys Pro Pro Ile Thr Asp Gly Glu Gly Ile Ala Ile Ala Asp Leu
               260                 265                 270
   Asp Phe Ser Leu Ile Thr Lys Arg Lys Arg Met Met Asp Cys Val Gly
           275                 280                 285
   His Tyr Ser Arg Pro Asp Leu Leu Lys Leu Gln Leu Asn Ala Thr Ala
       290                 295                 300
   Trp Ser Val Leu Ala Gly Glu Gln Gly Ala Gly Ala Arg Glu Gln Gly
   305                 310                 315                 320
   Leu Gly Val Pro Asp Ala Met Leu Ser Thr Pro Lys Pro Glu Tyr Ser
                   325                 330                 335
   Thr Leu Asp Gln
               340
<210> 275
   <211> 849
   <212> DNA
   <213> Unknown
<220>
   <223> Obtained from an environmental sample
<400> 275
   atggagacgg ctcacaaagc aaaggtcgat ttccttgtgc tgggtgagac gtggctctca      60
   ggctacccgg cttggctgga ccactgcccc gatgttggcc ggtgggatta tgaaccgatg     120
   aaaaaagtgt atttgagatt tcgacaaagt gctatttctg ttcctggcaa agaatttgat     180
   ttccttactg gcctctgtaa aaaatattca caaacgcttg ccatcggtgt taatgagaaa     240
   gtagatcatg gggtaggtaa tggtaccatt tataattcat ttctactgat tgattctgat      300
   ggaacactgt tgaatcatca tcgcaagtta gttcccactt ttactgagaa attattatac     360
   ggccatggag atggccatgg gctgaagtcg atggatactt cggtgggaag aatcggaggg     420
   agcatttgtt gggaacattg gatgccacta tgcagacaag cacttcatga tgcaggtgag     480
   caaatccatg ttgccctttg gccgactgtt catgacatcc atcaagtggc aagtagaagc     540
   tatgcatttg aagggcgctg ctttgtattg gctgccgggc agatttttgc tgctaaagat     600
   tttccaaagg aacttgtctt accagactat ctaaagcaaa atccggatca gctcattttg     660
   aatgggggga gctgcgtgat cggccctgat gggaaatatt tgattgagcc cgtgtttgat     720
   cgggaagaac tgattgtgtg tgaacttgac cttgacgaag cttataaaga aagaatgacg     780
   atggacgttt caggtcacta ccaaagacga gacgttttca gttttgacgt gaaccaacat     840
   cgacattga                                                             849
<210> 276
   <211> 310
   <212> PRT
   <213> Unknown
<220>
   <223> Obtained from an environmental sample
<400> 276
   Met Thr Lys Leu Lys Ile Ala Ile Gly Gln Phe Ser Ser Asn His Leu
   1               5                   10                  15
   Asp Leu Lys Cys Ser Leu Glu Lys Leu Glu Lys Ile Met Glu Thr Ala
               20                  25                  30
   His Lys Ala Lys Val Asp Phe Leu Val Leu Gly Glu Thr Trp Leu Ser
           35                  40                  45
   Gly Tyr Pro Ala Trp Leu Asp His Cys Pro Asp Val Gly Arg Trp Asp
       50                  55                  60
   Tyr Glu Pro Met Lys Lys Val Tyr Leu Arg Phe Arg Gln Ser Ala Ile
   65                  70                  75                  80
   Ser Val Pro Gly Lys Glu Phe Asp Phe Leu Thr Gly Leu Cys Lys Lys
                   85                  90                  95
   Tyr Ser Gln Thr Leu Ala Ile Gly Val Asn Glu Lys Val Asp His Gly
               100                 105                 110
   Val Gly Asn Gly Thr Ile Tyr Asn Ser Phe Leu Leu Ile Asp Ser Asp
           115                 120                 125
   Gly Thr Leu Leu Asn His His Arg Lys Leu Val Pro Thr Phe Thr Glu
       130                 135                 140
   Lys Leu Leu Tyr Gly His Gly Asp Gly His Gly Leu Lys Ser Met Asp
   145                 150                 155                 160
   Thr Ser Val Gly Arg Ile Gly Gly Ser Ile Cys Trp Glu His Trp Met
                   165                 170                 175
   Pro Leu Cys Arg Gln Ala Leu His Asp Ala Gly Glu Gln Ile His Val
               180                 185                 190
   Ala Leu Trp Pro Thr Val His Asp Ile His Gln Val Ala Ser Arg Ser
           195                 200                 205
   Tyr Ala Phe Glu Gly Arg Cys Phe Val Leu Ala Ala Gly Gln Ile Phe
       210                 215                 220
   Ala Ala Lys Asp Phe Pro Lys Glu Leu Val Leu Pro Asp Tyr Leu Lys
   225                 230                 235                 240
   Gln Asn Pro Asp Gln Leu Ile Leu Asn Gly Gly Ser Cys Val Ile Gly
                   245                 250                 255
   Pro Asp Gly Lys Tyr Leu Ile Glu Pro Val Phe Asp Arg Glu Glu Leu
               260                 265                 270
   Ile Val Cys Glu Leu Asp Leu Asp Glu Ala Tyr Lys Glu Arg Met Thr
           275                 280                 285
   Met Asp Val Ser Gly His Tyr Gln Arg Arg Asp Val Phe Ser Phe Asp
       290                 295                 300
   Val Asn Gln His Arg His
   305                 310
<210> 277
   <211> 1056
   <212> DNA
   <213> Unknown
<220>
   <223> Obtained from an environmental sample
<400> 277
   atgccaaccc ccagcgatca tttcaaaatc gccgctgttc aggcctcgcc cgtgtttctg      60
   gaccgggagg ccactgtgga aaaggcctgc cggttgatcg ccgaagccgc aaagcagggc     120
   gcccgcctca tcgtctttcc ggaatctttc atcccgacct acccggactg ggtatgggcc     180
   gttcccccgg gaagggaaag aatcctgaac cagctgtatt ctgaattcct ggccaatgcc     240
   gtcgatgttc ccggcgcggc gaccgaacaa cttgcccagg ctgcacgaat ggccggcgcc     300
   tatgtgatta tgggcgtcac cgaaagagac acctcggcca gcggggccag cctctacaac     360
   accctgctct acttcagccc cgaaggcatc ctaatgggca aacaccggaa gctggttccc     420
   acggggggcg aacggctggt ctgggcctac ggagacggca gcacgctgga ggtctacgac     480
   actccgctgg gaaagatcgg cgggctgatc tgctgggaga actacatgcc cctggcccgg     540
   tacacgatgt acgcctgggg cacccagatt tacatcgccg ccacctggga ccgcggggaa     600
   ccgtggctct ccaccctgcg gcatatcgcc aaggaaggaa gggtctacgt catcgggtgc     660
   tgcatcgccc tgcgccaggg ggatatcccg gaccggttcg agtacaaggg aaaattttat     720
   tccgggtccc gggagtggat caatgagggc gacagcgcca tcgtgaaccc ggacggggaa     780
   ttcatcgccg ggccggtgcg gatgaaggag gagatcctgt atgccgagat agacccccgg     840
   cagatgcggg gccccaagtg gatgctcgat gtggccggtc attacgcccg gccggatatc     900
   ttcgagctca tcgtccaccg gaatccccac ccgatgatca aaatcgccga agacaggggc     960
   gcggggatcg cctcaagttt gattcgcccc cgccctaacc ttcccccatc aagggggagg    1020
   aaatcggcaa gaagcaaacg caagcccaaa aaatga                              1056
<210> 278
   <211> 351
   <212> PRT
   <213> Unknown
<220>
   <223> Obtained from an environmental sample
<400> 278
   Met Pro Thr Pro Ser Asp His Phe Lys Ile Ala Ala Val Gln Ala Ser
   1               5                   10                  15
   Pro Val Phe Leu Asp Arg Glu Ala Thr Val Glu Lys Ala Cys Arg Leu
               20                  25                  30
   Ile Ala Glu Ala Ala Lys Gln Gly Ala Arg Leu Ile Val Phe Pro Glu
           35                  40                  45
   Ser Phe Ile Pro Thr Tyr Pro Asp Trp Val Trp Ala Val Pro Pro Gly
       50                  55                  60
   Arg Glu Arg Ile Leu Asn Gln Leu Tyr Ser Glu Phe Leu Ala Asn Ala
   65                  70                  75                  80
   Val Asp Val Pro Gly Ala Ala Thr Glu Gln Leu Ala Gln Ala Ala Arg
                   85                  90                  95
   Met Ala Gly Ala Tyr Val Ile Met Gly Val Thr Glu Arg Asp Thr Ser
               100                 105                 110
   Ala Ser Gly Ala Ser Leu Tyr Asn Thr Leu Leu Tyr Phe Ser Pro Glu
           115                 120                 125
   Gly Ile Leu Met Gly Lys His Arg Lys Leu Val Pro Thr Gly Gly Glu
       130                 135                 140
   Arg Leu Val Trp Ala Tyr Gly Asp Gly Ser Thr Leu Glu Val Tyr Asp
   145                 150                 155                 160
   Thr Pro Leu Gly Lys Ile Gly Gly Leu Ile Cys Trp Glu Asn Tyr Met
                   165                 170                 175
   Pro Leu Ala Arg Tyr Thr Met Tyr Ala Trp Gly Thr Gln Ile Tyr Ile
               180                 185                 190
   Ala Ala Thr Trp Asp Arg Gly Glu Pro Trp Leu Ser Thr Leu Arg His
           195                 200                 205
   Ile Ala Lys Glu Gly Arg Val Tyr Val Ile Gly Cys Cys Ile Ala Leu
       210                 215                 220
   Arg Gln Gly Asp Ile Pro Asp Arg Phe Glu Tyr Lys Gly Lys Phe Tyr
   225                 230                 235                 240
   Ser Gly Ser Arg Glu Trp Ile Asn Glu Gly Asp Ser Ala Ile Val Asn
                   245                 250                 255
   Pro Asp Gly Glu Phe Ile Ala Gly Pro Val Arg Met Lys Glu Glu Ile
               260                 265                 270
   Leu Tyr Ala Glu Ile Asp Pro Arg Gln Met Arg Gly Pro Lys Trp Met
           275                 280                 285
   Leu Asp Val Ala Gly His Tyr Ala Arg Pro Asp Ile Phe Glu Leu Ile
       290                 295                 300
   Val His Arg Asn Pro His Pro Met Ile Lys Ile Ala Glu Asp Arg Gly
   305                 310                 315                 320
   Ala Gly Ile Ala Ser Ser Leu Ile Arg Pro Arg Pro Asn Leu Pro Pro
                   325                 330                 335
   Ser Arg Gly Arg Lys Ser Ala Arg Ser Lys Arg Lys Pro Lys Lys
               340                 345                 350
<210> 279
   <211> 1014
   <212> DNA
   <213> Unknown
<220>
   <223> Obtained from an environmental sample
<400> 279
   atgggcatta cccatcccaa atacaaagtc gctgcagtcc aggccgcgcc ggtctggctg      60
   gacctggatg ccacggtgga caagtgcatc cgcctgatcc aggaggccgc tgacaagggc     120
   tgcaagctga tcgcgtttcc ggagacgttc attcccggct atccctggca catctggatg     180
   ggtgcgccgg cctgggccat cggccggggc tttgtgcagc ggtatttcga caactccctg     240
   tcctatgaca gcccgcaggc cgaaaagctg cgccaggccg tcaaggccgc aggcatcacc     300
   gcgtcgctgg gactgtcgga gcgctcgggc ggcagtctct acatcgcgca gtggctcatt     360
   ggccccgacg gcgaaacgat ctcgcagcgg cgtaagctgc ggcccacgca ttccgaacgc     420
   accgtcttcg gcgacggcga tgggagcgac ctcaaggtgc acgacacgcc gctgggccgt     480
   gtgggtgagc tggcgtgctg ggagaacatc ctgtcgctga acaagtacgc catgttctcg     540
   cagcacgagc aggtgcacat tgcagcctgg cctagcttct ccacctacga gccctttgca     600
   catgccctgg gctgggaagt gaacaacgca gtcagcaagg tgtacgcagt ggaaggcggg     660
   tgtttcgtgg tggcaccctg cgccatcatt tccaaggaga tggtcgatga actgtgcgac     720
   accccggaca agcacaccct gacccatgtg ggaggcggcc acgcggtgat ctacggcccg     780
   gatggcgccc ccctggcgga caagctgcct gaagacgccg agggcctgct gatcgcggag     840
   atcgacctcg ggatgattgg cgtggccaag aacgccatgg acccggtagg gcactactcg     900
   cgccccgacg tgcaccgcct gcttctcaac cgcaagaagg cagcgcaggt ggagcacttt     960
   gcgctgccgg tggacgccat cgattccgag ccccaagcca ccacggcgca ctga          1014
<210> 280
   <211> 337
   <212> PRT
   <213> Unknown
<220>
   <223> Obtained from an environmental sample
<400> 280
   Met Gly Ile Thr His Pro Lys Tyr Lys Val Ala Ala Val Gln Ala Ala
   1               5                   10                  15
   Pro Val Trp Leu Asp Leu Asp Ala Thr Val Asp Lys Cys Ile Arg Leu
               20                  25                  30
   Ile Gln Glu Ala Ala Asp Lys Gly Cys Lys Leu Ile Ala Phe Pro Glu
           35                  40                  45
   Thr Phe Ile Pro Gly Tyr Pro Trp His Ile Trp Met Gly Ala Pro Ala
       50                  55                  60
   Trp Ala Ile Gly Arg Gly Phe Val Gln Arg Tyr Phe Asp Asn Ser Leu
   65                  70                  75                  80
   Ser Tyr Asp Ser Pro Gln Ala Glu Lys Leu Arg Gln Ala Val Lys Ala
                   85                  90                  95
   Ala Gly Ile Thr Ala Ser Leu Gly Leu Ser Glu Arg Ser Gly Gly Ser
               100                 105                 110
   Leu Tyr Ile Ala Gln Trp Leu Ile Gly Pro Asp Gly Glu Thr Ile Ser
           115                 120                 125
   Gln Arg Arg Lys Leu Arg Pro Thr His Ser Glu Arg Thr Val Phe Gly
       130                 135                 140
   Asp Gly Asp Gly Ser Asp Leu Lys Val His Asp Thr Pro Leu Gly Arg
   145                 150                 155                 160
   Val Gly Glu Leu Ala Cys Trp Glu Asn Ile Leu Ser Leu Asn Lys Tyr
                   165                 170                 175
   Ala Met Phe Ser Gln His Glu Gln Val His Ile Ala Ala Trp Pro Ser
               180                 185                 190
   Phe Ser Thr Tyr Glu Pro Phe Ala His Ala Leu Gly Trp Glu Val Asn
           195                 200                 205
   Asn Ala Val Ser Lys Val Tyr Ala Val Glu Gly Gly Cys Phe Val Val
       210                 215                 220
   Ala Pro Cys Ala Ile Ile Ser Lys Glu Met Val Asp Glu Leu Cys Asp
   225                 230                 235                 240
   Thr Pro Asp Lys His Thr Leu Thr His Val Gly Gly Gly His Ala Val
                   245                 250                 255
   Ile Tyr Gly Pro Asp Gly Ala Pro Leu Ala Asp Lys Leu Pro Glu Asp
               260                 265                 270
   Ala Glu Gly Leu Leu Ile Ala Glu Ile Asp Leu Gly Met Ile Gly Val
           275                 280                 285
   Ala Lys Asn Ala Met Asp Pro Val Gly His Tyr Ser Arg Pro Asp Val
       290                 295                 300
   His Arg Leu Leu Leu Asn Arg Lys Lys Ala Ala Gln Val Glu His Phe
   305                 310                 315                 320
   Ala Leu Pro Val Asp Ala Ile Asp Ser Glu Pro Gln Ala Thr Thr Ala
                   325                 330                 335
   His
<210> 281
   <211> 936
   <212> DNA
   <213> Unknown
<220>
   <223> Obtained from an environmental sample
<400> 281
   atgtccaaga tcgccgtcgt ccaagagcct ccggtgctgc tcgatcgcgc cgccaccctc      60
   gagcgcgccg tctcggccat cgagcgggcg gccgacgtgg gggcgcacct cgtcgtgttc     120
   cccgagacgt acgtcccggg gtaccccgac tgggtctggc ggacgcgccc cgacgacttc     180
   aagctcgcgg gcgcgctgca cgagcgcctc ctcgcgaacg cggtcgacct cgaaaaggac     240
   cagctcgcgc cgctccgcga ggccgcgcgc cggcgaggcg tcaccatcgc gtgcggcgtg     300
   aacgagcgcg aggggagcca cggccgcgcg accctctaca acaccgtcgt cgtcgtcgga     360
   cccgacggcg cgatcctcaa ccgccaccgc aagctcgtcc ccacgaaccc cgagcgcatg     420
   gtctggggcc cgggtgacgc gagcgggctg cgcgtcgtcg acacgccggc cggccgcgtg     480
   ggggcgctca tctgctggga gaactacatg ccgctcgcgc gcttcgcgct ctacgcgcag     540
   ggcgtcgagg tctacctcgc gccgacgtgg gatcacggcg acacttggct cgcctccatg     600
   cggcacatcg cgcgcgaggc gcgcgcctgg gtcgtctcgg gggccatctg catgcaggcg     660
   aaggacgtcc ccgccgactt cccgcagcgc gcggcgatct accccgacga ggaggagtgg     720
   ctcaaccccg gcgatgccgt cgtcgtcgat cccaccggcg ccgtcgccgc cggcccgctg     780
   caccgcgagc gcggcatcct ctacgccgag tgcgatcccg cgcgggcgtc gctcgcccgc     840
   cgcacgctcg acgtctccgg gcactacgga cggcccgacg tctttcacct gcagatcgac     900
   cgcacaccgc gcgtgccggc gtcgttccgg gactga                               936
<210> 282
   <211> 311
   <212> PRT
   <213> Unknown
<220>
   <223> Obtained from an environmental sample
<400> 282
   Met Ser Lys Ile Ala Val Val Gln Glu Pro Pro Val Leu Leu Asp Arg
   1 5 10 15
   Ala Ala Thr Leu Glu Arg Ala Val Ser Ala Ile Glu Arg Ala Ala Asp
   20 25 30
   Val Gly Ala His Leu Val Val Phe Pro Glu Thr Tyr Val Pro Gly Tyr
   35 40 45
   Pro Asp Trp Val Trp Arg Thr Arg Pro Asp Asp Phe Lys Leu Ala Gly
   50 55 60
   Ala Leu His Glu Arg Leu Leu Ala Asn Ala Val Asp Leu Glu Lys Asp
   65 70 75 80
   Gln Leu Ala Pro Leu Arg Glu Ala Ala Arg Arg Arg Gly Val Thr Ile
   85 90 95
   Ala Cys Gly Val Asn Glu Arg Glu Gly Ser His Gly Arg Ala Thr Leu
   100 105 110
   Tyr Asn Thr Val Val Val Val Gly Pro Asp Gly Ala Ile Leu Asn Arg
   115 120 125
   His Arg Lys Leu Val Pro Thr Asn Pro Glu Arg Met Val Trp Gly Pro
   130 135 140
   Gly Asp Ala Ser Gly Leu Arg Val Val Asp Thr Pro Ala Gly Arg Val
   145 150 155 160
   Gly Ala Leu Ile Cys Trp Glu Asn Tyr Met Pro Leu Ala Arg Phe Ala
   165 170 175
   Leu Tyr Ala Gln Gly Val Glu Val Tyr Leu Ala Pro Thr Trp Asp His
   180 185 190
   Gly Asp Thr Trp Leu Ala Ser Met Arg His Ile Ala Arg Glu Ala Arg
   195 200 205
   Ala Trp Val Val Ser Gly Ala Ile Cys Met Gln Ala Lys Asp Val Pro
   210 215 220
   Ala Asp Phe Pro Gln Arg Ala Ala Ile Tyr Pro Asp Glu Glu Glu Trp
   225 230 235 240
   Leu Asn Pro Gly Asp Ala Val Val Val Asp Pro Thr Gly Ala Val Ala
   245 250 255
   Ala Gly Pro Leu His Arg Glu Arg Gly Ile Leu Tyr Ala Glu Cys Asp
   260 265 270
   Pro Ala Arg Ala Ser Leu Ala Arg Arg Thr Leu Asp Val Ser Gly His
   275 280 285
   Tyr Gly Arg Pro Asp Val Phe His Leu Gln Ile Asp Arg Thr Pro Arg
   290 295 300
   Val Pro Ala Ser Phe Arg Asp
   305 310
<210> 283
   <211> 1017
   <212> DNA
   <213> Unknown
<220>
   <223> Obtained from an environmental sample
<400> 283
   atgggcatcg aacatccgaa gtacaaggtc gcggtggtgc aggcggcacc ggcctggctc      60
   gatctcgacg cgtcgatcga caagaccatc gggctgatcg aggaggccgc ccaaaaaggc     120
   gccaagctga ttgcattccc cgaggccttc atccccggtt acccctggca catctggatg     180
   gactcgccgg cctgggcgat cggccgcggt ttcgtgcagc gctattttga caattcgctc     240
   gcctatgaca gcccgcaggc cgagaaactg cgcgcggcgg ttcgcaaggc aaagctcacg     300
   gccgtgatcg ggctgtccga gcgcgacggc ggcagtcttt acctcgcgca atggctgatc     360
   gggcccgacg gtgagaccat cgccaagcgc cgcaagctgc ggccgacaca tgcggagcgc     420
   acggtatacg gcgagggcga cggcagcgac ctcgcggttc acaaccgtcc ggacattggc     480
   cgccttggcg cgctctgctg ctgggagcat ctccagccgc tgtcgaaata cgcgatgtac     540
   gcgcagaacg agcaggtgca tgtcgcggcc tggccgagct tttcgctgta cgatcccttc     600
   gcggtggcgc tcggcgccga agtgaacaac gcggcctcgc gcgtctatgc ggtcgaaggc     660
   tcctgcttcg tgctggcgcc gtgcgcgacg gtctcgcagg ccatgatcga cgaactctgc     720
   gaccggccgg acaagcacgc gctgttgcat gtcggcggcg gttttgccgc gatctacggt     780
   cctgacggca gccagatcgg cgacaaactc gctcccgacc aggaagggtt gctgatcgcg     840
   gagatcgatc tcggcgccat cggcgtcgcc aagaatgcgg cggatcccgc cgggcattat     900
   tcgcggcctg acgtgacgcg actgttgctc aacaagaagc cgtacaagcg cgtcgagcag     960
   ttttcgccgc cggccgaggc gctcgagccg acggatatcg cagcagcagc aagctaa       1017
<210> 284
   <211> 338
   <212> PRT
   <213> Unknown
<220>
   <223> obtained from an environmental sample
<400> 284
   Met Gly Ile Glu His Pro Lys Tyr Lys Val Ala Val Val Gln Ala Ala
   1               5                   10                  15
   Pro Ala Trp Leu Asp Leu Asp Ala Ser Ile Asp Lys Thr Ile Gly Leu
                   20                      25                      30
   Ile Glu Glu Ala Ala Gln Lys Gly Ala Lys Leu Ile Ala Phe Pro Glu
           35                  40                  45
   Ala Phe Ile Pro Gly Tyr Pro Trp His Ile Trp Met Asp Ser Pro Ala
       50                  55                  60
   Trp Ala Ile Gly Arg Gly Phe Val Gln Arg Tyr Phe Asp Asn Ser Leu
   65                  70                  75                  80
   Ala Tyr Asp Ser Pro Gln Ala Glu Lys Leu Arg Ala Ala Val Arg Lys
                   85                  90                  95
   Ala Lys Leu Thr Ala Val Ile Gly Leu Ser Glu Arg Asp Gly Gly Ser
               100                 105                 110
   Leu Tyr Leu Ala Gln Trp Leu Ile Gly Pro Asp Gly Glu Thr Ile Ala
           115                 120                 125
   Lys Arg Arg Lys Leu Arg Pro Thr His Ala Glu Arg Thr Val Tyr Gly
       130                 135                 140
   Glu Gly Asp Gly Ser Asp Leu Ala Val His Asn Arg Pro Asp Ile Gly
   145                 150                 155                 160
   Arg Leu Gly Ala Leu Cys Cys Trp Glu His Leu Gln Pro Leu Ser Lys
                   165                 170                 175
   Tyr Ala Met Tyr Ala Gln Asn Glu Gln Val His Val Ala Ala Trp Pro
               180                 185                 190
   Ser Phe Ser Leu Tyr Asp Pro Phe Ala Val Ala Leu Gly Ala Glu Val
           195                 200                 205
   Asn Asn Ala Ala Ser Arg Val Tyr Ala Val Glu Gly Ser Cys Phe Val
       210                 215                 220
   Leu Ala Pro Cys Ala Thr Val Ser Gln Ala Met Ile Asp Glu Leu Cys
   225                 230                 235                 240
   Asp Arg Pro Asp Lys His Ala Leu Leu His Val Gly Gly Gly Phe Ala
                   245                 250                 255
   Ala Ile Tyr Gly Pro Asp Gly Ser Gln Ile Gly Asp Lys Leu Ala Pro
               260                 265                 270
   Asp Gln Glu Gly Leu Leu Ile Ala Glu Ile Asp Leu Gly Ala Ile Gly
           275                 280                 285
   Val Ala Lys Asn Ala Ala Asp Pro Ala Gly His Tyr Ser Arg Pro Asp
       290                 295                 300
   Val Thr Arg Leu Leu Leu Asn Lys Lys Pro Tyr Lys Arg Val Glu Gln
   305                 310                 315                 320
   Phe Ser Pro Pro Ala Glu Ala Leu Glu Pro Thr Asp Ile Ala Ala Ala
                   325                 330                 335
   Ala Ser
<210> 285
   <211> 918
   <212> DNA
   <213> Unknown
<220>
   <223> Obtained from an environmental sample
<400> 285
   atgaatacta atctagtaaa ggtcgcggcg gctcaagttg ctccccattt tctcaatttg      60
   agcaatacgg tggaaaaaac ctgcaactta atttctgaag caggcaaaaa tggagcaaag     120
   ctaatcgtat ttccagaagc cttcatctct ggttatcccg attgggtctg gcttattccc     180
   aatgcgaatt ctgcaatgct ggatgattta taccaggaat tggttgagaa cgcagtaacg     240
   atccctgata caacaacaca taaactatgt caggctgcaa aagatgcagg ggtatatgtt     300
   gcggtaggta tacatgagag aaattcagaa gcaagtggct tcacgctttt caatacgctt     360
   ctatacatca acgatcaagg cgtaatcatc ggaaaacacc gaaaattaat ccctacaggg     420
   ggcgaacggc tggtctgggg gcagggtaat ggggatacac tttctgcatt cgatacagac     480
   ttcggcaaat taggaggatt gctttgttgg gaaaattata tgccactcgc gcgtcaagct     540
   atgtattccg ttggaactga agtatatgta gccccaacct gggactccag cgagaattgg     600
   ttgttaagca tgcgccatat tgcccgagag ggcggtatgt ttgtaattag tgtttgccag     660
   gctctccgaa aagacgacat ccctgatcag tatgaattta agaaactcta tcctgataat     720
   tcagaatgga tcaatagcgg taacagttgc atcatcaacc cgcgcggtga gattattgct     780
   ggaccaatct caaaccaaca agaaatactt tatgcagatt tagacctgag tttaattgca     840
   aaatctaaac gtatgttcga tgttactggg cattattccc ggccggatgt gtttagctat     900
   gaaatcaaca aaagctag                                                   918
<210> 286
   <211> 305
   <212> PRT
   <213> Unknown
<220>
   <223> Obtained from an environmental sample
<400> 286
   Met Asn Thr Asn Leu Val Lys Val Ala Ala Ala Gln Val Ala Pro His
    1               5                  10                  15
   Phe Leu Asn Leu Ser Asn Thr Val Glu Lys Thr Cys Asn Leu Ile Ser
               20                  25                  30
   Glu Ala Gly Lys Asn Gly Ala Lys Leu Ile Val Phe Pro Glu Ala Phe
           35                  40                  45
   Ile Ser Gly Tyr Pro Asp Trp Val Trp Leu Ile Pro Asn Ala Asn Ser
       50                  55                  60
   Ala Met Leu Asp Asp Leu Tyr Gln Glu Leu Val Glu Asn Ala Val Thr
   65                  70                  75                  80
   Ile Pro Asp Thr Thr Thr His Lys Leu Cys Gln Ala Ala Lys Asp Ala
                   85                  90                  95
   Gly Val Tyr Val Ala Val Gly Ile His Glu Arg Asn Ser Glu Ala Ser
               100                 105                 110
   Gly Phe Thr Leu Phe Asn Thr Leu Leu Tyr Ile Asn Asp Gln Gly Val
           115                 120                 125
   Ile Ile Gly Lys His Arg Lys Leu Ile Pro Thr Gly Gly Glu Arg Leu
       130                 135                 140
   Val Trp Gly Gln Gly Asn Gly Asp Thr Leu Ser Ala Phe Asp Thr Asp
   145                 150                 155                 160
   Phe Gly Lys Leu Gly Gly Leu Leu Cys Trp Glu Asn Tyr Met Pro Leu
                   165                 170                 175
   Ala Arg Gln Ala Met Tyr Ser Val Gly Thr Glu Val Tyr Val Ala Pro
               180                 185                 190
   Thr Trp Asp Ser Ser Glu Asn Trp Leu Leu Ser Met Arg His Ile Ala
           195                 200                 205
   Arg Glu Gly Gly Met Phe Val Ile Ser Val Cys Gln Ala Leu Arg Lys
       210                 215                 220
   Asp Asp Ile Pro Asp Gln Tyr Glu Phe Lys Lys Leu Tyr Pro Asp Asn
   225                 230                 235                 240
   Ser Glu Trp Ile Asn Ser Gly Asn Ser Cys Ile Ile Asn Pro Arg Gly
                   245                 250                 255
   Glu Ile Ile Ala Gly Pro Ile Ser Asn Gln Gln Glu Ile Leu Tyr Ala
               260                 265                 270
   Asp Leu Asp Leu Ser Leu Ile Ala Lys Ser Lys Arg Met Phe Asp Val
           275                 280                 285
   Thr Gly His Tyr Ser Arg Pro Asp Val Phe Ser Tyr Glu Ile Asn Lys
       290                 295                 300
   Ser
   305
<210> 287
   <211> 936
   <212> DNA
   <213> Unknown
<220>
   <223> Obtained from an environmental sample
<400> 287
   gtgatcaagg tagcaatcgc ccaggtggca ccggtggttc tggacaaggc gcgcaccatt      60
   gagaaagcgg taggaattat tcgcgctgcc gcgcaagagg gcattgagct cctggttttc     120
   ccggagacgt ttatcccgac ctatccagcc tgggtatggc gcttgcgtcc gggtactgat     180
   tacggcctga gcgaggaact gcacgcgctc ctgctggata attcggtaga tatggagagc     240
   aaggacctgg agccattgca agctgttgct gcagagacca gcatgaccgt ggtaataggt     300
   atgaacgagc gagacggccg attcagccgg ggtacaatct acaatgccct ggttgtgatc     360
   ggtccaggtg gcacgatcct gaacaggcac cgcaagctta tgcccaccaa ccccgagcgt     420
   atggtttggg gtatgggcga tgccagcggg ctgaaggtag tggaaatgtc ttacgggcgc     480
   ctgggtgggc tgatttgctg ggagaatttc atgcctctcg cgcgctatgg cttgtatgcc     540
   cagggtgtgg agatttacgt ggcgcccacc tatgaccagg gcgacggctg ggtcggcagc     600
   atgcagcata tagcccggga gggtcgttgc tgggtactct cggccgggac ccttttgcgt     660
   ggcagtgatt ttctgccgga ttttccgggc aagaccgagt tatatcccga tgaccaggag      720
   tgggtgaatc cgggtggctc ggtgatcgtg gcaccggggg gagagattgt ggccggcccc      780
   atgtatcgcg acgaaggtct gctggtctgc gagttggatg cgacgcttag tgtccgcggc      840
   aagcgctcgc tggatgtggc cggccattac tcccggccgg atttgtttga actggaaata      900
   gatggcgacc cgctggaacc catagagtgg gattga                                936
<210> 288
   <211> 311
   <212> PRT
   <213> Unknown
<220>
   <223> Obtained from an environmental sample
<400> 288
   Val Ile Lys Val Ala Ile Ala Gln Val Ala Pro Val Val Leu Asp Lys
   1               5                   10                  15
   Ala Arg Thr Ile Glu Lys Ala Val Gly Ile Ile Arg Ala Ala Ala Gln
               20                  25                  30
   Glu Gly Ile Glu Leu Leu Val Phe Pro Glu Thr Phe Ile Pro Thr Tyr
           35                  40                  45
   Pro Ala Trp Val Trp Arg Leu Arg Pro Gly Thr Asp Tyr Gly Leu Ser
       50                  55                  60
   Glu Glu Leu His Ala Leu Leu Leu Asp Asn Ser Val Asp Met Glu Ser
   65                  70                  75                  80
   Lys Asp Leu Glu Pro Leu Gln Ala Val Ala Ala Glu Thr Ser Met Thr
                   85                  90                  95
   Val Val Ile Gly Met Asn Glu Arg Asp Gly Arg Phe Ser Arg Gly Thr
               100                 105                 110
   Ile Tyr Asn Ala Leu Val Val Ile Gly Pro Gly Gly Thr Ile Leu Asn
           115                 120                 125
   Arg His Arg Lys Leu Met Pro Thr Asn Pro Glu Arg Met Val Trp Gly
       130                 135                 140
   Met Gly Asp Ala Ser Gly Leu Lys Val Val Glu Met Ser Tyr Gly Arg
   145                 150                 155                 160
   Leu Gly Gly Leu Ile Cys Trp Glu Asn Phe Met Pro Leu Ala Arg Tyr
                   165                 170                 175
   Gly Leu Tyr Ala Gln Gly Val Glu Ile Tyr Val Ala Pro Thr Tyr Asp
               180                 185                 190
   Gln Gly Asp Gly Trp Val Gly Ser Met Gln His Ile Ala Arg Glu Gly
           195                 200                 205
   Arg Cys Trp Val Leu Ser Ala Gly Thr Leu Leu Arg Gly Ser Asp Phe
       210                 215                 220
   Leu Pro Asp Phe Pro Gly Lys Thr Glu Leu Tyr Pro Asp Asp Gln Glu
   225                 230                 235                 240
   Trp Val Asn Pro Gly Gly Ser Val Ile Val Ala Pro Gly Gly Glu Ile
                   245                 250                 255
   Val Ala Gly Pro Met Tyr Arg Asp Glu Gly Leu Leu Val Cys Glu Leu
               260                 265                 270
   Asp Ala Thr Leu Ser Val Arg Gly Lys Arg Ser Leu Asp Val Ala Gly
           275                 280                 285
   His Tyr Ser Arg Pro Asp Leu Phe Glu Leu Glu Ile Asp Gly Asp Pro
       290                 295                 300
   Leu Glu Pro Ile Glu Trp Asp
   305                 310
<210> 289
   <211> 921
   <212> DNA
   <213> unknown
<220>
   <223> Obtained from an environmental sample
<400> 289
   atggtgacgg tggccgccgt acaggcaacg ccggtgttcc tcgaccgcga ggcgacctcg      60
   gacaaggtct gcgccttggt caaggaggcg gccggccacg gggcagaact gatcgtcttc     120
   cccgagtcct tcgtccctgc ctatccggac tgggtgtggc gcacccctgc ctggagtgac     180
   accgagttcg tgaagcgctt ctacgcgaac gcggtgaccg tccccggcgc gaccctcgag     240
   cgcatcggcg cagcggcggc ggaggcggag gcgtacgtcg tgatcggcgt gaccgagatc     300
   gacggcggaa ctctctacaa cacccttctc tacctgggcc cggacggaca gctgttgcaa     360
   cggcatcgca agctcatgcc caccggtggg gagcggaccg tgtggggaat gggagacggc     420
   tctgagctcg acgtcgtgag cacgccgttc ggcgtcgtcg gtgggttgtt gtgctgggag     480
   aactacatgc cgctcgcccg ggcggcgatc tacgcccagc actgtgacat ctacctggct     540
   ccgacatggg acaacagcga cacgtgggta gccacgttgc gtcacatcgc caaggagggg     600
   cggcagttcg tcatcggcgt cgccccgctg ctgcgcggct ccgacgtacc ggaggacctc     660
   cgcggcacgc tctacgggct gtcggacgac tggatgtcgc gcggctacac caccatcgtc     720
   gcaccaagcg gcgaggtgat cgccggcccg gtcctggagc gtgaggagat cctcttcgcg     780
   gacctcgacc tggccgacgt gcaggagcag agaaggatgt tcgaccctgt cggccactac     840
   tcacgacccg acgtcttcac gctccacgtc gacgcacgac cgaagagccc ggtcgtcttc     900
   gagagggatg caccgacctg a                                              921
<210> 290
   <211> 306
   <212> PRT
   <213> Unknown
<220>
   <223> Obtained from an environmental sample
<400> 290
   Met Val Thr Val Ala Ala Val Gln Ala Thr Pro Val Phe Leu Asp Arg
   1               5                   10                  15
   Glu Ala Thr Ser Asp Lys Val Cys Ala Leu Val Lys Glu Ala Ala Gly
               20                  25                  30
   His Gly Ala Glu Leu Ile Val Phe Pro Glu Ser Phe Val Pro Ala Tyr
           35                  40                  45
   Pro Asp Trp Val Trp Arg Thr Pro Ala Trp Ser Asp Thr Glu Phe Val
       50                  55                  60
   Lys Arg Phe Tyr Ala Asn Ala Val Thr Val Pro Gly Ala Thr Leu Glu
   65                  70                  75                  80
   Arg Ile Gly Ala Ala Ala Ala Glu Ala Glu Ala Tyr Val Val Ile Gly
                   85                  90                  95
   Val Thr Glu Ile Asp Gly Gly Thr Leu Tyr Asn Thr Leu Leu Tyr Leu
               100                 105                 110
   Gly Pro Asp Gly Gln Leu Leu Gln Arg His Arg Lys Leu Met Pro Thr
           115                 120                 125
   Gly Gly Glu Arg Thr Val Trp Gly Met Gly Asp Gly Ser Glu Leu Asp
       130                 135                 140
   Val Val Ser Thr Pro Phe Gly Val Val Gly Gly Leu Leu Cys Trp Glu
   145                 150                 155                 160
   Asn Tyr Met Pro Leu Ala Arg Ala Ala Ile Tyr Ala Gln His Cys Asp
                   165                 170                 175
   Ile Tyr Leu Ala Pro Thr Trp Asp Asn Ser Asp Thr Trp Val Ala Thr
               180                 185                 190
   Leu Arg His Ile Ala Lys Glu Gly Arg Gln Phe Val Ile Gly Val Ala
           195                 200                 205
   Pro Leu Leu Arg Gly Ser Asp Val Pro Glu Asp Leu Arg Gly Thr Leu
       210                 215                 220
   Tyr Gly Leu Ser Asp Asp Trp Met Ser Arg Gly Tyr Thr Thr Ile Val
   225                 230                 235                 240
   Ala Pro Ser Gly Glu Val Ile Ala Gly Pro Val Leu Glu Arg Glu Glu
                   245                 250                 255
   Ile Leu Phe Ala Asp Leu Asp Leu Ala Asp Val Gln Glu Gln Arg Arg
               260                 265                 270
   Met Phe Asp Pro Val Gly His Tyr Ser Arg Pro Asp Val Phe Thr Leu
           275                 280                 285
   His Val Asp Ala Arg Pro Lys Ser Pro Val Val Phe Glu Arg Asp Ala
       290                 295                 300
   Pro Thr
   305
<210> 291
   <211> 1002
   <212> DNA
   <213> Unknown
<220>
   <223> Obtained from an environmental sample
<400> 291
   atgatgaaaa caactgttac cgttgcctgc gttcaggccg cccccgtatt tatggattta      60
   gaaggcacca tagataaaac gatcaccctc atctctgaag ccgcacagaa aggcgcggag     120
   ctcatcgctt ttccggagac ctggataccc ggttacccgt ggttcttatg gctgaactcg     180
   cccgcgacaa atatgcccct ggtttatcaa tatcatcaga actctctggt gctggacagt     240
   gcccaggcga agcgaattgc ggatgctgca cagcagaata acatcactgt cgttctggga     300
   ttcagcgagc gcgatcatgg aagcctctat atctcacagt ggctgattgg cagcgacggg     360
   gaaactattg gcatccggcg caagctcaag gccacacacg tggagcgtac gctgttcggc     420
   gaaagcgacg gctcctccct gaccacctgg gagacacctc tgggtaacgt cggggccctc     480
   tgctgctggg agcacctgca gccgctgtct cgctatgcga tgtattccca gcatgaagag     540
   atccatatcg ctgcgtggcc cagcttcagt ctttacacca gcgcaactgc cgcgctcgga     600
   cctgacgtca acacggcggc ttcacgcctc tatgccgcgg aggggcagtg cttcgtgtta     660
   gccccatgtg ccgtggtttc tgatgagatg attgatttac tctgtcctga tgatgaccgg     720
   agagcgttac tcagtgccgg agggggacat gcccgtattt acggacctga tggaagagaa     780
   ctcgtcaccc ctctcgggga aaatgaggaa ggactgctta tcgctgagct cgactctgct     840
   gcgatcacct ttgccaaact ggcggcagat cccgtaggcc actattcccg ccctgacgtg     900
   acccgccttc tttttaatcc ttcagccaac aagactgtta ttaaacggca ttcgcctcct     960
   gagttaattg ccgaacaggc tccggaagaa gaggaggagt ag                       1002
<210> 292
   <211> 333
   <212> PRT
   <213> Unknown
<220>
   <223> Obtained from an environmental sample
<400> 292
   Met Met Lys Thr Thr Val Thr Val Ala Cys Val Gln Ala Ala Pro Val
    1               5                  10                  15
   Phe Met Asp Leu Glu Gly Thr Ile Asp Lys Thr Ile Thr Leu Ile Ser
               20                  25                  30
   Glu Ala Ala Gln Lys Gly Ala Glu Leu Ile Ala Phe Pro Glu Thr Trp
           35                  40                  45
   Ile Pro Gly Tyr Pro Trp Phe Leu Trp Leu Asn Ser Pro Ala Thr Asn
       50                  55                  60
   Met Pro Leu Val Tyr Gln Tyr His Gln Asn Ser Leu Val Leu Asp Ser
   65                  70                  75                  80
   Ala Gln Ala Lys Arg Ile Ala Asp Ala Ala Gln Gln Asn Asn Ile Thr
                   85                  90                  95
   Val Val Leu Gly Phe Ser Glu Arg Asp His Gly Ser Leu Tyr Ile Ser
               100                 105                 110
   Gln Trp Leu Ile Gly Ser Asp Gly Glu Thr Ile Gly Ile Arg Arg Lys
           115                 120                 125
   Leu Lys Ala Thr His Val Glu Arg Thr Leu Phe Gly Glu Ser Asp Gly
       130                 135                 140
   Ser Ser Leu Thr Thr Trp Glu Thr Pro Leu Gly Asn Val Gly Ala Leu
   145                 150                 155                 160
   Cys Cys Trp Glu His Leu Gln Pro Leu Ser Arg Tyr Ala Met Tyr Ser
                   165                 170                 175
   Gln His Glu Glu Ile His Ile Ala Ala Trp Pro Ser Phe Ser Leu Tyr
               180                 185                 190
   Thr Ser Ala Thr Ala Ala Leu Gly Pro Asp Val Asn Thr Ala Ala Ser
           195                 200                 205
   Arg Leu Tyr Ala Ala Glu Gly Gln Cys Phe Val Leu Ala Pro Cys Ala
       210                 215                 220
   Val Val Ser Asp Glu Met Ile Asp Leu Leu Cys Pro Asp Asp Asp Arg
   225                 230                 235                 240
   Arg Ala Leu Leu Ser Ala Gly Gly Gly His Ala Arg Ile Tyr Gly Pro
                   245                 250                 255
   Asp Gly Arg Glu Leu Val Thr Pro Leu Gly Glu Asn Glu Glu Gly Leu
               260                 265                 270
   Leu Ile Ala Glu Leu Asp Ser Ala Ala Ile Thr Phe Ala Lys Leu Ala
           275                 280                 285
   Ala Asp Pro Val Gly His Tyr Ser Arg Pro Asp Val Thr Arg Leu Leu
       290                 295                 300
   Phe Asn Pro Ser Ala Asn Lys Thr Val Ile Lys Arg His Ser Pro Pro
   305                 310                 315                 320
   Glu Leu Ile Ala Glu Gln Ala Pro Glu Glu Glu Glu Glu
                   325                 330
<210> 293
   <211> 1008
   <212> DNA
   <213> Unknown
<220>
   <223> Obtained from an environmental sample
<400> 293
   atgaaaaata tcaaaaactc agaaaaaagc agcacagtaa gagtcgctgc ggtacaaatc      60
   agtccggtgt tgtacaaccg cgaagctacc gttcaaaaag tagtcaacaa aatccttgaa     120
   ctaggaaaac aaggggtaca attcgccact tttccggaaa cgatagtgcc ttattatcct     180
   tatttctctt ttattcaggc gccttatgcc atgggcaaag aacacctgcg cttgcttgaa     240
   caatcagtta ctgttccgtc agccgcgacc gatgccataa gtgaggcggc aaaggaagcc     300
   aatatggtag tgtctattgg tgtcaatgaa cgagacggtg gtaccattta caatacgcaa     360
   ctcctttttg atgctgacgg aacattaatt cagcgcagac gtaaacttac accaacgtat     420
   catgaaagaa tgatttgggg acaaggtgac gcttcaggtc ttcgtgccac agacagcgct     480
   gttgggcgta tcgggcagtt ggcttgttgg gaacattaca atccattgtt ccgttatgct     540
   ttgattgctg atggagaaca aatccattct gccatgtatc ccggatcatt tttaggtgcg     600
   ttgcacggtg aacaaaccga aatcaatgta cgccaacacg ctttagaatc ggccagcttc     660
   gtcgtagtgg ctaccggttg gttggatgcc gatcaacaag cacaaattgc gaaagacacc     720
   ggtggaccaa tcggaccaat ttcgggaggt tgttttacag ccgttatagg ccctgacgga     780
   caactaatcg gggaagccct tacatcaggt gaaggggaag tgattgccga tattgatttg      840
   gcacaaattg atgcccgcaa aagattaatg gatgccagtg gtcactacaa ccgtcctgaa     900
   ttgttgagct tgcatatcga tcacactccg actgctccta tgcatgaaag agtagtttac     960
   actgagccgg gattagcaaa aagacaaaat gaaaattcat caaattaa                1008
<210> 294
   <211> 335
   <212> PRT
   <213> Unknown
<220>
   <223> Obtained from an environmental sample
<400> 294
   Met Lys Asn Ile Lys Asn Ser Glu Lys Ser Ser Thr Val Arg Val Ala
   1               5                   10                  15
   Ala Val Gln Ile Ser Pro Val Leu Tyr Asn Arg Glu Ala Thr Val Gln
               20                  25                  30
   Lys Val Val Asn Lys Ile Leu Glu Leu Gly Lys Gln Gly Val Gln Phe
           35                  40                  45
   Ala Thr Phe Pro Glu Thr Ile Val Pro Tyr Tyr Pro Tyr Phe Ser Phe
       50                  55                  60
   Ile Gln Ala Pro Tyr Ala Met Gly Lys Glu His Leu Arg Leu Leu Glu
   65                  70                  75                  80
   Gln Ser Val Thr Val Pro Ser Ala Ala Thr Asp Ala Ile Ser Glu Ala
                   85                  90                  95
   Ala Lys Glu Ala Asn Met Val Val Ser Ile Gly Val Asn Glu Arg Asp
               100                 105                 110
   Gly Gly Thr Ile Tyr Asn Thr Gln Leu Leu Phe Asp Ala Asp Gly Thr
           115                 120                 125
   Leu Ile Gln Arg Arg Arg Lys Leu Thr Pro Thr Tyr His Glu Arg Met
       130                 135                 140
   Ile Trp Gly Gln Gly Asp Ala Ser Gly Leu Arg Ala Thr Asp Ser Ala
   145                 150                 155                 160
   Val Gly Arg Ile Gly Gln Leu Ala Cys Trp Glu His Tyr Asn Pro Leu
                   165                 170                 175
   Phe Arg Tyr Ala Leu Ile Ala Asp Gly Glu Gln Ile His Ser Ala Met
               180                 185                 190
   Tyr Pro Gly Ser Phe Leu Gly Ala Leu His Gly Glu Gln Thr Glu Ile
           195                 200                 205
   Asn Val Arg Gln His Ala Leu Glu Ser Ala Ser Phe Val Val Val Ala
       210                 215                 220
   Thr Gly Trp Leu Asp Ala Asp Gln Gln Ala Gln Ile Ala Lys Asp Thr
   225                 230                 235                 240
   Gly Gly Pro Ile Gly Pro Ile Ser Gly Gly Cys Phe Thr Ala Val Ile
                   245                 250                 255
   Gly Pro Asp Gly Gln Leu Ile Gly Glu Ala Leu Thr Ser Gly Glu Gly
               260                 265                 270
   Glu Val Ile Ala Asp Ile Asp Leu Ala Gln Ile Asp Ala Arg Lys Arg
           275                 280                 285
   Leu Met Asp Ala Ser Gly His Tyr Asn Arg Pro Glu Leu Leu Ser Leu
       290                 295                 300
   His Ile Asp His Thr Pro Thr Ala Pro Met His Glu Arg Val Val Tyr
   305                 310                 315                 320
   Thr Glu Pro Gly Leu Ala Lys Arg Gln Asn Glu Asn Ser Ser Asn
                   325                 330                 335
<210> 295
   <211> 1134
   <212> DNA
   <213> Unknown
<220>
   <223> Obtained from an environmental sample
<400> 295
   atggccgcaa aagtacttgg aggacgtgac acagtaaaag tagcagttgt tcaaacacca      60
   tcagtcttta tggacaagaa agcctgtctc gaacttgcct gcgataagat tatcgaagcc     120
   ggcaaagagg gcgcggagct tgttgttttt cctgaaacct ggattccgac atatccttat     180
   tggaccatgg gatgggatac catcgcccat ggcttccatg atgtgatggc ggacctgcag     240
   gacaattccg tggtcgtcgg gagcgaagac accgacatat tgggcaaagc cgcccgggaa     300
   gctggcgcct acgtcgtcat gggctgtaat gagctcgatg accgggtcgg cagcaggacc     360
   ttgttcaact cgctggtcta tatggacaaa tatggcggcg tgctcggccg tcaccgtaaa     420
   ttaatcccgt cctttatcga acgcatctgg tggggcaatg gggacagccg cgatctcaaa      480
   gtttacgaca cggaaattgg gcgcatcggc ggtcaaatct gctgggaaaa tcacattgtg     540
   aacatcaccg catggtacat tgcccaaggc gtggatattc atgtctcggt ttggccggga     600
   atgtggaact gtggcgcaga agaaggagaa tccttcatat acgccggtca cgacatcaac     660
   aaatgtgacc tcatccctgc tacacgcgga cgggcattta cgggtcagtg ttatgtcctc     720
   tcagccaaca acctactgcg gatggaagac attcctgacg atttcccgtt ccgggatacc     780
   atgaactatg gcggtccggg ccaggaggat tttgtcggat gggcttgcgg tggcagccat     840
   attgttgcgc caacgtctga atttatggtg ccgccgacgt ttgatataga caccatcatc     900
   tatgcagaac ttcaggcgaa atacatcaaa gtggtgaagt cggtcttcga ttccctcggc     960
   cactacgcgc ggtgggacct cgtcaacttg accacgccgc caccgccgta tgaacctgaa    1020
   accgacgcac cagctctcac ggccgatatc cgtgatcggg tcatcgagag tgtggctaaa    1080
   gagttcaagc tcgaaccaga aaaagtggct gaagttgtgc gcaatgccgc ctag          1134
<210> 296
   <211> 377
   <212> PRT
   <213> Unknown
<220>
   <223> Obtained from an environmental sample
<400> 296
   Met Ala Ala Lys Val Leu Gly Gly Arg Asp Thr Val Lys Val Ala Val
    1               5                  10                  15
   Val Gln Thr Pro Ser Val Phe Met Asp Lys Lys Ala Cys Leu Glu Leu
               20                  25                  30
   Ala Cys Asp Lys Ile Ile Glu Ala Gly Lys Glu Gly Ala Glu Leu Val
           35                  40                  45
   Val Phe Pro Glu Thr Trp Ile Pro Thr Tyr Pro Tyr Trp Thr Met Gly
       50                  55                  60
   Trp Asp Thr Ile Ala His Gly Phe His Asp Val Met Ala Asp Leu Gln
   65                  70                  75                  80
   Asp Asn Ser Val Val Val Gly Ser Glu Asp Thr Asp Ile Leu Gly Lys
                   85                  90                  95
   Ala Ala Arg Glu Ala Gly Ala Tyr Val Val Met Gly Cys Asn Glu Leu
               100                 105                 110
   Asp Asp Arg Val Gly Ser Arg Thr Leu Phe Asn Ser Leu Val Tyr Met
           115                 120                 125
   Asp Lys Tyr Gly Gly Val Leu Gly Arg His Arg Lys Leu Ile Pro Ser
       130                 135                 140
   Phe Ile Glu Arg Ile Trp Trp Gly Asn Gly Asp Ser Arg Asp Leu Lys
   145                 150                 155                 160
   Val Tyr Asp Thr Glu Ile Gly Arg Ile Gly Gly Gln Ile Cys Trp Glu
                   165                 170                 175
   Asn His Ile Val Asn Ile Thr Ala Trp Tyr Ile Ala Gln Gly Val Asp
               180                 185                 190
   Ile His Val Ser Val Trp Pro Gly Met Trp Asn Cys Gly Ala Glu Glu
           195                 200                 205
   Gly Glu Ser Phe Ile Tyr Ala Gly His Asp Ile Asn Lys Cys Asp Leu
       210                 215                 220
   Ile Pro Ala Thr Arg Gly Arg Ala Phe Thr Gly Gln Cys Tyr Val Leu
   225                 230                 235                 240
   Ser Ala Asn Asn Leu Leu Arg Met Glu Asp Ile Pro Asp Asp Phe Pro
                   245                 250                 255
   Phe Arg Asp Thr Met Asn Tyr Gly Gly Pro Gly Gln Glu Asp Phe Val
               260                 265                 270
   Gly Trp Ala Cys Gly Gly Ser His Ile Val Ala Pro Thr Ser Glu Phe
           275                 280                 285
   Met Val Pro Pro Thr Phe Asp Ile Asp Thr Ile Ile Tyr Ala Glu Leu
       290                 295                 300
   Gln Ala Lys Tyr Ile Lys Val Val Lys Ser Val Phe Asp Ser Leu Gly
   305                 310                 315                 320
   His Tyr Ala Arg Trp Asp Leu Val Asn Leu Thr Thr Pro Pro Pro Pro
                   325                 330                 335
   Tyr Glu Pro Glu Thr Asp Ala Pro Ala Leu Thr Ala Asp Ile Arg Asp
               340                 345                 350
   Arg Val Ile Glu Ser Val Ala Lys Glu Phe Lys Leu Glu Pro Glu Lys
           355                 360                 365
   Val Ala Glu Val Val Arg Asn Ala Ala
       370                 375
<210> 297
   <211> 1059
   <212> DNA
   <213> Unknown
<220>
   <223> Obtained from an environmental sample
<400> 297
   atgacacgtt ttcgggacgt cacggtggcg gcggttcagg ccgcacccgt ctatttcgat      60
   cgggaggcct ccacagataa ggcgtgccaa ttgattcacg aagcggcgaa gaaaggcgca     120
   gccctcgcgg cgttcggcga aacgtggttg ccgggatatc cgttctttgc atgggggttc     180
   gcgcacaacc ggagcctgtt ctggaatgcg gccgccgagt acatcgccaa tgctgtggag     240
   attccgagtc caacgacgga ccgcctctgc gccgcagcga agatcgccgg gattgacgtg     300
   gtaatcggcg tcgtagaact ggatggacga acgcgagcgt cggtttacag cacactgctg     360
   ttcatcggga gagagggggc gatcctgggg cgccaccgca aattgaagcc aacccacatg      420
   gagcgaacgg tgtggggtga aggggacgct cacgggctcc gcgttcacga gcgtccgtac     480
   ggccgcctca gcgggctgaa ttgctgggaa cacaacatga tgctgcccgg ctatgtgctt     540
   gccgcgcagg gcacgcagtt tcacgtcgcg acatggcctg ggaaagagag gctcacagtt     600
   ccgccgaacg aggcggctta tacgcgccag cttctcctct ctcgcgccta tgcatcccag     660
   gcgggcgcgt acgtgatcag cgtcgcgggg ctgctcggac ccgactcgat gccggagcgt     720
   tatcgcgaac tgggacagtc ctatgagttg accggcgaca gcgtcatcat cgatccgcgc     780
   ggcgaggtca tcgcggggcc cgcgaagggc gagaccatcc tgctcgcgca atgcagccag     840
   gaagccctct tcgccgcaaa gtccgccatt gacgtcggcg gtcattactc gcgcccggat     900
   atttttcagc tgcgtgtcaa cgatcagcta cagcatcagg tccggagact cgaggcgact     960
   ctcacgcccc cagtcgccgt agttgtcggg cctgagggta gctcacatga gcaggagacg    1020
   gccttcgggc catccagcct cctggccacg acaagctag                           1059
<210> 298
   <211> 352
   <212> PRT
   <213> Unknown
<220>
   <223> Obtained from an environmental sample
<400> 298
   Met Thr Arg Phe Arg Asp Val Thr Val Ala Ala Val Gln Ala Ala Pro
   1 5 10 15
   Val Tyr Phe Asp Arg Glu Ala Ser Thr Asp Lys Ala Cys Gln Leu Ile
   20 25 30
   His Glu Ala Ala Lys Lys Gly Ala Ala Leu Ala Ala Phe Gly Glu Thr
   35 40 45
   Trp Leu Pro Gly Tyr Pro Phe Phe Ala Trp Gly Phe Ala His Asn Arg
   50 55 60
   Ser Leu Phe Trp Asn Ala Ala Ala Glu Tyr Ile Ala Asn Ala Val Glu
   65 70 75 80
   Ile Pro Ser Pro Thr Thr Asp Arg Leu Cys Ala Ala Ala Lys Ile Ala
   85 90 95
   Gly Ile Asp Val Val Ile Gly Val Val Glu Leu Asp Gly Arg Thr Arg
   100 105 110
   Ala Ser Val Tyr Ser Thr Leu Leu Phe Ile Gly Arg Glu Gly Ala Ile
   115 120 125
   Leu Gly Arg His Arg Lys Leu Lys Pro Thr His Met Glu Arg Thr Val
   130 135 140
   Trp Gly Glu Gly Asp Ala His Gly Leu Arg Val His Glu Arg Pro Tyr
   145 150 155 160
   Gly Arg Leu Ser Gly Leu Asn Cys Trp Glu His Asn Met Met Leu Pro
   165 170 175
   Gly Tyr Val Leu Ala Ala Gln Gly Thr Gln Phe His Val Ala Thr Trp
   180 185 190
   Pro Gly Lys Glu Arg Leu Thr Val Pro Pro Asn Glu Ala Ala Tyr Thr
   195 200 205
   Arg Gln Leu Leu Leu Ser Arg Ala Tyr Ala Ser Gln Ala Gly Ala Tyr
   210 215 220
   Val Ile Ser Val Ala Gly Leu Leu Gly Pro Asp Ser Met Pro Glu Arg
   225 230 235 240
   Tyr Arg Glu Leu Gly Gln Ser Tyr Glu Leu Thr Gly Asp Ser Val Ile
   245 250 255
   Ile Asp Pro Arg Gly Glu Val Ile Ala Gly Pro Ala Lys Gly Glu Thr
   260 265 270
   Ile Leu Leu Ala Gln Cys Ser Gln Glu Ala Leu Phe Ala Ala Lys Ser
   275 280 285
   Ala Ile Asp Val Gly Gly His Tyr Ser Arg Pro Asp Ile Phe Gln Leu
   290 295 300
   Arg Val Asn Asp Gln Leu Gln His Gln Val Arg Arg Leu Glu Ala Thr
   305 310 315 320
   Leu Thr Pro Pro Val Ala Val Val Val Gly Pro Glu Gly Ser Ser His
   325 330 335
   Glu Gln Glu Thr Ala Phe Gly Pro Ser Ser Leu Leu Ala Thr Thr Ser
   340 345 350
<210> 299
   <211> 987
   <212> DNA
   <213> Unknown
<220>
   <223> Obtained from an environmental sample
<400> 299
   atgactgttg ttaaggccgc cgcagtgcag atcagcccgg tgctctacag ccgtgcagga      60
   accgtcgaga aggtcgtgaa gaagatcgac gagctgggcc agaagggtgt cgagtttgcc     120
   gtcttccctg aaaccgttgt cccctactac ccctacttct ccttcgtgca gcccccctac     180
   aaactggcca cggagcacct gcgcctgctt gaggagtcgg tgaccgtgcc ctctgccgag     240
   acggacgcca tcggcgacgc cgcccgcaag gccaacatgg tcgtctcgat cggtgtcaac     300
   gaacgtgatg gcggcaccat ttacaacacc caactcctgt tcgacgccga cggaaccctg     360
   atccagcgcc gccgcaagat cacgccgacc taccacgagc ggatgatctg gggacaggga     420
   gacggatcag gcttgcgcgc ggtcgacagc gtcgtcggcc gcatcggcca gctcgcctgc     480
   tgggagcact accagccgct ggcccgttac gctctcatcg ccgacggcga gcagatccac     540
   gccgcgatgt accccggcgc cttcgggggc gatctgttcg cagagcagat cgaagtcaat     600
   gtccgtcagc acgctctgga atcggccagt ttcgtcgtca gcgccaccgc ctggctcgac     660
   gccgaccagc aggcccagat tgcgaaggac accggcggcc ccgtacaggc gatctccggc     720
   ggcttcttca cagccatcat cgaccccgac ggccgcatca tcggcgaacc gatcacctcc     780
   ggcgaaggcg aagtcatcgc tgacctcgac tttgcgctca tcgaccgccg caagcgcctg     840
   atggacgcca gcggccacta cagccgcccc gaactgctca gcctgcagat cgaccggacg     900
   ccggcacccg ccgtccacga tcgcaatcgc caggggtcct caagcgctcc ggcaactgaa     960
   aagggccgct cagccgaggc caagtga                                         987
<210> 300
   <211> 328
   <212> PRT
   <213> Unknown
<220>
   <223> Obtained from an environmental sample
<400> 300
   Met Thr Val Val Lys Ala Ala Ala Val Gln Ile Ser Pro Val Leu Tyr
   1               5                   10                  15
   Ser Arg Ala Gly Thr Val Glu Lys Val Val Lys Lys Ile Asp Glu Leu
               20                  25                  30
   Gly Gln Lys Gly Val Glu Phe Ala Val Phe Pro Glu Thr Val Val Pro
           35                  40                  45
   Tyr Tyr Pro Tyr Phe Ser Phe Val Gln Pro Pro Tyr Lys Leu Ala Thr
       50                  55                  60
   Glu His Leu Arg Leu Leu Glu Glu Ser Val Thr Val Pro Ser Ala Glu
   65                  70                  75                  80
   Thr Asp Ala Ile Gly Asp Ala Ala Arg Lys Ala Asn Met Val Val Ser
                   85                  90                  95
   Ile Gly Val Asn Glu Arg Asp Gly Gly Thr Ile Tyr Asn Thr Gln Leu
               100                 105                 110
   Leu Phe Asp Ala Asp Gly Thr Leu Ile Gln Arg Arg Arg Lys Ile Thr
           115                 120                 125
   Pro Thr Tyr His Glu Arg Met Ile Trp Gly Gln Gly Asp Gly Ser Gly
       130                 135                 140
   Leu Arg Ala Val Asp Ser Val Val Gly Arg Ile Gly Gln Leu Ala Cys
   145                 150                 155                 160
   Trp Glu His Tyr Gln Pro Leu Ala Arg Tyr Ala Leu Ile Ala Asp Gly
                   165                 170                 175
   Glu Gln Ile His Ala Ala Met Tyr Pro Gly Ala Phe Gly Gly Asp Leu
               180                 185                 190
   Phe Ala Glu Gln Ile Glu Val Asn Val Arg Gln His Ala Leu Glu Ser
           195                 200                 205
   Ala Ser Phe Val Val Ser Ala Thr Ala Trp Leu Asp Ala Asp Gln Gln
       210                 215                 220
   Ala Gln Ile Ala Lys Asp Thr Gly Gly Pro Val Gln Ala Ile Ser Gly
   225                 230                 235                 240
   Gly Phe Phe Thr Ala Ile Ile Asp Pro Asp Gly Arg Ile Ile Gly Glu
                   245                 250                 255
   Pro Ile Thr Ser Gly Glu Gly Glu Val Ile Ala Asp Leu Asp Phe Ala
               260                 265                 270
   Leu Ile Asp Arg Arg Lys Arg Leu Met Asp Ala Ser Gly His Tyr Ser
           275                 280                 285
   Arg Pro Glu Leu Leu Ser Leu Gln Ile Asp Arg Thr Pro Ala Pro Ala
       290                 295                 300
   Val His Asp Arg Asn Arg Gln Gly Ser Ser Ser Ala Pro Ala Thr Glu
   305                 310                 315                 320
   Lys Gly Arg Ser Ala Glu Ala Lys
                   325
<210> 301
   <211> 1032
   <212> DNA
   <213> Unknown
<220>
   <223> Obtained from an environmental sample
<400> 301
   atgaccacgc ccgcgccgtt caccgccgcc gtcgtccagg cgtcgcccgt gttcctcgac      60
   cgcgatgcca cggtcgataa ggcgtgcgcc ctgatcaccg cggccggagc gcgcggcgcg     120
   aagctcgtcg tgctgcccga gaccttcgtg cccgcatacc ccgcgtgggt ctggtacctg     180
   ccgctcacgc gtcggcccga cgtcgcgaag ctctaccgcg cgctcgtcga gaacgcgatc     240
   gacgtgccgg gtccggaaac cgagcgcctc gggcgcgcgg cgcgtgacgc gggcgcgtgg     300
   gtggcgatcg gtgtcaacga gcgcaacgcg aaggcgagcc gcacgtcgct ctacaacacc     360
   gtgctcctgt tcgacgacca gggcacgctg gtcgagtcgc ggcgcaagct gatgccgacc     420
   ggcggcgagc gcctggtgtg gacgccgggc gagccggtgc cgttgcgggt tcacgacacg     480
   ccgctcggcc gcgtcggcgc gctgatctgc tgggagaact acatgccgct cgcgcgcttc     540
   gcgctctacg agcagggcgt ccagatctat ctcgcgccga cctgggacta cagcgaggcg     600
   tggctcgcgt ccatgcggca cgtcgcgcgt gaaggtcgca cgtgggtgat cgggtgcagc     660
   caggcggtgc ggcgtgacga gatcccggac cacctgccgt tcaaggacgc gatccccgag     720
   agcctcgagt ggatcaacgc cgggaacagc atcgtcgtcg atccggacgg cgcggtggtc     780
   gcgggaccgc tggcgcgggc gcacgacacg ctctacgtcg agatcgatcc gggccgcgcc     840
   gccggctccc gatggatctt cgacgccgcc ggtcactatc accgtcccga cctgttccat     900
   ttcgcgatgc gcgcgccggt ggcgccagag accgcgccgg ttgtgcggaa gcgcggccgg     960
   cggctagcct cggccccggc gcggaagcca cggaaacggc cggcgcgtcc cacccgaagg    1020
   aggactcgat ga                                                        1032
<210> 302
   <211> 335
   <212> PRT
   <213> unknown
<220>
   <223> Obtained from an environmental sample
<400> 302
   Met Thr Thr Pro Ala Pro Phe Thr Ala Ala Val Val Gln Ala Ser Pro
   1 5 10 15
   Val Phe Leu Asp Arg Asp Ala Thr Val Asp Lys Ala Cys Ala Leu Ile
   20 25 30
   Thr Ala Ala Gly Ala Arg Gly Ala Lys Leu Val Val Leu Pro Glu Thr
   35 40 45
   Phe Val Pro Ala Tyr Pro Ala Trp Val Trp Tyr Leu Pro Leu Thr Arg
   50 55 60
   Arg Pro Asp Val Ala Lys Leu Tyr Arg Ala Leu Val Glu Asn Ala Ile
   65 70 75 80
   Asp Val Pro Gly Pro Glu Thr Glu Arg Leu Gly Arg Ala Ala Arg Asp
   85 90 95
   Ala Gly Ala Trp Val Ala Ile Gly Val Asn Glu Arg Asn Ala Lys Ala
   100 105 110
   Ser Arg Thr Ser Leu Tyr Asn Thr Val Leu Leu Phe Asp Asp Gln Gly
   115 120 125
   Thr Leu Val Glu Ser Arg Arg Lys Leu Met Pro Thr Gly Gly Glu Arg
   130 135 140
   Leu Val Trp Thr Pro Gly Glu Pro Val Pro Leu Arg Val His Asp Thr
   145 150 155 160
   Pro Leu Gly Arg Val Gly Ala Leu Ile Cys Trp Glu Asn Tyr Met Pro
   165 170 175
   Leu Ala Arg Phe Ala Leu Tyr Glu Gln Gly Val Gln Ile Tyr Leu Ala
   180 185 190
   Pro Thr Trp Asp Tyr Ser Glu Ala Trp Leu Ala Ser Met Arg His Val
   195 200 205
   Ala Arg Glu Gly Arg Thr Trp Val Ile Gly Cys Ser Gln Ala Val Arg
   210 215 220
   Arg Asp Glu Ile Pro Asp His Leu Pro Phe Lys Asp Ala Ile Pro Glu
   225 230 235 240
   Ser Leu Glu Trp Ile Asn Ala Gly Asn Ser Ile Val Val Asp Pro Asp
   245 250 255
   Gly Ala Val Val Ala Gly Pro Leu Ala Arg Ala His Asp Thr Leu Tyr
   260 265 270
   Val Glu Ile Asp Pro Gly Arg Ala Ala Gly Ser Arg Trp Ile Phe Asp
   275 280 285
   Ala Ala Gly His Tyr His Arg Pro Asp Leu Phe His Phe Ala Met Arg
   290 295 300
   Ala Pro Val Ala Pro Glu Thr Ala Pro Val Val Arg Lys Arg Gly Arg
   305 310 315 320
   Arg Leu Ala Ser Ala Pro Ala Arg Lys Pro Arg Lys Arg Pro Ala
   325 330 335
<210> 303
   <211> 1011
   <212> DNA
   <213> Unknown
<220>
   <223> Obtained from an environmental sample
<400> 303
   atgggcatcg ttcaccccaa gtacaaagtc gctgtcgttc aggctgcccc tgtatggcta      60
   gacctggagg ccactgttga taaatgcatt cagttgattg aagaagcagc cagcaagggc     120
   tgcaagctca tcgccttccc tgagaccttc attcccggat acccttggta tatctggatg     180
   ggaacgcctg cctggactat tcagcgcggc tttgtacagc gctattttga taattctctg     240
   tcttatgaca gtccgcaagc ggagaagcta aggcaggcag tcaaaaaggc tgagatcacg     300
   gccgtactag gtctttctga gcgcagcggc ggtagcttgt acattgcaca atggaccatt     360
   ggccctgacg gagaaaccat acacaaacgc agaaaagtgc gtccaacgca tggtgagcgt     420
   acggtatttg gcgacggtga cggtagtgat cttgcggtgc acgatacccc cctggggcgg     480
   ctgggcgcgc ttgcgtgctg ggagaacata ctgtcactga acaagtatgc gatgtattca     540
   cagaatgagc aggtgcacgt agccgcttgg cctagcttct cggtctacga gcctttcgcc     600
   catgcattgg gttgggaggt caataacgca gtcagcaagg tttacgcggt agaaggcggc     660
   tgttttgtat tggcgccttg cgcagtggtc tccgaggaaa tgatcgaagc actgtgcgat     720
   acacccgata agcaccaact ggctcatgcg ggtggagggc atgctgtcat ttacggacca     780
   gatggcagtc ctctggcaga taagttaccc gaaggagagg aggggctatt aattgcagaa     840
   attgatctcg gtctcatcag cttggcgaaa aatgccatgg acccggtggg gcattactct     900
   cgacctgacg tacatcgctt gctattgaat cgcaatccag caaagcgggt tgaggaattt     960
   tctctgccca ttgatttggc agagacaact ccgccaatat taggcacgta g              1011
<210> 304
   <211> 336
   <212> PRT
   <213> Unknown
<220>
   <223> Obtained from an environmental sample
<400> 304
   Met Gly Ile Val His Pro Lys Tyr Lys Val Ala Val Val Gln Ala Ala
   1               5                   10                  15
   Pro Val Trp Leu Asp Leu Glu Ala Thr Val Asp Lys Cys Ile Gln Leu
               20                  25                  30
   Ile Glu Glu Ala Ala Ser Lys Gly Cys Lys Leu Ile Ala Phe Pro Glu
           35                  40                  45
   Thr Phe Ile Pro Gly Tyr Pro Trp Tyr Ile Trp Met Gly Thr Pro Ala
       50                  55                  60
   Trp Thr Ile Gln Arg Gly Phe Val Gln Arg Tyr Phe Asp Asn Ser Leu
   65                  70                  75                  80
   Ser Tyr Asp Ser Pro Gln Ala Glu Lys Leu Arg Gln Ala Val Lys Lys
                   85                  90                  95
   Ala Glu Ile Thr Ala Val Leu Gly Leu Ser Glu Arg Ser Gly Gly Ser
               100                 105                 110
   Leu Tyr Ile Ala Gln Trp Thr Ile Gly Pro Asp Gly Glu Thr Ile His
           115                 120                 125
   Lys Arg Arg Lys Val Arg Pro Thr His Gly Glu Arg Thr Val Phe Gly
       130                 135                 140
   Asp Gly Asp Gly Ser Asp Leu Ala Val His Asp Thr Pro Leu Gly Arg
   145                 150                 155                 160
   Leu Gly Ala Leu Ala Cys Trp Glu Asn Ile Leu Ser Leu Asn Lys Tyr
                   165                 170                 175
   Ala Met Tyr Ser Gln Asn Glu Gln Val His Val Ala Ala Trp Pro Ser
               180                 185                 190
   Phe Ser Val Tyr Glu Pro Phe Ala His Ala Leu Gly Trp Glu Val Asn
           195                 200                 205
   Asn Ala Val Ser Lys Val Tyr Ala Val Glu Gly Gly Cys Phe Val Leu
       210                 215                 220
   Ala Pro Cys Ala Val Val Ser Glu Glu Met Ile Glu Ala Leu Cys Asp
   225                 230                 235                 240
   Thr Pro Asp Lys His Gln Leu Ala His Ala Gly Gly Gly His Ala Val
                   245                 250                 255
   Ile Tyr Gly Pro Asp Gly Ser Pro Leu Ala Asp Lys Leu Pro Glu Gly
               260                 265                 270
   Glu Glu Gly Leu Leu Ile Ala Glu Ile Asp Leu Gly Leu Ile Ser Leu
           275                 280                 285
   Ala Lys Asn Ala Met Asp Pro Val Gly His Tyr Ser Arg Pro Asp Val
       290                 295                 300
   His Arg Leu Leu Leu Asn Arg Asn Pro Ala Lys Arg Val Glu Glu Phe
   305                 310                 315                 320
   Ser Leu Pro Ile Asp Leu Ala Glu Thr Thr Pro Pro Ile Leu Gly Thr
                   325                 330                 335
<210> 305
   <211> 1068
   <212> DNA
   <213> Unknown
<220>
   <223> Obtained from an environmental sample
<400> 305
   atgacgatgc aacatcccaa gtttcgcgct gctgccgtgc aggcggcacc ggtcttcctt      60
   gaccttgatg cgtcgataga taaagccatc gacctgatcg cgcaagccgc caaaggcggc     120
   gcgcaattga ttgcctttcc ggaaacctgg ctgcccggct accccttttt catctggctc     180
   gattcgccgg cctggggcat gcaattcatc cagcgctacc atgacaattc cctggtctac     240
   ggcacaccgc aggccgagcg catcgcgcag gctgcgaaaa agcatcgcat catggtcgtc     300
   atggggcaca gcgagcggga tcatggaagt ctgtacatcg ctcagtggat catcggtgcc     360
   gatggggaaa cggttgcgac acgtcgtaag ctcaaaccga ctcatgccga gcgcaccttg     420
   tttggggaag gcgatggcag tgacctgagc gtgttcgata caccgctggg aagggttggc     480
   gcactatgct gctgggagca cctccagccc ctgtcgaaat acgcgctata cgcacagaat     540
   gagcaggtcc acattgcttc ctggccaagc ttttccctgt atcgcggggg cgcctacgcg     600
   ctcggcgccg aagtgaacaa tgcggccagc cagatttatg ctgtcgaagg ccagtgtttt     660
   gtgatcgcgc cgtgcggggt cgtcacgaaa gaaatgctgg acgtgctgtg caccgacgaa     720
   atgaaaaagc agttgttggt tgaaggcggc gggttcgcgc gaatttacgc gcccgatgga     780
   cagatgatgc acgcgccgct ggcggaaaac gaagagggcc tggtgtatgc cgatctcgac     840
   ctgggcatga tctcgctggc caaagtagtc gccgacccgg ccgggcatta tgcgcggccc     900
   gacgtaaccc ggttactgct ggacaagact ccgggagacc gcgtcatgct ggcgagccgt     960
   cgcggcaagg aggtcagccg cgctggtaac gacgagccgc aagtgctggt ctcgcgcaac    1020
   gacacgctga cctcgccgaa gccagcgtct tcgcgcaaag caagctga                1068
<210> 306
   <211> 355
   <212> PRT
   <213> Unknown
<220>
   <223> Obtained from an environmental sample
<400> 306
   Met Thr Met Gln His Pro Lys Phe Arg Ala Ala Ala Val Gln Ala Ala
   1               5                   10                  15
   Pro Val Phe Leu Asp Leu Asp Ala Ser Ile Asp Lys Ala Ile Asp Leu
               20                  25                  30
   Ile Ala Gln Ala Ala Lys Gly Gly Ala Gln Leu Ile Ala Phe Pro Glu
           35                  40                  45
   Thr Trp Leu Pro Gly Tyr Pro Phe Phe Ile Trp Leu Asp Ser Pro Ala
       50                  55                  60
   Trp Gly Met Gln Phe Ile Gln Arg Tyr His Asp Asn Ser Leu Val Tyr
   65                  70                  75                  80
   Gly Thr Pro Gln Ala Glu Arg Ile Ala Gln Ala Ala Lys Lys His Arg
                   85                  90                  95
   Ile Met Val Val Met Gly His Ser Glu Arg Asp His Gly Ser Leu Tyr
               100                 105                 110
   Ile Ala Gln Trp Ile Ile Gly Ala Asp Gly Glu Thr Val Ala Thr Arg
           115                 120                 125
   Arg Lys Leu Lys Pro Thr His Ala Glu Arg Thr Leu Phe Gly Glu Gly
       130                 135                 140
   Asp Gly Ser Asp Leu Ser Val Phe Asp Thr Pro Leu Gly Arg Val Gly
   145                 150                 155                 160
   Ala Leu Cys Cys Trp Glu His Leu Gln Pro Leu Ser Lys Tyr Ala Leu
                   165                 170                 175
   Tyr Ala Gln Asn Glu Gln Val His Ile Ala Ser Trp Pro Ser Phe Ser
               180                 185                 190
   Leu Tyr Arg Gly Gly Ala Tyr Ala Leu Gly Ala Glu Val Asn Asn Ala
           195                 200                 205
   Ala Ser Gln Ile Tyr Ala Val Glu Gly Gln Cys Phe Val Ile Ala Pro
       210                 215                 220
   Cys Gly Val Val Thr Lys Glu Met Leu Asp Val Leu Cys Thr Asp Glu
   225                 230                 235                 240
   Met Lys Lys Gln Leu Leu Val Glu Gly Gly Gly Phe Ala Arg Ile Tyr
                   245                 250                 255
   Ala Pro Asp Gly Gln Met Met His Ala Pro Leu Ala Glu Asn Glu Glu
               260                 265                 270
   Gly Leu Val Tyr Ala Asp Leu Asp Leu Gly Met Ile Ser Leu Ala Lys
           275                 280                 285
   Val Val Ala Asp Pro Ala Gly His Tyr Ala Arg Pro Asp Val Thr Arg
       290                 295                 300
   Leu Leu Leu Asp Lys Thr Pro Gly Asp Arg Val Met Leu Ala Ser Arg
   305                 310                 315                 320
   Arg Gly Lys Glu Val Ser Arg Ala Gly Asn Asp Glu Pro Gln Val Leu
                   325                 330                 335
   Val Ser Arg Asn Asp Thr Leu Thr Ser Pro Lys Pro Ala Ser Ser Arg
               340                 345                 350
   Lys Ala Ser
           355
<210> 307
   <211> 942
   <212> DNA
   <213> Unknown
<220>
   <223> Obtained from an environmental sample
<400> 307
   atggctgaac cggaatcctt tatcgtcgct gctgtacagg ctacaccgat ctttcttgac      60
   cgccaggcaa cccttgagaa agcgtgcgac ctgattgccg aagctggcag caatggcgca     120
   aaactcgttc tttttcccga agcctttatc cccacctatc ctgattggat atgggcggtg     180
   acaggctcac aatctgcgct gctcgacgaa ctttatgtgg aactactgga aaactccgtg     240
   accatccccg acgcgaccac tgagcaactt tgtgaagcag cacgtaacgc cggtctctac     300
   gtcgtcatgg gagtgaatga gcgcaacgcc gaggcgagca acgccacact ctataacacc     360
   ctgctctata ttgacgatca gggcaaaatt ctcggcaagc atcgcaaatt ggtcccgacc     420
   gccctggagc gaatcgtctg gggctatggc gatggcagca cgcttgacgc ctttgaaacg     480
   ccgctgggca agattggcgg gctgatctgt tgggaaaatt acatgccact ggcgcgccaa     540
   acactttatg cctggggggt gcaaatttac ttggccgcaa cgtgggatcg cggcgaagtt     600
   tggcaggcga ccatgcgcca tattgccagg gaaggcggcg tctatgtagt cgcctcctgt     660
   attccatttc acatcaaaga cattcctgac cacatgcctg aaatccgcaa tctctatgca     720
   ccggggacag actggatcaa cgtcggccaa agctgcatca tcaaccccag cggcgactat     780
   attgcaggcc ctgtcgagtg tcgcgaggag attctttatg ccgaggtaaa tctgcgccag     840
   agtgcggcgg caaaacgtat gttggatgtg gcgggccatt atggacgccc tgatgtcttt     900
   cacctcaccg tcaaccgcac gcccaatccg catattcgat aa                        942
<210> 308
   <211> 313
   <212> PRT
   <213> Unknown
<220>
   <223> Obtained from an environmental sample
<400> 308
   Met Ala Glu Pro Glu Ser Phe Ile Val Ala Ala Val Gln Ala Thr Pro
   1               5                   10                  15
   Ile Phe Leu Asp Arg Gln Ala Thr Leu Glu Lys Ala Cys Asp Leu Ile
               20                  25                  30
   Ala Glu Ala Gly Ser Asn Gly Ala Lys Leu Val Leu Phe Pro Glu Ala
           35                  40                  45
   Phe Ile Pro Thr Tyr Pro Asp Trp Ile Trp Ala Val Thr Gly Ser Gln
       50                  55                  60
   Ser Ala Leu Leu Asp Glu Leu Tyr Val Glu Leu Leu Glu Asn Ser Val
   65                  70                  75                  80
   Thr Ile Pro Asp Ala Thr Thr Glu Gln Leu Cys Glu Ala Ala Arg Asn
                   85                  90                  95
   Ala Gly Leu Tyr Val Val Met Gly Val Asn Glu Arg Asn Ala Glu Ala
               100                 105                 110
   Ser Asn Ala Thr Leu Tyr Asn Thr Leu Leu Tyr Ile Asp Asp Gln Gly
           115                 120                 125
   Lys Ile Leu Gly Lys His Arg Lys Leu Val Pro Thr Ala Leu Glu Arg
       130                 135                 140
   Ile Val Trp Gly Tyr Gly Asp Gly Ser Thr Leu Asp Ala Phe Glu Thr
   145                 150                 155                 160
   Pro Leu Gly Lys Ile Gly Gly Leu Ile Cys Trp Glu Asn Tyr Met Pro
                   165                 170                 175
   Leu Ala Arg Gln Thr Leu Tyr Ala Trp Gly Val Gln Ile Tyr Leu Ala
               180                 185                 190
   Ala Thr Trp Asp Arg Gly Glu Val Trp Gln Ala Thr Met Arg His Ile
           195                 200                 205
   Ala Arg Glu Gly Gly Val Tyr Val Val Ala Ser Cys Ile Pro Phe His
       210                 215                 220
   Ile Lys Asp Ile Pro Asp His Met Pro Glu Ile Arg Asn Leu Tyr Ala
   225                 230                 235                 240
   Pro Gly Thr Asp Trp Ile Asn Val Gly Gln Ser Cys Ile Ile Asn Pro
                   245                 250                 255
   Ser Gly Asp Tyr Ile Ala Gly Pro Val Glu Cys Arg Glu Glu Ile Leu
               260                 265                 270
   Tyr Ala Glu Val Asn Leu Arg Gln Ser Ala Ala Ala Lys Arg Met Leu
           275                 280                 285
   Asp Val Ala Gly His Tyr Gly Arg Pro Asp Val Phe His Leu Thr Val
       290                 295                 300
   Asn Arg Thr Pro Asn Pro His Ile Arg
   305                 310
<210> 309
   <211> 951
   <212> DNA
   <213> Unknown
<220>
   <223> Obtained from an environmental sample
<400> 309
   ttgaccgact gtctcaaaat agccgccgcc caaatcactc cggtcttcct cgaccgcgtt     60
   gcgaccacga agaaggtcgt cgaaaccatc gaaaaagcgg ccgccggcgg tgcgcggctg    120
   gtcgcattcg gcgaagcgct gttgcccgcc tatccattgt ggctgacgcg caccgacgcc    180
   gcgcggttca attccgacgt gcaaaaaaac ttgcacgcga tctatctcaa gcaatccgtc    240
   tcgatagcag gcggtcacct atctccgatt tgcaaaatcg caagcgaacg caagattgcc    300
   gtcatcctcg gcatcgccga gcgcgcgacc gaccggggcg accacaccat ttactgctcg    360
   tgcgtgttca tcgatgccga cggccgaatc gcgtcggtcc atcgcaagct gatgccgaca    420
   tacgaagaac gcctcagttg gggattcggc gacggtgcgg gactcgtcac gcatccggtc    480
   gggccgttca cggtgggcgc gttgaactgc tgggaaaact ggatgcccct cgcgcgcacc    540
   gcgctgtatg ccggcggaga agatttgcac gttgcgatct ggcccggcgg atcggtgctc    600
   acggaagaca tcacgcgctt catcgcacgc gagtcgcgct cgttcgtcct gtccgtcagc    660
   ggcatcattc gcgaaagcga catccccagc ggggtcccct atcgcgatga aatgtgtgcg    720
   aaaggcgaaa ccatctacaa cggcggaagc tgcatcgccg gacccgacgg tcagtggatc    780
   atcgcgcccg taaccgaccg tgaagagttg atcttcgcgg agatcgacca cgaacacgtc    840
   cgccgcgagc ggcagaattt cgacccggcc gggcattacg cgcggcccga tgtgttgcaa    900
   ataaccgttg atcgtcgacg acaaacagcg gcgaatttta ttgatgacta a             951
<210> 310
   <211> 316
   <212> PRT
   <213> Unknown
<220>
   <223> Obtained from an environmental sample
<400> 310
   Leu Thr Asp Cys Leu Lys Ile Ala Ala Ala Gln Ile Thr Pro Val Phe
   1               5                   10                  15
   Leu Asp Arg Val Ala Thr Thr Lys Lys Val Val Glu Thr Ile Glu Lys
               20                  25                  30
   Ala Ala Ala Gly Gly Ala Arg Leu Val Ala Phe Gly Glu Ala Leu Leu
           35                  40                  45
   Pro Ala Tyr Pro Leu Trp Leu Thr Arg Thr Asp Ala Ala Arg Phe Asn
       50                  55                  60
   Ser Asp Val Gln Lys Asn Leu His Ala Ile Tyr Leu Lys Gln Ser Val
   65                  70                  75                  80
   Ser Ile Ala Gly Gly His Leu Ser Pro Ile Cys Lys Ile Ala Ser Glu
                   85                  90                  95
   Arg Lys Ile Ala Val Ile Leu Gly Ile Ala Glu Arg Ala Thr Asp Arg
               100                 105                 110
   Gly Asp His Thr Ile Tyr Cys Ser Cys Val Phe Ile Asp Ala Asp Gly
           115                 120                 125
   Arg Ile Ala Ser Val His Arg Lys Leu Met Pro Thr Tyr Glu Glu Arg
       130                 135                 140
   Leu Ser Trp Gly Phe Gly Asp Gly Ala Gly Leu Val Thr His Pro Val
   145                 150                 155                 160
   Gly Pro Phe Thr Val Gly Ala Leu Asn Cys Trp Glu Asn Trp Met Pro
                   165                 170                 175
   Leu Ala Arg Thr Ala Leu Tyr Ala Gly Gly Glu Asp Leu His Val Ala
               180                 185                 190
   Ile Trp Pro Gly Gly Ser Val Leu Thr Glu Asp Ile Thr Arg Phe Ile
           195                 200                 205
   Ala Arg Glu Ser Arg Ser Phe Val Leu Ser Val Ser Gly Ile Ile Arg
       210                 215                 220
   Glu Ser Asp Ile Pro Ser Gly Val Pro Tyr Arg Asp Glu Met Cys Ala
   225                 230                 235                 240
   Lys Gly Glu Thr Ile Tyr Asn Gly Gly Ser Cys Ile Ala Gly Pro Asp
                   245                 250                 255
   Gly Gln Trp Ile Ile Ala Pro Val Thr Asp Arg Glu Glu Leu Ile Phe
               260                 265                 270
   Ala Glu Ile Asp His Glu His Val Arg Arg Glu Arg Gln Asn Phe Asp
           275                 280                 285
   Pro Ala Gly His Tyr Ala Arg Pro Asp Val Leu Gln Ile Thr Val Asp
       290                 295                 300
   Arg Arg Arg Gln Thr Ala Ala Asn Phe Ile Asp Asp
   305                 310                 315
<210> 311
   <211> 1011
   <212> DNA
   <213> Unknown
<220>
   <223> Obtained from an environmental sample
<400> 311
   atgtcagaaa agcgaataat cagagcagct gcagttcaga tcacacctga atttgactca      60
   gcagatggaa cagttaagaa ggtatgcaag gtaatcgatg aagcaggcgc aaagggtgta     120
   caaattatag tattccccga aaccttcatt ccgtattacc catacttctc attcattacc     180
   cccccagttt ctgctggcgc tgagcatttg aagctttatg aaaaaagtgt cgtgatacct     240
   ggtcctgtca ctcaagcgat cgccgagaga gctagagtga atcaaatggt cgtcgtactc     300
   ggtgtaaacg agagagataa cggtagcctc tataacactc aactgatctt cgataccaat     360
   ggtgagttga tgttgaagag aagaaaaatc actcctacat atcatgagcg catgatctgg     420
   ggacaaggtg atgcttcagg cttaaaagta gttgaaacga gcattgcccg ggtaggtgct     480
   ctagcttgct gggaacatta caacccgctg gccagatatt ctctcatgac acagcatgaa     540
   gaaattcact gcgcacaatt cccaggttct atggttggcc aaatatttgc cgaccaaatg     600
   gatgtcacta tcagacatca cgcattggaa tctggctgtt tcgtcattaa tgccaccggc     660
   tggctcacag acgagcaaat ccagtccatt acagatgacc caaaaatgca gaaagcatta     720
   cgtggcggct gcaacacagc aatcatttct cccgaaggtg tgcacttaac agagccctta     780
   cgtgaaggtg aaggcatttt gattgctgac ctggacatgt cactcatcac aaaacgaaaa     840
   agaatgatgg attcagtagg tcattattca agacctgaac tattaagtct ggcgatcaat     900
   gacaagccag caacaacaaa attttcaatg actgaggggt gtactcaaac tgagcaattt     960
   cgaatcgcag aggagttgaa aaatgacgac aagcttagca ccggaaacta a             1011
<210> 312
   <211> 336
   <212> PRT
   <213> Unknown
<220>
   <223> Obtained from an environmental sample
<400> 312
   Met Ser Glu Lys Arg Ile Ile Arg Ala Ala Ala Val Gln Ile Thr Pro
   1               5                   10                  15
   Glu Phe Asp Ser Ala Asp Gly Thr Val Lys Lys Val Cys Lys Val Ile
               20                  25                  30
   Asp Glu Ala Gly Ala Lys Gly Val Gln Ile Ile Val Phe Pro Glu Thr
           35                  40                  45
   Phe Ile Pro Tyr Tyr Pro Tyr Phe Ser Phe Ile Thr Pro Pro Val Ser
       50                  55                  60
   Ala Gly Ala Glu His Leu Lys Leu Tyr Glu Lys Ser Val Val Ile Pro
   65                  70                  75                  80
   Gly Pro Val Thr Gln Ala Ile Ala Glu Arg Ala Arg Val Asn Gln Met
                   85                  90                  95
   Val Val Val Leu Gly Val Asn Glu Arg Asp Asn Gly Ser Leu Tyr Asn
               100                 105                 110
   Thr Gln Leu Ile Phe Asp Thr Asn Gly Glu Leu Met Leu Lys Arg Arg
           115                 120                 125
   Lys Ile Thr Pro Thr Tyr His Glu Arg Met Ile Trp Gly Gln Gly Asp
       130                 135                 140
   Ala Ser Gly Leu Lys Val Val Glu Thr Ser Ile Ala Arg Val Gly Ala
   145                 150                 155                 160
   Leu Ala Cys Trp Glu His Tyr Asn Pro Leu Ala Arg Tyr Ser Leu Met
                   165                 170                 175
   Thr Gln His Glu Glu Ile His Cys Ala Gln Phe Pro Gly Ser Met Val
               180                 185                 190
   Gly Gln Ile Phe Ala Asp Gln Met Asp Val Thr Ile Arg His His Ala
           195                 200                 205
   Leu Glu Ser Gly Cys Phe Val Ile Asn Ala Thr Gly Trp Leu Thr Asp
       210                 215                 220
   Glu Gln Ile Gln Ser Ile Thr Asp Asp Pro Lys Met Gln Lys Ala Leu
   225                 230                 235                 240
   Arg Gly Gly Cys Asn Thr Ala Ile Ile Ser Pro Glu Gly Val His Leu
                   245                 250                 255
   Thr Glu Pro Leu Arg Glu Gly Glu Gly Ile Leu Ile Ala Asp Leu Asp
               260                 265                 270
   Met Ser Leu Ile Thr Lys Arg Lys Arg Met Met Asp Ser Val Gly His
           275                 280                 285
   Tyr Ser Arg Pro Glu Leu Leu Ser Leu Ala Ile Asn Asp Lys Pro Ala
       290                 295                 300
   Thr Thr Lys Phe Ser Met Thr Glu Gly Cys Thr Gln Thr Glu Gln Phe
   305                 310                 315                 320
   Arg Ile Ala Glu Glu Leu Lys Asn Asp Asp Lys Leu Ser Thr Gly Asn
                   325                 330                 335
<210> 313
   <211> 987
   <212> DNA
   <213> Unknown
<220>
   <223> Obtained from an environmental sample
<400> 313
   atggcggccg ttcaggctgc gccggttccg tttgatgctg aggcttcggt ggataaggcc      60
   tgtcgcttaa ttcaagaagc tgcagccaaa ggcgcagata tagttgcttt cggtgaggca     120
   tggctacccg gctaccccta ttttgcctgg ttaccccaag taacaccaga gtggtatagt     180
   gcggctgccg attatcttgc cagctctgtt gatatccctg gcccgatcac cgataaactt     240
   tgccaggctg cccgtcgtgc atcggttgaa ctcatcatgg gtgtggtaga acgcagcaag     300
   tctcagggaa ccacctattg cacgcttctt tttattagca aggatggcga aataattggc     360
   aagcaccgca agctgaagcc cacactcgcc gaacgaaccg tctggggtga aggcgatgcc     420
   agcggactga gggttcacga tcggcctatt gccagaatca gtgggctctc ctgctgggag     480
   aacaaaatga tgctgccagg ttacgcactg atggcgcagg gtacgcaggt gcatgtctcc     540
   gcctggccag ggatcccaga ggattcaccc atggaggtgc ctgcacaccc ccgtcaaaag     600
   ctgctttccc aagcctttgc actgcaaggc gggtgctatg ttatttctcc ctccattgtc     660
   cttagggcag aagatgtgcc cgagaaacac gccgctctac tgatgggaga ccaagtgggc     720
   ggtagctata tcattgaccc ctgcggaaaa gtgatcgccg aggccggtgc gggtgagact     780
   atcctgattg ccaaaggcaa ccttgacctc gtaagggccg ccaaaatggc cagtgatgta     840
   ggggggtctt attcacgccc ggatcttttg cagttgatga tcaataaccg accactcgaa     900
   cagctgattg agttcagtgc tgaaggtgca ggtaggggga atctagtatc caactcaccc     960
   gaggtgtcag aacaagaagg tgagtaa                                          987
<210> 314
   <211> 328
   <212> PRT
   <213> Unknown
<220>
   <223> Obtained from an environmental sample
<400> 314
   Met Ala Ala Val Gln Ala Ala Pro Val Pro Phe Asp Ala Glu Ala Ser
   1               5                   10                  15
   Val Asp Lys Ala Cys Arg Leu Ile Gln Glu Ala Ala Ala Lys Gly Ala
               20                  25                  30
   Asp Ile Val Ala Phe Gly Glu Ala Trp Leu Pro Gly Tyr Pro Tyr Phe
           35                  40                  45
   Ala Trp Leu Pro Gln Val Thr Pro Glu Trp Tyr Ser Ala Ala Ala Asp
       50                  55                  60
   Tyr Leu Ala Ser Ser Val Asp Ile Pro Gly Pro Ile Thr Asp Lys Leu
   65                  70                  75                  80
   Cys Gln Ala Ala Arg Arg Ala Ser Val Glu Leu Ile Met Gly Val Val
                   85                  90                  95
   Glu Arg Ser Lys Ser Gln Gly Thr Thr Tyr Cys Thr Leu Leu Phe Ile
               100                 105                 110
   Ser Lys Asp Gly Glu Ile Ile Gly Lys His Arg Lys Leu Lys Pro Thr
           115                 120                 125
   Leu Ala Glu Arg Thr Val Trp Gly Glu Gly Asp Ala Ser Gly Leu Arg
       130                 135                 140
   Val His Asp Arg Pro Ile Ala Arg Ile Ser Gly Leu Ser Cys Trp Glu
   145                 150                 155                 160
   Asn Lys Met Met Leu Pro Gly Tyr Ala Leu Met Ala Gln Gly Thr Gln
                   165                 170                 175
   Val His Val Ser Ala Trp Pro Gly Ile Pro Glu Asp Ser Pro Met Glu
               180                 185                 190
   Val Pro Ala His Pro Arg Gln Lys Leu Leu Ser Gln Ala Phe Ala Leu
           195                 200                 205
   Gln Gly Gly Cys Tyr Val Ile Ser Pro Ser Ile Val Leu Arg Ala Glu
       210                 215                 220
   Asp Val Pro Glu Lys His Ala Ala Leu Leu Met Gly Asp Gln Val Gly
   225                 230                 235                 240
   Gly Ser Tyr Ile Ile Asp Pro Cys Gly Lys Val Ile Ala Glu Ala Gly
                   245                 250                 255
   Ala Gly Glu Thr Ile Leu Ile Ala Lys Gly Asn Leu Asp Leu Val Arg
               260                 265                 270
   Ala Ala Lys Met Ala Ser Asp Val Gly Gly Ser Tyr Ser Arg Pro Asp
           275                 280                 285
   Leu Leu Gln Leu Met Ile Asn Asn Arg Pro Leu Glu Gln Leu Ile Glu
       290                 295                 300
   Phe Ser Ala Glu Gly Ala Gly Arg Gly Asn Leu Val Ser Asn Ser Pro
   305                 310                 315                 320
   Glu Val Ser Glu Gln Glu Gly Glu
                   325
<210> 315
   <211> 960
   <212> DNA
   <213> Unknown
<220>
   <223> obtained from an environmental sample
<400> 315
   atgacaatgc ttaagaccaa attccgggtt gctgcggtgc aggcagcgcc tgtattcctg      60
   gatcgagagg ctacgctgga gaaagcctgc ggactgattg aggaggcggg tcgcaacggg     120
   gccagcctgg tggtctttcc tgagtcgttc attccggcct atcccgattg ggtctgggct     180
   gtgccggcgg gtgaagaagc tttacttaat gaactgtatg ctcaactgct ggccaacgcc     240
   gttgaaattc ccagcccggc caccgaacgc ttgagccagg cagcgaaaaa ggctaaagtc    300
   catgtggtta tgggcctgac cgaacgcaac agcgaggcca gcggcggcag cctctacaat    360
   accttgctct atcttgaccc acagggccaa attctgggca aacatcgcaa gctggtgccc    420
   accggcggcg agcggctggt ttgggcccag ggcgacggca gtaccctgca agtctacgag    480
   acccccttgg gtaaactcag cggcttgatt tgctgggaaa attatatgcc gctggcccgc    540
   tacgcgctct atgcctgggg tacgcaaatc tatatcgccg ccacctggga tcgaggcgag    600
   ccgtggcttt cgacgctgcg gcatatcgcc aaagagggcc gggtgtttgt catcggctgt    660
   ggcatggcct tgcgtaaggc tgatattccc gaccattttg aattcaagca gcgcttttat    720
   caaaatgccg gcgagtggat caatggaggc gacagcgcca ttgtcaatcc tgagggtgaa    780
   tttattgctg gacccctgag cgagcaggaa ggtattttgt acgccgagat tgatcctggc    840
   cagatggccg gaccaaaatg gatgctcgat gtggccgggc actatgctcg cccggatgtc    900
   tttgaactga ccgtccagac cgtagctcgg cccatgatta cctctactca gccgcgatag    960
<210> 316
   <211> 319
   <212> PRT
   <213> Unknown
<220>
   <223> Obtained from an environmental sample
<400> 316
   Met Thr Met Leu Lys Thr Lys Phe Arg Val Ala Ala Val Gln Ala Ala
   1               5                   10                  15
   Pro Val Phe Leu Asp Arg Glu Ala Thr Leu Glu Lys Ala Cys Gly Leu
               20                  25                  30
   Ile Glu Glu Ala Gly Arg Asn Gly Ala Ser Leu Val Val Phe Pro Glu
           35                  40                  45
   Ser Phe Ile Pro Ala Tyr Pro Asp Trp Val Trp Ala Val Pro Ala Gly
       50                  55                  60
   Glu Glu Ala Leu Leu Asn Glu Leu Tyr Ala Gln Leu Leu Ala Asn Ala
   65                  70                  75                  80
   Val Glu Ile Pro Ser Pro Ala Thr Glu Arg Leu Ser Gln Ala Ala Lys
                   85                  90                  95
   Lys Ala Lys Val His Val Val Met Gly Leu Thr Glu Arg Asn Ser Glu
               100                 105                 110
   Ala Ser Gly Gly Ser Leu Tyr Asn Thr Leu Leu Tyr Leu Asp Pro Gln
           115                 120                 125
   Gly Gln Ile Leu Gly Lys His Arg Lys Leu Val Pro Thr Gly Gly Glu
       130                 135                 140
   Arg Leu Val Trp Ala Gln Gly Asp Gly Ser Thr Leu Gln Val Tyr Glu
   145                 150                 155                 160
   Thr Pro Leu Gly Lys Leu Ser Gly Leu Ile Cys Trp Glu Asn Tyr Met
                   165                 170                 175
   Pro Leu Ala Arg Tyr Ala Leu Tyr Ala Trp Gly Thr Gln Ile Tyr Ile
               180                 185                 190
   Ala Ala Thr Trp Asp Arg Gly Glu Pro Trp Leu Ser Thr Leu Arg His
           195                 200                 205
   Ile Ala Lys Glu Gly Arg Val Phe Val Ile Gly Cys Gly Met Ala Leu
       210                 215                 220
   Arg Lys Ala Asp Ile Pro Asp His Phe Glu Phe Lys Gln Arg Phe Tyr
   225                 230                 235                 240
   Gln Asn Ala Gly Glu Trp Ile Asn Gly Gly Asp Ser Ala Ile Val Asn
                   245                 250                 255
   Pro Glu Gly Glu Phe Ile Ala Gly Pro Leu Ser Glu Gln Glu Gly Ile
               260                 265                 270
   Leu Tyr Ala Glu Ile Asp Pro Gly Gln Met Ala Gly Pro Lys Trp Met
           275                 280                 285
   Leu Asp Val Ala Gly His Tyr Ala Arg Pro Asp Val Phe Glu Leu Thr
       290                 295                 300
   Val Gln Thr Val Ala Arg Pro Met Ile Thr Ser Thr Gln Pro Arg
   305                 310                 315
<210> 317
   <211> 993
   <212> DNA
   <213> Unknown
<220>
   <223> Obtained from an environmental sample
<400> 317
   atgaccattg taaaagccgc tgccgtccaa ataagccctg tcctttacag ccgcgaaggc      60
   accgtggaca aggttgtcca gaagatcctc gaactcggca agcaaggcgt ccagttcgcc     120
   actttcccgg agacggtggt cccctactac ccctacttct ccttcgtcca gtcgggctac     180
   gccctcaagg tgggcaagga acatctgcgc ttgctcgaac agtcggtcac cgtgccatcg     240
   gccaccacgc tcgccatcgg cgaagcctgc aagcaggcgg ggatggtggt gtccatcggc     300
   gtcaacgaac gcgacggcag cacgatctac aacacgcagc tgctcttcga tgccgacggc     360
   accttgattc agcgccgccg aaagatcagc ccgaccttcc atgaacgcat ggtctggggc     420
   cagggcgacg gctccgggct gcgcgcggtc gacagcgcgg tcgggcgcat cggccagttg     480
   gcgtgctggg agcactacaa cccgctggcc cgctacgcca tgatggccga cggcgagcag     540
   atccactcgg cgatgtaccc cggttccttc gcaggcgacg ccttctccga acagatccag     600
   gtcaacatcc gccagcacgc attggaagcc ggctgcttcg tcgtgaacgc caccgcgtgg     660
   ctggacgccg atcagcaggc gcagatcatg caggacaccg gttgcgccat cggcccgatc     720
   tccagtggtt gcttcaccgc catcgtttcg ccggacggcg tgttgctggg cgagcctctg     780
   cggtccggtg agggcgaggt gattgccgat ctcgacttca cgctgatcga caagcgcaag     840
   cagatgatgg attcacgcgg gcactatgcg cgcccggaat tgctcagcct gttgatcgac     900
   cgcacggcga ccgcgcatgt gcatgagcgc agcgcgcatc cgaaggcgac tgccgagcag     960
   gcggacggtc catccgccgt gaatgcgcag taa                                  993
<210> 318
   <211> 330
   <212> PRT
   <213> Unknown
<220>
   <223> Obtained from an environmental sample
<400> 318
   Met Thr Ile Val Lys Ala Ala Ala Val Gln Ile Ser Pro Val Leu Tyr
   1 5 10 15
   Ser Arg Glu Gly Thr Val Asp Lys Val Val Gln Lys Ile Leu Glu Leu
   20 25 30
   Gly Lys Gln Gly Val Gln Phe Ala Thr Phe Pro Glu Thr Val Val Pro
   35 40 45
   Tyr Tyr Pro Tyr Phe Ser Phe Val Gln Ser Gly Tyr Ala Leu Lys Val
   50 55 60
   Gly Lys Glu His Leu Arg Leu Leu Glu Gln Ser Val Thr Val Pro Ser
   65 70 75 80
   Ala Thr Thr Leu Ala Ile Gly Glu Ala Cys Lys Gln Ala Gly Met Val
   85 90 95
   Val Ser Ile Gly Val Asn Glu Arg Asp Gly Ser Thr Ile Tyr Asn Thr
   100 105 110
   Gln Leu Leu Phe Asp Ala Asp Gly Thr Leu Ile Gln Arg Arg Arg Lys
   115 120 125
   Ile Ser Pro Thr Phe His Glu Arg Met Val Trp Gly Gln Gly Asp Gly
   130 135 140
   Ser Gly Leu Arg Ala Val Asp Ser Ala Val Gly Arg Ile Gly Gln Leu
   145 150 155 160
   Ala Cys Trp Glu His Tyr Asn Pro Leu Ala Arg Tyr Ala Met Met Ala
   165 170 175
   Asp Gly Glu Gln Ile His Ser Ala Met Tyr Pro Gly Ser Phe Ala Gly
   180 185 190
   Asp Ala Phe Ser Glu Gln Ile Gln Val Asn Ile Arg Gln His Ala Leu
   195 200 205
   Glu Ala Gly Cys Phe Val Val Asn Ala Thr Ala Trp Leu Asp Ala Asp
   210 215 220
   Gln Gln Ala Gln Ile Met Gln Asp Thr Gly Cys Ala Ile Gly Pro Ile
   225 230 235 240
   Ser Ser Gly Cys Phe Thr Ala Ile Val Ser Pro Asp Gly Val Leu Leu
   245 250 255
   Gly Glu Pro Leu Arg Ser Gly Glu Gly Glu Val Ile Ala Asp Leu Asp
   260 265 270
   Phe Thr Leu Ile Asp Lys Arg Lys Gln Met Met Asp Ser Arg Gly His
   275 280 285
   Tyr Ala Arg Pro Glu Leu Leu Ser Leu Leu Ile Asp Arg Thr Ala Thr
   290 295 300
   Ala His Val His Glu Arg Ser Ala His Pro Lys Ala Thr Ala Glu Gln
   305 310 315 320
   Ala Asp Gly Pro Ser Ala Val Asn Ala Gln
   325 330
<210> 319
   <211> 1017
   <212> DNA
   <213> Unknown
<220>
   <223> Obtained from an environmental sample
<400> 319
   atgacgaccc cgcgtatcgt ccgcgtcgcc gcggtgcaga tggcgcccga cctggaatcc      60
   gcacacggca cggtagacaa ggtctgccgc gccattctgg aggccggcga aaaaggcgcg     120
   cgcatggtgg tctttcccga gaccttcgtg ccctactacc cctacttctc cttcatccag     180
   ccggcggtga cgatgggcgc cgcccacctc gagctctacg agcgcgcggt gacggtgccc     240
   ggcccggtca ccctggccgt gggcgaagcg gcgcggcggg cgggcgcggt cgtcgtgctc     300
   ggcgtcaatg aacgcgacca cggctcgctc tacaacaccc agctgatctt cgacgagacc     360
   ggcgcgctgg tgctcaagcg ccgcaagctc accccgacct atcacgagcg catggtgtgg     420
   ggccagggcg atggcagcgg tctcaaggtg gtggacaccg gcatcggccg catcggcgcg     480
   ctggcctgct gggagcacta caacccgctc gcccgctaca ccctgatggc gcagcatgaa     540
   gagatccacg ccgcgcagtt ccccggttcc atggtcggcc agatctttgc cgaccagatg     600
   gcggtgacca tccgccacca cgcgctggaa tccggctgct tcgtggtgaa cgccaccggc     660
   tggctcaccg atgcgcagat caccgccatc acccccgacc cggccatgca gcgcgcgctg     720
   cgcggcggct gccacaccgc catcgtgtcg ccggaaggca gctacgtctg cgaaccgctc     780
   accgagggcg aaggcatgct ggtggccgac ctcgacatgc ggctggtcac caagcgcaag     840
   cggatgatgg attcggtcgg ccactacgcc cggccggagc tgctttcgct caacgccgat     900
   cttgccccca agccggcgct gcacacacag ccggctgcgt ccctccctct ctcccttcag     960
   gcaggagccg accatgtcga cgacgaccgc accgcgtccg caacagctga tctctga       1017
<210> 320
   <211> 338
   <212> PRT
   <213> Unknown
<220>
   <223> Obtained from an environmental sample
<400> 320
   Met Thr Thr Pro Arg Ile Val Arg Val Ala Ala Val Gln Met Ala Pro
   1               5                   10                  15
   Asp Leu Glu Ser Ala His Gly Thr Val Asp Lys Val Cys Arg Ala Ile
               20                  25                  30
   Leu Glu Ala Gly Glu Lys Gly Ala Arg Met Val Val Phe Pro Glu Thr
           35                  40                  45
   Phe Val Pro Tyr Tyr Pro Tyr Phe Ser Phe Ile Gln Pro Ala Val Thr
       50                  55                  60
   Met Gly Ala Ala His Leu Glu Leu Tyr Glu Arg Ala Val Thr Val Pro
   65                  70                  75                  80
   Gly Pro Val Thr Leu Ala Val Gly Glu Ala Ala Arg Arg Ala Gly Ala
                   85                  90                  95
   Val Val Val Leu Gly Val Asn Glu Arg Asp His Gly Ser Leu Tyr Asn
               100                 105                 110
   Thr Gln Leu Ile Phe Asp Glu Thr Gly Ala Leu Val Leu Lys Arg Arg
           115                 120                 125
   Lys Leu Thr Pro Thr Tyr His Glu Arg Met Val Trp Gly Gln Gly Asp
       130                 135                 140
   Gly Ser Gly Leu Lys Val Val Asp Thr Gly Ile Gly Arg Ile Gly Ala
   145                 150                 155                 160
   Leu Ala Cys Trp Glu His Tyr Asn Pro Leu Ala Arg Tyr Thr Leu Met
                   165                 170                 175
   Ala Gln His Glu Glu Ile His Ala Ala Gln Phe Pro Gly Ser Met Val
               180                 185                 190
   Gly Gln Ile Phe Ala Asp Gln Met Ala Val Thr Ile Arg His His Ala
           195                 200                 205
   Leu Glu Ser Gly Cys Phe Val Val Asn Ala Thr Gly Trp Leu Thr Asp
       210                 215                 220
   Ala Gln Ile Thr Ala Ile Thr Pro Asp Pro Ala Met Gln Arg Ala Leu
   225                     230                     235                     240
   Arg Gly Gly Cys His Thr Ala Ile Val Ser Pro Glu Gly Ser Tyr Val
                       245                     250                     255
   Cys Glu Pro Leu Thr Glu Gly Glu Gly Met Leu Val Ala Asp Leu Asp
                   260                     265                     270
   Met Arg Leu Val Thr Lys Arg Lys Arg Met Met Asp Ser Val Gly His
               275                     280                     285
   Tyr Ala Arg Pro Glu Leu Leu Ser Leu Asn Ala Asp Leu Ala Pro Lys
           290                     295                     300
   Pro Ala Leu His Thr Gln Pro Ala Ala Ser Leu Pro Leu Ser Leu Gln
   305                     310                     315                     320
   Ala Gly Ala Asp His Val Asp Asp Asp Arg Thr Ala Ser Ala Thr Ala
                       325                     330                     335
   Asp Leu
<210> 321
   <211> 993
   <212> DNA
   <213> Unknown
<220>
   <223> Obtained from an environmental sample
<400> 321
   atgagagttg tcaaagccgc tgctgtccaa ctgagtcctg tcctccatag ccgcgacgga      60
   acggtcgaaa aggtcgtgcg gaagatccat gaactcgccg aagagggagt cgagttcgcc     120
   acctttcctg agaccgtggt gccttactac ccgtactttt ccttcgttca gacgcccttg     180
   cagcaaatct tcggaactga gtatctgagg ctgctcgacc aggcagtcac cgtgccatcc     240
   gctgccaccg acgcgatcgg cgaggctgcc aggtgggctg gacttgttgt ctcgatcggc     300
   gtcaacgagc gagacggggg aactctctac aacactcagc ttctcttcga tgccgacggc     360
   agcttaattc agcggcgtcg caagatcaca cccacccatt acgagcgcat gatctggggc     420
   cagggcgacg gctcaggtct gcgggccgtt gatagcaagg ccggccgcat tggtcagctg     480
   gcatgctggg aacacaacaa ccccctggcg cgctacgcgc tgatggccga cggcgagcag     540
   atccattcgg ccatgtatcc gggctccatg ttcggcgact cgttttccca aaagaccgaa     600
   atcaatatcc ggcagcatgc gctggaatct gcgtgcttcg tcgtgaacgc aacggcctgg     660
   ctggacgccg atcagcaggc gcaaatcatg aaggacaccg gctgcggcat cggcccgatc     720
   tccggcggtt gcttcactgc gatcgttgca cccgatggta gcctgctggg cgaacccatc     780
   cgttccggtg agggcgtcgt cgtcgccaac ctcgacttca cgctgatcga caggcgtaag     840
   caggtgatgg actcgcgagg ccactacagc cggccggagt tgctcagcct cttaatagac     900
   cgcactccta ccgcgcacgt tcacgaacgc gctacgcacc ccacgacagg agctgagcaa     960
   ggctccgagg atgtgttcga ggctcgcatt taa                                 993
<210> 322
   <211> 330
   <212> PRT
   <213> Unknown
<220>
   <223> Obtained from an environmental sample
<400> 322
   Met Arg Val Val Lys Ala Ala Ala Val Gln Leu Ser Pro Val Leu His
    1               5                  10                  15
   Ser Arg Asp Gly Thr Val Glu Lys Val Val Arg Lys Ile His Glu Leu
               20                  25                  30
   Ala Glu Glu Gly Val Glu Phe Ala Thr Phe Pro Glu Thr Val Val Pro
           35                  40                  45
   Tyr Tyr Pro Tyr Phe Ser Phe Val Gln Thr Pro Leu Gln Gln Ile Phe
       50                  55                  60
   Gly Thr Glu Tyr Leu Arg Leu Leu Asp Gln Ala Val Thr Val Pro Ser
   65                  70                  75                  80
   Ala Ala Thr Asp Ala Ile Gly Glu Ala Ala Arg Trp Ala Gly Leu Val
                   85                  90                  95
   Val Ser Ile Gly Val Asn Glu Arg Asp Gly Gly Thr Leu Tyr Asn Thr
               100                 105                 110
   Gln Leu Leu Phe Asp Ala Asp Gly Ser Leu Ile Gln Arg Arg Arg Lys
           115                 120                 125
   Ile Thr Pro Thr His Tyr Glu Arg Met Ile Trp Gly Gln Gly Asp Gly
       130                 135                 140
   Ser Gly Leu Arg Ala Val Asp Ser Lys Ala Gly Arg Ile Gly Gln Leu
   145                 150                 155                 160
   Ala Cys Trp Glu His Asn Asn Pro Leu Ala Arg Tyr Ala Leu Met Ala
                   165                 170                 175
   Asp Gly Glu Gln Ile His Ser Ala Met Tyr Pro Gly Ser Met Phe Gly
               180                 185                 190
   Asp Ser Phe Ser Gln Lys Thr Glu Ile Asn Ile Arg Gln His Ala Leu
           195                 200                 205
   Glu Ser Ala Cys Phe Val Val Asn Ala Thr Ala Trp Leu Asp Ala Asp
       210                 215                 220
   Gln Gln Ala Gln Ile Met Lys Asp Thr Gly Cys Gly Ile Gly Pro Ile
   225                 230                 235                 240
   Ser Gly Gly Cys Phe Thr Ala Ile Val Ala Pro Asp Gly Ser Leu Leu
                   245                 250                 255
   Gly Glu Pro Ile Arg Ser Gly Glu Gly Val Val Val Ala Asn Leu Asp
               260                 265                 270
   Phe Thr Leu Ile Asp Arg Arg Lys Gln Val Met Asp Ser Arg Gly His
           275                 280                 285
   Tyr Ser Arg Pro Glu Leu Leu Ser Leu Leu Ile Asp Arg Thr Pro Thr
       290                 295                 300
   Ala His Val His Glu Arg Ala Thr His Pro Thr Thr Gly Ala Glu Gln
   305                 310                 315                 320
   Gly Ser Glu Asp Val Phe Glu Ala Arg Ile
                   325                 330
<210> 323
   <211> 951
   <212> DNA
   <213> Unknown
<220>
   <223> Obtained from an environmental sample
<400> 323
   atgagcaacg tgaaagtcgc ggtcgtgcaa cgcgcgccgg tgttcggcaa ccgcgccgcc     60
   accctcgatc gcgccgtcgc cgcgctggcc gaagcggcgc agcagggggc gaagctggtc    120
   gtgatgcccg agcacttcat ccccggctac ccggcctgga tctggcgcct gcgtccgggc    180
   accgacctgc gattgtgcga acagctgcac gcgatgctgc gcgccaacgc cgtgaggctg    240
   gatgacggtg acctggcccc gttgaccgag gccgcgcagc ggcatgcgct caccgtggtc    300
   tgcggcgtct gcgagatcga caccgaattc agtcgcggca ccctgtacaa caccgtggtc    360
   gtgatcgggc ccgacggcac gctgctcaac cggcatcgca agctgatgcc caccaacccc    420
   gagcgcatgg tctggggcat gggcgacgcc acggggctga aggtggtcga cacgccctgc    480
   gggcgcatcg gcacgctgat ttgctgggag aactacatgc cattcgcacg cgccgcgctg    540
   tacgcgcagg gggtcgaggt cctggttgca ccgacctacg acgaaggccc ggtatggctg    600
   gcgtcgatgc agcacatcgc ccgcgaaggc ggctgctggg tggtgggcaa cggctgcgca    660
   ttccagggcc gcgacatgcc ggacaccttg ccgggcaagg cccagctgtt tcccgaggcc    720
   gacgcctggg tcaacgccgg ggactcggtc atcgtcgcgc caggcggccg gacagtggcg    780
   ggtccgttgc acgaggcgtt cgggctgttc accgccgaga tcgacctctc ccgggtcgga    840
   atggcccggc gcagcctgga tgtggccggg cactatggac ggcccgacat cttctgcctg    900
   caggtcaacg cccgggcgca gccgccggtt gaggtgacgc accatggctg a             951
<210> 324
   <211> 316
   <212> PRT
   <213> Unknown
<220>
   <223> Obtained from an environmental sample
<400> 324
   Met Ser Asn Val Lys Val Ala Val Val Gln Arg Ala Pro Val Phe Gly
   1               5                   10                  15
   Asn Arg Ala Ala Thr Leu Asp Arg Ala Val Ala Ala Leu Ala Glu Ala
               20                  25                  30
   Ala Gln Gln Gly Ala Lys Leu Val Val Met Pro Glu His Phe Ile Pro
           35                  40                  45
   Gly Tyr Pro Ala Trp Ile Trp Arg Leu Arg Pro Gly Thr Asp Leu Arg
       50                  55                  60
   Leu Cys Glu Gln Leu His Ala Met Leu Arg Ala Asn Ala Val Arg Leu
   65                  70                  75                  80
   Asp Asp Gly Asp Leu Ala Pro Leu Thr Glu Ala Ala Gln Arg His Ala
                   85                  90                  95
   Leu Thr Val Val Cys Gly Val Cys Glu Ile Asp Thr Glu Phe Ser Arg
               100                 105                 110
   Gly Thr Leu Tyr Asn Thr Val Val Val Ile Gly Pro Asp Gly Thr Leu
           115                 120                 125
   Leu Asn Arg His Arg Lys Leu Met Pro Thr Asn Pro Glu Arg Met Val
       130                 135                 140
   Trp Gly Met Gly Asp Ala Thr Gly Leu Lys Val Val Asp Thr Pro Cys
   145                 150                 155                 160
   Gly Arg Ile Gly Thr Leu Ile Cys Trp Glu Asn Tyr Met Pro Phe Ala
                   165                 170                 175
   Arg Ala Ala Leu Tyr Ala Gln Gly Val Glu Val Leu Val Ala Pro Thr
               180                 185                 190
   Tyr Asp Glu Gly Pro Val Trp Leu Ala Ser Met Gln His Ile Ala Arg
           195                 200                 205
   Glu Gly Gly Cys Trp Val Val Gly Asn Gly Cys Ala Phe Gln Gly Arg
       210                 215                 220
   Asp Met Pro Asp Thr Leu Pro Gly Lys Ala Gln Leu Phe Pro Glu Ala
   225                 230                 235                 240
   Asp Ala Trp Val Asn Ala Gly Asp Ser Val Ile Val Ala Pro Gly Gly
                   245                 250                 255
   Arg Thr Val Ala Gly Pro Leu His Glu Ala Phe Gly Leu Phe Thr Ala
               260                 265                 270
   Glu Ile Asp Leu Ser Arg Val Gly Met Ala Arg Arg Ser Leu Asp Val
           275                 280                 285
   Ala Gly His Tyr Gly Arg Pro Asp Ile Phe Cys Leu Gln Val Asn Ala
       290                 295                 300
   Arg Ala Gln Pro Pro Val Glu Val Thr His His Gly
   305                 310                 315
<210> 325
   <211> 1077
   <212> DNA
   <213> Unknown
<220>
   <223> Obtained from an environmental sample
<400> 325
   atgactgcaa aaaagattgt ccgcgccgcc gctgtccagc tcaatcccgt gctggacagc      60
   gccgacggca cccttgtgaa agtgttgcag gcgattgccg acgccgcagc gcagggcgtg     120
   caactgatcg tctttcccga gacggtggtg ccctactatc cttacttttc cttcgtcacg     180
   cctgcggttt cgatgggcgc ggcgcatctg aaactgtacg aacaatcgcc cacggtgcca     240
   ggtccactga ccgacgccgt cgccgcagcc gcgcgggcac atcagatggt ggttgtgctc     300
   ggcgtcaacg agcgcgatca cggcacgctc tacaacacgc aactgatctt cgacgccgac     360
   ggcacgctcc cactgaagcg tcgcaagatc acgccgacct atcacgagcg catggtctgg     420
   ggcatgggcg atggctccgg cctgcgcacg gtgaagaccg aggtcggaac cgttggcgcg     480
   ctggcctgct gggaacacta caacccgctg gcacgctacg cgctgatggc gcagcacgaa     540
   gagatccatt gcagccagtt ccccggctcg ctggtcggcc cgatcttttc cgagcagatg     600
   gaaatcacca tgcgtcatca cgcgctggaa tccggctgct tcgtcgtcaa cgccactgca     660
   tggctgacgc ctgagcaggt gcgatcacag gcgccaacac cggcaatgga aaaagccttc     720
   tccggtggtt gctacaccgc gatcatttcg ccggaaggaa aacatctggg cgaacctctt     780
   cgcgacggcg aaggcatggt catcgccgat cttgattttg atctcatcac caagcgcaag     840
   cgaatgatgg attcggttgg ccactacgca cggccggaat tgttgagcct gcagctcgac     900
   aaccgatcaa ctgcaccgct gacaacgtcg ccggtggccg ccgcagcgcc gtcgcttgca     960
   gagatggaag cacagcgcct gtcacgttat ctcgatgcca gctccggcag cgccgcacaa    1020
   ggcatcgaag ccgcctacat caatgccctc agctcttttt caggaaaacc ctcatga       1077
<210> 326
   <211> 358
   <212> PRT
   <213> Unknown
<220>
   <223> Obtained from an environmental sample
<400> 326
   Met Thr Ala Lys Lys Ile Val Arg Ala Ala Ala Val Gln Leu Asn Pro
   1               5                   10                  15
   Val Leu Asp Ser Ala Asp Gly Thr Leu Val Lys Val Leu Gln Ala Ile
               20                  25                  30
   Ala Asp Ala Ala Ala Gln Gly Val Gln Leu Ile Val Phe Pro Glu Thr
           35                  40                  45
   Val Val Pro Tyr Tyr Pro Tyr Phe Ser Phe Val Thr Pro Ala Val Ser
       50                  55                  60
   Met Gly Ala Ala His Leu Lys Leu Tyr Glu Gln Ser Pro Thr Val Pro
   65                  70                  75                  80
   Gly Pro Leu Thr Asp Ala Val Ala Ala Ala Ala Arg Ala His Gln Met
                   85                  90                  95
   Val Val Val Leu Gly Val Asn Glu Arg Asp His Gly Thr Leu Tyr Asn
               100                 105                 110
   Thr Gln Leu Ile Phe Asp Ala Asp Gly Thr Leu Pro Leu Lys Arg Arg
           115                 120                 125
   Lys Ile Thr Pro Thr Tyr His Glu Arg Met Val Trp Gly Met Gly Asp
       130                 135                 140
   Gly Ser Gly Leu Arg Thr Val Lys Thr Glu Val Gly Thr Val Gly Ala
   145                 150                 155                 160
   Leu Ala Cys Trp Glu His Tyr Asn Pro Leu Ala Arg Tyr Ala Leu Met
                   165                 170                 175
   Ala Gln His Glu Glu Ile His Cys Ser Gln Phe Pro Gly Ser Leu Val
               180                 185                 190
   Gly Pro Ile Phe Ser Glu Gln Met Glu Ile Thr Met Arg His His Ala
           195                 200                 205
   Leu Glu Ser Gly Cys Phe Val Val Asn Ala Thr Ala Trp Leu Thr Pro
       210                 215                 220
   Glu Gln Val Arg Ser Gln Ala Pro Thr Pro Ala Met Glu Lys Ala Phe
   225                 230                 235                 240
   Ser Gly Gly Cys Tyr Thr Ala Ile Ile Ser Pro Glu Gly Lys His Leu
                   245                 250                 255
   Gly Glu Pro Leu Arg Asp Gly Glu Gly Met Val Ile Ala Asp Leu Asp
               260                 265                 270
   Phe Asp Leu Ile Thr Lys Arg Lys Arg Met Met Asp Ser Val Gly His
           275                 280                 285
   Tyr Ala Arg Pro Glu Leu Leu Ser Leu Gln Leu Asp Asn Arg Ser Thr
       290                 295                 300
   Ala Pro Leu Thr Thr Ser Pro Val Ala Ala Ala Ala Pro Ser Leu Ala
   305                 310                 315                 320
   Glu Met Glu Ala Gln Arg Leu Ser Arg Tyr Leu Asp Ala Ser Ser Gly
                   325                 330                 335
   Ser Ala Ala Gln Gly Ile Glu Ala Ala Tyr Ile Asn Ala Leu Ser Ser
               340                 345                 350
   Phe Ser Gly Lys Pro Ser
           355
<210> 327
   <211> 975
   <212> DNA
   <213> Unknown
<220>
   <223> Obtained from an environmental sample
<400> 327
   atggttgatc aaatacttaa cgatcgttct gaattattaa cagttggttt ggcgcagatt      60
   gctccaatct ggctgaaccg cgaaaaaaca cttgcgaaag tggttgagaa agtaaatcaa     120
   gctgcaaagc aagattgtca tcttgttgct tttggtgaag ctttggtccc cggatatccc     180
   ttttggattg aacttacaga tggcgcgcga ttcaactcca atgttcaaaa agaaattcac     240
   gcgcactata tggatcaggc agtacagata gagaatggtc atttaaaagc gctttgtgaa     300
   acatcggccg caaacaagat tgccgtgatc gttgggtgca ttgaacgcgc agccgatcgc     360
   ggcgggcaca gcttatatgc ttcattggtt tttattaatc ctcaagggca aatcggatcg     420
   gtgcatcgca aacttatgcc aacttatgaa gaacgtttaa cctggtcacc tggcgatgga     480
   catggtttgc gaacgcatca actaggtgct ttcactgttg gcggcttgaa ttgttgggaa     540
   aactggatgc cattgccacg cgccgcattg tacgcacaag gtgaagactt tcatgtcgca     600
   atctggccgg gaagcattca caatacgcaa gatattacgc gctatattgc gaaggaatcc     660
   agatcgtttg taatgtctac ttccgggttc atgcgtaaag aagactttcc atctgatacg     720
   cctcacttag acaaaatact tgcaaactct ccgaatgtac ttgcaaatgg tggatcctgt     780
   ctcgccggac cggacggtca gtggatcgtt gaaccatttg tgaatgagga aaaattagtt     840
   gttgcgactg tgaaccacaa gcaggtccgt gaagaaagac aaaattttga tccggtcgga     900
   cactattccc gtcctgatgt cacgcagttg attgtgaaca gacaaagaca atcaacaatc     960
   aagctcaacg attaa                                                      975
<210> 328
   <211> 324
   <212> PRT
   <213> Unknown
<220>
   <223> Obtained from an environmental sample
<400> 328
   Met Val Asp Gln Ile Leu Asn Asp Arg Ser Glu Leu Leu Thr Val Gly
    1               5                  10                  15
   Leu Ala Gln Ile Ala Pro Ile Trp Leu Asn Arg Glu Lys Thr Leu Ala
               20                  25                  30
   Lys Val Val Glu Lys Val Asn Gln Ala Ala Lys Gln Asp Cys His Leu
           35                  40                  45
   Val Ala Phe Gly Glu Ala Leu Val Pro Gly Tyr Pro Phe Trp Ile Glu
       50                  55                  60
   Leu Thr Asp Gly Ala Arg Phe Asn Ser Asn Val Gln Lys Glu Ile His
   65                  70                  75                  80
   Ala His Tyr Met Asp Gln Ala Val Gln Ile Glu Asn Gly His Leu Lys
                   85                  90                  95
   Ala Leu Cys Glu Thr Ser Ala Ala Asn Lys Ile Ala Val Ile Val Gly
               100                 105                 110
   Cys Ile Glu Arg Ala Ala Asp Arg Gly Gly His Ser Leu Tyr Ala Ser
           115                 120                 125
   Leu Val Phe Ile Asn Pro Gln Gly Gln Ile Gly Ser Val His Arg Lys
       130                 135                 140
   Leu Met Pro Thr Tyr Glu Glu Arg Leu Thr Trp Ser Pro Gly Asp Gly
   145                 150                 155                 160
   His Gly Leu Arg Thr His Gln Leu Gly Ala Phe Thr Val Gly Gly Leu
                   165                 170                 175
   Asn Cys Trp Glu Asn Trp Met Pro Leu Pro Arg Ala Ala Leu Tyr Ala
               180                 185                 190
   Gln Gly Glu Asp Phe His Val Ala Ile Trp Pro Gly Ser Ile His Asn
           195                 200                 205
   Thr Gln Asp Ile Thr Arg Tyr Ile Ala Lys Glu Ser Arg Ser Phe Val
       210                 215                 220
   Met Ser Thr Ser Gly Phe Met Arg Lys Glu Asp Phe Pro Ser Asp Thr
   225                 230                 235                 240
   Pro His Leu Asp Lys Ile Leu Ala Asn Ser Pro Asn Val Leu Ala Asn
                   245                 250                 255
   Gly Gly Ser Cys Leu Ala Gly Pro Asp Gly Gln Trp Ile Val Glu Pro
               260                 265                 270
   Phe Val Asn Glu Glu Lys Leu Val Val Ala Thr Val Asn His Lys Gln
           275                 280                 285
   Val Arg Glu Glu Arg Gln Asn Phe Asp Pro Val Gly His Tyr Ser Arg
       290                 295                 300
   Pro Asp Val Thr Gln Leu Ile Val Asn Arg Gln Arg Gln Ser Thr Ile
   305                 310                 315                 320
   Lys Leu Asn Asp
<210> 329
   <211> 1023
   <212> DNA
   <213> Unknown
<220>
   <223> obtained from an environmental sample
<400> 329
   gtgccggcca aagtacgcgc cgccgccgtg cagctcagcc ccgtgctctt cagccgtgaa      60
   ggcacgacca gcaaggtctg cgacaagatt gccgaggcgg cggcgcaggg cgccgagctg     120
   gtggtgtttc ccgaaaccgt agtgccgtat tacccgtatt tctcgttcat caaggctccg    180
   gccgtgatcg gcgccgagca cttactcttg ctcgaacaag ccgtcacggt gcccgggccc    240
   agcgtcgaag ccatcgcaga agccgctcgc aaggcgggcg cggtggtttc gatcggcgtc    300
   aacgaacgcg atcacggcac gctgtacaac acccagctgt tgttcgacgc cgacggtcgg    360
   ttggcgcaag cccgccgcaa gatcaccccc acgtatcacg agcggatgat ctgggggcag    420
   ggcgatggct cgggcttggt ggcggtggat acgcgagtcg gcaggattgg ctccctggcg    480
   tgctgggagc actacaaccc gctggctcgc tatgcgctga tggccgacca cgagcaaatt    540
   cacgtggcca tgttccctgg ctcgctcgtg ggcgacatct tccgcgagca aatcgaggtc    600
   acgattcggc accacgcgct cgagtcgggc tgcttcgtcg tcaacgcgac gggctacctg    660
   agcgacgcgc aggtgacgca gatcgcgggc gacaccaagc tcgaccgcgc cctgcgcggc    720
   ggttgcttca ccgccatcgt atcgcccgag ggcacgctgc tggcgccacc gctcaccgac    780
   ggtgagggca tggtcattgc cgacctcgat ctgtcgctca tcgccaaacg caaacgcatg    840
   atggacagcg tcggccatta cagccggccc gagctgctca gcgtgctgat cgaccgctcg    900
   ccgcagccgc atttgcgcga gaaaactgcc tctttacccg aacctcggat gtcccatgaa    960
   tcgctcgctc ccgatagtaa cggcctccgc gacgctgacg cgaaagcctc gaccctctcc   1020
   tga                                                                 1023
<210> 330
   <211> 340
   <212> PRT
   <213> Unknown
<220>
   <223> Obtained from an environmental sample
<400> 330
   Val Pro Ala Lys Val Arg Ala Ala Ala Val Gln Leu Ser Pro Val Leu
   1 5 10 15
   Phe Ser Arg Glu Gly Thr Thr Ser Lys Val Cys Asp Lys Ile Ala Glu
   20 25 30
   Ala Ala Ala Gln Gly Ala Glu Leu Val Val Phe Pro Glu Thr Val Val
   35 40 45
   Pro Tyr Tyr Pro Tyr Phe Ser Phe Ile Lys Ala Pro Ala Val Ile Gly
   50 55 60
   Ala Glu His Leu Leu Leu Leu Glu Gln Ala Val Thr Val Pro Gly Pro
   65 70 75 80
   Ser Val Glu Ala Ile Ala Glu Ala Ala Arg Lys Ala Gly Ala Val Val
   85 90 95
   Ser Ile Gly Val Asn Glu Arg Asp His Gly Thr Leu Tyr Asn Thr Gln
   100 105 110
   Leu Leu Phe Asp Ala Asp Gly Arg Leu Ala Gln Ala Arg Arg Lys Ile
   115 120 125
   Thr Pro Thr Tyr His Glu Arg Met Ile Trp Gly Gln Gly Asp Gly Ser
   130 135 140
   Gly Leu Val Ala Val Asp Thr Arg Val Gly Arg Ile Gly Ser Leu Ala
   145 150 155 160
   Cys Trp Glu His Tyr Asn Pro Leu Ala Arg Tyr Ala Leu Met Ala Asp
   165 170 175
   His Glu Gln Ile His Val Ala Met Phe Pro Gly Ser Leu Val Gly Asp
   180 185 190
   Ile Phe Arg Glu Gln Ile Glu Val Thr Ile Arg His His Ala Leu Glu
   195 200 205
   Ser Gly Cys Phe Val Val Asn Ala Thr Gly Tyr Leu Ser Asp Ala Gln
   210 215 220
   Val Thr Gln Ile Ala Gly Asp Thr Lys Leu Asp Arg Ala Leu Arg Gly
   225 230 235 240
   Gly Cys Phe Thr Ala Ile Val Ser Pro Glu Gly Thr Leu Leu Ala Pro
   245 250 255
   Pro Leu Thr Asp Gly Glu Gly Met Val Ile Ala Asp Leu Asp Leu Ser
   260 265 270
   Leu Ile Ala Lys Arg Lys Arg Met Met Asp Ser Val Gly His Tyr Ser
   275 280 285
   Arg Pro Glu Leu Leu Ser Val Leu Ile Asp Arg Ser Pro Gln Pro His
   290 295 300
   Leu Arg Glu Lys Thr Ala Ser Leu Pro Glu Pro Arg Met Ser His Glu
   305 310 315 320
   Ser Leu Ala Pro Asp Ser Asn Gly Leu Arg Asp Ala Asp Ala Lys Ala
   325 330 335
   Ser Thr Leu Ser
   340
<210> 331
   <211> 1041
   <212> DNA
   <213> Unknown
<220>
   <223> Obtained from an environmental sample
<400> 331
   atgtccggaa caggacctat cggaccggtc gtgaaggtcg ccgtcgtaca agccgcgcca      60
   tgtctgctcg atcttgatgc gggcgtcgag aaggcgatcg ccttgatcga ccaagcgggc     120
   aaggctggcg cgcggctcat caattttccc gaaatttggc tgccgggtta tccttggtgg     180
   atctggctga atccacccgc catcaacatg cagtatgtcg cgccttacat gaacaactcc     240
   attgtcgcag gcagcaagca tgaccacgca cttcgggccg ccgcgcgccg caacaatatt     300
   cacgtcgtga tcggtgtctc cgagcgcgcc ggcggcagtc tgtacatggc tcagtggcac     360
   tatgggcccg agggcgaggt gatctcgcgt cgtcgtaagc taaaacccac ccatgtcgaa     420
   cgcagcgtct ttggcgaagg cgacggcagc gacatgatcg ttactcagac cgatttcggg     480
   cgcgtcggcg cgctatgctg ttgggaacac ctgcaaccgc tgtcgaaata cgcgctcttt     540
   tcccaggacg agcagattca ttgcgcggcg tggcccgctt tcagcctcta tgcaaaactc     600
   tcgaaggcct tcagccccga agtcagcgtc aacgtgaacc aaatctacgc cgtagaaggg     660
   caatgcttcg tcctgtcgtc gtgctcggtt atcgatcagg cgatctacga cacgttggtg     720
   cagaacgaat tgcaccagaa gttcctcgag gtgggcgggg gctacagccg aatcttcggg     780
   ccgaacggtg cggaattcgg tgagaatctc ccacccgata gggaaggcct ggtggttgcc     840
   gatatcgatc tcggcctgat ctcgcactcc aagagtgccg ctgatccggc tggtcactac     900
   gcgagggccg acgctctggc gctcatgcat aaccgtaatc cccgccgtcc ggttatcggt     960
   ttcggcgagg cgacgcgcaa ggttgccgac gcactgccta aaggcgcgga acccgcggaa    1020
   gcgctcgaag cggccgagtg a                                              1041
<210> 332
   <211> 346
   <212> PRT
   <213> Unknown
<220>
   <223> Obtained from an environmental sample
<400> 332
   Met Ser Gly Thr Gly Pro Ile Gly Pro Val Val Lys Val Ala Val Val
   1               5                   10                  15
   Gln Ala Ala Pro Cys Leu Leu Asp Leu Asp Ala Gly Val Glu Lys Ala
               20                  25                  30
   Ile Ala Leu Ile Asp Gln Ala Gly Lys Ala Gly Ala Arg Leu Ile Asn
           35                  40                  45
   Phe Pro Glu Ile Trp Leu Pro Gly Tyr Pro Trp Trp Ile Trp Leu Asn
       50                  55                  60
   Pro Pro Ala Ile Asn Met Gln Tyr Val Ala Pro Tyr Met Asn Asn Ser
   65                  70                  75                  80
   Ile Val Ala Gly Ser Lys His Asp His Ala Leu Arg Ala Ala Ala Arg
                   85                  90                  95
   Arg Asn Asn Ile His Val Val Ile Gly Val Ser Glu Arg Ala Gly Gly
               100                 105                 110
   Ser Leu Tyr Met Ala Gln Trp His Tyr Gly Pro Glu Gly Glu Val Ile
           115                 120                 125
   Ser Arg Arg Arg Lys Leu Lys Pro Thr His Val Glu Arg Ser Val Phe
       130                 135                 140
   Gly Glu Gly Asp Gly Ser Asp Met Ile Val Thr Gln Thr Asp Phe Gly
   145                 150                 155                 160
   Arg Val Gly Ala Leu Cys Cys Trp Glu His Leu Gln Pro Leu Ser Lys
                   165                 170                 175
   Tyr Ala Leu Phe Ser Gln Asp Glu Gln Ile His Cys Ala Ala Trp Pro
               180                 185                 190
   Ala Phe Ser Leu Tyr Ala Lys Leu Ser Lys Ala Phe Ser Pro Glu Val
           195                 200                 205
   Ser Val Asn Val Asn Gln Ile Tyr Ala Val Glu Gly Gln Cys Phe Val
       210                 215                 220
   Leu Ser Ser Cys Ser Val Ile Asp Gln Ala Ile Tyr Asp Thr Leu Val
   225                 230                 235                 240
   Gln Asn Glu Leu His Gln Lys Phe Leu Glu Val Gly Gly Gly Tyr Ser
                   245                 250                 255
   Arg Ile Phe Gly Pro Asn Gly Ala Glu Phe Gly Glu Asn Leu Pro Pro
               260                 265                 270
   Asp Arg Glu Gly Leu Val Val Ala Asp Ile Asp Leu Gly Leu Ile Ser
           275                 280                 285
   His Ser Lys Ser Ala Ala Asp Pro Ala Gly His Tyr Ala Arg Ala Asp
       290                 295                 300
   Ala Leu Ala Leu Met His Asn Arg Asn Pro Arg Arg Pro Val Ile Gly
   305                 310                 315                 320
   Phe Gly Glu Ala Thr Arg Lys Val Ala Asp Ala Leu Pro Lys Gly Ala
                   325                 330                 335
   Glu Pro Ala Glu Ala Leu Glu Ala Ala Glu
               340                 345
<210> 333
   <211> 1038
   <212> DNA
   <213> Unknown
<220>
   <223> Obtained from an environmental sample
<400> 333
   atggccattg aacatccccg ctatcgggtc gctgccgttc aggcggcgcc agagttcctt      60
   aatctcgagg caaccgttga caagacgatt gcgcttatcg aggaggccgc ccgcggaggc     120
   gcaagtctca ttgctttccc tgaaacttgg atacctggat atccatggtt tgcctggctt     180
   ggtgcgccga tctggggcat gaaattcatc caggcgtacc atgacaactc gatggtgatc     240
   gacggcgccc agtttgagcg tattgcgcaa gcagcatcgc gttgcaacat cactgttgtg     300
   cttggcttta gcgagaagga cgcaggaagc ctgtatattg cccaggccat cctgagccct     360
   gaagggaaga ccatcgccac gcgtcgcaag ctgaaaccca ctcatgtcga acgcgcgatc     420
   ttcggcgaag gcgacggcag cgacctggca gttcacgaca ccaagctcgg cagggtgggc     480
   gccctttgct gctgggagca tcttcagcca ctttccaaat acgcgatgta tgctcagaac     540
   gagcaggtcc atatcgccgc ctggcccagt tttccccttt acgtcgacgc ggcttacgcg     600
   cttgggccag aggtgaacaa cgccgcgagt cggttgtatg cggtcgaggg ccagtgcttc     660
   gtggttgcgc cgtgtgcaac ggtttcgcaa aagatgatcg atatgctttg cgagacaccc     720
   gagcaacaag cgctcttgaa gccggggggt ggtcacgcgc aaatctacgg tcccgacgga     780
   cgatcgctgg ccgatccgct gcctccggac gcggaggggc tgttgtatgc agacattgac     840
   cttgcagcca tcaccctcgc gaaagcagct gcagatcctg ctggccatta ctctcgccct     900
   gacgtgacac aactgctgct tgaccgcaat ccaaagcccc gtgtcgtgca tgctaagccc     960
   ggccaaagcg ccaacaacag ctcacccggc atgcgggccg tcgagcatac cgagctcgaa    1020
   gaaggtgaac aggcctga                                                  1038
<210> 334
   <211> 345
   <212> PRT
   <213> Unknown
<220>
   <223> Obtained from an environmental sample
<400> 334
   Met Ala Ile Glu His Pro Arg Tyr Arg Val Ala Ala Val Gln Ala Ala
   1               5                   10                  15
   Pro Glu Phe Leu Asn Leu Glu Ala Thr Val Asp Lys Thr Ile Ala Leu
               20                  25                  30
   Ile Glu Glu Ala Ala Arg Gly Gly Ala Ser Leu Ile Ala Phe Pro Glu
           35                  40                  45
   Thr Trp Ile Pro Gly Tyr Pro Trp Phe Ala Trp Leu Gly Ala Pro Ile
       50                  55                  60
   Trp Gly Met Lys Phe Ile Gln Ala Tyr His Asp Asn Ser Met Val Ile
   65                  70                  75                  80
   Asp Gly Ala Gln Phe Glu Arg Ile Ala Gln Ala Ala Ser Arg Cys Asn
                   85                  90                  95
   Ile Thr Val Val Leu Gly Phe Ser Glu Lys Asp Ala Gly Ser Leu Tyr
               100                 105                 110
   Ile Ala Gln Ala Ile Leu Ser Pro Glu Gly Lys Thr Ile Ala Thr Arg
           115                 120                 125
   Arg Lys Leu Lys Pro Thr His Val Glu Arg Ala Ile Phe Gly Glu Gly
       130                 135                 140
   Asp Gly Ser Asp Leu Ala Val His Asp Thr Lys Leu Gly Arg Val Gly
   145                 150                 155                 160
   Ala Leu Cys Cys Trp Glu His Leu Gln Pro Leu Ser Lys Tyr Ala Met
                   165                 170                 175
   Tyr Ala Gln Asn Glu Gln Val His Ile Ala Ala Trp Pro Ser Phe Ser
               180                 185                 190
   Leu Tyr Val Asp Ala Ala Tyr Ala Leu Gly Pro Glu Val Asn Asn Ala
           195                 200                 205
   Ala Ser Arg Leu Tyr Ala Val Glu Gly Gln Cys Phe Val Val Ala Pro
       210                 215                 220
   Cys Ala Thr Val Ser Gln Lys Met Ile Asp Met Leu Cys Glu Thr Pro
   225                 230                 235                 240
   Glu Gln Gln Ala Leu Leu Lys Pro Gly Gly Gly His Ala Gln Ile Tyr
                   245                 250                 255
   Gly Pro Asp Gly Arg Ser Leu Ala Asp Pro Leu Pro Pro Asp Ala Glu
               260                 265                 270
   Gly Leu Leu Tyr Ala Asp Ile Asp Leu Ala Ala Ile Thr Leu Ala Lys
           275                 280                 285
   Ala Ala Ala Asp Pro Ala Gly His Tyr Ser Arg Pro Asp Val Thr Gln
       290                 295                 300
   Leu Leu Leu Asp Arg Asn Pro Lys Pro Arg Val Val His Ala Lys Pro
   305                 310                 315                 320
   Gly Gln Ser Ala Asn Asn Ser Ser Pro Gly Met Arg Ala Val Glu His
                   325                 330                 335
   Thr Glu Leu Glu Glu Gly Glu Gln Ala
               340                 345
<210> 335
   <211> 1053
   <212> DNA
   <213> Unknown
<220>
   <223> Obtained from an environmental sample
<400> 335
   atgactacgc gcgtgattaa agtcgctgcc gcgcagctgt ctccggtgct ggcaactgca      60
   tcggagcaca gccgcgagga cacgattgcg aaagtgatcg acgccatcgc tgcggcgtcg     120
   caacagggcg cgcaactgat tgtgtttccg gaaacagtcg ttccgtatta cccgtatttt     180
   tcatttatca cgcccgcggt aacgatgggt gccgaacatt tgaaattgta cgaacaggca     240
   gtgacggtac ccagcgcagc gacagatgct gtcgctgcgg cggcaaaaaa ttatggcatg     300
   gtagtggtgc tcggaattaa tgaacgcgat cacggctcgc tgtacaacgc gcaattaatt     360
   ttcgacgccg atggtgagct gctattaaag cgtcgaaaaa ttacgccgac ttatcacgaa     420
   cgcatggtgt ggggacaagg cgacggcagc ggtttaaaag ttgtcgatac tgctgccggc     480
   cgtgtcggcg cgctcgcgtg ctgggaacat tacaacccgc tcgcgcgtta cagcctgatg     540
   gcacaacacg aagaaattca ttgcagtcaa tttccgggat cgttggtcgg ccctattttc     600
   gccgaacaaa tggaaatcac catgcgtcat cacgcactcg aatccggctg ttttgtggtg     660
   aatgcaacgg cctggcttag cgatacacaa atccaatcaa ttacccccga taaagccatg     720
   cagaaagcac tgcgcggcgg ttgctacacc gcaatcatct cgcccgaagg caaacatctg     780
   tgcccaccgc tgtatgacgg agaaggaata attgtggcgg aattggactt cgcgttaatc     840
   accaaacgta aacgcatgat ggattccgtc ggccattacg cgcgaccaga actactttct     900
   ttgctcctcg atgatcgcgt aactgcgccg ctcaaaaact tacagacgac gatggcctct     960
   gccaaatccg ctgaagatgg ttttccttta tttgcagacg ttttatatcc agacagttct    1020
   ttcattgaga cgtcgaaatt cgcggagtca tga                                 1053
<210> 336
   <211> 350
   <212> PRT
   <213> Unknown
<220>
   <223> Obtained from an environmental sample
<400> 336
   Met Thr Thr Arg Val Ile Lys Val Ala Ala Ala Gln Leu Ser Pro Val
   1               5                   10                  15
   Leu Ala Thr Ala Ser Glu His Ser Arg Glu Asp Thr Ile Ala Lys Val
               20                  25                  30
   Ile Asp Ala Ile Ala Ala Ala Ser Gln Gln Gly Ala Gln Leu Ile Val
           35                  40                  45
   Phe Pro Glu Thr Val Val Pro Tyr Tyr Pro Tyr Phe Ser Phe Ile Thr
       50                  55                  60
   Pro Ala Val Thr Met Gly Ala Glu His Leu Lys Leu Tyr Glu Gln Ala
   65                  70                  75                  80
   Val Thr Val Pro Ser Ala Ala Thr Asp Ala Val Ala Ala Ala Ala Lys
                   85                  90                  95
   Asn Tyr Gly Met Val Val Val Leu Gly Ile Asn Glu Arg Asp His Gly
               100                 105                 110
   Ser Leu Tyr Asn Ala Gln Leu Ile Phe Asp Ala Asp Gly Glu Leu Leu
           115                 120                 125
   Leu Lys Arg Arg Lys Ile Thr Pro Thr Tyr His Glu Arg Met Val Trp
       130                 135                 140
   Gly Gln Gly Asp Gly Ser Gly Leu Lys Val Val Asp Thr Ala Ala Gly
   145                 150                 155                 160
   Arg Val Gly Ala Leu Ala Cys Trp Glu His Tyr Asn Pro Leu Ala Arg
                   165                 170                 175
   Tyr Ser Leu Met Ala Gln His Glu Glu Ile His Cys Ser Gln Phe Pro
               180                 185                 190
   Gly Ser Leu Val Gly Pro Ile Phe Ala Glu Gln Met Glu Ile Thr Met
           195                 200                 205
   Arg His His Ala Leu Glu Ser Gly Cys Phe Val Val Asn Ala Thr Ala
       210                 215                 220
   Trp Leu Ser Asp Thr Gln Ile Gln Ser Ile Thr Pro Asp Lys Ala Met
   225                 230                 235                 240
   Gln Lys Ala Leu Arg Gly Gly Cys Tyr Thr Ala Ile Ile Ser Pro Glu
                   245                 250                 255
   Gly Lys His Leu Cys Pro Pro Leu Tyr Asp Gly Glu Gly Ile Ile Val
               260                 265                 270
   Ala Glu Leu Asp Phe Ala Leu Ile Thr Lys Arg Lys Arg Met Met Asp
           275                 280                 285
   Ser Val Gly His Tyr Ala Arg Pro Glu Leu Leu Ser Leu Leu Leu Asp
       290                 295                 300
   Asp Arg Val Thr Ala Pro Leu Lys Asn Leu Gln Thr Thr Met Ala Ser
   305                 310                 315                 320
   Ala Lys Ser Ala Glu Asp Gly Phe Pro Leu Phe Ala Asp Val Leu Tyr
                   325                 330                 335
   Pro Asp Ser Ser Phe Ile Glu Thr Ser Lys Phe Ala Glu Ser
               340                 345                 350
<210> 337
   <211> 957
   <212> DNA
   <213> Unknown
<220>
   <223> Obtained from an environmental sample
<400> 337
   atgaagggag tgtgtgccat gtcgacgaaa gttgccatcg tccaggcgcc gccggtcctg      60
   ctgcatcgcg acaggacgat tgcgaaggtg cgttcgtcga tcgaggatgc cgccaatgcg     120
   ggcgcctcgc tgatcgtatt tcccgaagct tatgtacccg gctatccgag ttggatctgg     180
   cgtctcaggc ccggaggcga catgggactg tcgtctgaga ttcacgcaag attgcgggaa     240
   aatgccgttg atctcgcgaa cggaggcctg gcgcatgtcc agggggctgc agcaaaattc     300
   ggcgcgactg tcgttatcgg catcaatgaa ctcgacagcg agttcagcgg aacgacattg     360
   ttcaacaccg tggtggtcat cggccccgac ggaacgcgcc tcaacaggca tcgaaaatta     420
   atgccgacca acccggagcg catggtgtgg ggcacgggcg atgcctcggg tctgcgtgtc     480
   atcgatacgc cggcgggacg gctgggaacc atgatctgct gggagagcta catgccgctg     540
   gcgcgctatg ctctctatgc gcaaggcatc gagatatacg tcgctcccac gtgggacgca     600
   ggcgagagct ggattgctac gatgcgccac atcgccaagg aggccggctg ctgggtgatc     660
   ggcacggcaa ccgtcatcca gggcagcgat gttccggacg attttcccga acgcgacaag     720
   ctcttcaagc cggaggagtg gatcaacgac ggcgatgcgg tcgtggtcaa gcccatgggc     780
   gcgattgctg ccggaccgca caatcgacag aaaagcatac tctacgccga catcgaccgg     840
   gaggccgcgc ggcgagcccg ccggtcgctc gatgtctgtg gccactattc ccgcccagac     900
   gttttctctt tctcggtcaa ccgaaagcca ttccgccctg ccgactttgt gggttga        957
<210> 338
   <211> 313
   <212> PRT
   <213> Unknown
<220>
   <223> Obtained from an environmental sample
<400> 338
   Met Lys Gly Val Cys Ala Met Ser Thr Lys Val Ala Ile Val Gln Ala
    1               5                  10                  15
   Pro Pro Val Leu Leu His Arg Asp Gly Arg Leu Arg Arg Cys Val Arg
               20                  25                  30
   Arg Ser Arg Met Pro Pro Met Arg Ala Pro Arg Ser Tyr Phe Pro Ala
           35                  40                  45
   Tyr Val Pro Gly Tyr Pro Ser Trp Ile Trp Arg Leu Arg Pro Gly Gly
       50                  55                  60
   Asp Met Gly Leu Ser Ser Glu Ile Thr Gln Asp Cys Gly Lys Met Pro
   65                  70                  75                  80
   Leu Ile Ser Arg Thr Glu Ala Trp Arg Met Ser Arg Gly Leu Gln Gln
                   85                  90                  95
   Asn Ser Ala Arg Leu Ser Leu Ser Ala Ser Met Asn Ser Thr Ala Ser
               100                 105                 110
   Ser Ala Glu Arg His Cys Ser Thr Val Val Val Ile Gly Pro Asp Gly
           115                 120                 125
   Thr Arg Leu Asn Arg His Arg Lys Leu Met Pro Thr Asn Pro Glu His
       130                 135                 140
   Gly Val Gly His Gly Arg Cys Leu Gly Ser Ala Cys His Arg Tyr Ala
   145                 150                 155                 160
   Gly Gly Thr Ala Gly Asn His Ile Cys Trp Glu Ser Tyr Met Pro Leu
                   165                 170                 175
   Ala Arg Tyr Ala Leu Tyr Ala Gln Gly Ile Glu Ile Tyr Val Ala Pro
               180                 185                 190
   Thr Trp Asp Ala Gly Glu Ser Trp Ile Ala Thr Met Arg His Ile Ala
           195                 200                 205
   Lys Glu Ala Gly Cys Trp Val Ile Gly Thr Ala Thr Val Ile Gln Gly
       210                 215                 220
   Ser Asp Val Pro Asp Asp Phe Pro Glu Arg Asp Lys Leu Phe Lys Pro
   225                 230                 235                 240
   Arg Ser Gly Ser Thr Thr Ala Met Arg Ser Trp Ser Ser Pro Trp Ala
                   245                 250                 255
   Arg Leu Leu Pro Asp Arg Thr Ile Asp Arg Lys Ala Tyr Ser Thr Pro
               260                 265                 270
   Thr Ser Thr Gly Arg Pro Arg Gly Glu Pro Ala Gly Arg Ser Met Cys
           275                 280                 285
   Gly His Tyr Ser Arg Pro Asp Val Phe Ser Phe Ser Val Asn Arg Lys
       290                 295                 300
   Pro Phe Arg Pro Ala Asp Phe Val Gly
   305                 310
<210> 339
   <211> 1020
   <212> DNA
   <213> Unknown
<220>
   <223> Obtained from an environmental sample
<400> 339
   atgtcaggat cattcaaagt tgcagcagtg caggcggctc cggccttcct caatctcgat      60
   gcgggcatcg acaaggcggt ggcgctgatc gagcaagccg ccgcgcagga cgttcagctc     120
   atcgcctttc ccgagacctg gttgcccgga tacccctggt ggatctggct cgatgcaccc     180
   gccgttacca tgggctatgt cgttccctac aatctcaatt cactagaggc gggcagcccg     240
   caggacaagc gtctggcaaa tgccgcgcgt gagaacaaca tccaggtggt gatgggtctg     300
   tctgaacgcc atgacggcac gctctacatc gcgcagtggc actatggtga agatggcgag     360
   gtgatctcgc gacggcgcaa gctcaagccg acccatgtcg aacgcacggt gttcggggaa     420
   ggcgacggca gcgacatggt ggtcaaggac acaagtctgg gacgggtcgg cgctctgtgc     480
   tgttgggaac acctgcagcc gctcaacaaa tacgcgatgt actcccagaa cgagcagatc     540
   cacatcggtt cctggcccag cttcagcctc tacaagggcg gcgcctatgc gcttggggca     600
   gacctcaaca cggcggcaag ccagatgtat gcggccgagg gccagtgctt tgttctggct     660
   gcctgcgcca cggtcagtca ggacatgttc gacatgctct gcgacacgga aatgaagcag     720
   cagttcctga ccaccggtgg cggattcgcc cgcattttcg gacctgacgg ctcgcccatg     780
   ggcaatgtgc ttgaagaaca tgaagaaggg ctggtgatcg ccgaaatcga tctcaccatg     840
   attgcgatcg ccaaggcggc ggctgacccg tgcgggcact attcccggcc cgatgtgttc     900
   cgactgatgt tcaaccagaa gccaagcccg gtggtgatgc cattcgaaaa cgacgtggcc     960
   cgcgagattg tcgaggccgc cgaggatcag gtcagcgcaa accggctcgc agcggaatga    1020
<210> 340
   <211> 329
   <212> PRT
   <213> Unknown
<220>
   <223> Obtained from an environmental sample
<400> 340
   Met Ser Gly Ser Phe Lys Val Ala Ala Val Gln Ala Ala Pro Ala Phe
   1               5                   10                  15
   Leu Asn Leu Asp Ala Gly Ile Asp Gly Gly Gly Ala Asp Arg Ala Ser
               20                  25                  30
   Arg Arg Ala Gly Arg Ser Ala His Arg Leu Ser Arg Asp Leu Val Ala
           35                  40                  45
   Gly Tyr Pro Trp Trp Ile Trp Leu Asp Ala Pro Ala Val Thr Met Gly
       50                  55                  60
   Tyr Val Val Pro Tyr Asn Leu Asn Ser Arg Gly Gly Gln Pro Ala Gly
   65                  70                  75                  80
   Gln Ala Ser Gly Lys Cys Arg Ala Glu Gln His Pro Gly Gly Asp Gly
                   85                  90                  95
   Leu Ser Glu Arg His Asp Gly Thr Leu Tyr Ile Ala Gln Trp His Tyr
               100                 105                 110
   Gly Glu Asp Gly Glu Val Ile Ala Thr Ala Gln Ala Gln Ala Asp Pro
           115                 120                 125
   Cys Arg Thr His Gly Val Arg Gly Arg Arg Arg Gln Arg His Val Val
       130                 135                 140
   Lys Asp Thr Ser Leu Gly Arg Val Gly Ala Leu Cys Cys Trp Glu His
   145                 150                 155                 160
   Leu Gln Pro Leu Asn Lys Thr Arg Cys Thr Pro Arg Thr Ser Arg Ser
                   165                 170                 175
   Thr Ser Val Pro Gly Pro Ala Ser Ala Ser Thr Arg Ala Ala Leu Cys
               180                 185                 190
   Ala Trp Gly Arg Pro Gln His Gly Gly Lys Pro Asp Val Cys Gly Arg
           195                 200                 205
   Gly Pro Val Leu Cys Ser Leu Pro Ala Pro Arg Ser Val Arg Thr Cys
       210                 215                 220
   Ser Thr Cys Ser Ala Thr Arg Lys Ser Ser Ser Ser Thr Gly Gly Gly
   225                 230                 235                 240
   Phe Ala Arg Ile Phe Gly Pro Asp Gly Ser Pro Met Gly Asn Val Leu
                   245                 250                 255
   Glu Glu His Glu Arg Ala Gly Asp Arg Arg Asn Arg Ser His His Asp
               260                 265                 270
   Cys Asp Arg Gln Gly Gly Gly Pro Val Arg Ala Leu Phe Pro Ala Arg
           275                 280                 285
   Cys Val Pro Thr Asp Val Gln Pro Glu Ala Lys Pro Gly Gly Asp Ala
       290                 295                 300
   Ile Arg Lys Thr Trp Pro Ala Arg Leu Ser Arg Pro Pro Arg Ile Arg
   305                 310                 315                 320
   Ser Ala Gln Thr Gly Ser Gln Arg Asn
                   325
<210> 341
   <211> 1056
   <212> DNA
   <213> Unknown
<220>
   <223> Obtained from an environmental sample
<400> 341
   atggcactga ccaacccaaa atataaagtc gccgccgtcc aggccgcgcc agcgttcctc      60
   gatctggatg cgtccgtcga aaaagcggtc cggctgatcg acgaggccgg cgccaagggc     120
   gcgcgcctca ttgcgttccc ggaaacctgg atccccggct atccctggtg gatctggctc     180
   ggcgcgccgg cctgggcgat catgaaaggt tttgtctcgg cctatttcga caattcgctc     240
   acctatgaca gtccggccgc ggacaaattg cgccaggccg ccaagcgcaa cgatatcgtc     300
   gtggtgctcg gcctgtcgga gcgcgacggc ggcagtctct atatcgcgca atggatcatc     360
   ggcccggacg gcgaaactgt cgcccagcgc cgcaagctca agccgaccca tgtcgagcgt     420
   tcggtgttcg gcgagggcga tggcagcgac cttgccgtgc atgagctcgc gatcgggcgc     480
   gtcggcgcgc tgtgctgctg ggagcatctg caaccgctgt cgaaatacgc catgtatgcg     540
   cagaacgagc aggttcacgt ggcggcctgg ccgagctttt cgctgtacga tccgttcgcc     600
   catgccctcg gtgcggaggt caacaacgcc gccagcaaga tctacgcggt cgagggatcg     660
   tgcttcgtcg tggcaccctg cgccaccgtt tcaaaggaaa tgatcgacct gttgtgcgac     720
   acgccggaca agcacggtct gctgcacgcc ggcggcgggt ttgccgcgat ctacggccct     780
   gacggctcgc cgatcggcga ccgcctggcg cccgaccagg aaggtctgat ctatgccgat     840
   gtcgatctcg gcatgatctc ggtcgcgaag gccgccgccg atccggccgg acattatgcg     900
   cggccggatg tcacaaggtt gctgctcaac aaacgcccgg gcaatcgtgt cgaggcgctg     960
   gcacttccgg tggatcaggt tgcggcaggt gaggagatcc cctcgatatc gcgatcggcc    1020
   agaggggttg ccgaactgcc aaacgcggcc gaatag                              1056
<210> 342
   <211> 342
   <212> PRT
   <213> Unknown
<220>
   <223> Obtained from an environmental sample
<400> 342
   Met Ala Leu Thr Asn Pro Lys Tyr Lys Val Ala Ala Val Gln Ala Ala
   1               5                   10                  15
   Pro Ala Phe Leu Asp Leu Asp Ala Pro Ser Lys Lys Arg Ser Gly Ser
               20                  25                  30
   Thr Arg Pro Ala Pro Arg Ala Arg Ala Ser Leu Arg Ser Arg Thr Trp
           35                  40                  45
   Ile Pro Gly Tyr Pro Trp Trp Ile Trp Leu Gly Ala Pro Ala Trp Ala
       50                  55                  60
   Ile Met Lys Gly Phe Val Ser Pro Ile Ser Thr Ile Arg Ser Pro Met
   65                  70                  75                  80
   Thr Val Arg Pro Arg Thr Asn Cys Ala Arg Pro Pro Ser Ala Thr Tyr
                   85                  90                  95
   Arg Arg Gly Ala Arg Pro Val Gly Ala Arg Arg Arg Gln Ser Leu Tyr
               100                 105                 110
   Arg Ala Met Asp His Arg Pro Thr Ala Lys Leu Ser Pro Ser Ala Ala
           115                 120                 125
   Ser Ser Ser Arg Pro Met Ser Ser Val Arg Cys Ser Ala Arg Ala Met
       130                 135                 140
   Ala Ala Thr Leu Pro Cys Met Ser Ser Arg Ser Gly Ala Ser Ala Arg
   145                 150                 155                 160
   Cys Ala Ala Gly Ser Ile Cys Thr Ala Val Glu Ile Arg His Val Cys
                   165                 170                 175
   Ala Glu Arg Ala Gly Ser Arg Gly Gly Leu Ala Glu Leu Phe Ala Thr
               180                 185                 190
   Ile Arg Ser Pro Met Pro Ser Val Arg Arg Ser Thr Thr Pro Pro Ala
           195                 200                 205
   Arg Ser Thr Arg Ser Arg Asp Arg Ala Ser Ser Trp His Pro Ala Pro
       210                 215                 220
   Pro Phe Gln Arg Lys Ser Thr Cys Cys Ala Thr Arg Arg Thr His Gly
   225                 230                 235                 240
   Leu Leu His Ala Gly Gly Gly Phe Ala Ala Ile Tyr Gly Pro Asp Gly
                   245                 250                 255
   Ser Pro Ile Gly Asp Arg Trp Arg Pro Thr Arg Lys Val Ser Met Pro
               260                 265                 270
   Met Ser Ile Ser Ala Ser Arg Ser Arg Arg Pro Pro Asp Pro Ala Gly
           275                 280                 285
   His Tyr Ala Arg Pro Asp Val Thr Arg Leu Leu Leu Asn Lys Arg Pro
       290                 295                 300
   Gly Asn Arg Val Arg Arg Trp His Phe Arg Trp Ile Arg Leu Arg Gln
   305                 310                 315                 320
   Val Arg Arg Ser Pro Arg Tyr Arg Asp Arg Pro Glu Gly Cys Arg Thr
                   325                 330                 335
   Ala Lys Arg Gly Arg Ile
           340
<210> 343
   <211> 942
   <212> DNA
   <213> Unknown
<220>
   <223> Obtained from an environmental sample
<400> 343
   atgaagtcaa aaattgcggt tattcagcga cctcctgtat tgttagacct tcaggcttca      60
   atcgcccgtg ccattacttc tgttgaggaa gcggcaggca agggatctga gctccttgtt     120
   tttcccgaga catttctgcc tggttatccg tcctggatct ggcgtctcaa gccgggcgga     180
   gacatggtgc tgacatctga aatccacgca aaatatcgcg cgaactctgt tgatgttgag     240
   cgcggggatc tggccccttt atgcgaagcg gcggcgaaac acggcgtcac aattgtcatg     300
   gggctcagtg aaattgatgg gcgctacagc gggactacac tctttaatac agtggtgacc     360
   attggcgcgg aaggagagct ccttaataga caccgcaagc tcatgccgac aaacccagag     420
   cgtatggtct gggggcaagg ggatgcctct ggtctgcggg ttgtcgacac gcccgtgggc     480
   cgcgtcggca cgctgatctg ctgggaaaac tacatgccgc tatcgcgcta tgcgctttat     540
   tctcaaaaca ttgacatcta tgtggcgccg acctgggacg cgggcgagag ctggatcgcc     600
   tccatgcagc atatcgccaa agaaggtggc tgctgggtga tcggcacggc cacggcgatg     660
   gagggctctg atgtcccagc cgacttccct cagcgggagg tgcttttccc tgatagcagc     720
   gaatggatca atgacggtga cgctgtagtg gttaaaccca tgggggcgat tgtcgcgggt      780
   ccgcatcacc gggataagag tattctctat gctgagattg acgtcgaagt ggcacgcaat     840
   gcgcggcgct cgctcgatgt ggcggggcat tactcccggc cggatatttt ttcctttggc     900
   gtggatcgcc ggcctttgcc gccggttacg tttgaggatt ga                        942
<210> 344
   <211> 303
   <212> PRT
   <213> Unknown
<220>
   <223> Obtained from an environmental sample
<400> 344
   Met Lys Ser Lys Ile Ala Val Ile Gln Arg Pro Pro Val Leu Leu Asp
   1               5                   10                  15
   Leu Gln Ala Ser Ile Ala Arg Ala Ile Thr Leu Leu Arg Lys Arg Gln
               20                  25                  30
   Ala Arg Asp Leu Ser Ser Leu Phe Phe Pro Arg His Phe Cys Leu Val
           35                  40                  45
   Ile Arg Pro Gly Ser Gly Val Ser Ser Arg Ala Glu Thr Trp Cys His
       50                  55                  60
   Leu Lys Ser Thr Gln Asn Ile Ala Arg Thr Leu Asp Val Glu Arg Gly
   65                  70                  75                  80
   Asp Leu Ala Pro Leu Cys Glu Ala Ala Ala Lys His Gly Val Thr Ile
                   85                  90                  95
   Val Met Gly Gln Asn Trp Ala Leu Gln Arg Asp Tyr Thr Leu Tyr Ser
               100                 105                 110
   Gly Asp His Trp Arg Gly Arg Arg Leu Leu Asn Arg His Arg Lys Leu
           115                 120                 125
   Met Pro Thr Asn Pro Glu Arg Met Val Trp Gly Gln Gly Asp Ala Ser
       130                 135                 140
   Val Cys Gly Leu Ser Thr Arg Pro Trp Ala Ala Ser Ala Arg Ser Ala
   145                 150                 155                 160
   Gly Lys Thr Thr Cys Arg Tyr Arg Tyr Ala Leu Tyr Ser Gln Asn Ile
                   165                 170                 175
   Asp Ile Tyr Val Ala Pro Thr Trp Asp Ala Gly Glu Ser Trp Ile Ala
               180                 185                 190
   Ser Met Gln His Ile Ala Lys Glu Gly Gly Cys Trp Val Ile Gly Thr
           195                 200                 205
   Ala Thr Ala Met Glu Gly Ser Asp Ser Gln Pro Thr Ser Leu Ser Gly
       210                 215                 220
   Arg Cys Phe Ser Leu Ile Ala Ala Asn Gly Ser Met Thr Val Thr Leu
   225                 230                 235                 240
   Trp Leu Asn Pro Trp Gly Arg Leu Ser Arg Val Arg Ile Thr Gly Ile
                   245                 250                 255
   Arg Val Phe Ser Met Leu Arg Leu Thr Arg Ser Gly Thr Gln Cys Ala
               260                 265                 270
   Ala Leu Ala Arg Cys Gly Gly Ala Leu Leu Pro Ala Gly Tyr Phe Phe
           275                 280                 285
   Leu Trp Val Asp Arg Arg Pro Leu Pro Pro Val Thr Phe Glu Asp
       290                 295                 300
<210> 345
   <211> 1011
   <212> DNA
   <213> Unknown
<220>
   <223> Obtained from an environmental sample
<400> 345
   atgaaagcga tcaaggccgc tgccgtgcag gcagcaccgg tattcctgaa cctcgacgca      60
   tcgatcacca aggcggaaac attcgtcgcc gaggccgccg cgaatggtgc caagctggtg     120
   gcgtttccgg aaacctggct gccgggctat ccctggttca tctggctcgg tgcgcccgcc     180
   gaaggcatgc agttcatccc gcgctatcac gaaaacagca tggagctccg ctcgcccgag     240
   atgcgccgct tgcaggcgat cgcgcgcaag tatgaagtga cgctcgtcat gggctattcc     300
   gagcgcgatg gtggcagccg ctacatgtcc caggtcatta ttggcgatca gggcgacatc     360
   cttctcaatc gccgtaaatt gaagccaacc catgtcgagc ggacggtctt cggcgaaggc     420
   gacggttcgg acctggtggt ggtcgaaacg gcattcggca ggctcggtgc gctcaattgc     480
   tgggaacata tccagccgct cgtcaagatg tcgatgtatg cccagcatga ggaaatccat     540
   gtcgcgggtt ggccgagctt ctgcgtctac cgcgatctcg cctatgccct gggaccggaa     600
   gtcaacaatg ccgtcagtca ggtctatgcc gtggagggta gcgcctatgt tctggcaccc     660
   tgtgcgatcg taagccagga gatgttcgac attctggccg acaagcctga aaaggccttt     720
   ctcctcaatc cccgcacatc caagcccggc ggtggcttca cgcagatcta tgcgccggat     780
   ggtcgaccgc tttgcgagcc gcttgccgac gatgtggaag gcatcctcta tgccgatctc     840
   gatccggcaa cgatcgccgt cgcgaaggcg gccgccgatc ctgcggggca ctattcgcgg     900
   ccggacgcac tctcgctggt gatcaatcgc gaaaagcgcg cggtgatggc tgaaatcaac     960
   gcgccggcga cgccgacctt cacccccatc tccctggacg ctgcggagta g             1011
<210> 346
   <211> 329
   <212> PRT
   <213> Unknown
<220>
   <223> Obtained from an environmental sample
<400> 346
   Met Lys Ala Ile Lys Ala Ala Ala Val Gln Ala Ala Pro Val Phe Leu
   1               5                   10                  15
   Asn Leu Asp Ala Ser Ile Thr Lys Ala Glu Thr Phe Val Ala Glu Ala
               20                  25                  30
   Ala Ala Asn Gly Ala Lys Leu Val Ala Phe Pro Glu Thr Trp Leu Pro
           35                  40                  45
   Gly Tyr Pro Trp Phe Ile Trp Leu Gly Ala Pro Ala Glu Gly Met Gln
       50                  55                  60
   Phe Ile Pro Arg Tyr His Glu Asn His Gly Ala Pro Leu Ala Arg Asp
   65                  70                  75                  80
   Ala Pro Leu Ala Gly Asp Arg Ala Gln Val Ser Asp Ala Arg His Gly
                   85                  90                  95
   Tyr Ser Glu Arg Asp Gly Gly Ser Arg Tyr Met Ser Gln Val Ile Ile
               100                 105                 110
   Gly Asp Gln Gly Asp Ile Leu Leu Asn Arg Arg Lys Leu Lys Pro Thr
           115                 120                 125
   His Val Glu Arg Thr Val Phe Gly Glu Gly Asp Gly Ser Asp Leu Val
       130                 135                 140
   Trp Ser Lys Arg His Ser Ala Gly Ser Val Arg Ser Ile Ala Gly Asn
   145                 150                 155                 160
   Ile Ser Ser Arg Ser Ser Arg Cys Met Tyr Ala Gln His Glu Glu Ile
                   165                 170                 175
   His Val Ala Gly Trp Pro Ser Phe Cys Val Tyr Arg Asp Leu Ala Tyr
               180                 185                 190
   Ala Trp Asp Arg Lys Ser Thr Met Pro Ser Val Arg Ser Met Pro Trp
           195                 200                 205
   Arg Val Ala Pro Met Phe Trp His Pro Val Arg Ser Ala Arg Arg Cys
       210                 215                 220
   Ser Thr Phe Trp Pro Thr Ser Leu Lys Arg Pro Phe Ser Ser Ile Pro
   225                 230                 235                 240
   Ala His Pro Ser Pro Ala Val Ala Ser Arg Arg Ser Met Arg Arg Met
                   245                 250                 255
   Val Asp Arg Phe Ala Ser Arg Leu Pro Thr Val Glu Gly Ile Leu Tyr
               260                 265                 270
   Ala Asp Leu Asp Pro Ala Thr Ile Ala Val Ala Lys Ala Ala Ala Asp
           275                 280                 285
   Pro Ala Gly Thr Ile Arg Gly Arg Thr His Ser Arg Trp Ser Ile Ala
       290                 295                 300
   Lys Ser Ala Arg Trp Leu Lys Ser Arg Ala Gly Asp Ala Asp Leu His
   305                 310                 315                 320
   Pro His Leu Pro Gly Arg Cys Gly Val
                   325
<210> 347
   <211> 909
   <212> DNA
   <213> Unknown
<220>
   <223> Obtained from an environmental sample
<400> 347
   atgaaagtcg cggcgattca agcggcgccg gtctacttgg accggcaagc cacgctggaa      60
   aaggcgcttt ctctaatgga tgaagcggcc gcaaacggcg cccaagtctg cgccttccct     120
   gagaccttcc tcgcgggcta tcccgtctgg atggacttga cggacggcgc caagtggaat     180
   gacgacaaac aaaaagcggc ctacgcatgt tatgtagatg cggccgtcga agccgacgga     240
   cctgagttgc aagccattgc caagaaatcg aaggcgctcg gcctgttcac ctatctgggc     300
   atggtcgaac gcgcggcgtc ggccgggtca gtatattgtt ccctcgctgc cttcgatccc     360
   gacaagggtc tcgtcagcct acaccgaaaa ctcatgccca cctacaccga gcgcctcgtg     420
   tggagccaag gcgacggaca cggactccag gtgcatgaat tcgccggctt caaaatcggc     480
   gcgctaaact gctgggaaaa ttggatgccc ctcgcccgtt acgcaatgta cgcccagggc     540
   gaacagctcc acgtcgcgac ctggcccggg tccccctggc tcaccaagga catcacccgt     600
   ttcatcgccc ttgagggacg catctacgtc atgtccgttg gcggcgtcct tagcgcaaac     660
   gatatccccg actccttccc gcttaaaacc gacctcctca agatccgaga ccgctacctc     720
   agcggcggca ccatgatagt cgccccagac ggcaccaccc tcgaaggccc cgccaaaaac     780
   gaagagacca tactctacgc ggagctcgac ctcaacaccg tcctccaaga acgccaaaat     840
   ttcgatcccg cgggacacta cgcgcgccca gacgtcttcc aattggaaat cgacaaaaat     900
   cgacgatag                                                             909
<210> 348
   <211> 297
   <212> PRT
   <213> Unknown
<220>
   <223> Obtained from an environmental sample
<400> 348
   Met Lys Val Ala Ala Ile Gln Ala Ala Pro Val Tyr Leu Asp Arg Gln
   1               5                   10                  15
   Ala Thr Leu Glu Lys Ala Leu Ser Trp Met Lys Arg Pro Gln Thr Ala
               20                  25                  30
   Pro Lys Ser Ala Pro Ser Leu Arg Pro Ser Ser Arg Ala Ile Pro Trp
           35                  40                  45
   Met Asp Leu Thr Asp Gly Ala Lys Trp Asn Asp Asp Lys Gln Lys Ala
       50                  55                  60
   Ala Tyr Ala Cys Tyr Val Asp Ala Ala Val Glu Ala Asp Gly Pro Glu
   65                  70                  75                  80
   Leu Gln Ala Ile Ala Lys Lys Ser Lys Ala Leu Gly Leu Phe Thr Ile
                   85                  90                  95
   Trp Ala Trp Ser Asn Ala Arg Arg Arg Pro Gly Gln Tyr Ile Val Pro
               100                 105                 110
   Ser Leu Pro Ser Ile Pro Thr Arg Val Ser Ser Ala Tyr Thr Glu Asn
           115                 120                 125
   Ser Cys Pro Pro Thr Pro Ser Ala Ser Cys Gly Ala Lys Ala Thr Asp
           130                             135                         140
   Thr Asp Ser Arg Cys Met Asn Ser Pro Ala Ser Lys Ser Ala Arg Thr
   145                 150                     155                     160
   Ala Gly Lys Ile Gly Cys Pro Ala Arg Tyr Ala Met Tyr Ala Gln Gly
                   165                     170                     175
   Glu Gln Leu His Val Ala Thr Trp Pro Gly Ser Pro Trp Leu Thr Lys
               180                     185                     190
   Asp Ile Thr Arg Phe Ile Ala Leu Glu Gly Arg Ile Tyr Val Met Ser
           195                     200                     205
   Val Gly Gly Val Leu Ser Ala Asn Asp Ile Pro Asp Ser Phe Pro Leu
       210                     215                     220
   Lys Thr Asp Leu Leu Lys Ile Arg Asp Arg Tyr Leu Ser Gly Gly Thr
   225                 230                     235                     240
   Asp Ser Arg Pro Arg Arg His His Pro Arg Arg Pro Arg Gln Lys Arg
                   245                     250                     255
   Arg Asp His Thr Leu Arg Gly Leu Asp Leu Asn Thr Val Leu Gln Glu
               260                     265                     270
   Arg Gln Asn Phe Asp Pro Ala Gly His Tyr Ala Arg Pro Asp Val Ser
           275                     280                     285
   Asn Trp Lys Ser Thr Lys Ile Asp Asp
       290                     295
<210> 349
   <211> 1002
   <212> DNA
   <213> Unknown
<220>
   <223> Obtained from an environmental sample
<400> 349
   atgacgtccc ctgttcaaac caagtacaaa gtcgcctgtg ttcaggcggc gcccgagttt      60
   ctcgatctcg acaaaggcgt tgccaaagcg gtgcgcctga tcgaagaagc cgccacccaa     120
   aaggcctcgc tgatcgcgtt tcccgaagtc tggctccccg gctatccgtg gtggatctgg     180
   ctcgactcgc cggcctgggg cttgcagttc gtccagcgct acttcgaaaa cgctctggtc     240
   gtcggcagcc cccaatggga gcgcttgtgc aaggccgccg ccgacaacaa tatccatgtt     300
   gtgctcggat tctccgaacg ggacggcagc acgctgtacc tcgcacaggc catcatcgat     360
   aacaccggga aggtgatcgc cacgcggcgc aaactcaagc caacacacgc cgaacgcacg     420
   gttttcggcg aaggcgacgg gagccacatc gcggtgcatg aaaccacttt gggccgcatg     480
   ggtgcactct gctgcgccga gcacatccag ccactgacca agtacgccat gtactcgcag     540
   cacgagcaga ttcacattgc cgcatggccc agcttctcgg tctaccgcgg agcagcgttc     600
   cagctgagcg ccgaagccaa caacgccgcg agccaggtct atgccctgga gggcagttgc     660
   tacgtggtgg ccccttgcgc gacggtgtcc aaggagatgt tggacatgct ggctgattcg     720
   ccgcaaaaga agcagctcct gctggaaggc ggtggctacg ccatgattta tgggcccgac     780
   gccaagcccc tgtgcgagcc cattccagag acagaagaag gcattcttta cgcagatgtg     840
   gacctgggct tcatcggtgt caccaaggca gcgtatgacc ccgccggtca ctattcacgc     900
   cccgacgtgc tgcgcctttt gttcaatcgg aagcctgccc ctcgggttca cgatttcgat     960
   cctgaataca cggccaccga gcagaagaca gacgcggcct ga                       1002
<210> 350
   <211> 333
   <212> PRT
   <213> Unknown
<220>
   <223> Obtained from an environmental sample
<400> 350
   Met Thr Ser Pro Val Gln Thr Lys Tyr Lys Val Ala Cys Val Gln Ala
   1               5                   10                  15
   Ala Pro Glu Phe Leu Asp Leu Asp Lys Gly Val Ala Lys Ala Val Arg
               20                  25                  30
   Leu Ile Glu Glu Ala Ala Thr Gln Lys Ala Ser Leu Ile Ala Phe Pro
           35                  40                  45
   Glu Val Trp Leu Pro Gly Tyr Pro Trp Trp Ile Trp Leu Asp Ser Pro
       50                  55                  60
   Ala Trp Gly Leu Gln Phe Val Gln Arg Tyr Phe Glu Asn Ala Leu Val
   65                  70                  75                  80
   Val Gly Ser Pro Gln Trp Glu Arg Leu Cys Lys Ala Ala Ala Asp Asn
                   85                  90                  95
   Asn Ile His Val Val Leu Gly Phe Ser Glu Arg Asp Gly Ser Thr Leu
               100                 105                 110
   Tyr Leu Ala Gln Ala Ile Ile Asp Asn Thr Gly Lys Val Ile Ala Thr
           115                 120                 125
   Arg Arg Lys Leu Lys Pro Thr His Ala Glu Arg Thr Val Phe Gly Glu
       130                 135                 140
   Gly Asp Gly Ser His Ile Ala Val His Glu Thr Thr Leu Gly Arg Met
   145                 150                 155                 160
   Gly Ala Leu Cys Cys Ala Glu His Ile Gln Pro Leu Thr Lys Tyr Ala
                   165                 170                 175
   Met Tyr Ser Gln His Glu Gln Ile His Ile Ala Ala Trp Pro Ser Phe
               180                 185                 190
   Ser Val Tyr Arg Gly Ala Ala Phe Gln Leu Ser Ala Glu Ala Asn Asn
           195                 200                 205
   Ala Ala Ser Gln Val Tyr Ala Leu Glu Gly Ser Cys Tyr Val Val Ala
       210                 215                 220
   Pro Cys Ala Thr Val Ser Lys Glu Met Leu Asp Met Leu Ala Asp Ser
   225                 230                 235                 240
   Pro Gln Lys Lys Gln Leu Leu Leu Glu Gly Gly Gly Tyr Ala Met Ile
                   245                 250                 255
   Tyr Gly Pro Asp Ala Lys Pro Leu Cys Glu Pro Ile Pro Glu Thr Glu
               260                 265                 270
   Glu Gly Ile Leu Tyr Ala Asp Val Asp Leu Gly Phe Ile Gly Val Thr
           275                 280                 285
   Lys Ala Ala Tyr Asp Pro Ala Gly His Tyr Ser Arg Pro Asp Val Leu
       290                 295                 300
   Arg Leu Leu Phe Asn Arg Lys Pro Ala Pro Arg Val His Asp Phe Asp
   305                 310                 315                 320
   Pro Glu Tyr Thr Ala Thr Glu Gln Lys Thr Asp Ala Ala
                   325                 330
<210> 351
   <211> 936
   <212> DNA
   <213> Unknown
<220>
   <223> Obtained from an environmental sample
<400> 351
   atgattacag caggcatcgc agtcgccgct ccggtggtgt tggacaaaac aaaaaccatt      60
   gagaaagccg ttggcattat tcacgaggca gcgggtaagg gtgtgaacct gcttgtgttt     120
   cccgaggcat ttattccctc ctatcccgcc tggggttggc gcctgcgtcc cggtggagat     180
   ttcgggttgt gcgaggagtt gcacgccctg ttgcttgata attcggtaaa tttgcaaggt     240
   gatgacctgg accctgtccg gggcgctgca gccgagcatt caatgaccgt ggtgatggga     300
   ttgaatgagc gcgaaggcca gttcggtcgg gctaccctgt ttaacgccat ggtatttatc     360
   ggtccggacg gcagcatcct gaaccatcat cggaaactta tgccaaccaa tcatgagcgt     420
   acgattcatg gcttcggcga tgcgcgggga ttgaaagtgg tggatacccc gtgcggtcgc     480
   gtgggtggtc tgatttgctg ggagaatttc atgcccctgg ctcgctacgg cctgtatgcc     540
   cagggcgtag aagtgtatgt tgcgcccacc tacgaccagg gtgatgggtg gataggatcc     600
   atgcagcata ttgcccggga aggacggtgc tgggtgttat cggccggaac accgctacgc     660
   ggcagtgatt ttcccgcgga catgccgggc aaggctcaac tgtttcccga tgacgatgaa     720
   tgggtgaatc ccggtgggtc agtggttatc gcaccgggtg gggaattagt ggctggaccg     780
   cttttccgtg aggagggcat ccttgtctgt gaattggatc cggcgaaaag tgctcatgcc     840
   aagcggtcct ttgacgtggc cggtcattac gccaggccag atattttcga gttggaaata     900
   gaccgtgatc cacaggatcc cgtcgagtgg gactga                               936
<210> 352
   <211> 301
   <212> PRT
   <213> Unknown
<220>
   <223> Obtained from an environmental sample
<400> 352
   Met Ile Thr Ala Gly Ile Ala Val Ala Ala Pro Val Val Leu Asp Lys
   1               5                   10                  15
   Thr Lys Thr Ile Glu Lys Ala Val Ala Leu Phe Thr Arg Gln Arg Val
               20                  25                  30
   Arg Val Thr Cys Leu Cys Phe Pro Arg His Leu Phe Pro Pro Ile Pro
           35                  40                  45
   Pro Gly Val Gly Ala Cys Val Pro Val Glu Ile Ser Gly Cys Ala Arg
       50                  55                  60
   Ser Cys Thr Pro Cys Cys Leu Ile Ile Arg Asn Leu Gln Gly Asp Asp
   65                  70                  75                  80
   Leu Asp Pro Val Arg Gly Ala Ala Ala Glu His Ser Met Thr Val Val
                   85                  90                  95
   Met Gly Glu Ala Arg Arg Pro Val Arg Ser Gly Tyr Pro Val Arg His
               100                 105                 110
   Gly Ile Tyr Arg Ser Gly Arg Gln His Pro Glu Pro Ser Ser Glu Thr
           115                 120                 125
   Tyr Ala Asn Gln Ser Ala Tyr Asp Ser Trp Leu Arg Arg Cys Ala Gly
       130                 135                 140
   Ile Glu Ser Gly Gly Tyr Pro Val Arg Ser Arg Gly Trp Ser Asp Leu
   145                 150                 155                 160
   Leu Gly Glu Phe His Ala Pro Gly Ser Leu Gly Leu Tyr Ala Gln Gly
                   165                 170                 175
   Val Glu Val Tyr Val Ala Pro Thr Tyr Asp Gln Gly Asp Gly Trp Ile
               180                 185                 190
   Gly Ser Ala Ala Tyr Cys Pro Gly Arg Thr Val Leu Gly Val Ile Gly
           195                 200                 205
   Arg Asn Thr Ala Thr Arg Gln Phe Ser Arg Thr Cys Arg Ala Arg Leu
       210                 215                 220
   Asn Cys Phe Pro Met Thr Met Asn Gly Ile Pro Val Gly Gln Trp Leu
   225                 230                 235                 240
   Ala Pro Gly Gly Glu Leu Val Ala Gly Pro Leu Phe Arg Glu Glu Gly
                   245                 250                 255
   Ile Leu Val Cys Glu Leu Asp Pro Ala Lys Ser Ala His Ala Lys Arg
               260                 265                 270
   Ser Phe Asp Val Ala Gly His Tyr Ala Arg Pro Asp Ile Phe Glu Leu
           275                 280                 285
   Glu Ile Asp Arg Asp Pro Gln Asp Pro Val Glu Trp Asp
       290                 295                 300
<210> 353
   <211> 1035
   <212> DNA
   <213> Psuedomonas putida ATCC 700801
<400> 353
   atgaatgctt caaaaacaca ttataaagtt gcagctgtgc aagccgctcc tgaattcctg      60
   gatttggaca aaggggttga taaggctata cgcctcatta aagaagcagc tgataacgga     120
   gcatcgctgg ttgcctttcc ggaagtttat cttccagggt atccgtggtg gatctggctt     180
   ggctctcccg catggggaat gcagtttgtt cagcgctacg tagaaaactc gcttgattta     240
   aaaagcgagc agttcgagcg actgtgcaaa gcggctgcta cttaccgtat tcacgttgta     300
   atgggttaca gcgaacgctc gtttggcacc ctctacctcg gtcaagcaat tatcgatgac     360
   agcgggaaag taattggtac gcgccgtaag ctcaagccca cccatgctga gcgtactgtt     420
   tacggtgagg gtaacggcag tgacctcagg gttttcaatt cgcaacttgg aagggtaggt     480
   gcactctgct gcgcagagca cgtacaacca ctctcgaagt ttgcgatgta cagccagcat     540
   gagcagttgc acattgcctc ttggccgagc ttctcggtat atcgcggtgg cgcatatcaa     600
   ctgagcgctg aggctaactg cgcggccacc caagtctatg ctcttgaagg ccagtgcttc     660
   gtaatttcag catgcgcaat cgtatctaaa gacatgctaa acgttctcat cgacacccct     720
   gacaagggta acctgttgca ggatggcggt ggcttcgcga tgatttatgg ccccgatggc     780
   gcaccgctgt gtgaacccct gggcgaacat gaagaaggca tcctttatgc cgacgtcgat     840
   ttgggcgcca tttccgtagc taaagcggca ctcgacccgg ttggccatta ctcgcggcca     900
   gatgttttgc gtctgctttt caacgatcaa ccgacacctt gcgtggaggc gttcaatccg     960
   gctcctgtcg gtactgatgc tccaggtaca gaccttcagg gtgatgagcc cgatgcgcaa    1020
   ccgatatctg aataa                                                     1035
<210> 354
   <211> 312
   <212> PRT
   <213> Psuedomonas putida ATCC 700801
<400> 354
   Met Asn Ala Ser Lys Thr His Tyr Lys Val Ala Ala Val Gln Ala Ala
   1               5                   10                  15
   Pro Glu Phe Leu Asp Leu Asp Lys Gly Val Asp Lys Ala Ile Arg Leu
               20                  25                  30
   Ile Lys Glu Ala Ala Asp Asn Gly Ala Ser Leu Val Ala Phe Pro Glu
           35                  40                  45
   Val Tyr Ser Arg Val Ser Val Val Asp Leu Ala Trp Leu Ser Arg Met
       50                  55                  60
   Gly Asn Ala Val Cys Ser Ala Leu Arg Arg Lys Arg Leu Ile Lys Ala
   65                  70                  75                  80
   Ser Ser Ser Ser Asp Cys Ala Lys Arg Leu Leu Leu Thr Val Phe Thr
                   85                  90                  95
   Leu Trp Leu Gln Arg Thr Leu Val Trp His Pro Leu Pro Arg Ser Ser
               100                 105                 110
   Asn Tyr Arg Gln Arg Glu Ser Asn Trp Tyr Ala Pro Ala Gln Ala His
           115                 120                 125
   Pro Cys Ala Tyr Cys Leu Arg Gly Arg Gln Pro Gln Gly Phe Gln Phe
       130                 135                 140
   Ala Thr Trp Lys Gly Arg Cys Thr Leu Leu Arg Arg Ala Arg Thr Thr
   145                 150                 155                 160
   Thr Leu Glu Val Cys Asp Val Gln Pro Ala Ala Val Ala His Cys Leu
                   165                 170                 175
   Leu Ala Glu Leu Leu Gly Ile Ser Arg Trp Arg Ile Ser Thr Glu Arg
               180                 185                 190
   Gly Leu Arg Gly His Pro Ser Leu Cys Ser Arg Pro Val Leu Arg Asn
           195                 200                 205
   Phe Ser Met Arg Asn Val Ser Lys Asp Met Leu Asn Val Leu Ile Asp
       210                 215                 220
   Thr Pro Asp Lys Gly Asn Leu Leu Gln Asp Gly Gly Gly Phe Ala Met
   225                 230                 235                 240
   Ile Tyr Gly Pro Asp Gly Ala Pro Leu Cys Glu Pro Leu Gly Glu His
                   245                 250                 255
   Glu Glu Gly Ile Leu Tyr Arg Arg Arg Phe Gly Arg His Phe Arg Ser
               260                 265                 270
   Ser Gly Thr Arg Pro Gly Trp Pro Leu Leu Ala Ala Arg Cys Leu Arg
           275                 280                 285
   Leu Leu Phe Asn Asp Gln Pro Thr Pro Cys Val Glu Ala Phe Asn Pro
       290                 295                 300
   Ala Pro Val Gly Thr Asp Ala Pro
   305                 310
<210> 355
   <211> 1014
   <212> DNA
   <213> Unknown
<220>
   <223> Obtained from an environmental sample
<400> 355
   atggccgtct ctaaagacgg tactgtttca ggaaagtcgc ctatccgatt gcatgtcgcc      60
   gcgatacaga tggtcccaaa gctgggtgac gcgcaggcga acgtgaatca ggcagaagcc     120
   cttattcgga aggctcttgg gctgggtgcg cgttggatcg tgttaccaga gatgtttacc     180
   tccggtgcgg cgtttcatcc cgacatgctc aaagccattc agccattcga tggcgcccca     240
   ctccagttgc tgaaagacct ttctcgcaag ggcaatgctg tcatcggcgg ctcgtttctc     300
   gccaagcgtg ggcaacaagt attcaatacc ttcgttttgg tttctccgga cgggtcagtc     360
   gtaacgcatg acaaggattc accgacctat tgggaaaatt gctattaccg gggcggtacc     420
   gatgatggcg tgttgtctac gcccattggc ccggtcggct ccgtcctctg ttgggaattt     480
   atccgctcaa gaaccgcgag acggctggcg aacaaggtca agatggtcgt gggaggctcc     540
   tgttggtgga cgctccccga tgatgctgat ccagacagcc cgcgcagagc cgtgaacctc     600
   aagatgctgc aagaagcgcc ggttcgcatg gcgcggatgc tgggtgttcc ggtaatacat     660
   ggctcccacg cgggcagctt cgaaggattc ttcagtccgg aacttgcgga tgttccctat     720
   aactcgacgt acctgggcga gacaatgatt gtcgacgcgg gtggccgggt acttgcccgt     780
   agagcgcaag atgcaggcga aggcgtggta acggcagaag tggttttgcc cgacaagtcc     840
   gtaccaagcg aacccatccc ggagactttc tggattccca aggaaatgcc ggatgattgg     900
   aaagaagcct gggagcgttg gttcgatacc ggtgcggatt actacgagat ggtgaccgcg     960
   ccctttatca agacgggtgt gataaacgag tacacaccgg aatatcttag gtag          1014
<210> 356
   <211> 325
   <212> PRT
   <213> Unknown
<220>
   <223> Obtained from an environmental sample
<400> 356
   Met Ala Val Ser Lys Asp Gly Thr Val Ser Gly Lys Ser Pro Ile Arg
   1               5                   10                  15
   Leu His Val Ala Ala Ile Gln Met Val Gln Ser Trp Val Thr Arg Arg
               20                  25                  30
   Arg Thr Ile Arg Gln Lys Pro Leu Phe Gly Arg Leu Leu Gly Trp Val
           35                  40                  45
   Arg Val Gly Ser Cys Tyr Gln Arg Cys Leu Pro Pro Val Arg Arg Phe
       50                  55                  60
   Ile Pro Thr Cys Ser Lys Pro Phe Ser Phe Asp Gly Ala Pro Leu Gln
   65                  70                  75                  80
   Leu Leu Lys Asp Leu Ser Arg Lys Gly Asn Ala Val Ile Gly Gly Ser
                   85                  90                  95
   Phe Leu Gln Ala Trp Ala Thr Ser Ile Gln Tyr Leu Arg Phe Gly Phe
               100                 105                 110
   Ser Gly Arg Val Ser Arg Asn Ala Gln Gly Ser Pro Thr Tyr Trp Glu
           115                 120                 125
   Asn Cys Tyr Tyr Arg Gly Gly Thr Asp Asp Gly Val Leu Ser Thr Pro
       130                 135                 140
   Ile Gly Pro Ser Ala Pro Ser Ser Val Gly Asn Leu Ser Ala Gln Glu
   145                 150                 155                 160
   Pro Arg Asp Gly Trp Arg Thr Arg Ser Arg Trp Val Gly Gly Ser Cys
                   165                 170                 175
   Trp Trp Thr Leu Pro Asp Asp Ala Asp Pro Asp Ser Pro Arg Arg Ala
               180                 185                 190
   Val Asn Leu Arg Cys Cys Lys Lys Arg Arg Phe Ala Trp Arg Gly Cys
           195                 200                 205
   Trp Val Phe Arg Tyr Met Ala Pro Thr Arg Gln Leu Arg Arg Ile Leu
       210                 215                 220
   Gln Ser Gly Thr Cys Gly Cys Ser Leu Leu Asp Val Pro Gly Arg Asp
   225                 230                 235                 240
   Asn Asp Val Asp Ala Gly Gly Arg Val Leu Ala Arg Arg Ala Gln Asp
                   245                 250                 255
   Ala Gly Glu Gly Val Val Thr Ala Glu Gly Phe Ala Arg Gln Val Arg
               260                 265                 270
   Thr Lys Arg Thr His Pro Gly Asp Phe Leu Asp Ser Gln Gly Asn Ala
           275                 280                 285
   Gly Ile Gly Lys Lys Pro Gly Ser Val Gly Ser Ile Pro Val Arg Ile
       290                 295                 300
   Thr Thr Arg Trp Pro Arg Pro Leu Ser Arg Arg Val Thr Ser Thr His
   305                 310                 315                 320
   Arg Asn Ile Leu Gly
                   325
<210> 357
   <211> 951
   <212> DNA
   <213> unknown
<220>
   <223> Obtained from an environmental sample
<400> 357
   atgacaaaac tagccatcgt acaaaaaccg ccagtctttc tggataagca aaaaaccatt     60
   gagctggccg tcgccaacat tgaagaggcc gccgccaagg gtgccgatct cgtggtgttt    120
   tctgaagctt tcattcccgg ctatcctgcc tggatctggc gtctacgccc cggcggtgac    180
   tgggggcttt cagaagagtt gcaccagcgt ttgctgcgca atgccgtcaa tgtggactcc    240
   gatgatctgg ctccgttgtt tgaggtcgcc cgcaagcacg aactcaccat cgtttgcggt    300
   atcgaggagc gtgacaacaa actaagtcaa acaaccttat ataacaccgt catcacgatt    360
   ggtcccgatg gatcgttact gaacaaacat cgcaagctta tgcccaccaa cccggagcga    420
   atggtgtggg ggtttggtga cgcatccggt ttaaaagtcg ttgataccaa tgctggtcga    480
   attggctcat taatgtgctg ggaaaattac atgccgctgg ctcgctatgc cctatatgca    540
   caaggtgtcg agatctatat cgcaccgacc tacgacagcg gtgatggctg gataggcagc    600
   atgcagcaca tcgcacgtga agggggctgt tgggtggtgg gatgtgggtg tctcatgaaa      660
   ggcagtgata ttccagatga tttcccggag aaatccacgt tgtatccaga tgcagatgaa      720
   tgggtgaacc cgggtgattc tgtagtgata gcacccggcg gtgaaattat ggccggccca      780
   atgaacagag agtccggtat tttgtatcac gagctagaca gagaaaaagt cagcaacgct      840
   aaacgagcat tcgatgttgc cgggcattat tcacgtcccg atatctttca gctgcatgta      900
   aatacacagg agcagtcacc ctgcgtattc gaaaataatt ccataactta a               951
<210> 358
   <211> 300
   <212> PRT
   <213> Unknown
<220>
   <223> Obtained from an environmental sample
<400> 358
   Met Thr Lys Leu Ala Ile Val Gln Lys Pro Pro Val Phe Leu Asp Lys
   1               5                   10                  15
   Gln Lys Thr Ile Glu Leu Ala Val Pro Thr Leu Lys Arg Pro Pro Pro
               20                  25                  30
   Arg Val Pro Ile Ser Trp Cys Phe Leu Lys Leu Ser Phe Pro Ala Ile
           35                  40                  45
   Leu Trp Ile Trp Arg Leu Arg Pro Gly Gly Asp Trp Gly Leu Ser Glu
       50                  55                  60
   Glu Leu His Gln Arg Leu Leu Arg Asn Ala Val Asn Val Asp Ser Asp
   65                  70                  75                  80
   Asp Leu Ala Pro Leu Phe Glu Val Ala Arg Lys His Glu Leu Thr Ile
                   85                  90                  95
   Val Cys Gly Arg Gly Ala Gln Gln Thr Lys Ser Asn Asn Leu Ile His
               100                 105                 110
   Arg His His Asp Trp Ser Arg Trp Ile Tyr Thr Asn Ile Ala Ser Leu
           115                 120                 125
   Cys Pro Pro Thr Arg Ser Glu Trp Cys Gly Gly Leu Val Thr His Pro
       130                 135                 140
   Leu Lys Val Val Asp Thr Asn Ala Gly Arg Ile Gly Ser Leu Met Cys
   145                 150                 155                 160
   Trp Glu Asn Tyr Met Pro Leu Ala Arg Tyr Ala Leu Tyr Ala Gln Gly
                   165                 170                 175
   Val Glu Ile Tyr Ile Ala Pro Thr Tyr Asp Ser Gly Asp Gly Trp Ile
               180                 185                 190
   Gly Ser Ala Ala His Arg Thr Arg Gly Leu Leu Gly Gly Gly Met Trp
           195                 200                 205
   Val Ser His Glu Arg Gln Tyr Ser Met Ile Ser Arg Arg Asn Pro Arg
       210                 215                 220
   Cys Ile Gln Met Gln Met Asn Gly Thr Arg Val Ile Leu Thr Arg Arg
   225                 230                 235                 240
   Asn Tyr Gly Arg Pro Asn Glu Gln Arg Val Arg Tyr Phe Val Ser Arg
                   245                 250                 255
   Ala Arg Gln Arg Lys Val Ser Asn Ala Lys Arg Ala Phe Asp Val Ala
               260                 265                 270
   Gly His Tyr Ser Arg Pro Asp Ile Phe Gln Leu His Val Ile His Arg
           275                 280                 285
   Ser Ser His Pro Ala Tyr Ser Lys Ile Ile Pro Leu
       290                 295                 300
<210> 359
   <211> 1029
   <212> DNA
   <213> Unknown
<220>
   <223> Obtained from an environmental sample
<400> 359
   atggcaaacg tcgttcgtgc tgcggccgta cagttgagcc ctgttttggg tagtcgcgag      60
   ggtacggtag agaaggtagt tgctgcgatc cgtgacgccg cctcgcaggg cgcacagctg     120
   tgcgttttcc cggagacggt tgttccctat tatccgtatt tctcgttcat tcggccgccc     180
   gcggccatgg gcaaagacca catgcagctg tacgagcaag ctgtggtcgt gccttctccc     240
   agcacgaacg cgattgccgc ggcggccaaa caacactcga tcgtcgtttc aatcggcgtc     300
   aatgaacgcg atcacggtac gatatacaac acgcagttgt tgttcgatgc cgacgggaca     360
   ctcgtgcaac ggcgtcgcaa gataaccccc acgttccacg agcgtatggt gtggggtcaa     420
   ggtgatgggt cgggtttgcg ctgtgtcgac acacaaatcg ggcgcatcgg tagcctggct     480
   tgttgggaac attacaatcc cttggcgcgc tacgcattga tggccgatca cgaagagatc     540
   cacgtcgcca tgtttccggg ttcgatggtg ggtcagatct tcgccgatca aattcaggta     600
   accattcgcc accacgcgct cgaaagcggc tgtttcgtcg tcaacgctac ggggtatctg     660
   agcaaggaac aggtcgccca gttgtcacaa ggcacgtcgc tcgacgcggc gttgaccggt     720
   ggttgttaca ccgcgattgt atcgcctgaa ggcgtcgtac tgggcgaacc gctcaccgac     780
   ggcgaaggca tggtcgtggc ggatatggat ctcagcctca tcaccaaacg caaacgcatg     840
   atggatagcg tcgggcacta cagtcgcccg gaattgctgt ctctgctgat caatcgaacg     900
   ccaacccaca cggcggtcga cgtcgaattc aactccaatt ccgagtctca tcatgtcagc     960
   aatacacgaa caccaaagcg cacaactggc ccacgttcga accttcaagt tgccgctgat    1020
   cgcgagtaa                                                            1029
<210> 360
   <211> 335
   <212> PRT
   <213> Unknown
<220>
   <223> Obtained from an environmental sample
<400> 360
   Met Ala Asn Val Val Arg Ala Ala Ala Val Gln Leu Ser Pro Val Leu
   1               5                   10                  15
   Gly Ser Arg Glu Gly Thr Val Glu Lys Val Val Ala Ala Ile Arg Asp
               20                  25                  30
   Ala Ala Ser Gln Gly Ala Gln Leu Cys Val Phe Pro Glu Thr Val Val
           35                  40                  45
   Pro Tyr Ser Val Phe Leu Val His Ser Ala Ala Arg Gly His Gly Gln
       50                  55                  60
   Arg Pro His Ala Ala Val Arg Ala Ser Cys Gly Arg Ala Phe Ser Gln
   65                  70                  75                  80
   His Glu Arg Asp Cys Arg Gly Gly Gln Thr Thr Leu Asp Arg Arg Phe
                   85                  90                  95
   Asn Arg Arg Met Asn Ala Ile Thr Val Arg Tyr Thr Thr Arg Ser Cys
               100                 105                 110
   Cys Ser Met Pro Thr Gly His Ser Cys Asn Gly Arg Lys Ile Thr Pro
           115                 120                 125
   Thr Phe His Glu Arg Met Val Trp Gly Gln Gly Asp Gly Ser Gly Leu
       130                 135                 140
   Arg Cys Val Asp Thr Gln Ile Gly Arg Ile Gly Ser Leu Ala Cys Trp
   145                 150                 155                 160
   Glu His Tyr Asn Pro Leu Ala Arg Tyr Ala Leu Met Ala Asp His Glu
                   165                 170                 175
   Glu Ile His Val Ala Met Phe Pro Gly Ser Met Val Gly Gln Ile Phe
               180                 185                 190
   Ala Asp Gln Ile Gln Val Pro Phe Ala Thr Thr Arg Ser Lys Ala Ala
           195                 200                 205
   Val Ser Ser Ser Thr Leu Arg Gly Ile Ala Arg Asn Arg Ala Gln Leu
       210                 215                 220
   Ser Gln Gly Thr Ser Leu Asp Ala Ala Leu Thr Gly Gly Cys Tyr Thr
   225                 230                 235                 240
   Ala Ile Val Ser Pro Glu Gly Val Val Leu Gly Glu Pro Leu Thr Asp
                   245                 250                 255
   Gly Glu Gly Met Val Val Ala Asp Met Asp Leu Ser Leu Ile Pro Asn
               260                 265                 270
   Ala Asn Ala Trp Ile Ala Ser Gly Thr Thr Val Ala Arg Asn Cys Cys
           275                 280                 285
   Leu Cys Ser Ile Thr Pro Thr His Thr Ala Val Asp Val Glu Phe Asn
       290                 295                 300
   Ser Asn Ser Glu Ser His His Val Ser Asn Thr Arg His Gln Ser Ala
   305                 310                 315                 320
   Gln Leu Ala His Val Arg Thr Phe Lys Leu Pro Leu Ile Ala Ser
                   325                 330                 335
<210> 361
   <211> 951
   <212> DNA
   <213> Unknown
<220>
   <223> Obtained from an environmental sample
<400> 361
   atgctaaacg agaacaatgg cgctactttc aaggttgctg ccgtgcaggc ttcaccggta      60
   tttcttgatc gcgctgctac aatcgacaag gcttgcgatt taattgctac ggccggacgc     120
   gagggggctc gcctgatcgt ctttccagaa gcgttcgtcc cggcctatcc tgattgggta     180
   tgggcgattc ccgcgggtga tgagggcatg ctcaatgagc tgtatgcaga attacttgcc     240
   aatgctgtca ccattcccag cgatgcgacc gagaggttgt gtcgcgcggc gcggcttgct     300
   aatgcttacg tggtgatggg gatgagcgaa cgcaatgccg aagcgagtgg cgccagcctg     360
   tataatacgc tgttgtatat tgatgcacag ggacaaatcc tgggaaagca ccggaagctg     420
   gttccaacgg gcggcgagcg cctggtatgg gcacagggag atggcagcac cctggaggtt     480
   tacgatactc ctttgggaaa actcggtggc ttaatctgct gggagaatta tatgccgctg     540
   gcacgctata ctatgtatgc ctggggcacg caaatctaca ttgcagcgac gtgggatcgc     600
   gggcagccat ggctatccac tttgcgacac attgctaaag agggcagagt atatgtgatc     660
   ggctgttgta ttgctatgcg caaagatgat atccccgacc attacgcgat gaaggagaag     720
   tattacgcgg aagaagacga gtggatcaat attggcgata gcgcaatcgt caatccagaa     780
   ggggtattta ttgccgggcc agtgcgtaag caagaagaaa tcctctacgc cgaggttgac     840
   ccgcgaatga tgcaggggcc aaagtggatg ctcgacgtgg caggacatta cgcgcgcccg     900
   gatgtattcc agttgacggt gcacacggag aggcggcaga tgatccacta g              951
<210> 362
   <211> 302
   <212> PRT
   <213> Unknown
<220>
   <223> Obtained from an environmental sample
<400> 362
   Met Leu Asn Glu Asn Asn Gly Ala Thr Phe Lys Val Ala Ala Val Gln
    1               5                  10                  15
   Ala Ser Pro Val Phe Leu Asp Arg Ala Tyr Asn Arg Gln Gly Leu Arg
               20                  25                  30
   Phe Asn Cys Tyr Gly Arg Thr Arg Gly Gly Ser Pro Asp Arg Leu Ser
           35                  40                  45
   Arg Ser Val Arg Pro Gly Leu Ser Leu Gly Met Gly Asp Ser Arg Gly
       50                  55                  60
   Gly His Ala Gln Ala Val Cys Glu Leu Leu Ala Asn Ala Val Thr Ile
   65                  70                  75                  80
   Pro Ser Asp Ala Thr Glu Arg Leu Cys Arg Ala Ala Arg Leu Ala Asn
                   85                  90                  95
   Ala Tyr Val Val Met Gly Met Ser Glu Arg Asn Ala Glu Ala Ser Gly
               100                 105                 110
   Ala Ser Leu Tyr Asn Thr Leu Leu Tyr Cys Thr Gly Thr Asn Pro Gly
           115                 120                 125
   Lys Ala Pro Glu Ala Gly Ser Asn Gly Arg Arg Ala Pro Gly Met Gly
       130                 135                 140
   Thr Gly Arg Trp Gln His Pro Gly Gly Leu Arg Tyr Ser Phe Gly Lys
   145                 150                 155                 160
   Thr Arg Trp Leu Asn Leu Leu Arg Ile Ile Cys Arg Trp His Ala Ile
                   165                 170                 175
   Leu Cys Met Pro Gly Ala Arg Lys Ser Thr Leu Gln Arg Arg Gly Ile
               180                 185                 190
   Ala Gly Ser His Gly Tyr Pro Leu Cys Asp Thr Leu Leu Lys Arg Ala
           195                 200                 205
   Glu Tyr Met Ser Ala Val Val Leu Tyr Ala Gln Arg Tyr Pro Arg Pro
       210                 215                 220
   Leu Arg Asp Glu Gly Glu Val Leu Arg Gly Arg Arg Arg Val Asp Gln
   225                 230                 235                 240
   Tyr Trp Arg Arg Asn Arg Gln Ser Arg Arg Gly Ile Tyr Cys Arg Ala
                   245                 250                 255
   Ser Ala Ala Arg Arg Ile Leu Tyr Ala Glu Val Asp Pro Arg Met Met
               260                 265                 270
   Gln Gly Pro Lys Trp Met Leu Asp Val Ala Gly His Tyr Arg Ala Arg
           275                 280                 285
   Met Tyr Ser Ser Arg Cys Thr Arg Arg Gly Gly Arg Ser Thr
   290 295 300
<210> 363
   <211> 1053
   <212> DNA
   <213> Unknown
<220>
   <223> Obtained from an environmental sample
<400> 363
   atgccgaaaa agtcgaccgt ccgggtcgca gccgtccaga ttgcgccgga tctgacatcg 60
   cgggaaaaga cggtggcacg cgtgatcgag gcgatcgccc aggcatccgc caaaggtgcg 120
   gagcttgtgg tttttcccga gacctttgtg ccgtggtatc cttatttctc gttcgtgttg 180
   cccccggtct tgtcgggcaa ggagcacctg cggctctacg aagaggcggt tgcggtgcca 240
   agtgccgcca caagaagcgt agcggctgcc gctcgcgaac atggcatcgt cgtggcgctt 300
   ggcgtcaacg agcgcgacta tggcacgctc tacaatacgc aactgctttt cgatgccgat 360
   ggcagtctga tcctgaagcg gcgcaagatc accccgactt tccacgagcg gatgatctgg 420
   ggccagggcg atgcctcagg cctgaaggtt gtcgacagcg ccattggccg catcggcgcg 480
   ctggcctgct gggaacacta caatccgcta gcccgctatg cgctgatggc gcagcacgag 540
   gaaatccaca ttgcgcagtt tcccggctcc atggtcgggc cgatctttgc cgatcagatg 600
   gaggtgacga tccgccatca cgcgctggaa agcggctgct tcgtcgtcaa tgccacggga 660
   tggctgacgg atgatcagat cgtctcgatc acaccggata ccggcctgca aaaagcgctg 720
   cggggtggct gcatgacggc gatcatttcc cccgaaggca agcatctcgt gccgccgctc 780
   accgaaggtg agggtatcct cgtcgccgat ctcgacatga gcctcattct caagcgcaag 840
   cgcatgatgg attcggtcgg ccactatgcc cggcccgagt tgctgcacct cgtcatggac 900
   gcccggccgg ctgcgccgat gagggaatcg tccatgccca ctgccttccc cggcgaaaca 960
   ttgacaaccg acatgaccga tggagaacag gatgcgtctt tcgacggaaa cgctgatcaa 1020
   cgaattgcag tccttcggag cccggctggt tga 1053
<210> 364
   <211> 335
   <212> PRT
   <213> Unknown
<220>
   <223> Obtained from an environmental sample
<400> 364
   Met Pro Lys Lys Ser Thr Val Arg Val Ala Ala Val Gln Ile Ala Pro
   1 5 10 15
   Asp Leu Thr Ser Arg Glu Lys Gly Gly Thr Arg Asp Arg Gly Asp Arg
   20 25 30
   Pro Gly Ile Arg Gln Arg Cys Gly Ala Cys Gly Phe Ser Arg Asp Leu
   35 40 45
   Cys Arg Gly Ile Leu Ile Ser Arg Ser Cys Cys Pro Arg Ser Cys Arg
   50 55 60
   Ala Arg Ser Thr Cys Gly Ser Thr Lys Arg Arg Leu Arg Cys Gln Val
   65 70 75 80
   Pro Pro Gln Glu Ala Arg Leu Pro Leu Ala Asn Met Ala Ser Ser Trp
   85 90 95
   Arg Leu Ala Ser Thr Ser Ala Thr Met Ala Arg Ser Thr Ile Arg Asn
   100 105 110
   Cys Phe Ser Met Pro Met Ala Val Ser Ser Gly Ala Arg Ser Pro Arg
   115 120 125
   Leu Ser Thr Ser Gly Ser Gly Ala Arg Ala Met Pro Gln Ala Glu Gly
   130 135 140
   Cys Arg Gln Arg His Trp Pro His Arg Arg Ala Gly Leu Leu Gly Thr
   145 150 155 160
   Leu Gln Ser Ala Ser Pro Tyr Ala Leu Met Ala Gln His Glu Glu Ile
   165 170 175
   His Ile Ala Gln Phe Pro Gly Ser Met Val Gly Pro Ile Phe Ala Asp
   180 185 190
   Gln Met Glu Val Thr Ile Arg His His Ala Leu Glu Ser Gly Cys Phe
   195 200 205
   Val Val Asn Ala Thr Gly Trp Arg Met Ile Arg Ser Ser Arg Ser His
   210 215 220
   Arg Ile Pro Ala Cys Lys Lys Arg Cys Gly Val Ala Ala Gly Asp His
   225 230 235 240
   Phe Pro Arg Arg Gln Ala Ser Arg Ala Ala Ala His Arg Arg Gly Tyr
   245 250 255
   Pro Arg Arg Arg Ser Thr Ala Ser Phe Ser Ser Ala Ser Ala Trp Ile
   260 265 270
   Arg Ser Ala Thr Met Pro Gly Pro Ser Cys Cys Thr Ser Ser Trp Thr
   275 280 285
   Pro Gly Arg Leu Arg Arg Gly Asn Arg Pro Cys Pro Leu Pro Ser Pro
   290 295 300
   Ala Lys Ile Asp Asn Arg His Asp Arg Trp Arg Thr Gly Cys Val Phe
   305 310 315 320
   Arg Arg Lys Arg Ser Thr Asn Cys Ser Leu Arg Ser Pro Ala Gly
   325 330 335
<210> 365
   <211> 975
   <212> DNA
   <213> Unknown
<220>
   <223> Obtained from an environmental sample
<400> 365
   atgacaaagc tagccatcgt tcaaaaaccc cccgtctttc tggataaaga aaaaaccata      60
   gcgaagacgg ttgattccat aaaagaggcc gcgacacaaa atgccgactt ggtcatcttc     120
   accgaagcct tcatcccggg ctaccccacc tggatatggc gacttaggcc aggcgctgat     180
   tggggcctct cagaagagct gcacgagcag ttattgcgta acgcggtgag tatgggatcg     240
   accgacctgg atccgcttta tgaagccgcc caacagcata acgtcactat tgtttgcggc     300
   atcgtagaaa gagaccacca actcagccaa tcaaccctct acaacagcat ggtcgtcatt     360
   gacacagacg gaacccttct caacaagcac cgcaaactca tgccaactaa tcccgaacgc     420
   atggtgtggg gctttggcga cgcctcagga ctcaaagccg ttgcaacacc tgcaggccgc     480
   atcagcacgt tgttgtgttg ggagaactac atgccattgg cccgatatgc tctgtatgca     540
   caaggcgtgg aaatctatat cgcgccaact tacgacagtg gtgcgggttg gataggaagc     600
   ttgcaacaca tagcacgcga aggtcgatgc tgggtcgtgg gctgtggcaa cctgattcag     660
   gccagtgatc tgcctgaaga cttcccggac aaggacaacc tctacccgga cgcagaagag     720
   tgggtgaacc cgggtgactc catagtcatt gcaccagacg gtgagattgt ggccggtcca     780
   atgcacaaag agacaggaat tttgtactgc gagatagatc tggagaaagt cagaattgca     840
   aaacgagcat tagacgtgac cgggcattat tcgcgaccgg acgttttcaa actgcatgtg     900
   aatacccgac ctcaatcacc tgtggaattt gaaggtcagg agaccaacaa tccaacaaca     960
   ggagaaagct catga                                                      975
<210> 366
   <211> 315
   <212> PRT
   <213> Unknown
<220>
   <223> Obtained from an environmental sample
<400> 366
   Met Thr Lys Leu Ala Ile Val Gln Lys Pro Pro Val Phe Leu Asp Lys
   1               5                   10                  15
   Glu Lys Thr Ile Ala Lys Thr Val Ile Pro Lys Arg Pro Arg His Lys
               20                  25                  30
   Met Pro Thr Trp Ser Ser Ser Pro Lys Pro Ser Ser Arg Ala Thr Thr
           35                  40                  45
   Trp Ile Trp Arg Leu Arg Pro Gly Ala Asp Trp Gly Leu Ser Glu Glu
       50                  55                  60
   Leu His Glu Gln Leu Leu Arg Arg Gly Glu Tyr Gly Ile Asp Arg Pro
   65                  70                  75                  80
   Gly Ser Ala Leu Ser Arg Pro Thr Ala Arg His Tyr Cys Leu Arg His
                   85                  90                  95
   Arg Arg Lys Arg Pro Pro Thr Gln Pro Ile Asn Pro Leu Gln Gln His
               100                 105                 110
   Gly Arg His His Arg Arg Asn Pro Ser Gln Gln Ala Pro Gln Thr His
           115                 120                 125
   Ala Asn Ser Arg Thr His Gly Val Gly Leu Trp Arg Ala Ser Gly Leu
       130                 135                 140
   Lys Ala Val Ala Thr Pro Ala Gly Arg Ile Ser Thr Leu Leu Cys Trp
   145                 150                 155                 160
   Glu Asn Tyr Met Ile Gly Pro Ile Cys Ser Val Cys Thr Arg Arg Gly
                   165                 170                 175
   Asn Leu Tyr Arg Ala Asn Leu Arg Gln Trp Cys Gly Leu Asp Arg Lys
               180                 185                 190
   Leu Ala Thr His Ser Thr Arg Arg Ser Met Leu Gly Arg Gly Leu Trp
           195                 200                 205
   Gln Pro Asp Ser Gly Ser Asp Leu Pro Glu Asp Phe Pro Asp Lys Asp
       210                 215                 220
   Asn Leu Tyr Pro Asp Ala Glu Glu Trp Val Asn Pro Gly Asp Ser Ile
   225                 230                 235                 240
   Val Ile Ala Pro Asp Gly Glu Ile Val Ala Gly Pro Met His Lys Glu
                   245                 250                 255
   Thr Gly Ile Leu Tyr Arg Asp Arg Ser Gly Glu Ser Gln Asn Cys Lys
               260                 265                 270
   Thr Ser Ile Arg Arg Asp Arg Ala Leu Phe Ala Thr Gly Arg Phe Gln
           275                 280                 285
   Thr Ala Cys Glu Tyr Pro Thr Ser Ile Thr Cys Gly Ile Arg Ser Gly
       290                 295                 300
   Asp Gln Gln Ser Asn Asn Arg Arg Lys Leu Met
   305                 310                 315
<210> 367
   <211> 981
   <212> DNA
   <213> Unknown
<220>
   <223> Obtained from an environmental sample
<400> 367
   atgactgccc actttgccga taccctgacc gttgccgtgg cccagatcgc gccggtgtgg      60
   ctacagcgcg aagctaccct gcaaaagatg ctggtctggg tggagcgcgc cgcgcgcgaa     120
   ggcgcgggcc tggtcgcctt cagtgagggc ctgctgcctg gctacccctt ctggattgaa     180
   cacacggacg gcgcccgctt cgagtcgccg ttacagaagc gcctgtacgc ccactactgc     240
   gatcagtcgg tccagatcaa tgctgggcac ctcgcaccac tctgcgctgc cgcggccaga     300
   caccagattt gggtggtctg cggcgtcatc gagcgcgaca gcgcacgcgg actcagcgtg     360
   tttgcatcaa tggtcaccat cgatgccgaa ggcgcgatcc gcagtgtgca ccgcaagctg     420
   atgccgacct acgaagaacg cctggtgtgg tcgcccggcg acgcgcacgg actgcgctgc     480
   catccgctcg gccagttccg cctcggcagc ctcaattgct gggagaactg gatgccgctg     540
   gcgcgcgccg ccctgtacgc ccagggcgag tctttgcatg ttgcatcctg gcccggcagt     600
   cgccgcaaca ccgagaccat tactcccttc atcgcccgcg aaggccgcag ttacgcgctg     660
   tccgccagtt ccgtgctgca ccgggatgat ctgcccgact ccgttccggc gctgtcggtg     720
   ctgcgcgact gcctgccgga cgtgatggcc gacggcggct cctgcgtcgc cggccccgac     780
   ggacatttcc tgatcgagcc ggtcgtcggc cgggaagagc tgctgctcgc gcagatcgat     840
   catgcccggg tacgcgagga acgtcagaac ttcgacccct tcggccacta ctcgcggccg     900
   gaactcctgt cgctggtggt ggatacgcgc cgggcgagcg gagtgcagat agtgaatgct     960
   gaccatggct ttaagccctg a                                               981
<210> 368
   <211> 317
   <212> PRT
   <213> Unknown
<220>
   <223> Obtained from an environmental sample
<400> 368
   Met Thr Ala His Phe Ala Asp Thr Leu Thr Val Ala Val Ala Gln Ile
   1               5                   10                  15
   Ala Pro Val Trp Leu Gln Arg Glu Tyr Pro Ala Lys Asp Ala Gly Leu
               20                  25                  30
   Gly Gly Ala Arg Arg Ala Arg Arg Arg Gly Pro Gly Arg Leu Gln Gly
           35                  40                  45
   Pro Ala Ala Trp Leu Pro Leu Leu Asp Thr His Gly Arg Arg Pro Leu
       50                  55                  60
   Arg Val Ala Val Thr Glu Ala Cys Thr Pro Thr Thr Ala Ile Ser Arg
   65                  70                  75                  80
   Ser Arg Ser Met Leu Gly Thr Ser His His Ser Ala Leu Pro Ala Arg
                   85                  90                  95
   His Gln Ile Trp Val Val Cys Gly Val Ile Glu Arg Asp Ser Ala Arg
               100                 105                 110
   Gly Leu Ser Val Phe Ala Gln Trp Ser Pro Ser Met Pro Lys Ala Arg
           115                 120                 125
   Ser Ala Val Cys Thr Ala Ser Cys Arg Pro Thr Lys Asn Ala Trp Cys
       130                 135                 140
   Gly Arg Pro Ala Thr Arg Thr Asp Cys Ala Ala Ile Arg Ser Ala Ser
   145                 150                 155                 160
   Ser Ala Ser Ala Ala Gln Leu Leu Gly Glu Leu Asp Ala Ala Gly Ala
                   165                 170                 175
   Arg Arg Pro Val Arg Pro Gly Arg Val Phe Ala Cys Cys Ile Leu Ala
               180                 185                 190
   Arg Gln Ser Pro Gln His Arg Asp His Tyr Ser Leu His Arg Pro Arg
           195                 200                 205
   Arg Pro Gln Leu Arg Ala Ser Ala Ser Ser Val Leu His Arg Asp Asp
       210                 215                 220
   Leu Pro Asp Ser Val Pro Ala Leu Ser Val Leu Arg Asp Cys Leu Arg
   225                 230                 235                 240
   Thr Trp Pro Thr Ala Ala Pro Ala Ser Pro Ala Pro Thr Asp Ile Ser
                   245                 250                 255
   Ser Ser Arg Ser Ser Pro Gly Arg Ala Ala Ala Arg Ala Asp Arg Ser
               260                 265                 270
   Cys Pro Gly Thr Arg Gly Thr Ser Glu Leu Arg Pro Leu Gly His Tyr
           275                 280                 285
   Ser Arg Pro Glu Leu Leu Ser Leu Val Val Asp Thr Arg Arg Ala Ser
       290                 295                 300
   Gly Val Gln Ile Val Asn Ala Asp His Gly Phe Lys Pro
   305                 310                 315
<210> 369
   <211> 1074
   <212> DNA
   <213> Unknown
<220>
   <223> Obtained from an environmental sample
<400> 369
   atgtccgaag agaacaccac caccgaatca agctcggacg cgatctggac cgttgcggcc      60
   gtccaggcgg cgccggtgtt cttgaatcgg gatgcgacgg tgcgtaaggc cgtctcgctg     120
   attgccgagg ccgcggcgca cggcgcgcgt ctcattgttt ttcccgaggc gttcattcca     180
   tcgtacccgg attgggcgtg ggcggtcccg cccggacagg gcggaaccaa ctcgagactg     240
   tatgccaaac tgctcgacaa ttcggttacg gtgcccagcc cagccaccga tgccctggcc     300
   agggcggctc gcgacgcggg cgcctacgtc gtcatgggca taaacgagcg gaacacggcg     360
   gcgagcggcg gaagtctcta caacagcttg ctctatattg gtccggacgg tcgcatcctg     420
   ggcattcatc ggaagttggt gccgacctcg gcggagcggc tgatctgggc acaaggcgat     480
   ggaagcacgc ttggcgtgtt cgatacgccg ggcggccggc ttggcggctt gatctgctgg     540
   gaaaactata tgccgctggc ccgatattcg atgtacgcgc gcggcgtgca aatatatgtt     600
   gcggcgacct gggacagggg cgagccatgg ctttcgacgc tgcgacacat agccaaggaa     660
   ggccagacct acgtgatagg ctgttgcatc gccatgcgga cggccgacat cgacgacgcc     720
   gagctcgtcg agaagtatta cgcggacgcc ggtgagtgga tcaatgaagg cgacagcgcg     780
   attgtcgatc cgagcggaac aatcattgcc ggtccggccc atcagaccaa tgaaatcctc     840
   tacgccgcga tcgatcgcca gaaggtgctg gaatcaaaat ggatgttgga cgtggccggg     900
   cactacgcgc gtccggacgt gttttcattt ggcgttcgaa ccgatgccaa cccgataatg     960
   accatgaacg aaccaagcgc gacggccgag ccgaggcaca atagcgcggg agccgagggt    1020
   cgcgacggcc tacgcgggcg tcgtgaccct cgctcgagaa ttcggcagat gtag          1074
<210> 370
   <211> 346
   <212> PRT
   <213> Unknown
<220>
   <223> Obtained from an environmental sample
<400> 370
   Met Ser Glu Glu Asn Thr Thr Thr Glu Ser Ser Ser Asp Ala Ile Trp
   1               5                   10                  15
   Thr Val Ala Ala Val Gln Ala Ala Gly Val Leu Glu Ser Gly Cys Asp
                   20                  25                  30
   Gly Ala Gly Arg Leu Ala Asp Cys Arg Gly Arg Gly Ala Arg Ala Arg
           35                  40                  45
   Leu Ile Val Phe Pro Glu Ala Phe Ile Pro Ser Tyr Pro Asp Trp Ala
       50                  55                  60
   Trp Ala Val Pro Pro Gly Gln Ala Glu Pro Thr Arg Asp Cys Met Pro
   65                  70                  75                  80
   Asn Cys Ser Thr Ile Arg Leu Arg Cys Pro Ala Gln Pro Pro Met Pro
                   85                  90                  95
   Gly Gln Gly Gly Ser Arg Arg Gly Arg Leu Arg Arg His Gly His Lys
               100                 105                 110
   Arg Ala Glu His Gly Gly Ser Gly Gly Ser Leu Tyr Asn Ser Leu Leu
           115                 120                 125
   Tyr Ile Gly Pro Asp Gly Arg Ile Leu Gly Ile His Arg Lys Leu Cys
       130                 135                 140
   Arg Pro Arg Arg Ser Gly Ser Gly His Lys Ala Met Glu Ala Arg Leu
   145                 150                 155                 160
   Ala Cys Ser Ile Arg Arg Ala Ala Gly Leu Ala Ala Ser Ala Gly Lys
                   165                 170                 175
   Thr Ile Cys Arg Trp Pro Asp Ile Arg Cys Thr Arg Ala Val Gln Ile
               180                 185                 190
   Tyr Val Ala Ala Thr Trp Asp Arg Gly Glu Pro Trp Leu Ser Thr Leu
           195                 200                 205
   Arg His Ile Ala Lys Lys Ala Arg Pro Thr Ala Val Ala Ser Pro Cys
       210                 215                 220
   Gly Arg Pro Thr Ser Thr Thr Pro Ser Ser Ser Arg Ser Ile Thr Arg
   225                 230                 235                 240
   Thr Pro Val Ser Gly Ser Met Lys Ala Thr Ala Arg Leu Ser Ile Arg
                   245                 250                 255
   Ala Glu Ile Ile Ala Gly Pro Ala His Gln Thr Asn Glu Ile Leu Tyr
               260                 265                 270
   Ala Ala Ile Asp Arg Gln Lys Val Leu Glu Lys Met Asp Val Gly Arg
           275                 280                 285
   Gly Arg Ala Leu Arg Ala Ser Gly Arg Val Phe Ile Trp Arg Ser Asn
       290                 295                 300
   Arg Cys Gln Pro Asp Asn Asp His Glu Arg Thr Lys Arg Asp Gly Arg
   305                 310                 315                 320
   Ala Glu Ala Gln Arg Gly Ser Arg Val Ala Thr Ala Tyr Ala Gly Val
                   325                 330                 335
   Val Thr Leu Ala Arg Glu Phe Gly Arg Cys
               340                 345
<210> 371
   <211> 1014
   <212> DNA
   <213> Unknown
<220>
   <223> Obtained from an environmental sample
<400> 371
   atgggtatcg aacatccgaa gtacaaggtt gcggtggtac aggcggcgcc ggcctggctc      60
   gatctcgacg catcgatcgc caagaccatc gcgctgatcg aggaggcggc cgccaagggc     120
   gccaagctga tcgcattccc tgaggccttc attcccggct atccttggca tatctggatg     180
   gactcgccgg cctgggcgat cgggcgcgga tttgtgcagc gctatttcga caattcgctt     240
   tcctacgaca gcccgcaggc cgaacggctg cggctcgcgg tgaagaaggc cggcatcacc     300
   gccgtgctcg gcctgtccga gcgggaaggc ggcagccttt atctcgcgca atggctgatc     360
   ggtcccgacg gcgagaccat cgccaagcgg cgcaagctgc ggccgacgca tgccgagcgc     420
   accgtctacg gcgaaggcga cggcagtgac cttgcggtcc atgaccgcgc tgacattggc     480
   cgtctcggcg cgctgtgctg ctgggaacat ctgcagccgc tgtcgaaata cgccatgtat     540
   gcccagaacg agcaggtgca tgtcgcggcc tggccgagtt tttcgctgta cgacccgttc     600
   gcgccggcgc tgggctggga ggtcaacaac gcggcatccc gcgtctatgc ggtcgaaggc     660
   tcctgcttcg tgctggcgcc gtgtgccacc gtctcgcagg cgatggtgga cgaactctgc     720
   gaccgcgacg acaagcatgc gctgctgcat gtcggcggcg gccacgccgc gatctacgga     780
   ccggacggca gctcgatggc gaacaagctc gatcccgagc aggagggcct gctgttcgcc     840
   gacatcgatc tcggggcgat cggggtggca aagaacgccg ccgatccggc cgggcactat     900
   tcgcggccgg atgtgacccg tctgctcttg aacagaaaac cctcaaagcg cgtcgagcac     960
   tttgcgctgc cgctcgacca tctcgcggac gagggcgttg ctccggtgac ctga          1014
<210> 372
   <211> 327
   <212> PRT
   <213> Unknown
<220>
   <223> Obtained from an environmental sample
<400> 372
   Met Gly Ile Glu His Pro Lys Tyr Lys Val Ala Val Val Gln Ala Ala
   1               5                   10                  15
   Pro Ala Trp Leu Asp Leu Asp Ala Arg Ser Pro Arg Pro Ser Arg Ser
               20                  25                  30
   Arg Arg Arg Pro Pro Arg Ala Pro Ser Ser His Ser Leu Ala Phe Ile
           35                  40                  45
   Pro Gly Tyr Pro Trp His Ile Trp Met Asp Ser Pro Ala Trp Ala Ile
       50                  55                  60
   Gly Arg Gly Phe Val Gln Ala Ile Ser Thr Ile Arg Phe Pro Thr Thr
   65                  70                  75                  80
   Ala Arg Arg Pro Asn Gly Cys Gly Ser Arg Arg Arg Pro His His Arg
                   85                  90                  95
   Arg Ala Arg Pro Val Arg Ala Gly Arg Arg Gln Pro Leu Ser Arg Ala
               100                 105                 110
   Met Ala Asp Arg Ser Thr Ala Arg Pro Ser Pro Ser Gly Ala Ser Cys
           115                 120                 125
   Gly Arg Arg Met Pro Ser Ala Pro Ser Thr Ala Lys Ala Thr Ala Val
       130                 135                 140
   Thr Leu Arg Ser Met Thr Ala Leu Thr Leu Ala Val Ser Ala Arg Cys
   145                 150                 155                 160
   Ala Ala Gly Asn Leu Gln Pro Leu Ser Lys Tyr Ala Met Tyr Ala Gln
                   165                 170                 175
   Asn Glu Gln Val His Val Ala Ala Trp Pro Ser Phe Ala Val Arg Pro
               180                 185                 190
   Val Arg Ala Gly Ala Gly Leu Gly Gly Gln Gln Arg Gly Ile Pro Arg
           195                 200                 205
   Leu Cys Gly Arg Gly Ser Cys Phe Val Leu Ala Pro Cys Ala Thr Val
       210                 215                 220
   Ser Gln Ala Met Val Asp Glu Leu Cys Asp Arg Asp Gln Ala Cys Ala
   225                 230                 235                 240
   Ala Ala Cys Arg Arg Arg Pro Arg Arg Asp Leu Arg Thr Gly Arg Gln
                   245                 250                 255
   Leu Asp Gly Thr Ser Ser Ile Pro Ser Arg Arg Ala Cys Cys Ser Pro
               260                 265                 270
   Thr Ser Ile Ser Gly Arg Ser Gly Trp Gln Arg Thr Pro Pro Ile Arg
           275                 280                 285
   Pro Gly Thr Ile Arg Gly Arg Met Pro Val Cys Ser Thr Glu Asn Pro
       290                 295                 300
   Gln Ala Arg Arg Ala Leu Cys Ala Ala Ala Arg Pro Ser Arg Gly Arg
   305                 310                 315                 320
   Gly Arg Cys Ser Gly Asp Leu
                   325
<210> 373
   <211> 1056
   <212> DNA
   <213> Unknown
<220>
   <223> Obtained from an environmental sample
<400> 373
   atgacgaatt ttctggacgt gacggtggca gcggttcagg cggctcccgt ctatttcgat     60
   cgggaggcat cgacggagaa agcgtgccgg ttgatccacg aagcggcagg gctcggcgcg    120
   acgctcgcag cgttcggcga aacctggttg ccagggtatc cgttcttcgt ctgggggttc    180
   gcgcacaacc ggagcctgtt ctggcaggcc gccgccgagt acatcgccaa tgcggtggag    240
   attccgagtc ccacaacgga ccgtctctgt gcggcggcga aggctgccgg ggtcgacgtc    300
   gtcattggcg tcgttgaact ggatgaacga acacgagctt cggtttacag tacgctgctt    360
   ttcatcggtc gcgacgggac gatcctgggc cgccaccgca agctgaagcc aacacacatg    420
   gagcggacga tctggggcga aggggacgca tatggactcc gcgtctacga acgttcgtac    480
   gggcggctga gcggcctgaa ttgctgggaa cacaatatga tgctgcccgg ctacgtgctt     540
   gccgcacagg gcacgcagtt tcacgtcgcc gcatggcccg gaaaggagag gctcaccgtc     600
   ccgccgaacg aagcggctta tacgcgccag cttctcctct ctcgcgcgta tgcatcccag     660
   gccggcgcgt acgtgatcag cgtcgccggg ctgctcgcac cagactccat gcccgagcgt     720
   tatcgcgagt tagggcggtc atatgagttg accggcgaca gcgtcatcgt cgacccgcgc     780
   ggcgaggtca ttgccgggcc tgcaaaaggc gagaccatcc tgctcgcgca gtgcagtcag     840
   gaagctctcc tcgcggccaa gtccgccatc gacctcggcg gccattactc acgcccggat     900
   atctttcagc tgcgtgtcaa cgatcaactg cagcatcggg tccggagagt tgagccacac     960
   ttcacggcgg cgatcggaca tatcggagcc gagcgccgat cccaggagga tggtactggt    1020
   cccttcgacc tggcggaatc tctcacgaac tcctag                              1056
<210> 374
   <211> 351
   <212> PRT
   <213> Unknown
<220>
   <223> Obtained from an environmental sample
<400> 374
   Met Thr Asn Phe Leu Asp Val Thr Val Ala Ala Val Gln Ala Ala Pro
   1               5                   10                  15
   Val Tyr Phe Asp Arg Glu Ala Ser Thr Glu Lys Ala Cys Arg Leu Ile
               20                  25                  30
   His Glu Ala Ala Gly Leu Gly Ala Thr Leu Ala Ala Phe Gly Glu Thr
           35                  40                  45
   Trp Leu Pro Gly Tyr Pro Phe Phe Val Trp Gly Phe Ala His Asn Arg
       50                  55                  60
   Ser Leu Phe Trp Gln Ala Ala Ala Glu Tyr Ile Ala Asn Ala Val Glu
   65                  70                  75                  80
   Ile Pro Ser Pro Thr Thr Asp Arg Leu Cys Ala Ala Ala Lys Ala Ala
                   85                  90                  95
   Gly Val Asp Val Val Ile Gly Val Val Glu Leu Asp Glu Arg Thr Arg
               100                 105                 110
   Ala Ser Val Tyr Ser Thr Leu Leu Phe Ile Gly Arg Asp Gly Thr Ile
           115                 120                 125
   Leu Gly Arg His Arg Lys Leu Lys Pro Thr His Met Glu Arg Thr Ile
       130                 135                 140
   Trp Gly Glu Gly Asp Ala Tyr Gly Leu Arg Val Tyr Glu Arg Ser Tyr
   145                 150                 155                 160
   Gly Arg Leu Ser Gly Leu Asn Cys Trp Glu His Asn Met Met Leu Pro
                   165                 170                 175
   Gly Tyr Val Leu Ala Ala Gln Gly Thr Gln Phe His Val Ala Ala Trp
               180                 185                 190
   Pro Gly Lys Glu Arg Leu Thr Val Pro Pro Asn Glu Ala Ala Tyr Thr
           195                 200                 205
   Arg Gln Leu Leu Leu Ser Arg Ala Tyr Ala Ser Gln Ala Gly Ala Tyr
       210                 215                 220
   Val Ile Ser Val Ala Gly Leu Leu Ala Pro Asp Ser Met Pro Glu Arg
   225                 230                 235                 240
   Tyr Arg Glu Leu Gly Arg Ser Tyr Glu Leu Thr Gly Asp Ser Val Ile
                   245                 250                 255
   Val Asp Pro Arg Gly Glu Val Ile Ala Gly Pro Ala Lys Gly Glu Thr
               260                 265                 270
   Ile Leu Leu Ala Gln Cys Ser Gln Glu Ala Leu Leu Ala Ala Lys Ser
           275                 280                 285
   Ala Ile Asp Leu Gly Gly His Tyr Ser Arg Pro Asp Ile Phe Gln Leu
       290                 295                 300
   Arg Val Asn Asp Gln Leu Gln His Arg Val Arg Arg Val Glu Pro His
   305                 310                 315                 320
   Phe Thr Ala Ala Ile Gly His Ile Gly Ala Glu Arg Arg Ser Gln Glu
                   325                 330                 335
   Asp Gly Thr Gly Pro Phe Asp Leu Ala Glu Ser Leu Thr Asn Ser
               340                 345                 350
<210> 375
   <211> 939
   <212> DNA
   <213> Unknown
<220>
   <223> Obtained from an environmental sample
<400> 375
   atgaccaaaa tcgctgtcat tcaagaaccc ccggtctatc tgaatctgag caaatcgatg      60
   gaccgagcgg tcgacttgat tgccgatgcc gcgagccagg ggtgtcagtt gattgtgttt     120
   cccgaagcct ggcttgcagg ttaccccacc ttcgtctggc gtcttgcgcc gggcagcgga     180
   atgggaaaaa ccgatgagct ttacgcgcgt ttgctcgcca actcggtcga ccgcagcaaa     240
   gaggggcttc ggcctttgca ggaggctgca aaagagcatg gcgttgtcat tgttctgggt     300
   tatcaagagg tggacggctc gggcagcagc agcacaatct tcaacagctg cgcgattatt     360
   gatgccgacg ggcgactggc caacaaccat cgcaagttga tgcccaccaa tgcggagcgg     420
   atggtgtggg ggtttggcga cggttcgggc ctgaacgttg ttgacaccgc ggtgggcagg     480
   atcggcacgc tgatttgctg ggaaaactac atgcccttgg cgcgctacgc gctgtatgcc     540
   caaaacatcg aaatctatgt ggcgccgacc tgggacagcg gtgccatgtg gcaagccacc     600
   ctgcaacata tcgcacgtga aggtggctgc tgggtcatcg gatgtgcaac ctcgctgcaa     660
   gcctctgaca tcccggacga ccttccccat cgggatgagt tattcccgaa caaagacgaa     720
   tgggtgaacc ctggcgatgc ggtggtttac aaaccttttg gcggccttgt ggccggcccc     780
   atgcatcagg aaaaggggct tctcatcgca gagttggacg tcgccgctgt tcaggtctca     840
   cgtcggaagt tcgatgcgac cgggcattac gctcgccccg atgtcttcca actgcacgtg     900
   aatcgcagcg cgatgcggcc ggttgagttc acgaattag                            939
<210> 376
   <211> 312
   <212> PRT
   <213> Unknown
<220>
   <223> Obtained from an environmental sample
<400> 376
   Met Thr Lys Ile Ala Val Ile Gln Glu Pro Pro Val Tyr Leu Asn Leu
   1 5 10 15
   Ser Lys Ser Met Asp Arg Ala Val Asp Leu Ile Ala Asp Ala Ala Ser
   20 25 30
   Gln Gly Cys Gln Leu Ile Val Phe Pro Glu Ala Trp Leu Ala Gly Tyr
   35 40 45
   Pro Thr Phe Val Trp Arg Leu Ala Pro Gly Ser Gly Met Gly Lys Thr
   50 55 60
   Asp Glu Leu Tyr Ala Arg Leu Leu Ala Asn Ser Val Asp Arg Ser Lys
   65 70 75 80
   Glu Gly Leu Arg Pro Leu Gln Glu Ala Ala Lys Glu His Gly Val Val
   85 90 95
   Ile Val Leu Gly Tyr Gln Glu Val Asp Gly Ser Gly Ser Ser Ser Thr
   100 105 110
   Ile Phe Asn Ser Cys Ala Ile Ile Asp Ala Asp Gly Arg Leu Ala Asn
   115 120 125
   Asn His Arg Lys Leu Met Pro Thr Asn Ala Glu Arg Met Val Trp Gly
   130 135 140
   Phe Gly Asp Gly Ser Gly Leu Asn Val Val Asp Thr Ala Val Gly Arg
   145 150 155 160
   Ile Gly Thr Leu Ile Cys Trp Glu Asn Tyr Met Pro Leu Ala Arg Tyr
   165 170 175
   Ala Leu Tyr Ala Gln Asn Ile Glu Ile Tyr Val Ala Pro Thr Trp Asp
   180 185 190
   Ser Gly Ala Met Trp Gln Ala Thr Leu Gln His Ile Ala Arg Glu Gly
   195 200 205
   Gly Cys Trp Val Ile Gly Cys Ala Thr Ser Leu Gln Ala Ser Asp Ile
   210 215 220
   Pro Asp Asp Leu Pro His Arg Asp Glu Leu Phe Pro Asn Lys Asp Glu
   225 230 235 240
   Trp Val Asn Pro Gly Asp Ala Val Val Tyr Lys Pro Phe Gly Gly Leu
   245 250 255
   Val Ala Gly Pro Met His Gln Glu Lys Gly Leu Leu Ile Ala Glu Leu
   260 265 270
   Asp Val Ala Ala Val Gln Val Ser Arg Arg Lys Phe Asp Ala Thr Gly
   275 280 285
   His Tyr Ala Arg Pro Asp Val Phe Gln Leu His Val Asn Arg Ser Ala
   290 295 300
   Met Arg Pro Val Glu Phe Thr Asn
   305 310
<210> 377
   <211> 1050
   <212> DNA
   <213> Unknown
<220>
   <223> Obtained from an environmental sample
<400> 377
   atgtccagca ccacccatcc ccgcctgcgc gtcgccgcgg tgcaagccgc ccccgtcttc     60
   ctcgacctcg acggcaccat cgacaagacc atcgacctga tggcccgggc ggccggccag    120
   ggcgtgcagc tgatcgcctt tcccgagacc tgggtgcccg gctatccgtg gtggatctgg    180
   ctcgattcgc cggcctgggg catgcagttc gtgcagcgct accacgacaa cgccctggtc    240
   gtcggctcgc ccgagttcga ccgcattcgc gaggccgcgc gcaagcaccg catctgggtc    300
   tcgctcggct acagcgagaa ggccgccggc agcctctaca tcgcccaggc gctgatcgac    360
   gaccagggca acacgctgca gactcggcgc aagctcaagc cgacgcacgt ggagcgcacc    420
   gtgttcggcg agggcgacgg atcggacctg agcgtggtcg agacggctat cggcaacatc    480
   ggctcgctgt cgtgctggga gcacctgcag ccgctcagca agtacgcgat gtactcgcag    540
   aacgagcaga tccattgcgg cgcctggccc agcttctcgc tctaccgcgg cggcgcctac    600
   gcgctcggcg ccgaagtgaa caacgccgcc agccaggtgt acgcggccga gggccagtgc    660
   ttcgtgatcg cgccctgcgc cacggtctcg aaggcgatgc acgaactgct gtgcaccgac    720
   cctggcaagc agcagatgct gctggtcggc ggcggcttcg cgcgcatcta cggacccgac    780
   ggatcgccgc tcggcaagaa cctggcagag gacgaggaag ggctggtggt ggccgacatc    840
   gacctcggca tgatctccct ggccaaggcg gccggcgacc cggccggcca ctattcgcgg    900
   cccgacgtga cgcagttgct gttcaacagg aagcggcgcg agccggtggt gctgcaaggc    960
   ccggccgagc ccgagaaggc ggtcgccgag ccggtgtcca cgccgagcga agcggcggcg   1020
   gccgccgccc gccagccggt cgtggcctga                                    1050
<210> 378
   <211> 349
   <212> PRT
   <213> Unknown
<220>
   <223> Obtained from an environmental sample
<400> 378
   Met Ser Ser Thr Thr His Pro Arg Leu Arg Val Ala Ala Val Gln Ala
   1               5                   10                  15
   Ala Pro Val Phe Leu Asp Leu Asp Gly Thr Ile Asp Lys Thr Ile Asp
               20                  25                  30
   Leu Met Ala Arg Ala Ala Gly Gln Gly Val Gln Leu Ile Ala Phe Pro
           35                  40                  45
   Glu Thr Trp Val Pro Gly Tyr Pro Trp Trp Ile Trp Leu Asp Ser Pro
       50                  55                  60
   Ala Trp Gly Met Gln Phe Val Gln Arg Tyr His Asp Asn Ala Leu Val
   65                  70                  75                  80
   Val Gly Ser Pro Glu Phe Asp Arg Ile Arg Glu Ala Ala Arg Lys His
                   85                  90                  95
   Arg Ile Trp Val Ser Leu Gly Tyr Ser Glu Lys Ala Ala Gly Ser Leu
               100                 105                 110
   Tyr Ile Ala Gln Ala Leu Ile Asp Asp Gln Gly Asn Thr Leu Gln Thr
           115                 120                 125
   Arg Arg Lys Leu Lys Pro Thr His Val Glu Arg Thr Val Phe Gly Glu
       130                 135                 140
   Gly Asp Gly Ser Asp Leu Ser Val Val Glu Thr Ala Ile Gly Asn Ile
   145                 150                 155                 160
   Gly Ser Leu Ser Cys Trp Glu His Leu Gln Pro Leu Ser Lys Tyr Ala
                   165                 170                 175
   Met Tyr Ser Gln Asn Glu Gln Ile His Cys Gly Ala Trp Pro Ser Phe
               180                 185                 190
   Ser Leu Tyr Arg Gly Gly Ala Tyr Ala Leu Gly Ala Glu Val Asn Asn
           195                 200                 205
   Ala Ala Ser Gln Val Tyr Ala Ala Glu Gly Gln Cys Phe Val Ile Ala
       210                 215                 220
   Pro Cys Ala Thr Val Ser Lys Ala Met His Glu Leu Leu Cys Thr Asp
   225                 230                 235                 240
   Pro Gly Lys Gln Gln Met Leu Leu Val Gly Gly Gly Phe Ala Arg Ile
                   245                 250                 255
   Tyr Gly Pro Asp Gly Ser Pro Leu Gly Lys Asn Leu Ala Glu Asp Glu
               260                 265                 270
   Glu Gly Leu Val Val Ala Asp Ile Asp Leu Gly Met Ile Ser Leu Ala
           275                 280                 285
   Lys Ala Ala Gly Asp Pro Ala Gly His Tyr Ser Arg Pro Asp Val Thr
       290                 295                 300
   Gln Leu Leu Phe Asn Arg Lys Arg Arg Glu Pro Val Val Leu Gln Gly
   305                 310                 315                 320
   Pro Ala Glu Pro Glu Lys Ala Val Ala Glu Pro Val Ser Thr Pro Ser
                   325                 330                 335
   Glu Ala Ala Ala Ala Ala Ala Arg Gln Pro Val Val Ala
               340                 345
<210> 379
   <211> 936
   <212> DNA
   <213> Unknown
<220>
   <223> obtained from an environmental sample
<400> 379
   atggctaaaa tagcgattgt acaaaaggcg tctgttacct tgaataaaca agaaactgtc      60
   gccagtgctg taagagaggt agagctggca gcggcggaag gtgccgagct ggtagtgttt     120
   actgaggcat ttattgctgg gtatccggcc tggatctggc gtttgcggcc aggtggtgac     180
   tgggggctat ctgaagatct tcattcccgt ttgttgacaa gcgccgtaga cctgggtggt     240
   gatgacctgg atccacttta tgccgcagct aaagaaaata acgtgacgat agtgtgcggt     300
   attaatgaac gtgataaccg gctcagtaag gcaacgctat ataattctat cgttattatt     360
   ggttccgatg gttcattgtt aaatcgacat cgtaagttga tgccgacgaa tccggagaga     420
   atggtatggg gctttggtga tgcctctggt ctgaaggtcg ttgatacccc cgttggtcgt     480
   gttggtacgc ttgtctgttg ggaaaactat atgcccttgg ccagatatgc gttgtattcg     540
   cagggggtag aggtttatat tgcgccgacc tacgatagcg gtgatgactg gatttctaca     600
   ttacagcata ttgccaggga gggtcgttgt tgggttgttg gctgtggcaa tctattgcgt     660
   ggcagcgata taccggatga cttccctgag aagttggcgt tatacccaga tgaggatgag     720
   tggataaatc ctggggattc cgttgtgatt gcacctgggg gtaaaatcat ggccgggcca     780
   ttgcgccagg aggcggggat tgtctattgt gatattgcgt ctgaaagtgc cagtcaggca     840
   aaacgtgcgc tggatgtggc tggacattat tcccggcctg atatctttga gttgcatgtg     900
   aatacgaagg tgcagacccc ggttgtatat gattag                               936
<210> 380
   <211> 311
   <212> PRT
   <213> Unknown
<220>
   <223> Obtained from an environmental sample
<400> 380
   Met Ala Lys Ile Ala Ile Val Gln Lys Ala Ser Val Thr Leu Asn Lys
   1 5 10 15
   Gln Glu Thr Val Ala Ser Ala Val Arg Glu Val Glu Leu Ala Ala Ala
   20 25 30
   Glu Gly Ala Glu Leu Val Val Phe Thr Glu Ala Phe Ile Ala Gly Tyr
   35 40 45
   Pro Ala Trp Ile Trp Arg Leu Arg Pro Gly Gly Asp Trp Gly Leu Ser
   50 55 60
   Glu Asp Leu His Ser Arg Leu Leu Thr Ser Ala Val Asp Leu Gly Gly
   65 70 75 80
   Asp Asp Leu Asp Pro Leu Tyr Ala Ala Ala Lys Glu Asn Asn Val Thr
   85 90 95
   Ile Val Cys Gly Ile Asn Glu Arg Asp Asn Arg Leu Ser Lys Ala Thr
   100 105 110
   Leu Tyr Asn Ser Ile Val Ile Ile Gly Ser Asp Gly Ser Leu Leu Asn
   115 120 125
   Arg His Arg Lys Leu Met Pro Thr Asn Pro Glu Arg Met Val Trp Gly
   130 135 140
   Phe Gly Asp Ala Ser Gly Leu Lys Val Val Asp Thr Pro Val Gly Arg
   145 150 155 160
   Val Gly Thr Leu Val Cys Trp Glu Asn Tyr Met Pro Leu Ala Arg Tyr
   165 170 175
   Ala Leu Tyr Ser Gln Gly Val Glu Val Tyr Ile Ala Pro Thr Tyr Asp
   180 185 190
   Ser Gly Asp Asp Trp Ile Ser Thr Leu Gln His Ile Ala Arg Glu Gly
   195 200 205
   Arg Cys Trp Val Val Gly Cys Gly Asn Leu Leu Arg Gly Ser Asp Ile
   210 215 220
   Pro Asp Asp Phe Pro Glu Lys Leu Ala Leu Tyr Pro Asp Glu Asp Glu
   225 230 235 240
   Trp Ile Asn Pro Gly Asp Ser Val Val Ile Ala Pro Gly Gly Lys Ile
   245 250 255
   Met Ala Gly Pro Leu Arg Gln Glu Ala Gly Ile Val Tyr Cys Asp Ile
   260 265 270
   Ala Ser Glu Ser Ala Ser Gln Ala Lys Arg Ala Leu Asp Val Ala Gly
   275 280 285
   His Tyr Ser Arg Pro Asp Ile Phe Glu Leu His Val Asn Thr Lys Val
   290 295 300
   Gln Thr Pro Val Val Tyr Asp
   305 310
<210> 381
   <211> 945
   <212> DNA
   <213> Clostridium acetobutylicum ATCC 3625
<400> 381
   atgggcgaat tcggtgaagt taccctgggc gtggctcagg ctgctcccgt gtactttgac 60
   cgcgaggcct cgaccgagaa agcctgtggc ctgatccgcg aggcgggcga aaagggtgta 120
   gatcttctgg cgttcggtga aacgtggtta accggctatc catactggaa agatgcgcct 180
   tggtctcgcg aatacaacga cttgcgtgca cgttatgttg cgaacggtgt gatgattcct 240
   ggtccggaaa cggacgctct gtgccaagca gccgcggaag caggtgtgga tgtggcgatc 300
   ggagtagtag aactggagcc gggctctctt tcctcggttt attgcactct gttatttata 360
   agccgcgagg gcgagatcct gggtcgtcac cgcaaactga aaccgaccga tagcgaacgt 420
   cgttactggt ctgaaggcga cgcgactggt ctgcgcgttt atgagcgccc atatggtcgg 480
   cttagcggcc tgaattgctg ggagcacact atgatgctgc cggggtacgc cctggcggcg 540
   cagggcaccc agttccatgt ggccgcttgg ccaaacatgg catcctcgaa ttctgaactt 600
   ctgtctcgtg cctacgctat gcaggcgggc tgctacgttt tatgcgcggg tggcctgggc 660
   ccggccccag gtgaactgcc ggatggtatc gcggcggaaa gtttagatca cttgactgga 720
   gagtcatgta tcatcgaccc gtgggggaaa gtaattgctg gtccggtgtc ttgcgaggaa 780
   acccttatca cggctcgcgt tagcaccgca tctatttatc gccgcaaaag tttgacggac 840
   gtgggcggtc attatagccg cccggatgtt ttccgttttg aagtcgatcg ctctgagcgt 900
   ccccgtgtcg tgtttcgcga tggtgacgtg gatgaccgag gttaa 945
<210> 382
   <211> 314
   <212> PRT
   <213> Clostridium acetobutylicum ATCC 3625
<400> 382
   Met Gly Glu Phe Gly Glu Val Thr Leu Gly Val Ala Gln Ala Ala Pro
   1               5                   10                  15
   Val Tyr Phe Asp Arg Glu Ala Ser Thr Glu Lys Ala Cys Gly Leu Ile
               20                  25                  30
   Arg Glu Ala Gly Glu Lys Gly Val Asp Leu Leu Ala Phe Gly Glu Thr
           35                  40                  45
   Trp Leu Thr Gly Tyr Pro Tyr Trp Lys Asp Ala Pro Trp Ser Arg Glu
       50                  55                  60
   Tyr Asn Asp Leu Arg Ala Arg Tyr Val Ala Asn Gly Val Met Ile Pro
   65                  70                  75                  80
   Gly Pro Glu Thr Asp Ala Leu Cys Gln Ala Ala Ala Glu Ala Gly Val
                   85                  90                  95
   Asp Val Ala Ile Gly Val Val Glu Leu Glu Pro Gly Ser Leu Ser Ser
               100                 105                 110
   Val Tyr Cys Thr Leu Leu Phe Ile Ser Arg Glu Gly Glu Ile Leu Gly
           115                 120                 125
   Arg His Arg Lys Leu Lys Pro Thr Asp Ser Glu Arg Arg Tyr Trp Ser
       130                 135                 140
   Glu Gly Asp Ala Thr Gly Leu Arg Val Tyr Glu Arg Pro Tyr Gly Arg
   145                 150                 155                 160
   Leu Ser Gly Leu Asn Cys Trp Glu His Thr Met Met Leu Pro Gly Tyr
                   165                 170                 175
   Ala Leu Ala Ala Gln Gly Thr Gln Phe His Val Ala Ala Trp Pro Asn
               180                 185                 190
   Met Ala Ser Ser Asn Ser Glu Leu Leu Ser Arg Ala Tyr Ala Met Gln
           195                 200                 205
   Ala Gly Cys Tyr Val Leu Cys Ala Gly Gly Leu Gly Pro Ala Pro Gly
       210                 215                 220
   Glu Leu Pro Asp Gly Ile Ala Ala Glu Ser Leu Asp His Leu Thr Gly
   225                 230                 235                 240
   Glu Ser Cys Ile Ile Asp Pro Trp Gly Lys Val Ile Ala Gly Pro Val
                   245                 250                 255
   Ser Cys Glu Glu Thr Leu Ile Thr Ala Arg Val Ser Thr Ala Ser Ile
               260                 265                 270
   Tyr Arg Arg Lys Ser Leu Thr Asp Val Gly Gly His Tyr Ser Arg Pro
           275                 280                 285
   Asp Val Phe Arg Phe Glu Val Asp Arg Ser Glu Arg Pro Arg Val Val
       290                 295                 300
   Phe Arg Asp Gly Asp Val Asp Asp Arg Gly
   305                 310
<210> 383
   <211> 1041
   <212> DNA
   <213> Unknown
<220>
   <223> Obtained from an environmental sample
<400> 383
   atgtcggagc ccatgacgaa gtatcgcggc gcggcggtgc aggccgcgcc ggtgttcctc      60
   gatctcgacc gcacagtcga gaaagcgatc ggcctgatcg agcaggcggc caagcaggac     120
   gtgcgcctga tcgcattccc agagacttgg attcccggct atcccttttg gatatggctg    180
   ggcgcgccgg cttggggcat gcgcttcgtc cagcgctatt tcgagaattc gctcgtgcgc     240
   ggcagcaagc agtggcaggc cctggcggat gcggcccgcc gccacggcat gcatgtcgtg     300
   gccggctata gcgagcgcgc gggcggcagc ctctatatgg gccaggcgat cttcggcccc     360
   gatggcgatc tgatcgccgc gcgccgcaag ctcaagccta cccatgcgga gcgcaccgtg     420
   ttcggcgagg gagacggcag ccatctcgcg gtgcacgata ccgccatcgg gcgcctcggc     480
   gcgctctgtt gctgggagca catccagcca ttgtcgaaat acgccatgta cgccgccgac     540
   gaacaggtcc acgtcgcgtc gtggccgagc ttcagcctct atcgcggcat ggcctatgcg     600
   ctcggaccgg aggtcaatac cgccgcaagc cagatctacg cggtcgaggg cggctgctac     660
   gtgctggcgt cgtgcgcgac cgtttcgccg gagatgatca aggtattggt ggatacgccc     720
   gacaaggaga tgttcctcaa ggccggcggc ggttttgcca tgattttcgg gcccgacggc     780
   cgcgccctgg ccgagccgct cccggagacc gaagagggac tgctggtcgc cgatatcgac     840
   ctcggcatga tcgcgttggc caaggcggcg gccgatccgg cgggccacta ttcacggccc     900
   gacgtaacgc ggctgctgct ggatcgacgt ccggcccaac gcgtcgtcac gcttgatgcc     960
   gcattcgaac cgcaaaacga ggacaagggc gacgcgcccg cgctgcgcgt ggtggcggaa    1020
   agcgccgccg ccgcgcagta g                                              1041
<210> 384
   <211> 346
   <212> PRT
   <213> Unknown
<220>
   <223> Obtained from an environmental sample
<400> 384
   Met Ser Glu Pro Met Thr Lys Tyr Arg Gly Ala Ala Val Gln Ala Ala
   1               5                   10                  15
   Pro Val Phe Leu Asp Leu Asp Arg Thr Val Glu Lys Ala Ile Gly Leu
               20                  25                  30
   Ile Glu Gln Ala Ala Lys Gln Asp Val Arg Leu Ile Ala Phe Pro Glu
           35                  40                  45
   Thr Trp Ile Pro Gly Tyr Pro Phe Trp Ile Trp Leu Gly Ala Pro Ala
       50                  55                  60
   Trp Gly Met Arg Phe Val Gln Arg Tyr Phe Glu Asn Ser Leu Val Arg
   65                  70                  75                  80
   Gly Ser Lys Gln Trp Gln Ala Leu Ala Asp Ala Ala Arg Arg His Gly
                   85                  90                  95
   Met His Val Val Ala Gly Tyr Ser Glu Arg Ala Gly Gly Ser Leu Tyr
               100                 105                 110
   Met Gly Gln Ala Ile Phe Gly Pro Asp Gly Asp Leu Ile Ala Ala Arg
           115                 120                 125
   Arg Lys Leu Lys Pro Thr His Ala Glu Arg Thr Val Phe Gly Glu Gly
       130                 135                 140
   Asp Gly Ser His Leu Ala Val His Asp Thr Ala Ile Gly Arg Leu Gly
   145                 150                 155                 160
   Ala Leu Cys Cys Trp Glu His Ile Gln Pro Leu Ser Lys Tyr Ala Met
                   165                 170                 175
   Tyr Ala Ala Asp Glu Gln Val His Val Ala Ser Trp Pro Ser Phe Ser
               180                 185                 190
   Leu Tyr Arg Gly Met Ala Tyr Ala Leu Gly Pro Glu Val Asn Thr Ala
           195                 200                 205
   Ala Ser Gln Ile Tyr Ala Val Glu Gly Gly Cys Tyr Val Leu Ala Ser
       210                 215                 220
   Cys Ala Thr Val Ser Pro Glu Met Ile Lys Val Leu Val Asp Thr Pro
   225                 230                 235                 240
   Asp Lys Glu Met Phe Leu Lys Ala Gly Gly Gly Phe Ala Met Ile Phe
                   245                 250                 255
   Gly Pro Asp Gly Arg Ala Leu Ala Glu Pro Leu Pro Glu Thr Glu Glu
               260                 265                 270
   Gly Leu Leu Val Ala Asp Ile Asp Leu Gly Met Ile Ala Leu Ala Lys
           275                 280                 285
   Ala Ala Ala Asp Pro Ala Gly His Tyr Ser Arg Pro Asp Val Thr Arg
       290                 295                 300
   Leu Leu Leu Asp Arg Arg Pro Ala Gln Arg Val Val Thr Leu Asp Ala
   305                 310                 315                 320
   Ala Phe Glu Pro Gln Asn Glu Asp Lys Gly Asp Ala Pro Ala Leu Arg
                   325                 330                 335
   Val Val Ala Glu Ser Ala Ala Ala Ala Gln
               340                 345
<210> 385
   <211> 1014
   <212> DNA
   <213> Unknown
<220>
   <223> Obtained from an environmental sample
<400> 385
   atgaaagaag ctatcaaggt cgcctgcgtg caagccgccc cgatctacat ggatttgaag     60
   gcgacggtgg acaaaaccat tgagttgatg gaagaagcag cacgtaataa tgctcgtctg    120
   atcgcctttc cggaaacttg gattccaggc tacccatggt ttctttggct tgactcacca    180
   gcatgggcaa tgcaatttgt acgccaatac catgagaact cattggagtt ggatggccct    240
   caagctaagc gcatttcaga tgcagccaag cggttgggaa tcatggtcac cctggggatg    300
   agtgaacggg tcggtggcac cctttacatc agtcagtggt tcataggcga taatggtgac    360
   accattgggg cccggcgaaa gttgaaacct acttttgttg aacgtacttt gttcggcgaa    420
   ggggatggtt catcgctagc ggttttcgag acgtctgttg gaaggctggg tggcttatgc    480
   tgttgggagc accttcaacc gctaacaaaa tacgctttgt atgcacaaaa tgaagagatt    540
   cattgtgcgg cttggccgag ctttagcctt tatcctaatg cggcgaaagc cctggggcct    600
   gatgtcaatg tagcggcctc tcgaatctat gccgttgaag ggcaatgctt cgtactagcg    660
   tcgtgtgcgc tcgtttcaca atccatgatc gatatgcttt gtacagatga cgaaaagcat    720
   gcgttgcttc tggctggtgg tggacactca cgtatcatag ggcctgatgg tggtgacttg    780
   gtcgcgcctc ttgccgaaaa tgaagagggt attctctacg caaaccttga tcctggagta    840
   cgcatccttg ctaaaatggc ggcagaccct gctggtcatt attcccgtcc cgacattact    900
   cgcttgctaa tagatcgcag ccctaaatta ccggtagttg aaattgaagg tgatcttcgt    960
   ccttacgctt tgggtaaagc gtctgagacg ggtgcgcaac tcgaagaaat ttga         1014
<210> 386
   <211> 337
   <212> PRT
   <213> Unknown
<220>
   <223> Obtained from an environmental sample
<400> 386
   Met Lys Glu Ala Ile Lys Val Ala Cys Val Gln Ala Ala Pro Ile Tyr
    1               5                  10                  15
   Met Asp Leu Lys Ala Thr Val Asp Lys Thr Ile Glu Leu Met Glu Glu
               20                  25                  30
   Ala Ala Arg Asn Asn Ala Arg Leu Ile Ala Phe Pro Glu Thr Trp Ile
           35                  40                  45
   Pro Gly Tyr Pro Trp Phe Leu Trp Leu Asp Ser Pro Ala Trp Ala Met
       50                  55                  60
   Gln Phe Val Arg Gln Tyr His Glu Asn Ser Leu Glu Leu Asp Gly Pro
   65                  70                  75                  80
   Gln Ala Lys Arg Ile Ser Asp Ala Ala Lys Arg Leu Gly Ile Met Val
                   85                  90                  95
   Thr Leu Gly Met Ser Glu Arg Val Gly Gly Thr Leu Tyr Ile Ser Gln
               100                 105                 110
   Trp Phe Ile Gly Asp Asn Gly Asp Thr Ile Gly Ala Arg Arg Lys Leu
           115                 120                 125
   Lys Pro Thr Phe Val Glu Arg Thr Leu Phe Gly Glu Gly Asp Gly Ser
       130                 135                 140
   Ser Leu Ala Val Phe Glu Thr Ser Val Gly Arg Leu Gly Gly Leu Cys
   145                 150                 155                 160
   Cys Trp Glu His Leu Gln Pro Leu Thr Lys Tyr Ala Leu Tyr Ala Gln
                   165                 170                 175
   Asn Glu Glu Ile His Cys Ala Ala Trp Pro Ser Phe Ser Leu Tyr Pro
               180                 185                 190
   Asn Ala Ala Lys Ala Leu Gly Pro Asp Val Asn Val Ala Ala Ser Arg
           195                 200                 205
   Ile Tyr Ala Val Glu Gly Gln Cys Phe Val Leu Ala Ser Cys Ala Leu
       210                 215                 220
   Val Ser Gln Ser Met Ile Asp Met Leu Cys Thr Asp Asp Glu Lys His
   225                 230                 235                 240
   Ala Leu Leu Leu Ala Gly Gly Gly His Ser Arg Ile Ile Gly Pro Asp
                   245                 250                 255
   Gly Gly Asp Leu Val Ala Pro Leu Ala Glu Asn Glu Glu Gly Ile Leu
               260                 265                 270
   Tyr Ala Asn Leu Asp Pro Gly Val Arg Ile Leu Ala Lys Met Ala Ala
           275                 280                 285
   Asp Pro Ala Gly His Tyr Ser Arg Pro Asp Ile Thr Arg Leu Leu Ile
       290                 295                 300
   Asp Arg Ser Pro Lys Leu Pro Val Val Glu Ile Glu Gly Asp Leu Arg
   305                 310                 315                 320
   Pro Tyr Ala Leu Gly Lys Ala Ser Glu Thr Gly Ala Gln Leu Glu Glu
                   325                 330                 335
   Ile
<210> 387
   <211> 5
   <212> PRT
   <213> Unknown
<220>
   <223> Obtained from an environmental sample
<400> 387
   Phe Pro Glu Thr Phe
   1 5
<210> 388
   <211> 7
   <212> PRT
   <213> Unknown
<220>
   <223> Obtained from an environmental sample
<220>
   <221>
   <222>
   <223> Xaa = a residue not conserved
<400> 388
   Arg Arg Lys Leu Xaa Pro Thr
   1 5
<210> 389
   <211> 9
   <212> PRT
   <213> Unknown
<220>
   <223> Obtained from an environmental sample
<220>
   <221>
   <222>
   <223> Xaa = a residue not conserved
<400> 389
   Leu Xaa Cys Trp Glu His Xaa Xaa Pro
   1 5
<210> 390
   <211> 5
   <212> PRT
   <213> Unknown
<220>
   <223> Obtained from an environmental sample
<220>
   <221>
   <222>
   <223> Xaa = a residue not conserved
<400> 390
   Phe Pro Glu Xaa Xaa
   1 5
<210> 391
   <211> 7
   <212> PRT
   <213> Unknown
<220>
   <223> Obtained from an environmental sample
<220>
   <221>
   <222>
   <223> Xaa = a residue not conserved
<400> 391
   Xaa Arg Lys Leu Xaa Pro Thr
   1 5
<210> 392
   <211> 9
   <212> PRT
   <213> Unknown
<220>
   <223> Obtained from an environmental sample
<220>
   <221>
   <222>
   <223> Xaa = a residue not conserved
<400> 392
   Leu Xaa Cys Trp Glu Xaa Xaa Xaa Pro
   1 5